(19) **Europäisches Patentamt / European Patent Office / Office européen des brevets**

(11) **EP 1 851 310 B1**

(12) **EUROPEAN PATENT SPECIFICATION**

(45) Date of publication and mention
of the grant of the patent:
**26.01.2011 Bulletin 2011/04**

(21) Application number: **06735797.0**

(22) Date of filing: **23.02.2006**

(51) Int Cl.:
*C12N 9/12* (2006.01)          *C12N 15/54* (2006.01)
*C12N 15/62* (2006.01)       *G01N 33/48* (2006.01)
*G06F 19/00* (2011.01)       *C30B 29/58* (2006.01)

(86) International application number:
**PCT/US2006/006286**

(87) International publication number:
**WO 2006/091673 (31.08.2006 Gazette 2006/35)**

(54) **CRYSTAL STRUCTURE OF TAK1-TAB1**

KRISTALLSTRUKTUR VON TAK1-TAB1

STRUCTURE CRISTALLINE DE TAK1-TAB1

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.02.2005 US 655606 P**

(43) Date of publication of application:
**07.11.2007 Bulletin 2007/45**

(73) Proprietor: **VERTEX PHARMACEUTICALS INCORPORATED**
**Cambridge, MA 02139 (US)**

(72) Inventors:
• **CHEETHAM, Graham**
**Abingdon Oxfordshire OX14 4RY (GB)**
• **BROWN, Kieron**
**Abingdon Oxfordshire OX14 4RY (GB)**
• **VIAL, Sarah**
**Abingdon Oxfordshire OX14 4RY (GB)**
• **DEDI, Neesha**
**Abingdon Oxfordshire OX14 4RY (GB)**

(74) Representative: **Vossius & Partner**
**Siebertstrasse 4**
**81675 München (DE)**

(56) References cited:
**WO-A-03/048340          WO-A-03/092607**
**WO-A-2004/087862     US-A1- 2004 204 863**

• **SAKURAI HIROAKI ET AL: "TAK1-TAB1 fusion protein: a novel constitutively active mitogen-activated protein kinase kinase kinase that stimulates AP-1 and NF-kappaB signaling pathways." BIOCHEMICAL AND BIOPHYSICAL RESEARCH COMMUNICATIONS. 11 OCT 2002, vol. 297, no. 5, 11 October 2002 (2002-10-11), pages 1277-1281, XP002418083 ISSN: 0006-291X cited in the application**
• **DATABASE WPI Week 200360 Derwent Publications Ltd., London, GB; AN 2003-630296 XP002418088 -& JP 2003 135070 A (TANABE SEIYAKU CO) 13 May 2003 (2003-05-13)**
• **SAKURAI H ET AL: "Phosphorylation-dependent activation of TAK1 mitogen-activated protein kinase kinase kinase by TAB1" FEBS LETTERS, ELSEVIER, AMSTERDAM, NL, vol. 474, no. 2-3, 2 June 2000 (2000-06-02), pages 141-145, XP004337196 ISSN: 0014-5793**
• **ONO K ET AL: "An evolutionarily conserved motif in the TAB1 C-terminal region is necessary for interaction with and activation of TAK1 MAPKKK." THE JOURNAL OF BIOLOGICAL CHEMISTRY. 29 JUN 2001, vol. 276, no. 26, 29 June 2001 (2001-06-29), pages 24396-24400, XP002418111 ISSN: 0021-9258**
• **BROWN KIERON ET AL: "Structural Basis for the Interaction of TAK1 Kinase with its Activating Protein TAB1." JOURNAL OF MOLECULAR BIOLOGY. 16 DEC 2005, vol. 354, no. 5, 16 December 2005 (2005-12-16), pages 1013-1020, XP002418084 ISSN: 0022-2836**

**Description**

TECHNICAL FIELD OF THE INVENTION

The present invention relates to the design of crystallisable transforming growth factor-beta-activated kinase 1 (TAK1) and TAK1 binding protein (TAB1) complexes and the X-ray analysis of crystalline molecules or molecular complexes of this TAK1-TAB 1 chimera. The present invention provides a chimera of TAK1 and a region of the TAK1 activating domain of its protein activator TAB1. The present invention also provides for the first time the crystal structure of a TAK1-TAB1 chimera protein bound to adenosine and TAK1-TAB1 bound to a potent ATP-competitive inhibitor. The present invention also provides crystalline molecules or molecular complexes that comprise binding pockets of TAK1 kinase (TAK1) and/or its structural homologues, the structure of these molecules or molecular complexes. The present invention further provides crystals of TAK1-TAB1 complexed with adenosine and methods for producing these crystals. This invention also relates to a general strategy for the design of crystallisable protein kinases based on both sequence and structural alignments of related protein kinases with close homology that have previously been crystallized in the literature. This invention also relates to crystallizable compositions from which the protein-ligand complexes may be obtained. The present invention also relates to a data storage medium encoded with the structural coordinates of molecules and molecular complexes that comprise the ATP-binding pockets and TAB1-binding pockets of TAK1 or their structural homologues. The present invention also relates to a computer comprising such data storage material. The computer may generate a three-dimensional structure or graphical three-dimensional representation of such molecules or molecular complexes. This invention also relates to methods of using the structure coordinates to solve the structure of homologous proteins or protein complexes. This invention also relates to computational methods of using structure coordinates of the TAK1 complex(es) to screen for and design compounds, including inhibitory compounds and antibodies, that interact with TAK1, TAB1 or homologues thereof.

BACKGROUND OF THE INVENTION

The search for new therapeutic agents has been greatly aided in recent years by a better understanding of the structure of enzymes and other biomolecules associated with diseases. One important class of enzymes that has been the subject of extensive study is protein kinases.

Protein kinases constitute a large family of structurally related enzymes that are responsible for the control of a variety of signal transduction processes within the cell. (See, Hardie, G. and Hanks, S. The Protein Kinase Facts Book, I and II, Academic Press, San Diego, CA: 1995). Protein kinases are thought to have evolved from a common ancestral gene due to the conservation of their structure and catalytic function. Almost all kinases contain a similar 250-300 amino acid catalytic domain. The kinases may be categorized into families by the substrates they phosphorylate (*e.g.*, protein-tyrosine, protein-serine/threonine, lipids, etc.). Sequence motifs have been identified that generally correspond to each of these kinase families (See, for example, Hanks, S.K., Hunter, T., FASEB J., 9:576-596 (1995); Knighton et al., Science, 253:407-414 (1991); Hiles et al., Cell, 70:419-429 (1992); Kunz et al., Cell, 73:585-596 (1993); Garcia-Bustos et al., EMBO J., 13:2352-2361 (1994)).

In general, protein kinases mediate intracellular signaling by effecting a phosphoryl transfer from a nucleoside triphosphate to a protein acceptor that is involved in a signaling pathway. These phosphorylation events act as molecular on/off switches that can modulate or regulate the target protein biological function. These phosphorylation events are ultimately triggered in response to a variety of extracellular and other stimuli. Examples of such stimuli include environmental and chemical stress signals (*e.g.*, osmotic shock, heat shock, ultraviolet radiation, bacterial endotoxin, and $H_2O_2$), cytokines (*e.g.*, interleukin-1 (IL-1) and tumor necrosis factor $\alpha$ (TNF-$\alpha$)), and growth factors (*e.g.*, granulocyte macrophage-colony-stimulating factor (GM-CSF), and fibroblast growth factor (FGF)). An extracellular stimulus may affect one or more cellular responses related to cell growth, migration, differentiation, secretion of hormones, activation of transcription factors, muscle contraction, glucose metabolism, control of protein synthesis, and regulation of the cell cycle.

Many diseases are associated with abnormal cellular responses triggered by protein kinase-mediated events as described above. These diseases include, but are not limited to, autoimmune diseases, inflammatory diseases, bone diseases, metabolic diseases, neurological and neurodegenerative diseases, cancer, cardiovascular diseases, allergies and asthma, Alzheimer's disease, and hormone-related diseases. Accordingly, there has been a substantial effort in medicinal chemistry to find protein kinase inhibitors that are effective as therapeutic agents.

Among medically important serine/threonine kinases is the family of mitogen-activated protein kinases (MAPKs), which have been shown to function in a wide variety of biological processes (Davis D.J. Trends in Biochem Sci. 19 470-473 (1994); Su B. & Karin M Curr. Opin. Immunol 8 402-411 (1996); Treisman R. Curr. Opin. Cell Biol. 8 205-215 (1996)). MAPKs are activated by phosphorylation on specific tyrosine and threonine residues by MAPK kinases (MAPKKs), which are in turn activated by phosphorylation on serine and serine/threonine residues by MAPKK kinases (MAPKKKs). The MAPKKK family comprises several members including MEKK1, MEKK3, NIK and ASK1 and Raf. Different mechanisms are involved in the activation of MAPKKKs in response to a variety of extracellular stimuli including cytokines, growth factors and environmental stresses (refs).

Transforming growth factor-β (TGF-β)-activated kinase 1 (TAK1) is a member of the mitogen-activated protein kinase kinase kinase (MAPKKK) family and has been shown to play critical roles in signaling pathways stimulated by transforming growth factor-β, interleukin-1 (IL-1), tumor necrosis factor-α (TNF-α), lipopolysaccharide, receptor activator of NF-κB ligand where it regulates osteoclast differentiation and activation, and IL-8 (Yamaguchi K et al. Science 270 2008-11 (1995); Ninomiya-Tsuji J et al. Nature 398 252-256 (1999); Sakurai H. et al. J. Biol. Chem 274 10641-10648 (1999); Irie T. et al. FEBS Lett. 467 160-164 (2000); Lee J. et al. J. Leukoc Biol. 68 909-915 (2000); Mizukami J et al. Mol. Cell. Biol. 22 992-1000 (2002); Wald D. et al. J. Immunol 31 3747-3754 (2002)). TAK1 regulates both the c-Jun N-terminal kinase (JNK) and p38 MAPK cascades in which it phosphorylates MAPK kinases MKK4 and MKK3/6, respectively (Wang W. et al. J. Biol. Chem. 272 22771-22775 (1997); Moriguchi T. et al. J. Biol. Chem. 271 13675-13679 (1996)). NF-kB factors regulate expression of a variety of genes involved in apoptosis, cell cycle, transformation, immune response, and cell adhesion (Barkett M and Gilmore TD. Oncogene, 18, 6910-6924 (1999). TAK1 regulates the IκB kinase (IKK) signaling pathways, leading to the activation of transcription factors AP-1 and NF-κB (Ninomiya-Tsuji J et al. Nature 398 252-256 (1999); Sakurai H. et al. J. Biol. Chem 274 10641-10648 (1999); Takaesu G. et al. J. Mol. Biol 326 110-115 (2003)). In early embryos of the amphibian Xenopus, TAK1 also participates in mesoderm induction and patterning mediated by bone morphogenetic protein (BMP), which is another transforming growth factor β family ligand (Shibuya H. et al. EMBO J. 17 1019-1028 (1998)). In addition, TAK1 is a negative regulator of the Wnt signaling pathway, in which TAK1 down-regulates transcription regulation mediated by a complex of β-catenin and T-cell factor/lymphoid enhancer factor (Meneghini M.D. et al. Nature 399 793-797 (1999); Ishitani T. et al. Nature 399 798-802 (1999)). The role of TAK1 in TNF-α and IL-1β-induced signaling events is evident from TAK1 RNAi experiments in mammalian cells (Takaesu G. et al. J. Mol. Biol. 326 105-115 (2003)) in which IL-1 and TNF-α induced NF-κB and MAPK activation were both inhibited. Over-expression of kinase dead TAK1 inhibits IL-1 and TNK-induced activation of both JNK/p38 and NF-kB (Ninomiya-Tsuji J et al. Nature 398 252-256 (1999); Sakurai H. et al. J. Biol. Chem 274 10641-10648 (1999)). TAK1-/- mouse embryonic fibroblasts have diminished IL-1-induced signaling and are embryonic lethal (E11.5) (S. Akira, personal communication). In adult mouse, TAK1 is activated in the myocardium after pressure overload. Expression of constitutively-active TAK1 in myocardium induced myocardial hypertrophy and heart failure in transgenic mice (Zhang D. et al. Nature Med. 6 556-563 (2000)).

TAK1 is activated by the TAK1 binding protein (TAB1) (Shibuya H et al. Science 272 1179-1182 (1996)) via an association with the N-terminal kinase domain of TAK1. It has been reported that the C-terminal 68 amino acids of TAB1 is sufficient for the association and activation of TAK1 (Shibuya H et al. Science 272 1179-1182 (1996)). However, more recent work indicates that the minimum TAB1 segment required includes only residues 480-495 (Ono K. et al. J. Biol. Chem. 276 24396-24400 (2001); Sakurai H. et al. FEBS Lett 474 141-145 (2000)). Deletion mutants of TAB 1 show that the aromatic Phe484 residue is critical for TAK1 binding (Ono K. et al. J. Biol. Chem. 276 24396-24400 (2001)). Autophosphorylation of threonine/serine residues in the kinase activation loop are necessary for TAB1-induced TAK1 activation (Sakurai H. et al. FEBS Lett 474 141-145 (2000); Kishimoto K. et al. J. Biol. Chem. 275 7359-7364 (2000)), Ser192 appears as the most likely candidate since a Ser192Ala mutation shows no kinase activity (Kishimoto K. et al. J. Biol. Chem. 275 7359-7364 (2000)).

Since TAK1 is a key molecule in the pro-inflammatory NF-κB signaling pathway a TAK1 inhibitor would be effective in diseases associated with inflammation and tissue destruction such as rheumatoid arthritis and inflammatory bowel disease (Crohn's), as well as in cellular processes such as stress responses, apoptosis, proliferation and differentiation. Various pro-inflammatory cytokines and endotoxins trigger the kinase activity of endogenous TAK1 (Ninomiya-Tsuji J et al. Nature 398 252-256 (1999); Irie T et al. FEBS Lett. 467 160-164 (2000); Sakurai H. et al. J. Biol. Chem. 274 10641-10648 (1999)) and the Drosophila homolog of TAK1 was recently identified as an essential molecule for host defense signaling in Drosophila (Vidal S. et al. Genes Dev. 15 1900-1912 (1999)). A natural inhibitor of TAK1, 5Z-7-oxozeaenol, has been identified with an IC50 value of 8nM. 5Z-7-oxozeaenol has been shown to be selective for TAK1 within the MAPKKK family and relieves inflammation in a picryl chloride-induced ear swelling mouse model (Ninomiya-Tsuji J. et al. J. Biol. Chem. 278 18485 (2003)).

Accordingly, there has been an interest in finding selective inhibitors of TAK1 that are effective as therapeutic agents. A challenge has been to find protein kinase inhibitors that act in a selective manner, targeting only TAK1. Since there are numerous protein kinases that are involved in a variety of cellular responses, non-selective inhibitors may lead to unwanted side effects. In this regard, the three-dimensional structure of the kinase would assist in the rational design of inhibitors. The determination of the amino acid residues in TAK1 binding pockets and the determination of the shape of those binding pockets would allow one to design selective inhibitors that bind favorably to this class of enzymes. The determination of the amino acid residues in TAK1 binding pockets and the determination of the shape of those binding pockets would also allow one to determine the binding of compounds to the binding pockets and to, e.g., design inhibitors that can bind to TAK1.

For example, a general approach to designing inhibitors that are selective for an enzyme target is to determine how a putative inhibitor interacts with the three dimensional structure of the enzyme. For this reason it is useful to obtain the enzyme protein in crystal form and perform X-ray diffraction techniques to determine its three dimensional structure coordinates. If the enzyme is crystallized as a complex with a ligand, one can determine both the shape of the enzyme binding pocket when bound to the ligand, as well as the amino acid residues that are capable of close contact with the ligand. By knowing the shape and amino acid residues in the binding pocket, one may design new ligands that will interact favorably with the enzyme. With such structural information, available computational methods may be used to predict how strong the ligand binding interaction will be. Such methods thus enable the design of inhibitors that bind strongly, as well as selectively to the target enzyme.

Despite the fact that the genes for TAK1 has been isolated and the amino acid sequence of TAK1 is known, no one has described X-ray crystal structural coordinate information of TAK protein. As disclosed herein, such information would be extremely useful in identifying and designing potential inhibitors of the TAK kinase or homologues thereof, which, in turn, could have therapeutic utility.

The structures of several serine/threonine kinases have been solved by X-ray diffraction and analyzed. Specifically, the crystal structures of P38 kinase (Wilson et al., J. Biol. Chem., 271, pp. 27696-27700 (1996)) and MAPKAP Kinase 2 (United States Provisional application 60/337,513) have been studied in detail.

To date, no crystal structures of TAK kinase have been reported. Thus the crystal structure of unphosphorylated TAK kinase domain complexes with inhibitors are of great importance for defining the active conformation of TAK kinase. This information is essential for the rational design of selective and potent inhibitors of TAK.

SUMMARY OF THE INVENTION

The present invention discloses for the first time, crystallizable compositions, crystals, and the crystal structures of a TAK1 - inhibitor complex. The TAK1 protein used in these studies corresponds to a single polypeptide chain, which encompasses the complete catalytic kinase domain, amino acids 31 to 303 fused to the C-terminal 36 amino acids of TAB1 (468 to 504). Solving this crystal structure has allowed the applicants to determine the key structural features of TAK1, particularly the shape of its substrate and ATP-binding pockets.

Thus, in one aspect, the present invention discloses molecules or molecular complexes comprising all or parts of these binding pockets, or homologues of these binding pockets that have similar three-dimensional shapes.

In another aspect, the present invention further discloses crystals of TAK1 complexed with adenosine and methods for producing these crystals. In this embodiment, TAK1 is unphosphorylated.

In a further aspect, the present invention discloses crystallizable compositions from which TAK1-ligand complexes may be obtained.

In another aspect, the present invention discloses for a general strategy for the design of protein constructs for producing crystallisable kinase domains.

In another aspect, the invention discloses a data storage medium that comprises the structure coordinates of molecules and molecular complexes that comprise all or part of the TAK1 binding pockets. Such storage medium encoded with these data when read and utilized by a computer programmed with appropriate software displays, on a computer screen or similar viewing device, a three-dimensional graphical representation of a molecule or molecular complex comprising such binding pockets or similarly shaped homologous binding pockets.

In yet another aspect, the invention discloses computational methods of using structure coordinates of the TAK1 complex to screen for and design compounds, including inhibitory compounds and antibodies that interact with TAK1 or homologues thereof. In certain embodiments, the invention discloses methods for designing, evaluating and identifying compounds, which bind to the aforementioned binding pockets. In certain embodiments, such compounds are potential inhibitors of TAK1 or their homologues.

In a further aspect, the invention discloses a method for determining at least a portion of the three-dimensional structure of molecules or molecular complexes which contain at least some structurally similar features to TAK1, particularly ITK, LCK and MAPKAP kinase2 and their homologues. In certain embodiments, this is achieved by using at least some of the structural coordinates obtained from the TAK1 complexes. The present invention provides an isolated purified TAK1 construct as claimed in claim 1, crystals comprising a TAK1 kinase domain directly fused to a TAK1-activating domain of TAB1 as claimed in claims 2-8. A. crystallizable composition comprising the same according to claims 9-13. Moreover, the present invention provides for the methods claimed in claims 14-16.

BRIEF DESCRIPTION OF THE FIGURES

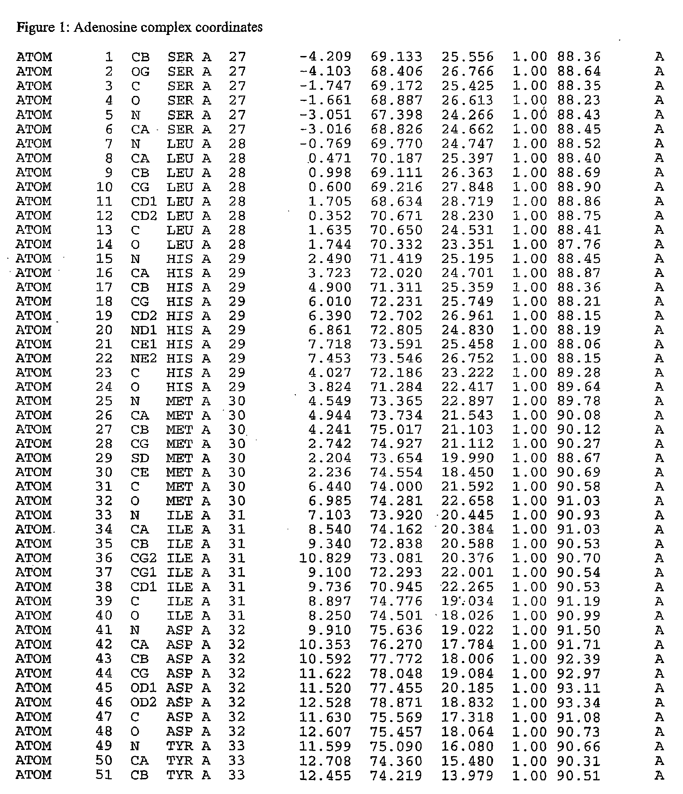

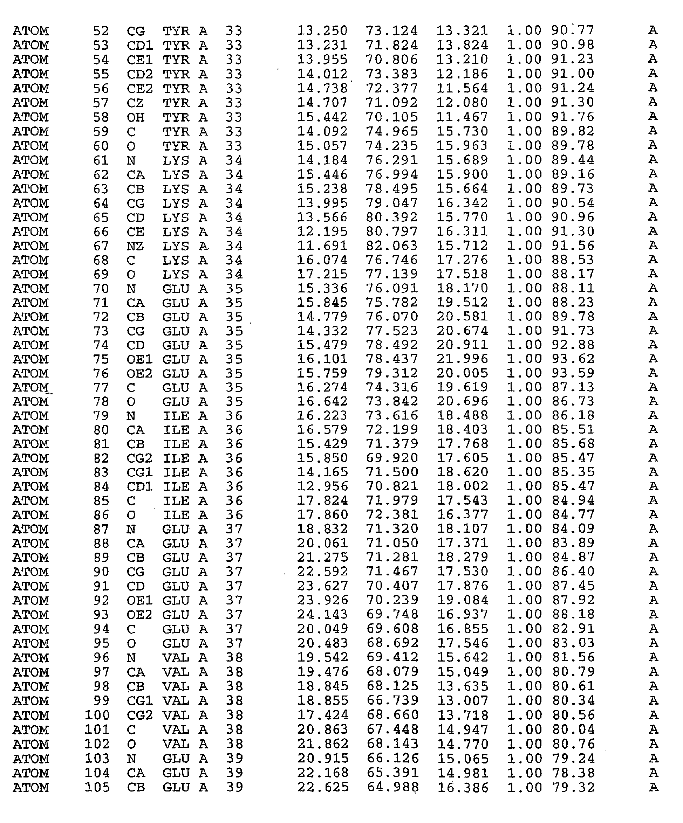

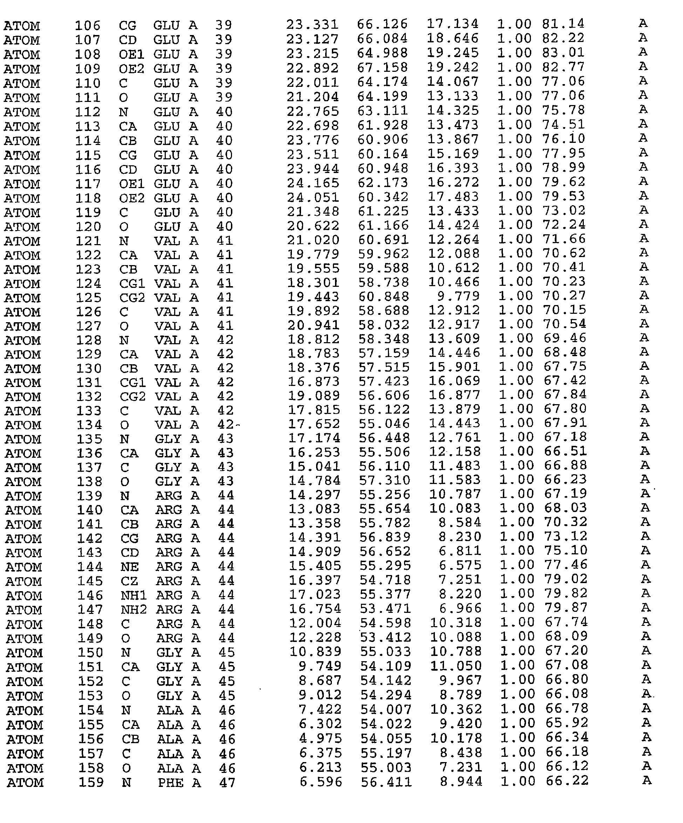

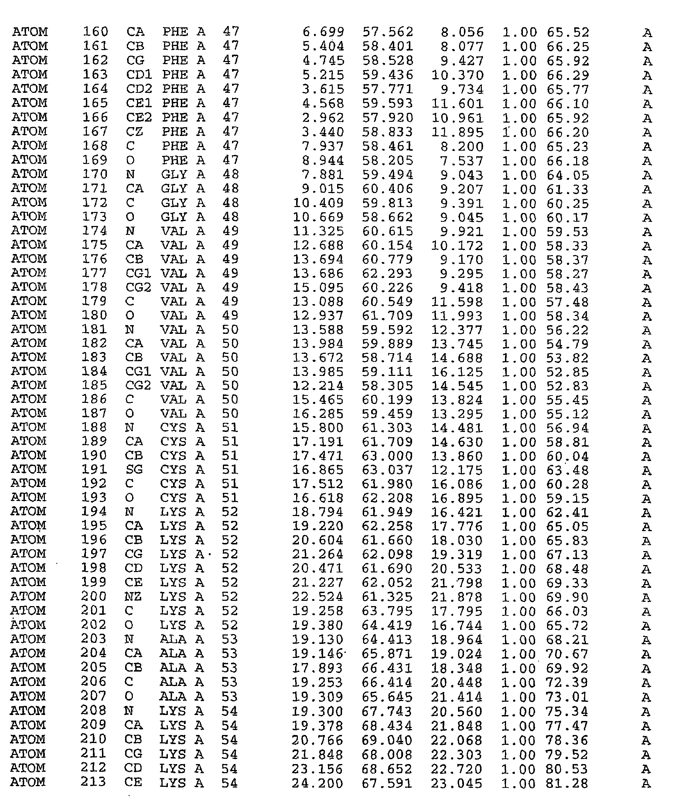

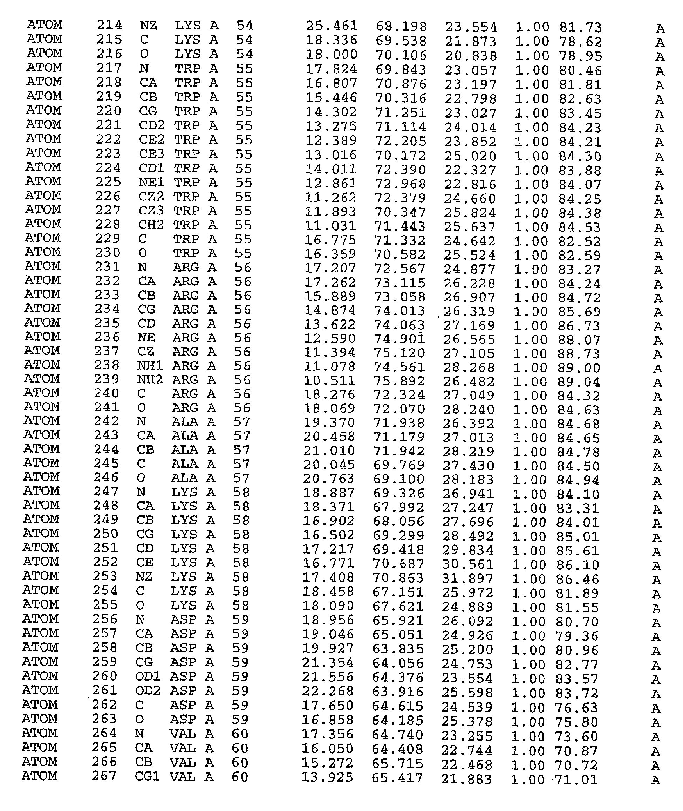

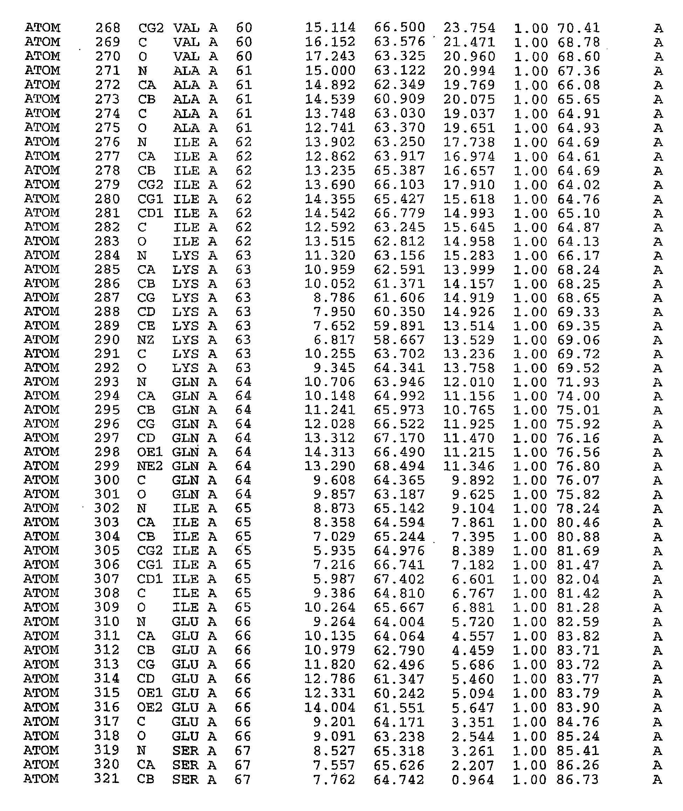

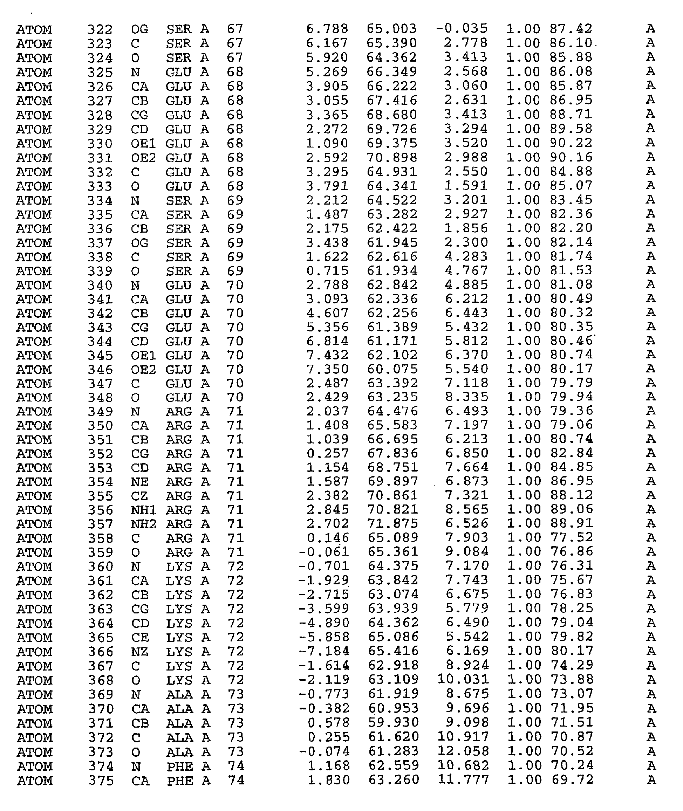

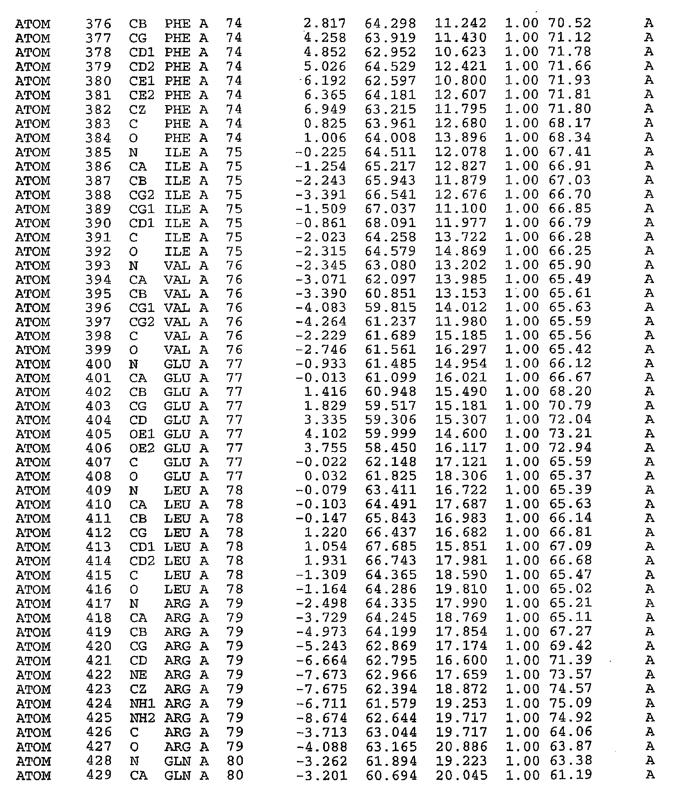

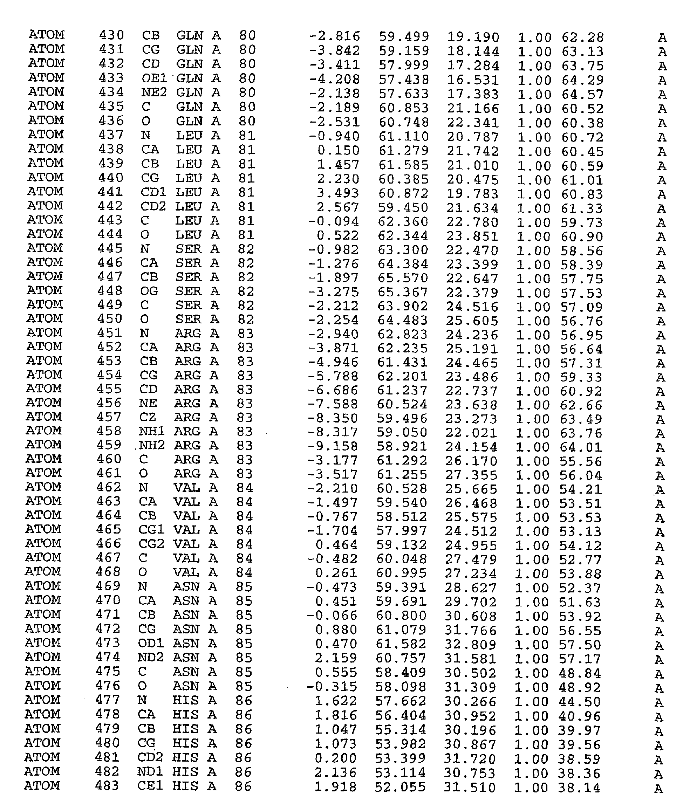

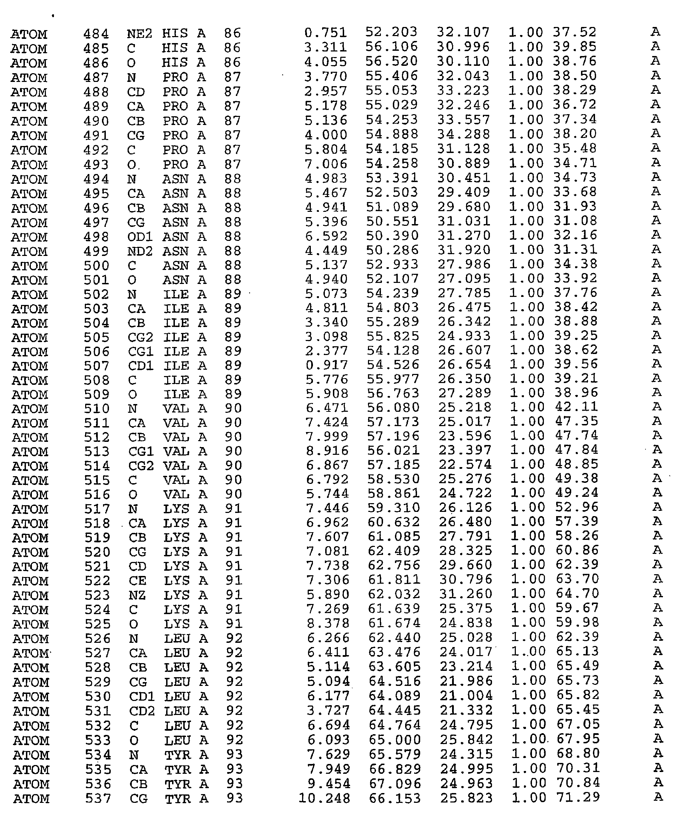

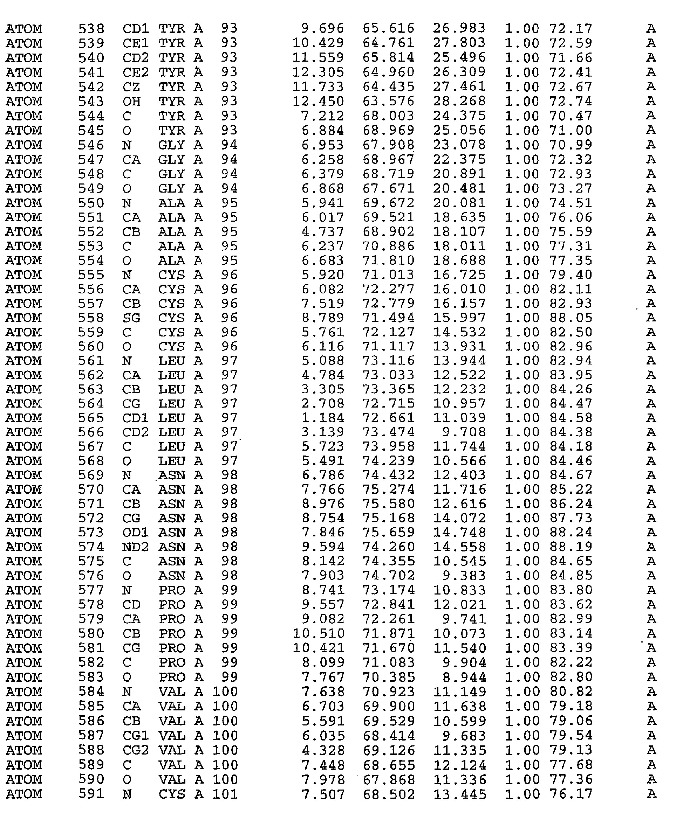

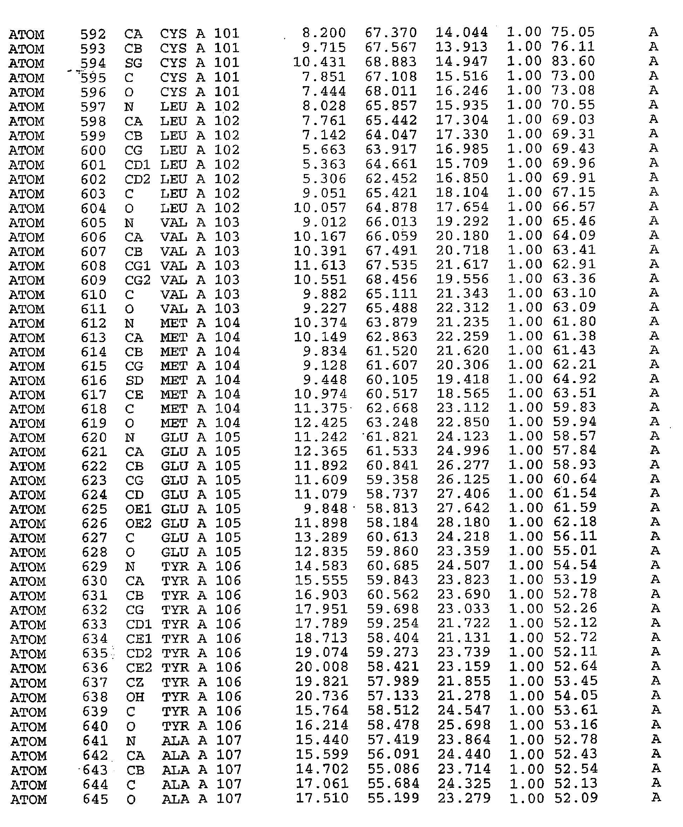

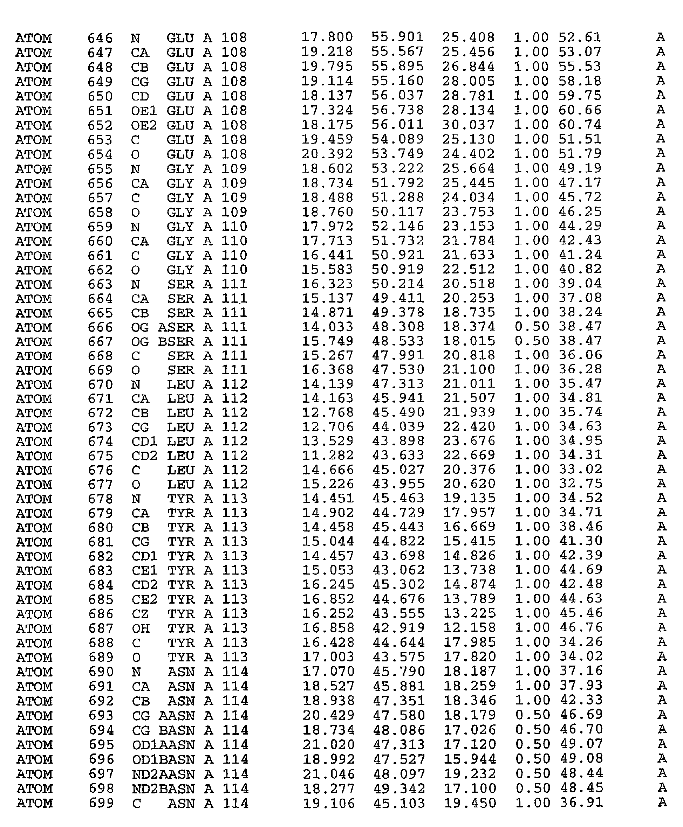

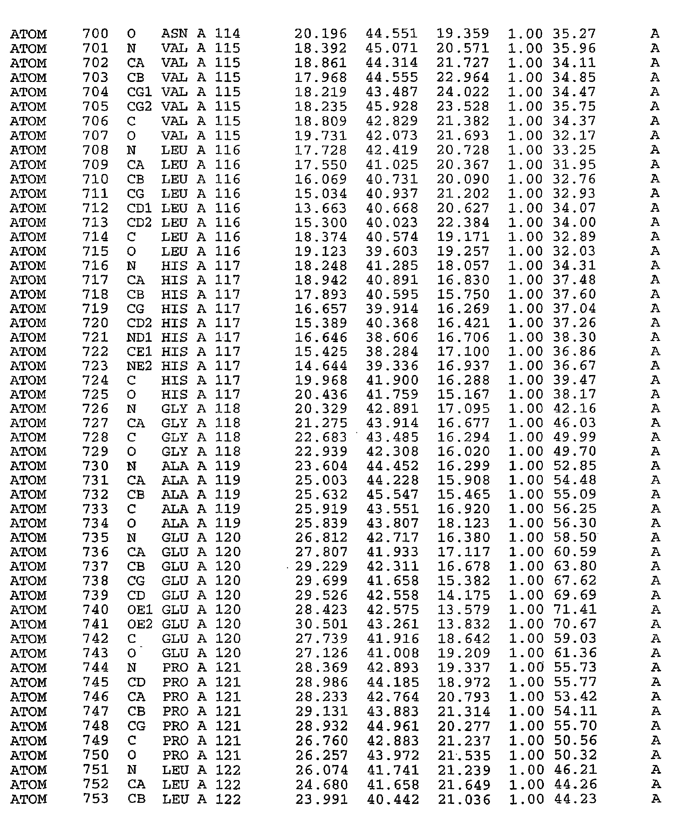

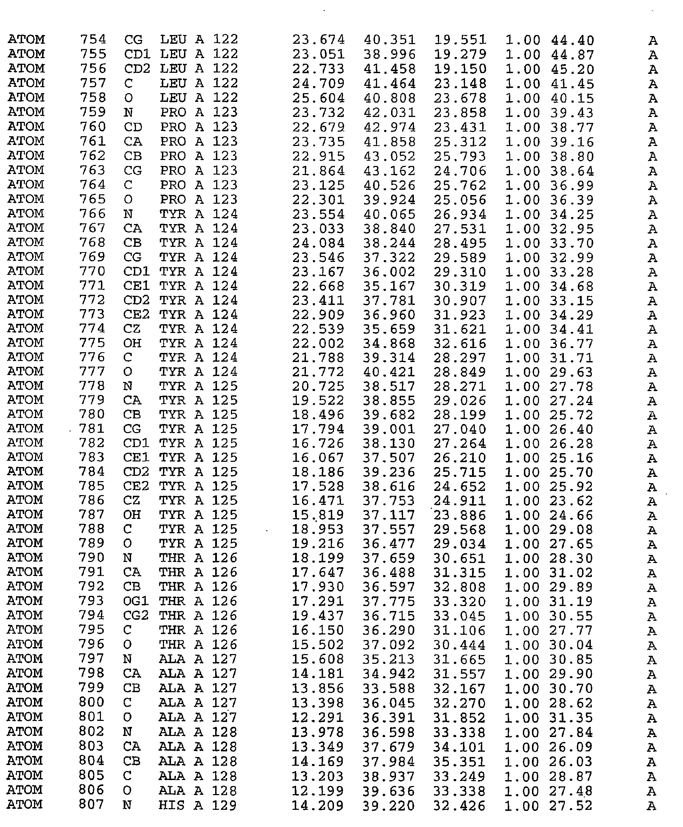

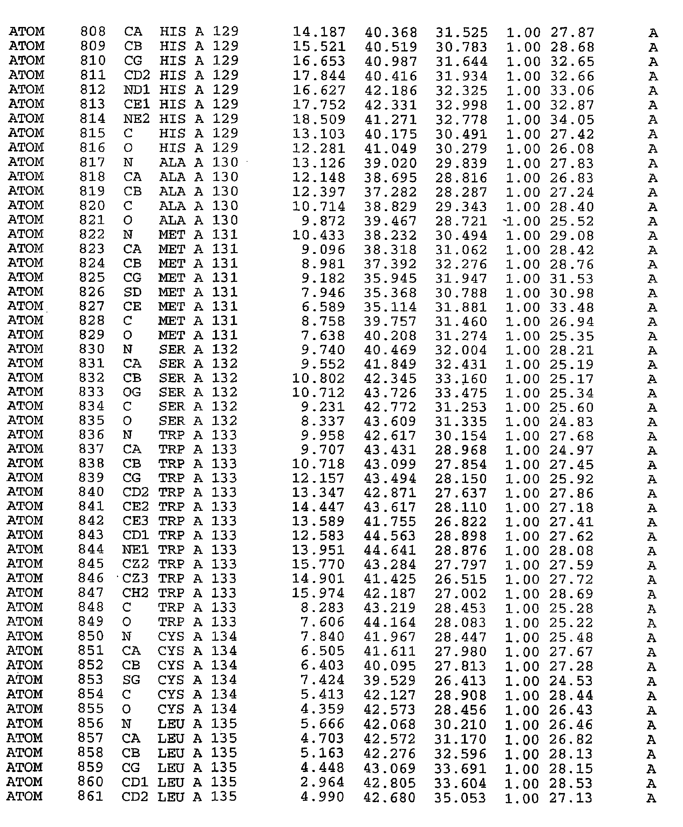

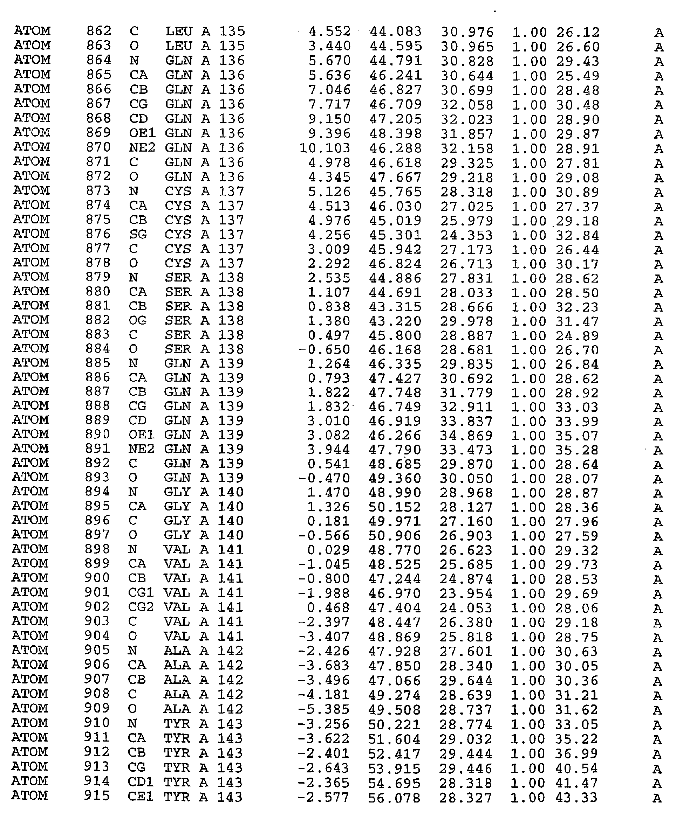

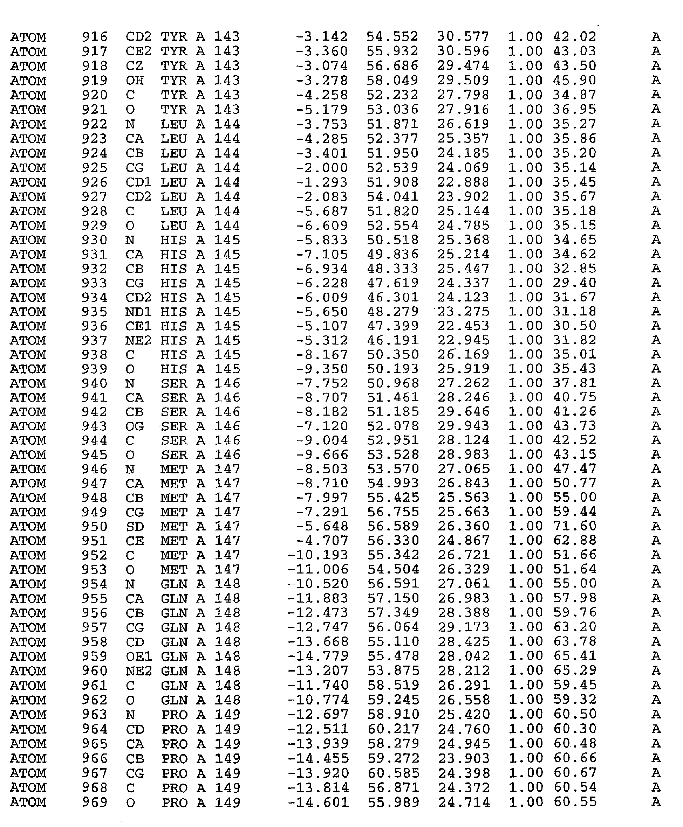

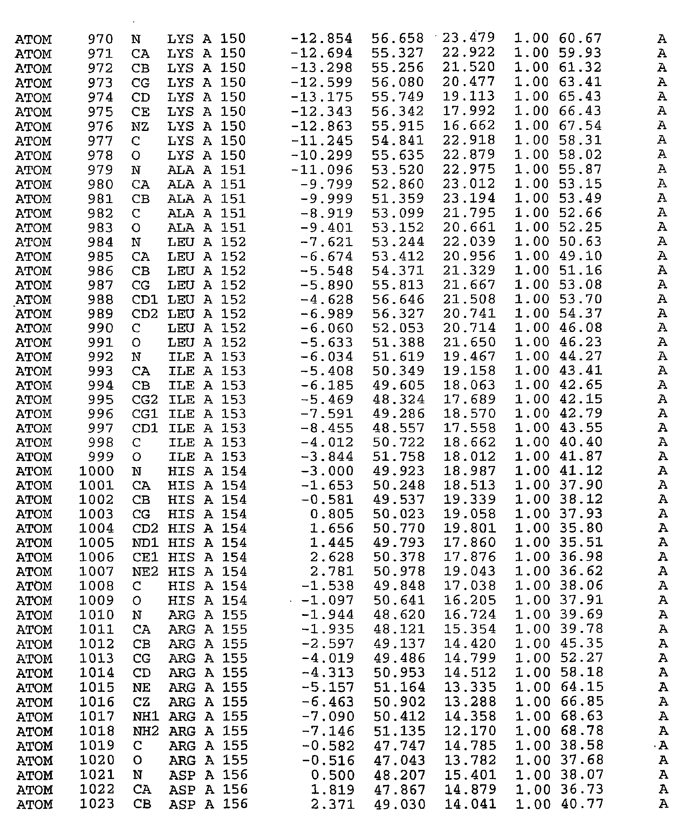

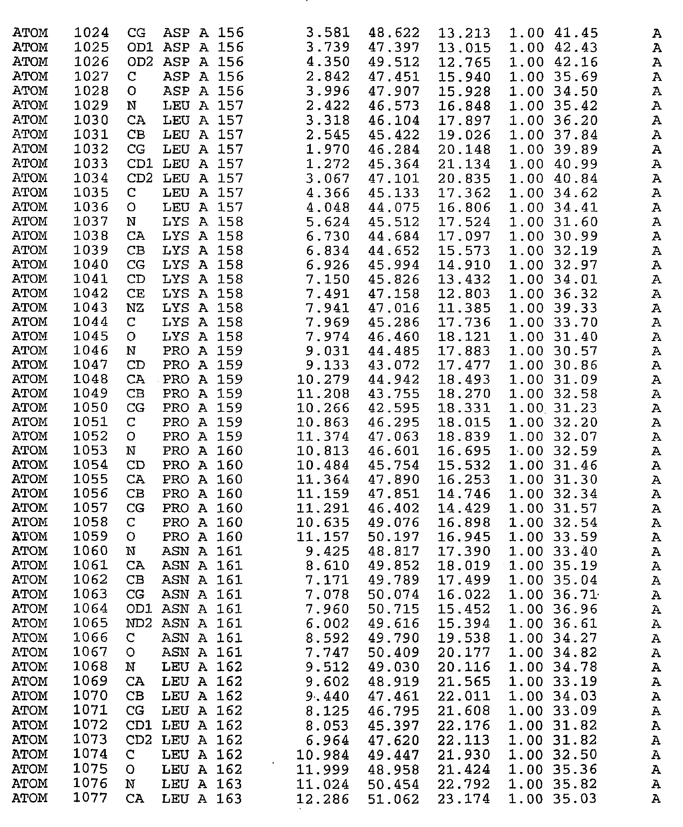

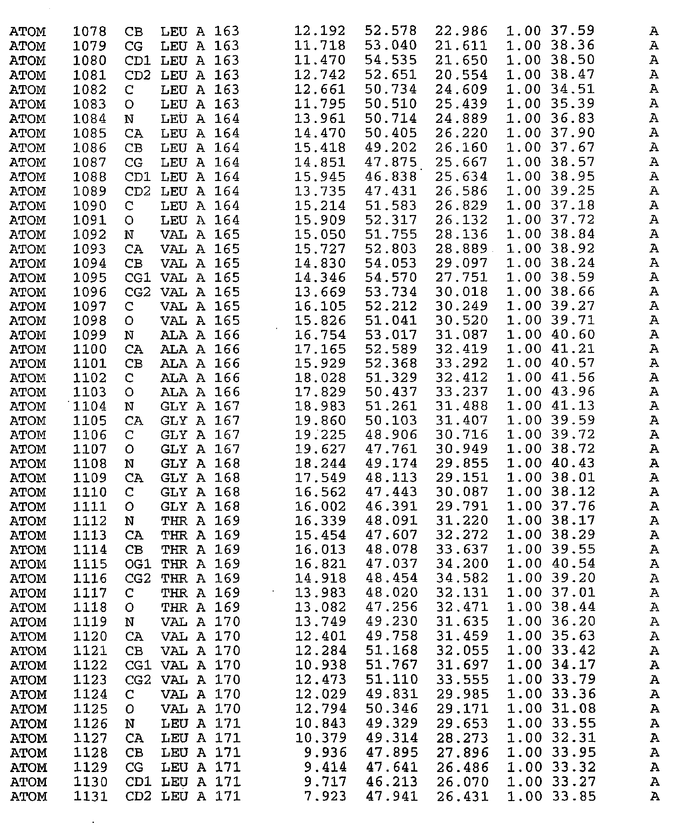

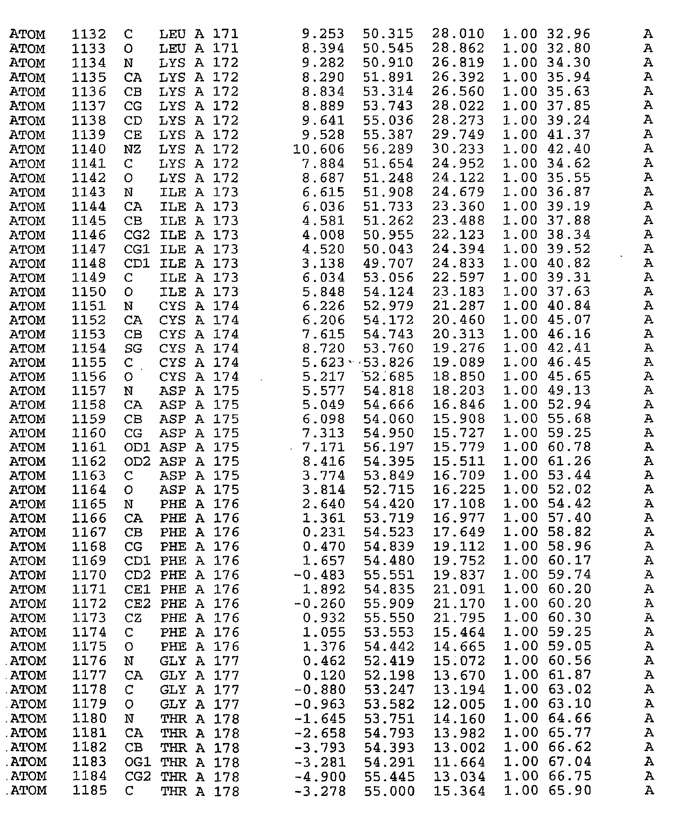

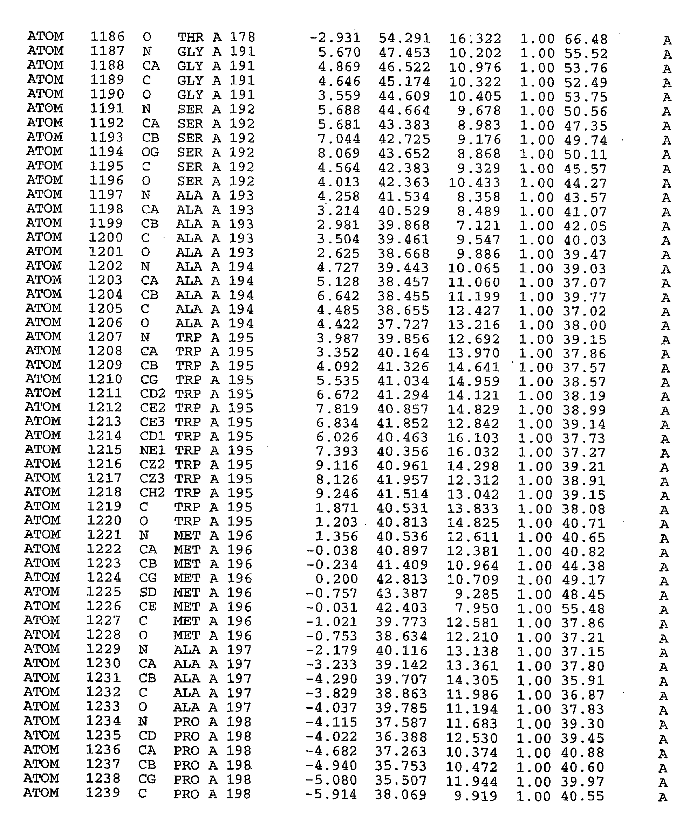

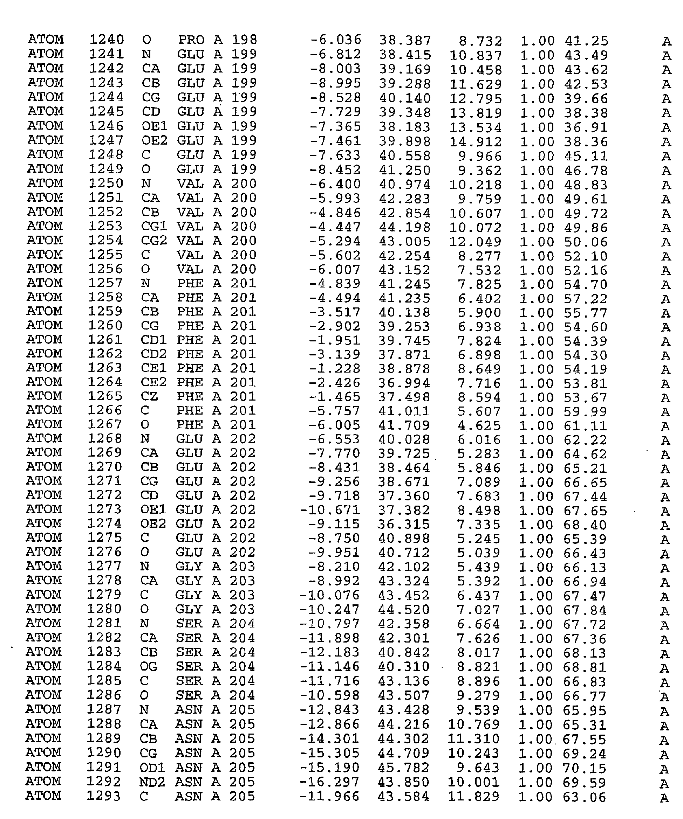

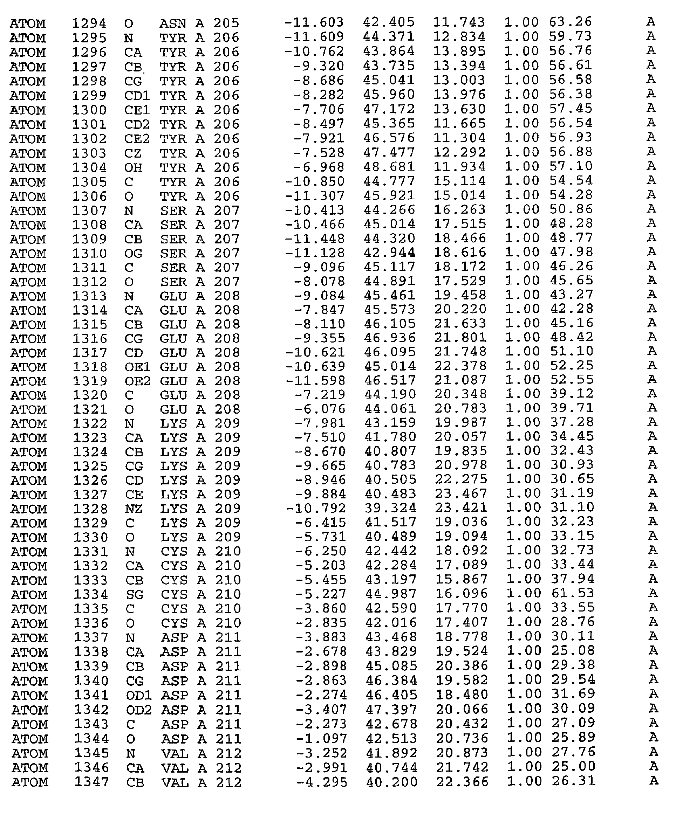

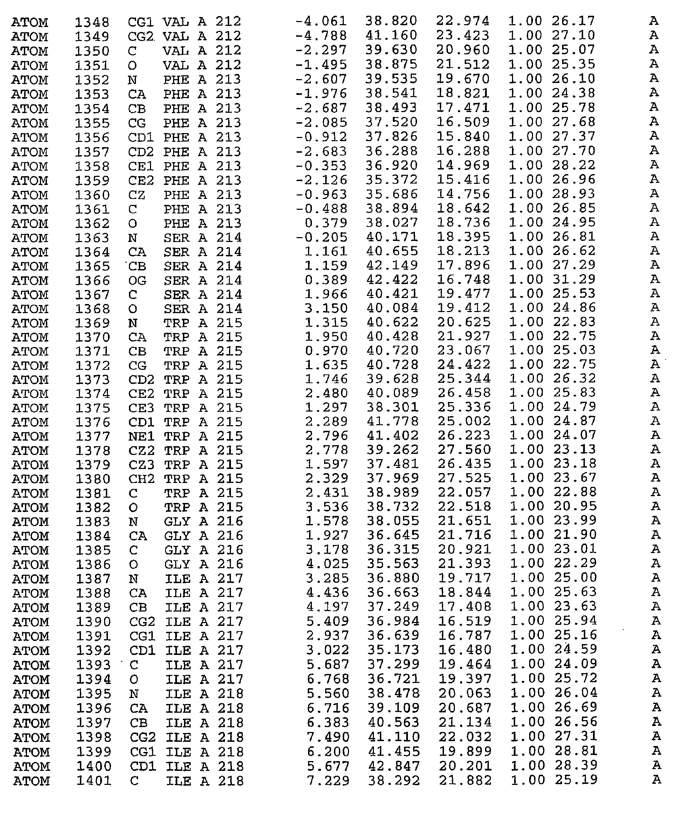

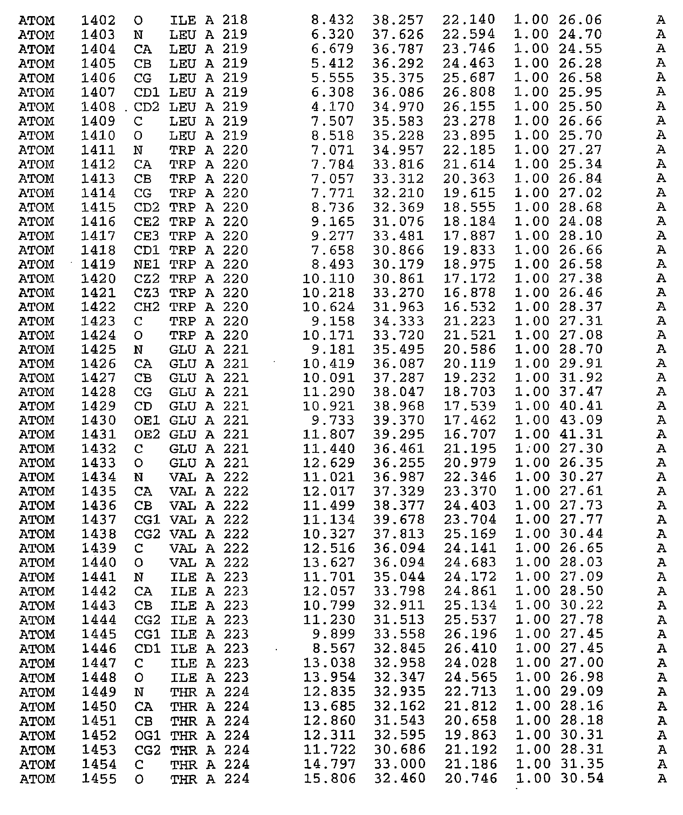

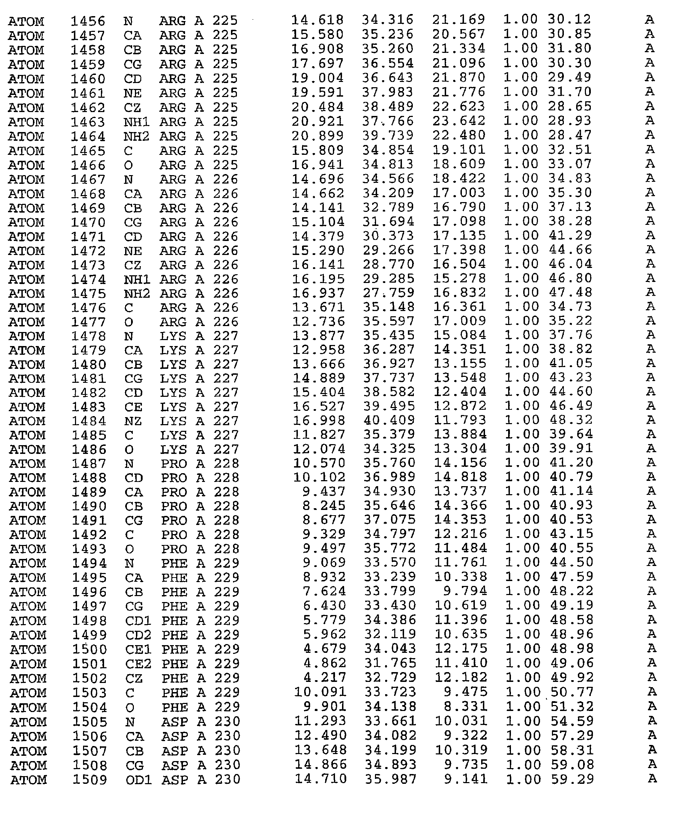

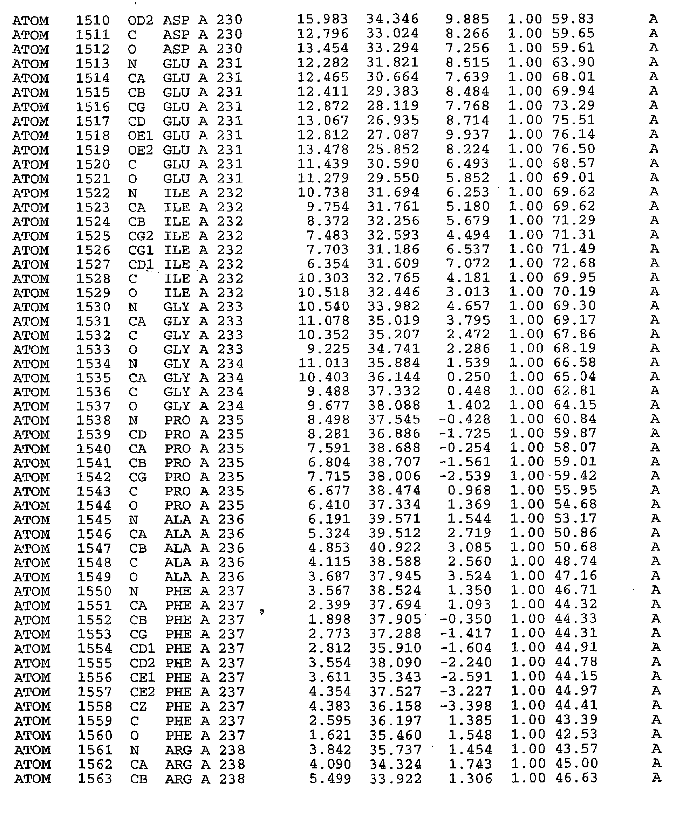

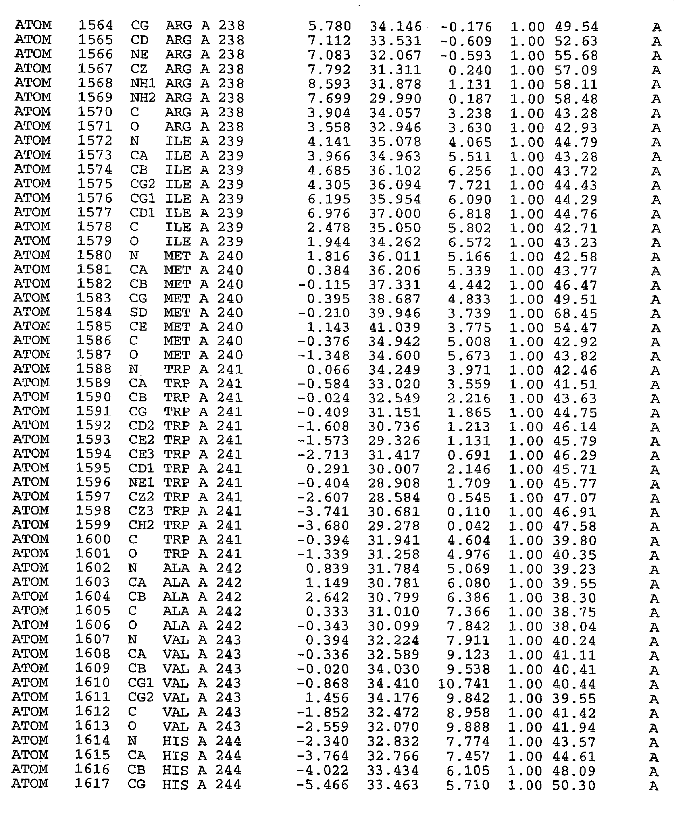

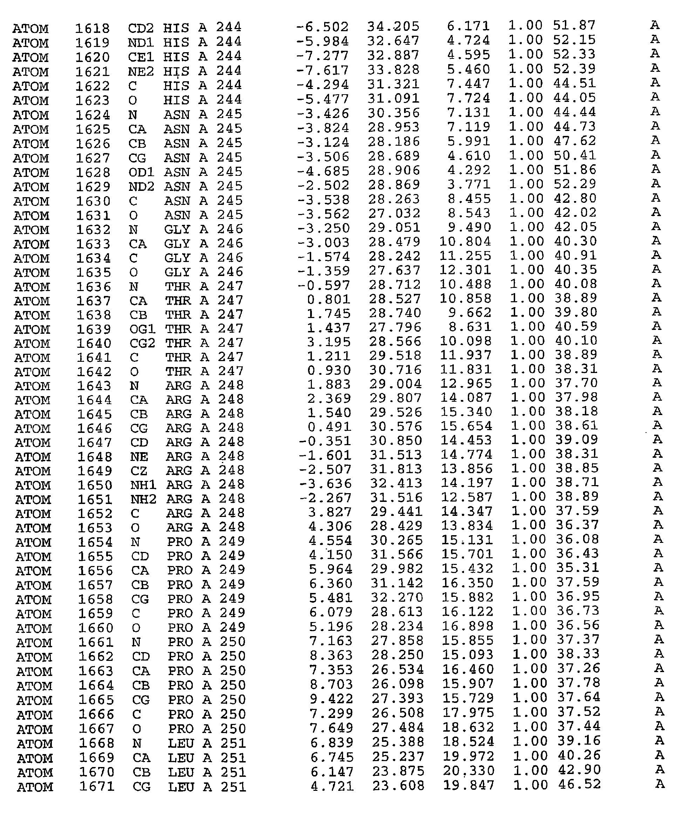

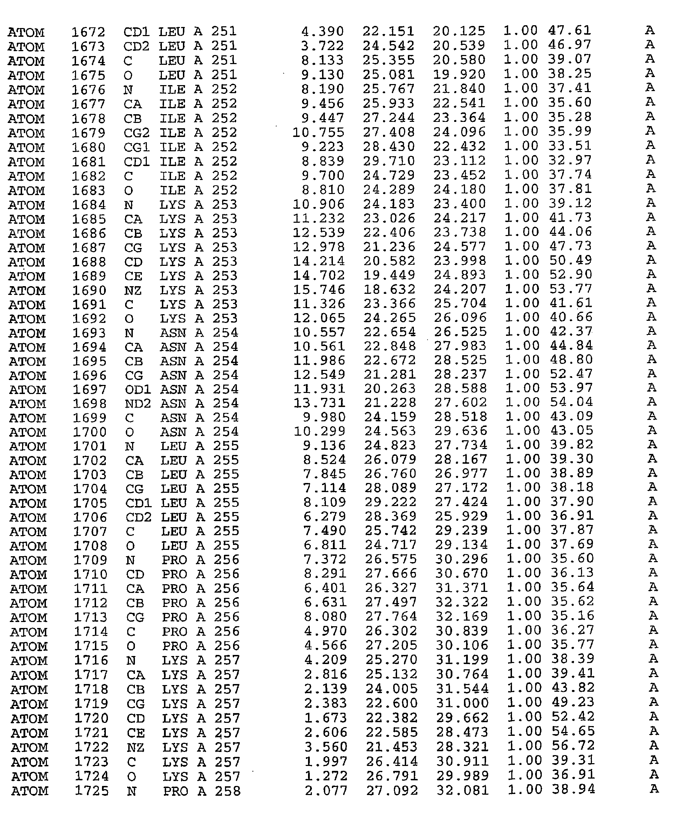

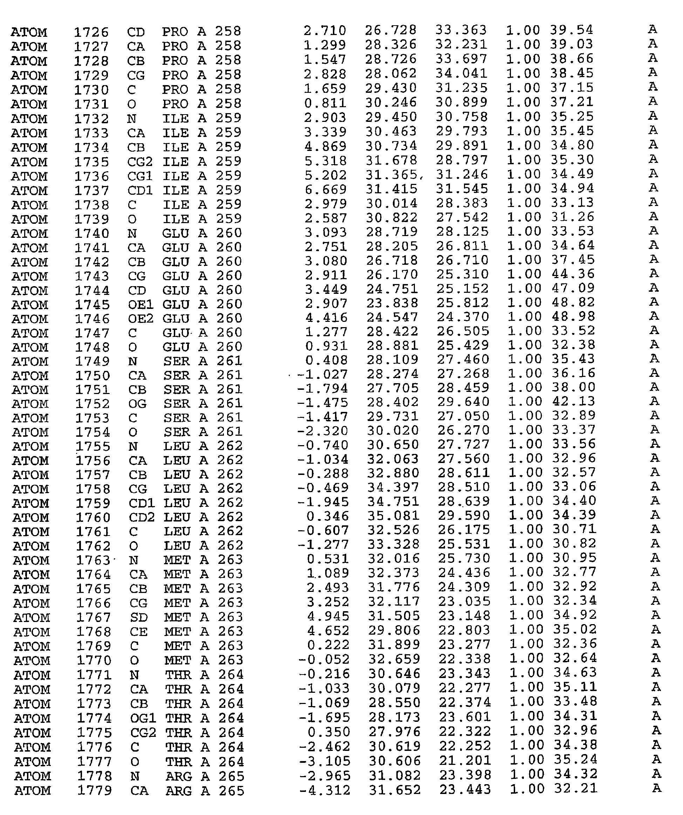

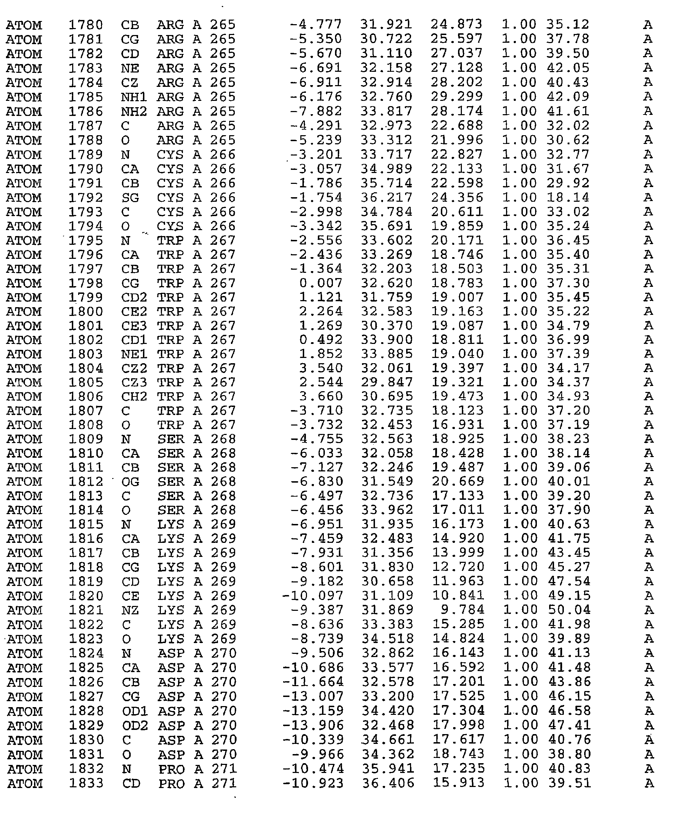

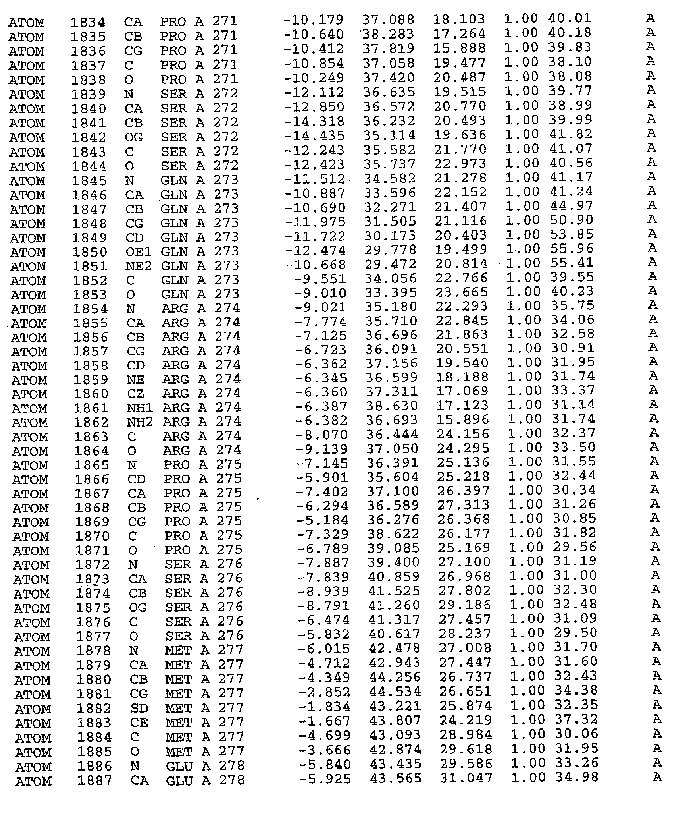

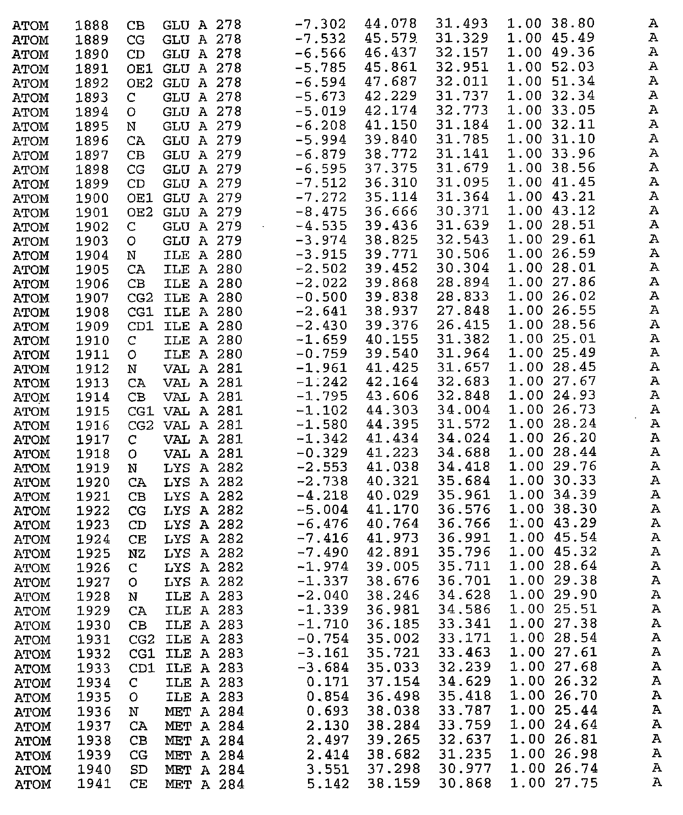

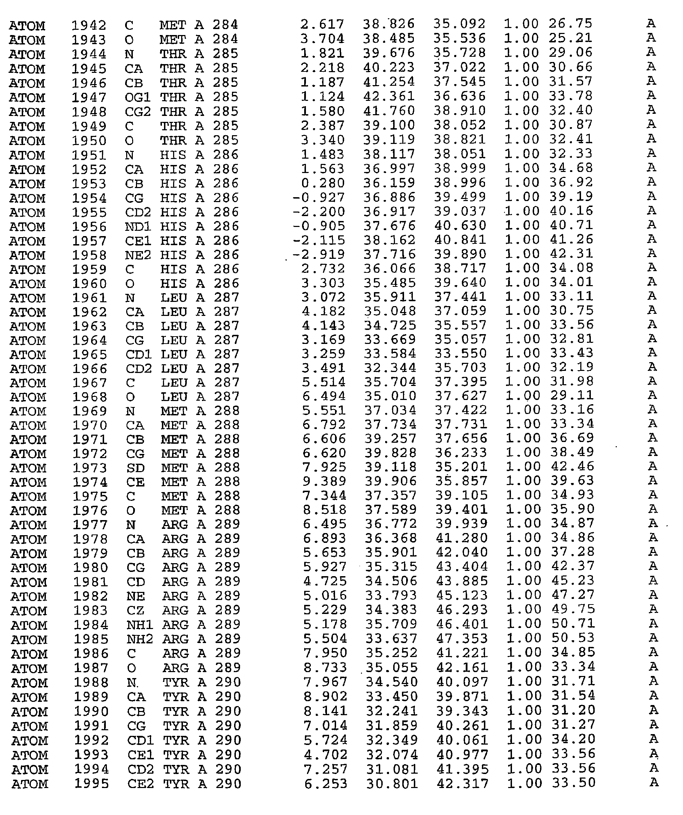

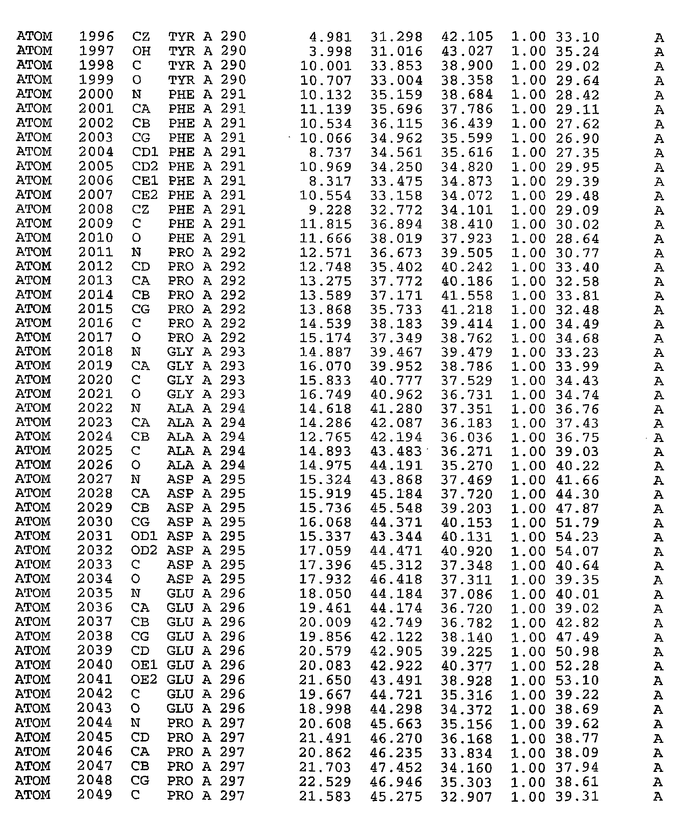

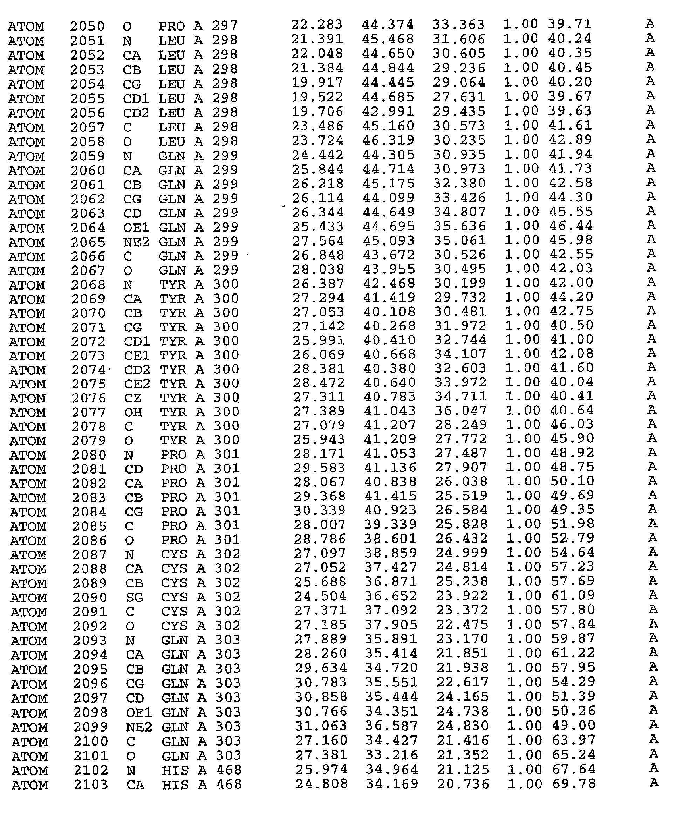

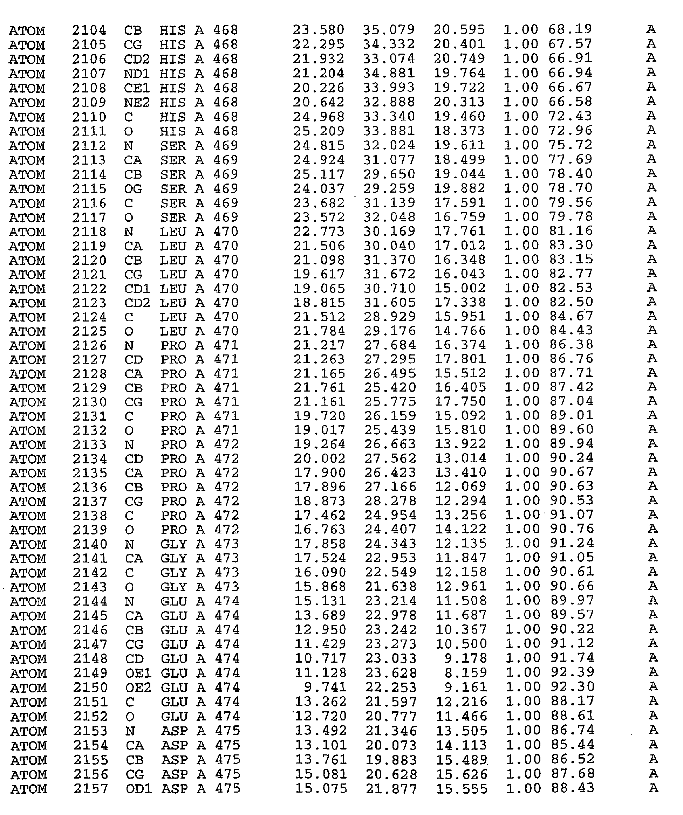

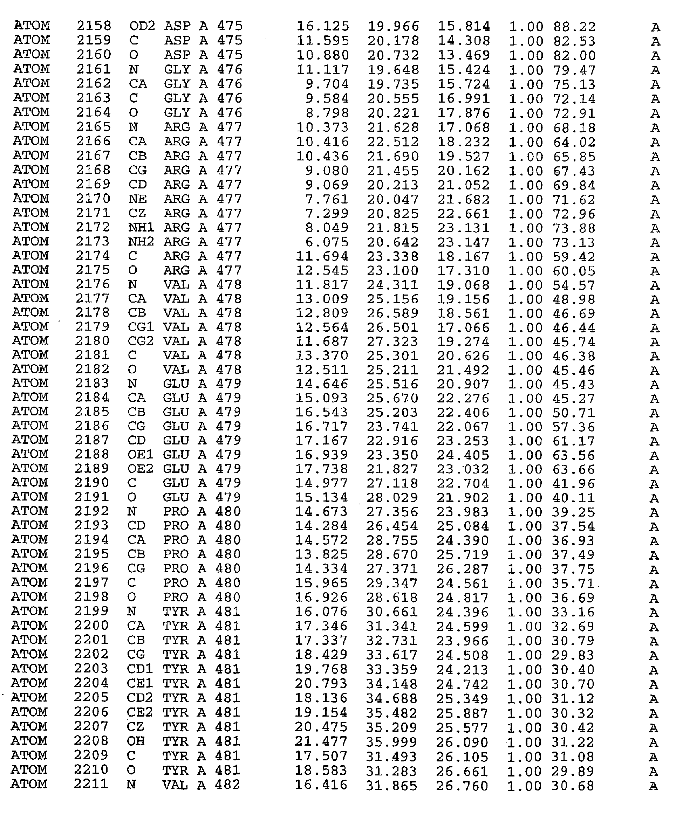

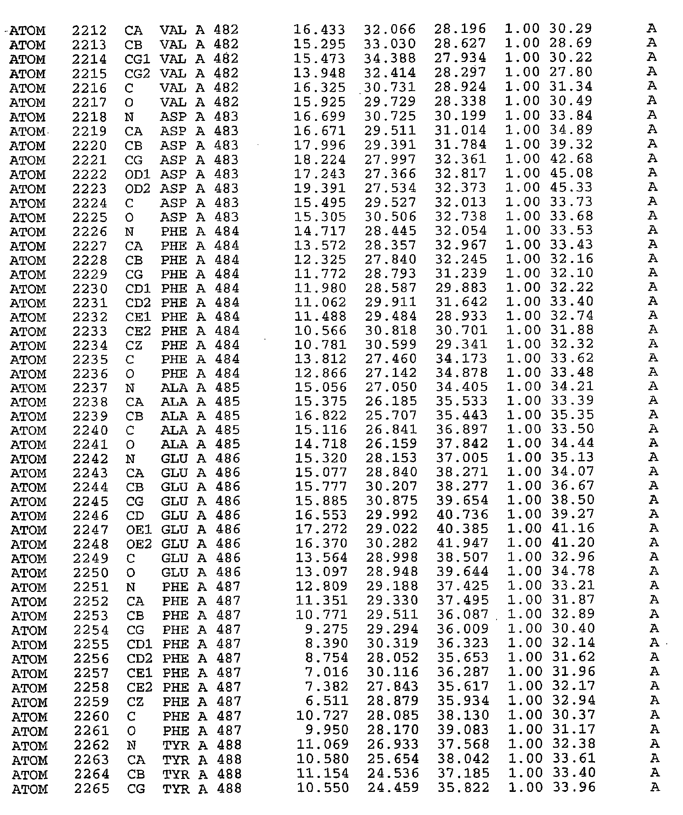

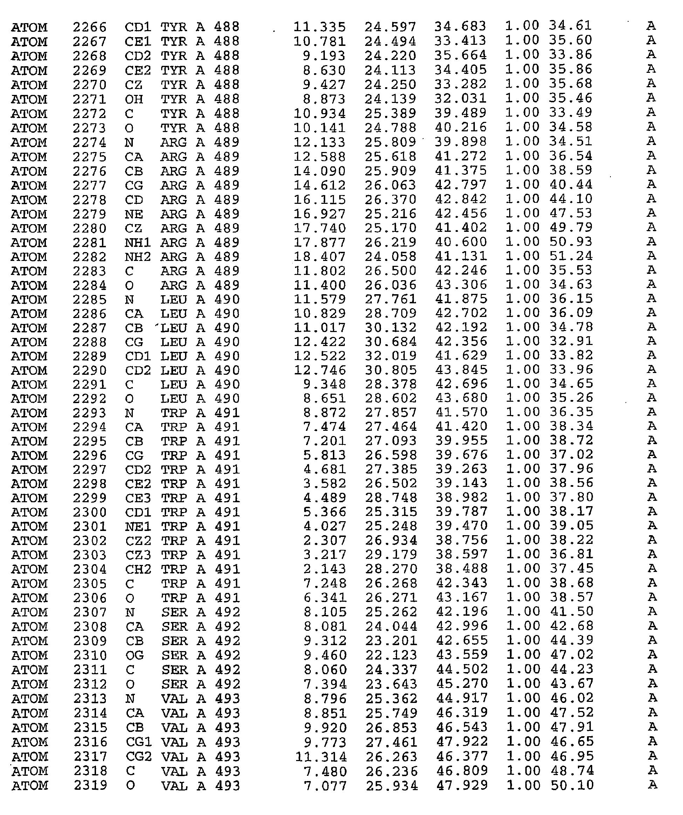

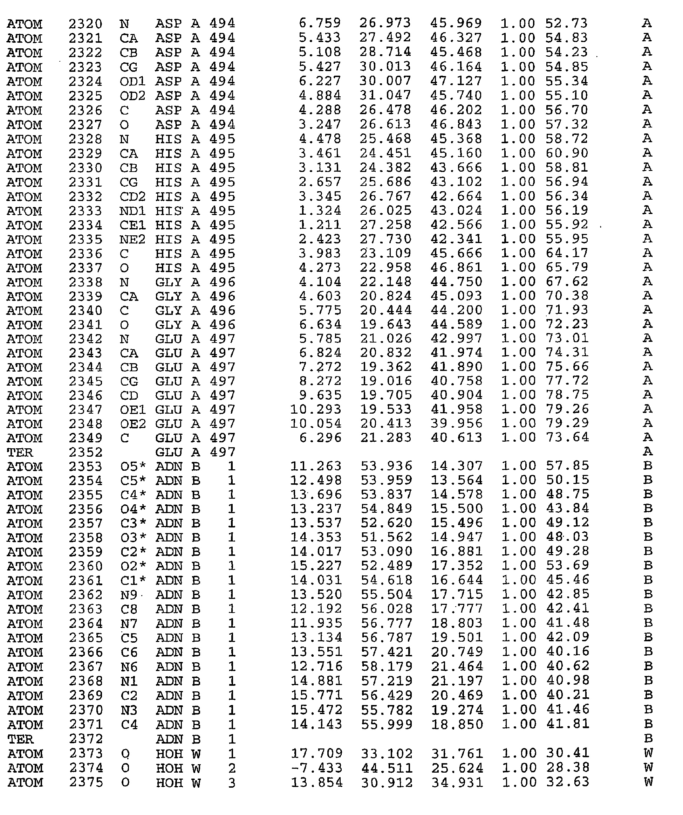

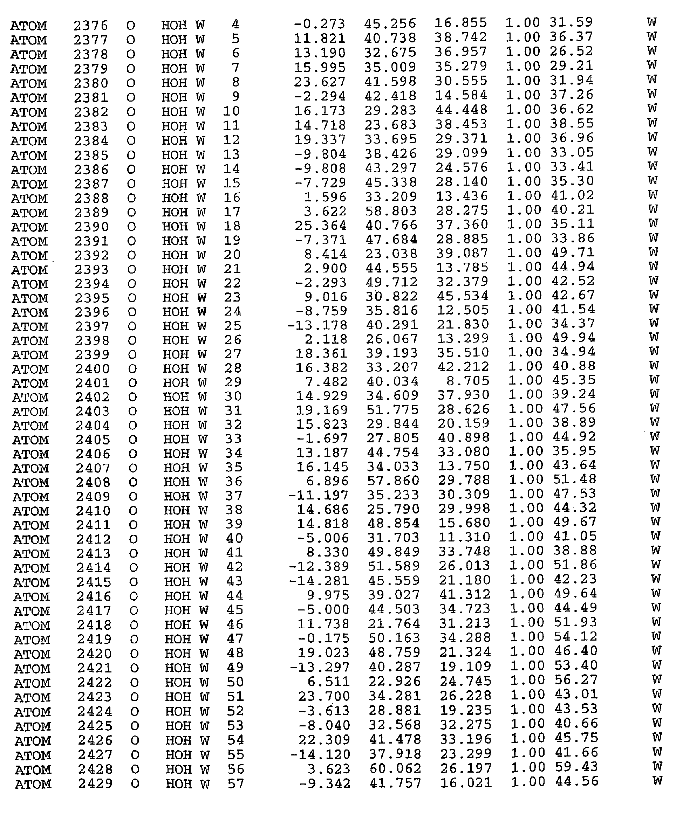

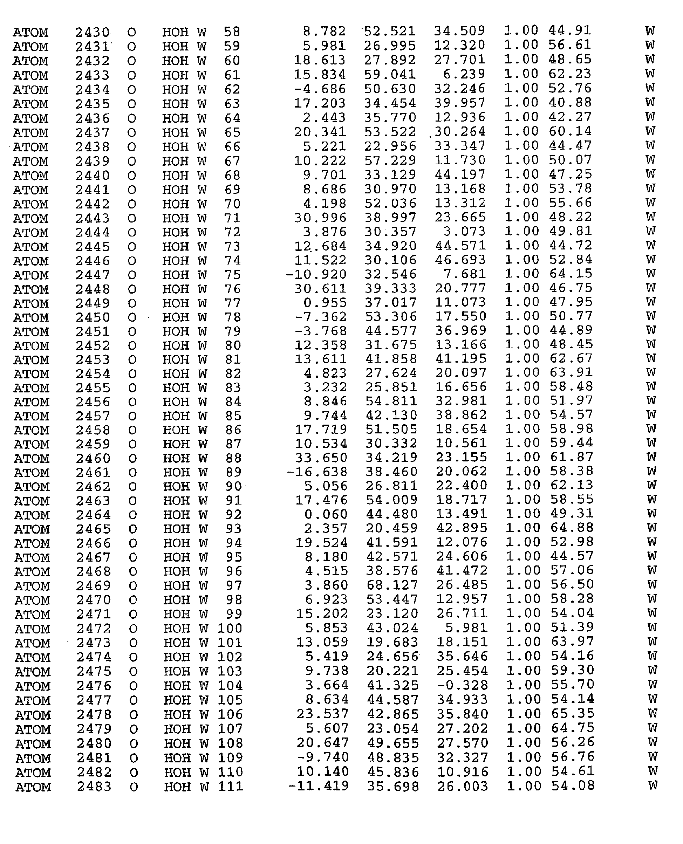

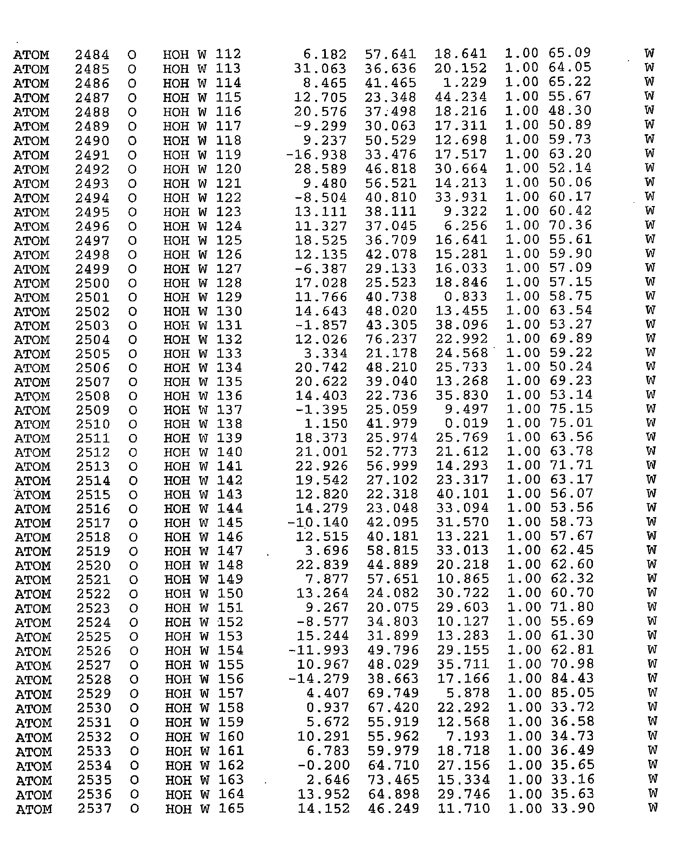

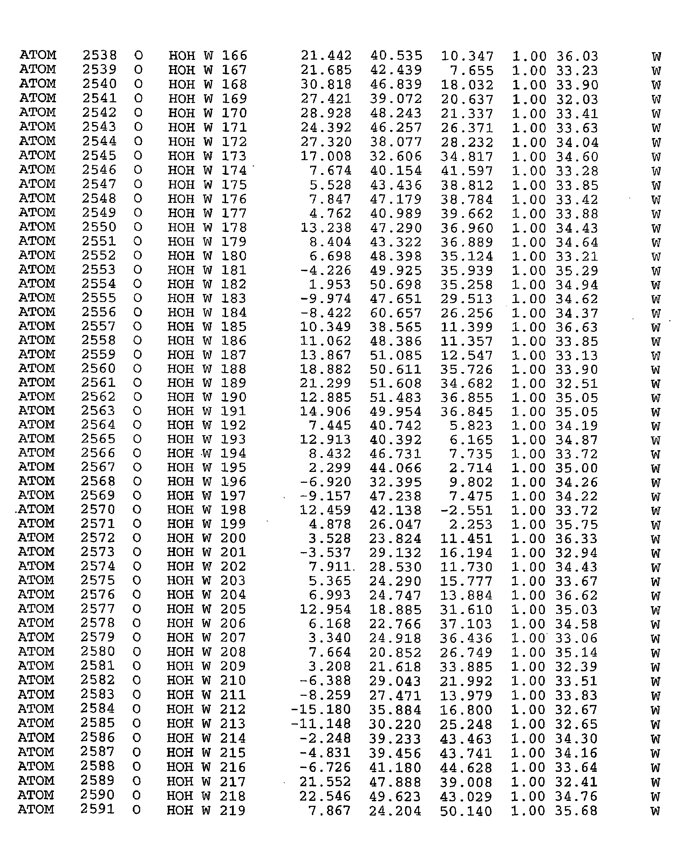

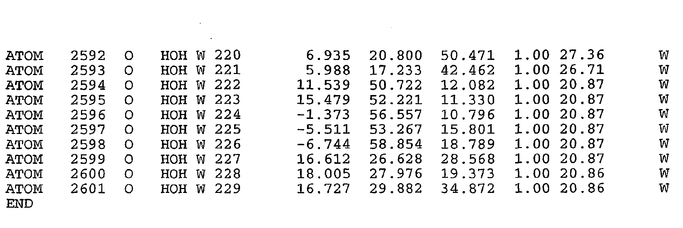

-Figure 1 lists the atomic structure coordinates for the unphosphorylated TAK1 - 9-β-D-Ribofuranosyladenine ("adenosine") inhibitor complex as derived by X-ray diffraction from the crystal.

The following abbreviations are used in Figures 1-2:

"Atom type" refers to the element whose coordinates are measured. The first letter in the column defines the element.

"Resid" refers to the amino acid residue identity in the molecular model.

"X, Y, Z" crystallographically define the atomic position of the element measured.

"B" is a thermal factor that measures movement of the atom around its atomic center.

"Occ" is an occupancy factor that refers to the fraction of the molecules in which each atom occupies the position specified by the coordinates. A value of "1" indicates that each atom has the same conformation, *i.e.*, the same position, in all molecules of the crystal.

"Mol" refers to the molecule in the asymmetric unit.

Figure 2 lists the atomic structure coordinates for the unphosphorylated TAK1 - 3-[6-(4-Acetyl-3,5-dimethyl-piperazin-1-yl)-pyridin-2-yl]-1H-pyrrolo[2,3-b]pyridine-5-carboxylic acid methyl ester inhibitor complex as derived by X-ray diffraction from the crystal.

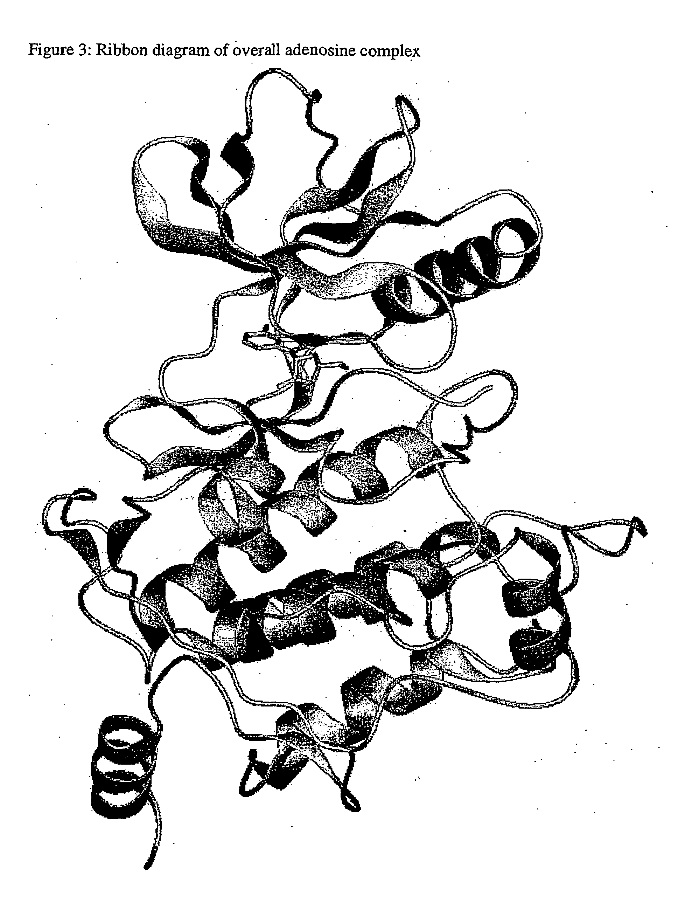

Figure 3 depicts ribbon diagrams of the overall fold of TAK1-adenosine and TAK1-3-[6-(4-Acetyl-3,5-dimethyl-piperazin-1-yl)-pyridin-2-yl]-1H-pyrrolo[2,3-b]pyridine-5-carboxylic acid methyl ester complexes. The N-terminal lobe of the TAK1 catalytic domain corresponds to the β-strand sub-domain and encompasses residues 31 to 104. The α-helical sub-domain corresponds to residues 112 to 303. Residues 304 to 340 correspond to the 36 C-terminal residues of TAB1. Key features of the kinase-fold such as the hinge (approximately residues 105 to 111), glycine rich loop (approximately residues 39 to 52) and activation loop or phosphorylation lip (approximately residues 175 to 191) are indicated. A number of residues in the activation loop (-178 to 520) and at the C-terminus (334 to 340) are disordered in each of the TAK1 crystal structures. They exhibited only weak electron density and could not be fitted.

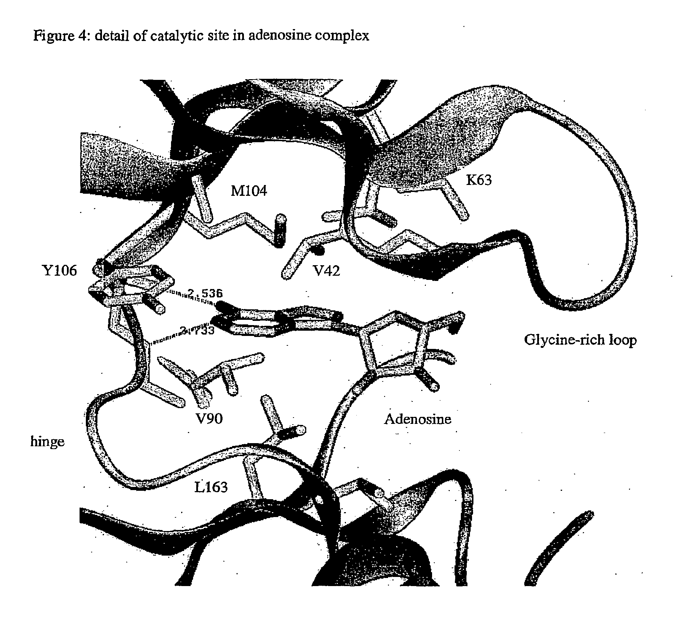

Figure 4 shows a detailed representation of pockets in the catalytic active site of the TAK1 - adenosine complex.

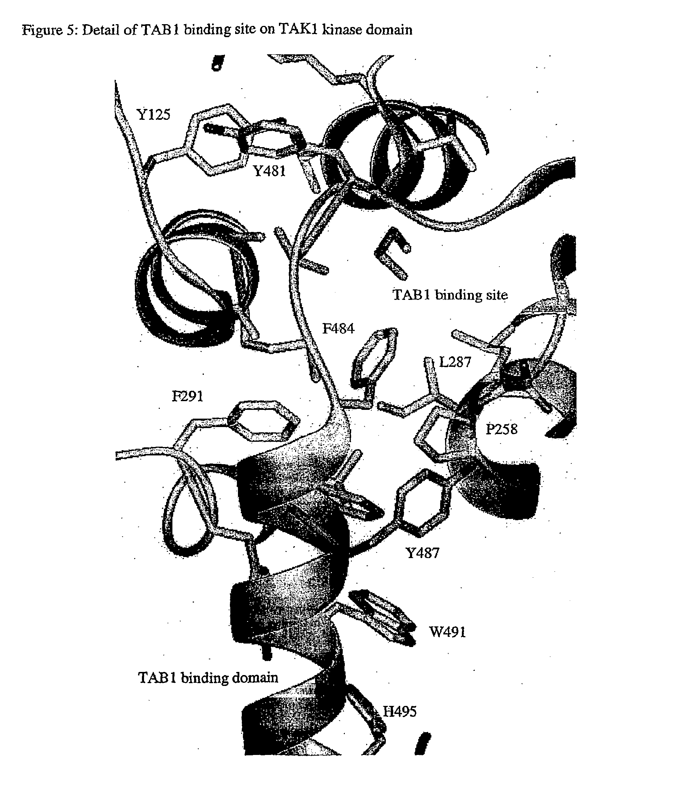

Figure 5 shows a detailed representation of pockets in the TAB1 binding site of the TAK1 - TAB 1 structure.

Figure 6 shows a sequence alignment of the C-terminii of TAK1, TAB 1 and AKT2, showing the conserved phenylalanine (Phe439 in AKT2, Phe319 in TAK1, Phe484 in TAB1).

Figure 7 shows a sequence alignment of the N-terminus of TAK1 with related protein kinases with close homology to TAK1 that have previously been crystallized in the literature.

Figure 8 shows a diagram of a system used to carry out the instructions encoded by the storage medium of Figures 6 and 7.

Figure 9 shows a cross section of a magnetic storage medium.

Figure 10 shows a cross section of an optically-readable data storage medium.

<u>DETAILED DESCRIPTION OF THE INVENTION</u>

In order that the invention described herein may be more fully understood, the following detailed description is set forth.

Throughout the specification, the word "comprise", or variations such as "comprises" or "comprising" will be understood to imply the inclusion of a stated integer or groups of integers but not exclusion of any other integer or groups of integers.

The following abbreviations are used throughout the application:

| | | | | | |
|---|---|---|---|---|---|
| A = | Ala = | Alanine | T = | Thr = | Threonine |
| V = | Val = | Valine | C = | Cys = | Cysteine |
| L = | Leu = | Leucine | Y = | Tyr = | Tyrosine |
| I = | Ile = | Isoleucine | N = | Asn = | Asparagine |
| P = | Pro = | Proline | Q = | Gln = | Glutamine |
| F = | Phe = | Phenylalanine | D = | Asp = | Aspartic Acid |
| W = | Trp = | Tryptophan | E = | Glu = | Glutamic Acid |
| M = | Met = | Methionine | K = | Lys = | Lysine |
| G = | Gly = | Glycine | R = | Arg = | Arginine |
| S = | Ser = | Serine | H = | His = | Histidine |

Additional definitions are set forth herein.

The term "associating with" refers to a condition of proximity between a chemical entity or compound, or portions thereof, and a binding pocket or binding site on a protein. The association may be non-covalent - wherein the juxtaposition is energetically favored by hydrogen bonding or van der Waals or electrostatic interactions - or it may be covalent.

The term "binding pocket", as used herein, refers to a region of a molecule or molecular complex, that, as a result of its shape and charge, favorably associates with another chemical entity or compound. The term "pocket" includes, but is not limited to, cleft, channels or site. TAK1 or TAK1-like molecules may have binding pockets which include, but are not limited to, peptide or substrate binding, ATP-binding and antibody binding sites.

The term "chemical entity", as used herein, refers to chemical compounds, complexes of at least two chemical compounds, and fragments of such compounds or complexes. The chemical entity may be, for example, a ligand, a substrate, a nucleotide triphosphate, a nucleotide diphosphate, phosphate, a nucleotide, an agonist, antagonist, inhibitor, antibody, drug, peptide, protein or compound.

"Conservative substitutions" refers to residues that are physically or functionally similar to the corresponding reference residues. That is, a conservative substitution and its reference residue have similar size, shape, electric charge, chemical properties including the ability to form covalent or hydrogen bonds, or the like. Preferred conservative substitutions are those fulfilling the criteria defined for an accepted point mutation in Dayhoff et al., Atlas of Protein Sequence and Structure, 5, pp. 345-352 (1978 & Supp.). Examples of conservative substitutions are substitutions including but not limited to the following groups: (a) valine, glycine; (b) glycine, alanine; (c) valine, isoleucine, leucine; (d) aspartic acid, glutamic acid; (e) asparagine, glutamine; (f) serine, threonine; (g) lysine, arginine, methionine; and (h) phenylalanine, tyrosine.

The term "corresponding amino acid" or residue which corresponds to" refers to a particular amino acid or analogue thereof in a TAK1 homologue that corresponds to an amino acid in the TAK structure. The corresponding amino acid may be an identical, mutated, chemically modified, conserved, conservatively substituted, functionally equivalent or homologous amino acid when compared to the TAK1 amino acid to which it corresponds.

Methods for identifying a corresponding amino acid are known in the art and are based upon sequence, structural alignment, its functional position or a combination thereof as compared to the TAK1 structure. For example, corresponding amino acids may be identified by superimposing the backbone atoms of the amino acids in TAK1 and the TAK1 homologue using well known software applications, such as QUANTA [Molecular Simulations, Inc., San Diego, CA ©1998,2000]. The corresponding amino acids may also be identified using sequence alignment programs such as the "bestfit" program available from the Genetics Computer Group which uses the local homology algorithm described by Smith and Waterman in Adv. Appl. Math., 2, 482 (1981).

The term "domain" refers to a portion of the TAK1 protein or homologue that can be separated according to its biological function, for example, catalysis. The domain is usually conserved in sequence or structure when compared to other kinases or related proteins. The domain can comprise a binding pocket, or a sequence or structural motif.

The term "sub-domain" refers to a portion of the domain as defined above in the TAK1 protein or homologue. The catalytic kinase domain (amino acid residues 31 to 303) of TAK1 is a bi-lobal structure consisting of an N-terminal, β-strand sub-domain (residues 31 to 104) and a C-terminal, α-helical sub-domain (residues 112 to 303).

The term "catalytic active site" refers to the area of the protein kinase to which nucleotide substrates bind. The catalytic active site of TAK1 is at the interface between the N-terminal, β-strand sub-domain and the C-terminal, α-helical sub-domain.

The "TAK1 ATP-binding pocket" of a molecule or molecular complex is defined by the structure coordinates of a certain set of amino acid residues present in the TAK1 structure, as described below. In general, the ligand for the ATP-binding pocket is a nucleotide such as ATP. This binding pocket is in the catalytic active site of the kinase domain. In the protein kinase family, the ATP-binding pocket is generally located at the interface of the α-helical and β-strand sub-domains, and is bordered by the glycine rich loop and the hinge [See, Xie et al., Structure, 6, pp. 983-991 (1998).

The "TAB1 binding pocket" of a molecule or molecular complex is defined by the structure coordinates of a certain set of amino acid residues present in the TAK1 structure, as described below. In general, the ligand for the TAB1 binding pocket is the TAK1 binding domain of TAB1. This pocket is the site of association of TAK1 with its protein activator TAB1. The TAB1 binding pocket is located at near the bottom of the a-helical sub-domain, and is over 16 Å long, 8 Å wide and approximately 8 Å deep, and is formed by residues belonging to the top of helices E and H and the tail of F and I.

The term "TAK1-like" refers to all or a portion of a molecule or molecular complex that has a commonality of shape to all or a portion of the TAK1 protein. In the TAK1-like ATP-binding pocket, the commonality of shape is defined by a root mean square deviation of the structure coordinates of the backbone atoms between the amino acids in the TAK1-like ATP-binding pocket and the amino acids in the TAK1 ATP-binding pocket (as set forth in Figures 1, 2 or 3). Compared to an amino acid in the TAK1 ATP-binding pocket, the corresponding amino acids in the TAK1-like ATP-binding pocket may or may not be identical.

The term "part of an TAK1 ATP-binding pocket" or "part of an TAK1-like ATP-binding pocket" refers to less than all of the amino acid residues that define the TAK1 or TAK1-like ATP-binding pocket. The structure coordinates of residues that constitute part of an TAK1 or TAK1-like ATP-binding pocket may be specific for defining the chemical environment of the binding pocket, or useful in designing fragments of an inhibitor that may interact with those residues. For example, the portion of residues may be key residues that play a role in ligand binding, or may be residues that are spatially related and define a three-dimensional compartment of the binding pocket. The residues may be contiguous or non-contiguous in primary sequence. In one embodiment, part of the TAK1 or TAK1-like ATP-binding pocket is at least two amino acid residues, preferably, E105 and A107. In another embodiment, the amino acids are selected from the group consisting of V42, V90, M104, E105, A107 and L163.

The term "TAK1 kinase domain" refers to the catalytic domain of TAK1. The kinase domain includes, for example, the catalytic active site which comprises the catalytic residues, the activation loop or phosphorylation lip, the DFG motif, and the glycine-rich phosphate anchor or glycine-rich loop [See, Xie et al., Structure, 6, pp. 983-991 (1998); R. Giet and C. Prigent, J. Cell Sci., 112, pp. 3591-3601 (1999),]. The kinase domain in the TAK1 protein comprises residues from about 31 to 303.

The term "part of a TAK1 kinase domain" or "part of a TAK1-like kinase domain" refers to a portion of the TAK1 or TAK1-like catalytic domain. The structure coordinates of residues that constitute part of a TAK1 or TAK1-like kinase domain may be specific for defining the chemical environment of the domain, or useful in designing fragments of an inhibitor that may interact with those residues. For example, the portion of residues may be key residues that play a role in ligand binding, or may be residues that are spatially related and define a three-dimensional compartment of the domain. The residues may be contiguous or non-contiguous in primary sequence. For example, part of a TAK1 kinase domain can be the active site, the glycine-rich loop, the activation loop, or the catalytic loop [see Xie *et al.,* supra].

The term "homologue of TAK1" refers to a molecule or molecular complex that is homologous to TAK1 by three-dimensional structure or sequence. Examples of homologues include but are not limited to the following: human TAK1 with mutations, conservative substitutions, additions, deletions or a combination thereof; TAK1 from a species other than human; a protein comprising an TAK1-like ATP-binding pocket, a kinase domain; another member of the protein kinase family, preferably the MAPKKK kinase family or the CDK Kinase family; or another member of the MAPK family of protein kinases. It should be understood that a homologue of TAK1 would include a truncation or extension of TAK1. Additionally, heavy atom derivatives of TAK1 may be obtained, for example, by soaking crystals in solutions containing a heavy atom compound. As is recognized, certain amino acids are particularly useful for preparing heavy atom derivatives, such as methionine, selenomethionine, cysteine, and selenocysteine. mutations are particularly useful for making heavy-atom derivative crystals. Such TAK1 homologues are within this invention.

Any of these changes are not mutually exclusive. A TAK1 homologue may have one or more than one of these changes. However, it is generally understood that in a homologue no more than about 20-30% of the amino acids should be changed relative to the wild-type protein. More specific homologues would be those wherein no more than about 25%, about 10%, or about 5% of the amino acids have been changed relative to the wild-type protein. In the case of protein crystals, a homologue should have no more than about 5-10% of the amino acids changed relative to the wild-type protein. More specific homologues would be those wherein no more than about 10%, about 5%, or about 1% of the amino acids have been changed relative to the wild-type protein. The wild-type TAK1 protein used herein is human TAK1. The wild-type TAB 1 protein used herein is human TAK1.

TAB 1 homologues are similarly included in this invention.

The term "part of a TAK1 protein" or "part of a TAK1 homologue" refers to a portion of the amino acid residues of a TAK1 protein or homologue. In one embodiment, part of a TAK1 protein or homologue defines the binding pockets, domains, sub-domains, and motifs of the protein or homologue. The structure coordinates of residues that constitute part of a TAK1 protein or homologue may be specific for defining the chemical environment of the protein, or useful in designing fragments of an inhibitor that may interact with those residues. The portion of residues may also be residues that are spatially related and define a three-dimensional compartment of a binding pocket, motif or domain. The residues may be contiguous or non-contiguous in primary sequence. For example, the portion of residues may be key residues that play a role in ligand or substrate binding, peptide binding, antibody binding, catalysis, structural stabilization or degradation.

The term "TAK1 protein complex" or "TAK1 homologue complex" refers to a molecular complex formed by associating the TAK1 protein or TAK1 homologue with a chemical entity, for example, a ligand, a substrate, nucleotide triphosphate, an agonist or antagonist, inhibitor, drug or compound. In one embodiment, the chemical entity is selected from the group consisting of an ATP, a nucleotide triphosphate and an inhibitor for the ATP-binding pocket. In another embodiment, the inhibitor is an ATP analog such as MgAMP-PNP (adenylyl imidodiphosphate), adenosine, staurosporine or 3-(8-Phenyl-5,6-dihydrothieno[2,3-h]quinazolin-2-ylamino)benzenesulfonamide.

The term "motif" refers to a portion of the TAK1 protein or homologue that defines a structural compartment or carries out a function in the protein, for example, catalysis, structural stabilization, or phosphorylation. The motif may be conserved in sequence, structure and function when compared to other kinases or related proteins. The motif can be contiguous in primary sequence or three-dimensional space. The motif can comprise $\alpha$-helices and/or $\beta$-sheets. Examples of a motif include but are not limited to a binding pocket, active site, phosphorylation lip or activation loop, the glycine-rich phosphate anchor loop, the catalytic loop [See, Xie et al., Structure, 6, pp. 983-991 (1998); R. Giet and C. Prigent, J. Cell Sci., 112, pp. 3591-3601 (1999)], and the degradation box.

The term "root mean square deviation" or "RMSD" means the square root of the arithmetic mean of the squares of the deviations from the mean. It is a way to express the deviation or variation from a trend or object. For purposes of this invention, the "root mean square deviation" defines the variation in the backbone of a protein from the backbone of TAK1, a binding pocket, a motif, a domain, or portion thereof, as defined by the structure coordinates of TAK1 described herein.

The term "sufficiently homologous to TAK1" refers to a protein that has a sequence homology of at least 35% compared to TAK1 protein. In one embodiment, the sequence homology is at least 40%, at least 60%, at least 80%, at least 90% or at least 95%.

The term "soaked" refers to a process in which the crystal is transferred to a solution containing the compound of interest. In certain embodiments, the compound is diffused into the crystal.

The term "structure coordinates" refers to Cartesian coordinates derived from mathematical equations related to the patterns obtained on diffraction of a monochromatic beam of X-rays by the atoms (scattering centers) of a protein or protein complex in crystal form. The diffraction data are used to calculate an electron density map of the repeating unit of the crystal. The electron density maps are then used to establish the positions of the individual atoms of the molecule or molecular complex. It would be readily apparent to those skilled in the art that all or part of the structure coordinates of Figure 1 (either molecule A or B) may have a RMSD deviation of 0.1 Å because of standard error.

The term "about" when used in the context of RMSD values takes into consideration the standard error of the RMSD value, which is $\pm$ 0.1 Å.

The term "crystallization solution" refers to a solution that promotes crystallization. The solution comprises at least one agent, and may include a buffer, one or more salts, a precipitating agent, one or more detergents, sugars or organic compounds, lanthanide ions, a poly-ionic compound and/or a stabilizer.

The term "generating a three-dimensional structure" or "generating a three-dimensional graphical representation" refers to converting the lists of structure coordinates into structural models in three-dimensional space. This can be achieved through commercially or publicly available software. The three-dimensional structure may be displayed as a graphical representation or used to perform computer modeling or fitting operations. In addition, the structure coordinates themselves may be used to perform computer modeling and fitting operations.

The term "homologue of TAK1" or "TAK1 homologue" refers to a molecule that is homologous to TAK1 by three-dimensional structure or sequence and retains the kinase activity of TAK1. Examples of homologues include, but are not limited to, TAK1 having one or more amino acid residues that are chemically modified, mutated, conservatively substituted, added, deleted or a combination thereof.

The term "homology model" refers to a structural model derived from known three-dimensional structure(s). Generation of the homology model, termed "homology modeling", can include sequence alignment, residue replacement, residue conformation adjustment through energy minimization, or a combination thereof

The term "three-dimensional structural information" refers to information obtained from the structure coordinates. Structural information generated can include the three-dimensional structure or graphical representation of the structure. Structural information can also be generated when subtracting distances between atoms in the structure coordinates, calculating chemical energies for a TAK1 molecule or molecular complex or homologues thereof, calculating or minimizing energies for an association of a TAK1 molecule or molecular complex or homologues thereof to a chemical entity.

The present invention thus provides the following:

1. An isolated, purified human TAK1 construct of amino acids I31-Q303 that is directly fused to a human TAB1 segment of amino acids H468-P504.

2. A crystal comprising a TAK1 kinase domain directly fused to a TAK1-activating domain of TAB1.

3. The crystal according to 2 wherein the TAK1 kinase domain is complexed with an active site inhibitor.

4. The crystal according to 3, wherein the active site inhibitor is an ATP, a nucleotide triphosphate or an ATP analogue.

5. The crystal according to 4, wherein the active site inhibitor is an ATP analog selected from the group consisting of adenosine, adenylyl imidodiphosphate, staurosporine and 3-(8-phenyl-5,6-dihydrothieno[2,3-h]quinazolin-2-ylamino)benzenesulfonamide.

6. The crystal according to 4, wherein the active site inhibitor is adenosine or 3-[6-(4-acetyl-3,5-dimethyl-piperazine-1-yl)-pyridin-2yl]-1H-pyrrolo[2,3-b]pyridine-5-carboxylic acid methyl ester.

7. The crystal according to any one of 2-6, wherein the TAK1 kinase domain comprises residues I31-Q303 of human TAK1.

8. The crystal according to any one of 2-6, wherein the TAK1 kinase domain comprises residues I31-Q303 of human TAK1 and the TAB1 segment comprises amino acids H468-P504 of human TAB 1.

9. A crystallizable composition comprising a TAK1 kinase domain directly fused to a TAK1-activating domain of TAB1.

10. The crystallizable composition according to 9 further comprising 600-900 mM sodium citrate, 1 to 200 mM sodium chloride, and a buffer that maintains pH at between about 6.5 and about 8.5.

11. The crystallizable composition according to 10 further comprising a reducing agent at between about 1 to about 20 mM.

12. The crystallizable composition according to any one of 9-11, wherein the TAK1 kinase domain comprises residues I31-Q303 of human TAK1.

13. The crystallizable composition according to any one of 9-11, wherein the TAK1 kinase domain comprises residues I31-Q303 of human TAK1 and the TAB1 segment comprises amino acids H468-P504 of human TAB1.

14. A method of utilizing molecular replacement to obtain structural information about a molecule or a molecular complex of unknown structure, wherein the molecule is sufficiently homologous to TAK1, comprising the steps of:

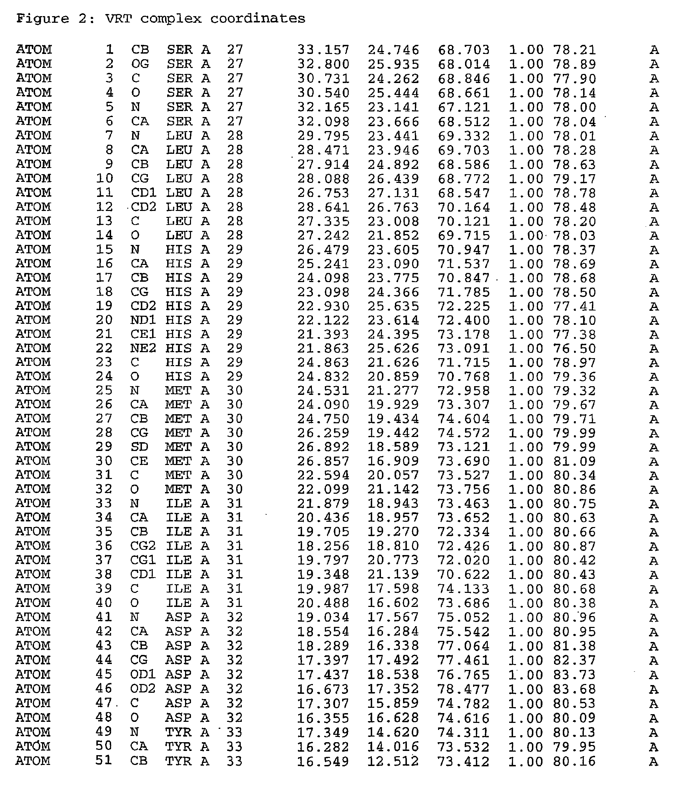

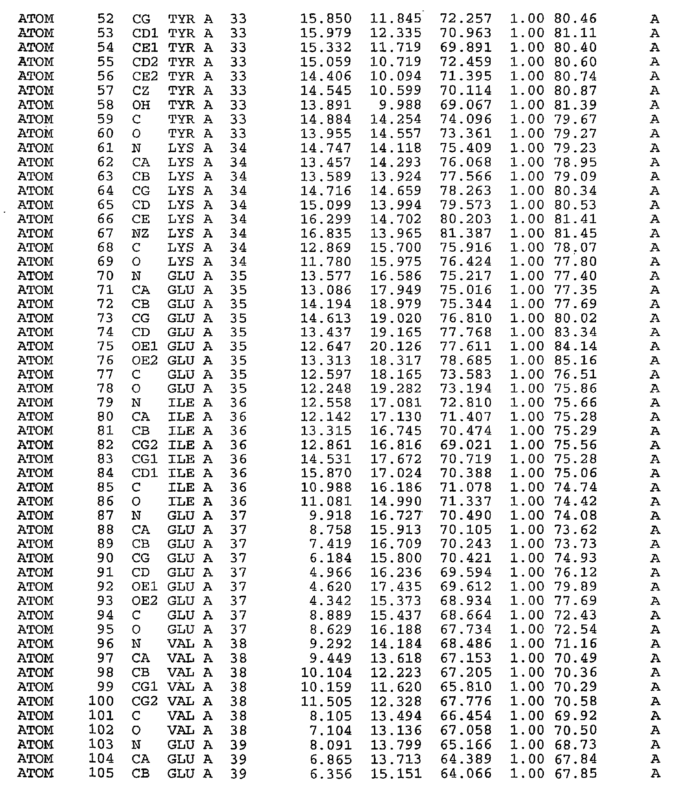

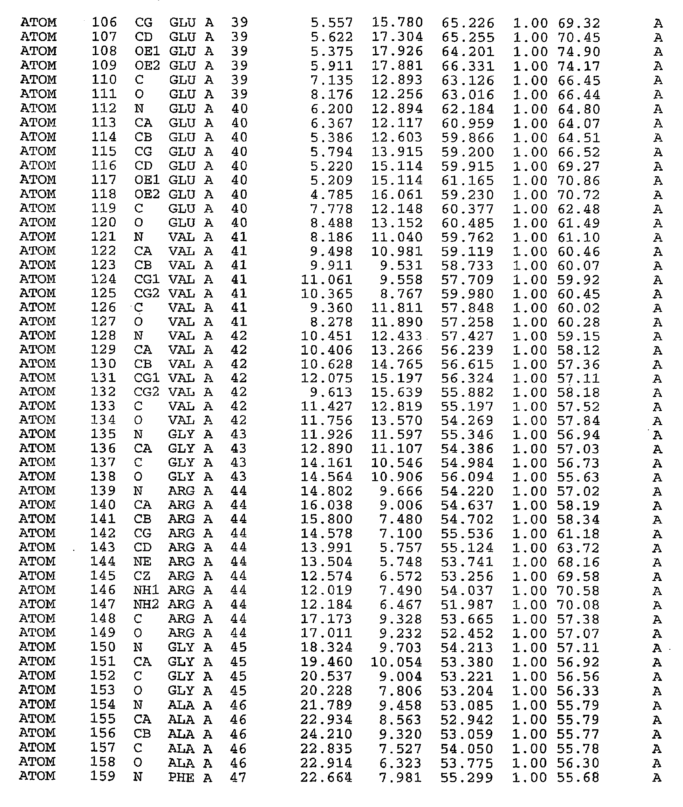

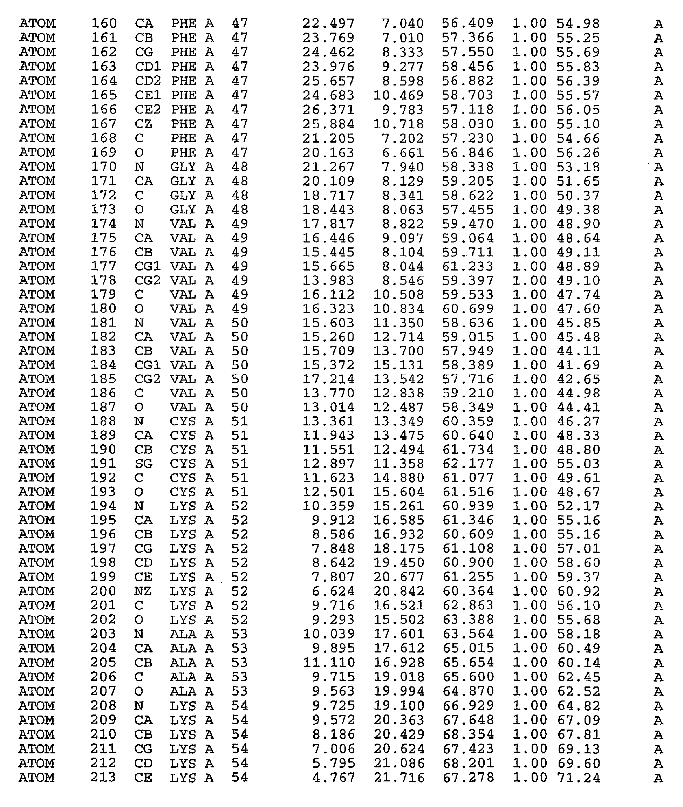

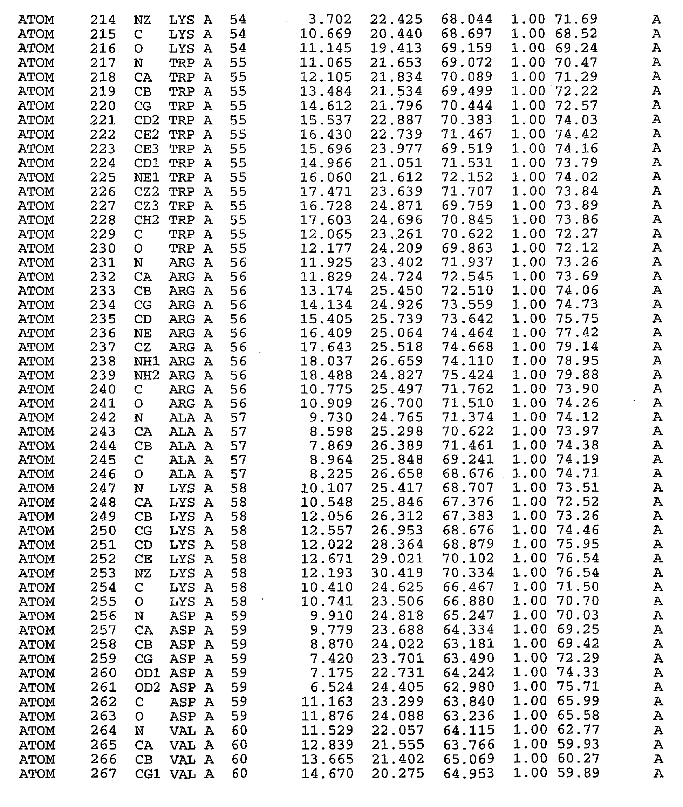

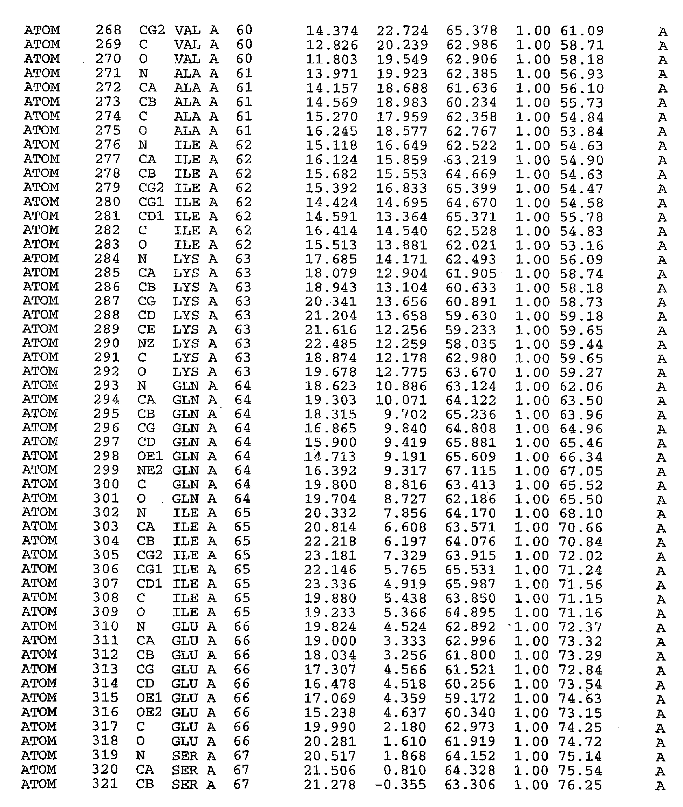

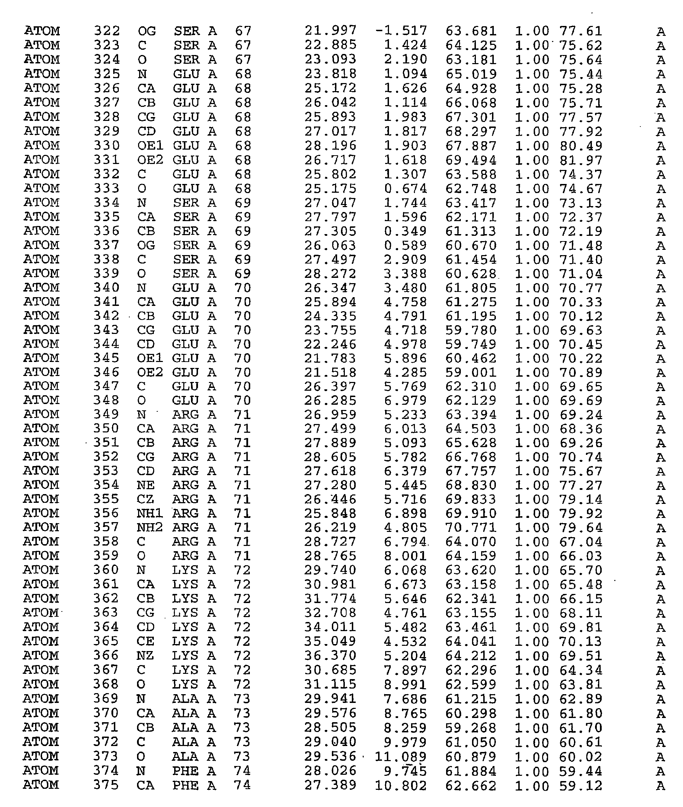

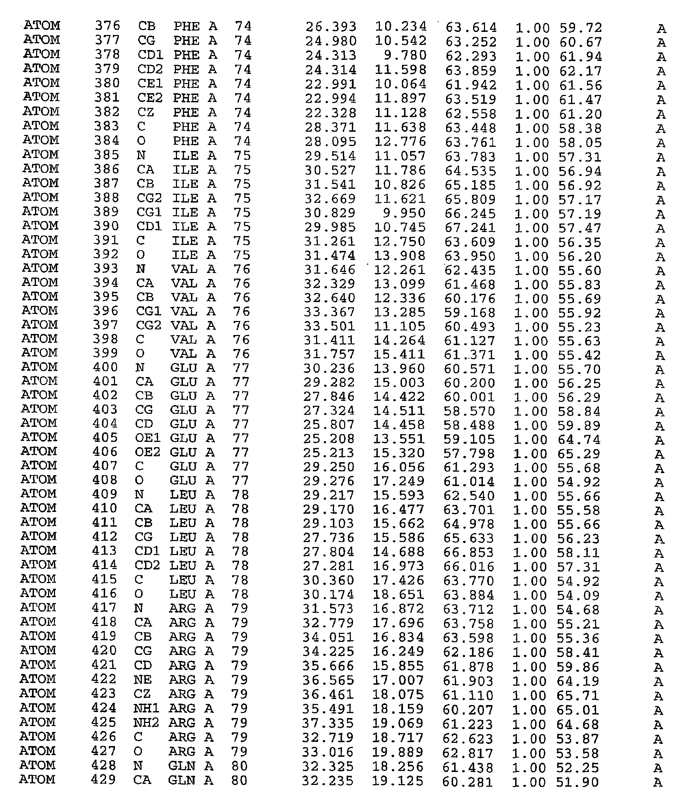

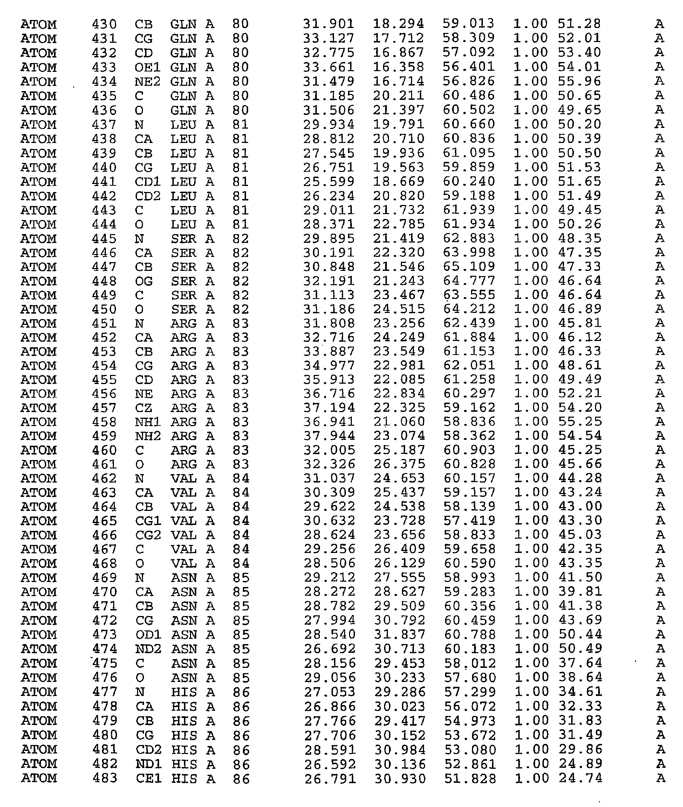

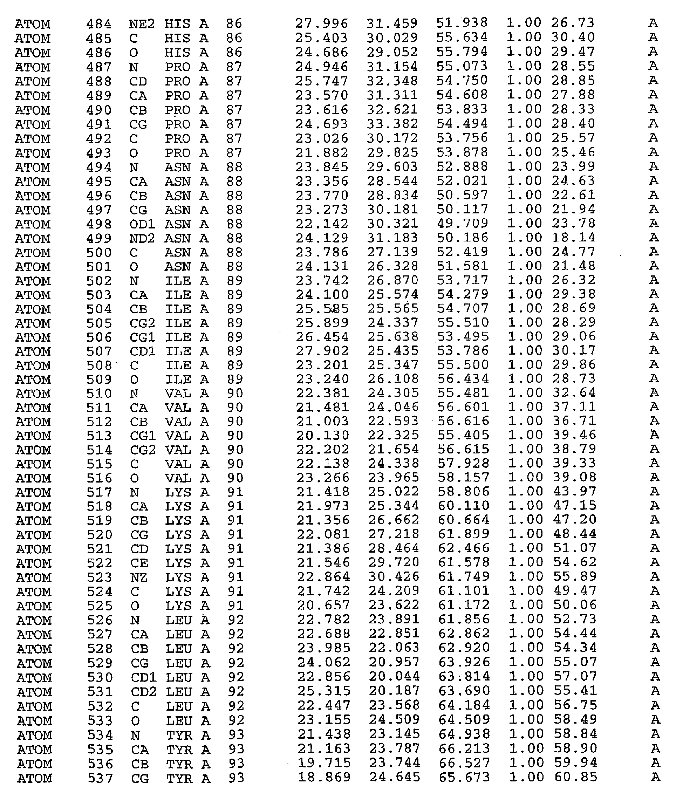

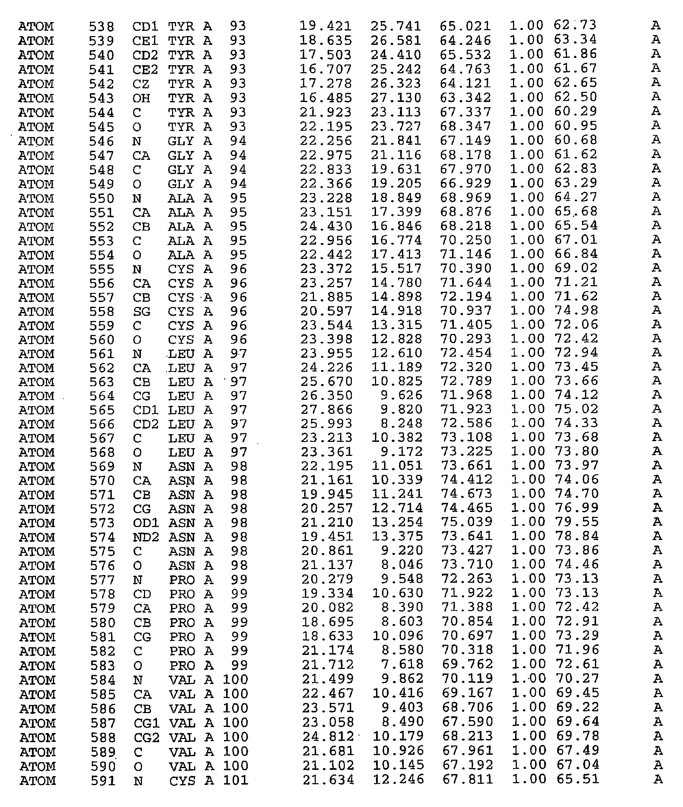

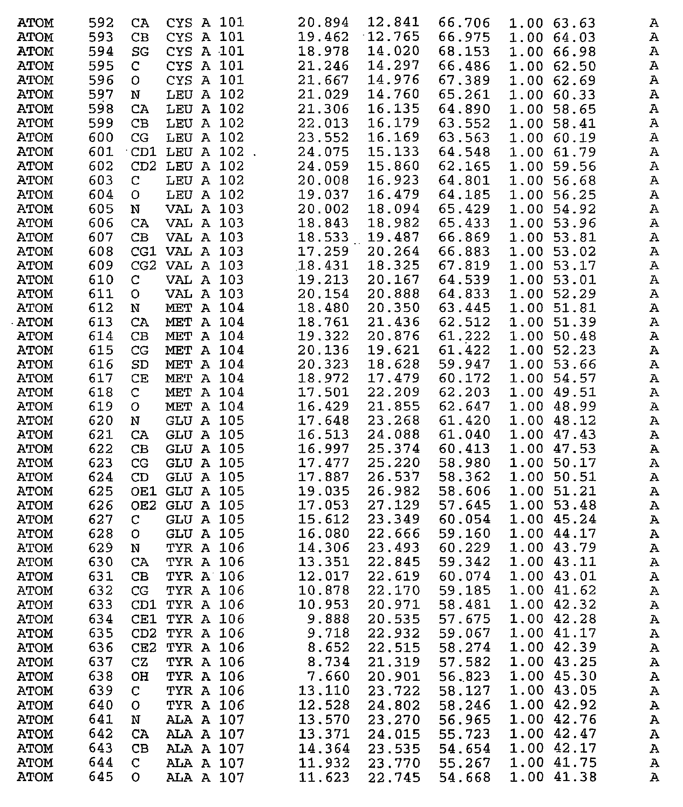

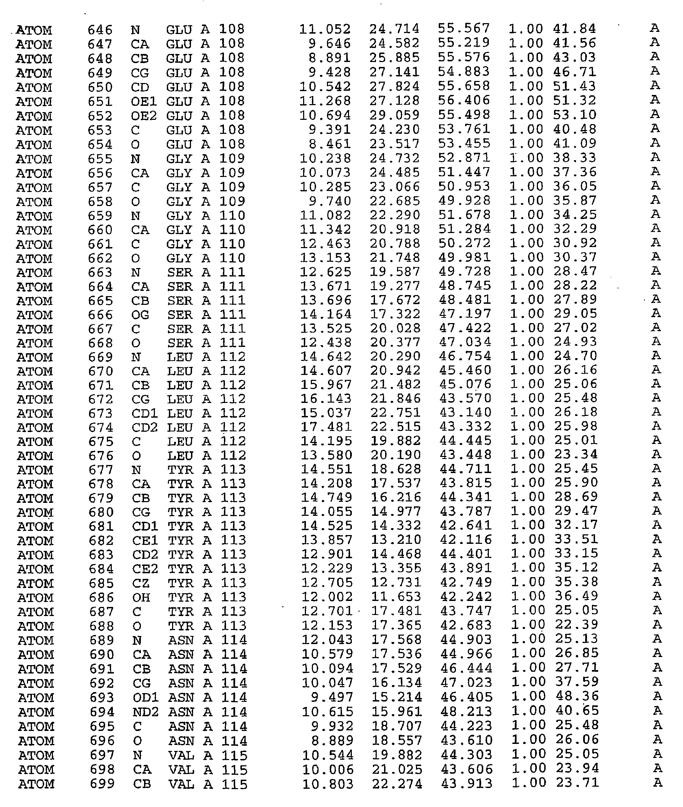

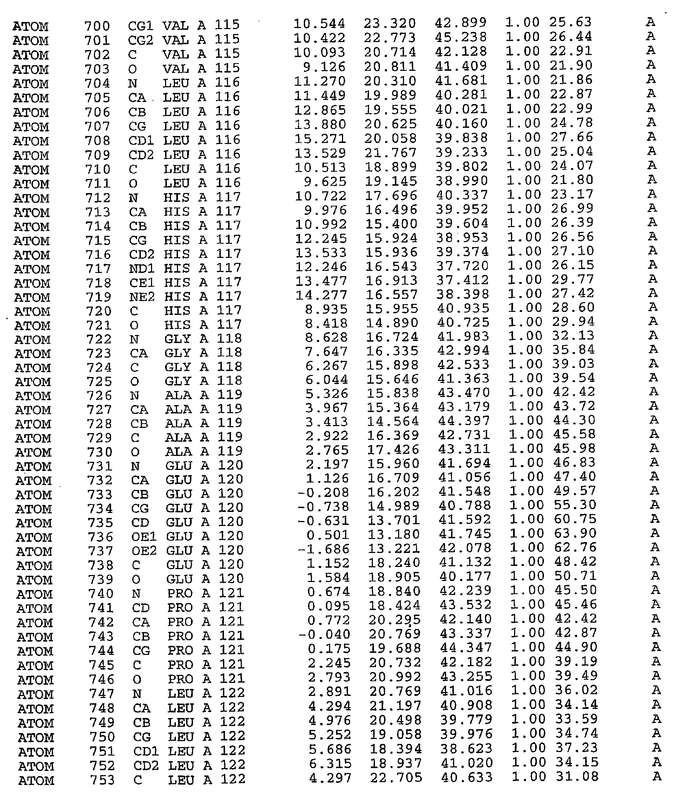

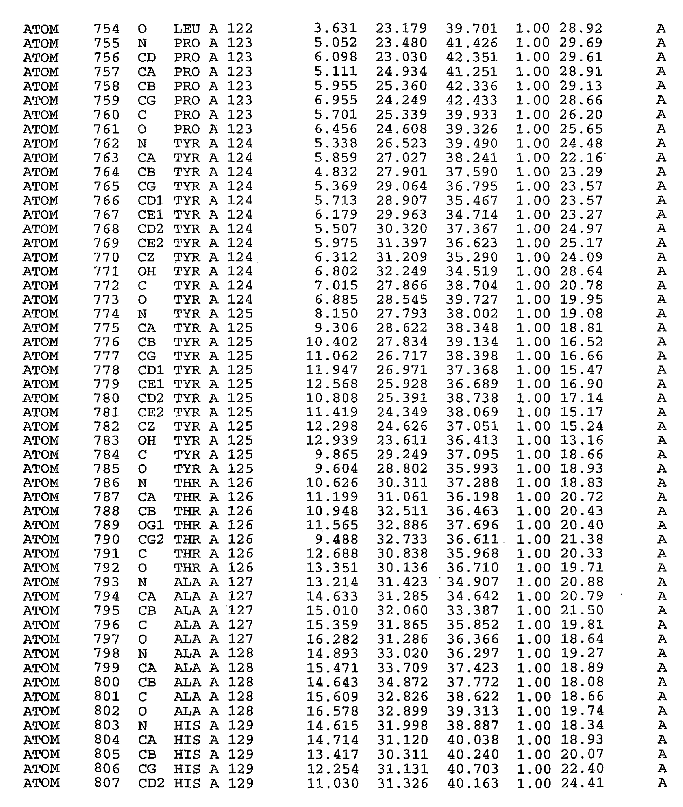

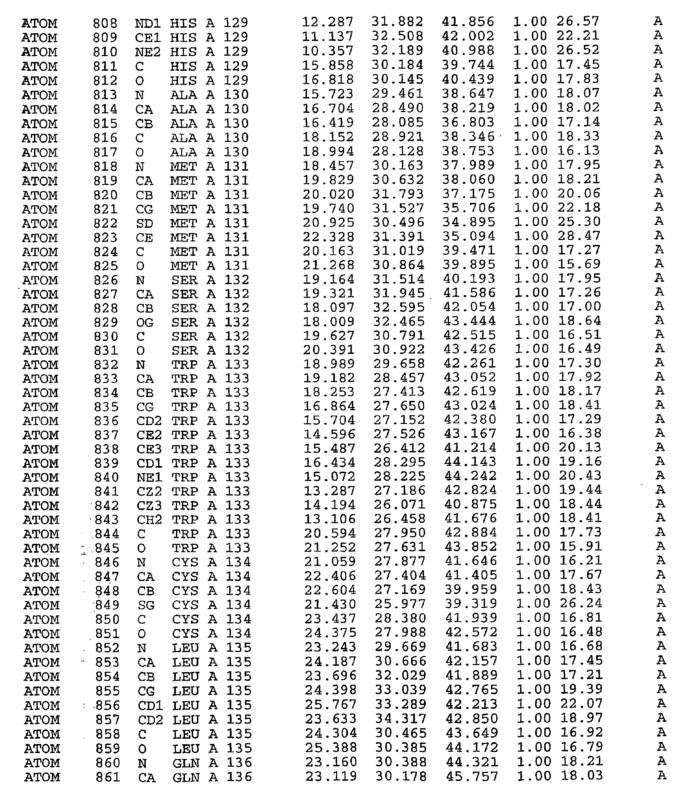

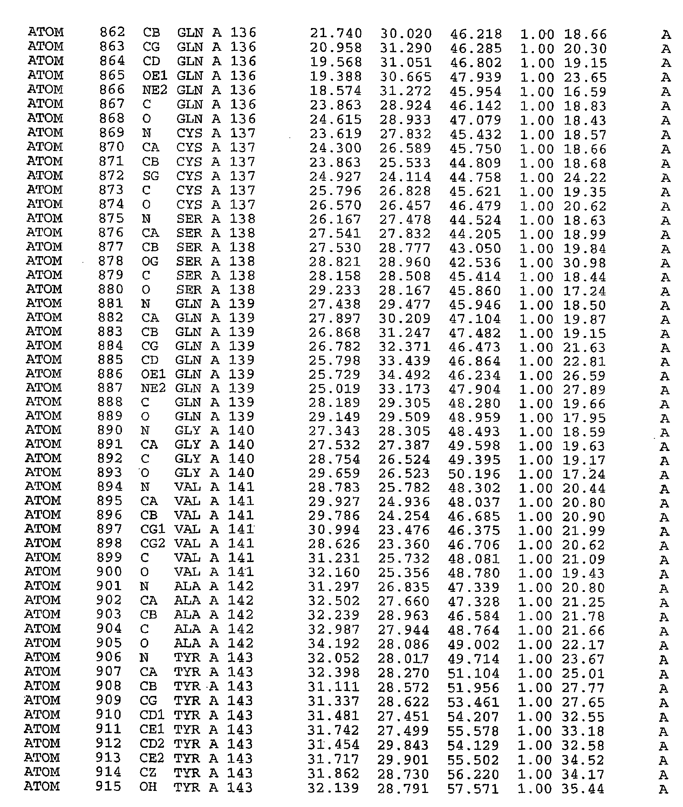

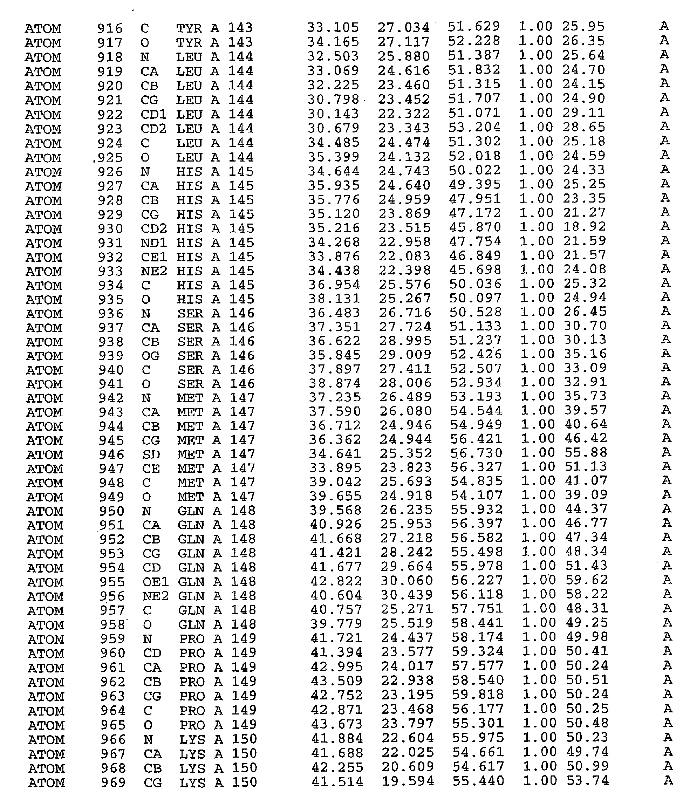

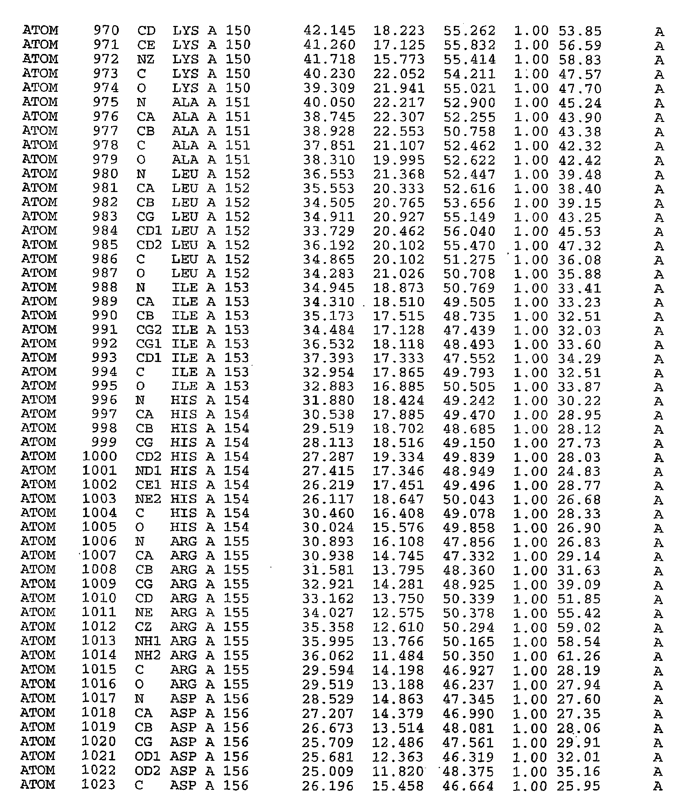

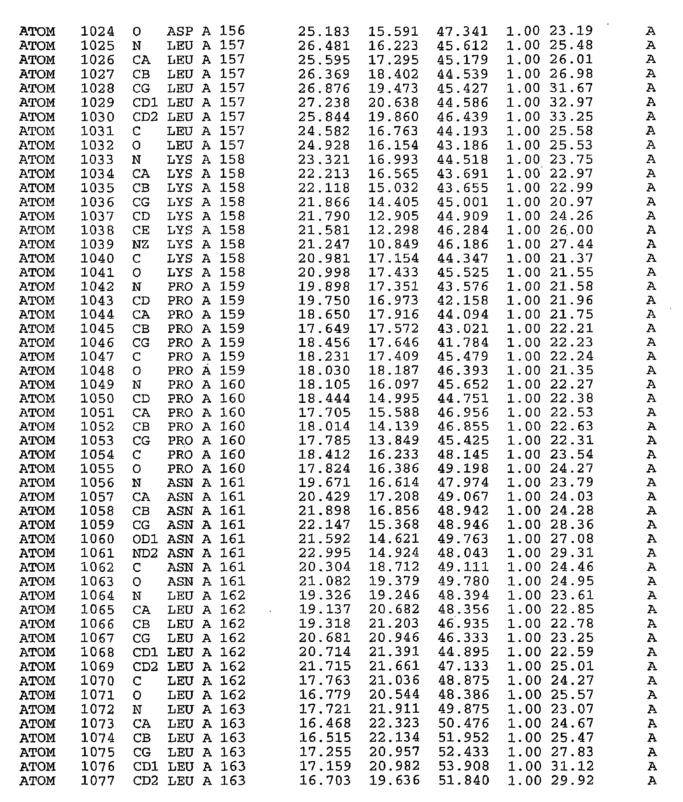

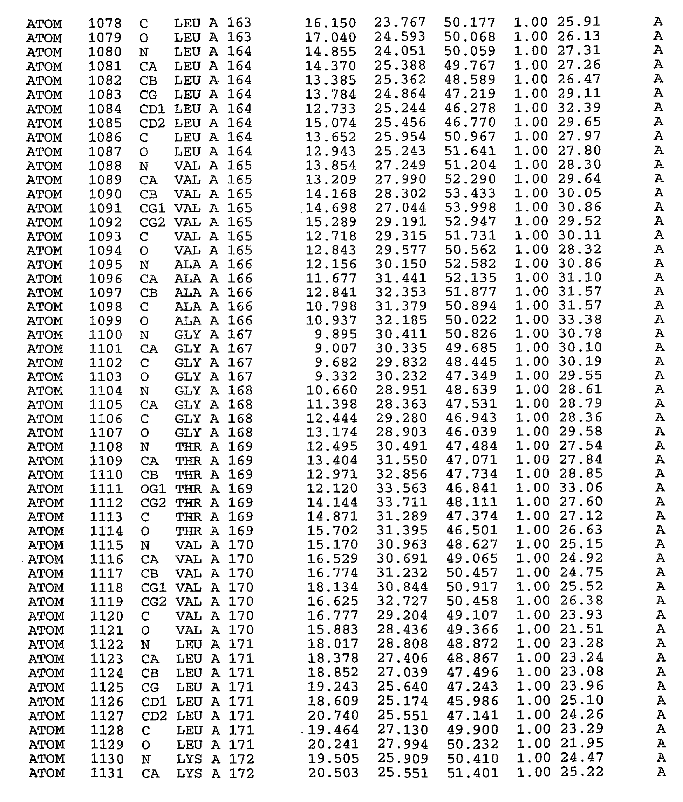

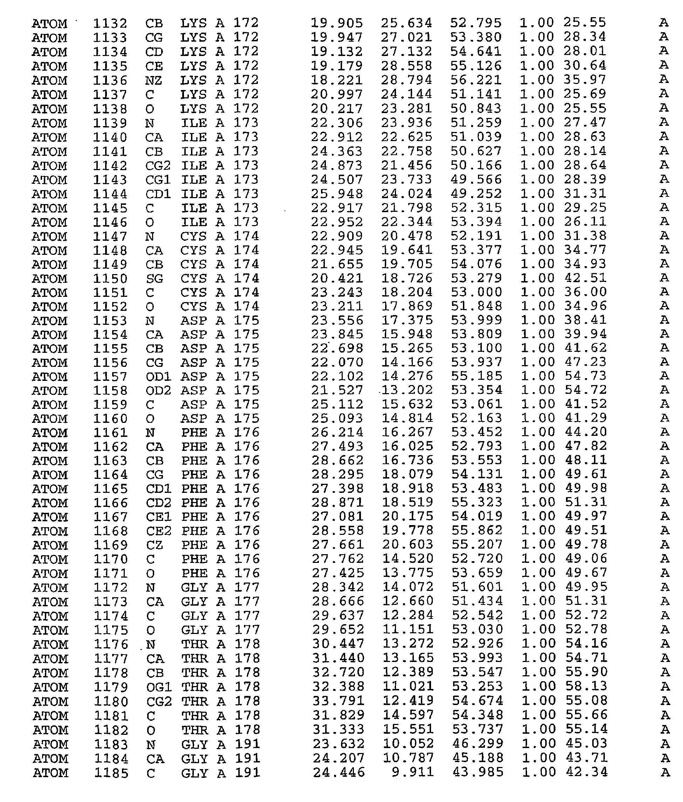

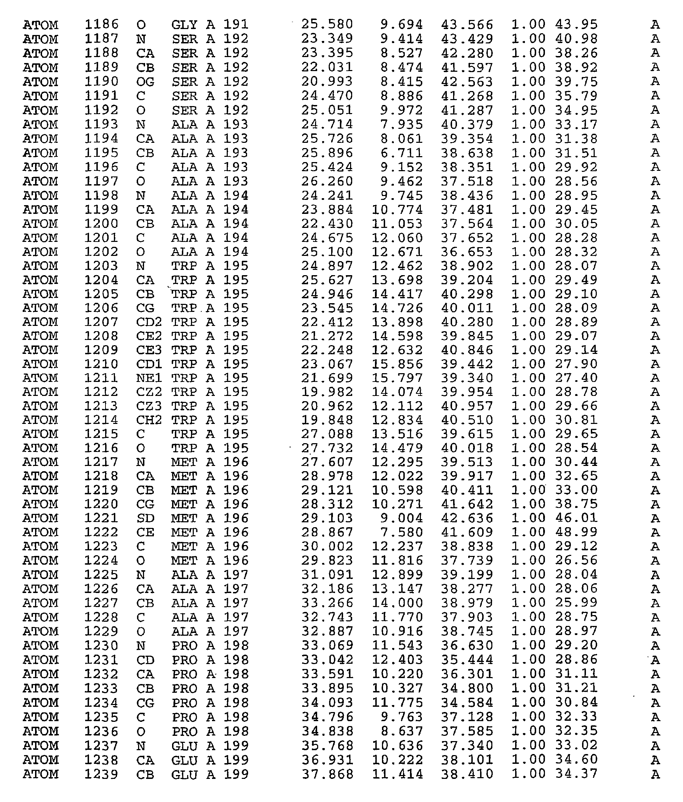

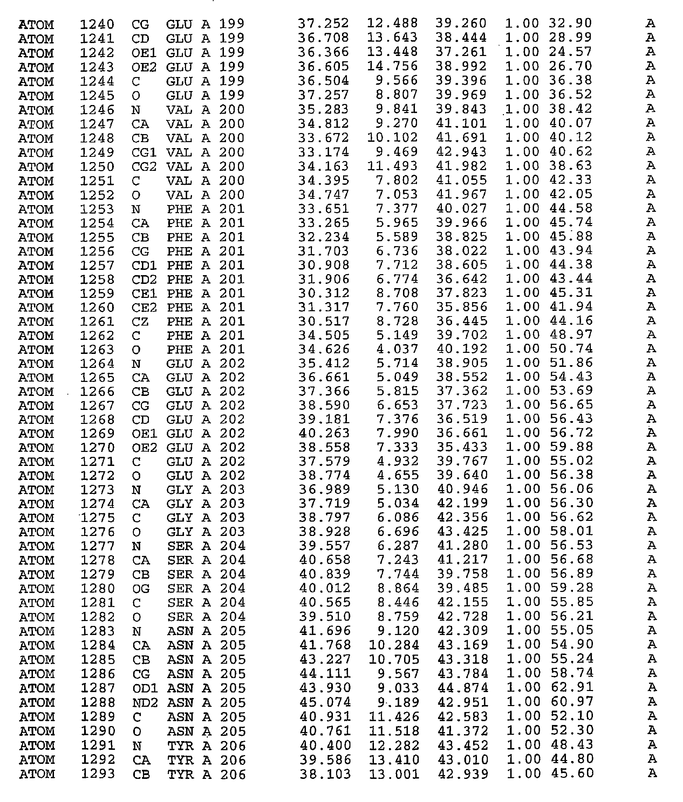

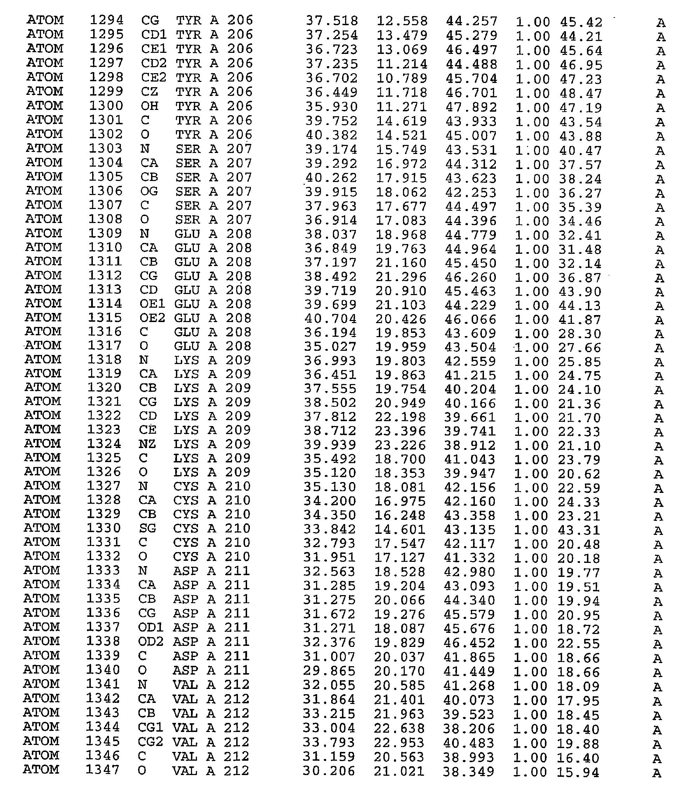

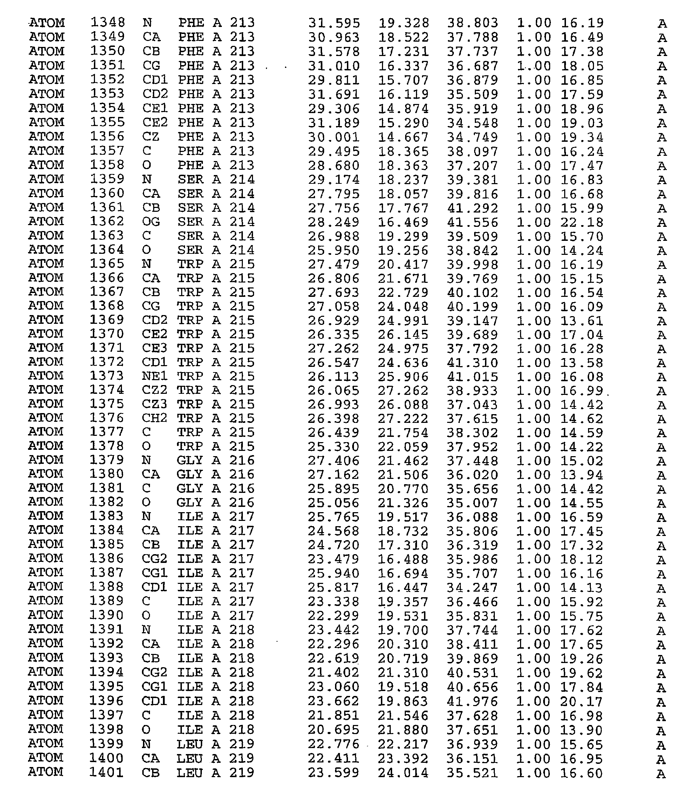

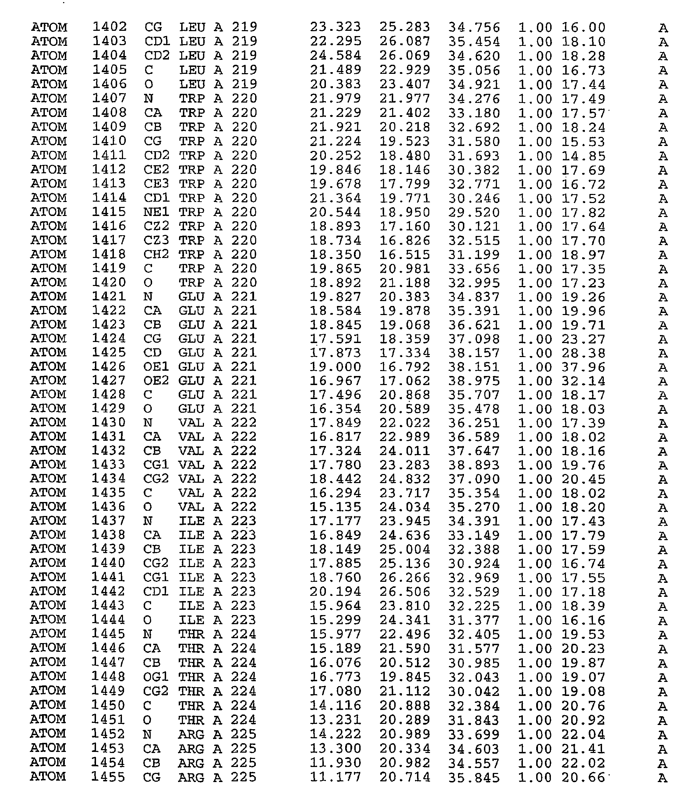

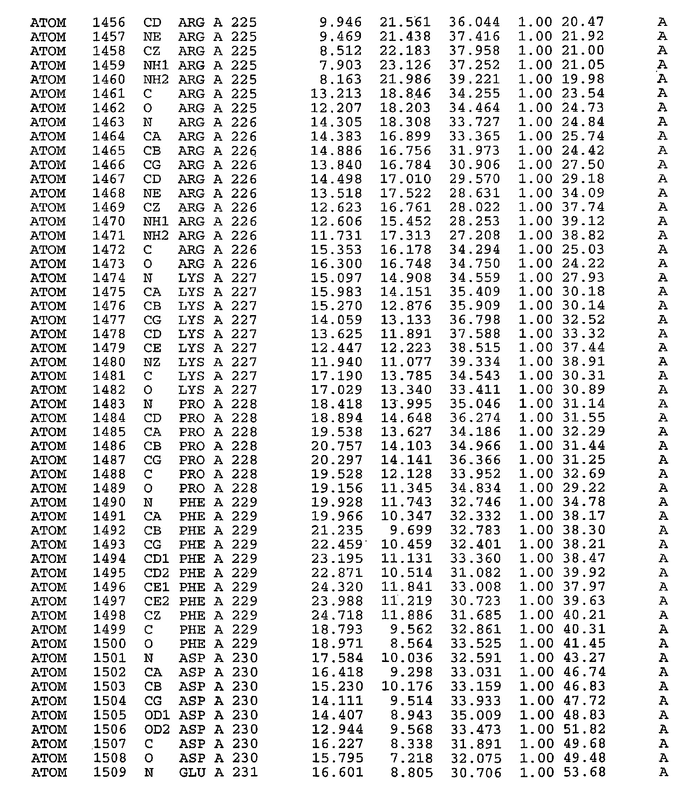

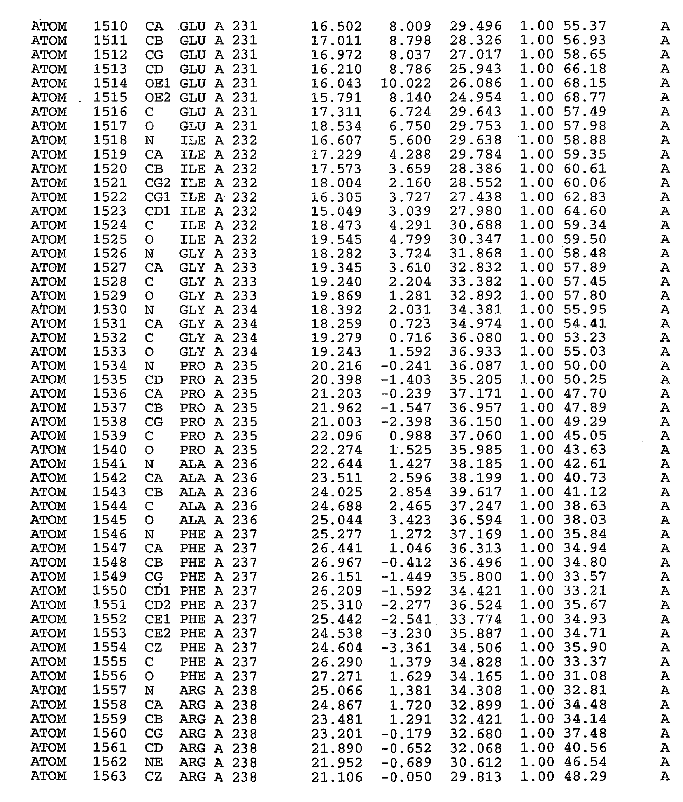

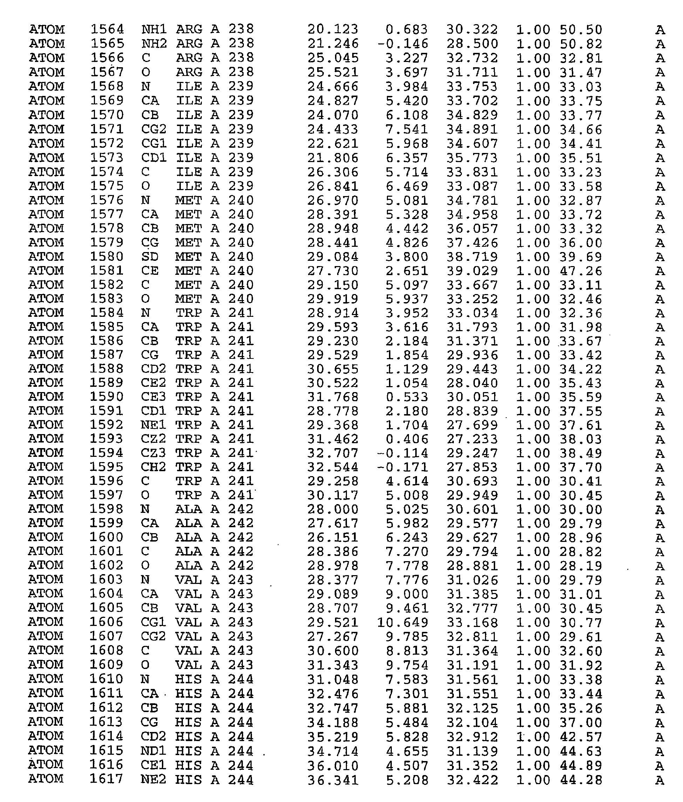

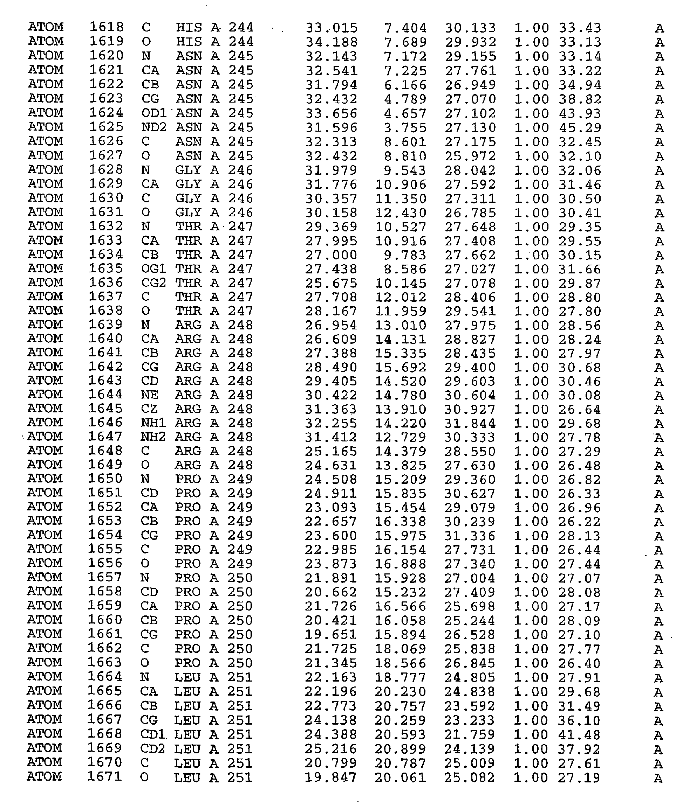

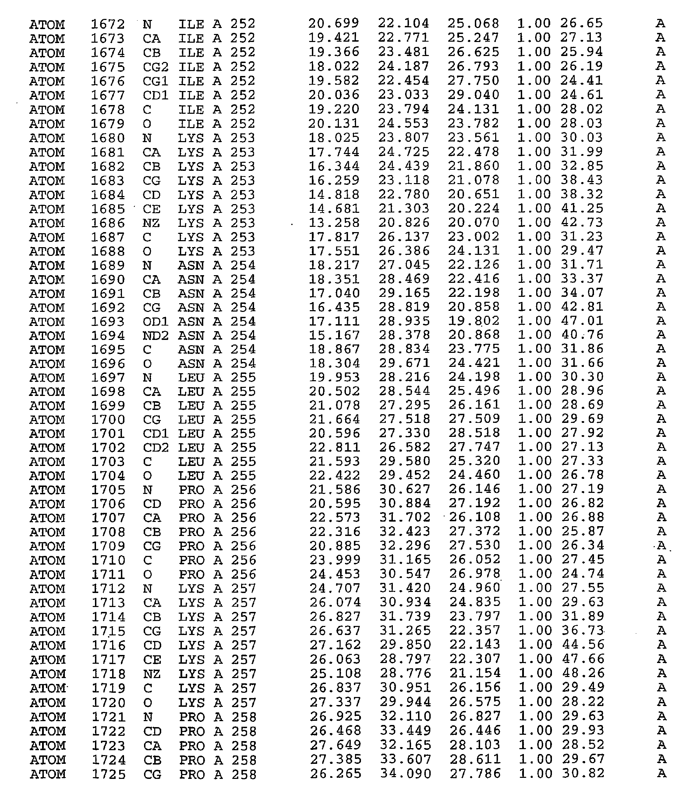

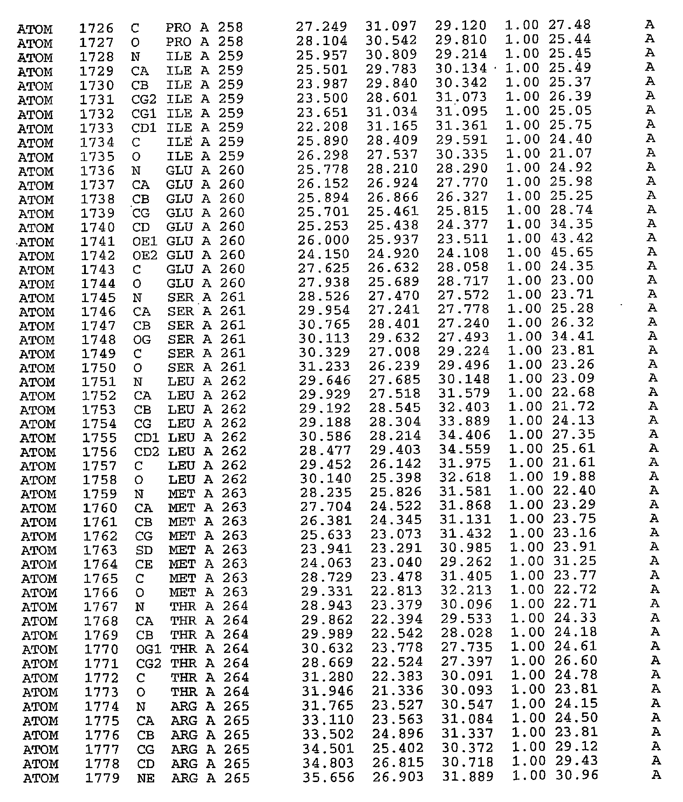

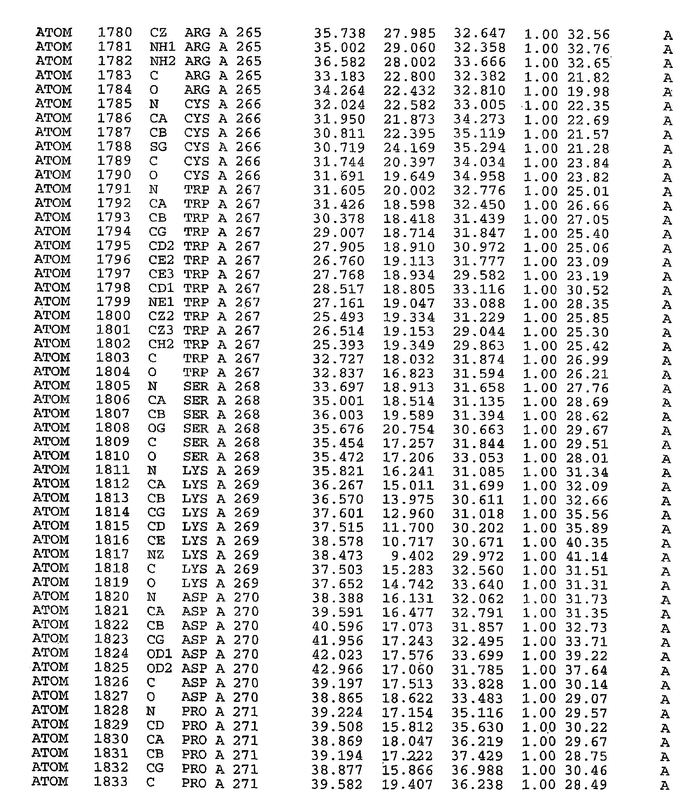

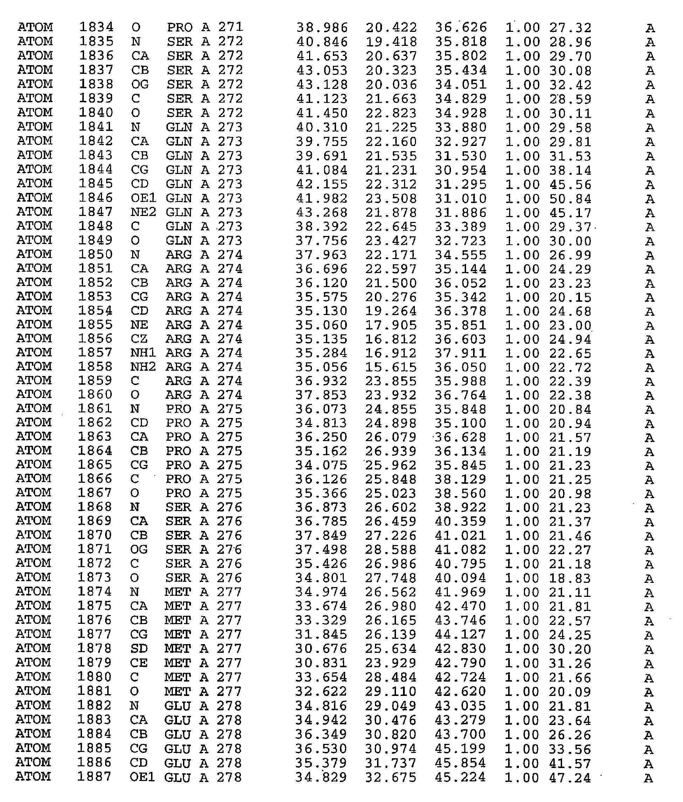

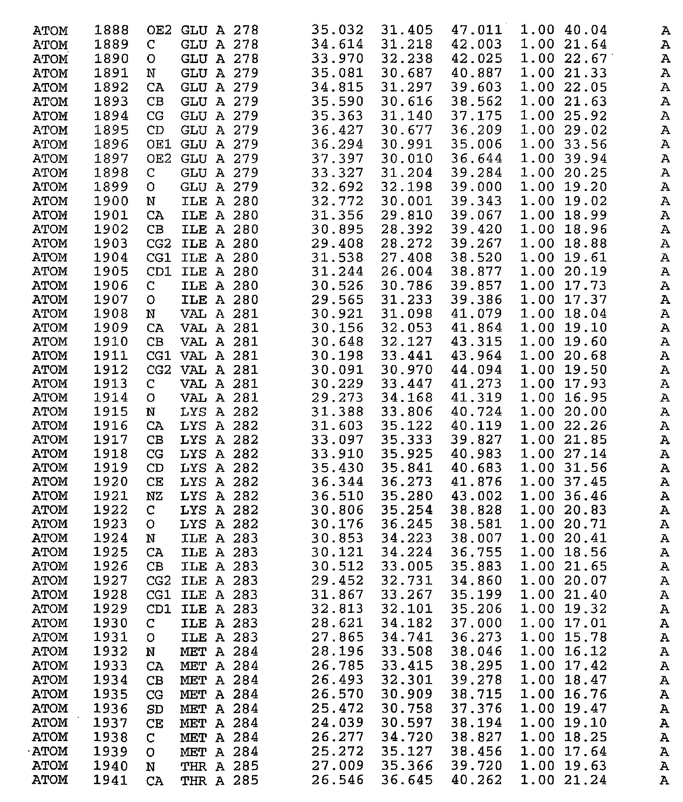

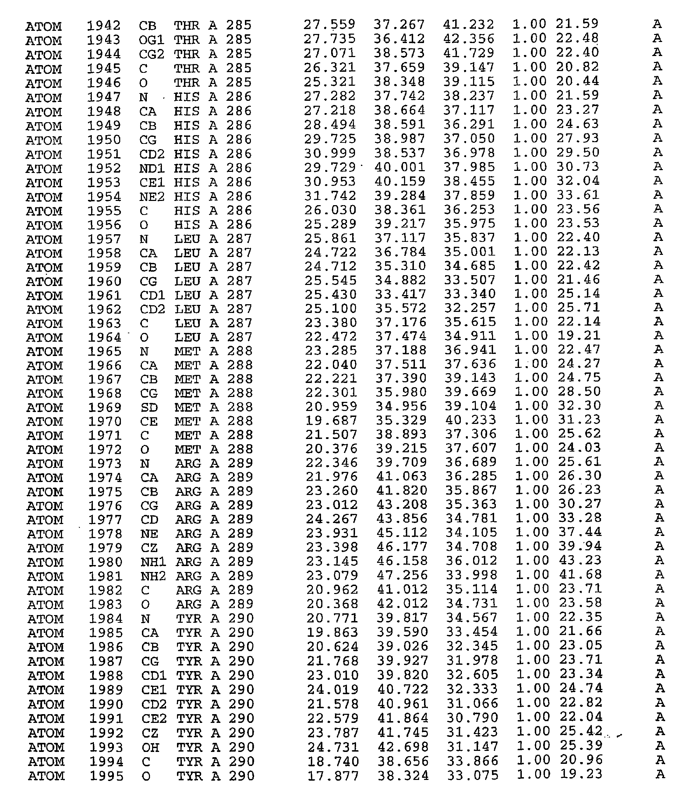

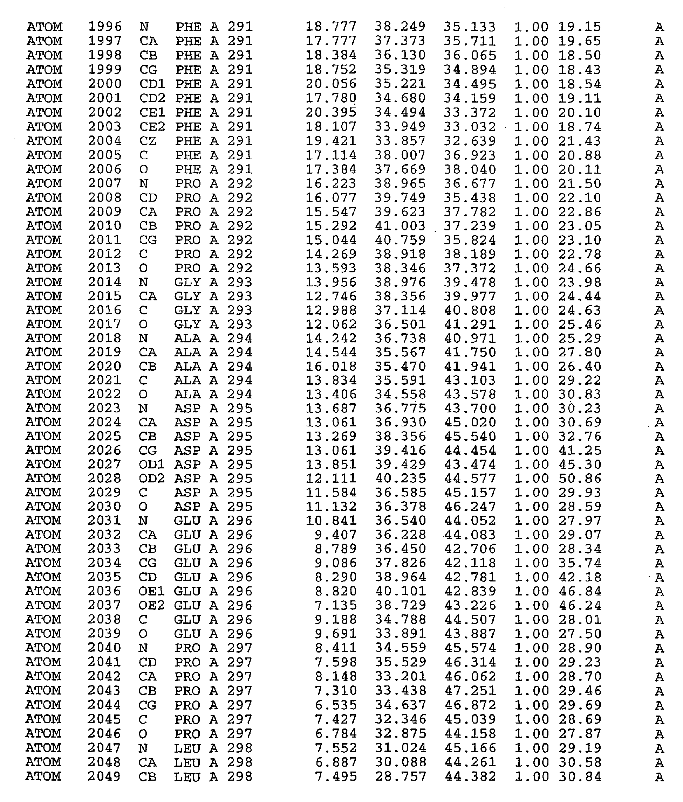

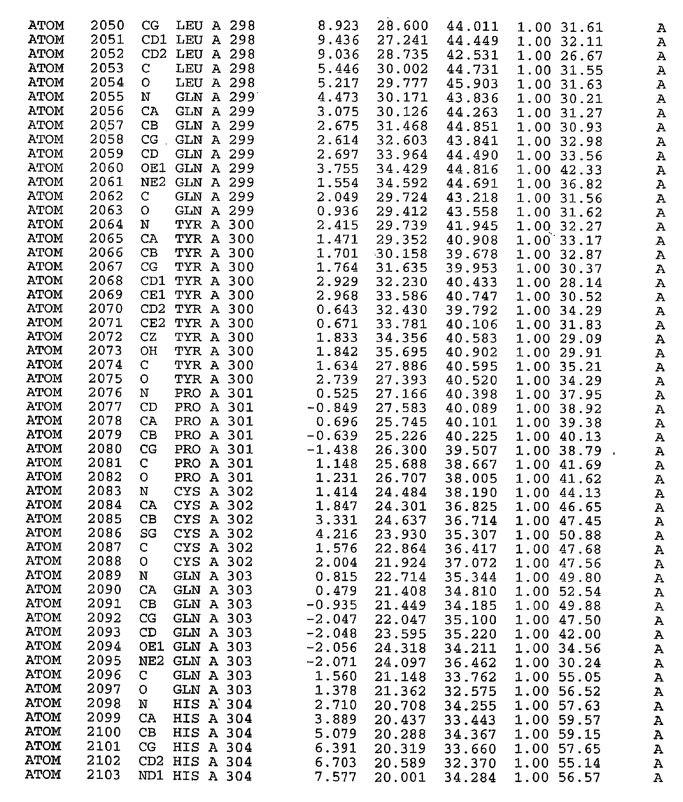

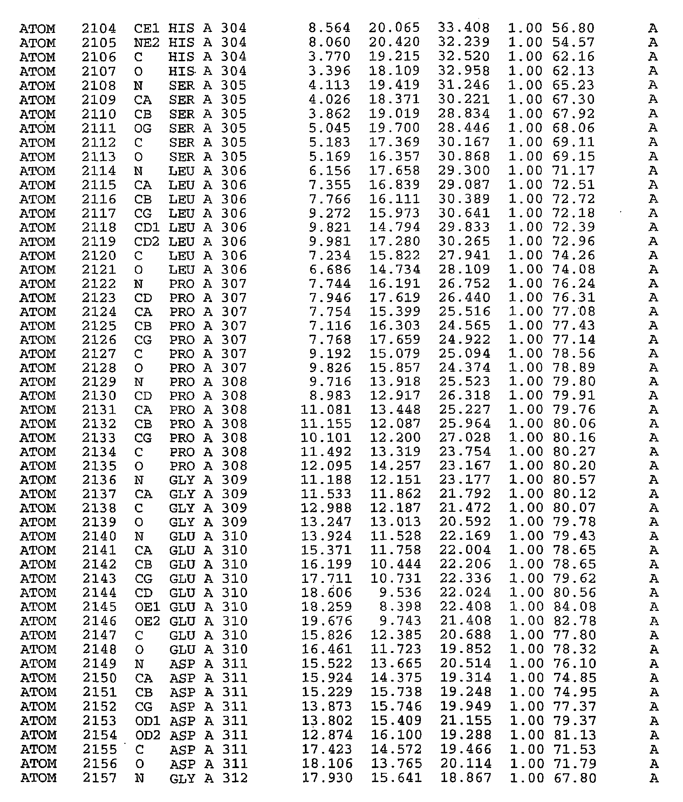

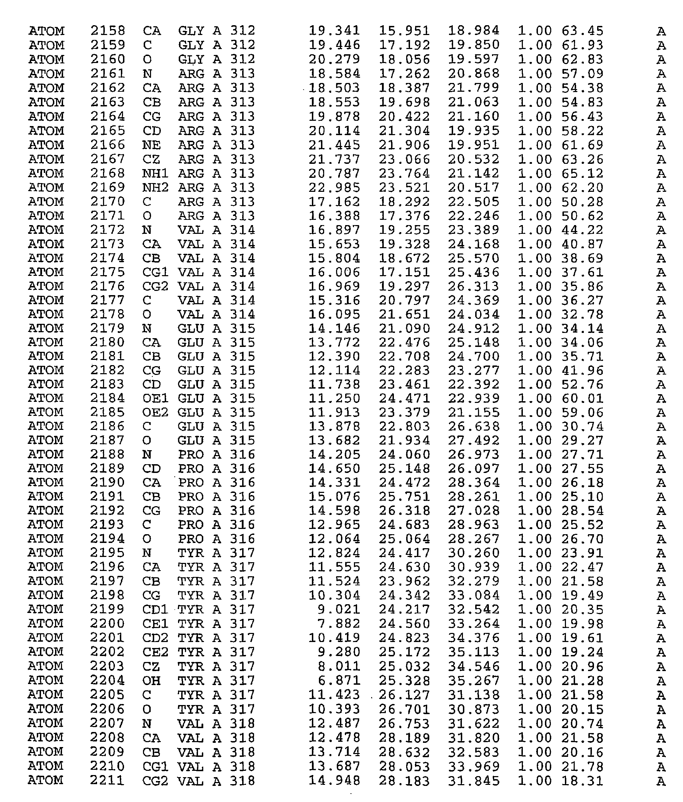

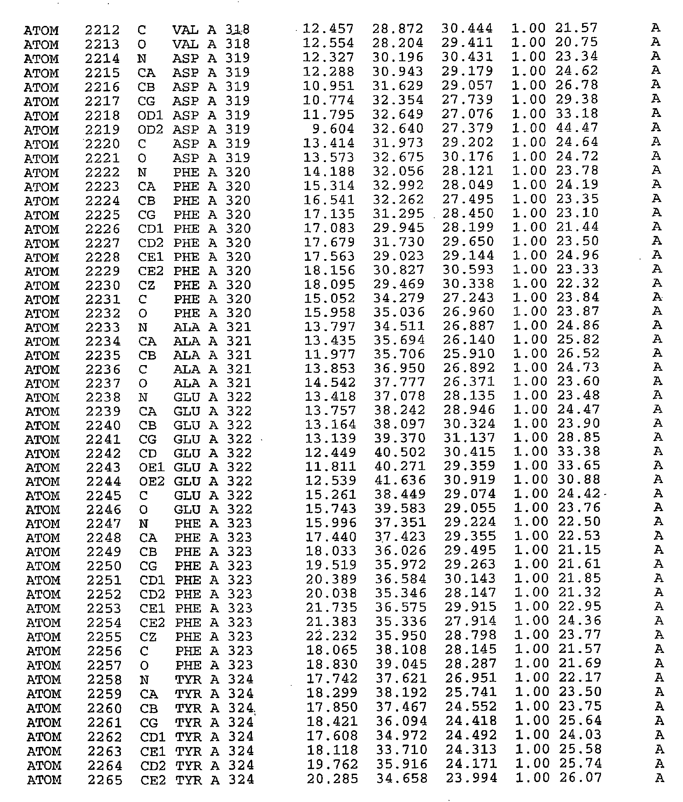

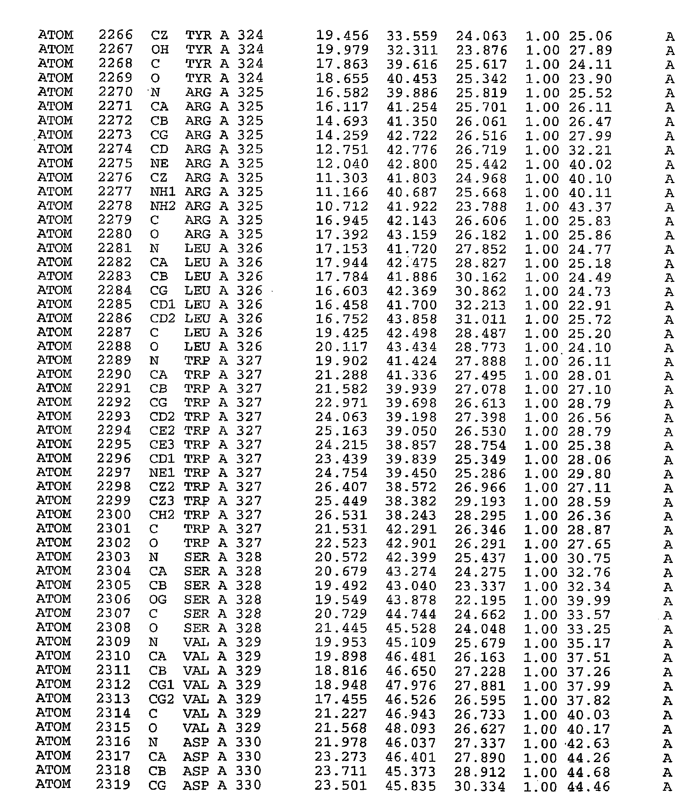

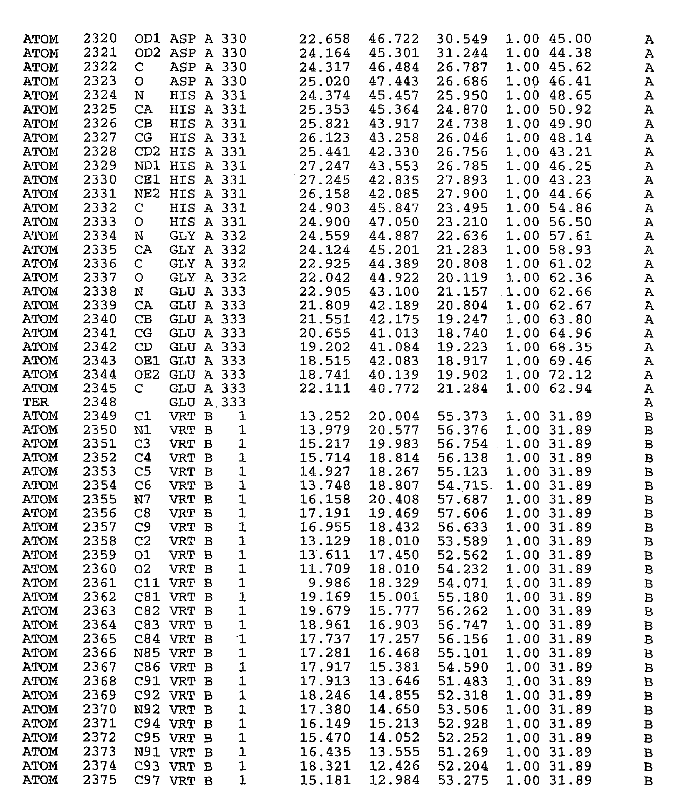

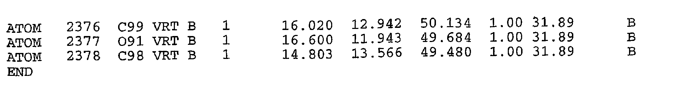

(a) crystallizing said molecule or molecular complex;
(b) generating an X-ray diffraction pattern from said crystallized molecule or molecular complex; and
(c) applying the structure coordinates of Figure 1 or Figure 2 to the X-ray diffraction pattern to generate a three-dimensional electron density map of at least a portion of the molecule or molecular complex of unknown structure; and
(d) generating a structural model of the molecule or molecular complex from the three-dimensional electron density map.

15. A method of identifying a TAK1 binding compound, comprising the steps of:

(a) using an ATP-binding pocket of the structure coordinates of Figure 1 or Figure 2, or a TAK1-TAB1-binding pocket of the structure coordinates of Figure 1 or Figure 2, to computationally screen a candidate compound for an ability to bind an ATP-binding site of TAK1, wherein said ATP-binding pocket comprises the amino acids of V42, V90, M104, E105, A107 and L163 according to Figures 1 and 2, and wherein said TAK1-TAB1-binding pocket comprises the amino acids of M131, P256, Y290 and F291 according to Figures 1 and 2,
(b) synthesizing the candidate compound and
(c) screening the synthesized compound for TAK1 binding activity.

16. A method of utilizing molecular replacement to obtain structural information about a molecule or a molecular complex of unknown structure, wherein the molecule is sufficiently homologous to TAK1, comprising the steps of:

(a) crystallizing said molecule or molecular complex;
(b) generating an X-ray diffraction pattern from said crystallized molecule or molecular complex; and
(c) applying an ATP-binding pocket of the structure coordinates of Figure 1 or Figure 2, or a TAK1-TAB1-binding pocket of the structure coordinates of Figure 1 or Figure 2, to the X-ray diffraction pattern to generate a three-dimensional electron density map of at least a portion of the molecule or molecular complex of unknown structure, wherein said ATP-binding pocket comprises the amino acids of V42, V90, M104, E105, A107 and L163 according to Figures 1 and 2, and wherein said TAK1-TAB1-binding pocket comprises the amino acids of M131, P256, Y290 and F291 according to Figures 1 and 2; and
(d) generating a structural model of the molecule or molecular complex from the three-dimensional electron density map.

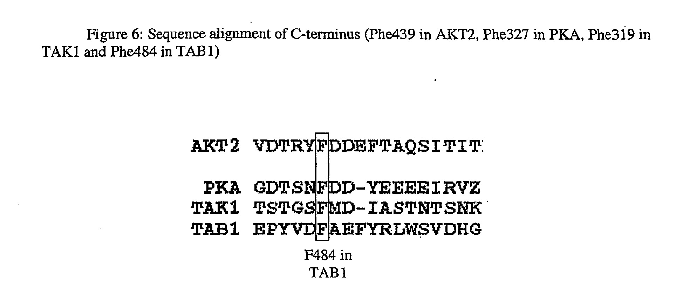

Rational design of TAK1 proteins for crystallization
The invention discloses a method for designing crystallizable TAK-1 proteins. Initial attempts at crystallization of a TAK1-TAB 1 complex based on a previously reported fusion protein (Sakurai H., Nishi A., Sato N., Mizukami J., Miyoshi H. and Sugita T. Biochem Biophys Res Comm. 297 1277-1281 (2002)) proved unsuccessful and so a shorter TAK1 construct comprising Ile31-Gln303 fused directly to the C-terminal 36 residues of TAB1 (His468-Pro504) was expressed in baculovirus and purified. The choice of C-terminus was based on the sequence alignment of the TAK1 (Tyr304-Pro339), TAB1 (His468-Pro504) and AKT2 (Leu423-Leu459) (Figure 6). AKT2 is a member of the AGC kinase family and requires the protein activator PDK1 to induce full activation (Frodin M, Antal TL, Dummler BA, Jensen CJ, Deak M, Gammeltoft S, Biondi RM EMBO J. 2002 Oct 15;21(20):5396-407). The alignment highlights a conserved phenylalanine residue (Phe439 in AKT2, Phe319 in TAK1, Phe484 in TAB1), which in AKT2 lies adjacent to the ATP binding site (Yang J, Cron P, Thompson V, Good VM, Hess D, Hemmings BA, Barford D. Mol Cell. 2002 Jun;9(6):

1227-40.). The TAK1-TAB1 chimera was designed by replacing the C-terminal residues of TAK1 with the corresponding residues of TAB1 preserving the conserved phenylalanine. An additional 18 residues were selected N-terminal to the conserved phenylalanine, up to and including Tyr304 of TAK1, as this preserved a conserved serine residue (Ser305 in TAK1, Ser469 in TAB1). A total of 36 residues were thereby replaced and TAK1 and TAB1 were fused together without a linker region to assist crystallization by removing structurally unimportant residues. Without being bound by theory it is believed that it is advantageous to eliminate the unwieldy linker that had been used in previously studied fusion protein (Glu-Phe-(Gly)5). The N-terminus of the TAK1 kinase domain was chosen based on both sequence and structural alignments of related protein kinases with close homology to TAK1 that have previously been crystallized in the literature. This revealed an interaction between residue I31, a highly conserved hydrophobic residue in the N-terminus of β-strand β1, and a neighbouring hydrophobic pocket.

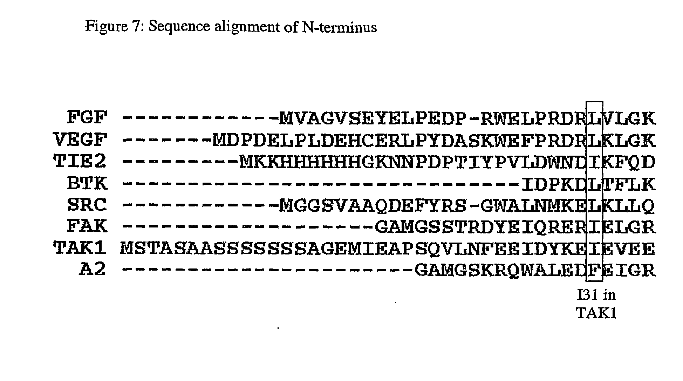

Applicants have observed that both the hydrophobic nature of this residue and hydrophobic nature of the neighbouring pocket is retained in kinases giving crystallizable proteins (Figure 7). Consequently, residue I31 was chosen as the N-terminus of the TAK1-TAB1 chimera. The choice of N- and C-termini was validated since the construct both retained catalytic activity and crystallized.

Crystallizable Compositions and Crystals of TAK1 Complexes

This invention provides an isolated, purified TAK1 protein and a crystal thereof as disclosed in claims 1 to 8.

The invention discloses a crystallizable composition comprising unphosphorylated TAK1 protein. In another embodiment, the invention provides a crystallizable composition comprising unphosphorylated TAK1 protein and a substrate analogue, such as but not limited to adenosine. In one embodiment, the aforementioned crystallizable composition further comprises a precipitant, 600-900mM sodium citrate (the precipitant), 1 to 200mM sodium chloride and a buffer that maintains pH at between about 6.5 and about 8.5. The composition may further comprise a reducing agent, such as dithiothreitol (DTT) at between about 1 to about 20 mM. The unphosphorylated TAK1 protein or complex is preferably about 85-100% pure prior to forming the composition.

The invention discloses a crystal composition comprising TAK1 protein complex. In one embodiment, the crystal has a unit cell dimension of a=58 Å, b= 144 Å, c= 134 Å, $\alpha = \beta = \gamma = 90°$ and belongs to space group 1222. It will be readily apparent to those skilled in the art that the unit cells of the crystal compositions may deviate $\pm$ 1-2 Å from the above cell dimensions depending on the deviation in the unit cell calculations.

As used herein, the TAK1 protein in the crystal or crystallizable compositions can be a truncated protein with amino acids 31 to 340 as shown in Figures 1-3; and the truncated protein with conservative substitutions.

The TAK1 protein may be produced by any well-known method, including synthetic methods, such as solid phase, liquid phase and combination solid phase/liquid phase syntheses; recombinant DNA methods, including cDNA cloning, optionally combined with site directed mutagenesis; and/or purification of the natural products. Preferably, the protein is overexpressed from a baculovirus system.

The invention also discloses a method of making crystals of TAK1 complexes or TAK1 homologue complexes. Such methods comprise the steps of:

a) producing a composition comprising a crystallization solution and TAK1 protein or homologue thereof complexed with a chemical entity; and
b) subjecting said composition to devices or conditions which promote crystallization.

In one embodiment, the chemical entity is selected from the group consisting of an ATP analogue, nucleotide triphosphate, nucleotide diphosphate, phosphate, adenosine, or active site inhibitor. In another embodiment, the chemical entity is an ATP analogue. In certain exemplary embodiments, the chemical entity is 3-(8-Phenyl-5,6-dihydrothieno[2,3-h]quinazolin-2-ylamino)benzenesulfon-amide. In yet another embodiment, the crystallization solution is as described previously. In another embodiment, the composition is treated with micro-crystals of TAK1 or TAK1 complexes or homologues thereof. In another embodiment, the composition is treated with micro-crystals of TAK1 complexes or homologues thereof.

The invention discloses a method of making TAK1 crystals, the method comprising steps of:

a) producing and purifying TAK1 protein;
b) producing a crystallizable composition; and
c) subjecting said composition to devices which promote crystallization.

In one embodiment, the crystallizable composition of step b) is made according to the conditions dislcosed above.

Devices for promoting crystallization can include but are not limited to the hanging-drop, sitting-drop, dialysis or microtube batch devices. [U.S. patent 4,886,646, 5,096,676, 5,130,105, 5,221,410 and 5,400,741; Pav et al., Proteins: Structure. Function, and Genetics, 20, pp. 98-102 (1994), The hanging-drop or sitting-drop methods produce crystals by vapor diffusion. The hanging-drop, sitting-drop, and some adaptations of the microbatch methods [D'Arcy et al., J. Cryst. Growth, 168, pp. 175-180 (1996) and Chayen, J. Appl. Cryst., 30, pp. 198-202 (1997)] produce crystals by vapor diffusion. The hanging drop and sitting drop containing the crystallizable composition is equilibrated in a reservoir containing a higher or lower concentration of the precipitant As the drop approaches equilibrium with the reservoir, the saturation of protein in the solution leads to the formation of crystals.

Microseeding or seeding may be used to obtain larger, or better quality (*i.e.,* crystals with higher resolution diffraction or single crystals) crystals from initial micro-crystals. Microseeding involves the use of crystalline particles to provide nucleation under controlled crystallization conditions. Microseeding is used to increase the size and quality of crystals. In this instance, micro-crystals are crushed to yield a stock seed solution. The stock seed solution is diluted in series. Using a needle, glass rod or strand of hair, a small sample from each diluted solution is added to a set of equilibrated drops containing a protein concentration equal to or less than a concentration needed to create crystals without the presence of seeds. The aim is to end up with a single seed crystal that will act to nucleate crystal growth in the drop.

It would be readily apparent to one of skill in the art following the teachings of the specification to vary the crystallization conditions disclosed herein to identify other crystallization conditions that would produce crystals of TAK1 homologue, TAK1 homologue complex, TAK1 protein or other TAK1 protein complexes. Such variations include, but are not limited to, adjusting pH, protein concentration and/or crystallization temperature, changing the identity or concentration of salt and/or precipitant used, using a different method of crystallization, or introducing additives such as detergents (*e.g.*, TWEEN 20 (monolaurate), LDAO, Brij 30 (4 lauryl ether)), sugars (*e.g.*, glucose, maltose), organic compounds (*e.g.*, dioxane, dimethylformamide), lanthanide ions or polyionic compounds that aid in crystallization. High throughput crystallization assays may also be used to assist in finding or optimizing the crystallization conditions.

Binding Pockets of TAK1 Protein or Homologues thereof

As disclosed above, applicants have provided for the first time the three-dimensional X-ray crystal structures of a TAK1 - inhibitor complex. The crystal structure of TAK1 presented here is the first reported for TAK1. The invention will be useful for inhibitor design to study the role of TAK1 in cell signaling. The atomic coordinate data is presented in Figures 1-3.

In order to use the structure coordinates generated for TAK1, their complexes, one of their binding pockets, or a TAK1-like binding pocket thereof, it is often at times necessary to convert the coordinates into a three-dimensional shape. This is achieved through the use of commercially available software that is capable of generating three-dimensional graphical representations (*e.g.*, three-dimensional structures) of molecules or portions thereof from a set of structure coordinates.

Binding pockets, also referred to as binding sites in the present invention, are of significant utility in fields such as drug discovery. The association of natural ligands or substrates with the binding pockets of their corresponding receptors or enzymes is the basis of many biological mechanisms of action. Similarly, many drugs exert their biological effects through association with the binding pockets of receptors and enzymes. Such associations may occur with all or part of the binding pocket. An understanding of such associations will help lead to the design of drugs having more favorable associations with their target receptor or enzyme, and thus, improved biological effects. Therefore, this information is valuable in designing potential inhibitors of the binding pockets of biologically important targets. The ATP and substrate binding pockets of this invention will be useful for drug design.

Applicants invention has confirmed that amino acids E105 and A107 are in the hinge region of the TAK1 protein. A binding pocket comporising these amino acids would be useful for drug design. Accordingly, in one embodiment, this invention provides a binding pocket comprising amino acids E105 and A107.

In another embodiment, the ATP-binding pocket comprises amino acids V42, G43, V50, A61, K63, V90, M104, E105, Y106, A107, E108, G109, S111, N114, L163 and C174 using the structure of the TAK1 - adenosine complex according to Figure 1. In another embodiment, the ATP-binding pocket comprises amino acids V42, G43, V50, A61, K63, V90, M104, E105, Y106, A107, E108, G109, S111, N114, L163 and C174 using the structure of the TAK1 - 3-[6-(4-Acetyl-3,5-dimethyl-piperazin-1-yl)-pyridin-2-yl]-1H-pyrrolo[2,3-b]pyridine-5-carboxylic acid methyl ester complex according to Figure 2.

In resolving the crystal structures of the unphosphorylated TAK1 - inhibitor complexes, applicants have determined that the above amino acids are within 5 Å ("5 Å sphere amino acids") of the inhibitor bound in the ATP-binding pockets. These residues were identified using the program QUANTA [Molecular Simulations, Inc., San Diego, CA ©1998,2000], O [T.A. Jones et al., Acta Cryst. A, 47, pp. 110-119 (1991)] and RIBBONS [Carson, J. Appl. Cryst., 24, pp. 958-961 (1991)]. The programs allow one to display and output all residues within 5 Å from the inhibitor. Thus, a binding pocket defined by the structural coordinates of these amino acids, as set forth in Figures 1 and 2 is considered an TAK1-ATP binding pocket of this invention.

In another embodiment, the ATP-binding pocket comprises amino acids E40, V42, G43, G48, V50, C51, K52, D59, V60, A61, I62, K63, V90, L97, L102, V103, M104, E105, Y106, A107, E108, G109, S111, L112, Y113, N114, P160, N161, L162, L163, L164, K172, I173, C174 and D175 using the structure of the TAK1 - adenosine complex to Figure 1.

In another embodiment, the ATP-binding pocket comprises amino acids E40, V42, G43, G48, V50, C51, K52, D59, V60, A61, I62, K63, V90, L97, L102, V103, M104, E105, Y106, A107, E108, G109, S111, L112, Y113, N114, P160, N161, L162, L163, L164, K172, I173, C174 and D175 using the structure of the TAK1 - 3-[6-(4-Acetyl-3,5-dimethyl-piperazin-1-yl)-pyridin-2-yl]-1H-pyrrolo[2,3-b]pyridine-5-carboxylic acid methyl ester complex according to Figure 2. In the crystal structures of the TAK1 - inhibitor complexes, applicants have determined that the above amino acids are within 8 Å ("8 Å sphere amino acids") of the inhibitor bound in the ATP-binding pockets. These residues were identified using the programs QUANTA, O and RIBBONS, supra. Thus, a binding pocket defined by the structural coordinates of these amino acids, as set forth in Figures 1 and 2 is considered an TAK1-ATP binding pocket of this invention.

In another embodiment, the ATP-binding pocket comprises amino acids I36, V38, E39, E40, A46, G48, V50, C51, K52, A53, K54, WW55, D59, V60, A61, I62, K63, Q64, L78, R79, Q80, H86, N88, I89, V90, L97, L92, Y93, G94, A95, C96, V100, C101, L102, V103, M104, E105, Y106, A107, E108, G109, S111, L112, Y113, N114, V115, H129, A130, M131, S132, W133, C134, L135, Q136, C137, S138, Q139, G140, V141, A142, Y143, L144, H145, S146, M147, A151, L152, I153, H154, R155, D156, L157, K158, P159, P160, N161, L162, L163, L164, V165, A166, T169, V170, L171, K172, I173, C174 and D175 using the structure of the TAK1 - adenosine complex to Figure 1. In another embodiment, the ATP-binding pocket comprises amino acids I36, V38, E39, E40, A46, G48, V50, C51, K52, A53, K54, WW55, D59, V60, A61, 162, K63, Q64, L78, R79, Q80, H86, N88, I89, V90, L97, L92, Y93, G94, A95, C96, V100, C101, L102, V103, M104, E105, Y106, A107, E108, G109, S111, L112, Y113, N114, V115, H129, A130, M131, S132, W133, C134, L135, Q136, C137, S138, Q139, G140, V141, A142, Y143, L144, H145, S146, M147, A151, L152, I153, H154, R155, D156, L157, K158, P159, P160, N161, L162, L163, L164, V165, A166, T169, V170, L171, K172, I173, C174 and D175 using the structure of the TAK1 - 3-[6-(4-Acetyl-3,5-dimethyl-piperazin-1-yl)-pyndin-2-yl]-1H-pyrrolo[2,3-b]pyridine-5-carboxylic acid methyl ester complex according to Figure 2. Using a multiple alignment program to compare each TAK1 structure and structures of other members of the protein kinase family [Gerstein et al., J. Mol. Biol., 251, pp. 161-175 (1995), applicants have identified the above amino acids as the ATP-binding pocket. First, a sequence alignment between members of the protein kinase family including Aurora-2 [PDB Accession number 1MUO], p38 [K. P. Wilson et al., J. Biol. Chem., 271, pp. 27696-27700 (1996); Z. Wang et al., Proc. Natl. Acad. Sci. U.S.A., 94, pp. 2327-32 (1997)], CDK2 [PDB Accession number 1B38], SRC [Xu, W., et al., Cell 3, pp. 629-638 (1999); PDB Accession number 2SRC], MK2 [United States Provisional application 60/337,513] and LCK [Yamaguchi H., Hendrickson W.A., Nature. 384, pp. 484-489 (1996); PDB Accession number 3LCK] is performed. Then, a putative core is constructed by superimposing a series of corresponding structures in the protein kinase family. Then, residues of high spatial variation are discarded, and the core alignment is iteratively refined. The amino acids that make up the final core structure have low structural variance and have the same local and global conformation relative to the corresponding residues in the protein family.

In one embodiment, the ATP-binding pocket comprises the amino acids of V42, V90, M104, E105, A107 and L163 according to Figures 1 and 2. It will be readily apparent to those of skill in the art that the numbering of amino acids in other homologues of TAK1 may be different than that set forth for TAK1. Corresponding amino acids in homologues of TAK1 are easily identified by visual inspection of the amino acid sequences or by using commercially available sequence homology, structural homology or structure superimposition software programs.

This invention also provides a novel TAB1 binding pocket on the kinase domain. This binding pocket is located on the bottom of the C-lobe and not near the ATP binding pocket. This TAB1-binding pocket will be useful for drug design.

Accordingly, another embodiment of this invention provides a TAK1 TAB1-binding pocket defined by structure coordinates of TAK1 amino acids M131, P256, Y290 and F291 according to Figure 1; or a TAK1-like TAB1-binding pocket defined by structure coordinates of corresponding amino acids that are identical to said TAK1 amino acids, wherein the root mean square deviation of the backbone atoms between said corresponding amino acids and said TAK1 amino acids is not more than about 3.0 Å, 2.5 Å, 2.0 Å, 1.5 Å, or 1.0 Å; or a TAK1-like TAB1-binding pocket defined by structure coordinates of a set of corresponding amino acids, wherein the root mean square deviation of the backbone atoms between said set of corresponding amino acids and said TAK1 amino acids is not more than about 1.1 Å, 0.9 Å, 0.7 Å, or 0.5 Å and wherein at least one of said corresponding amino acids is not identical to the TAK1 amino acid to which it corresponds.

Another embodiment of this invention provides a TAK1 TAB 1-binding pocket defined by structure coordinates of TAK1 amino acids A127, M131, P256, I259, Y290 and F291 according to Figure 1; or a TAK1-like TAB1-binding pocket defined by structure coordinates of corresponding amino acids that are identical to said TAK1 amino acids, wherein the root mean square deviation of the backbone atoms between said corresponding amino acids and said TAK1 amino acids is not more than about 3.0 Å, 2.5 Å, 2.0 Å, 1.5 Å, or 1.0 Å; or a TAK1-like TAB 1-binding pocket defined by structure coordinates of a set of corresponding amino acids, wherein the root mean square deviation of the backbone atoms between said set of corresponding amino acids and said TAK1 amino acids is not more than about 1.0 Å, 0.8 Å, or 0.6 Å, and wherein at least one of said corresponding amino acids is not identical to the TAK1 amino acid to which it corresponds.

Another embodiment of this invention provides a TAK1 TAB1-binding pocket defined by structure coordinates of TAK1 amino acids M131, P256, Y290 and F291 according to Figure 2; or a TAK1-like TAB 1-binding pocket defined by structure coordinates of corresponding amino acids that are identical to said TAK1 amino acids, wherein the root mean square deviation of the backbone atoms between said corresponding amino acids and said TAK1 amino acids is not more than about 3.0 Å, 2.5 Å, 2.0 Å, 1.5 Å, or 1.0 Å; or a TAK1-like TAB1-binding pocket defined by structure coordinates of a set of corresponding amino acids, wherein the root mean square deviation of the backbone atoms between said set of corresponding amino acids and said TAK1 amino acids is not more than about 1.1 Å, 0.9 Å, 0.7 Å, or 0.5 Å and wherein at least one of said corresponding amino acids is not identical to the TAK1 amino acid to which it corresponds.

Another embodiment of this invention provides a TAK1 TAB 1-binding pocket defined by structure coordinates of TAK1 amino acids A127, M131, P256, I259, Y290 and F291 according to Figure 2; or a TAK1-like TAB1-binding pocket defined by structure coordinates of corresponding amino acids that are identical to said TAK1 amino acids, wherein the root mean square deviation of the backbone atoms between said corresponding amino acids and said TAK1 amino acids is not more than about 3.0 Å, 2.5 Å, 2.0 Å, 1.5 Å, or 1.0 Å; or a TAK1-like TAB1-binding pocket defined by structure coordinates of a set of corresponding amino acids, wherein the root mean square deviation of the backbone atoms between said set of corresponding amino acids and said TAK1 amino acids is not more than about 1.0 Å, 0.8 Å, or 0.6 Å, and wherein at least one of said corresponding amino acids is not identical to the TAK1 amino acid to which it corresponds.

Applicants have also determined a unique re-orientation of the N-lobe relative to the C-lobe. This determination was crucial in solving the three-dimensional structure of TAK1.

Those of skill in the art understand that a set of structure coordinates for a molecule or a molecular-complex or a portion thereof, is a relative set of points that define a shape in three dimensions. Thus, it is possible that an entirely different set of coordinates could define a similar or identical shape. Moreover, slight variations in the individual coordinates will have little effect on overall shape. In terms of binding pockets, these variations would not be expected to significantly alter the nature of ligands that could associate with those pockets.

The variations in coordinates indicated above may be generated because of mathematical manipulations of the TAK1 structure coordinates. For example, the structure coordinates set forth in Figure 1 and 2 could be manipulated by crystallographic permutations of the structure coordinates, fractionalization of the structure coordinates, integer additions or subtractions to sets of the structure coordinates, inversion of the structure coordinates or any combination of the above.

Alternatively, modifications in the crystal structure due to mutations, additions, substitutions, and/or deletions of amino acids, or other changes in any of the components that make up the crystal could also account for variations in structure coordinates. If such variations are within a certain root mean square deviation as compared to the original coordinates, the resulting three-dimensional shape is considered encompassed by this invention. Thus, for example, a ligand that bound to the binding pocket of TAK1 would also be expected to bind to another binding pocket whose structure coordinates defined a shape that fell within the acceptable root mean square deviation.

Various computational analyses maybe necessary to determine whether a binding pocket, motif, domain or portion thereof of a molecule or molecular complex is sufficiently similar to the binding pocket, motif, domain or portion thereof of TAK1. Such analyses may be carried out in well known software applications, such as ProFit [A. C.R. Martin, SciTech Software, ProFit version 1.8, University College London, http://www.bioinf.org.uk/software], Swiss-Pdb Viewer [Guex et al., Electrophoresis, 18, pp. 2714-2723 (1997)], the Molecular Similarity application of QUANTA [Molecular Simulations Inc., San Diego, CA © 1998,2000] and as described in the accompanying User's Guide.

The above programs permit comparisons between different structures, different conformations of the same structure, and different parts of the same structure. The procedure used in QUANTA [Molecular Simulations, Inc., San Diego, CA ©1998,2000] and Swiss-Pdb Viewer to compare structures is divided into four steps: 1) load the structures to be compared; 2) define the atom equivalences in these structures; 3) perform a fitting operation on the structures; and 4) analyze the results. The procedure used in ProFit to compare structures includes the following steps: 1) load the structures to be compared; 2) specify selected residues of interest; 3) define the atom equivalence in the selected residues; 4) perform a fitting operation on the selected residues; and 5) analyze the results.

Each structure in the comparison is identified by a name. One structure is identified as the target (*i.e.*, the fixed structure); all remaining structures are working structures (*i.e.*, moving structures). Since atom equivalency within the above programs is defined by user input, for the purpose of this invention we will define equivalent atoms as protein backbone atoms (N, Cα, C and O) for TAK1 amino acids and corresponding amino acids in the structures being compared.

The corresponding amino acids may be identified by sequence alignment programs such as the "bestfit" program available from the Genetics Computer Group which uses the local homology algorithm described by Smith and Waterman in Advances in Applied Mathematics 2, 482 (1981).

A suitable amino acid sequence alignment will require that the proteins being aligned share minimum percentage of identical amino acids. Generally, a first protein being aligned with a second protein should share in excess of about 35% identical amino acids with the second protein [Hanks et al., Science, 241, 42 (1988); Hanks and Quinn, Meth. Enzymol., 200, 38 (1991)]. The identification of equivalent residues can also be assisted by secondary structure alignment, for example, aligning the a-helices, β-sheets in the structure. The program Swiss-Pdb Viewer has its own best fit algorithm that is based on secondary sequence alignment.

When a rigid fitting method is used, the working structure is translated and rotated to obtain an optimum fit with the target structure. The fitting operation uses an algorithm that computes the optimum translation and rotation to be applied to the moving structure, such that the root mean square difference of the fit over the specified pairs of equivalent atom is an absolute minimum. This number, given in angstroms, is reported by the above programs. The Swiss-Pdb Viewer program sets an RMSD cutoff for eliminating pairs of equivalent atoms that have high RMSD values. For programs that calculate an average of the individual RMSD values of the backbone atoms, an RMSD cutoff value can be used to exclude pairs of equivalent atoms with extreme individual RMSD values. In the program ProFit, the RMSD cutoff value can be specified by the user.

The RMSD values between other protein kinases the TAK1 protein complexes (Figures 1 and 2) and other kinases are illustrated in Table 1. The RMSD values were determined by the programs ProFit from initial rigid fitting results from QUANTA. The RMSD values provided in Table 1 are averages of individual RMSD values calculated for the backbone atoms in the kinase or ATP-binding pocket. The RMSD cutoff value in ProFit was specified as 3Å.

For the 5 Å and 8 Å sphere amino acids, the values for the RMSD values of the ATP-binding pocket between the TAK1 - 3-[6-(4-Acetyl-3,5-dimethyl-piperazin-1-yl)-pyridin-2-yl]-1H-pyrrolo[2,3-b]pyridine-5-carboxylic acid methyl ester inhibitor complex and the TAK1 - adenosine complex are 0.33 Å and 0.30 Å, respectively. The comparison of the whole kinase domain yields RMSD values of 0.11 Å using the TAK1 - 3-[6-(4-Acetyl-3,5-dimethyl-piperazin-1-yl)-pyridin-2-yl]-1H-pyrrolo[2,3-b]pyridine-5-carboxylic acid methyl ester inhibitor complex as a reference.

TABLE 1: RMSD values for TAK1 - 3-[6-(4-Acetyl-3,5-dimethyl-piperazin-1-yl)-pyridin-2-yl]-1H-pyrrolo[2,3-b] pyridine-5-carboxylic acid methyl ester inhibitor complex

| Protein | RMSD value between ATP-binding pocket (8 Å sphere of amino acids) and corresponding amino acids in protein (Å) | RMSD value between ATP-binding pocket (5 Å sphere of amino acids)and corresponding amino acids in protein (Å) | RMSD value between TAK-complex kinase domain and kinase domain in protein (Å) |
|---|---|---|---|
| Aur-2[a] | 1.87 | 2.11 | 2.36 |
| P38[b] | 3.63 | 1.50 | 8.56 |
| FLT-3[c] | 2.11 | 0.92 | 2.53 |
| ITK[d] | 1.54 | 0.69 | 2.55 |
| MK2[e] | 1.15 | 1.25 | 8.84 |
| LCK[f] | 2.07 | 1.27 | 1.86 |
| Adenosine[g] | 0.30 | 0.33 | 0.11 |
| a. Aurora-2 kinase: United States Provisional application VPI/01-12. b. p38: Wilson et al., J. Biol. Chem., 271, pp. 27696-27700 (1996); Z. Wang et al., Proc. Natl. Acad. Sci. U.S.A., 94, pp. 2327-2332 (1997); PDB Accession number 1WFC. c. FMS-like Tyrosine Kinase 3 (FLT3): Griffith et al., et al., Mol. Cell. 13, pp. 169 (2004); PDB Accession number 1RJB. d. Interleukin-2 Tyrosine Kinase: Brown et al. , J. Biol. Chem. 279, pp. 18727 (2004); PDB Accession number 1SM2. e. Mitogen activated protein kinase activated protein (MAPKAP) kinase 2: United States Provisional application 60/337,513. f. Lymphocyte-specific kinase (LCK):ref Yamaguchi H., Hendrickson W.A., Nature. 384, pp. 484-489 (1996); PDB Accession number 3LCK. g. TAK-1/TAB-1 Adenosine complex (this application). | | | |

For the purpose of this invention, any molecule, molecular complex, binding pocket, motif, domain thereof or portion thereof that is within a root mean square deviation for backbone atoms (N, Cα, C, O) when superimposed on the relevant backbone atoms described by structure coordinates listed in Figure 1 and 2 are encompassed by this invention.

Therefore, one embodiment of this invention provides a molecule or molecular complex comprising all or part of a TAK1 ATP-binding pocket as defined herein. Another embodiment of this invention provides a molecule or molecular complex comprising all or part of a TAK1 TAB 1-binding pocket defined.

In one embodiment, the above molecules or molecular complexes are in crystalline form.

<u>Computer Systems</u> (not encompassed in the present invention)

As would be recognized, this invention is ideally suited for use in computer-implemented methods. Accordingly, this invention discloses a computer system comprising one or more of a) atomic coordinate data as disclosed herein +/- a root mean square deviation from the Ca atoms of not more than 1.5 Å (or 1.0 Å or 0.5 Å); b) structure factor data (where a structure factor comprises the amplitude and phase of the diffracted wave) for TAK1, said structure factor data being derivable from the atomic coordinate data of Table 1 ±a root mean square deviation from the Ca atoms of not more than 1.5 Å (or 1.0 Å or 0.5 Å); c) atomic coordinate data of a target TAK1 protein generated by homology modeling of the target based on the data disclosed herein ± a root mean square deviation from the Ca atoms of not more than 1.5 Å (or 1.0 Å or 0.5 Å); d ) atomic coordinate data of a target TAK1 protein generated by interpreting X-ray crystallographic data or NMR data by reference to the data disclosed herein ±a root mean square deviation from the Ca atoms of not more than 1.5 Å (or 1.0 Å or 0.5 Å); or (e) structure factor data a derivable from the atomic coordinate data of (c) or (d). In certain embodiments, a computer system comprises: a computer-readable data storage medium comprising data storage material encoded with the computer -readable data; (a) a working memory for storing instructions for processing said computer -readable data; and (b) a central-processing unit coupled to said working memory and to said computer -readable data storage medium for processing said computer - readable data and thereby generating structures and/or performing rational drug design

Additionally a machine-readable data storage medium is disclosed, comprising a data storage material encoded with machine-readable data, wherein said data comprises all or part of an TAK1 ATP-binding pocket defined by structure coordinates of TAK1 amino acids V42, G43, V50, A61, K63, V90, M104, E105, A107, E108, G109, S111, N114, L163 and C 174, according to Figure 1; or a molecule or molecular complex comprising all or part of an TAK1-like ATP-binding pocket defined by structure coordinates of corresponding amino acids that are identical to said TAK1 amino acids, wherein the root mean square deviation of the backbone atoms between said corresponding amino acids and said TAK1 amino acids is not more than about 3.0 Å, 2.5 Å, 2.0 Å, 1.5 Å, or 1.0; or a molecule or molecular complex comprising all or part of an TAK1-like ATP-binding pocket defined by structure coordinates of a set of corresponding amino acids, wherein the root mean square deviation of the backbone atoms between said set of corresponding amino acids and said TAK1 amino acids is not more than about 1.1, 0.9, 0.7 or 0.5 Å, and wherein at least one of said corresponding amino acids is not identical to the TAK1 amino acid to which it corresponds.

Additionally a machine-readable data storage medium is disclosed, comprising a data storage material encoded with machine-readable data, wherein said data comprises all or part of any molecule or molecular complex disclosed herein. Alternatively, the data storage material is encoded with machine-readable data comprising all or part of a binding pocket of this invention or a TAK1 according to this invention.

Additionally a computer is disclosed comprising:

a machine-readable data storage medium, comprising a data storage material encoded with machine-readable data, wherein said data comprises all or part of a TAK1 ATP-binding pocket defined by structure coordinates of TAK1 amino acids V42, G43, V50, A61, K63, V90, M104, E105, A107, E108, G109, S111, N114, L163 and C174, according to Figure 1; or a molecule or molecular complex comprising all or part of a TAK1-like ATP-binding pocket defined by structure coordinates of corresponding amino acids that are identical to said TAK1 amino acids, wherein the root mean square deviation of the backbone atoms between said corresponding amino acids and said TAK1 amino acids is not more than about 3.0 Å, 2.5 Å, 2.0 Å, 1.5 Å, or 1.0 Å; or a molecule or molecular complex comprising all or part of a TAK1-like ATP-binding pocket defined by structure coordinates of a set of corresponding amino acids, wherein the root mean square deviation of the backbone atoms between said set of corresponding amino acids and said TAK1 amino acids is not more than about 0.5 Å, and wherein at least one of said corresponding amino acids is not identical to the TAK1 amino acid to which it corresponds.

Additionally a machine-readable data storage medium is disclosed, comprising a data storage material encoded with machine-readable data, wherein said data comprises all or part of a TAB1-binding pocket defined by structure coordinates of TAK1 amino acids A127, M131, P256, I259, Y290 and F291 according to Figure 1; or a molecule or molecular complex comprising all or part of a TAK1-like TAB1-binding pocket defined by structure coordinates of corresponding amino acids that are identical to said TAK1 amino acids, wherein the root mean square deviation of the backbone atoms between said corresponding amino acids and said TAK1 amino acids is not more than about 3.0 Å, 2.5 Å, 2.0 Å, 1.5 Å, or 1.0 Å; or a molecule or molecular complex comprising all or part of a TAK1-like ATP-binding pocket defined by structure coordinates of a set of corresponding amino acids, wherein the root mean square deviation of the backbone atoms between said set of corresponding amino acids and said TAK1 amino acids is not more than about 1.1, 0.9, 0.7 or 0.5 Å, and wherein at least one of said corresponding amino acids is not identical to the TAK1 amino acid to which it corresponds.

Additionally a machine-readable data storage medium is disclosed, comprising a data storage material encoded with machine-readable data, wherein said data comprises all or part of any molecule or molecular complex disclosed in the above paragraphs.

Additionally a computer is disclosed comprising:

a machine-readable data storage medium, comprising a data storage material encoded with machine-readable data, wherein said data comprises all or part of a TAB1-binding pocket defined by structure coordinates of TAK1 amino acids A127, M131, P256, I259, Y290 and F291, according to Figure 1; or a molecule or molecular complex comprising all or part of a TAK1-like TAB1-binding pocket defined by structure coordinates of corresponding amino acids that are identical to said TAK1 amino acids, wherein the root mean square deviation of the backbone atoms between said corresponding amino acids and said TAK1 amino acids is not more than about 3.0 Å, 2.5 Å, 2.0 Å, 1.5 Å, or 1.0 Å; or a molecule or molecular complex comprising all or part of a TAK1-like ATP-binding pocket defined by structure coordinates of a set of corresponding amino acids, wherein the root mean square deviation of the backbone atoms between said set of corresponding amino acids and said TAK1 amino acids is not more than about 0.5 Å, and wherein at least one of said corresponding amino acids is not identical to the TAK1 amino acid to which it corresponds.

Additionally a computer is disclosed comprising:

a machine-readable data storage medium, comprising a data storage material encoded with machine-readable data, wherein said data comprises all or part of any molecule or molecular complex disclosed in the above paragraphs.

Additionally a computer as described above comprises a working memory for storing instructions for processing the machine-readable data, a central-processing unit coupled to the working memory and to said machine-readable data storage medium for processing said machine-readable data into the three-dimensional structure. The computer may further comprise a display for displaying the three-dimensional structure as a graphical representation. The computer may further comprise commercially available software program to display the graphical representation. Examples of software programs include but are not limited to QUANTA [Molecular Simulations, Inc., San Diego, CA ©1998,2000], O [Jones et al., Acta Cryst. A, 47, pp. 110-119 (1991)] and RIBBONS [M. Carson, J. Appl. Cryst., 24, pp. 958-961 (1991)].

Additionally a computer is disclosed comprising:

a) a machine-readable data storage medium comprising a data storage material encoded with machine-readable data, wherein the data defines any one of the above binding pockets or protein of the molecule or molecular complex;

b) a working memory for storing instructions for processing said machine-readable data;

c) a central processing unit (CPU) coupled to the working memory and to the machine-readable data storage medium for processing said machine readable data as well as an instruction or set of instructions for generating three-dimensional structural information of said binding pocket or protein; and

d) output hardware coupled to the CPU for outputting three-dimensional structural information of the binding pocket or protein, or information produced by using the three-dimensional structural information of said binding pocket or protein. The output hardware may include monitors, touchscreens, printers, facsimile machines, modems, disk drives, CD-ROMs, etc.

In the above embodiment, the outputting involves three-dimensional structural information. A computer may also be adapted to output other information or results. For example, a list of test compounds or potential inhibitor compounds may be outputted.

Three-dimensional data generation may be provided by an instruction or set of instructions such as a computer program or commands for generating a three-dimensional structure or graphical representation from structure coordinates, or by subtracting distances between atoms, calculating chemical energies for a TAK1 molecule or molecular complex or homologues thereof, or calculating or minimizing energies for an association of a TAK1 molecule or molecular complex or homologues thereof to a chemical entity. The graphical representation can be generated or displayed by commercially available software programs. Examples of software programs include but are not limited to QUANTA [Accelrys ©2001, 2002], O [Jones et al., Acta Crystallogr. A47, pp. 110-119 (1991)] and RIBBONS [Carson, J. Appl. Crystallogr., 24, pp. 9589-961 (1991)].

Certain software programs may imbue this representation with physico-chemical attributes which are known from the chemical composition of the molecule, such as residue charge, hydrophobicity, torsional and rotational degrees of freedom for the residue or segment, etc. Examples of software programs for calculating chemical energies are described in the Rational Drug Design section.

Information about said binding pocket or information produced by using said binding pocket can be outputted through display terminals, touchscreens, printers, modems, facsimile machines, CD-ROMs or disk drives. The information can be in graphical or alphanumeric form.

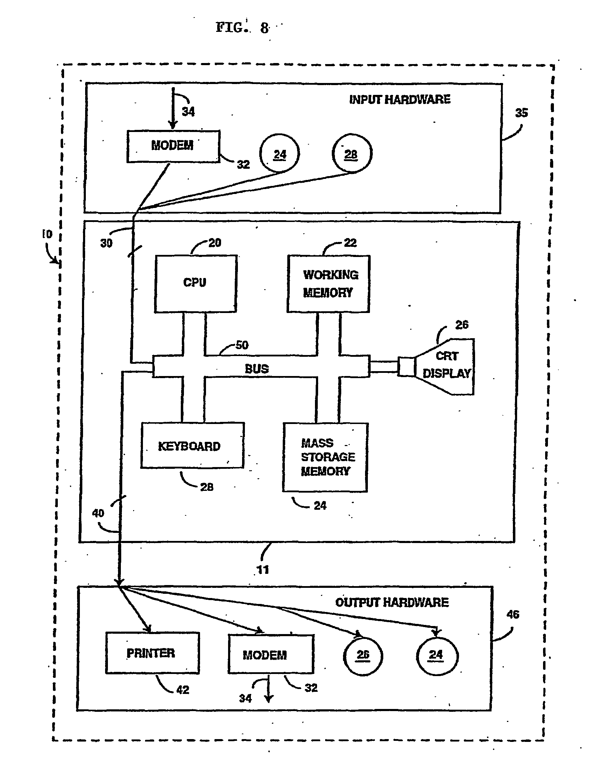

Figure 8 demonstrates one version of these embodiments. System 10 includes a computer 11 comprising a central processing unit ("CPU") 20, a working memory 22 which may be, *e.g.*, RAM (random-access memory) or "core" memory, mass storage memory 24 (such as one or more disk drives or CD-ROM drives), one or more cathode-ray tube ("CRT") display terminals 26, one or more keyboards 28, one or more input lines 30, and one or more output lines 40, all of which are interconnected by a conventional bi-directional system bus 50.

Input hardware 36, coupled to computer 11 by input lines 30, may be implemented in a variety of ways. Machine-readable data of this invention may be inputted via the use of a modem or modems 32 connected by a telephone line or dedicated data line 34. Alternatively or additionally, the input hardware 36 may comprise CD-ROM drives or disk drives 24. In conjunction with display terminal 26, keyboard 28 may also be used as an input device.

Output hardware 46, coupled to computer 11 by output lines 40, may similarly be implemented by conventional devices. By way of example, output hardware 46 may include CRT display terminal 26 for displaying a graphical representation of a binding pocket of this invention using a program such as QUANTA [Molecular Simulations, Inc., San Diego, CA ©1998,2000] as described herein. Output hardware might also include a printer 42, so that hard copy output may be produced, or a disk drive 24, to store system output for later use. Output hardware may also include a display terminal, a CD or DVD recorder, ZIP™ or JAZ™ drive, or other machine-readable data storage device.

In operation, CPU 20 coordinates the use of the various input and output devices 36, 46, coordinates data accesses from mass storage 24 and accesses to and from working memory 22, and determines the sequence of data processing steps. A number of programs may be used to process the machine-readable data of this invention. Such programs are addressed in reference to the computational methods of drug discovery as described herein. Specific references to components of the hardware system 10 are included as appropriate throughout the following description of the data storage medium.

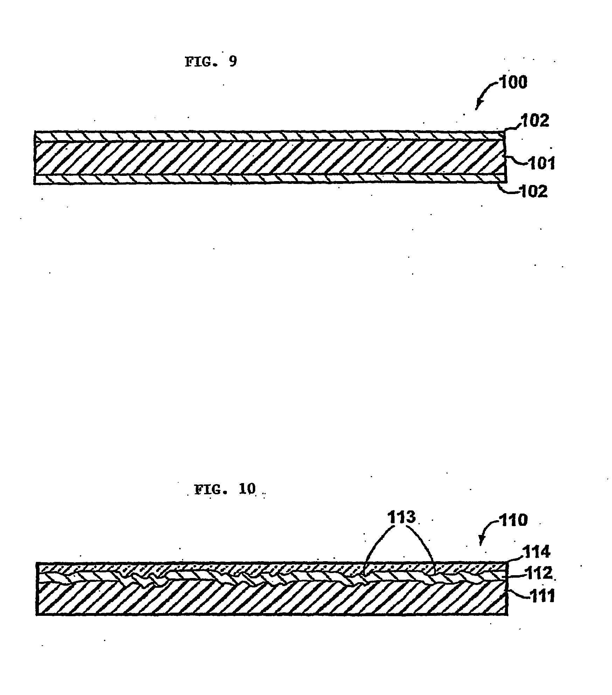

Figure 9 shows a cross section of a magnetic data storage medium 100 which can be encoded with a machine-readable data that can be carried out by a system such as system 10 of Figure 10. Medium 100 can be a conventional floppy diskette or hard disk, having a suitable substrate 101, which may be conventional, and a suitable coating 102, which may be conventional, on one or both sides, containing magnetic domains (not visible) whose polarity or orientation can be altered magnetically. Medium 100 may also have an opening (not shown) for receiving the spindle of a disk drive or other data storage device 24.

The magnetic domains of coating 102 of medium 100 are polarized or oriented so as to encode in a manner that may be conventional, machine readable data such as that described herein, for execution by a system such as system 10 of Figure 8.

Figure 10 shows a cross section of an optically-readable data storage medium 110 which also can be encoded with such a machine-readable data, or set of instructions, which can be carried out by a system such as system 10 of Figure 7. Medium 110 can be a conventional compact disk read only memory (CD-ROM) or a rewritable medium such as a magneto-optical disk that is optically readable and magneto-optically writable. Medium 100 preferably has a suitable substrate 111, which may be conventional, and a suitable coating 112, which may be conventional, usually of one side of substrate 111.

In the case of CD-ROM, as is well known, coating 112 is reflective and is impressed with a plurality of pits 113 to encode the machine-readable data. The arrangement of pits is read by reflecting laser light off the surface of coating 112. A protective coating 114, which preferably is substantially transparent, is provided on top of coating 112.

In the case of a magneto-optical disk, as is well known, coating 112 has no pits 113, but has a plurality of magnetic domains whose polarity or orientation can be changed magnetically when heated above a certain temperature, as by a laser (not shown). The orientation of the domains can be read by measuring the polarization of laser light reflected from coating 112. The arrangement of the domains encodes the data as described above.

In one embodiment, the data defines the above-mentioned binding pockets by comprising the structure coordinates of said amino acid residues according to Figure 1, 2 or 3.

To use the structure coordinates generated for TAK1 or TAK1 homologue, one of its binding pockets, motifs, domains, or portion thereof, it is at times necessary to convert them into a three-dimensional shape or to generate three-dimensional structural information from them. This is achieved through the use of commercially or publicly available software that is capable of generating a three-dimensional structure of molecules or portions thereof from a set of structure coordinates. In one embodiment, the three-dimensional structure may be displayed as a graphical representation.

Therefore, according to another embodiment, is disclosed a machine-readable data storage medium comprising a data storage material encoded with machine readable data. In one embodiment, a machine programmed with instructions for using said data, is capable of generating a three-dimensional structure of any of the molecule or molecular complexes, or binding pockets thereof, that are described herein.

Additionally a computer is disclosed for producing a three-dimensional structure of:

a) a molecule or molecular complex

comprising all or part of a TAK1 ATP-binding pocket defined by structure coordinates of TAK1 amino acids V377, A389, V419, F435, E436, F437, M438, C442, L489 and S499, according to Figure 1;

b) a molecule or molecular complex

comprising all or part of a TAK1-like ATP-binding pocket defined by structure coordinates of corresponding amino acids that are identical to said TAK1 amino acids, wherein the root mean square deviation of the backbone atoms between said corresponding amino acids and said TAK1 amino acids is not more than about 3.0 Å, 2.5 Å, 2.0 Å, 1.5 Å or 1.0 Å; or 0.5 Å; and/or

c) a molecule or molecular complex

comprising all or part of a TAK1-like ATP-binding pocket defined by structure coordinates of a set of corresponding amino acids, wherein the root mean square deviation of the backbone atoms between said set of corresponding amino acids and said TAK1 amino acids is not more than about 0.6 Å, 0.5 Å or 0.4 Å, and wherein at least one of said corresponding amino acids is not identical to the TAK1 amino acid to which it corresponds, comprising:

i) a machine-readable data storage medium, comprising a data storage material encoded with machine-readable data, wherein said data comprises all or part of a TAK1 ATP-binding pocket defined by structure coordinates of TAK1 amino acids V377, A389, V419, F435, E436, F437, M438, C442, L489 and S499, according to Figure 1; all or part of a TAK1-like ATP-binding pocket defined by structure coordinates of corresponding amino acids that are identical to said TAK1 amino acids, wherein the root mean square deviation of the backbone atoms between said corresponding amino acids and said TAK1 amino acids is not more than about 3.0 Å, 2.5 Å, 2.0 Å 1.5 Å or 1.0 Å; or all or part of a TAK1-like ATP-binding pocket defined by structure coordinates of a set of corresponding amino acids, wherein the root mean square deviation of the backbone atoms between said set of corresponding amino acids and said TAK1 amino acids is not more than about 0.6 Å, 0.5 Å or 0.4 Å, and wherein at least one of said corresponding amino acids is not identical to the TAK1 amino acid to which it corresponds; and

ii) instructions for processing said machine-readable data into said three-dimensional structure.

Additionally, the computer is also for producing the three-dimensional structure of the aforementioned molecules and molecular complexes and comprises the corresponding machine-readable data storage mediums. In one embodiment, the three-dimensional structure is displayed as a graphical representation.

In one embodiment, the structure coordinates of said molecules or molecular complexes are produced by homology modeling of at least a portion of the structure coordinates of Figure 1 or 2. Homology modeling can be used to generate structural models of TAK1 homologues or other homologous proteins based on the known structure of TAK1. This can be achieved by performing one or more of the following steps: performing sequence alignment between the amino acid sequence of an unknown molecule against the amino acid sequence of TAK1; identifying conserved and variable regions by sequence or structure; generating structure co-ordinates for structurally conserved residues of the unknown structure from those of TAK1; generating conformations for the structurally variable residues in the unknown structure; replacing the non-conserved residues of TAK1 with residues in the unknown structure; building side chain conformations; and refining and/or evaluating the unknown structure.

For example, since the protein sequence of the catalytic domains of TAK1 and homologues thereof can be aligned relative to each other, it is possible to construct models of the structures of TAK1 homologues, particularly in the regions of the active site, using the TAK1 structure. Software programs that are useful in homology modeling include MALIGN [Wishart, D. S. et al., Comput Appl. Biosci., 10, pp. 687-88 (1994)] and CLUSTAL W Alignment Tool [Higgins D. G. et al., Methods Enzymol, 266, pp. 383-402 (1996)]. See also, U.S. Patent No. 5,884,230.

To perform the sequence alignment, programs such as the "bestfit" program available from the Genetics Computer Group [Waterman in Advances in Applied Mathematics 2, 482 (1981), which is incorporated herein by reference] and CLUSTAL W Alignment Tool [Higgins D. G. et al., Methods Enzymol, 266, pp. 383-402 (1996), can be used. To model the amino acid side chains of homologous TAK1 proteins, the amino acid residues in TAK1 can be replaced, using a computer graphics program such as "O" [Jones et al, (1991) Acta Cryst, Sect. A, 47: 110-119], by those of the homologous protein, where they differ. The same orientation or a different orientation of the amino acid can be used. Insertions and deletions of amino acid residues may be necessary where gaps occur in the sequence alignment.

Homology modeling can be performed using, for example, the computer programs SWISS-MODEL available through Glaxo Wellcome Experimental Research in Geneva, Switzerland; WHATIF available on EMBL servers; Schnare et al., J. Mol. Biol, 256: 701-719 (1996); Blundell et al., Nature 326: 347-352 (1987); Fetrow and Bryant, Bio/Technology 11:479-484 (1993); Greer, Methods in Enzymology 202: 239-252 (1991); and Johnson et al, Crit. Rev. Biochem: Mol Biol. 29:1-68 (1994). An example of homology modeling can be found, for example, in Szklarz G.D., Life Sci. 61: 2507-2520 (1997).

Thus, in accordance with the present invention, data capable of generating the three dimensional structure of the above molecules or molecular complexes (e.g., TAK1, homologues and portions thereof), or binding pockets thereof, can be stored in a machine-readable storage medium, which is capable of displaying three-dimensional structural information or a graphical three-dimensional representation of the structure.

Rational Drug Design

The TAK1 structure coordinates or the three-dimensional graphical representation generated from these coordinates may be used in conjunction with a computer for a variety of purposes, including drug discovery. Accordingly, this invention allows for a method for structure based drug design comprising the applying the coordinates disclosed herein to identify TAK1 inhibitors. In certain embodiments, the computer is programmed with software to translate those coordinates into the three-dimensional structure of TAK1.

For example, the structure encoded by the data may be computationally evaluated for its ability to associate with chemical entities. Chemical entities that associate with TAK1 may inhibit or activate TAK1 or its homologues, and are potential drug candidates. Alternatively, the structure encoded by the data may be displayed in a graphical three-dimensional representation on a computer screen. This allows visual inspection of the structure, as well as visual inspection of the structure's association with chemical entities.

Thus, according to another embodiment, the invention discloses a method for designing, selecting and/or optimizing a chemical entity that binds to the molecule or molecular complex comprising the steps of:

(a) providing the structure coordinates of said molecule or molecular compiex on a computer comprising the means for generating three-dimensional structural information from said structure coordinates; and

(b) designing, selecting and/or optimizing said chemical entity by employing means for performing a fitting operation between said chemical entity and said three-dimensional structural information of said molecule or molecular complex.

Three-dimensional structural information in step (a) may be generated by instructions such as a computer program or commands that can generate a three-dimensional structure or graphical representation; subtract distances between atoms; calculate chemical energies for a TAK1 molecule, molecular complex or homologues thereof; or calculate or minimize energies of an association of TAK1 molecule, molecular complex or homologues thereof to a chemical entity. These types of computer programs are known in the art. The graphical representation can be generated or displayed by commercially available software programs. Examples of software programs include but are not limited to QUANTA [Accelrys ©2001, 2002], O [Jones et al., Acta Crystallogr. A47, pp. 110-119 (1991)] and RIBBONS [Carson, J. Appl. Crystallogr., 24, pp. 9589-961 (1991)].

Certain software programs may imbue this representation with physico-chemical attributes which are known from the chemical composition of the molecule, such as residue charge, hydrophobicity, torsional and rotational degrees of freedom for the residue or segment, etc. Examples of software programs for calculating chemical energies are described below.

Another embodiment of the invention discloses method for evaluating the potential of a chemical entity to associate with the molecule or molecular complex as described previously. This evaluating may be for the purposes of optimizing or minimizing associating with a TAK1 protein.

This method comprises the steps of: a) employing computational means to perform a fitting operation between the chemical entity and the molecule or molecular complex described before; b) analyzing the results of said fitting operation to quantify the association between the chemical entity and the molecule or molecular complex; and, optionally, c) outputting said quantified association to a suitable output hardware, such as a CRT display terminal, a printer, a CD or DVD recorder, ZIP™ or JAZ™ drive, a disk drive, or other machine-readable data storage device, as described previously. The method may further comprise generating a three-dimensional structure, graphical representation thereof, or both, of the molecule or molecular complex prior to step a). In one embodiment, the method is for evaluating the ability of a chemical entity to associate with the binding pocket of a molecule or molecular complex.

The method comprises the steps of:

a) constructing a computer model of a binding pocket of the molecule or molecular complex;

b) selecting a chemical entity to be evaluated by a method selected from the group consisting of assembling said chemical entity; selecting a chemical entity from a small molecule database; de novo ligand design of said chemical entity; and modifying a known agonist or inhibitor, or a portion thereof, of a TAK1 protein or homologue thereof;

c) employing computational means to perform a fitting program operation between computer models of said chemical entity to be evaluated and said binding pocket in order to provide an energy-minimized configuration of said chemical entity in the binding pocket; and

d) evaluating the results of said fitting operation to quantify the association between said chemical entity and the binding pocket model, thereby evaluating the ability of said chemical entity to associate with said binding pocket.

In another embodiment, the invention discloses a method of using a computer for evaluating the ability of a chemical entity to associate with the molecule or molecular complex, wherein said computer comprises a machine-readable data storage medium comprising a data storage material encoded with said structure coordinates defining said binding pocket and means for generating a three-dimensional graphical representation of the binding pocket, and wherein said method comprises the steps of:

(a) positioning a first chemical entity within all or part of said binding pocket using a graphical three-dimensional representation of the structure of the chemical entity and the binding pocket;
(b) performing a fitting operation between said chemical entity and said binding pocket by employing computational means;
(c) analyzing the results of said fitting operation to quantitate the association between said chemical entity and all or part of the binding pocket; and
(d) outputting said quantitated association to a suitable output hardware.

The above method may further comprise the steps of:

(e) repeating steps (a) through (d) with a second chemical entity; and
(f) selecting at least one of said first or second chemical entity that associates with said all or part of said binding pocket based on said quantitated association of said first or second chemical entity.

Alternatively, the structure coordinates of the TAK1 binding pockets may be utilized in a method for identifying an agonist or antagonist of a molecule comprising a binding pocket of TAK1. In certain embodiments, the method comprises steps of:

a) using a three-dimensional structure of the molecule or molecular complex to design, select or optimize a chemical entity;
b) contacting the chemical entity with the molecule or molecular complex;
c) monitoring the catalytic activity of the molecule or molecular complex; and
d) classifying the chemical entity as an agonist or antagonist based on the effect of the chemical entity on the catalytic activity of the molecule or molecular complex.

In one embodiment, step a) is performed using a graphical representation of the binding pocket or portion thereof of the molecule or molecular complex.
In one embodiment, the three-dimensional structure is displayed as a graphical representation.
In another embodiment, the method comprises the steps of:

a) constructing a computer model of a binding pocket of the molecule or molecular complex;
b) selecting a chemical entity to be evaluated by a method selected from the group consisting of assembling said chemical entity; selecting a chemical entity from a small molecule database; de novo ligand design of said chemical entity; and modifying a known agonist or inhibitor, or a portion thereof, of a TAK1 protein or homologue thereof;
c) employing computational means to perform a fitting program operation between computer models of said chemical entity to be evaluated and said binding pocket in order to provide an energy-minimized configuration of said chemical entity in the binding pocket; and
d) evaluating the results of said fitting operation to quantify the association between said chemical entity and the binding pocket model, thereby evaluating the ability of said chemical entity to associate with said binding pocket;
e) synthesizing said chemical entity; and
f) contacting said chemical entity with said molecule or molecular complex to determine the ability of said compound to activate or inhibit said molecule.

In another embodiment is provided a method of using a computer for selecting an orientation of a chemical entity that interacts favorably with a binding pocket or domain comprising amino acid residues selected from the group consisting of:

(i) a set of amino acid residues which are identical to TAK1 amino acid residues E105 and A107 as disclosed herein, wherein the root mean square deviation of the backbone atoms between the set of amino acid residues and the TAK1 amino acid residues which are identical is not greater than about 1.5 Å; said method comprising the steps of:

(a) providing the structure coordinates of said binding pocket, domain or complex thereof on a computer comprising means for generating three-dimensional structural information from said structure coordinates;

(b) employing computational means to dock a first chemical entity in all or part of the binding pocket or domain;

(c) quantifying the association between said chemical entity and all or part of the binding pocket or domain for different orientations of the chemical entity; and

(d) selecting the orientation of the chemical entity with the most favorable interaction based on said quantified association. This method optionally further comprises the step of generating a three-dimensional graphical representation of the binding pocket or domain prior to step (b). The method also optionally comprises. This method optionally employs energy minimization, molecular dynamics simulations, or rigid-body minimizations are performed simultaneously with or following step (b). This method optionally further comprises the steps of:

(e) repeating steps (b) through (d) with a second chemical entity; and (f) selecting at least one of said first or second chemical entity that interacts more favorably with said binding pocket or domain based on said quantified association of said first or second chemical entity.

In another embodiment, this invention discloses a method of using a computer for selecting an orientation of a chemical entity with a favorable shape complementarity in a binding pocket comprising amino acid residues selected from the group consisting of:

(i) a set of amino acid residues which are identical to TAK1 amino acid residues E105 and A107 as disclosed herein, wherein the root mean square deviation of the backbone atoms between the set of amino acid residues and the TAK1 amino acid residues which are identical is not greater than about 1.5 Å; said method comprising the steps of:

(a) providing the structure coordinates of said binding pocket and all or part of the ligand bound therein on a computer comprising the means for generating three-dimensional structural information from said structure coordinates;

(b) employing computational means to dock a first chemical entity in all or part of the binding pocket;

(c) quantitating the contact score of said chemical entity in different orientations in the binding pocket; and

(d) selecting an orientation with the highest contact score. This method optionally comprises the further step of generating a three-dimensional graphical representation of all or part of the binding pocket and all or part of the ligand bound therein prior to step (b)

This method optionally comprises the further steps of (e) repeating steps (b) through (d) with a second chemical entity; and (f) selecting at least one of said first or second chemical entity that has a higher contact score based on said quantitated contact score of said first or second chemical entity.

This invention also discloses a method of designing a compound or complex that interacts with a binding pocket or domain comprising amino acid residues selected from the group consisting of:

(i) a set of amino acid residues which are identical to TAK1 amino acid residues E105 and A107 as disclosed herein, wherein the root mean square deviation of the backbone atoms between the set of amino acid residues and the TAK1 amino acid residues which are identical is not greater than about 1.5 Å; comprising the steps of:

(a) providing the structure coordinates of said binding pocket or domain on a computer comprising the means for generating three-dimensional structural information from said structure coordinates;

(b) using the computer to dock a first chemical entity in part of the binding pocket or domain;

(c) docking at least a second chemical entity in another part of the binding pocket or domain;

(d) quantifying the association between the first or second chemical entity and part of the binding pocket or domain;

(e) repeating steps (b) to (d) with another first and second chemical entity;

(f) selecting a first and a second chemical entity based on said quantified association of both of said first and second chemical entity;

(g) optionally, visually inspecting the relationship of the selected first and second chemical entity to each other in relation to the binding pocket or domain on a computer screen using the three-dimensional graphical representation of the binding pocket or domain and said first and second chemical entity; and

(h) assembling the selected first and second chemical entity into a compound or complex that interacts with said binding pocket or domain by model building.

This invention also discloses a method of a method for identifying a candidate inhibitor that interacts with a binding site of a TAK1 protein or TAK1 kinase domain, or a homologue thereof, comprising the steps of:

(a) obtaining a crystal comprising a TAK1 protein or TAK1 kinase domain, or homologue thereof;

(b) obtaining the structure coordinates of amino acids of the crystal of step (a);

(c) generating a three-dimensional structure of the a TAK1 protein or TAK1 kinase domain or homologue thereof using the structure coordinates of the amino acids obtained in step (b) with a root mean square deviation from backbone atoms of said amino acids of not more than ± 3.0 Å;

(d) determining a binding site of the a TAK1 protein or TAK1 kinase domain or homologue thereof from said three-dimensional structure; and

(e) performing docking to identify the candidate inhibitor which interacts with said binding site. This method optionally further comprises the step of:

(f) contacting the identified candidate inhibitor with the a TAK1 protein or TAK1 kinase domain or homologue thereof in order to determine the effect of the inhibitor on catalytic activity. In certain embodiments, the binding site of the TAK1 kinase domain or homologue thereof determined in step (d) comprises the structure coordinates of E105 and A107 according to this invention, wherein the root mean square deviation from the backbone atoms of said amino acids is not more than ± 1.5 Å. In other embodiments, the binding site is any of the binding pockets defined herein.

Also disclosed is a method for identifying a candidate inhibitor that interacts with a binding site of a TAK1 kinase domain, or homologue thereof, comprising the step of determining a binding site from a three-dimensional structure of the TAK1 kinase domain or homologue thereof to design or identify the candidate inhibitor which interacts with said binding site.

Also disclosed is a method for identifying a candidate inhibitor of a molecule or molecular complex comprising a binding pocket or domain comprising amino acid residues selected from the group consisting of:

(i) a set of amino acid residues which are identical to TAK1 amino acid residues E105 and A107 as disclosed herein, wherein the root mean square deviation of the backbone atoms between said set of amino acid residues and said TAK1 amino acid residues which are identical is not greater than about 1.5 Å; and

(ii) a set of amino acid residues which are identical to TAK1 amino acid residues V42, V90, M104, E105, A107, and as disclosed herein, wherein the root mean square deviation of the backbone atoms between said set of amino acid residues and said TAK1 amino acid residues which are identical is not greater than about 1.5 Å; comprising the steps of:

(a) using a three-dimensional structure of all or part of the binding pocket or domain to design, select or optimize a plurality of chemical entities; and

(b) selecting said candidate inhibitor based on the inhibitory effect of said chemical entities on the catalytic activity of the molecule or molecular complex.

Also disclosed is a method of using a crystal according to any one of the embodiments herein in an inhibitor screening assay comprising:

(a) selecting a potential inhibitor by performing rational drug design with a three-dimensional structure determined for the crystal, wherein said selecting is performed in conjunction with computer modeling;

(b) contacting the potential inhibitor with a kinase; and

(c) detecting the ability of the potential inhibitor to inhibit the kinase.

Also disclosed is a method for identifying a potential inhibitor of a kinase comprising:

a) selecting or designing a potential inhibitor by performing rational drug design with a computer readable data storage material encoded with computer readable data comprising structure coordinates disclosed herein, wherein said selecting is performed in conjunction with computer modeling;

b) contacting the potential inhibitor with a kinase; and

c) detecting the ability of the potential inhibitor for inhibiting the kinase.

Also disclosed is a method for performing iterative drug design comprising crystallizing a TAK1 protein according to the method disclosed herein.

Also disclosed is a method for identifying an agent that interacts with an active site of a TAK1 (including a homologue or complex thereof), comprising the steps of:

a) obtaining a crystallized complex comprising TAK1;

b) obtaining the structural coordinates of amino acids of the crystallized complex of step a), wherein the structural coordinates are set forth herein;

c) generating a three dimensional model of TAK1 using the structural coordinates of the amino acids generated in step b) +/- a root mean square deviation from the backbone atoms of said amino acids of not more than 1.5Å;

d) determining an active site of the TAK1 from the three dimensional model; and

e) performing computer fitting analysis to identify an agent which interacts with the active sits.

In another embodiment, this invention discloses a method of identifying a TAK1 binding compound or a TAK1 inhibitor, comprising the step of using a three-dimensional structural representation of TAK1 or a fragment thereof comprising a TAK1 ATP-binding site or a TAK1 TAB1-binding site, to computationally screen a candidate compound for an ability to bind the TAK1 ATP-binding site or a TAK1 TAB1-binding site, respectively.

In another embodiment, this invention discloses a method of identifying a TAK1 binding compound or a TAK1 inhibitor comprising the step of using a three-dimensional structural representation of TAK1, or a fragment thereof comprising a TAK1 ATP-binding site or a TAK1 TAB1-binding site, to computationally design a synthesizable candidate compound that binds or inhibits TAK1.

Any methods of this invention optionally comprise the steps of: synthesizing or otherwise obtaining the candidate compound; and testing the candidate compound for TAK1 binding activity or inhibitory activity.

Any methods of this invention optionally comprise a step of computationally design a binding compound comprises the steps of: identifying chemical entities or fragments capable of associating with the TAK1 ATP-binding site or a TAK1 TAB1-binding site; and assembling the chemical entities or fragments into a single molecule to provide the structure of the candidate compound.

In a specific embodiment, this invention provides a method of identifying a TAK1 binding compound or a TAK1 inhibitor, comprising the steps of (a) using a three-dimensional structural representation of TAK1, or a fragment thereof comprising a TAK1 ATP-binding site or a TAK1 TAB1-binding site, to computationally screen a candidate compound for an ability to bind the TAK1 ATP-binding site or a TAK1 TAB1-binding site, (b) synthesizing the candidate compound; and (c) screening the candidate compound for TAK1 binding activity or TAK1 inhibitory activity, wherein the structural information comprises the atomic structure coordinates of residues comprising a TAK1 ATP-binding site or a TAK1 TAB1-binding site as claimed in claim 15.

The three-dimensional structural representation comprises the three-dimensional structure defined by atomic structure coordinates according to FIG. 1 or FIG. 2

A method for designing an agent that I-nteracts with TAK1 is disclosed, comprising: providing a composition including TAK1; generating a three dimensional model of TAK1; and utilizing the three dimensional model to design an agent that interacts with TAK1, wherein the three dimensional model of TAK-1 includes relative structural coordinates of a plurality of atoms of TAK1, and the relative structural coordinates are selected according to: FIG. 1, $\pm$ a root mean square deviation from the backbone atoms of amino acids of not more than 1.5 Å; or FIG. 2, $\pm$ a root mean square deviation from the backbone atoms of amino acids of not more than 1.5 Å. The three dimensional model optionally includes an agent that interact with TAK1. In one aspect, the method and the the three dimensional model is used to alter the chemical structure of the agent. This method optionally further comprises synthesizing or obtaining an agent and/or contacting the agent with TAK1 to determine the interaction between the agent and TAK1.

The invention discloses a method for designing an agent that interacts with TAK1, comprising: generating a three dimensional model of TAK1 including relative structural coordinates of a plurality of atoms of an active site of TAK1 and relative structural coordinates of a first agent that interacts with TAK1; utilizing the three dimensional model to design a second agent that interacts with TAK1, wherein utilizing includes altering the relative structural coordinates of the first agent; synthesizing or obtaining the second agent; and determining the interaction of TAK1 with the second agent, wherein the relative structural coordinates of TAK1 are selected according to: FIG. 1, $\pm$ a root mean square deviation from the backbone atoms of amino acids of not more than 1.5 Å; or FIG. 2, $\pm$ a root mean square deviation from the backbone atoms of amino acids of not more than 1.5 Å.

In this embodiment, the altering of the relative structural coordinates of the first agent includes adding, removing, or changing the position of an atom of the first agent. This method optionally further comprises comparing the model including relative structural coordinates of the first agent to a model including the second agent.

The invention discloses a method for designing an agent that interacts with TAK1, comprising: generating a three dimensional model of TAK1; utilizing the three dimensional model to design an agent that interacts with TAK1; and synthesizing or obtaining the agent, wherein the three dimensional model of TAK1 includes relative structural coordinates of a plurality of atoms of TAK1, and the relative structural coordinates are selected according to: FIG. 1, $\pm$ a root mean square deviation from the backbone atoms of amino acids of not more than 1.5 Å; or FIG. 2, $\pm$ a root mean square deviation from the backbone atoms of amino acids of not more than 1.5 Å. In this embodiment, the three dimensional model optionally includes the agent and the method can involve utilizing the three dimensional model to alter the chemical structure of the agent. Further, this embodiment, optionally further comprises providing a composition including TAK1.

**EP 1 851 310 B1**

In embodiments involving a composition, the composition optionally includes a crystal including TAK1 or the composition includes an isotopically labeled TAK1 and the method optionally comprises determining the relative structural coordinates of atoms of TAK1 from the composition.

This invention discloses utilizing the three dimensional includes designing an agent that interacts more strongly with TAK1 than with another kinase. In embodiments involving an agent, the agent is an agent designed by a structure based drug design method. In certain embodiments, the method optionally further comprises contacting the agent with TAK1 to determine the interaction between the agent and TAK1.

A would be recognized, in a method of this invention, a three dimensional model may be used to determine the fit of an agent with an active site of TAK1. In any embodiment of this invention, a three dimensional model may be used to identify residues of TAK1 that can influence the interaction of an agent with TAK1. In another aspect, this invention involves comparing a three dimensional model of TAK1 to another kinase.

In any of these methods, the relative structural coordinates include relative structural coordinates of an atom of TAK1 binding pocket, more specifically an ATP-binding pocket or a TAB1-binding pocket of TAK1. In specific embodiments, the ATP-binding pocket and a TAB1-binding pocket of TAK1 are as defined herein.

In another embodiment, this invention discloses a method of identifying a compound that binds to a TAK1 binding site, said method comprising: modeling a test compound that fits spatially into the TAK1 binding site using an atomic structural model of the TAK1 binding site or portion thereof; and screening said test compound in an assay that measures binding of the test compound to the TAK1 binding site, thereby identifying a test compound that binds to the TAK1 binding site.

In certain embodiments, the TAK1 binding site is the ATP-binding site or the TAB1-binding site.

In certain embodiments, the atomic structural model is a model of human TAK1 and comprises atomic coordinates of amino acid residues selected from the group consisting those amino acids as shown in FIG. 1 or FIG. 2. In a more specific embodiment, the atomic structural model is a model of human TAK1 ATP-binding site or the TAB1-binding site and comprises atomic coordinates of amino acid residues selected from the group consisting those amino acids of the ATP-binding site or the TAB1-binding site as disclosed herein according to FIG. 1 or FIG. 2.

This invention discloses atomic structural models wherein data which is experimentally derived. As would be recognized, in computer-aided drug design methods of this invention, the atomic structural model is provided to a computerized modeling system.

In certain embodiments, the assay is in vitro. In other embodiments, the assay is in vivo. As would be recognized, screening can include high throughput screening. Test compounds may be obtained by any means (e.g., synthses or purchase) and the test compound can be from a library of compounds. The test compound is an agonist or antagonist of TAK1 binding. Ideally, the test compound is an inhibitor of TAK1.

In certain embodiments, the test compound interacts with one or more amino acid residue of the TAK1 ATP-binding pocket or the TAK1 TAB-1-binding pocket. Nothing herein limits the structure of the test compound. In certain embodiments, the test compound is a small organic molecule, a peptide, or a peptidomimetic, with small organic molecules being preferred as test compounds for the ATP-binding pocket and small organic molecules or peptidomimetic being preferred as test compounds for the TAB1-binding site.

In any embodiment of this invention involving a crystal, the crystal is optionally a TAK1 kinase domain bound to an active site inhibitor. In certain embodiments, the crystal belongs to space group I222 and has unit cell parameters of a=58.4, b=144.3, and c=134.7.

Any of the methods involving a binding pocket may employ a binding pocket defined by the TAK1-TAB1 chimera of FIG. 1 or FIG. 2. In preferred embodiments, a binding pocket employed in a method of this invention is one or more of the binding pockets defined herein.

For the first time, the present invention permits the use of molecular design techniques to identify, select and design chemical entities, including inhibitory compounds, capable of binding to TAK1 or TAK1-like binding pockets, motifs and domains. It should be understood that these chemical entities may be peptides, peptidomimetics, small organic compounds, or antibodies.

Applicants' elucidation of binding pockets on TAK1 provides the necessary information for designing new chemical entities and compounds that may interact with TAK1 or TAK1-like substrate or ATP-binding pockets, in whole or in part.

Throughout this description, disclosure about the ability of a chemical entity to bind to, associate with or inhibit TAK1 binding pockets refers to features of the entity alone. Assays to determine if a compound binds to TAK1 are well known in the art and are exemplified below.

The design of chemical entities that bind to or inhibit TAK1 binding pockets according to this invention generally involves consideration of two factors. First, the entity must be capable of physically and structurally associating with parts or all of the TAK1 binding pockets. Non-covalent molecular interactions important in this association include hydrogen bonding, van der Waals interactions, hydrophobic interactions and electrostatic interactions.

Second, the entity must be able to assume a conformation that allows it to associate with the TAK1 binding

24

pockets directly. Although certain portions of the entity will not directly participate in these associations, those portions of the entity may still influence the overall conformation of the molecule. This, in turn, may have a significant impact on potency. Such conformational requirements include the overall three-dimensional structure and orientation of the chemical entity in relation to all or a portion of the binding pocket, or the spacing between functional groups of an entity comprising several chemical entities that directly interact with the TAK1 or TAK1-like binding pockets.

The potential inhibitory or binding effect of a chemical entity on TAK1 binding pockets may be analyzed prior to its actual synthesis and testing by the use of computer modeling techniques. If the theoretical structure of the given entity suggests insufficient interaction and association between it and the TAK1 binding pockets, testing of the entity is obviated. However, if computer modeling indicates a strong interaction, the compound may then be synthesized and tested for its ability to bind to a TAK1 binding pocket. This may be achieved by testing the ability of the molecule to inhibit TAK1 using the assays described in Example 7. In this manner, synthesis of inoperative compounds may be avoided.

A potential inhibitor of a TAK1 binding pocket may be computationally evaluated by means of a series of steps in which chemical entities or fragments are screened and selected for their ability to associate with the TAK1 binding pockets.

One skilled in the art may use one of several methods to screen chemical entities or fragments for their ability to associate with a TAK1 binding pocket. This process may begin by visual inspection of, for example, a TAK1 binding pocket on the computer screen based on the TAK1 structure coordinates in Figure 1 or 2 or other coordinates which define a similar shape generated from the machine-readable storage medium. Selected fragments or chemical entities may then be positioned in a variety of orientations, or docked, within that binding pocket as defined *supra*. Docking may be accomplished using software such as QUANTA and Sybl [Tripos Associates, St. Louis, MO], followed by energy minimization and molecular dynamics with standard molecular mechanics force fields, such as CHARMM and AMBER.

Specialized computer programs may also assist in the process of selecting fragments or chemical entities. These include:

1. GRID [P. J. Goodford, "A Computational Procedure for Determining Energetically Favorable Binding Sites on Biologically Important Macromolecules", J. Med. Chem., 28, pp. 849-857 (1985)]. GRID is available from Oxford University, Oxford, UK.
2. MCSS [A. Miranker et al., "Functionality Maps of Binding Sites: A Multiple Copy Simultaneous Search Method." Proteins: Structure, Function and Genetics, 11, pp. 29-34 (1991)]. MCSS is available from Molecular Simulations, San Diego, CA.
3. AUTODOCK [D. S. Goodsell et al., "Automated Docking of Substrates to Proteins by Simulated Annealing", Proteins: Structure, Function, and Genetics, 8, pp. 195-202 (1990)]. AUTODOCK is available from Scripps Research Institute, La Jolla, CA.
4. DOCK [I. D. Kuntz et al., "A Geometric Approach to Macromolecule-Ligand Interactions", J. Mol. Biol., 161, pp. 269-288 (1982)]. DOCK is available from University of California, San Francisco, CA.

Once suitable chemical entities or fragments have been selected, they can be assembled into a single compound or complex of compounds. Assembly may be preceded by visual inspection of the relationship of the fragments to each other on the three-dimensional image displayed on a computer screen in relation to the structure coordinates of TAK1. This would be followed by manual model building using software such as QUANTA or Sybl [Tripos Associates, St. Louis, MO].

*-Useful programs to aid one of skill in the art in connecting the individual chemical entities or fragments include:

1. CAVEAT [P. A. Bartlett et al., "CAVEAT: A Program to Facilitate the Structure-Derived Design of Biologically Active Molecules", in Molecular Recognition in Chemical and Biological Problems", Special Pub., Royal Chem. Soc., 78, pp. 182-196 (1989); G. Lauri and P. A. Bartlett, "CAVEAT: a Program to Facilitate the Design of Organic Molecules", J. Comput. Aided Mol. Des. , 8, pp. 51-66 (1994)]. CAVEAT is available from the University of California, Berkeley, CA.
2. 3D Database systems such as ISIS (MDL Information Systems, San Leandro, CA). This area is reviewed in Y. C. Martin, "3D Database Searching in Drug Design", J. Med. Chem., 35, pp. 2145-2154 (1992).
3. HOOK [M. B. Eisen et al., "HOOK: A Program for Finding Novel Molecular Architectures that Satisfy the Chemical and Steric Requirements of a Macromolecule Binding Site", Proteins: Struct., Funct., Genet., 19, pp. 199-221 (1994)]. HOOK is available from Molecular Simulations, San Diego, CA.

Instead of proceeding to build an inhibitor of a TAK1 binding pocket in a step-wise fashion one fragment or chemical entity at a time as described above, inhibitory or other TAK1 binding compounds may be designed as a whole or "de novo" using either an empty binding pocket or optionally including some portion(s) of a known inhibitor(s). There are many de novo ligand design methods including:

1. LUDI [H.-J. Bohm, "The Computer Program LUDI: A New Method for the De Novo Design of Enzyme Inhibitors", J. Comp. Aid. Molec. Design, 6, pp. 61-78 (1992)]. LUDI is available from Molecular Simulations Incorporated, San Diego, CA.

2. LEGEND [Y. Nishibata et al., Tetrahedron, 47, p. 8985 (1991)]. LEGEND is available from Molecular Simulations Incorporated, San Diego, CA.

3. LeapFrog [available from Tripos Associates, St. Louis, MO].

4. SPROUT [V. Gillet et al., "SPROUT: A Program for Structure Generation)", J. Comput. Aided Mol. Design, 7, pp. 127-153 (1993)]. SPROUT is available from the University of Leeds, UK.

Other molecular modeling techniques may also be employed in accordance with this invention [see, *e.g.,* N. C. Cohen et al., "Molecular Modeling Software and Methods for Medicinal Chemistry, J. Med. Chem., 33, pp. 883-894 (1990); see also, M. A. Navia and M. A. Murcko, "The Use of Structural Information in Drug Design", Current Opinions in Structural Biology, 2, pp. 202-210 (1992); L. M. Balbes et al., "A Perspective of Modem Methods in Computer-Aided Drug Design", Reviews in Computational Chemistry, Vol. 5, K. B. Lipkowitz and D. B. Boyd, Eds., VCH, New York, pp. 337-380 (1994); see also, W. C. Guida, "Software For Structure-Based Drug Design", Curr. Opin. Struct. Biology, 4, pp. 777-781 (1994)].

Once a chemical entity has been designed or selected by the above methods, the efficiency with which that chemical entity may bind to a TAK1 binding pocket may be tested and optimized by computational evaluation. For example, an effective TAK1 binding pocket inhibitor must preferably demonstrate a relatively small difference in energy between its bound and free states (*i.e.*, a small deformation energy of binding). Thus, the most efficient TAK1 binding pocket inhibitors should preferably be designed with a deformation energy of binding of not greater than about 10 kcal/mole, more preferably, not greater than 7 kcal/mole. TAK1 binding pocket inhibitors may interact with the binding pocket in more than one conformation that is similar in overall binding energy. In those cases, the deformation energy of binding is taken to be the difference between the energy of the free entity and the average energy of the conformations observed when the inhibitor binds to the protein.

An entity designed or selected as binding to a TAK1 binding pocket may be further computationally optimized so that in its bound state it would preferably lack repulsive electrostatic interaction with the target enzyme and with the surrounding water molecules. Such non-complementary electrostatic interactions include repulsive charge-charge, dipole-dipole and charge-dipole interactions.

Specific computer software is available in the art to evaluate compound deformation energy and electrostatic interactions. Examples of programs designed for such uses include: Gaussian 94, revision C [M. J. Frisch, Gaussian, Inc., Pittsburgh, PA ©1995]; AMBER, version 4.1 [P. A. Kollman, University of California at San Francisco, ©1995]; QUANTA/CHARMM [Accelrys, San Diego, CA ©2001, 2002]; Insight II/Discover [Accelrys, San Diego, CA ©2001, 2002]; DelPhi [Accelrys, San Diego, CA ©2001, 2002]; and AMSOL [Quantum Chemistry Program Exchange, Indiana University]. These programs may be implemented, for instance, using a Silicon Graphics workstation such as an Indigo2 with "IMPACT" graphics. Other hardware systems and software packages will be known to those skilled in the art.

Another approach enabled by this invention, is the computational screening of small molecule databases for chemical entities or compounds that can bind in whole, or in part, to a TAK1 binding pocket. In this screening, the quality of fit of such entities to the binding pocket may be judged either by shape complementarity or by estimated interaction energy [E. C. Meng et al., J. Comp. Chem., 13, pp. 505-524 (1992)].

Another particularly useful drug design technique enabled by this invention is iterative drug design. Iterative drug design is a method for optimizing associations between a protein and a compound by determining and evaluating the three-dimensional structures of successive sets of protein/compound complexes.

According to another embodiment, the invention discloses compounds which associate with a TAK1 binding pocket produced or identified by the method set forth above.

Another particularly useful drug design technique enabled by this invention is iterative drug design. Iterative drug design is a method for optimizing associations between a protein and a compound by determining and evaluating the three-dimensional structures of successive sets of protein/compound complexes.

In iterative drug design, crystals of a series of protein or protein complexes are obtained and then the three-dimensional structures of each crystal is solved. Such an approach provides insight into the association between the proteins and compounds of each complex. This is accomplished by selecting compounds with inhibitory activity, obtaining crystals of this new protein/compound complex, solving the three-dimensional structure of the complex, and comparing the associations between the new protein/compound complex and previously solved protein/compound complexes. By observing how changes in the compound affected the protein/compound associations, these associations may be optimized.

In some cases, iterative drug design is carried out by forming successive protein-compound complexes and then crystallizing each new complex. Alternatively, a pre-formed protein crystal is soaked in the presence of an inhibitor, thereby forming a protein/compound complex and obviating the need to crystallize each individual protein/compound complex.

Structure Determination of Other Molecules

The structure coordinates set forth in Figure 1 or 2 can also be used to aid in obtaining structural information about another crystallized molecule or molecular complex. This may be achieved by any of a number of well-known techniques, including molecular replacement.

The machine-readable data storage medium disclosed comprises a data storage material encoded with a first set of machine readable data which comprises the Fourier transform of at least a portion of the structure coordinates set forth in Figure 1 or 2 or homology model thereof, and which, when using a machine programmed with instructions for using said data, can be combined with a second set of machine readable data comprising the X-ray diffraction pattern of a molecule or molecular complex to determine at least a portion of the structure coordinates corresponding to the second set of machine readable data.

A computer for determining at least a portion of the structure coordinates corresponding to X-ray diffraction data obtained from a molecule or molecular complex is disclosed, wherein said computer comprises:

a) a machine-readable data storage medium comprising a data storage material encoded with machine-readable data, wherein said data comprises at least a portion of the structural coordinates of TAK1 according to Figure 1 or 2 or homology model thereof;

b) a machine-readable data storage medium comprising a data storage material encoded with machine-readable data, wherein said data comprises X-ray diffraction data obtained from said molecule or molecular complex; and

c) instructions for performing a Fourier transform of the machine readable data of (a) and for processing said machine readable data of (b) into structure coordinates.

For example, the Fourier transform of at least a portion of the structure coordinates set forth in Figure 1 or 2 or homology model thereof may be used to determine at least a portion of the structure coordinates of TAK1 homologues.

This invention discloses a method of utilizing molecular replacement to obtain structural information about a molecule or molecular complex whose structure is unknown comprising the steps of:

a) crystallizing said molecule or molecular complex of unknown structure;

b) generating an X-ray diffraction pattern from said crystallized molecule or molecular complex;

c) applying at least a portion of the structure coordinates set forth in Figure 1 or 2 or homology model thereof to the X-ray diffraction pattern to generate a three-dimensional electron density map of the molecule or molecular complex whose structure is unknown; and

d) generating a structural model of the molecule or molecular complex from the three-dimensional electron density map.

The method may be performed using a computer. The molecule may be selected from the group consisting of TAK1 and TAK1 homologues. The molecule may be a TAK1 molecular complex or homologue thereof.

By using molecular replacement, all or part of the structure coordinates of the TAK1 as provided by this invention (and set forth in Figure 1 or 2) can be used to determine the structure of a crystallized molecule or molecular complex whose structure is unknown more quickly and efficiently than attempting to determine such information *ab initio*.

Molecular replacement provides an accurate estimation of the phases for an unknown structure. Phases are a factor in equations used to solve crystal structures that can not be determined directly. Obtaining accurate values for the phases, by methods other than molecular replacement, is a time-consuming process that involves iterative cycles of approximations and refinements and greatly hinders the solution of crystal structures. However, when the crystal structure of a protein containing at least a homologous portion has been solved, the phases from the known structure provide a satisfactory estimate of the phases for the unknown structure.

Thus, this method involves generating a preliminary model of a molecule or molecular complex whose structure coordinates are unknown, by orienting and positioning the relevant portion of the TAK1 according to Figure 1 or 2 or homology model thereof within the unit cell of the crystal of the unknown molecule or molecular complex so as best to account for the observed X-ray diffraction pattern of the crystal of the molecule or molecular complex whose structure is unknown. Phases can then be calculated from this model and combined with the observed X-ray diffraction pattern amplitudes to generate an electron density map of the structure whose coordinates are unknown. This, in turn, can be subjected to any well-known model building and structure refinement techniques to provide a final, accurate structure of the unknown crystallized molecule or molecular complex [E. Lattman, "Use of the Rotation and Translation Functions", in Meth. Enzymol., 115, pp. 55-77 (1985); M. G. Rossmann, ed., "The Molecular Replacement Method", Int. Sci. Rev. Ser., No. 13, Gordon & Breach, New York (1972)].

The structure of any portion of any crystallized molecule or molecular complex that is sufficiently homologous to any portion of the TAK1 can be resolved by this method.

The method of molecular replacement may be utilized to obtain structural information about a TAK1 homologue. The structure coordinates of TAK1 as provided by this invention are particularly useful in solving the structure of TAK1 complexes that are bound by ligands, substrates and inhibitors.

Furthermore, the structure coordinates of TAK1 as provided by this invention are useful in solving the structure of TAK1 proteins that have amino acid substitutions, additions and/or deletions (referred to collectively as "TAK1 mutants", as compared to naturally occurring TAK1). These TAK1 mutants may optionally be crystallized in co-complex with a chemical entity, such as a non-hydrolyzable ATP analog or a suicide substrate. The crystal structures of a series of such complexes may then be solved by molecular replacement and compared with that of wild-type TAK1. Potential sites for modification within the various binding pockets of the enzyme may thus be identified. This information provides an additional tool for determining the most efficient binding interactions, for example, increased hydrophobic interactions, between TAK1 and a chemical entity or compound.

The structure coordinates are also particularly useful in solving the structure of crystals of TAK1 or TAK1 homologues co-complexed with a variety of chemical entities. This approach enables the determination of the optimal sites for interaction between chemical entities, including candidate TAK1 inhibitors. For example, high resolution X-ray diffraction data collected from crystals exposed to different types of solvent allows the determination of where each type of solvent molecule resides. Small molecules that bind tightly to those sites can then be designed and synthesized and tested for their TAK1 inhibition activity.

All of the complexes referred to above may be studied using well-known X-ray diffraction techniques and may be refined versus 1.5-3.4Å resolution X-ray data to an R value of about 0.30 or less using computer software, such as X-PLOR (Yale University, ©1992, distributed by Molecular Simulations, Inc.; see, *e.g.*, Blundell & Johnson, supra; Meth. Enzymol., vol. 114 & 115, H. W. Wyckoff et al., eds., Academic Press (1985)), CNS (Brunger et al., Acta Crystallogr. D. Biol. Crystallogr., 54, pp. 905-921, (1998)) or CNX (Accelrys, ©2000,2001). This information may thus be used to optimize known TAK1 inhibitors, and more importantly, to design new TAK1 inhibitors.

In order that this invention be more fully understood, the following examples are set forth. These examples are for the purpose of illustration only and are not to be construed as limiting the scope of the invention in any way.

<u>Example 1:</u> Expression and Purification of TAK-TAB constructs for crystallography and enzymology

The expression of TAK1 was carried out using standard procedures known in the art.

A truncated version of the TAK1 kinase domain (residues I31-Q303) fused to a 36-residue TAB1 segment (residues H468-P504) was cloned downstream of the polyhedrin promoter in the baculovirus donor vector, pBEV10TOPO, using the BamHI and EcoRI sites. The vector incorporated an N-terminal hexa-histidine purification tag and thrombin cleavage site. pBEV10TOPO is a Bac-to-Bac compatible vector and recombinant virus was generated according to the manufacturer's recommendations. These initially transfected Spodoptera frugiperda (Sf9) cells were tested for the expression of TAK1(I31-Q303)/TAB1(H468-P504) protein by loading a crude extract of the transfected insect cells onto an SDS-PAGE gel and immunoblot analysis using an anti-His (Sigma) antibody. Upon confirmation of the expression of the TAK1(I31-Q303)/TAB1(H468-P504) protein the virus was further amplified two times to obtain high titer stocks and used for optimisation of expression studies in Hi5 and Sf9 insect cells (1.5x10$^6$ cells/ml) at 27°C with shaking at 100rpm, using defined volumes of virus. After infection, cells were harvested at regular intervals of 24, 48 and 72 hours and optimum expression was determined by SDS-PAGE gel and immunoblot analysis using an anti-His (Sigma) antibody. Large-scale cultivation/expression was conducted with Hi5 insect cells (1.5x10$^6$ cells/ml) using a multiplicity of infection (M.O.I.) of between 2 and 5 of recombinant TAK1(I31-Q303)/TAB1(H468-P504) virus particles/cell, incubated at 27°C, 100rpm and harvested 48 hours post-infection.

Cell pellets were resuspended into Lysis buffer (50mM Hepes, pH 7.8, 250mM NaCl, 5mM β-mercaptoethanol, 10% Glycerol (v/v), 0.05% Tween, 5mM Imidazole, 0.5mM Sodium orthovanadate, 50mM sodium fluoride, 10mM β-glycerophosphate, Protease inhibitor Cocktail I and Phosphatase Inhibitor) and disrupted by dounce homogenization, on ice. Resuspended cells were further mechanically disrupted using a Microfluidizer (Microfluidics, Newton, MA). The cell debris was removed by centrifugation (21,000rpm, 15min at 4°C) and the supernatant incubated for 2.5 hours at 4°C with pre-equilibrated Nickel-NTA metal affinity resin. The NiNTA resin was collected by centrifugation (1000g, 4 min) and the non-specifically bound protein was removed by washing with 30x bead volume of Lysis buffer. The TAK1(I31-Q303)/TAB(H468-P504) protein was eluted 3 times with Lysis buffer containing 200mM Imidazole and a final elution was carried out using Lysis buffer containing 500mM Imidazole. The N-terminal hexa-histidine tag was removed by a 4°C overnight incubation using 20 Units thrombin (Sigma) per mg of eluted protein and successful cleavage analysed by SDS-PAGE gel. The cleaved protein was then isolated by size-exclusion on a Superdex 200(26/60) column (Amersham Biotech, Sweden) pre-equilibrated in Gel Filtration Buffer (50mM Hepes, pH 7.8, 500mM NaCl, 5mM DTT and 10% Glycerol). Further purification of TAK1(I31-Q303)/TAB(H468-P504) was performed using anion exchange chromatography. Protein was applied to a 6ml Resource Q column (Amersham Biotech, Sweden) pre-equilibrated with Buffer A (25mM Tris, pH 8.0, 50mM NaCl and 5mM DTT) at a flow rate of 0.5ml/min. Unbound protein was extensively washed with 10CV of Buffer A. Bound protein was eluted with a linear salt gradient (0-15%) of Buffer B (25mM Tris, pH 8.0, 1M NaCl and 5mM DTT) over 10CV at a flow rate of 0.5ml/min. A second gradient (15-100%) was applied over 2CV to elute remaining proteins .The resultant protein fractions were analysed by SDS-PAGE gel and fractions containing purified TAK1(I31 Q303)/TAB1(H468-P504 were pooled accordingly. The purified protein sample was dialysed against 50mM Hepes pH8.0 containing 200mM NaCl, 10% glycerol and 5mM DTT at 4°C and concentrated to 10 mg/ml for crystallization. All proteins TAK-TAB chimera proteins were prepared using a similar protocol.

<u>Example</u> 2: Enzymatic <u>characterization</u> of <u>chimeric</u> <u>TAK-TAB proteins</u>

Activity of the TAK-1:TAB-1 fusion contructs was determined using a standard coupled enzyme system (Fox et al., Protein Sci., 7, pp. 2249 (1998)). Reactions were carried out in a solution containing 100 mM HEPES (pH 7.5), 10 mM $MgCl_2$, 25 mM NaCl, 2 mM DTT and 3% DMSO. Final peptidic substrate (full length Myelin Basic Protein, Vertex Pharmaceuticals Inc., Cambridge, MA) concentration in the assay was 15 mM. Reactions were carried out at 30 °C in the presence of 500 nM TAK-1:TAB1 construct and a titration of ATP (Sigma Chemicals, St Louis, MO) at final assay concentrations spanning 0 to $500\mu M$. Final concentrations of the components of the coupled enzyme system were 2.5 mM phosphoenolpyruvate, 300 mM NADH, 60 mg/ml pyruvate kinase and 20 mg/ml lactate dehydrogenase. An assay stock buffer solution was prepared containing all of the reagents listed above with the exception of ATP and DMSO. The assay stock buffer solution (60 ml) was incubated in a 96 well plate with 2 ml DMSO. The reaction was initiated by the addition of 5 ml of ATP (final assay concentrations spanning 0 to 500 mM). Rates of reaction were obtained using a Molecular Devices Spectramax plate reader (Sunnyvale, CA) over 10 min at 30 °C. The ATP Km and Vmax values were determined from the rate data as a function of ATP concentration using computerized nonlinear regression (Prism 4.0a, Graphpad Software, San Diego, CA).

**Example 3:** Formation of TAK1 - inhibitor Complex for crystallization

Crystals of TAK1- inhibitor complex crystals were formed by co-crystallizing the protein with the inhibitors or with adenosine. The inhibitor was added to the TAK1 protein solution immediately after the final protein concentration step (Example 1), immediately prior to setting up the crystallization drop.

**Example 4:** Crystallization of TAK1 and TAK1 - inhibitor complexes

Crystallization of TAK1 was carried out using the hanging drop vapor diffusion technique. The TAK1 formed lozenge-like crystals over a reservoir containing 600-800mM sodium citrate, 200mM sodium chloride, 100 mM Tris-HCl pH7.0 and 10mM DTT. The crystallization droplet contained 0.25 $\mu$l of 10 mg ml$^{-1}$ protein solution and 0.25 $\mu$l of reservoir solution. Crystals formed in approximately 72 hours.

The formed crystals were transferred to a reservoir solution containing 15% ethylene glycol. After soaking the crystals in 15% ethylene glycol for less than 2 minutes, the crystals were scooped up with a cryo-loop, frozen in liquid nitrogen and stored for data collection.

**Example 5:** Soaking of preformed TAK1 complex crystals in solutions of other inhibitors

An alternative method for preparing complex crystals of TAK1 is to remove a co-complex crystal grown by hanging drop vapour diffusion (Example 3) from the hanging drop and place it in a solution consisting of a reservoir solution containing 0.5mM staurosporine or another inhibitor for a period of time between 1 and 24 hours.

The crystals can then be transferred to a reservoir solution containing 15% ethylene glycol and 0.5mM staurosporine or another inhibitor. After soaking the crystal in this solution for less than 2 minutes, the crystals were scooped up with a cryo-loop, frozen in liquid nitrogen and stored for data collection. Subsequent data collection and structure determination (Example 5) reveals that inhibitors bound to the ATP-binding site of TAK1 can be exchanged for the TAK1 complex crystals.

**Example 6:** X-Ray Data Collection and Structure Determination

The TAK1 - inhibitor complex structures and the TAK1 - adenosine structure were solved by molecular replacement using X-ray diffraction data collected either (i) at beam line 14.2 of the CCLRC Synchrotron Radiation Source, Daresbury, Cheshire, UK, or (ii) Vertex Pharmaceuticals (Europe) Ltd, 88 Milton Park, Abingdon, Oxfordshire OX14 4RY, UK. The diffraction images were processed with the program MOSFLM [A.G. Leslie, Acta Cryst. D, 55, pp. 1696-1702 (1999)] and the data was scaled using SCALA [Collaborative Computational Project, N., Acta Cryst. D, 50, pp. 760-763 (1994)].

The data statistics, unit cell parameters and spacegroup of the TAK1 - adenosine crystal structure is given in Table 2. The starting phases for the TAK1 complexes were obtained by molecular replacement using coordinates of Aurora-2 as a search model in the program AMoRe [J. Navaza, Acta. Cryst. A, 50, pp. 157-163 (1994)]. The asymmetric unit contained a single TAK1 complex. Multiple rounds of rebuilding with QUANTA [Molecular Simulations, Inc., San Diego, CA ©1998,2000] and refinement with CNX [Accelrys Inc., San Diego, CA ©2000] resulted in a final model that included residues 31 to 178 and 191 to 303 of TAK1 and 468 to 495 of TAB1. The refined model has a crystallographic R-factor of 21.2% and R-free of 23.1%.

The data statistics, unit cell parameters and spacegroup of the TAK1 - 3-[6-(4-Acetyl-3,5-dimethyl-piperazin-1-yl)-pyridin-2-yl]-1H-pyrrolo[2,3-b]pyridine-5-carboxylic acid methyl ester crystal structure is given in Table 3. The starting phases were obtained by molecular replacement using coordinates of the TAK1-adenosinecomplex as a search model in the program AMoRe. Multiple rounds of rebuilding with QUANTA [Molecular Simulations, Inc., San Diego, CA ©1998,2000] and refinement with CNX [Accelrys Inc., San Diego, CA ©2000] resulted in a final model that included residues 31 to 178 and 191 to 303 of TAK1 and 468 to 495 of TAB1. The refined model has a crystallographic R-factor of 27.8% and R-free of 32.1%.

In the above models, disordered residues were not included in the model. Alanine or glycine residues were used in the model if the side chains of certain residues could not be located in the electron density.

<u>Example 7:</u> Overall Structure of the TAK1-TAB1 chimera

TAK1 has the typical bi-lobal catalytic kinase fold or structural domain [S. K. Hanks, et al., Science, 241, pp. 42-52 (1988); Hanks, S.K. and A.M. Quinn, Meth. Enzymol., 200, pp. 38-62 (1991)] with a β-stand sub-domain (residues 31-104) at the N-terminal end and an α-helical sub-domain at the C-terminal end (residues 112-303) (Figure 3). The ATP-binding pocket is at the interface of the α-helical and β-strand domains, and is bordered by the glycine rich loop and the hinge. The activation loop is disorder in both crystal structures.

Comparison with other kinases such as LCK, p38 and Aurora2 revealed that the structure of TAK1 resembles closely the substrate-bound, activated, form of a kinase. The overall topology of the kinase domain is similar to other serine/threonine kinases and several other tyrosine kinases, particularly LCK, ITK and Aurora-2, and distinct from other members of the MAP kinase family (P38 and MK2; Table 1).

<u>Example 8:</u> Catalytic active site of TAK1- inhibitor Complexes

The inhibitor 3-[6-(4-Acetyl-3,5-dimethyl-piperazin-1-yl)-pyridin-2-yl]-1H-pyrrolo[2,3-b]pyridine-5-carboxylic acid methyl ester is bound in the deep cleft of the catalytic active site in the TAK1 structure. The inhibitor forms two hydrogen bonds with the hinge portion of the ATP-binding pocket (dotted lines).

The side chains of D175 and K63 are positioned inside the ATP-binding pocket and make a salt-bridge interaction with each other. Like other kinases, K63 and D175 are catalytically important residues and resemble a catalytically active conformation.

<u>Example 9:</u> The Use of TAK1 Coordinates for Inhibitor Design

The coordinates of Figure 1 or 2 are used to design compounds, including inhibitory compounds, that associate with TAK1 or homologues of TAK1. This process may be aided by using a computer comprising a machine-readable data storage medium encoded with a set of machine-executable instructions, wherein the recorded instructions are capable of displaying a three-dimensional representation of the TAK1 or a portion thereof. The graphical representation is used according to the methods described herein to design compounds. Such compounds associate with the TAK1 at the ATP-binding pocket, substrate binding pocket or TAB1 binding pocket.

<u>Example 10:</u> The Use of TAK1 Coordinates in the Design of TAK1-specific Antibodies

The atomic coordinates in Figures 1 or 2 also define, in great detail, the external solvent-accessible, hydrophilic, and mobile surface regions of the TAK1 catalytic kinase domain. Anti-peptide antibodies are known to react strongly against highly mobile regions but do not react with well-ordered regions of proteins. Mobility is therefore a major factor in the recognition of proteins by anti-peptide antibodies [J. A. Tainer et al., Nature, 312, pp. 127-134 (1984)]

One skilled in the art would therefore be able to use the X-ray crystallography data to determine possible antigenic sites in the TAK1 kinase domain. Possible antigenic sites are exposed, small and mobile regions on the kinase surface which have atomic B-factors of greater than 80 Å$^2$ in Figures 1 and 2. This information can be used in conjunction with data from immunological studies to design and produce specific monoclonal or polyclonal antibodies.

This process may be aided by using a computer comprising a machine-readable data storage medium encoded with a set of machine-executable instructions, wherein the recorded instructions are capable of displaying a three-dimensional representation of the TAK1 or a portion thereof.

<u>Example 11:</u> Enzymatic investigation of TAK1-TAB1 chimeras

Many studies have attempted to further elucidate the molecular mechanisms that regulate the activation of Tak1 and more specifically the role that Tab1 plays in the process. The activity of Tak1 is dependent on a series of Tak1 catalysed intramolecular phosphorylation events mapped to three residues on Tak1 (Thr184, Thr187, Ser192) (ref 15, 16, 17) along with as yet unmapped phosphorylation sites on Tab1 (Sakurai 2000).

We have prepared and analysed a number of Tak1-Tab1 fusion proteins to explore the effect that varied truncations in both Tak1 and Tab1 had on substrate kinetics. The purified recombinant fusion protein first described by Sakurai et al (Table1) shows a high affinity for ATP with Km of 24±μM and moderate kinase activity with kcat of 7.2min$^{-1}$. Truncation of the TAB1 region to just 36 residues had no significant effect on the substrate kinetics with the purified recombinant protein showing a Km of 21±1μM and kcat of 11.4min$^{-1}$, contrasting with data for co-expression of Tab1 and Tak1 in mammalian cells, which showed differences in enzyme activity (15). Our data shows that varying the length of the Tab1 peptide has no direct effect on either ATP binding affinity or enzyme rate. These differences might arise from enhanced stability of cellular TAK-TAB complexes.

We then examined the role of the Tak1 N-terminus on activity by characterising the kinetics of a truncated fusion protein consisting of the Tak1 residues 31-301 and the short 37-residue Tab1 peptide. No significant differences were observed in either substrate affinity (Km 27μM) or enzyme turnover (kcat 15min$^{-1}$) when compared with the analogous construct containing the full Tak1 N-terminus. This data suggests that the N-terminus has no direct inhibitory capacity for our proteins.

TABLE 2: Summary of data collection for TAK1 - adenosine complex

Space Group: I222

Unit Cell: a=58.4 Å, b=144.3 Å, c=134.7 Å; α=β=γ= 90°

| Source | Daresbury SRS 14.2 |
|---|---|
| Wavelength ($\lambda$) | 1.488 |
| Resolution (Å) | 1 . 9 |
| No. of Reflections (measured/unique) | 137639/38477 |
| Completeness (%) (overall/outer shell) | 85.2/48.8 |
| I/σ(I) (overall/outer shell) | 10.7/1.5 |
| $R_{merge}$*(%) (overall/outer shell) | 4.4/52.2 |
| Molecules per asymmetric unit | 1 |
| * | |
| $$R_{merge} = 100 \times \Sigma_h \Sigma_j \, |<I(h)> - I(h)_j| \, / \, \Sigma_h \Sigma_j <I(h)>$$ | |

Structure refinement

| Resolution (Å) | 20-1.9 |
|---|---|
| No. of reflections | 35019 |
| R factor (%) | 21.2 |
| Free R factor (%) † | 23.1 |
| RMSD values Bond lengths (Å)/angles (°) | 0.008/1.6 |
| † The Free R factor was calculated with 2.0% of the data. | |

TABLE 3: Summary of data collection for TAK1 - 3-[6-(4-Acetyl-3,5-dimethyl-piperazin-1-yl)-pyridin-2-yl]-1H-pyrrolo[2,3-b]pyridine-5-carboxylic acid methyl ester complex

Space Group: I222

Unit Cell: a=58.1 Å, b=133.3 Å, c=143.4 Å; α=β=γ= 90°

| Source | Vertex |
|---|---|
| Wavelength ($\lambda$) | 1.5438 |
| Resolution (Å) | 3.3 |
| No. of Reflections (measured/unique) | 73870/8654 |
| Completeness (%) (overall/outer shell) | 99.2/98.6 |
| I/σ(I) (overall/outer shell) | 5.6/1.6 |
| $R_{merge}$* (%) (overall/outer shell) | 13.3/50.6 |
| Molecules per asymmetric unit | 1 |
| * | |
| $$R_{merge} = 100 \times \Sigma_h \Sigma_j \, |<I(h)> - I(h)_j| \, / \, \Sigma_h \Sigma_j <I(h)>$$ | |

Structure refinement

| Resolution (Å) | 20-3.3 |
|---|---|
| No. of reflections | 7852 |
| R factor (%) | 7.8 |
| Free R factor (%) †† | 32.2 |
| RMSD values Bond lengths (Å)/angles (°) | 0.019/1.6 |
| †† The Free R factor was calculated with 2.5% of the data. | |

TABLE 4: Enzymatic characterization of proteins

| Protein | Km (ATP) ($\mu$M) | Kcat (s-1) |
|---|---|---|
| M1-Q303:EFG$_5$:Q437-P504 | 24 $\pm$ 2 | 0.12 |
| I31-Q303:H468-P504 | 27 $\pm$ 1 | 0.26 |
| M1-Q303:H468-P504 | 21 $\pm$ 1 | 0.19 |

## Claims

1. An isolated, purified human TAK1 construct of amino acids I31-Q303 that is directly fused to a human TAB1 segment of amino acids H468-P504.

2. A crystal comprising a TAK1 kinase domain directly fused to a TAK1-activating domain of TAB1.

3. The crystal according to claim 2 wherein the TAK1 kinase domain is complexed with an active site inhibitor.

4. The crystal according to claim 3, wherein the active site inhibitor is an ATP, a nucleotide triphosphate or an ATP analogue.

5. The crystal according to claim 4, wherein the active site inhibitor is an ATP analog selected from the group consisting of adenosine, adenylyl imidodiphosphate, staurosporine and 3-(8-phenyl-5,6-dihydrothieno[2,3-h]quinazolin-2-ylamino)benzenesulfonamide.

6. The crystal according to claim 4, wherein the active site inhibitor is adenosine or 3-[6-(4-acetyl-3,5-dimethyl-piperazine-1-yl)-pyridin-2yl]-1H-pyrrolo[2,3-b]pyridine-5-carboxylic acid methyl ester.

7. The crystal according to any one of claims 2-6, wherein the TAK1 kinase domain comprises residues I31-Q303 of human TAK1.

8. The crystal according to any one of claims 2-6, wherein the TAK1 kinase domain comprises residues I31-Q303 of human TAK1 and the TAB1 segment comprises amino acids H468-P504 of human TAB1.

9. A crystallizable composition comprising a TAK1 kinase domain directly fused to a TAK1-activating domain of TAB1.

10. The crystallizable composition according to claim 9 further comprising 600-900 mM sodium citrate, 1 to 200 mM sodium chloride, and a buffer that maintains pH at between about 6.5 and about 8.5.

11. The crystallizable composition according to claim 10 further comprising a reducing agent at between about 1 to about 20 mM.

**12.** The crystallizable composition according to any one of claims 9-11, wherein the TAK1 kinase domain comprises residues I31-Q303 of human TAK1.

**13.** The crystallizable composition according to any one of claims 9-11, wherein the TAK1 kinase domain comprises residues I31-Q303 of human TAK1 and the TAB1 segment comprises amino acids H468-P504 of human TAB1.

**14.** A method of utilizing molecular replacement to obtain structural information about a molecule or a molecular complex of unknown structure, wherein the molecule is sufficiently homologous to TAK1, comprising the steps of:

(a) crystallizing said molecule or molecular complex;
(b) generating an X-ray diffraction pattern from said crystallized molecule or molecular complex; and
(c) applying the structure coordinates of Figure 1 or Figure 2 to the X-ray diffraction pattern to generate a three-dimensional electron density map of at least a portion of the molecule or molecular complex of unknown structure; and
(d) generating a structural model of the molecule or molecular complex from the three-dimensional electron density map.

**15.** A method of identifying a TAK1 binding compound, comprising the steps of:

(a) using an ATP-binding pocket of the structure coordinates of Figure 1 or Figure 2, or a TAK1-TAB1-binding pocket of the structure coordinates of Figure 1 or Figure 2, to computationally screen a candidate compound for an ability to bind an ATP-binding site of TAK1, wherein said ATP-binding pocket comprises the amino acids of V42, V90, M104, E105, A107 and L163 according to Figures 1 and 2, and wherein said TAK1-TAB1-binding pocket comprises the amino acids of M131, P256, Y290 and F291 according to Figures 1 and 2,
**(b) synthesizing the candidate compound and**
**(c) screening the synthesized compound for TAK1 binding activity.**

**16.** A method of utilizing molecular replacement to obtain structural information about a molecule or a molecular complex of unknown structure, wherein the molecule is sufficiently homologous to TAK1, comprising the steps of:

(a) crystallizing said molecule or molecular complex;
(b) generating an X-ray diffraction pattern from said crystallized molecule or molecular complex; and
(c) applying an ATP-binding pocket of the structure coordinates of Figure 1 or Figure 2, or a TAK1-TAB1-binding pocket of the structure coordinates of Figure 1 or Figure 2, to the X-ray diffraction pattern to generate a three-dimensional electron density map of at least a portion of the molecule or molecular complex of unknown structure, wherein said ATP-binding pocket comprises the amino acids of V42, V90, M104, E105, A107 and L163 according to Figures 1 and 2, and wherein said TAK1-TAB1-binding pocket comprises the amino acids of M131, P256, Y290 and F291 according to Figures 1 and 2; and
(d) generating a structural model of the molecule or molecular complex from the three-dimensional electron density map.

**Patentansprüche**

**1.** Isoliertes, aufgereinigtes menschliches TAK1-Konstrukt von Aminosäuren 131-Q303, das direkt an ein menschliches TAB1-Segment von Aminosäuren H468-P504 fusioniert ist.

**2.** Kristall umfassend eine TAK1-Kinasedomäne, die direkt an eine TAK1-aktivierende Domäne von TAB1 fusioniert ist.

**3.** Kristall nach Anspruch 2, wobei die TAK1-Kinasedomäne mit einem Inhibitor des aktiven Zentrums komplexiert ist.

**4.** Kristall nach Anspruch 3, wobei der Inhibitor des aktiven Zentrums ein ATP, ein Nucleotidtriphosphat oder ein ATP-Analogon ist.

**5.** Kristall nach Anspruch 4, wobei der Inhibitor des aktiven Zentrums ein ATP-Analogon ist ausgewählt aus der Gruppe bestehend aus Adenosin, Adenylylimidodiphosphat, Staurosporin und 3-(8-Phenyl-5,6-dihydrothieno[2,3-h]quinazolin-2-ylamino)benzolsulfonamid.

6. Kristall nach Anspruch 4, wobei der Inhibitor des aktiven Zentrums Adenosin oder 3-[6-(4-Acetyl-3,5-dimethyl-piperazin-1-yl)-pyridin-2yl]-1H-pyrrolo[2,3-b]pyridin-5-carbonsäuremethylester ist.

7. Kristall nach einem der Ansprüche 2 bis 6, wobei die TAK1-Kinasedomäne die Reste I31-Q303 von menschlichem TAK1 umfasst.

8. Kristall nach einem der Ansprüche 2 bis 6, wobei die TAK1-Kinasedomäne die Reste I31-Q303 von menschlichem TAK1 umfasst und das TAB1-Segment Aminosäuren H468-P504 von menschlichem TAB1 umfasst.

9. Kristallisierbare Zusammensetzung umfassend eine TAK1-Kinasedomäne, die direkt an eine TAK1-aktivierende Domäne von TAB1 fusioniert ist.

10. Kristallisierbare Zusammensetzung nach Anspruch 9, ferner umfassend 600-900 mM Natriumcitrat, 1 bis 200 mM Natriumchlorid und einen Puffer, der einen pH-Wert von zwischen etwa 6,5 und etwa 8,5 aufrecht erhält.

11. Kristallisierbare Zusammensetzung nach Anspruch 10, ferner umfassend ein Reduktionsmittel von zwischen etwa 1 bis etwa 20 mM.

12. Kristallisierbare Zusammensetzung nach einem der Ansprüche 9 bis 11, wobei die TAK1-Kinasedomäne die Reste 131 bis Q303 von menschlichem TAK1 umfasst.

13. Kristallisierbare Zusammensetzung nach einem der Ansprüche 9 bis 11, wobei die TAK1-Kinasedomäne die Reste I31-Q303 von menschlichem TAK1 umfasst und das TAB1-Segment Aminosäuren H468-P504 von menschlichem TAB1 umfasst.

14. Verfahren zur Verwendung von molekularer Erneuerung, um strukturelle Informationen über ein Molekül oder einen Molekülkomplex von unbekannter Struktur zu erhalten, wobei das Molekül ausreichend homolog zu TAK1 ist, umfassend die Schritte:

    (a) Kristallisieren des Moleküls oder Molekülkomplexes;
    (b) Erzeugen eines Röntgenbeugungsmusters des kristallisierten Moleküls oder Molekülkomplexes; und
    (c) Anwenden der Strukturkoordinaten von Figur 1 oder Figur 2 auf das Röntgenbeugungsmuster, um eine dreidimensionale Elektronendichtekarte von mindestens einem Teil des Moleküls oder Molekülkomplexes von unbekannter Struktur zu erzeugen; und
    (d) Erzeugen eines Strukturmodells des Moleküls oder Molekülkomplexes aus der dreidimensionalen Elektronendichtekarte.

15. Verfahren zur Identifizierung einer TAK1-bindenden Verbindung, umfassend die Schritte:

    (a) Verwenden einer ATP-bindenden Tasche der Strukturkoordinaten von Figur 1 oder Figur 2, oder einer TAK1-TAB1-bindenden Tasche der Strukturkoordinaten von Figur 1 oder Figur 2, um rechenbetont eine Kandidatenverbindung auf eine Fähigkeit zu überprüfen, an eine ATP-Bindestelle von TAK1 zu binden, wobei die ATP-bindende Tasche die Aminosäuren V42, V90, M104, E105, A107 und L163 nach Figur 1 und 2 umfasst, und wobei die TAK1-TAB1-bindende Tasche die Aminosäuren M131, P256, Y290 und F291 nach Figur 1 und 2 umfasst,
    (b) Synthetisieren der Kandidatenverbindung und
    (c) Überprüfen der synthetisierten Verbindung auf TAK1-Bindeaktivität.

16. Verfahren zur Verwendung von molekularer Erneuerung, um strukturelle Informationen über ein Molekül oder einen Molekülkomplex von unbekannter Struktur zu erhalten, wobei das Molekül ausreichend homolog zu TAK1 ist, umfassend die Schritte:

    (a) Kristallisieren des Moleküls oder Molekülkomplexes;
    (b) Erzeugen eines Röntgenbeugungsmusters des kristallisierten Moleküls oder Molekülkomplexes; und
    (c) Anwenden einer ATP-bindenden Tasche der Strukturkoordinaten von Figur 1 oder Figur 2, oder einer TAK1-TAB1-bindenen Tasche der Strukturkoordinaten von Figur 1 oder Figur 2 auf das Röntgenbeugungsmuster, um eine dreidimensionale Elektronendichtekarte von mindestens einem Teil des Moleküls oder Molekülkomplexes von unbekannter Struktur zu erzeugen, wobei die ATP-bindende Tasche die Aminosäuren V42, V90, M104, E105, A107 und L163 gemäß Figur 1 und 2 umfasst, und wobei die TAK1-TAB1-bindende Tasche die Aminosäuren M131, P256, Y290 und F291 gemäß Figur 1 und 2 umfasst; und

(d) Erzeugen eines Strukturmodells des Moleküls oder Molekülverbunds aus der dreidimensionalen Elektronendichtekarte.

**Revendications**

1. Produit de construction de la TAK1 humaine purifié, isolé, des acides aminés I31 à Q303 qui est fusionné directement à un segment de la TAB1 humaine des acides aminés H468 à P504.

2. Cristal comprenant un domaine kinase de la TAK1 fusionné directement à un domaine d'activation de la TAK1 de la TAB1.

3. Cristal selon la revendication 2, dans lequel le domaine kinase de la TAK1 est complexé à un inhibiteur de site actif.

4. Cristal selon la revendication 3, dans lequel l'inhibiteur de site actif est un ATP, un nucléotide triphosphate ou un analogue de l'ATP.

5. Cristal selon la revendication 4, dans lequel l'inhibiteur de site actif est un analogue de l'ATP choisi dans le groupe consistant en l'adénosine, l'adénylyl-imidodiphosphate, la staurosporine et le 3-(8-phényl-5,6-dihydrothiéno[2,3-h]-quinazolin-2-ylamino)benzène-sulfonamide.

6. Cristal selon la revendication 4, dans lequel l'inhibiteur de site actif est l'adénosine ou l'ester méthylique de l'acide 3-[6-(4-acétyl-3,5-diméthyl-pipérazin-1-yl)-pyridin-2-yl]-1H-pyrrolo[2,3-b]pyridine-5-carboxylique.

7. Cristal selon l'une quelconque des revendications 2 à 6, dans lequel le domaine kinase de la TAK1 comprend les résidus I31 à Q303 de la TAK1 humaine.

8. Cristal selon l'une quelconque des revendications 2 à 6, dans lequel le domaine kinase de la TAK1 comprend les résidus I31 à Q303 de la TAK1 humaine et le segment de la TAB1 comprend les acides aminés H468 à P504 de la TAB1 humaine.

9. Composition cristallisable comprenant un domaine kinase de la TAK1 fusionné directement à un domaine d'activation de la TAK1 de la TAB1.

10. Composition cristallisable selon la revendication 9, comprenant en outre 600 à 900 mM de citrate de sodium, 1 à 200 mM de chlorure de sodium et un tampon qui maintient le pH entre environ 6,5 et environ 8,5.

11. Composition cristallisable selon la revendication 10, comprenant en outre un agent réducteur à une concentration entre environ 1 et environ 20 mM.

12. Composition cristallisable selon l'une quelconque des revendications 9 à 11, dans laquelle le domaine kinase de la TAK1 comprend les résidus I31 à Q303 de la TAK1 humaine.

13. Composition cristallisable selon l'une quelconque des revendications 9 à 11, dans laquelle le domaine kinase de la TAK1 comprend les résidus I31 à Q303 de la TAK1 humaine et le segment de la TAB1 comprend les acides aminés H468 à P504 de la TAB1 humaine.

14. Procédé d'utilisation du remplacement moléculaire pour obtenir des informations structurales sur une molécule ou un complexe moléculaire de structure inconnue, où la molécule est suffisamment homologue à la TAK1, comprenant les étapes consistant à :

    (a) cristalliser ladite molécule ou ledit complexe moléculaire ;
    (b) générer un profil de diffraction des rayons X à partir de ladite molécule ou dudit complexe moléculaire cristallisé(e) ; et
    (c) appliquer les coordonnées structurales de la figure 1 ou de la figure 2 au profil de diffraction des rayons X pour générer une carte de densité électronique tridimensionnelle d'au moins une partie de la molécule ou du complexe moléculaire de structure inconnue ; et
(d) générer un modèle structural de la molécule ou du complexe moléculaire à partir de la carte de densité électronique tridimensionnelle.

**15.** Procédé d'identification d'un composé de liaison de la TAK1, comprenant les étapes consistant à :

(a) utiliser une poche de liaison de l'ATP des coordonnées structurales de la figure 1 ou de la figure 2, ou une poche de liaison de la TAK1-TAB1 des coordonnées structurales de la figure 1 ou de la figure 2, pour cribler par ordinateur un composé candidat ayant une capacité de liaison d'un site de liaison de l'ATP de la TAK1, où ladite poche de liaison de l'ATP comprend les acides aminés de V42, V90, M104, E105, A107 et L163 selon les figures 1 et 2, et où ladite poche de liaison de la TAK1-TAB1 comprend les acides aminés de M131, P256, Y290 et F291 selon les figures 1 et 2,
(b) synthétiser le composé candidat et
(c) cribler le composé synthétisé ayant une activité de liaison de la TAK1.

**16.** Procédé d'utilisation du remplacement moléculaire pour obtenir des informations structurales sur une molécule ou un complexe moléculaire de structure inconnue, où la molécule est suffisamment homologue à la TAK1, comprenant les étapes consistant à :

(a) cristalliser ladite molécule ou ledit complexe moléculaire ;
(b) générer un profil de diffraction des rayons X à partir de ladite molécule ou dudit complexe moléculaire cristallisé(e) ; et
(c) appliquer une poche de liaison de l'ATP des coordonnées structurales de la figure 1 ou de la figure 2, ou une poche de liaison de la TAK1-TAB1 des coordonnées structurales de la figure 1 ou de la figure 2, au profil de diffraction des rayons X pour générer une carte de densité électronique tridimensionnelle d'au moins une partie de la molécule ou du complexe moléculaire de structure inconnue, où ladite poche de liaison de l'ATP comprend les acides aminés de V42, V90, M104, E105, A107 et L163 selon les figures 1 et 2, et où ladite poche de liaison de la TAK1-TAB1 comprend les acides aminés de M131, P256, Y290 et F291 selon les figures 1 et 2 ; et
(d) générer un modèle structural de la molécule ou du complexe moléculaire à partir de la carte de densité électronique tridimensionnelle.

Figure 1: Adenosine complex coordinates

```
ATOM      1   CB    SER A  27      -4.209  69.133  25.556  1.00 88.36        A
ATOM      2   OG    SER A  27      -4.103  68.406  26.766  1.00 88.64        A
ATOM      3   C     SER A  27      -1.747  69.172  25.425  1.00 88.35        A
ATOM      4   O     SER A  27      -1.661  68.887  26.613  1.00 88.23        A
ATOM      5   N     SER A  27      -3.051  67.398  24.266  1.00 88.43        A
ATOM      6   CA    SER A  27      -3.016  68.826  24.662  1.00 88.45        A
ATOM      7   N     LEU A  28      -0.769  69.770  24.747  1.00 88.52        A
ATOM      8   CA    LEU A  28       0.471  70.187  25.397  1.00 88.40        A
ATOM      9   CB    LEU A  28       0.998  69.111  26.363  1.00 88.69        A
ATOM     10   CG    LEU A  28       0.600  69.216  27.848  1.00 88.90        A
ATOM     11   CD1   LEU A  28       1.705  68.634  28.719  1.00 88.86        A
ATOM     12   CD2   LEU A  28       0.352  70.671  28.230  1.00 88.75        A
ATOM     13   C     LEU A  28       1.635  70.650  24.531  1.00 88.41        A
ATOM     14   O     LEU A  28       1.744  70.332  23.351  1.00 87.76        A
ATOM     15   N     HIS A  29       2.490  71.419  25.195  1.00 88.45        A
ATOM     16   CA    HIS A  29       3.723  72.020  24.701  1.00 88.87        A
ATOM     17   CB    HIS A  29       4.900  71.311  25.359  1.00 88.36        A
ATOM     18   CG    HIS A  29       6.010  72.231  25.749  1.00 88.21        A
ATOM     19   CD2   HIS A  29       6.390  72.702  26.961  1.00 88.15        A
ATOM     20   ND1   HIS A  29       6.861  72.805  24.830  1.00 88.19        A
ATOM     21   CE1   HIS A  29       7.718  73.591  25.458  1.00 88.06        A
ATOM     22   NE2   HIS A  29       7.453  73.546  26.752  1.00 88.15        A
ATOM     23   C     HIS A  29       4.027  72.186  23.222  1.00 89.28        A
ATOM     24   O     HIS A  29       3.824  71.284  22.417  1.00 89.64        A
ATOM     25   N     MET A  30       4.549  73.365  22.897  1.00 89.78        A
ATOM     26   CA    MET A  30       4.944  73.734  21.543  1.00 90.08        A
ATOM     27   CB    MET A  30       4.241  75.017  21.103  1.00 90.12        A
ATOM     28   CG    MET A  30       2.742  74.927  21.112  1.00 90.27        A
ATOM     29   SD    MET A  30       2.204  73.654  19.990  1.00 88.67        A
ATOM     30   CE    MET A  30       2.236  74.554  18.450  1.00 90.69        A
ATOM     31   C     MET A  30       6.440  74.000  21.592  1.00 90.58        A
ATOM     32   O     MET A  30       6.985  74.281  22.658  1.00 91.03        A
ATOM     33   N     ILE A  31       7.103  73.920  20.445  1.00 90.93        A
ATOM     34   CA    ILE A  31       8.540  74.162  20.384  1.00 91.03        A
ATOM     35   CB    ILE A  31       9.340  72.838  20.588  1.00 90.53        A
ATOM     36   CG2   ILE A  31      10.829  73.081  20.376  1.00 90.70        A
ATOM     37   CG1   ILE A  31       9.100  72.293  22.001  1.00 90.54        A
ATOM     38   CD1   ILE A  31       9.736  70.945  22.265  1.00 90.53        A
ATOM     39   C     ILE A  31       8.897  74.776  19.034  1.00 91.19        A
ATOM     40   O     ILE A  31       8.250  74.501  18.026  1.00 90.99        A
ATOM     41   N     ASP A  32       9.910  75.636  19.022  1.00 91.50        A
ATOM     42   CA    ASP A  32      10.353  76.270  17.784  1.00 91.71        A
ATOM     43   CB    ASP A  32      10.592  77.772  18.006  1.00 92.39        A
ATOM     44   CG    ASP A  32      11.622  78.048  19.084  1.00 92.97        A
ATOM     45   OD1   ASP A  32      11.520  77.455  20.185  1.00 93.11        A
ATOM     46   OD2   ASP A  32      12.528  78.871  18.832  1.00 93.34        A
ATOM     47   C     ASP A  32      11.630  75.569  17.318  1.00 91.08        A
ATOM     48   O     ASP A  32      12.607  75.457  18.064  1.00 90.73        A
ATOM     49   N     TYR A  33      11.599  75.090  16.080  1.00 90.66        A
ATOM     50   CA    TYR A  33      12.708  74.360  15.480  1.00 90.31        A
ATOM     51   CB    TYR A  33      12.455  74.219  13.979  1.00 90.51        A
```

37

| ATOM | 52  | CG  | TYR | A | 33 | 13.250 | 73.124 | 13.321 | 1.00 | 90.77 | A |
|------|-----|-----|-----|---|----|--------|--------|--------|------|-------|---|
| ATOM | 53  | CD1 | TYR | A | 33 | 13.231 | 71.824 | 13.824 | 1.00 | 90.98 | A |
| ATOM | 54  | CE1 | TYR | A | 33 | 13.955 | 70.806 | 13.210 | 1.00 | 91.23 | A |
| ATOM | 55  | CD2 | TYR | A | 33 | 14.012 | 73.383 | 12.186 | 1.00 | 91.00 | A |
| ATOM | 56  | CE2 | TYR | A | 33 | 14.738 | 72.377 | 11.564 | 1.00 | 91.24 | A |
| ATOM | 57  | CZ  | TYR | A | 33 | 14.707 | 71.092 | 12.080 | 1.00 | 91.30 | A |
| ATOM | 58  | OH  | TYR | A | 33 | 15.442 | 70.105 | 11.467 | 1.00 | 91.76 | A |
| ATOM | 59  | C   | TYR | A | 33 | 14.092 | 74.965 | 15.730 | 1.00 | 89.82 | A |
| ATOM | 60  | O   | TYR | A | 33 | 15.057 | 74.235 | 15.963 | 1.00 | 89.78 | A |
| ATOM | 61  | N   | LYS | A | 34 | 14.184 | 76.291 | 15.689 | 1.00 | 89.44 | A |
| ATOM | 62  | CA  | LYS | A | 34 | 15.446 | 76.994 | 15.900 | 1.00 | 89.16 | A |
| ATOM | 63  | CB  | LYS | A | 34 | 15.238 | 78.495 | 15.664 | 1.00 | 89.73 | A |
| ATOM | 64  | CG  | LYS | A | 34 | 13.995 | 79.047 | 16.342 | 1.00 | 90.54 | A |
| ATOM | 65  | CD  | LYS | A | 34 | 13.566 | 80.392 | 15.770 | 1.00 | 90.96 | A |
| ATOM | 66  | CE  | LYS | A | 34 | 12.195 | 80.797 | 16.311 | 1.00 | 91.30 | A |
| ATOM | 67  | NZ  | LYS | A | 34 | 11.691 | 82.063 | 15.712 | 1.00 | 91.56 | A |
| ATOM | 68  | C   | LYS | A | 34 | 16.074 | 76.746 | 17.276 | 1.00 | 88.53 | A |
| ATOM | 69  | O   | LYS | A | 34 | 17.215 | 77.139 | 17.518 | 1.00 | 88.17 | A |
| ATOM | 70  | N   | GLU | A | 35 | 15.336 | 76.091 | 18.170 | 1.00 | 88.11 | A |
| ATOM | 71  | CA  | GLU | A | 35 | 15.845 | 75.782 | 19.512 | 1.00 | 88.23 | A |
| ATOM | 72  | CB  | GLU | A | 35 | 14.779 | 76.070 | 20.581 | 1.00 | 89.78 | A |
| ATOM | 73  | CG  | GLU | A | 35 | 14.332 | 77.523 | 20.674 | 1.00 | 91.73 | A |
| ATOM | 74  | CD  | GLU | A | 35 | 15.479 | 78.492 | 20.911 | 1.00 | 92.88 | A |
| ATOM | 75  | OE1 | GLU | A | 35 | 16.101 | 78.437 | 21.996 | 1.00 | 93.62 | A |
| ATOM | 76  | OE2 | GLU | A | 35 | 15.759 | 79.312 | 20.005 | 1.00 | 93.59 | A |
| ATOM | 77  | C   | GLU | A | 35 | 16.274 | 74.316 | 19.619 | 1.00 | 87.13 | A |
| ATOM | 78  | O   | GLU | A | 35 | 16.642 | 73.842 | 20.696 | 1.00 | 86.73 | A |
| ATOM | 79  | N   | ILE | A | 36 | 16.223 | 73.616 | 18.488 | 1.00 | 86.18 | A |
| ATOM | 80  | CA  | ILE | A | 36 | 16.579 | 72.199 | 18.403 | 1.00 | 85.51 | A |
| ATOM | 81  | CB  | ILE | A | 36 | 15.429 | 71.379 | 17.768 | 1.00 | 85.68 | A |
| ATOM | 82  | CG2 | ILE | A | 36 | 15.850 | 69.920 | 17.605 | 1.00 | 85.47 | A |
| ATOM | 83  | CG1 | ILE | A | 36 | 14.165 | 71.500 | 18.620 | 1.00 | 85.35 | A |
| ATOM | 84  | CD1 | ILE | A | 36 | 12.956 | 70.821 | 18.002 | 1.00 | 85.47 | A |
| ATOM | 85  | C   | ILE | A | 36 | 17.824 | 71.979 | 17.543 | 1.00 | 84.94 | A |
| ATOM | 86  | O   | ILE | A | 36 | 17.860 | 72.381 | 16.377 | 1.00 | 84.77 | A |
| ATOM | 87  | N   | GLU | A | 37 | 18.832 | 71.320 | 18.107 | 1.00 | 84.09 | A |
| ATOM | 88  | CA  | GLU | A | 37 | 20.061 | 71.050 | 17.371 | 1.00 | 83.89 | A |
| ATOM | 89  | CB  | GLU | A | 37 | 21.275 | 71.281 | 18.279 | 1.00 | 84.87 | A |
| ATOM | 90  | CG  | GLU | A | 37 | 22.592 | 71.467 | 17.530 | 1.00 | 86.40 | A |
| ATOM | 91  | CD  | GLU | A | 37 | 23.627 | 70.407 | 17.876 | 1.00 | 87.45 | A |
| ATOM | 92  | OE1 | GLU | A | 37 | 23.926 | 70.239 | 19.084 | 1.00 | 87.92 | A |
| ATOM | 93  | OE2 | GLU | A | 37 | 24.143 | 69.748 | 16.937 | 1.00 | 88.18 | A |
| ATOM | 94  | C   | GLU | A | 37 | 20.049 | 69.608 | 16.855 | 1.00 | 82.91 | A |
| ATOM | 95  | O   | GLU | A | 37 | 20.483 | 68.692 | 17.546 | 1.00 | 83.03 | A |
| ATOM | 96  | N   | VAL | A | 38 | 19.542 | 69.412 | 15.642 | 1.00 | 81.56 | A |
| ATOM | 97  | CA  | VAL | A | 38 | 19.476 | 68.079 | 15.049 | 1.00 | 80.79 | A |
| ATOM | 98  | CB  | VAL | A | 38 | 18.845 | 68.125 | 13.635 | 1.00 | 80.61 | A |
| ATOM | 99  | CG1 | VAL | A | 38 | 18.855 | 66.739 | 13.007 | 1.00 | 80.34 | A |
| ATOM | 100 | CG2 | VAL | A | 38 | 17.424 | 68.660 | 13.718 | 1.00 | 80.56 | A |
| ATOM | 101 | C   | VAL | A | 38 | 20.863 | 67.448 | 14.947 | 1.00 | 80.04 | A |
| ATOM | 102 | O   | VAL | A | 38 | 21.862 | 68.143 | 14.770 | 1.00 | 80.76 | A |
| ATOM | 103 | N   | GLU | A | 39 | 20.915 | 66.126 | 15.065 | 1.00 | 79.24 | A |
| ATOM | 104 | CA  | GLU | A | 39 | 22.168 | 65.391 | 14.981 | 1.00 | 78.38 | A |
| ATOM | 105 | CB  | GLU | A | 39 | 22.625 | 64.988 | 16.386 | 1.00 | 79.32 | A |

| ATOM | 106 | CG | GLU A | 39 | 23.331 | 66.126 | 17.134 | 1.00 | 81.14 | A |
|------|-----|-----|-------|-----|--------|--------|--------|------|-------|---|
| ATOM | 107 | CD | GLU A | 39 | 23.127 | 66.084 | 18.646 | 1.00 | 82.22 | A |
| ATOM | 108 | OE1 | GLU A | 39 | 23.215 | 64.988 | 19.245 | 1.00 | 83.01 | A |
| ATOM | 109 | OE2 | GLU A | 39 | 22.892 | 67.158 | 19.242 | 1.00 | 82.77 | A |
| ATOM | 110 | C | GLU A | 39 | 22.011 | 64.174 | 14.067 | 1.00 | 77.06 | A |
| ATOM | 111 | O | GLU A | 39 | 21.204 | 64.199 | 13.133 | 1.00 | 77.06 | A |
| ATOM | 112 | N | GLU A | 40 | 22.765 | 63.111 | 14.325 | 1.00 | 75.78 | A |
| ATOM | 113 | CA | GLU A | 40 | 22.698 | 61.928 | 13.473 | 1.00 | 74.51 | A |
| ATOM | 114 | CB | GLU A | 40 | 23.776 | 60.906 | 13.867 | 1.00 | 76.10 | A |
| ATOM | 115 | CG | GLU A | 40 | 23.511 | 60.164 | 15.169 | 1.00 | 77.95 | A |
| ATOM | 116 | CD | GLU A | 40 | 23.944 | 60.948 | 16.393 | 1.00 | 78.99 | A |
| ATOM | 117 | OE1 | GLU A | 40 | 24.165 | 62.173 | 16.272 | 1.00 | 79.62 | A |
| ATOM | 118 | OE2 | GLU A | 40 | 24.051 | 60.342 | 17.483 | 1.00 | 79.53 | A |
| ATOM | 119 | C | GLU A | 40 | 21.348 | 61.225 | 13.433 | 1.00 | 73.02 | A |
| ATOM | 120 | O | GLU A | 40 | 20.622 | 61.166 | 14.424 | 1.00 | 72.24 | A |
| ATOM | 121 | N | VAL A | 41 | 21.020 | 60.691 | 12.264 | 1.00 | 71.66 | A |
| ATOM | 122 | CA | VAL A | 41 | 19.779 | 59.962 | 12.088 | 1.00 | 70.62 | A |
| ATOM | 123 | CB | VAL A | 41 | 19.555 | 59.588 | 10.612 | 1.00 | 70.41 | A |
| ATOM | 124 | CG1 | VAL A | 41 | 18.301 | 58.738 | 10.466 | 1.00 | 70.23 | A |
| ATOM | 125 | CG2 | VAL A | 41 | 19.443 | 60.848 | 9.779 | 1.00 | 70.27 | A |
| ATOM | 126 | C | VAL A | 41 | 19.892 | 58.688 | 12.912 | 1.00 | 70.15 | A |
| ATOM | 127 | O | VAL A | 41 | 20.941 | 58.032 | 12.917 | 1.00 | 70.54 | A |
| ATOM | 128 | N | VAL A | 42 | 18.812 | 58.348 | 13.609 | 1.00 | 69.46 | A |
| ATOM | 129 | CA | VAL A | 42 | 18.783 | 57.159 | 14.446 | 1.00 | 68.48 | A |
| ATOM | 130 | CB | VAL A | 42 | 18.376 | 57.515 | 15.901 | 1.00 | 67.75 | A |
| ATOM | 131 | CG1 | VAL A | 42 | 16.873 | 57.423 | 16.069 | 1.00 | 67.42 | A |
| ATOM | 132 | CG2 | VAL A | 42 | 19.089 | 56.606 | 16.877 | 1.00 | 67.84 | A |
| ATOM | 133 | C | VAL A | 42 | 17.815 | 56.122 | 13.879 | 1.00 | 67.80 | A |
| ATOM | 134 | O | VAL A | 42- | 17.652 | 55.046 | 14.443 | 1.00 | 67.91 | A |
| ATOM | 135 | N | GLY A | 43 | 17.174 | 56.448 | 12.761 | 1.00 | 67.18 | A |
| ATOM | 136 | CA | GLY A | 43 | 16.253 | 55.506 | 12.158 | 1.00 | 66.51 | A |
| ATOM | 137 | C | GLY A | 43 | 15.041 | 56.110 | 11.483 | 1.00 | 66.88 | A |
| ATOM | 138 | O | GLY A | 43 | 14.784 | 57.310 | 11.583 | 1.00 | 66.23 | A |
| ATOM | 139 | N | ARG A | 44 | 14.297 | 55.256 | 10.787 | 1.00 | 67.19 | A |
| ATOM | 140 | CA | ARG A | 44 | 13.083 | 55.654 | 10.083 | 1.00 | 68.03 | A |
| ATOM | 141 | CB | ARG A | 44 | 13.358 | 55.782 | 8.584 | 1.00 | 70.32 | A |
| ATOM | 142 | CG | ARG A | 44 | 14.391 | 56.839 | 8.230 | 1.00 | 73.12 | A |
| ATOM | 143 | CD | ARG A | 44 | 14.909 | 56.652 | 6.811 | 1.00 | 75.10 | A |
| ATOM | 144 | NE | ARG A | 44 | 15.405 | 55.295 | 6.575 | 1.00 | 77.46 | A |
| ATOM | 145 | CZ | ARG A | 44 | 16.397 | 54.718 | 7.251 | 1.00 | 79.02 | A |
| ATOM | 146 | NH1 | ARG A | 44 | 17.023 | 55.377 | 8.220 | 1.00 | 79.82 | A |
| ATOM | 147 | NH2 | ARG A | 44 | 16.754 | 53.471 | 6.966 | 1.00 | 79.87 | A |
| ATOM | 148 | C | ARG A | 44 | 12.004 | 54.598 | 10.318 | 1.00 | 67.74 | A |
| ATOM | 149 | O | ARG A | 44 | 12.228 | 53.412 | 10.088 | 1.00 | 68.09 | A |
| ATOM | 150 | N | GLY A | 45 | 10.839 | 55.033 | 10.788 | 1.00 | 67.20 | A |
| ATOM | 151 | CA | GLY A | 45 | 9.749 | 54.109 | 11.050 | 1.00 | 67.08 | A |
| ATOM | 152 | C | GLY A | 45 | 8.687 | 54.142 | 9.967 | 1.00 | 66.80 | A |
| ATOM | 153 | O | GLY A | 45 | 9.012 | 54.294 | 8.789 | 1.00 | 66.08 | A |
| ATOM | 154 | N | ALA A | 46 | 7.422 | 54.007 | 10.362 | 1.00 | 66.78 | A |
| ATOM | 155 | CA | ALA A | 46 | 6.302 | 54.022 | 9.420 | 1.00 | 65.92 | A |
| ATOM | 156 | CB | ALA A | 46 | 4.975 | 54.055 | 10.178 | 1.00 | 66.34 | A |
| ATOM | 157 | C | ALA A | 46 | 6.375 | 55.197 | 8.438 | 1.00 | 66.18 | A |
| ATOM | 158 | O | ALA A | 46 | 6.213 | 55.003 | 7.231 | 1.00 | 66.12 | A |
| ATOM | 159 | N | PHE A | 47 | 6.596 | 56.411 | 8.944 | 1.00 | 66.22 | A |

| ATOM | 160 | CA | PHE | A | 47 | 6.699 | 57.562 | 8.056 | 1.00 | 65.52 | A |
|------|-----|-----|-----|---|-----|--------|--------|--------|------|-------|---|
| ATOM | 161 | CB | PHE | A | 47 | 5.404 | 58.401 | 8.077 | 1.00 | 66.25 | A |
| ATOM | 162 | CG | PHE | A | 47 | 4.745 | 58.528 | 9.427 | 1.00 | 65.92 | A |
| ATOM | 163 | CD1 | PHE | A | 47 | 5.215 | 59.436 | 10.370 | 1.00 | 66.29 | A |
| ATOM | 164 | CD2 | PHE | A | 47 | 3.615 | 57.771 | 9.734 | 1.00 | 65.77 | A |
| ATOM | 165 | CE1 | PHE | A | 47 | 4.568 | 59.593 | 11.601 | 1.00 | 66.10 | A |
| ATOM | 166 | CE2 | PHE | A | 47 | 2.962 | 57.920 | 10.961 | 1.00 | 65.92 | A |
| ATOM | 167 | CZ | PHE | A | 47 | 3.440 | 58.833 | 11.895 | 1.00 | 66.20 | A |
| ATOM | 168 | C | PHE | A | 47 | 7.937 | 58.461 | 8.200 | 1.00 | 65.23 | A |
| ATOM | 169 | O | PHE | A | 47 | 8.944 | 58.205 | 7.537 | 1.00 | 66.18 | A |
| ATOM | 170 | N | GLY | A | 48 | 7.881 | 59.494 | 9.043 | 1.00 | 64.05 | A |
| ATOM | 171 | CA | GLY | A | 48 | 9.015 | 60.406 | 9.207 | 1.00 | 61.33 | A |
| ATOM | 172 | C | GLY | A | 48 | 10.409 | 59.813 | 9.391 | 1.00 | 60.25 | A |
| ATOM | 173 | O | GLY | A | 48 | 10.669 | 58.662 | 9.045 | 1.00 | 60.17 | A |
| ATOM | 174 | N | VAL | A | 49 | 11.325 | 60.615 | 9.921 | 1.00 | 59.53 | A |
| ATOM | 175 | CA | VAL | A | 49 | 12.688 | 60.154 | 10.172 | 1.00 | 58.33 | A |
| ATOM | 176 | CB | VAL | A | 49 | 13.694 | 60.779 | 9.170 | 1.00 | 58.37 | A |
| ATOM | 177 | CG1 | VAL | A | 49 | 13.686 | 62.293 | 9.295 | 1.00 | 58.27 | A |
| ATOM | 178 | CG2 | VAL | A | 49 | 15.095 | 60.226 | 9.418 | 1.00 | 58.43 | A |
| ATOM | 179 | C | VAL | A | 49 | 13.088 | 60.549 | 11.598 | 1.00 | 57.48 | A |
| ATOM | 180 | O | VAL | A | 49 | 12.937 | 61.709 | 11.993 | 1.00 | 58.34 | A |
| ATOM | 181 | N | VAL | A | 50 | 13.588 | 59.592 | 12.377 | 1.00 | 56.22 | A |
| ATOM | 182 | CA | VAL | A | 50 | 13.984 | 59.889 | 13.745 | 1.00 | 54.79 | A |
| ATOM | 183 | CB | VAL | A | 50 | 13.672 | 58.714 | 14.688 | 1.00 | 53.82 | A |
| ATOM | 184 | CG1 | VAL | A | 50 | 13.985 | 59.111 | 16.125 | 1.00 | 52.85 | A |
| ATOM | 185 | CG2 | VAL | A | 50 | 12.214 | 58.305 | 14.545 | 1.00 | 52.83 | A |
| ATOM | 186 | C | VAL | A | 50 | 15.465 | 60.199 | 13.824 | 1.00 | 55.45 | A |
| ATOM | 187 | O | VAL | A | 50 | 16.285 | 59.459 | 13.295 | 1.00 | 55.12 | A |
| ATOM | 188 | N | CYS | A | 51 | 15.800 | 61.303 | 14.481 | 1.00 | 56.94 | A |
| ATOM | 189 | CA | CYS | A | 51 | 17.191 | 61.709 | 14.630 | 1.00 | 58.81 | A |
| ATOM | 190 | CB | CYS | A | 51 | 17.471 | 63.000 | 13.860 | 1.00 | 60.04 | A |
| ATOM | 191 | SG | CYS | A | 51 | 16.865 | 63.037 | 12.175 | 1.00 | 63.48 | A |
| ATOM | 192 | C | CYS | A | 51 | 17.512 | 61.980 | 16.086 | 1.00 | 60.28 | A |
| ATOM | 193 | O | CYS | A | 51 | 16.618 | 62.208 | 16.895 | 1.00 | 59.15 | A |
| ATOM | 194 | N | LYS | A | 52 | 18.794 | 61.949 | 16.421 | 1.00 | 62.41 | A |
| ATOM | 195 | CA | LYS | A | 52 | 19.220 | 62.258 | 17.776 | 1.00 | 65.05 | A |
| ATOM | 196 | CB | LYS | A | 52 | 20.604 | 61.660 | 18.030 | 1.00 | 65.83 | A |
| ATOM | 197 | CG | LYS | A · | 52 | 21.264 | 62.098 | 19.319 | 1.00 | 67.13 | A |
| ATOM | 198 | CD | LYS | A | 52 | 20.471 | 61.690 | 20.533 | 1.00 | 68.48 | A |
| ATOM | 199 | CE | LYS | A | 52 | 21.227 | 62.052 | 21.798 | 1.00 | 69.33 | A |
| ATOM | 200 | NZ | LYS | A | 52 | 22.524 | 61.325 | 21.878 | 1.00 | 69.90 | A |
| ATOM | 201 | C | LYS | A | 52 | 19.258 | 63.795 | 17.795 | 1.00 | 66.03 | A |
| ATOM | 202 | O | LYS | A | 52 | 19.380 | 64.419 | 16.744 | 1.00 | 65.72 | A |
| ATOM | 203 | N | ALA | A | 53 | 19.130 | 64.413 | 18.964 | 1.00 | 68.21 | A |
| ATOM | 204 | CA | ALA | A | 53 | 19.146 | 65.871 | 19.024 | 1.00 | 70.67 | A |
| ATOM | 205 | CB | ALA | A | 53 | 17.893 | 66.431 | 18.348 | 1.00 | 69.92 | A |
| ATOM | 206 | C | ALA | A | 53 | 19.253 | 66.414 | 20.448 | 1.00 | 72.39 | A |
| ATOM | 207 | O | ALA | A | 53 | 19.309 | 65.645 | 21.414 | 1.00 | 73.01 | A |
| ATOM | 208 | N | LYS | A | 54 | 19.300 | 67.743 | 20.560 | 1.00 | 75.34 | A |
| ATOM | 209 | CA | LYS | A | 54 | 19.378 | 68.434 | 21.848 | 1.00 | 77.47 | A |
| ATOM | 210 | CB | LYS | A | 54 | 20.766 | 69.040 | 22.068 | 1.00 | 78.36 | A |
| ATOM | 211 | CG | LYS | A | 54 | 21.848 | 68.008 | 22.303 | 1.00 | 79.52 | A |
| ATOM | 212 | CD | LYS | A | 54 | 23.156 | 68.652 | 22.720 | 1.00 | 80.53 | A |
| ATOM | 213 | CE | LYS | A | 54 | 24.200 | 67.591 | 23.045 | 1.00 | 81.28 | A |

| ATOM | 214 | NZ | LYS | A | 54 | 25.461 | 68.198 | 23.554 | 1.00 | 81.73 | A |
|------|-----|-----|-----|---|----|--------|--------|--------|------|-------|---|
| ATOM | 215 | C | LYS | A | 54 | 18.336 | 69.538 | 21.873 | 1.00 | 78.62 | A |
| ATOM | 216 | O | LYS | A | 54 | 18.000 | 70.106 | 20.838 | 1.00 | 78.95 | A |
| ATOM | 217 | N | TRP | A | 55 | 17.824 | 69.843 | 23.057 | 1.00 | 80.46 | A |
| ATOM | 218 | CA | TRP | A | 55 | 16.807 | 70.876 | 23.197 | 1.00 | 81.81 | A |
| ATOM | 219 | CB | TRP | A | 55 | 15.446 | 70.316 | 22.798 | 1.00 | 82.63 | A |
| ATOM | 220 | CG | TRP | A | 55 | 14.302 | 71.251 | 23.027 | 1.00 | 83.45 | A |
| ATOM | 221 | CD2 | TRP | A | 55 | 13.275 | 71.114 | 24.014 | 1.00 | 84.23 | A |
| ATOM | 222 | CE2 | TRP | A | 55 | 12.389 | 72.205 | 23.852 | 1.00 | 84.21 | A |
| ATOM | 223 | CE3 | TRP | A | 55 | 13.016 | 70.172 | 25.020 | 1.00 | 84.30 | A |
| ATOM | 224 | CD1 | TRP | A | 55 | 14.011 | 72.390 | 22.327 | 1.00 | 83.88 | A |
| ATOM | 225 | NE1 | TRP | A | 55 | 12.861 | 72.968 | 22.816 | 1.00 | 84.07 | A |
| ATOM | 226 | CZ2 | TRP | A | 55 | 11.262 | 72.379 | 24.660 | 1.00 | 84.25 | A |
| ATOM | 227 | CZ3 | TRP | A | 55 | 11.893 | 70.347 | 25.824 | 1.00 | 84.38 | A |
| ATOM | 228 | CH2 | TRP | A | 55 | 11.031 | 71.443 | 25.637 | 1.00 | 84.53 | A |
| ATOM | 229 | C | TRP | A | 55 | 16.775 | 71.332 | 24.642 | 1.00 | 82.52 | A |
| ATOM | 230 | O | TRP | A | 55 | 16.359 | 70.582 | 25.524 | 1.00 | 82.59 | A |
| ATOM | 231 | N | ARG | A | 56 | 17.207 | 72.567 | 24.877 | 1.00 | 83.27 | A |
| ATOM | 232 | CA | ARG | A | 56 | 17.262 | 73.115 | 26.228 | 1.00 | 84.24 | A |
| ATOM | 233 | CB | ARG | A | 56 | 15.889 | 73.058 | 26.907 | 1.00 | 84.72 | A |
| ATOM | 234 | CG | ARG | A | 56 | 14.874 | 74.013 | 26.319 | 1.00 | 85.69 | A |
| ATOM | 235 | CD | ARG | A | 56 | 13.622 | 74.063 | 27.169 | 1.00 | 86.73 | A |
| ATOM | 236 | NE | ARG | A | 56 | 12.590 | 74.901 | 26.565 | 1.00 | 88.07 | A |
| ATOM | 237 | CZ | ARG | A | 56 | 11.394 | 75.120 | 27.105 | 1.00 | 88.73 | A |
| ATOM | 238 | NH1 | ARG | A | 56 | 11.078 | 74.561 | 28.268 | 1.00 | 89.00 | A |
| ATOM | 239 | NH2 | ARG | A | 56 | 10.511 | 75.892 | 26.482 | 1.00 | 89.04 | A |
| ATOM | 240 | C | ARG | A | 56 | 18.276 | 72.324 | 27.049 | 1.00 | 84.32 | A |
| ATOM | 241 | O | ARG | A | 56 | 18.069 | 72.070 | 28.240 | 1.00 | 84.63 | A |
| ATOM | 242 | N | ALA | A | 57 | 19.370 | 71.938 | 26.392 | 1.00 | 84.68 | A |
| ATOM | 243 | CA | ALA | A | 57 | 20.458 | 71.179 | 27.013 | 1.00 | 84.65 | A |
| ATOM | 244 | CB | ALA | A | 57 | 21.010 | 71.942 | 28.219 | 1.00 | 84.78 | A |
| ATOM | 245 | C | ALA | A | 57 | 20.045 | 69.769 | 27.430 | 1.00 | 84.50 | A |
| ATOM | 246 | O | ALA | A | 57 | 20.763 | 69.100 | 28.183 | 1.00 | 84.94 | A |
| ATOM | 247 | N | LYS | A | 58 | 18.887 | 69.326 | 26.941 | 1.00 | 84.10 | A |
| ATOM | 248 | CA | LYS | A | 58 | 18.371 | 67.992 | 27.247 | 1.00 | 83.31 | A |
| ATOM | 249 | CB | LYS | A | 58 | 16.902 | 68.056 | 27.696 | 1.00 | 84.01 | A |
| ATOM | 250 | CG | LYS | A | 58 | 16.502 | 69.299 | 28.492 | 1.00 | 85.01 | A |
| ATOM | 251 | CD | LYS | A | 58 | 17.217 | 69.418 | 29.834 | 1.00 | 85.61 | A |
| ATOM | 252 | CE | LYS | A | 58 | 16.771 | 70.687 | 30.561 | 1.00 | 86.10 | A |
| ATOM | 253 | NZ | LYS | A | 58 | 17.408 | 70.863 | 31.897 | 1.00 | 86.46 | A |
| ATOM | 254 | C | LYS | A | 58 | 18.458 | 67.151 | 25.972 | 1.00 | 81.89 | A |
| ATOM | 255 | O | LYS | A | 58 | 18.090 | 67.621 | 24.889 | 1.00 | 81.55 | A |
| ATOM | 256 | N | ASP | A | 59 | 18.956 | 65.921 | 26.092 | 1.00 | 80.70 | A |
| ATOM | 257 | CA | ASP | A | 59 | 19.046 | 65.051 | 24.926 | 1.00 | 79.36 | A |
| ATOM | 258 | CB | ASP | A | 59 | 19.927 | 63.835 | 25.200 | 1.00 | 80.96 | A |
| ATOM | 259 | CG | ASP | A | 59 | 21.354 | 64.056 | 24.753 | 1.00 | 82.77 | A |
| ATOM | 260 | OD1 | ASP | A | 59 | 21.556 | 64.376 | 23.554 | 1.00 | 83.57 | A |
| ATOM | 261 | OD2 | ASP | A | 59 | 22.268 | 63.916 | 25.598 | 1.00 | 83.72 | A |
| ATOM | 262 | C | ASP | A | 59 | 17.650 | 64.615 | 24.539 | 1.00 | 76.63 | A |
| ATOM | 263 | O | ASP | A | 59 | 16.858 | 64.185 | 25.378 | 1.00 | 75.80 | A |
| ATOM | 264 | N | VAL | A | 60 | 17.356 | 64.740 | 23.255 | 1.00 | 73.60 | A |
| ATOM | 265 | CA | VAL | A | 60 | 16.050 | 64.408 | 22.744 | 1.00 | 70.87 | A |
| ATOM | 266 | CB | VAL | A | 60 | 15.272 | 65.715 | 22.468 | 1.00 | 70.72 | A |
| ATOM | 267 | CG1 | VAL | A | 60 | 13.925 | 65.417 | 21.883 | 1.00 | 71.01 | A |

| ATOM | 268 | CG2 | VAL | A | 60 | 15.114 | 66.500 | 23.754 | 1.00 | 70.41 | A |
|------|-----|-----|-----|---|----|--------|--------|--------|------|-------|---|
| ATOM | 269 | C   | VAL | A | 60 | 16.152 | 63.576 | 21.471 | 1.00 | 68.78 | A |
| ATOM | 270 | O   | VAL | A | 60 | 17.243 | 63.325 | 20.960 | 1.00 | 68.60 | A |
| ATOM | 271 | N   | ALA | A | 61 | 15.000 | 63.122 | 20.994 | 1.00 | 67.36 | A |
| ATOM | 272 | CA  | ALA | A | 61 | 14.892 | 62.349 | 19.769 | 1.00 | 66.08 | A |
| ATOM | 273 | CB  | ALA | A | 61 | 14.539 | 60.909 | 20.075 | 1.00 | 65.65 | A |
| ATOM | 274 | C   | ALA | A | 61 | 13.748 | 63.030 | 19.037 | 1.00 | 64.91 | A |
| ATOM | 275 | O   | ALA | A | 61 | 12.741 | 63.370 | 19.651 | 1.00 | 64.93 | A |
| ATOM | 276 | N   | ILE | A | 62 | 13.902 | 63.250 | 17.738 | 1.00 | 64.69 | A |
| ATOM | 277 | CA  | ILE | A | 62 | 12.862 | 63.917 | 16.974 | 1.00 | 64.61 | A |
| ATOM | 278 | CB  | ILE | A | 62 | 13.235 | 65.387 | 16.657 | 1.00 | 64.69 | A |
| ATOM | 279 | CG2 | ILE | A | 62 | 13.690 | 66.103 | 17.910 | 1.00 | 64.02 | A |
| ATOM | 280 | CG1 | ILE | A | 62 | 14.355 | 65.427 | 15.618 | 1.00 | 64.76 | A |
| ATOM | 281 | CD1 | ILE | A | 62 | 14.542 | 66.779 | 14.993 | 1.00 | 65.10 | A |
| ATOM | 282 | C   | ILE | A | 62 | 12.592 | 63.245 | 15.645 | 1.00 | 64.87 | A |
| ATOM | 283 | O   | ILE | A | 62 | 13.515 | 62.812 | 14.958 | 1.00 | 64.13 | A |
| ATOM | 284 | N   | LYS | A | 63 | 11.320 | 63.156 | 15.283 | 1.00 | 66.17 | A |
| ATOM | 285 | CA  | LYS | A | 63 | 10.959 | 62.591 | 13.999 | 1.00 | 68.24 | A |
| ATOM | 286 | CB  | LYS | A | 63 | 10.052 | 61.371 | 14.157 | 1.00 | 68.25 | A |
| ATOM | 287 | CG  | LYS | A | 63 | 8.786  | 61.606 | 14.919 | 1.00 | 68.65 | A |
| ATOM | 288 | CD  | LYS | A | 63 | 7.950  | 60.350 | 14.926 | 1.00 | 69.33 | A |
| ATOM | 289 | CE  | LYS | A | 63 | 7.652  | 59.891 | 13.514 | 1.00 | 69.35 | A |
| ATOM | 290 | NZ  | LYS | A | 63 | 6.817  | 58.667 | 13.529 | 1.00 | 69.06 | A |
| ATOM | 291 | C   | LYS | A | 63 | 10.255 | 63.702 | 13.236 | 1.00 | 69.72 | A |
| ATOM | 292 | O   | LYS | A | 63 | 9.345  | 64.341 | 13.758 | 1.00 | 69.52 | A |
| ATOM | 293 | N   | GLN | A | 64 | 10.706 | 63.946 | 12.010 | 1.00 | 71.93 | A |
| ATOM | 294 | CA  | GLN | A | 64 | 10.148 | 64.992 | 11.156 | 1.00 | 74.00 | A |
| ATOM | 295 | CB  | GLN | A | 64 | 11.241 | 65.973 | 10.765 | 1.00 | 75.01 | A |
| ATOM | 296 | CG  | GLN | A | 64 | 12.028 | 66.522 | 11.925 | 1.00 | 75.92 | A |
| ATOM | 297 | CD  | GLN | A | 64 | 13.312 | 67.170 | 11.470 | 1.00 | 76.16 | A |
| ATOM | 298 | OE1 | GLN | A | 64 | 14.313 | 66.490 | 11.215 | 1.00 | 76.56 | A |
| ATOM | 299 | NE2 | GLN | A | 64 | 13.290 | 68.494 | 11.346 | 1.00 | 76.80 | A |
| ATOM | 300 | C   | GLN | A | 64 | 9.608  | 64.365 | 9.892  | 1.00 | 76.07 | A |
| ATOM | 301 | O   | GLN | A | 64 | 9.857  | 63.187 | 9.625  | 1.00 | 75.82 | A |
| ATOM | 302 | N   | ILE | A | 65 | 8.873  | 65.142 | 9.104  | 1.00 | 78.24 | A |
| ATOM | 303 | CA  | ILE | A | 65 | 8.358  | 64.594 | 7.861  | 1.00 | 80.46 | A |
| ATOM | 304 | CB  | ILE | A | 65 | 7.029  | 65.244 | 7.395  | 1.00 | 80.88 | A |
| ATOM | 305 | CG2 | ILE | A | 65 | 5.935  | 64.976 | 8.389  | 1.00 | 81.69 | A |
| ATOM | 306 | CG1 | ILE | A | 65 | 7.216  | 66.741 | 7.182  | 1.00 | 81.47 | A |
| ATOM | 307 | CD1 | ILE | A | 65 | 5.987  | 67.402 | 6.601  | 1.00 | 82.04 | A |
| ATOM | 308 | C   | ILE | A | 65 | 9.386  | 64.810 | 6.767  | 1.00 | 81.42 | A |
| ATOM | 309 | O   | ILE | A | 65 | 10.264 | 65.667 | 6.881  | 1.00 | 81.28 | A |
| ATOM | 310 | N   | GLU | A | 66 | 9.264  | 64.004 | 5.720  | 1.00 | 82.59 | A |
| ATOM | 311 | CA  | GLU | A | 66 | 10.135 | 64.064 | 4.557  | 1.00 | 83.82 | A |
| ATOM | 312 | CB  | GLU | A | 66 | 10.979 | 62.790 | 4.459  | 1.00 | 83.71 | A |
| ATOM | 313 | CG  | GLU | A | 66 | 11.820 | 62.496 | 5.686  | 1.00 | 83.72 | A |
| ATOM | 314 | CD  | GLU | A | 66 | 12.786 | 61.347 | 5.460  | 1.00 | 83.77 | A |
| ATOM | 315 | OE1 | GLU | A | 66 | 12.331 | 60.242 | 5.094  | 1.00 | 83.79 | A |
| ATOM | 316 | OE2 | GLU | A | 66 | 14.004 | 61.551 | 5.647  | 1.00 | 83.90 | A |
| ATOM | 317 | C   | GLU | A | 66 | 9.201  | 64.171 | 3.351  | 1.00 | 84.76 | A |
| ATOM | 318 | O   | GLU | A | 66 | 9.091  | 63.238 | 2.544  | 1.00 | 85.24 | A |
| ATOM | 319 | N   | SER | A | 67 | 8.527  | 65.318 | 3.261  | 1.00 | 85.41 | A |
| ATOM | 320 | CA  | SER | A | 67 | 7.557  | 65.626 | 2.207  | 1.00 | 86.26 | A |
| ATOM | 321 | CB  | SER | A | 67 | 7.762  | 64.742 | 0.964  | 1.00 | 86.73 | A |

| ATOM | 322 | OG | SER | A | 67 | 6.788 | 65.003 | -0.035 | 1.00 | 87.42 | A |
| ATOM | 323 | C | SER | A | 67 | 6.167 | 65.390 | 2.778 | 1.00 | 86.10 | A |
| ATOM | 324 | O | SER | A | 67 | 5.920 | 64.362 | 3.413 | 1.00 | 85.88 | A |
| ATOM | 325 | N | GLU | A | 68 | 5.269 | 66.349 | 2.568 | 1.00 | 86.08 | A |
| ATOM | 326 | CA | GLU | A | 68 | 3.905 | 66.222 | 3.060 | 1.00 | 85.87 | A |
| ATOM | 327 | CB | GLU | A | 68 | 3.055 | 67.416 | 2.631 | 1.00 | 86.95 | A |
| ATOM | 328 | CG | GLU | A | 68 | 3.365 | 68.680 | 3.413 | 1.00 | 88.71 | A |
| ATOM | 329 | CD | GLU | A | 68 | 2.272 | 69.726 | 3.294 | 1.00 | 89.58 | A |
| ATOM | 330 | OE1 | GLU | A | 68 | 1.090 | 69.375 | 3.520 | 1.00 | 90.22 | A |
| ATOM | 331 | OE2 | GLU | A | 68 | 2.592 | 70.898 | 2.988 | 1.00 | 90.16 | A |
| ATOM | 332 | C | GLU | A | 68 | 3.295 | 64.931 | 2.550 | 1.00 | 84.88 | A |
| ATOM | 333 | O | GLU | A | 68 | 3.791 | 64.341 | 1.591 | 1.00 | 85.07 | A |
| ATOM | 334 | N | SER | A | 69 | 2.212 | 64.522 | 3.201 | 1.00 | 83.45 | A |
| ATOM | 335 | CA | SER | A | 69 | 1.487 | 63.282 | 2.927 | 1.00 | 82.36 | A |
| ATOM | 336 | CB | SER | A | 69 | 2.175 | 62.422 | 1.856 | 1.00 | 82.20 | A |
| ATOM | 337 | OG | SER | A | 69 | 3.438 | 61.945 | 2.300 | 1.00 | 82.14 | A |
| ATOM | 338 | C | SER | A | 69 | 1.622 | 62.616 | 4.283 | 1.00 | 81.74 | A |
| ATOM | 339 | O | SER | A | 69 | 0.715 | 61.934 | 4.767 | 1.00 | 81.53 | A |
| ATOM | 340 | N | GLU | A | 70 | 2.788 | 62.842 | 4.885 | 1.00 | 81.08 | A |
| ATOM | 341 | CA | GLU | A | 70 | 3.093 | 62.336 | 6.212 | 1.00 | 80.49 | A |
| ATOM | 342 | CB | GLU | A | 70 | 4.607 | 62.256 | 6.443 | 1.00 | 80.32 | A |
| ATOM | 343 | CG | GLU | A | 70 | 5.356 | 61.389 | 5.432 | 1.00 | 80.35 | A |
| ATOM | 344 | CD | GLU | A | 70 | 6.814 | 61.171 | 5.812 | 1.00 | 80.46 | A |
| ATOM | 345 | OE1 | GLU | A | 70 | 7.432 | 62.102 | 6.370 | 1.00 | 80.74 | A |
| ATOM | 346 | OE2 | GLU | A | 70 | 7.350 | 60.075 | 5.540 | 1.00 | 80.17 | A |
| ATOM | 347 | C | GLU | A | 70 | 2.487 | 63.392 | 7.118 | 1.00 | 79.79 | A |
| ATOM | 348 | O | GLU | A | 70 | 2.429 | 63.235 | 8.335 | 1.00 | 79.94 | A |
| ATOM | 349 | N | ARG | A | 71 | 2.037 | 64.476 | 6.493 | 1.00 | 79.36 | A |
| ATOM | 350 | CA | ARG | A | 71 | 1.408 | 65.583 | 7.197 | 1.00 | 79.06 | A |
| ATOM | 351 | CB | ARG | A | 71 | 1.039 | 66.695 | 6.213 | 1.00 | 80.74 | A |
| ATOM | 352 | CG | ARG | A | 71 | 0.257 | 67.836 | 6.850 | 1.00 | 82.84 | A |
| ATOM | 353 | CD | ARG | A | 71 | 1.154 | 68.751 | 7.664 | 1.00 | 84.85 | A |
| ATOM | 354 | NE | ARG | A | 71 | 1.587 | 69.897 | 6.873 | 1.00 | 86.95 | A |
| ATOM | 355 | CZ | ARG | A | 71 | 2.382 | 70.861 | 7.321 | 1.00 | 88.12 | A |
| ATOM | 356 | NH1 | ARG | A | 71 | 2.845 | 70.821 | 8.565 | 1.00 | 89.06 | A |
| ATOM | 357 | NH2 | ARG | A | 71 | 2.702 | 71.875 | 6.526 | 1.00 | 88.91 | A |
| ATOM | 358 | C | ARG | A | 71 | 0.146 | 65.089 | 7.903 | 1.00 | 77.52 | A |
| ATOM | 359 | O | ARG | A | 71 | -0.061 | 65.361 | 9.084 | 1.00 | 76.86 | A |
| ATOM | 360 | N | LYS | A | 72 | -0.701 | 64.375 | 7.170 | 1.00 | 76.31 | A |
| ATOM | 361 | CA | LYS | A | 72 | -1.929 | 63.842 | 7.743 | 1.00 | 75.67 | A |
| ATOM | 362 | CB | LYS | A | 72 | -2.715 | 63.074 | 6.675 | 1.00 | 76.83 | A |
| ATOM | 363 | CG | LYS | A | 72 | -3.599 | 63.939 | 5.779 | 1.00 | 78.25 | A |
| ATOM | 364 | CD | LYS | A | 72 | -4.890 | 64.362 | 6.490 | 1.00 | 79.04 | A |
| ATOM | 365 | CE | LYS | A | 72 | -5.858 | 65.086 | 5.542 | 1.00 | 79.82 | A |
| ATOM | 366 | NZ | LYS | A | 72 | -7.184 | 65.416 | 6.169 | 1.00 | 80.17 | A |
| ATOM | 367 | C | LYS | A | 72 | -1.614 | 62.918 | 8.924 | 1.00 | 74.29 | A |
| ATOM | 368 | O | LYS | A | 72 | -2.119 | 63.109 | 10.031 | 1.00 | 73.88 | A |
| ATOM | 369 | N | ALA | A | 73 | -0.773 | 61.919 | 8.675 | 1.00 | 73.07 | A |
| ATOM | 370 | CA | ALA | A | 73 | -0.382 | 60.953 | 9.696 | 1.00 | 71.95 | A |
| ATOM | 371 | CB | ALA | A | 73 | 0.578 | 59.930 | 9.098 | 1.00 | 71.51 | A |
| ATOM | 372 | C | ALA | A | 73 | 0.255 | 61.620 | 10.917 | 1.00 | 70.87 | A |
| ATOM | 373 | O | ALA | A | 73 | -0.074 | 61.283 | 12.058 | 1.00 | 70.52 | A |
| ATOM | 374 | N | PHE | A | 74 | 1.168 | 62.559 | 10.682 | 1.00 | 70.24 | A |
| ATOM | 375 | CA | PHE | A | 74 | 1.830 | 63.260 | 11.777 | 1.00 | 69.72 | A |

```
ATOM    376   CB    PHE  A   74        2.817   64.298   11.242   1.00  70.52       A
ATOM    377   CG    PHE  A   74        4.258   63.919   11.430   1.00  71.12       A
ATOM    378   CD1   PHE  A   74        4.852   62.952   10.623   1.00  71.78       A
ATOM    379   CD2   PHE  A   74        5.026   64.529   12.421   1.00  71.66       A
ATOM    380   CE1   PHE  A   74       ·6.192   62.597   10.800   1.00  71.93       A
ATOM    381   CE2   PHE  A   74        6.365   64.181   12.607   1.00  71.81       A
ATOM    382   CZ    PHE  A   74        6.949   63.215   11.795   1.00  71.80       A
ATOM    383   C     PHE  A   74        0.825   63.961   12.680   1.00  68.17       A
ATOM    384   O     PHE  A   74        1.006   64.008   13.896   1.00  68.34       A
ATOM    385   N     ILE  A   75       -0.225   64.511   12.078   1.00  67.41       A
ATOM    386   CA    ILE  A   75       -1.254   65.217   12.827   1.00  66.91       A
ATOM    387   CB    ILE  A   75       -2.243   65.943   11.879   1.00  67.03       A
ATOM    388   CG2   ILE  A   75       -3.391   66.541   12.676   1.00  66.70       A
ATOM    389   CG1   ILE  A   75       -1.509   67.037   11.100   1.00  66.85       A
ATOM    390   CD1   ILE  A   75       -0.861   68.091   11.977   1.00  66.79       A
ATOM    391   C     ILE  A   75       -2.023   64.258   13.722   1.00  66.28       A
ATOM    392   O     ILE  A   75       -2.315   64.579   14.869   1.00  66.25       A
ATOM    393   N     VAL  A   76       -2.345   63.080   13.202   1.00  65.90       A
ATOM    394   CA    VAL  A   76       -3.071   62.097   13.985   1.00  65.49       A
ATOM    395   CB    VAL  A   76       -3.390   60.851   13.153   1.00  65.61       A
ATOM    396   CG1   VAL  A   76       -4.083   59.815   14.012   1.00  65.63       A
ATOM    397   CG2   VAL  A   76       -4.264   61.237   11.980   1.00  65.59       A
ATOM    398   C     VAL  A   76       -2.229   61.689   15.185   1.00  65.56       A
ATOM    399   O     VAL  A   76       -2.746   61.561   16.297   1.00  65.42       A
ATOM    400   N     GLU  A   77       -0.933   61.485   14.954   1.00  66.12       A
ATOM    401   CA    GLU  A   77       -0.013   61.099   16.021   1.00  66.67       A
ATOM    402   CB    GLU  A   77        1.416   60.948   15.490   1.00  68.20       A
ATOM    403   CG    GLU  A   77        1.829   59.517   15.181   1.00  70.79       A
ATOM    404   CD    GLU  A   77        3.335   59.306   15.307   1.00  72.04       A
ATOM    405   OE1   GLU  A   77        4.102   59.999   14.600   1.00  73.21       A
ATOM    406   OE2   GLU  A   77        3.755   58.450   16.117   1.00  72.94       A
ATOM    407   C     GLU  A   77       -0.022   62.148   17.121   1.00  65.59       A
ATOM    408   O     GLU  A   77        0.032   61.825   18.306   1.00  65.37       A
ATOM    409   N     LEU  A   78       -0.079   63.411   16.722   1.00  65.39       A
ATOM    410   CA    LEU  A   78       -0.103   64.491   17.687   1.00  65.63       A
ATOM    411   CB    LEU  A   78       -0.147   65.843   16.983   1.00  66.14       A
ATOM    412   CG    LEU  A   78        1.220   66.437   16.682   1.00  66.81       A
ATOM    413   CD1   LEU  A   78        1.054   67.685   15.851   1.00  67.09       A
ATOM    414   CD2   LEU  A   78        1.931   66.743   17.981   1.00  66.68       A
ATOM    415   C     LEU  A   78       -1.309   64.365   18.590   1.00  65.47       A
ATOM    416   O     LEU  A   78       -1.164   64.286   19.810   1.00  65.02       A
ATOM    417   N     ARG  A   79       -2.498   64.335   17.990   1.00  65.21       A
ATOM    418   CA    ARG  A   79       -3.729   64.245   18.769   1.00  65.11       A
ATOM    419   CB    ARG  A   79       -4.973   64.199   17.854   1.00  67.27       A
ATOM    420   CG    ARG  A   79       -5.243   62.869   17.174   1.00  69.42       A
ATOM    421   CD    ARG  A   79       -6.664   62.795   16.600   1.00  71.39       A
ATOM    422   NE    ARG  A   79       -7.673   62.966   17.659   1.00  73.57       A
ATOM    423   CZ    ARG  A   79       -7.675   62.394   18.872   1.00  74.57       A
ATOM    424   NH1   ARG  A   79       -6.711   61.579   19.253   1.00  75.09       A
ATOM    425   NH2   ARG  A   79       -8.674   62.644   19.717   1.00  74.92       A
ATOM    426   C     ARG  A   79       -3.713   63.044   19.717   1.00  64.06       A
ATOM    427   O     ARG  A   79       -4.088   63.165   20.886   1.00  63.87       A
ATOM    428   N     GLN  A   80       -3.262   61.894   19.223   1.00  63.38       A
ATOM    429   CA    GLN  A   80       -3.201   60.694   20.045   1.00  61.19       A
```

| ATOM | 430 | CB | GLN | A | 80 | -2.816 | 59.499 | 19.190 | 1.00 | 62.28 | A |
|------|-----|-----|-----|---|----|--------|--------|--------|------|-------|---|
| ATOM | 431 | CG | GLN | A | 80 | -3.842 | 59.159 | 18.144 | 1.00 | 63.13 | A |
| ATOM | 432 | CD | GLN | A | 80 | -3.411 | 57.999 | 17.284 | 1.00 | 63.75 | A |
| ATOM | 433 | OE1 | GLN | A | 80 | -4.208 | 57.438 | 16.531 | 1.00 | 64.29 | A |
| ATOM | 434 | NE2 | GLN | A | 80 | -2.138 | 57.633 | 17.383 | 1.00 | 64.57 | A |
| ATOM | 435 | C | GLN | A | 80 | -2.189 | 60.853 | 21.166 | 1.00 | 60.52 | A |
| ATOM | 436 | O | GLN | A | 80 | -2.531 | 60.748 | 22.341 | 1.00 | 60.38 | A |
| ATOM | 437 | N | LEU | A | 81 | -0.940 | 61.110 | 20.787 | 1.00 | 60.72 | A |
| ATOM | 438 | CA | LEU | A | 81 | 0.150 | 61.279 | 21.742 | 1.00 | 60.45 | A |
| ATOM | 439 | CB | LEU | A | 81 | 1.457 | 61.585 | 21.010 | 1.00 | 60.59 | A |
| ATOM | 440 | CG | LEU | A | 81 | 2.230 | 60.385 | 20.475 | 1.00 | 61.01 | A |
| ATOM | 441 | CD1 | LEU | A | 81 | 3.493 | 60.872 | 19.783 | 1.00 | 60.83 | A |
| ATOM | 442 | CD2 | LEU | A | 81 | 2.567 | 59.450 | 21.634 | 1.00 | 61.33 | A |
| ATOM | 443 | C | LEU | A | 81 | -0.094 | 62.360 | 22.780 | 1.00 | 59.73 | A |
| ATOM | 444 | O | LEU | A | 81 | 0.522 | 62.344 | 23.851 | 1.00 | 60.90 | A |
| ATOM | 445 | N | SER | A | 82 | -0.982 | 63.300 | 22.470 | 1.00 | 58.56 | A |
| ATOM | 446 | CA | SER | A | 82 | -1.276 | 64.384 | 23.399 | 1.00 | 58.39 | A |
| ATOM | 447 | CB | SER | A | 82 | -1.897 | 65.570 | 22.647 | 1.00 | 57.75 | A |
| ATOM | 448 | OG | SER | A | 82 | -3.275 | 65.367 | 22.379 | 1.00 | 57.53 | A |
| ATOM | 449 | C | SER | A | 82 | -2.212 | 63.902 | 24.516 | 1.00 | 57.09 | A |
| ATOM | 450 | O | SER | A | 82 | -2.254 | 64.483 | 25.605 | 1.00 | 56.76 | A |
| ATOM | 451 | N | ARG | A | 83 | -2.940 | 62.823 | 24.236 | 1.00 | 56.95 | A |
| ATOM | 452 | CA | ARG | A | 83 | -3.871 | 62.235 | 25.191 | 1.00 | 56.64 | A |
| ATOM | 453 | CB | ARG | A | 83 | -4.946 | 61.431 | 24.465 | 1.00 | 57.31 | A |
| ATOM | 454 | CG | ARG | A | 83 | -5.788 | 62.201 | 23.486 | 1.00 | 59.33 | A |
| ATOM | 455 | CD | ARG | A | 83 | -6.686 | 61.237 | 22.737 | 1.00 | 60.92 | A |
| ATOM | 456 | NE | ARG | A | 83 | -7.588 | 60.524 | 23.638 | 1.00 | 62.66 | A |
| ATOM | 457 | CZ | ARG | A | 83 | -8.350 | 59.496 | 23.273 | 1.00 | 63.49 | A |
| ATOM | 458 | NH1 | ARG | A | 83 | -8.317 | 59.050 | 22.021 | 1.00 | 63.76 | A |
| ATOM | 459 | NH2 | ARG | A | 83 | -9.158 | 58.921 | 24.154 | 1.00 | 64.01 | A |
| ATOM | 460 | C | ARG | A | 83 | -3.177 | 61.292 | 26.170 | 1.00 | 55.56 | A |
| ATOM | 461 | O | ARG | A | 83 | -3.517 | 61.255 | 27.355 | 1.00 | 56.04 | A |
| ATOM | 462 | N | VAL | A | 84 | -2.210 | 60.528 | 25.665 | 1.00 | 54.21 | A |
| ATOM | 463 | CA | VAL | A | 84 | -1.497 | 59.540 | 26.468 | 1.00 | 53.51 | A |
| ATOM | 464 | CB | VAL | A | 84 | -0.767 | 58.512 | 25.575 | 1.00 | 53.53 | A |
| ATOM | 465 | CG1 | VAL | A | 84 | -1.704 | 57.997 | 24.512 | 1.00 | 53.13 | A |
| ATOM | 466 | CG2 | VAL | A | 84 | 0.464 | 59.132 | 24.955 | 1.00 | 54.12 | A |
| ATOM | 467 | C | VAL | A | 84 | -0.482 | 60.048 | 27.479 | 1.00 | 52.77 | A |
| ATOM | 468 | O | VAL | A | 84 | 0.261 | 60.995 | 27.234 | 1.00 | 53.88 | A |
| ATOM | 469 | N | ASN | A | 85 | -0.473 | 59.391 | 28.627 | 1.00 | 52.37 | A |
| ATOM | 470 | CA | ASN | A | 85 | 0.451 | 59.691 | 29.702 | 1.00 | 51.63 | A |
| ATOM | 471 | CB | ASN | A | 85 | -0.066 | 60.800 | 30.608 | 1.00 | 53.92 | A |
| ATOM | 472 | CG | ASN | A | 85 | 0.880 | 61.079 | 31.766 | 1.00 | 56.55 | A |
| ATOM | 473 | OD1 | ASN | A | 85 | 0.470 | 61.582 | 32.809 | 1.00 | 57.50 | A |
| ATOM | 474 | ND2 | ASN | A | 85 | 2.159 | 60.757 | 31.581 | 1.00 | 57.17 | A |
| ATOM | 475 | C | ASN | A | 85 | 0.555 | 58.409 | 30.502 | 1.00 | 48.84 | A |
| ATOM | 476 | O | ASN | A | 85 | -0.315 | 58.098 | 31.309 | 1.00 | 48.92 | A |
| ATOM | 477 | N | HIS | A | 86 | 1.622 | 57.662 | 30.266 | 1.00 | 44.50 | A |
| ATOM | 478 | CA | HIS | A | 86 | 1.816 | 56.404 | 30.952 | 1.00 | 40.96 | A |
| ATOM | 479 | CB | HIS | A | 86 | 1.047 | 55.314 | 30.196 | 1.00 | 39.97 | A |
| ATOM | 480 | CG | HIS | A | 86 | 1.073 | 53.982 | 30.867 | 1.00 | 39.56 | A |
| ATOM | 481 | CD2 | HIS | A | 86 | 0.200 | 53.399 | 31.720 | 1.00 | 38.59 | A |
| ATOM | 482 | ND1 | HIS | A | 86 | 2.136 | 53.114 | 30.753 | 1.00 | 38.36 | A |
| ATOM | 483 | CE1 | HIS | A | 86 | 1.918 | 52.055 | 31.510 | 1.00 | 38.14 | A |

| ATOM | 484 | NE2 | HIS | A | 86 | 0.751 | 52.203 | 32.107 | 1.00 | 37.52 | A |
|------|-----|-----|-----|---|----|--------|--------|--------|------|-------|---|
| ATOM | 485 | C | HIS | A | 86 | 3.311 | 56.106 | 30.996 | 1.00 | 39.85 | A |
| ATOM | 486 | O | HIS | A | 86 | 4.055 | 56.520 | 30.110 | 1.00 | 38.76 | A |
| ATOM | 487 | N | PRO | A | 87 | 3.770 | 55.406 | 32.043 | 1.00 | 38.50 | A |
| ATOM | 488 | CD | PRO | A | 87 | 2.957 | 55.053 | 33.223 | 1.00 | 38.29 | A |
| ATOM | 489 | CA | PRO | A | 87 | 5.178 | 55.029 | 32.246 | 1.00 | 36.72 | A |
| ATOM | 490 | CB | PRO | A | 87 | 5.136 | 54.253 | 33.557 | 1.00 | 37.34 | A |
| ATOM | 491 | CG | PRO | A | 87 | 4.000 | 54.888 | 34.288 | 1.00 | 38.20 | A |
| ATOM | 492 | C | PRO | A | 87 | 5.804 | 54.185 | 31.128 | 1.00 | 35.48 | A |
| ATOM | 493 | O | PRO | A | 87 | 7.006 | 54.258 | 30.889 | 1.00 | 34.71 | A |
| ATOM | 494 | N | ASN | A | 88 | 4.983 | 53.391 | 30.451 | 1.00 | 34.73 | A |
| ATOM | 495 | CA | ASN | A | 88 | 5.467 | 52.503 | 29.409 | 1.00 | 33.68 | A |
| ATOM | 496 | CB | ASN | A | 88 | 4.941 | 51.089 | 29.680 | 1.00 | 31.93 | A |
| ATOM | 497 | CG | ASN | A | 88 | 5.396 | 50.551 | 31.031 | 1.00 | 31.08 | A |
| ATOM | 498 | OD1 | ASN | A | 88 | 6.592 | 50.390 | 31.270 | 1.00 | 32.16 | A |
| ATOM | 499 | ND2 | ASN | A | 88 | 4.449 | 50.286 | 31.920 | 1.00 | 31.31 | A |
| ATOM | 500 | C | ASN | A | 88 | 5.137 | 52.933 | 27.986 | 1.00 | 34.38 | A |
| ATOM | 501 | O | ASN | A | 88 | 4.940 | 52.107 | 27.095 | 1.00 | 33.92 | A |
| ATOM | 502 | N | ILE | A | 89 | 5.073 | 54.239 | 27.785 | 1.00 | 37.76 | A |
| ATOM | 503 | CA | ILE | A | 89 | 4.811 | 54.803 | 26.475 | 1.00 | 38.42 | A |
| ATOM | 504 | CB | ILE | A | 89 | 3.340 | 55.289 | 26.342 | 1.00 | 38.88 | A |
| ATOM | 505 | CG2 | ILE | A | 89 | 3.098 | 55.825 | 24.933 | 1.00 | 39.25 | A |
| ATOM | 506 | CG1 | ILE | A | 89 | 2.377 | 54.128 | 26.607 | 1.00 | 38.62 | A |
| ATOM | 507 | CD1 | ILE | A | 89 | 0.917 | 54.526 | 26.654 | 1.00 | 39.56 | A |
| ATOM | 508 | C | ILE | A | 89 | 5.776 | 55.977 | 26.350 | 1.00 | 39.21 | A |
| ATOM | 509 | O | ILE | A | 89 | 5.908 | 56.763 | 27.289 | 1.00 | 38.96 | A |
| ATOM | 510 | N | VAL | A | 90 | 6.471 | 56.080 | 25.218 | 1.00 | 42.11 | A |
| ATOM | 511 | CA | VAL | A | 90 | 7.424 | 57.173 | 25.017 | 1.00 | 47.35 | A |
| ATOM | 512 | CB | VAL | A | 90 | 7.999 | 57.196 | 23.596 | 1.00 | 47.74 | A |
| ATOM | 513 | CG1 | VAL | A | 90 | 8.916 | 56.021 | 23.397 | 1.00 | 47.84 | A |
| ATOM | 514 | CG2 | VAL | A | 90 | 6.867 | 57.185 | 22.574 | 1.00 | 48.85 | A |
| ATOM | 515 | C | VAL | A | 90 | 6.792 | 58.530 | 25.276 | 1.00 | 49.38 | A |
| ATOM | 516 | O | VAL | A | 90 | 5.744 | 58.861 | 24.722 | 1.00 | 49.24 | A |
| ATOM | 517 | N | LYS | A | 91 | 7.446 | 59.310 | 26.126 | 1.00 | 52.96 | A |
| ATOM | 518 | CA | LYS | A | 91 | 6.962 | 60.632 | 26.480 | 1.00 | 57.39 | A |
| ATOM | 519 | CB | LYS | A | 91 | 7.607 | 61.085 | 27.791 | 1.00 | 58.26 | A |
| ATOM | 520 | CG | LYS | A | 91 | 7.081 | 62.409 | 28.325 | 1.00 | 60.86 | A |
| ATOM | 521 | CD | LYS | A | 91 | 7.738 | 62.756 | 29.660 | 1.00 | 62.39 | A |
| ATOM | 522 | CE | LYS | A | 91 | 7.306 | 61.811 | 30.796 | 1.00 | 63.70 | A |
| ATOM | 523 | NZ | LYS | A | 91 | 5.890 | 62.032 | 31.260 | 1.00 | 64.70 | A |
| ATOM | 524 | C | LYS | A | 91 | 7.269 | 61.639 | 25.375 | 1.00 | 59.67 | A |
| ATOM | 525 | O | LYS | A | 91 | 8.378 | 61.674 | 24.838 | 1.00 | 59.98 | A |
| ATOM | 526 | N | LEU | A | 92 | 6.266 | 62.440 | 25.028 | 1.00 | 62.39 | A |
| ATOM | 527 | CA | LEU | A | 92 | 6.411 | 63.476 | 24.017 | 1.00 | 65.13 | A |
| ATOM | 528 | CB | LEU | A | 92 | 5.114 | 63.605 | 23.214 | 1.00 | 65.49 | A |
| ATOM | 529 | CG | LEU | A | 92 | 5.094 | 64.516 | 21.986 | 1.00 | 65.73 | A |
| ATOM | 530 | CD1 | LEU | A | 92 | 6.177 | 64.089 | 21.004 | 1.00 | 65.82 | A |
| ATOM | 531 | CD2 | LEU | A | 92 | 3.727 | 64.445 | 21.332 | 1.00 | 65.45 | A |
| ATOM | 532 | C | LEU | A | 92 | 6.694 | 64.764 | 24.795 | 1.00 | 67.05 | A |
| ATOM | 533 | O | LEU | A | 92 | 6.093 | 65.000 | 25.842 | 1.00 | 67.95 | A |
| ATOM | 534 | N | TYR | A | 93 | 7.629 | 65.579 | 24.315 | 1.00 | 68.80 | A |
| ATOM | 535 | CA | TYR | A | 93 | 7.949 | 66.829 | 24.995 | 1.00 | 70.31 | A |
| ATOM | 536 | CB | TYR | A | 93 | 9.454 | 67.096 | 24.963 | 1.00 | 70.84 | A |
| ATOM | 537 | CG | TYR | A | 93 | 10.248 | 66.153 | 25.823 | 1.00 | 71.29 | A |

| | | | | | | | | | | |
|------|-----|-----|-----|---|-----|--------|--------|--------|------|-------|---|
| ATOM | 538 | CD1 | TYR | A | 93  | 9.696  | 65.616 | 26.983 | 1.00 | 72.17 | A |
| ATOM | 539 | CE1 | TYR | A | 93  | 10.429 | 64.761 | 27.803 | 1.00 | 72.59 | A |
| ATOM | 540 | CD2 | TYR | A | 93  | 11.559 | 65.814 | 25.496 | 1.00 | 71.66 | A |
| ATOM | 541 | CE2 | TYR | A | 93  | 12.305 | 64.960 | 26.309 | 1.00 | 72.41 | A |
| ATOM | 542 | CZ  | TYR | A | 93  | 11.733 | 64.435 | 27.461 | 1.00 | 72.67 | A |
| ATOM | 543 | OH  | TYR | A | 93  | 12.450 | 63.576 | 28.268 | 1.00 | 72.74 | A |
| ATOM | 544 | C   | TYR | A | 93  | 7.212  | 68.003 | 24.375 | 1.00 | 70.47 | A |
| ATOM | 545 | O   | TYR | A | 93  | 6.884  | 68.969 | 25.056 | 1.00 | 71.00 | A |
| ATOM | 546 | N   | GLY | A | 94  | 6.953  | 67.908 | 23.078 | 1.00 | 70.99 | A |
| ATOM | 547 | CA  | GLY | A | 94  | 6.258  | 68.967 | 22.375 | 1.00 | 72.32 | A |
| ATOM | 548 | C   | GLY | A | 94  | 6.379  | 68.719 | 20.891 | 1.00 | 72.93 | A |
| ATOM | 549 | O   | GLY | A | 94  | 6.868  | 67.671 | 20.481 | 1.00 | 73.27 | A |
| ATOM | 550 | N   | ALA | A | 95  | 5.941  | 69.672 | 20.081 | 1.00 | 74.51 | A |
| ATOM | 551 | CA  | ALA | A | 95  | 6.017  | 69.521 | 18.635 | 1.00 | 76.06 | A |
| ATOM | 552 | CB  | ALA | A | 95  | 4.737  | 68.902 | 18.107 | 1.00 | 75.59 | A |
| ATOM | 553 | C   | ALA | A | 95  | 6.237  | 70.886 | 18.011 | 1.00 | 77.31 | A |
| ATOM | 554 | O   | ALA | A | 95  | 6.683  | 71.810 | 18.688 | 1.00 | 77.35 | A |
| ATOM | 555 | N   | CYS | A | 96  | 5.920  | 71.013 | 16.725 | 1.00 | 79.40 | A |
| ATOM | 556 | CA  | CYS | A | 96  | 6.082  | 72.277 | 16.010 | 1.00 | 82.11 | A |
| ATOM | 557 | CB  | CYS | A | 96  | 7.519  | 72.779 | 16.157 | 1.00 | 82.93 | A |
| ATOM | 558 | SG  | CYS | A | 96  | 8.789  | 71.494 | 15.997 | 1.00 | 88.05 | A |
| ATOM | 559 | C   | CYS | A | 96  | 5.761  | 72.127 | 14.532 | 1.00 | 82.50 | A |
| ATOM | 560 | O   | CYS | A | 96  | 6.116  | 71.117 | 13.931 | 1.00 | 82.96 | A |
| ATOM | 561 | N   | LEU | A | 97  | 5.088  | 73.116 | 13.944 | 1.00 | 82.94 | A |
| ATOM | 562 | CA  | LEU | A | 97  | 4.784  | 73.033 | 12.522 | 1.00 | 83.95 | A |
| ATOM | 563 | CB  | LEU | A | 97  | 3.305  | 73.365 | 12.232 | 1.00 | 84.26 | A |
| ATOM | 564 | CG  | LEU | A | 97  | 2.708  | 72.715 | 10.957 | 1.00 | 84.47 | A |
| ATOM | 565 | CD1 | LEU | A | 97  | 1.184  | 72.661 | 11.039 | 1.00 | 84.58 | A |
| ATOM | 566 | CD2 | LEU | A | 97  | 3.139  | 73.474 | 9.708  | 1.00 | 84.38 | A |
| ATOM | 567 | C   | LEU | A | 97  | 5.723  | 73.958 | 11.744 | 1.00 | 84.18 | A |
| ATOM | 568 | O   | LEU | A | 97  | 5.491  | 74.239 | 10.566 | 1.00 | 84.46 | A |
| ATOM | 569 | N   | ASN | A | 98  | 6.786  | 74.432 | 12.403 | 1.00 | 84.67 | A |
| ATOM | 570 | CA  | ASN | A | 98  | 7.766  | 75.274 | 11.716 | 1.00 | 85.22 | A |
| ATOM | 571 | CB  | ASN | A | 98  | 8.976  | 75.580 | 12.616 | 1.00 | 86.24 | A |
| ATOM | 572 | CG  | ASN | A | 98  | 8.754  | 75.168 | 14.072 | 1.00 | 87.73 | A |
| ATOM | 573 | OD1 | ASN | A | 98  | 7.846  | 75.659 | 14.748 | 1.00 | 88.24 | A |
| ATOM | 574 | ND2 | ASN | A | 98  | 9.594  | 74.260 | 14.558 | 1.00 | 88.19 | A |
| ATOM | 575 | C   | ASN | A | 98  | 8.142  | 74.355 | 10.545 | 1.00 | 84.65 | A |
| ATOM | 576 | O   | ASN | A | 98  | 7.903  | 74.702 | 9.383  | 1.00 | 84.85 | A |
| ATOM | 577 | N   | PRO | A | 99  | 8.741  | 73.174 | 10.833 | 1.00 | 83.80 | A |
| ATOM | 578 | CD  | PRO | A | 99  | 9.557  | 72.841 | 12.021 | 1.00 | 83.62 | A |
| ATOM | 579 | CA  | PRO | A | 99  | 9.082  | 72.261 | 9.741  | 1.00 | 82.99 | A |
| ATOM | 580 | CB  | PRO | A | 99  | 10.510 | 71.871 | 10.073 | 1.00 | 83.14 | A |
| ATOM | 581 | CG  | PRO | A | 99  | 10.421 | 71.670 | 11.540 | 1.00 | 83.39 | A |
| ATOM | 582 | C   | PRO | A | 99  | 8.099  | 71.083 | 9.904  | 1.00 | 82.22 | A |
| ATOM | 583 | O   | PRO | A | 99  | 7.767  | 70.385 | 8.944  | 1.00 | 82.80 | A |
| ATOM | 584 | N   | VAL | A | 100 | 7.638  | 70.923 | 11.149 | 1.00 | 80.82 | A |
| ATOM | 585 | CA  | VAL | A | 100 | 6.703  | 69.900 | 11.638 | 1.00 | 79.18 | A |
| ATOM | 586 | CB  | VAL | A | 100 | 5.591  | 69.529 | 10.599 | 1.00 | 79.06 | A |
| ATOM | 587 | CG1 | VAL | A | 100 | 6.035  | 68.414 | 9.683  | 1.00 | 79.54 | A |
| ATOM | 588 | CG2 | VAL | A | 100 | 4.328  | 69.126 | 11.335 | 1.00 | 79.13 | A |
| ATOM | 589 | C   | VAL | A | 100 | 7.448  | 68.655 | 12.124 | 1.00 | 77.68 | A |
| ATOM | 590 | O   | VAL | A | 100 | 7.978  | 67.868 | 11.336 | 1.00 | 77.36 | A |
| ATOM | 591 | N   | CYS | A | 101 | 7.507  | 68.502 | 13.445 | 1.00 | 76.17 | A |

| ATOM | 592 | CA | CYS | A | 101 | 8.200 | 67.370 | 14.044 | 1.00 | 75.05 | A |
| ATOM | 593 | CB | CYS | A | 101 | 9.715 | 67.567 | 13.913 | 1.00 | 76.11 | A |
| ATOM | 594 | SG | CYS | A | 101 | 10.431 | 68.883 | 14.947 | 1.00 | 83.60 | A |
| ATOM | 595 | C | CYS | A | 101 | 7.851 | 67.108 | 15.516 | 1.00 | 73.00 | A |
| ATOM | 596 | O | CYS | A | 101 | 7.444 | 68.011 | 16.246 | 1.00 | 73.08 | A |
| ATOM | 597 | N | LEU | A | 102 | 8.028 | 65.857 | 15.935 | 1.00 | 70.55 | A |
| ATOM | 598 | CA | LEU | A | 102 | 7.761 | 65.442 | 17.304 | 1.00 | 69.03 | A |
| ATOM | 599 | CB | LEU | A | 102 | 7.142 | 64.047 | 17.330 | 1.00 | 69.31 | A |
| ATOM | 600 | CG | LEU | A | 102 | 5.663 | 63.917 | 16.985 | 1.00 | 69.43 | A |
| ATOM | 601 | CD1 | LEU | A | 102 | 5.363 | 64.661 | 15.709 | 1.00 | 69.96 | A |
| ATOM | 602 | CD2 | LEU | A | 102 | 5.306 | 62.452 | 16.850 | 1.00 | 69.91 | A |
| ATOM | 603 | C | LEU | A | 102 | 9.051 | 65.421 | 18.104 | 1.00 | 67.15 | A |
| ATOM | 604 | O | LEU | A | 102 | 10.057 | 64.878 | 17.654 | 1.00 | 66.57 | A |
| ATOM | 605 | N | VAL | A | 103 | 9.012 | 66.013 | 19.292 | 1.00 | 65.46 | A |
| ATOM | 606 | CA | VAL | A | 103 | 10.167 | 66.059 | 20.180 | 1.00 | 64.09 | A |
| ATOM | 607 | CB | VAL | A | 103 | 10.391 | 67.491 | 20.718 | 1.00 | 63.41 | A |
| ATOM | 608 | CG1 | VAL | A | 103 | 11.613 | 67.535 | 21.617 | 1.00 | 62.91 | A |
| ATOM | 609 | CG2 | VAL | A | 103 | 10.551 | 68.456 | 19.556 | 1.00 | 63.36 | A |
| ATOM | 610 | C | VAL | A | 103 | 9.882 | 65.111 | 21.343 | 1.00 | 63.10 | A |
| ATOM | 611 | O | VAL | A | 103 | 9.227 | 65.488 | 22.312 | 1.00 | 63.09 | A |
| ATOM | 612 | N | MET | A | 104 | 10.374 | 63.879 | 21.235 | 1.00 | 61.80 | A |
| ATOM | 613 | CA | MET | A | 104 | 10.149 | 62.863 | 22.259 | 1.00 | 61.38 | A |
| ATOM | 614 | CB | MET | A | 104 | 9.834 | 61.520 | 21.620 | 1.00 | 61.43 | A |
| ATOM | 615 | CG | MET | A | 104 | 9.128 | 61.607 | 20.306 | 1.00 | 62.21 | A |
| ATOM | 616 | SD | MET | A | 104 | 9.448 | 60.105 | 19.418 | 1.00 | 64.92 | A |
| ATOM | 617 | CE | MET | A | 104 | 10.974 | 60.517 | 18.565 | 1.00 | 63.51 | A |
| ATOM | 618 | C | MET | A | 104 | 11.375 | 62.668 | 23.112 | 1.00 | 59.83 | A |
| ATOM | 619 | O | MET | A | 104 | 12.425 | 63.248 | 22.850 | 1.00 | 59.94 | A |
| ATOM | 620 | N | GLU | A | 105 | 11.242 | 61.821 | 24.123 | 1.00 | 58.57 | A |
| ATOM | 621 | CA | GLU | A | 105 | 12.365 | 61.533 | 24.996 | 1.00 | 57.84 | A |
| ATOM | 622 | CB | GLU | A | 105 | 11.892 | 60.841 | 26.277 | 1.00 | 58.93 | A |
| ATOM | 623 | CG | GLU | A | 105 | 11.609 | 59.358 | 26.125 | 1.00 | 60.64 | A |
| ATOM | 624 | CD | GLU | A | 105 | 11.079 | 58.737 | 27.406 | 1.00 | 61.54 | A |
| ATOM | 625 | OE1 | GLU | A | 105 | 9.848 | 58.813 | 27.642 | 1.00 | 61.59 | A |
| ATOM | 626 | OE2 | GLU | A | 105 | 11.898 | 58.184 | 28.180 | 1.00 | 62.18 | A |
| ATOM | 627 | C | GLU | A | 105 | 13.289 | 60.613 | 24.218 | 1.00 | 56.11 | A |
| ATOM | 628 | O | GLU | A | 105 | 12.835 | 59.860 | 23.359 | 1.00 | 55.01 | A |
| ATOM | 629 | N | TYR | A | 106 | 14.583 | 60.685 | 24.507 | 1.00 | 54.54 | A |
| ATOM | 630 | CA | TYR | A | 106 | 15.555 | 59.843 | 23.823 | 1.00 | 53.19 | A |
| ATOM | 631 | CB | TYR | A | 106 | 16.903 | 60.562 | 23.690 | 1.00 | 52.78 | A |
| ATOM | 632 | CG | TYR | A | 106 | 17.951 | 59.698 | 23.033 | 1.00 | 52.26 | A |
| ATOM | 633 | CD1 | TYR | A | 106 | 17.789 | 59.254 | 21.722 | 1.00 | 52.12 | A |
| ATOM | 634 | CE1 | TYR | A | 106 | 18.713 | 58.404 | 21.131 | 1.00 | 52.72 | A |
| ATOM | 635 | CD2 | TYR | A | 106 | 19.074 | 59.273 | 23.739 | 1.00 | 52.11 | A |
| ATOM | 636 | CE2 | TYR | A | 106 | 20.008 | 58.421 | 23.159 | 1.00 | 52.64 | A |
| ATOM | 637 | CZ | TYR | A | 106 | 19.821 | 57.989 | 21.855 | 1.00 | 53.45 | A |
| ATOM | 638 | OH | TYR | A | 106 | 20.736 | 57.133 | 21.278 | 1.00 | 54.05 | A |
| ATOM | 639 | C | TYR | A | 106 | 15.764 | 58.512 | 24.547 | 1.00 | 53.61 | A |
| ATOM | 640 | O | TYR | A | 106 | 16.214 | 58.478 | 25.698 | 1.00 | 53.16 | A |
| ATOM | 641 | N | ALA | A | 107 | 15.440 | 57.419 | 23.864 | 1.00 | 52.78 | A |
| ATOM | 642 | CA | ALA | A | 107 | 15.599 | 56.091 | 24.440 | 1.00 | 52.43 | A |
| ATOM | 643 | CB | ALA | A | 107 | 14.702 | 55.086 | 23.714 | 1.00 | 52.54 | A |
| ATOM | 644 | C | ALA | A | 107 | 17.061 | 55.684 | 24.325 | 1.00 | 52.13 | A |
| ATOM | 645 | O | ALA | A | 107 | 17.510 | 55.199 | 23.279 | 1.00 | 52.09 | A |

```
ATOM    646  N    GLU A 108      17.800  55.901  25.408  1.00  52.61        A
ATOM    647  CA   GLU A 108      19.218  55.567  25.456  1.00  53.07        A
ATOM    648  CB   GLU A 108      19.795  55.895  26.844  1.00  55.53        A
ATOM    649  CG   GLU A 108      19.114  55.160  28.005  1.00  58.18        A
ATOM    650  CD   GLU A 108      18.137  56.037  28.781  1.00  59.75        A
ATOM    651  OE1  GLU A 108      17.324  56.738  28.134  1.00  60.66        A
ATOM    652  OE2  GLU A 108      18.175  56.011  30.037  1.00  60.74        A
ATOM    653  C    GLU A 108      19.459  54.089  25.130  1.00  51.51        A
ATOM    654  O    GLU A 108      20.392  53.749  24.402  1.00  51.79        A
ATOM    655  N    GLY A 109      18.602  53.222  25.664  1.00  49.19        A
ATOM    656  CA   GLY A 109      18.734  51.792  25.445  1.00  47.17        A
ATOM    657  C    GLY A 109      18.488  51.288  24.034  1.00  45.72        A
ATOM    658  O    GLY A 109      18.760  50.117  23.753  1.00  46.25        A
ATOM    659  N    GLY A 110      17.972  52.146  23.153  1.00  44.29        A
ATOM    660  CA   GLY A 110      17.713  51.732  21.784  1.00  42.43        A
ATOM    661  C    GLY A 110      16.441  50.921  21.633  1.00  41.24        A
ATOM    662  O    GLY A 110      15.583  50.919  22.512  1.00  40.82        A
ATOM    663  N    SER A 111      16.323  50.214  20.518  1.00  39.04        A
ATOM    664  CA   SER A 111      15.137  49.411  20.253  1.00  37.08        A
ATOM    665  CB   SER A 111      14.871  49.378  18.735  1.00  38.24        A
ATOM    666  OG  ASER A 111      14.033  48.308  18.374  0.50  38.47        A
ATOM    667  OG  BSER A 111      15.749  48.533  18.015  0.50  38.47        A
ATOM    668  C    SER A 111      15.267  47.991  20.818  1.00  36.06        A
ATOM    669  O    SER A 111      16.368  47.530  21.100  1.00  36.28        A
ATOM    670  N    LEU A 112      14.139  47.313  21.011  1.00  35.47        A
ATOM    671  CA   LEU A 112      14.163  45.941  21.507  1.00  34.81        A
ATOM    672  CB   LEU A 112      12.768  45.490  21.939  1.00  35.74        A
ATOM    673  CG   LEU A 112      12.706  44.039  22.420  1.00  34.63        A
ATOM    674  CD1  LEU A 112      13.529  43.898  23.676  1.00  34.95        A
ATOM    675  CD2  LEU A 112      11.282  43.633  22.669  1.00  34.31        A
ATOM    676  C    LEU A 112      14.666  45.027  20.376  1.00  33.02        A
ATOM    677  O    LEU A 112      15.226  43.955  20.620  1.00  32.75        A
ATOM    678  N    TYR A 113      14.451  45.463  19.135  1.00  34.52        A
ATOM    679  CA   TYR A 113      14.902  44.729  17.957  1.00  34.71        A
ATOM    680  CB   TYR A 113      14.458  45.443  16.669  1.00  38.46        A
ATOM    681  CG   TYR A 113      15.044  44.822  15.415  1.00  41.30        A
ATOM    682  CD1  TYR A 113      14.457  43.698  14.826  1.00  42.39        A
ATOM    683  CE1  TYR A 113      15.053  43.062  13.738  1.00  44.69        A
ATOM    684  CD2  TYR A 113      16.245  45.302  14.874  1.00  42.48        A
ATOM    685  CE2  TYR A 113      16.852  44.676  13.789  1.00  44.63        A
ATOM    686  CZ   TYR A 113      16.252  43.555  13.225  1.00  45.46        A
ATOM    687  OH   TYR A 113      16.858  42.919  12.158  1.00  46.76        A
ATOM    688  C    TYR A 113      16.428  44.644  17.985  1.00  34.26        A
ATOM    689  O    TYR A 113      17.003  43.575  17.820  1.00  34.02        A
ATOM    690  N    ASN A 114      17.070  45.790  18.187  1.00  37.16        A
ATOM    691  CA   ASN A 114      18.527  45.881  18.259  1.00  37.93        A
ATOM    692  CB   ASN A 114      18.938  47.351  18.346  1.00  42.33        A
ATOM    693  CG  AASN A 114      20.429  47.580  18.179  0.50  46.69        A
ATOM    694  CG  BASN A 114      18.734  48.086  17.026  0.50  46.70        A
ATOM    695  OD1 AASN A 114      21.020  47.313  17.120  0.50  49.07        A
ATOM    696  OD1 BASN A 114      18.992  47.527  15.944  0.50  49.08        A
ATOM    697  ND2 AASN A 114      21.046  48.097  19.232  0.50  48.44        A
ATOM    698  ND2 BASN A 114      18.277  49.342  17.100  0.50  48.45        A
ATOM    699  C    ASN A 114      19.106  45.103  19.450  1.00  36.91        A
```

| ATOM | 700 | O | ASN | A | 114 | 20.196 | 44.551 | 19.359 | 1.00 | 35.27 | A |
|------|-----|-----|-----|---|-----|--------|--------|--------|------|-------|---|
| ATOM | 701 | N | VAL | A | 115 | 18.392 | 45.071 | 20.571 | 1.00 | 35.96 | A |
| ATOM | 702 | CA | VAL | A | 115 | 18.861 | 44.314 | 21.727 | 1.00 | 34.11 | A |
| ATOM | 703 | CB | VAL | A | 115 | 17.968 | 44.555 | 22.964 | 1.00 | 34.85 | A |
| ATOM | 704 | CG1 | VAL | A | 115 | 18.219 | 43.487 | 24.022 | 1.00 | 34.47 | A |
| ATOM | 705 | CG2 | VAL | A | 115 | 18.235 | 45.928 | 23.528 | 1.00 | 35.75 | A |
| ATOM | 706 | C | VAL | A | 115 | 18.809 | 42.829 | 21.382 | 1.00 | 34.37 | A |
| ATOM | 707 | O | VAL | A | 115 | 19.731 | 42.073 | 21.693 | 1.00 | 32.17 | A |
| ATOM | 708 | N | LEU | A | 116 | 17.728 | 42.419 | 20.728 | 1.00 | 33.25 | A |
| ATOM | 709 | CA | LEU | A | 116 | 17.550 | 41.025 | 20.367 | 1.00 | 31.95 | A |
| ATOM | 710 | CB | LEU | A | 116 | 16.069 | 40.731 | 20.090 | 1.00 | 32.76 | A |
| ATOM | 711 | CG | LEU | A | 116 | 15.034 | 40.937 | 21.202 | 1.00 | 32.93 | A |
| ATOM | 712 | CD1 | LEU | A | 116 | 13.663 | 40.668 | 20.627 | 1.00 | 34.07 | A |
| ATOM | 713 | CD2 | LEU | A | 116 | 15.300 | 40.023 | 22.384 | 1.00 | 34.00 | A |
| ATOM | 714 | C | LEU | A | 116 | 18.374 | 40.574 | 19.171 | 1.00 | 32.89 | A |
| ATOM | 715 | O | LEU | A | 116 | 19.123 | 39.603 | 19.257 | 1.00 | 32.03 | A |
| ATOM | 716 | N | HIS | A | 117 | 18.248 | 41.285 | 18.057 | 1.00 | 34.31 | A |
| ATOM | 717 | CA | HIS | A | 117 | 18.942 | 40.891 | 16.830 | 1.00 | 37.48 | A |
| ATOM | 718 | CB | HIS | A | 117 | 17.893 | 40.595 | 15.750 | 1.00 | 37.60 | A |
| ATOM | 719 | CG | HIS | A | 117 | 16.657 | 39.914 | 16.269 | 1.00 | 37.04 | A |
| ATOM | 720 | CD2 | HIS | A | 117 | 15.389 | 40.368 | 16.421 | 1.00 | 37.26 | A |
| ATOM | 721 | ND1 | HIS | A | 117 | 16.646 | 38.606 | 16.706 | 1.00 | 38.30 | A |
| ATOM | 722 | CE1 | HIS | A | 117 | 15.425 | 38.284 | 17.100 | 1.00 | 36.86 | A |
| ATOM | 723 | NE2 | HIS | A | 117 | 14.644 | 39.336 | 16.937 | 1.00 | 36.67 | A |
| ATOM | 724 | C | HIS | A | 117 | 19.968 | 41.900 | 16.288 | 1.00 | 39.47 | A |
| ATOM | 725 | O | HIS | A | 117 | 20.436 | 41.759 | 15.167 | 1.00 | 38.17 | A |
| ATOM | 726 | N | GLY | A | 118 | 20.329 | 42.891 | 17.095 | 1.00 | 42.16 | A |
| ATOM | 727 | CA | GLY | A | 118 | 21.275 | 43.914 | 16.677 | 1.00 | 46.03 | A |
| ATOM | 728 | C | GLY | A | 118 | 22.683 | 43.485 | 16.294 | 1.00 | 49.99 | A |
| ATOM | 729 | O | GLY | A | 118 | 22.939 | 42.308 | 16.020 | 1.00 | 49.70 | A |
| ATOM | 730 | N | ALA | A | 119 | 23.604 | 44.452 | 16.299 | 1.00 | 52.85 | A |
| ATOM | 731 | CA | ALA | A | 119 | 25.003 | 44.228 | 15.908 | 1.00 | 54.48 | A |
| ATOM | 732 | CB | ALA | A | 119 | 25.632 | 45.547 | 15.465 | 1.00 | 55.09 | A |
| ATOM | 733 | C | ALA | A | 119 | 25.919 | 43.551 | 16.920 | 1.00 | 56.25 | A |
| ATOM | 734 | O | ALA | A | 119 | 25.839 | 43.807 | 18.123 | 1.00 | 56.30 | A |
| ATOM | 735 | N | GLU | A | 120 | 26.812 | 42.717 | 16.380 | 1.00 | 58.50 | A |
| ATOM | 736 | CA | GLU | A | 120 | 27.807 | 41.933 | 17.117 | 1.00 | 60.59 | A |
| ATOM | 737 | CB | GLU | A | 120 | 29.229 | 42.311 | 16.678 | 1.00 | 63.80 | A |
| ATOM | 738 | CG | GLU | A | 120 | 29.699 | 41.658 | 15.382 | 1.00 | 67.62 | A |
| ATOM | 739 | CD | GLU | A | 120 | 29.526 | 42.558 | 14.175 | 1.00 | 69.69 | A |
| ATOM | 740 | OE1 | GLU | A | 120 | 28.423 | 42.575 | 13.579 | 1.00 | 71.41 | A |
| ATOM | 741 | OE2 | GLU | A | 120 | 30.501 | 43.261 | 13.832 | 1.00 | 70.67 | A |
| ATOM | 742 | C | GLU | A | 120 | 27.739 | 41.916 | 18.642 | 1.00 | 59.03 | A |
| ATOM | 743 | O | GLU | A | 120 | 27.126 | 41.008 | 19.209 | 1.00 | 61.36 | A |
| ATOM | 744 | N | PRO | A | 121 | 28.369 | 42.893 | 19.337 | 1.00 | 55.73 | A |
| ATOM | 745 | CD | PRO | A | 121 | 28.986 | 44.185 | 18.972 | 1.00 | 55.77 | A |
| ATOM | 746 | CA | PRO | A | 121 | 28.233 | 42.764 | 20.793 | 1.00 | 53.42 | A |
| ATOM | 747 | CB | PRO | A | 121 | 29.131 | 43.883 | 21.314 | 1.00 | 54.11 | A |
| ATOM | 748 | CG | PRO | A | 121 | 28.932 | 44.961 | 20.277 | 1.00 | 55.70 | A |
| ATOM | 749 | C | PRO | A | 121 | 26.760 | 42.883 | 21.237 | 1.00 | 50.56 | A |
| ATOM | 750 | O | PRO | A | 121 | 26.257 | 43.972 | 21.535 | 1.00 | 50.32 | A |
| ATOM | 751 | N | LEU | A | 122 | 26.074 | 41.741 | 21.239 | 1.00 | 46.21 | A |
| ATOM | 752 | CA | LEU | A | 122 | 24.680 | 41.658 | 21.649 | 1.00 | 44.26 | A |
| ATOM | 753 | CB | LEU | A | 122 | 23.991 | 40.442 | 21.036 | 1.00 | 44.23 | A |

| ATOM | 754 | CG | LEU A 122 | 23.674 | 40.351 | 19.551 | 1.00 | 44.40 | A |
|------|-----|-----|-----------|--------|--------|--------|------|-------|---|
| ATOM | 755 | CD1 | LEU A 122 | 23.051 | 38.996 | 19.279 | 1.00 | 44.87 | A |
| ATOM | 756 | CD2 | LEU A 122 | 22.733 | 41.458 | 19.150 | 1.00 | 45.20 | A |
| ATOM | 757 | C | LEU A 122 | 24.709 | 41.464 | 23.148 | 1.00 | 41.45 | A |
| ATOM | 758 | O | LEU A 122 | 25.604 | 40.808 | 23.678 | 1.00 | 40.15 | A |
| ATOM | 759 | N | PRO A 123 | 23.732 | 42.031 | 23.858 | 1.00 | 39.43 | A |
| ATOM | 760 | CD | PRO A 123 | 22.679 | 42.974 | 23.431 | 1.00 | 38.77 | A |
| ATOM | 761 | CA | PRO A 123 | 23.735 | 41.858 | 25.312 | 1.00 | 39.16 | A |
| ATOM | 762 | CB | PRO A 123 | 22.915 | 43.052 | 25.793 | 1.00 | 38.80 | A |
| ATOM | 763 | CG | PRO A 123 | 21.864 | 43.162 | 24.706 | 1.00 | 38.64 | A |
| ATOM | 764 | C | PRO A 123 | 23.125 | 40.526 | 25.762 | 1.00 | 36.99 | A |
| ATOM | 765 | O | PRO A 123 | 22.301 | 39.924 | 25.056 | 1.00 | 36.39 | A |
| ATOM | 766 | N | TYR A 124 | 23.554 | 40.065 | 26.934 | 1.00 | 34.25 | A |
| ATOM | 767 | CA | TYR A 124 | 23.033 | 38.840 | 27.531 | 1.00 | 32.95 | A |
| ATOM | 768 | CB | TYR A 124 | 24.084 | 38.244 | 28.495 | 1.00 | 33.70 | A |
| ATOM | 769 | CG | TYR A 124 | 23.546 | 37.322 | 29.589 | 1.00 | 32.99 | A |
| ATOM | 770 | CD1 | TYR A 124 | 23.167 | 36.002 | 29.310 | 1.00 | 33.28 | A |
| ATOM | 771 | CE1 | TYR A 124 | 22.668 | 35.167 | 30.319 | 1.00 | 34.68 | A |
| ATOM | 772 | CD2 | TYR A 124 | 23.411 | 37.781 | 30.907 | 1.00 | 33.15 | A |
| ATOM | 773 | CE2 | TYR A 124 | 22.909 | 36.960 | 31.923 | 1.00 | 34.29 | A |
| ATOM | 774 | CZ | TYR A 124 | 22.539 | 35.659 | 31.621 | 1.00 | 34.41 | A |
| ATOM | 775 | OH | TYR A 124 | 22.002 | 34.868 | 32.616 | 1.00 | 36.77 | A |
| ATOM | 776 | C | TYR A 124 | 21.788 | 39.314 | 28.297 | 1.00 | 31.71 | A |
| ATOM | 777 | O | TYR A 124 | 21.772 | 40.421 | 28.849 | 1.00 | 29.63 | A |
| ATOM | 778 | N | TYR A 125 | 20.725 | 38.517 | 28.271 | 1.00 | 27.78 | A |
| ATOM | 779 | CA | TYR A 125 | 19.522 | 38.855 | 29.026 | 1.00 | 27.24 | A |
| ATOM | 780 | CB | TYR A 125 | 18.496 | 39.682 | 28.199 | 1.00 | 25.72 | A |
| ATOM | 781 | CG | TYR A 125 | 17.794 | 39.001 | 27.040 | 1.00 | 26.40 | A |
| ATOM | 782 | CD1 | TYR A 125 | 16.726 | 38.130 | 27.264 | 1.00 | 26.28 | A |
| ATOM | 783 | CE1 | TYR A 125 | 16.067 | 37.507 | 26.210 | 1.00 | 25.16 | A |
| ATOM | 784 | CD2 | TYR A 125 | 18.186 | 39.236 | 25.715 | 1.00 | 25.70 | A |
| ATOM | 785 | CE2 | TYR A 125 | 17.528 | 38.616 | 24.652 | 1.00 | 25.92 | A |
| ATOM | 786 | CZ | TYR A 125 | 16.471 | 37.753 | 24.911 | 1.00 | 23.62 | A |
| ATOM | 787 | OH | TYR A 125 | 15.819 | 37.117 | 23.886 | 1.00 | 24.66 | A |
| ATOM | 788 | C | TYR A 125 | 18.953 | 37.557 | 29.568 | 1.00 | 29.08 | A |
| ATOM | 789 | O | TYR A 125 | 19.216 | 36.477 | 29.034 | 1.00 | 27.65 | A |
| ATOM | 790 | N | THR A 126 | 18.199 | 37.659 | 30.651 | 1.00 | 28.30 | A |
| ATOM | 791 | CA | THR A 126 | 17.647 | 36.488 | 31.315 | 1.00 | 31.02 | A |
| ATOM | 792 | CB | THR A 126 | 17.930 | 36.597 | 32.808 | 1.00 | 29.89 | A |
| ATOM | 793 | OG1 | THR A 126 | 17.291 | 37.775 | 33.320 | 1.00 | 31.19 | A |
| ATOM | 794 | CG2 | THR A 126 | 19.437 | 36.715 | 33.045 | 1.00 | 30.55 | A |
| ATOM | 795 | C | THR A 126 | 16.150 | 36.290 | 31.106 | 1.00 | 27.77 | A |
| ATOM | 796 | O | THR A 126 | 15.502 | 37.092 | 30.444 | 1.00 | 30.04 | A |
| ATOM | 797 | N | ALA A 127 | 15.608 | 35.213 | 31.665 | 1.00 | 30.85 | A |
| ATOM | 798 | CA | ALA A 127 | 14.181 | 34.942 | 31.557 | 1.00 | 29.90 | A |
| ATOM | 799 | CB | ALA A 127 | 13.856 | 33.588 | 32.167 | 1.00 | 30.70 | A |
| ATOM | 800 | C | ALA A 127 | 13.398 | 36.045 | 32.270 | 1.00 | 28.62 | A |
| ATOM | 801 | O | ALA A 127 | 12.291 | 36.391 | 31.852 | 1.00 | 31.35 | A |
| ATOM | 802 | N | ALA A 128 | 13.978 | 36.598 | 33.338 | 1.00 | 27.84 | A |
| ATOM | 803 | CA | ALA A 128 | 13.349 | 37.679 | 34.101 | 1.00 | 26.09 | A |
| ATOM | 804 | CB | ALA A 128 | 14.169 | 37.984 | 35.351 | 1.00 | 26.03 | A |
| ATOM | 805 | C | ALA A 128 | 13.203 | 38.937 | 33.249 | 1.00 | 28.87 | A |
| ATOM | 806 | O | ALA A 128 | 12.199 | 39.636 | 33.338 | 1.00 | 27.48 | A |
| ATOM | 807 | N | HIS A 129 | 14.209 | 39.220 | 32.426 | 1.00 | 27.52 | A |

| ATOM | 808 | CA | HIS | A | 129 | 14.187 | 40.368 | 31.525 | 1.00 | 27.87 | A |
|------|-----|-----|-----|---|-----|--------|--------|--------|------|-------|---|
| ATOM | 809 | CB | HIS | A | 129 | 15.521 | 40.519 | 30.783 | 1.00 | 28.68 | A |
| ATOM | 810 | CG | HIS | A | 129 | 16.653 | 40.987 | 31.644 | 1.00 | 32.65 | A |
| ATOM | 811 | CD2 | HIS | A | 129 | 17.844 | 40.416 | 31.934 | 1.00 | 32.66 | A |
| ATOM | 812 | ND1 | HIS | A | 129 | 16.627 | 42.186 | 32.325 | 1.00 | 33.06 | A |
| ATOM | 813 | CE1 | HIS | A | 129 | 17.752 | 42.331 | 32.998 | 1.00 | 32.87 | A |
| ATOM | 814 | NE2 | HIS | A | 129 | 18.509 | 41.271 | 32.778 | 1.00 | 34.05 | A |
| ATOM | 815 | C | HIS | A | 129 | 13.103 | 40.175 | 30.491 | 1.00 | 27.42 | A |
| ATOM | 816 | O | HIS | A | 129 | 12.281 | 41.049 | 30.279 | 1.00 | 26.08 | A |
| ATOM | 817 | N | ALA | A | 130 | 13.126 | 39.020 | 29.839 | 1.00 | 27.83 | A |
| ATOM | 818 | CA | ALA | A | 130 | 12.148 | 38.695 | 28.816 | 1.00 | 26.83 | A |
| ATOM | 819 | CB | ALA | A | 130 | 12.397 | 37.282 | 28.287 | 1.00 | 27.24 | A |
| ATOM | 820 | C | ALA | A | 130 | 10.714 | 38.829 | 29.343 | 1.00 | 28.40 | A |
| ATOM | 821 | O | ALA | A | 130 | 9.872 | 39.467 | 28.721 | -1.00 | 25.52 | A |
| ATOM | 822 | N | MET | A | 131 | 10.433 | 38.232 | 30.494 | 1.00 | 29.08 | A |
| ATOM | 823 | CA | MET | A | 131 | 9.096 | 38.318 | 31.062 | 1.00 | 28.42 | A |
| ATOM | 824 | CB | MET | A | 131 | 8.981 | 37.392 | 32.276 | 1.00 | 28.76 | A |
| ATOM | 825 | CG | MET | A | 131 | 9.182 | 35.945 | 31.947 | 1.00 | 31.53 | A |
| ATOM | 826 | SD | MET | A | 131 | 7.946 | 35.368 | 30.788 | 1.00 | 30.98 | A |
| ATOM | 827 | CE | MET | A | 131 | 6.589 | 35.114 | 31.881 | 1.00 | 33.48 | A |
| ATOM | 828 | C | MET | A | 131 | 8.758 | 39.757 | 31.460 | 1.00 | 26.94 | A |
| ATOM | 829 | O | MET | A | 131 | 7.638 | 40.208 | 31.274 | 1.00 | 25.35 | A |
| ATOM | 830 | N | SER | A | 132 | 9.740 | 40.469 | 32.004 | 1.00 | 28.21 | A |
| ATOM | 831 | CA | SER | A | 132 | 9.552 | 41.849 | 32.431 | 1.00 | 25.19 | A |
| ATOM | 832 | CB | SER | A | 132 | 10.802 | 42.345 | 33.160 | 1.00 | 25.17 | A |
| ATOM | 833 | OG | SER | A | 132 | 10.712 | 43.726 | 33.475 | 1.00 | 25.34 | A |
| ATOM | 834 | C | SER | A | 132 | 9.231 | 42.772 | 31.253 | 1.00 | 25.60 | A |
| ATOM | 835 | O | SER | A | 132 | 8.337 | 43.609 | 31.335 | 1.00 | 24.83 | A |
| ATOM | 836 | N | TRP | A | 133 | 9.958 | 42.617 | 30.154 | 1.00 | 27.68 | A |
| ATOM | 837 | CA | TRP | A | 133 | 9.707 | 43.431 | 28.968 | 1.00 | 24.97 | A |
| ATOM | 838 | CB | TRP | A | 133 | 10.718 | 43.099 | 27.854 | 1.00 | 27.45 | A |
| ATOM | 839 | CG | TRP | A | 133 | 12.157 | 43.494 | 28.150 | 1.00 | 25.92 | A |
| ATOM | 840 | CD2 | TRP | A | 133 | 13.347 | 42.871 | 27.637 | 1.00 | 27.86 | A |
| ATOM | 841 | CE2 | TRP | A | 133 | 14.447 | 43.617 | 28.110 | 1.00 | 27.18 | A |
| ATOM | 842 | CE3 | TRP | A | 133 | 13.589 | 41.755 | 26.822 | 1.00 | 27.41 | A |
| ATOM | 843 | CD1 | TRP | A | 133 | 12.583 | 44.563 | 28.898 | 1.00 | 27.62 | A |
| ATOM | 844 | NE1 | TRP | A | 133 | 13.951 | 44.641 | 28.876 | 1.00 | 28.08 | A |
| ATOM | 845 | CZ2 | TRP | A | 133 | 15.770 | 43.284 | 27.797 | 1.00 | 27.59 | A |
| ATOM | 846 | CZ3 | TRP | A | 133 | 14.901 | 41.425 | 26.515 | 1.00 | 27.72 | A |
| ATOM | 847 | CH2 | TRP | A | 133 | 15.974 | 42.187 | 27.002 | 1.00 | 28.69 | A |
| ATOM | 848 | C | TRP | A | 133 | 8.283 | 43.219 | 28.453 | 1.00 | 25.28 | A |
| ATOM | 849 | O | TRP | A | 133 | 7.606 | 44.164 | 28.083 | 1.00 | 25.22 | A |
| ATOM | 850 | N | CYS | A | 134 | 7.840 | 41.967 | 28.447 | 1.00 | 25.48 | A |
| ATOM | 851 | CA | CYS | A | 134 | 6.505 | 41.611 | 27.980 | 1.00 | 27.67 | A |
| ATOM | 852 | CB | CYS | A | 134 | 6.403 | 40.095 | 27.813 | 1.00 | 27.28 | A |
| ATOM | 853 | SG | CYS | A | 134 | 7.424 | 39.529 | 26.413 | 1.00 | 24.53 | A |
| ATOM | 854 | C | CYS | A | 134 | 5.413 | 42.127 | 28.908 | 1.00 | 28.44 | A |
| ATOM | 855 | O | CYS | A | 134 | 4.359 | 42.573 | 28.456 | 1.00 | 26.43 | A |
| ATOM | 856 | N | LEU | A | 135 | 5.666 | 42.068 | 30.210 | 1.00 | 26.46 | A |
| ATOM | 857 | CA | LEU | A | 135 | 4.703 | 42.572 | 31.170 | 1.00 | 26.82 | A |
| ATOM | 858 | CB | LEU | A | 135 | 5.163 | 42.276 | 32.596 | 1.00 | 28.13 | A |
| ATOM | 859 | CG | LEU | A | 135 | 4.448 | 43.069 | 33.691 | 1.00 | 28.15 | A |
| ATOM | 860 | CD1 | LEU | A | 135 | 2.964 | 42.805 | 33.604 | 1.00 | 28.53 | A |
| ATOM | 861 | CD2 | LEU | A | 135 | 4.990 | 42.680 | 35.053 | 1.00 | 27.13 | A |

```
ATOM    862  C    LEU A 135      4.552  44.083  30.976  1.00 26.12      A
ATOM    863  O    LEU A 135      3.440  44.595  30.965  1.00 26.60      A
ATOM    864  N    GLN A 136      5.670  44.791  30.828  1.00 29.43      A
ATOM    865  CA   GLN A 136      5.636  46.241  30.644  1.00 25.49      A
ATOM    866  CB   GLN A 136      7.046  46.827  30.699  1.00 28.48      A
ATOM    867  CG   GLN A 136      7.717  46.709  32.058  1.00 30.48      A
ATOM    868  CD   GLN A 136      9.150  47.205  32.023  1.00 28.90      A
ATOM    869  OE1  GLN A 136      9.396  48.398  31.857  1.00 29.87      A
ATOM    870  NE2  GLN A 136     10.103  46.288  32.158  1.00 28.91      A
ATOM    871  C    GLN A 136      4.978  46.618  29.325  1.00 27.81      A
ATOM    872  O    GLN A 136      4.345  47.667  29.218  1.00 29.08      A
ATOM    873  N    CYS A 137      5.126  45.765  28.318  1.00 30.89      A
ATOM    874  CA   CYS A 137      4.513  46.030  27.025  1.00 27.37      A
ATOM    875  CB   CYS A 137      4.976  45.019  25.979  1.00 29.18      A
ATOM    876  SG   CYS A 137      4.256  45.301  24.353  1.00 32.84      A
ATOM    877  C    CYS A 137      3.009  45.942  27.173  1.00 26.44      A
ATOM    878  O    CYS A 137      2.292  46.824  26.713  1.00 30.17      A
ATOM    879  N    SER A 138      2.535  44.886  27.831  1.00 28.62      A
ATOM    880  CA   SER A 138      1.107  44.691  28.033  1.00 28.50      A
ATOM    881  CB   SER A 138      0.838  43.315  28.666  1.00 32.23      A
ATOM    882  OG   SER A 138      1.380  43.220  29.978  1.00 31.47      A
ATOM    883  C    SER A 138      0.497  45.800  28.887  1.00 24.89      A
ATOM    884  O    SER A 138     -0.650  46.168  28.681  1.00 26.70      A
ATOM    885  N    GLN A 139      1.264  46.335  29.835  1.00 26.84      A
ATOM    886  CA   GLN A 139      0.793  47.427  30.692  1.00 28.62      A
ATOM    887  CB   GLN A 139      1.822  47.748  31.779  1.00 28.92      A
ATOM    888  CG   GLN A 139      1.832  46.749  32.911  1.00 33.03      A
ATOM    889  CD   GLN A 139      3.010  46.919  33.837  1.00 33.99      A
ATOM    890  OE1  GLN A 139      3.082  46.266  34.869  1.00 35.07      A
ATOM    891  NE2  GLN A 139      3.944  47.790  33.473  1.00 35.28      A
ATOM    892  C    GLN A 139      0.541  48.685  29.870  1.00 28.64      A
ATOM    893  O    GLN A 139     -0.470  49.360  30.050  1.00 28.07      A
ATOM    894  N    GLY A 140      1.470  48.990  28.968  1.00 28.87      A
ATOM    895  CA   GLY A 140      1.326  50.152  28.127  1.00 28.36      A
ATOM    896  C    GLY A 140      0.181  49.971  27.160  1.00 27.96      A
ATOM    897  O    GLY A 140     -0.566  50.906  26.903  1.00 27.59      A
ATOM    898  N    VAL A 141      0.029  48.770  26.623  1.00 29.32      A
ATOM    899  CA   VAL A 141     -1.045  48.525  25.685  1.00 29.73      A
ATOM    900  CB   VAL A 141     -0.800  47.244  24.874  1.00 28.53      A
ATOM    901  CG1  VAL A 141     -1.988  46.970  23.954  1.00 29.69      A
ATOM    902  CG2  VAL A 141      0.468  47.404  24.053  1.00 28.06      A
ATOM    903  C    VAL A 141     -2.397  48.447  26.380  1.00 29.18      A
ATOM    904  O    VAL A 141     -3.407  48.869  25.818  1.00 28.75      A
ATOM    905  N    ALA A 142     -2.426  47.928  27.601  1.00 30.63      A
ATOM    906  CA   ALA A 142     -3.683  47.850  28.340  1.00 30.05      A
ATOM    907  CB   ALA A 142     -3.496  47.066  29.644  1.00 30.36      A
ATOM    908  C    ALA A 142     -4.181  49.274  28.639  1.00 31.21      A
ATOM    909  O    ALA A 142     -5.385  49.508  28.737  1.00 31.62      A
ATOM    910  N    TYR A 143     -3.256  50.221  28.774  1.00 33.05      A
ATOM    911  CA   TYR A 143     -3.622  51.604  29.032  1.00 35.22      A
ATOM    912  CB   TYR A 143     -2.401  52.417  29.444  1.00 36.99      A
ATOM    913  CG   TYR A 143     -2.643  53.915  29.446  1.00 40.54      A
ATOM    914  CD1  TYR A 143     -2.365  54.695  28.318  1.00 41.47      A
ATOM    915  CE1  TYR A 143     -2.577  56.078  28.327  1.00 43.33      A
```

| ATOM | 916 | CD2 | TYR | A | 143 | -3.142 | 54.552 | 30.577 | 1.00 | 42.02 | A |
|------|-----|-----|-----|---|-----|--------|--------|--------|------|-------|---|
| ATOM | 917 | CE2 | TYR | A | 143 | -3.360 | 55.932 | 30.596 | 1.00 | 43.03 | A |
| ATOM | 918 | CZ | TYR | A | 143 | -3.074 | 56.686 | 29.474 | 1.00 | 43.50 | A |
| ATOM | 919 | OH | TYR | A | 143 | -3.278 | 58.049 | 29.509 | 1.00 | 45.90 | A |
| ATOM | 920 | C | TYR | A | 143 | -4.258 | 52.232 | 27.798 | 1.00 | 34.87 | A |
| ATOM | 921 | O | TYR | A | 143 | -5.179 | 53.036 | 27.916 | 1.00 | 36.95 | A |
| ATOM | 922 | N | LEU | A | 144 | -3.753 | 51.871 | 26.619 | 1.00 | 35.27 | A |
| ATOM | 923 | CA | LEU | A | 144 | -4.285 | 52.377 | 25.357 | 1.00 | 35.86 | A |
| ATOM | 924 | CB | LEU | A | 144 | -3.401 | 51.950 | 24.185 | 1.00 | 35.20 | A |
| ATOM | 925 | CG | LEU | A | 144 | -2.000 | 52.539 | 24.069 | 1.00 | 35.14 | A |
| ATOM | 926 | CD1 | LEU | A | 144 | -1.293 | 51.908 | 22.888 | 1.00 | 35.45 | A |
| ATOM | 927 | CD2 | LEU | A | 144 | -2.083 | 54.041 | 23.902 | 1.00 | 35.67 | A |
| ATOM | 928 | C | LEU | A | 144 | -5.687 | 51.820 | 25.144 | 1.00 | 35.18 | A |
| ATOM | 929 | O | LEU | A | 144 | -6.609 | 52.554 | 24.785 | 1.00 | 35.15 | A |
| ATOM | 930 | N | HIS | A | 145 | -5.833 | 50.518 | 25.368 | 1.00 | 34.65 | A |
| ATOM | 931 | CA | HIS | A | 145 | -7.105 | 49.836 | 25.214 | 1.00 | 34.62 | A |
| ATOM | 932 | CB | HIS | A | 145 | -6.934 | 48.333 | 25.447 | 1.00 | 32.85 | A |
| ATOM | 933 | CG | HIS | A | 145 | -6.228 | 47.619 | 24.337 | 1.00 | 29.40 | A |
| ATOM | 934 | CD2 | HIS | A | 145 | -6.009 | 46.301 | 24.123 | 1.00 | 31.67 | A |
| ATOM | 935 | ND1 | HIS | A | 145 | -5.650 | 48.279 | 23.275 | 1.00 | 31.18 | A |
| ATOM | 936 | CE1 | HIS | A | 145 | -5.107 | 47.399 | 22.453 | 1.00 | 30.50 | A |
| ATOM | 937 | NE2 | HIS | A | 145 | -5.312 | 46.191 | 22.945 | 1.00 | 31.82 | A |
| ATOM | 938 | C | HIS | A | 145 | -8.167 | 50.350 | 26.169 | 1.00 | 35.01 | A |
| ATOM | 939 | O | HIS | A | 145 | -9.350 | 50.193 | 25.919 | 1.00 | 35.43 | A |
| ATOM | 940 | N | SER | A | 146 | -7.752 | 50.968 | 27.262 | 1.00 | 37.81 | A |
| ATOM | 941 | CA | SER | A | 146 | -8.707 | 51.461 | 28.246 | 1.00 | 40.75 | A |
| ATOM | 942 | CB | SER | A | 146 | -8.182 | 51.185 | 29.646 | 1.00 | 41.26 | A |
| ATOM | 943 | OG | SER | A | 146 | -7.120 | 52.078 | 29.943 | 1.00 | 43.73 | A |
| ATOM | 944 | C | SER | A | 146 | -9.004 | 52.951 | 28.124 | 1.00 | 42.52 | A |
| ATOM | 945 | O | SER | A | 146 | -9.666 | 53.528 | 28.983 | 1.00 | 43.15 | A |
| ATOM | 946 | N | MET | A | 147 | -8.503 | 53.570 | 27.065 | 1.00 | 47.47 | A |
| ATOM | 947 | CA | MET | A | 147 | -8.710 | 54.993 | 26.843 | 1.00 | 50.77 | A |
| ATOM | 948 | CB | MET | A | 147 | -7.997 | 55.425 | 25.563 | 1.00 | 55.00 | A |
| ATOM | 949 | CG | MET | A | 147 | -7.291 | 56.755 | 25.663 | 1.00 | 59.44 | A |
| ATOM | 950 | SD | MET | A | 147 | -5.648 | 56.589 | 26.360 | 1.00 | 71.60 | A |
| ATOM | 951 | CE | MET | A | 147 | -4.707 | 56.330 | 24.867 | 1.00 | 62.88 | A |
| ATOM | 952 | C | MET | A | 147 | -10.193 | 55.342 | 26.721 | 1.00 | 51.66 | A |
| ATOM | 953 | O | MET | A | 147 | -11.006 | 54.504 | 26.329 | 1.00 | 51.64 | A |
| ATOM | 954 | N | GLN | A | 148 | -10.520 | 56.591 | 27.061 | 1.00 | 55.00 | A |
| ATOM | 955 | CA | GLN | A | 148 | -11.883 | 57.150 | 26.983 | 1.00 | 57.98 | A |
| ATOM | 956 | CB | GLN | A | 148 | -12.473 | 57.349 | 28.388 | 1.00 | 59.76 | A |
| ATOM | 957 | CG | GLN | A | 148 | -12.747 | 56.064 | 29.173 | 1.00 | 63.20 | A |
| ATOM | 958 | CD | GLN | A | 148 | -13.668 | 55.110 | 28.425 | 1.00 | 63.78 | A |
| ATOM | 959 | OE1 | GLN | A | 148 | -14.779 | 55.478 | 28.042 | 1.00 | 65.41 | A |
| ATOM | 960 | NE2 | GLN | A | 148 | -13.207 | 53.875 | 28.212 | 1.00 | 65.29 | A |
| ATOM | 961 | C | GLN | A | 148 | -11.740 | 58.519 | 26.291 | 1.00 | 59.45 | A |
| ATOM | 962 | O | GLN | A | 148 | -10.774 | 59.245 | 26.558 | 1.00 | 59.32 | A |
| ATOM | 963 | N | PRO | A | 149 | -12.697 | 58.910 | 25.420 | 1.00 | 60.50 | A |
| ATOM | 964 | CD | PRO | A | 149 | -12.511 | 60.217 | 24.760 | 1.00 | 60.30 | A |
| ATOM | 965 | CA | PRO | A | 149 | -13.939 | 58.279 | 24.945 | 1.00 | 60.48 | A |
| ATOM | 966 | CB | PRO | A | 149 | -14.455 | 59.272 | 23.903 | 1.00 | 60.66 | A |
| ATOM | 967 | CG | PRO | A | 149 | -13.920 | 60.585 | 24.398 | 1.00 | 60.67 | A |
| ATOM | 968 | C | PRO | A | 149 | -13.814 | 56.871 | 24.372 | 1.00 | 60.54 | A |
| ATOM | 969 | O | PRO | A | 149 | -14.601 | 55.989 | 24.714 | 1.00 | 60.55 | A |

| ATOM | 970 | N | LYS | A | 150 | -12.854 | 56.658 | 23.479 | 1.00 | 60.67 | A |
| ATOM | 971 | CA | LYS | A | 150 | -12.694 | 55.327 | 22.922 | 1.00 | 59.93 | A |
| ATOM | 972 | CB | LYS | A | 150 | -13.298 | 55.256 | 21.520 | 1.00 | 61.32 | A |
| ATOM | 973 | CG | LYS | A | 150 | -12.599 | 56.080 | 20.477 | 1.00 | 63.41 | A |
| ATOM | 974 | CD | LYS | A | 150 | -13.175 | 55.749 | 19.113 | 1.00 | 65.43 | A |
| ATOM | 975 | CE | LYS | A | 150 | -12.343 | 56.342 | 17.992 | 1.00 | 66.43 | A |
| ATOM | 976 | NZ | LYS | A | 150 | -12.863 | 55.915 | 16.662 | 1.00 | 67.54 | A |
| ATOM | 977 | C | LYS | A | 150 | -11.245 | 54.841 | 22.918 | 1.00 | 58.31 | A |
| ATOM | 978 | O | LYS | A | 150 | -10.299 | 55.635 | 22.879 | 1.00 | 58.02 | A |
| ATOM | 979 | N | ALA | A | 151 | -11.096 | 53.520 | 22.975 | 1.00 | 55.87 | A |
| ATOM | 980 | CA | ALA | A | 151 | -9.799 | 52.860 | 23.012 | 1.00 | 53.15 | A |
| ATOM | 981 | CB | ALA | A | 151 | -9.999 | 51.359 | 23.194 | 1.00 | 53.49 | A |
| ATOM | 982 | C | ALA | A | 151 | -8.919 | 53.099 | 21.795 | 1.00 | 52.66 | A |
| ATOM | 983 | O | ALA | A | 151 | -9.401 | 53.152 | 20.661 | 1.00 | 52.25 | A |
| ATOM | 984 | N | LEU | A | 152 | -7.621 | 53.244 | 22.039 | 1.00 | 50.63 | A |
| ATOM | 985 | CA | LEU | A | 152 | -6.674 | 53.412 | 20.956 | 1.00 | 49.10 | A |
| ATOM | 986 | CB | LEU | A | 152 | -5.548 | 54.371 | 21.329 | 1.00 | 51.16 | A |
| ATOM | 987 | CG | LEU | A | 152 | -5.890 | 55.813 | 21.667 | 1.00 | 53.08 | A |
| ATOM | 988 | CD1 | LEU | A | 152 | -4.628 | 56.646 | 21.508 | 1.00 | 53.70 | A |
| ATOM | 989 | CD2 | LEU | A | 152 | -6.989 | 56.327 | 20.741 | 1.00 | 54.37 | A |
| ATOM | 990 | C | LEU | A | 152 | -6.060 | 52.053 | 20.714 | 1.00 | 46.08 | A |
| ATOM | 991 | O | LEU | A | 152 | -5.633 | 51.388 | 21.650 | 1.00 | 46.23 | A |
| ATOM | 992 | N | ILE | A | 153 | -6.034 | 51.619 | 19.467 | 1.00 | 44.27 | A |
| ATOM | 993 | CA | ILE | A | 153 | -5.408 | 50.349 | 19.158 | 1.00 | 43.41 | A |
| ATOM | 994 | CB | ILE | A | 153 | -6.185 | 49.605 | 18.063 | 1.00 | 42.65 | A |
| ATOM | 995 | CG2 | ILE | A | 153 | -5.469 | 48.324 | 17.689 | 1.00 | 42.15 | A |
| ATOM | 996 | CG1 | ILE | A | 153 | -7.591 | 49.286 | 18.570 | 1.00 | 42.79 | A |
| ATOM | 997 | CD1 | ILE | A | 153 | -8.455 | 48.557 | 17.558 | 1.00 | 43.55 | A |
| ATOM | 998 | C | ILE | A | 153 | -4.012 | 50.722 | 18.662 | 1.00 | 40.40 | A |
| ATOM | 999 | O | ILE | A | 153 | -3.844 | 51.758 | 18.012 | 1.00 | 41.87 | A |
| ATOM | 1000 | N | HIS | A | 154 | -3.000 | 49.923 | 18.987 | 1.00 | 41.12 | A |
| ATOM | 1001 | CA | HIS | A | 154 | -1.653 | 50.248 | 18.513 | 1.00 | 37.90 | A |
| ATOM | 1002 | CB | HIS | A | 154 | -0.581 | 49.537 | 19.339 | 1.00 | 38.12 | A |
| ATOM | 1003 | CG | HIS | A | 154 | 0.805 | 50.023 | 19.058 | 1.00 | 37.93 | A |
| ATOM | 1004 | CD2 | HIS | A | 154 | 1.656 | 50.770 | 19.801 | 1.00 | 35.80 | A |
| ATOM | 1005 | ND1 | HIS | A | 154 | 1.445 | 49.793 | 17.860 | 1.00 | 35.51 | A |
| ATOM | 1006 | CE1 | HIS | A | 154 | 2.628 | 50.378 | 17.876 | 1.00 | 36.98 | A |
| ATOM | 1007 | NE2 | HIS | A | 154 | 2.781 | 50.978 | 19.043 | 1.00 | 36.62 | A |
| ATOM | 1008 | C | HIS | A | 154 | -1.538 | 49.848 | 17.038 | 1.00 | 38.06 | A |
| ATOM | 1009 | O | HIS | A | 154 | -1.097 | 50.641 | 16.205 | 1.00 | 37.91 | A |
| ATOM | 1010 | N | ARG | A | 155 | -1.944 | 48.620 | 16.724 | 1.00 | 39.69 | A |
| ATOM | 1011 | CA | ARG | A | 155 | -1.935 | 48.121 | 15.354 | 1.00 | 39.78 | A |
| ATOM | 1012 | CB | ARG | A | 155 | -2.597 | 49.137 | 14.420 | 1.00 | 45.35 | A |
| ATOM | 1013 | CG | ARG | A | 155 | -4.019 | 49.486 | 14.799 | 1.00 | 52.27 | A |
| ATOM | 1014 | CD | ARG | A | 155 | -4.313 | 50.953 | 14.512 | 1.00 | 58.18 | A |
| ATOM | 1015 | NE | ARG | A | 155 | -5.157 | 51.164 | 13.335 | 1.00 | 64.15 | A |
| ATOM | 1016 | CZ | ARG | A | 155 | -6.463 | 50.902 | 13.288 | 1.00 | 66.85 | A |
| ATOM | 1017 | NH1 | ARG | A | 155 | -7.090 | 50.412 | 14.358 | 1.00 | 68.63 | A |
| ATOM | 1018 | NH2 | ARG | A | 155 | -7.146 | 51.135 | 12.170 | 1.00 | 68.78 | A |
| ATOM | 1019 | C | ARG | A | 155 | -0.582 | 47.747 | 14.785 | 1.00 | 38.58 | A |
| ATOM | 1020 | O | ARG | A | 155 | -0.516 | 47.043 | 13.782 | 1.00 | 37.68 | A |
| ATOM | 1021 | N | ASP | A | 156 | 0.500 | 48.207 | 15.401 | 1.00 | 38.07 | A |
| ATOM | 1022 | CA | ASP | A | 156 | 1.819 | 47.867 | 14.879 | 1.00 | 36.73 | A |
| ATOM | 1023 | CB | ASP | A | 156 | 2.371 | 49.030 | 14.041 | 1.00 | 40.77 | A |

| ATOM | 1024 | CG | ASP | A | 156 | 3.581 | 48.622 | 13.213 | 1.00 | 41.45 | A |
|------|------|-----|-----|---|-----|--------|--------|--------|------|-------|---|
| ATOM | 1025 | OD1 | ASP | A | 156 | 3.739 | 47.397 | 13.015 | 1.00 | 42.43 | A |
| ATOM | 1026 | OD2 | ASP | A | 156 | 4.350 | 49.512 | 12.765 | 1.00 | 42.16 | A |
| ATOM | 1027 | C | ASP | A | 156 | 2.842 | 47.451 | 15.940 | 1.00 | 35.69 | A |
| ATOM | 1028 | O | ASP | A | 156 | 3.996 | 47.907 | 15.928 | 1.00 | 34.50 | A |
| ATOM | 1029 | N | LEU | A | 157 | 2.422 | 46.573 | 16.848 | 1.00 | 35.42 | A |
| ATOM | 1030 | CA | LEU | A | 157 | 3.318 | 46.104 | 17.897 | 1.00 | 36.20 | A |
| ATOM | 1031 | CB | LEU | A | 157 | 2.545 | 45.422 | 19.026 | 1.00 | 37.84 | A |
| ATOM | 1032 | CG | LEU | A | 157 | 1.970 | 46.284 | 20.148 | 1.00 | 39.89 | A |
| ATOM | 1033 | CD1 | LEU | A | 157 | 1.272 | 45.364 | 21.134 | 1.00 | 40.99 | A |
| ATOM | 1034 | CD2 | LEU | A | 157 | 3.067 | 47.101 | 20.835 | 1.00 | 40.84 | A |
| ATOM | 1035 | C | LEU | A | 157 | 4.366 | 45.133 | 17.362 | 1.00 | 34.62 | A |
| ATOM | 1036 | O | LEU | A | 157 | 4.048 | 44.075 | 16.806 | 1.00 | 34.41 | A |
| ATOM | 1037 | N | LYS | A | 158 | 5.624 | 45.512 | 17.524 | 1.00 | 31.60 | A |
| ATOM | 1038 | CA | LYS | A | 158 | 6.730 | 44.684 | 17.097 | 1.00 | 30.99 | A |
| ATOM | 1039 | CB | LYS | A | 158 | 6.834 | 44.652 | 15.573 | 1.00 | 32.19 | A |
| ATOM | 1040 | CG | LYS | A | 158 | 6.926 | 45.994 | 14.910 | 1.00 | 32.97 | A |
| ATOM | 1041 | CD | LYS | A | 158 | 7.150 | 45.826 | 13.432 | 1.00 | 34.01 | A |
| ATOM | 1042 | CE | LYS | A | 158 | 7.491 | 47.158 | 12.803 | 1.00 | 36.32 | A |
| ATOM | 1043 | NZ | LYS | A | 158 | 7.941 | 47.016 | 11.385 | 1.00 | 39.33 | A |
| ATOM | 1044 | C | LYS | A | 158 | 7.969 | 45.286 | 17.736 | 1.00 | 33.70 | A |
| ATOM | 1045 | O | LYS | A | 158 | 7.974 | 46.460 | 18.121 | 1.00 | 31.40 | A |
| ATOM | 1046 | N | PRO | A | 159 | 9.031 | 44.485 | 17.883 | 1.00 | 30.57 | A |
| ATOM | 1047 | CD | PRO | A | 159 | 9.133 | 43.072 | 17.477 | 1.00 | 30.86 | A |
| ATOM | 1048 | CA | PRO | A | 159 | 10.279 | 44.942 | 18.493 | 1.00 | 31.09 | A |
| ATOM | 1049 | CB | PRO | A | 159 | 11.208 | 43.755 | 18.270 | 1.00 | 32.58 | A |
| ATOM | 1050 | CG | PRO | A | 159 | 10.266 | 42.595 | 18.331 | 1.00 | 31.23 | A |
| ATOM | 1051 | C | PRO | A | 159 | 10.863 | 46.295 | 18.015 | 1.00 | 32.20 | A |
| ATOM | 1052 | O | PRO | A | 159 | 11.374 | 47.063 | 18.839 | 1.00 | 32.07 | A |
| ATOM | 1053 | N | PRO | A | 160 | 10.813 | 46.601 | 16.695 | 1.00 | 32.59 | A |
| ATOM | 1054 | CD | PRO | A | 160 | 10.484 | 45.754 | 15.532 | 1.00 | 31.46 | A |
| ATOM | 1055 | CA | PRO | A | 160 | 11.364 | 47.890 | 16.253 | 1.00 | 31.30 | A |
| ATOM | 1056 | CB | PRO | A | 160 | 11.159 | 47.851 | 14.746 | 1.00 | 32.34 | A |
| ATOM | 1057 | CG | PRO | A | 160 | 11.291 | 46.402 | 14.429 | 1.00 | 31.57 | A |
| ATOM | 1058 | C | PRO | A | 160 | 10.635 | 49.076 | 16.898 | 1.00 | 32.54 | A |
| ATOM | 1059 | O | PRO | A | 160 | 11.157 | 50.197 | 16.945 | 1.00 | 33.59 | A |
| ATOM | 1060 | N | ASN | A | 161 | 9.425 | 48.817 | 17.390 | 1.00 | 33.40 | A |
| ATOM | 1061 | CA | ASN | A | 161 | 8.610 | 49.852 | 18.019 | 1.00 | 35.19 | A |
| ATOM | 1062 | CB | ASN | A | 161 | 7.171 | 49.789 | 17.499 | 1.00 | 35.04 | A |
| ATOM | 1063 | CG | ASN | A | 161 | 7.078 | 50.074 | 16.022 | 1.00 | 36.71 | A |
| ATOM | 1064 | OD1 | ASN | A | 161 | 7.960 | 50.715 | 15.452 | 1.00 | 36.96 | A |
| ATOM | 1065 | ND2 | ASN | A | 161 | 6.002 | 49.616 | 15.394 | 1.00 | 36.61 | A |
| ATOM | 1066 | C | ASN | A | 161 | 8.592 | 49.790 | 19.538 | 1.00 | 34.27 | A |
| ATOM | 1067 | O | ASN | A | 161 | 7.747 | 50.409 | 20.177 | 1.00 | 34.82 | A |
| ATOM | 1068 | N | LEU | A | 162 | 9.512 | 49.030 | 20.116 | 1.00 | 34.78 | A |
| ATOM | 1069 | CA | LEU | A | 162 | 9.602 | 48.919 | 21.565 | 1.00 | 33.19 | A |
| ATOM | 1070 | CB | LEU | A | 162 | 9.440 | 47.461 | 22.011 | 1.00 | 34.03 | A |
| ATOM | 1071 | CG | LEU | A | 162 | 8.125 | 46.795 | 21.608 | 1.00 | 33.09 | A |
| ATOM | 1072 | CD1 | LEU | A | 162 | 8.053 | 45.397 | 22.176 | 1.00 | 31.82 | A |
| ATOM | 1073 | CD2 | LEU | A | 162 | 6.964 | 47.620 | 22.113 | 1.00 | 31.82 | A |
| ATOM | 1074 | C | LEU | A | 162 | 10.984 | 49.447 | 21.930 | 1.00 | 32.50 | A |
| ATOM | 1075 | O | LEU | A | 162 | 11.999 | 48.958 | 21.424 | 1.00 | 35.36 | A |
| ATOM | 1076 | N | LEU | A | 163 | 11.024 | 50.454 | 22.792 | 1.00 | 35.82 | A |
| ATOM | 1077 | CA | LEU | A | 163 | 12.286 | 51.062 | 23.174 | 1.00 | 35.03 | A |

```
ATOM    1078  CB   LEU A 163      12.192  52.578  22.986  1.00  37.59       A
ATOM    1079  CG   LEU A 163      11.718  53.040  21.611  1.00  38.36       A
ATOM    1080  CD1  LEU A 163      11.470  54.535  21.650  1.00  38.50       A
ATOM    1081  CD2  LEU A 163      12.742  52.651  20.554  1.00  38.47       A
ATOM    1082  C    LEU A 163      12.661  50.734  24.609  1.00  34.51       A
ATOM    1083  O    LEU A 163      11.795  50.510  25.439  1.00  35.39       A
ATOM    1084  N    LEU A 164      13.961  50.714  24.889  1.00  36.83       A
ATOM    1085  CA   LEU A 164      14.470  50.405  26.220  1.00  37.90       A
ATOM    1086  CB   LEU A 164      15.418  49.202  26.160  1.00  37.67       A
ATOM    1087  CG   LEU A 164      14.851  47.875  25.667  1.00  38.57       A
ATOM    1088  CD1  LEU A 164      15.945  46.838  25.634  1.00  38.95       A
ATOM    1089  CD2  LEU A 164      13.735  47.431  26.586  1.00  39.25       A
ATOM    1090  C    LEU A 164      15.214  51.583  26.829  1.00  37.18       A
ATOM    1091  O    LEU A 164      15.909  52.317  26.132  1.00  37.72       A
ATOM    1092  N    VAL A 165      15.050  51.755  28.136  1.00  38.84       A
ATOM    1093  CA   VAL A 165      15.727  52.803  28.889  1.00  38.92       A
ATOM    1094  CB   VAL A 165      14.830  54.053  29.097  1.00  38.24       A
ATOM    1095  CG1  VAL A 165      14.346  54.570  27.751  1.00  38.59       A
ATOM    1096  CG2  VAL A 165      13.669  53.734  30.018  1.00  38.66       A
ATOM    1097  C    VAL A 165      16.105  52.212  30.249  1.00  39.27       A
ATOM    1098  O    VAL A 165      15.826  51.041  30.520  1.00  39.71       A
ATOM    1099  N    ALA A 166      16.754  53.017  31.087  1.00  40.60       A
ATOM    1100  CA   ALA A 166      17.165  52.589  32.419  1.00  41.21       A
ATOM    1101  CB   ALA A 166      15.929  52.368  33.292  1.00  40.57       A
ATOM    1102  C    ALA A 166      18.028  51.329  32.412  1.00  41.56       A
ATOM    1103  O    ALA A 166      17.829  50.437  33.237  1.00  43.96       A
ATOM    1104  N    GLY A 167      18.983  51.261  31.488  1.00  41.13       A
ATOM    1105  CA   GLY A 167      19.860  50.103  31.407  1.00  39.59       A
ATOM    1106  C    GLY A 167      19.225  48.906  30.716  1.00  39.72       A
ATOM    1107  O    GLY A 167      19.627  47.761  30.949  1.00  38.72       A
ATOM    1108  N    GLY A 168      18.244  49.174  29.855  1.00  40.43       A
ATOM    1109  CA   GLY A 168      17.549  48.113  29.151  1.00  38.01       A
ATOM    1110  C    GLY A 168      16.562  47.443  30.087  1.00  38.12       A
ATOM    1111  O    GLY A 168      16.002  46.391  29.791  1.00  37.76       A
ATOM    1112  N    THR A 169      16.339  48.091  31.220  1.00  38.17       A
ATOM    1113  CA   THR A 169      15.454  47.607  32.272  1.00  38.29       A
ATOM    1114  CB   THR A 169      16.013  48.078  33.637  1.00  39.55       A
ATOM    1115  OG1  THR A 169      16.821  47.037  34.200  1.00  40.54       A
ATOM    1116  CG2  THR A 169      14.918  48.454  34.582  1.00  39.20       A
ATOM    1117  C    THR A 169      13.983  48.020  32.131  1.00  37.01       A
ATOM    1118  O    THR A 169      13.082  47.256  32.471  1.00  38.44       A
ATOM    1119  N    VAL A 170      13.749  49.230  31.635  1.00  36.20       A
ATOM    1120  CA   VAL A 170      12.401  49.758  31.459  1.00  35.63       A
ATOM    1121  CB   VAL A 170      12.284  51.168  32.055  1.00  33.42       A
ATOM    1122  CG1  VAL A 170      10.938  51.767  31.697  1.00  34.17       A
ATOM    1123  CG2  VAL A 170      12.473  51.110  33.555  1.00  33.79       A
ATOM    1124  C    VAL A 170      12.029  49.831  29.985  1.00  33.36       A
ATOM    1125  O    VAL A 170      12.794  50.346  29.171  1.00  31.08       A
ATOM    1126  N    LEU A 171      10.843  49.329  29.653  1.00  33.55       A
ATOM    1127  CA   LEU A 171      10.379  49.314  28.273  1.00  32.31       A
ATOM    1128  CB   LEU A 171       9.936  47.895  27.896  1.00  33.95       A
ATOM    1129  CG   LEU A 171       9.414  47.641  26.486  1.00  33.32       A
ATOM    1130  CD1  LEU A 171       9.717  46.213  26.070  1.00  33.27       A
ATOM    1131  CD2  LEU A 171       7.923  47.941  26.431  1.00  33.85       A
```

| ATOM | 1132 | C   | LEU | A | 171 | 9.253  | 50.315 | 28.010 | 1.00 | 32.96 | A |
|------|------|-----|-----|---|-----|--------|--------|--------|------|-------|---|
| ATOM | 1133 | O   | LEU | A | 171 | 8.394  | 50.545 | 28.862 | 1.00 | 32.80 | A |
| ATOM | 1134 | N   | LYS | A | 172 | 9.282  | 50.910 | 26.819 | 1.00 | 34.30 | A |
| ATOM | 1135 | CA  | LYS | A | 172 | 8.290  | 51.891 | 26.392 | 1.00 | 35.94 | A |
| ATOM | 1136 | CB  | LYS | A | 172 | 8.834  | 53.314 | 26.560 | 1.00 | 35.63 | A |
| ATOM | 1137 | CG  | LYS | A | 172 | 8.889  | 53.743 | 28.022 | 1.00 | 37.85 | A |
| ATOM | 1138 | CD  | LYS | A | 172 | 9.641  | 55.036 | 28.273 | 1.00 | 39.24 | A |
| ATOM | 1139 | CE  | LYS | A | 172 | 9.528  | 55.387 | 29.749 | 1.00 | 41.37 | A |
| ATOM | 1140 | NZ  | LYS | A | 172 | 10.606 | 56.289 | 30.233 | 1.00 | 42.40 | A |
| ATOM | 1141 | C   | LYS | A | 172 | 7.884  | 51.654 | 24.952 | 1.00 | 34.62 | A |
| ATOM | 1142 | O   | LYS | A | 172 | 8.687  | 51.248 | 24.122 | 1.00 | 35.55 | A |
| ATOM | 1143 | N   | ILE | A | 173 | 6.615  | 51.908 | 24.679 | 1.00 | 36.87 | A |
| ATOM | 1144 | CA  | ILE | A | 173 | 6.036  | 51.733 | 23.360 | 1.00 | 39.19 | A |
| ATOM | 1145 | CB  | ILE | A | 173 | 4.581  | 51.262 | 23.488 | 1.00 | 37.88 | A |
| ATOM | 1146 | CG2 | ILE | A | 173 | 4.008  | 50.955 | 22.123 | 1.00 | 38.34 | A |
| ATOM | 1147 | CG1 | ILE | A | 173 | 4.520  | 50.043 | 24.394 | 1.00 | 39.52 | A |
| ATOM | 1148 | CD1 | ILE | A | 173 | 3.138  | 49.707 | 24.833 | 1.00 | 40.82 | A |
| ATOM | 1149 | C   | ILE | A | 173 | 6.034  | 53.056 | 22.597 | 1.00 | 39.31 | A |
| ATOM | 1150 | O   | ILE | A | 173 | 5.848  | 54.124 | 23.183 | 1.00 | 37.63 | A |
| ATOM | 1151 | N   | CYS | A | 174 | 6.226  | 52.979 | 21.287 | 1.00 | 40.84 | A |
| ATOM | 1152 | CA  | CYS | A | 174 | 6.206  | 54.172 | 20.460 | 1.00 | 45.07 | A |
| ATOM | 1153 | CB  | CYS | A | 174 | 7.615  | 54.743 | 20.313 | 1.00 | 46.16 | A |
| ATOM | 1154 | SG  | CYS | A | 174 | 8.720  | 53.760 | 19.276 | 1.00 | 42.41 | A |
| ATOM | 1155 | C   | CYS | A | 174 | 5.623  | 53.826 | 19.089 | 1.00 | 46.45 | A |
| ATOM | 1156 | O   | CYS | A | 174 | 5.217  | 52.685 | 18.850 | 1.00 | 45.65 | A |
| ATOM | 1157 | N   | ASP | A | 175 | 5.577  | 54.818 | 18.203 | 1.00 | 49.13 | A |
| ATOM | 1158 | CA  | ASP | A | 175 | 5.049  | 54.666 | 16.846 | 1.00 | 52.94 | A |
| ATOM | 1159 | CB  | ASP | A | 175 | 6.098  | 54.060 | 15.908 | 1.00 | 55.68 | A |
| ATOM | 1160 | CG  | ASP | A | 175 | 7.313  | 54.950 | 15.727 | 1.00 | 59.25 | A |
| ATOM | 1161 | OD1 | ASP | A | 175 | 7.171  | 56.197 | 15.779 | 1.00 | 60.78 | A |
| ATOM | 1162 | OD2 | ASP | A | 175 | 8.416  | 54.395 | 15.511 | 1.00 | 61.26 | A |
| ATOM | 1163 | C   | ASP | A | 175 | 3.774  | 53.849 | 16.709 | 1.00 | 53.44 | A |
| ATOM | 1164 | O   | ASP | A | 175 | 3.814  | 52.715 | 16.225 | 1.00 | 52.02 | A |
| ATOM | 1165 | N   | PHE | A | 176 | 2.640  | 54.420 | 17.108 | 1.00 | 54.42 | A |
| ATOM | 1166 | CA  | PHE | A | 176 | 1.361  | 53.719 | 16.977 | 1.00 | 57.40 | A |
| ATOM | 1167 | CB  | PHE | A | 176 | 0.231  | 54.523 | 17.649 | 1.00 | 58.82 | A |
| ATOM | 1168 | CG  | PHE | A | 176 | 0.470  | 54.839 | 19.112 | 1.00 | 58.96 | A |
| ATOM | 1169 | CD1 | PHE | A | 176 | 1.657  | 54.480 | 19.752 | 1.00 | 60.17 | A |
| ATOM | 1170 | CD2 | PHE | A | 176 | -0.483 | 55.551 | 19.837 | 1.00 | 59.74 | A |
| ATOM | 1171 | CE1 | PHE | A | 176 | 1.892  | 54.835 | 21.091 | 1.00 | 60.20 | A |
| ATOM | 1172 | CE2 | PHE | A | 176 | -0.260 | 55.909 | 21.170 | 1.00 | 60.20 | A |
| ATOM | 1173 | CZ  | PHE | A | 176 | 0.932  | 55.550 | 21.795 | 1.00 | 60.30 | A |
| ATOM | 1174 | C   | PHE | A | 176 | 1.055  | 53.553 | 15.464 | 1.00 | 59.25 | A |
| ATOM | 1175 | O   | PHE | A | 176 | 1.376  | 54.442 | 14.665 | 1.00 | 59.05 | A |
| ATOM | 1176 | N   | GLY | A | 177 | 0.462  | 52.419 | 15.072 | 1.00 | 60.56 | A |
| ATOM | 1177 | CA  | GLY | A | 177 | 0.120  | 52.198 | 13.670 | 1.00 | 61.87 | A |
| ATOM | 1178 | C   | GLY | A | 177 | -0.880 | 53.247 | 13.194 | 1.00 | 63.02 | A |
| ATOM | 1179 | O   | GLY | A | 177 | -0.963 | 53.582 | 12.005 | 1.00 | 63.10 | A |
| ATOM | 1180 | N   | THR | A | 178 | -1.645 | 53.751 | 14.160 | 1.00 | 64.66 | A |
| ATOM | 1181 | CA  | THR | A | 178 | -2.658 | 54.793 | 13.982 | 1.00 | 65.77 | A |
| ATOM | 1182 | CB  | THR | A | 178 | -3.793 | 54.393 | 13.002 | 1.00 | 66.62 | A |
| ATOM | 1183 | OG1 | THR | A | 178 | -3.281 | 54.291 | 11.664 | 1.00 | 67.04 | A |
| ATOM | 1184 | CG2 | THR | A | 178 | -4.900 | 55.445 | 13.034 | 1.00 | 66.75 | A |
| ATOM | 1185 | C   | THR | A | 178 | -3.278 | 55.000 | 15.364 | 1.00 | 65.90 | A |

| ATOM | 1186 | O | THR | A | 178 | -2.931 | 54.291 | 16.322 | 1.00 | 66.48 | A |
|------|------|------|-----|---|-----|--------|--------|--------|------|-------|---|
| ATOM | 1187 | N | GLY | A | 191 | 5.670 | 47.453 | 10.202 | 1.00 | 55.52 | A |
| ATOM | 1188 | CA | GLY | A | 191 | 4.869 | 46.522 | 10.976 | 1.00 | 53.76 | A |
| ATOM | 1189 | C | GLY | A | 191 | 4.646 | 45.174 | 10.322 | 1.00 | 52.49 | A |
| ATOM | 1190 | O | GLY | A | 191 | 3.559 | 44.609 | 10.405 | 1.00 | 53.75 | A |
| ATOM | 1191 | N | SER | A | 192 | 5.688 | 44.664 | 9.678 | 1.00 | 50.56 | A |
| ATOM | 1192 | CA | SER | A | 192 | 5.681 | 43.383 | 8.983 | 1.00 | 47.35 | A |
| ATOM | 1193 | CB | SER | A | 192 | 7.044 | 42.725 | 9.176 | 1.00 | 49.74 | A |
| ATOM | 1194 | OG | SER | A | 192 | 8.069 | 43.652 | 8.868 | 1.00 | 50.11 | A |
| ATOM | 1195 | C | SER | A | 192 | 4.564 | 42.383 | 9.329 | 1.00 | 45.57 | A |
| ATOM | 1196 | O | SER | A | 192 | 4.013 | 42.363 | 10.433 | 1.00 | 44.27 | A |
| ATOM | 1197 | N | ALA | A | 193 | 4.258 | 41.534 | 8.358 | 1.00 | 43.57 | A |
| ATOM | 1198 | CA | ALA | A | 193 | 3.214 | 40.529 | 8.489 | 1.00 | 41.07 | A |
| ATOM | 1199 | CB | ALA | A | 193 | 2.981 | 39.868 | 7.121 | 1.00 | 42.05 | A |
| ATOM | 1200 | C | ALA | A | 193 | 3.504 | 39.461 | 9.547 | 1.00 | 40.03 | A |
| ATOM | 1201 | O | ALA | A | 193 | 2.625 | 38.668 | 9.886 | 1.00 | 39.47 | A |
| ATOM | 1202 | N | ALA | A | 194 | 4.727 | 39.443 | 10.065 | 1.00 | 39.03 | A |
| ATOM | 1203 | CA | ALA | A | 194 | 5.128 | 38.457 | 11.060 | 1.00 | 37.07 | A |
| ATOM | 1204 | CB | ALA | A | 194 | 6.642 | 38.455 | 11.199 | 1.00 | 39.77 | A |
| ATOM | 1205 | C | ALA | A | 194 | 4.485 | 38.655 | 12.427 | 1.00 | 37.02 | A |
| ATOM | 1206 | O | ALA | A | 194 | 4.422 | 37.727 | 13.216 | 1.00 | 38.00 | A |
| ATOM | 1207 | N | TRP | A | 195 | 3.987 | 39.856 | 12.692 | 1.00 | 39.15 | A |
| ATOM | 1208 | CA | TRP | A | 195 | 3.352 | 40.164 | 13.970 | 1.00 | 37.86 | A |
| ATOM | 1209 | CB | TRP | A | 195 | 4.092 | 41.326 | 14.641 | 1.00 | 37.57 | A |
| ATOM | 1210 | CG | TRP | A | 195 | 5.535 | 41.034 | 14.959 | 1.00 | 38.57 | A |
| ATOM | 1211 | CD2 | TRP | A | 195 | 6.672 | 41.294 | 14.121 | 1.00 | 38.19 | A |
| ATOM | 1212 | CE2 | TRP | A | 195 | 7.819 | 40.857 | 14.829 | 1.00 | 38.99 | A |
| ATOM | 1213 | CE3 | TRP | A | 195 | 6.834 | 41.852 | 12.842 | 1.00 | 39.14 | A |
| ATOM | 1214 | CD1 | TRP | A | 195 | 6.026 | 40.463 | 16.103 | 1.00 | 37.73 | A |
| ATOM | 1215 | NE1 | TRP | A | 195 | 7.393 | 40.356 | 16.032 | 1.00 | 37.27 | A |
| ATOM | 1216 | CZ2 | TRP | A | 195 | 9.116 | 40.961 | 14.298 | 1.00 | 39.21 | A |
| ATOM | 1217 | CZ3 | TRP | A | 195 | 8.126 | 41.957 | 12.312 | 1.00 | 38.91 | A |
| ATOM | 1218 | CH2 | TRP | A | 195 | 9.246 | 41.514 | 13.042 | 1.00 | 39.15 | A |
| ATOM | 1219 | C | TRP | A | 195 | 1.871 | 40.531 | 13.833 | 1.00 | 38.08 | A |
| ATOM | 1220 | O | TRP | A | 195 | 1.203 | 40.813 | 14.825 | 1.00 | 40.71 | A |
| ATOM | 1221 | N | MET | A | 196 | 1.356 | 40.536 | 12.611 | 1.00 | 40.65 | A |
| ATOM | 1222 | CA | MET | A | 196 | -0.038 | 40.897 | 12.381 | 1.00 | 40.82 | A |
| ATOM | 1223 | CB | MET | A | 196 | -0.234 | 41.409 | 10.964 | 1.00 | 44.38 | A |
| ATOM | 1224 | CG | MET | A | 196 | 0.200 | 42.813 | 10.709 | 1.00 | 49.17 | A |
| ATOM | 1225 | SD | MET | A | 196 | -0.757 | 43.387 | 9.285 | 1.00 | 48.45 | A |
| ATOM | 1226 | CE | MET | A | 196 | -0.031 | 42.403 | 7.950 | 1.00 | 55.48 | A |
| ATOM | 1227 | C | MET | A | 196 | -1.021 | 39.773 | 12.581 | 1.00 | 37.86 | A |
| ATOM | 1228 | O | MET | A | 196 | -0.753 | 38.634 | 12.210 | 1.00 | 37.21 | A |
| ATOM | 1229 | N | ALA | A | 197 | -2.179 | 40.116 | 13.138 | 1.00 | 37.15 | A |
| ATOM | 1230 | CA | ALA | A | 197 | -3.233 | 39.142 | 13.361 | 1.00 | 37.80 | A |
| ATOM | 1231 | CB | ALA | A | 197 | -4.290 | 39.707 | 14.305 | 1.00 | 35.91 | A |
| ATOM | 1232 | C | ALA | A | 197 | -3.829 | 38.863 | 11.986 | 1.00 | 36.87 | A |
| ATOM | 1233 | O | ALA | A | 197 | -4.037 | 39.785 | 11.194 | 1.00 | 37.83 | A |
| ATOM | 1234 | N | PRO | A | 198 | -4.115 | 37.587 | 11.683 | 1.00 | 39.30 | A |
| ATOM | 1235 | CD | PRO | A | 198 | -4.022 | 36.388 | 12.530 | 1.00 | 39.45 | A |
| ATOM | 1236 | CA | PRO | A | 198 | -4.682 | 37.263 | 10.374 | 1.00 | 40.88 | A |
| ATOM | 1237 | CB | PRO | A | 198 | -4.940 | 35.753 | 10.472 | 1.00 | 40.60 | A |
| ATOM | 1238 | CG | PRO | A | 198 | -5.080 | 35.507 | 11.944 | 1.00 | 39.97 | A |
| ATOM | 1239 | C | PRO | A | 198 | -5.914 | 38.069 | 9.919 | 1.00 | 40.55 | A |

| | | | | | | | | | | |
|---|---|---|---|---|---|---|---|---|---|---|
| ATOM | 1240 | O | PRO | A | 198 | -6.036 | 38.387 | 8.732 | 1.00 41.25 | A |
| ATOM | 1241 | N | GLU | A | 199 | -6.812 | 38.415 | 10.837 | 1.00 43.49 | A |
| ATOM | 1242 | CA | GLU | A | 199 | -8.003 | 39.169 | 10.458 | 1.00 43.62 | A |
| ATOM | 1243 | CB | GLU | A | 199 | -8.995 | 39.288 | 11.629 | 1.00 42.53 | A |
| ATOM | 1244 | CG | GLU | A | 199 | -8.528 | 40.140 | 12.795 | 1.00 39.66 | A |
| ATOM | 1245 | CD | GLU | A | 199 | -7.729 | 39.348 | 13.819 | 1.00 38.38 | A |
| ATOM | 1246 | OE1 | GLU | A | 199 | -7.365 | 38.183 | 13.534 | 1.00 36.91 | A |
| ATOM | 1247 | OE2 | GLU | A | 199 | -7.461 | 39.898 | 14.912 | 1.00 38.36 | A |
| ATOM | 1248 | C | GLU | A | 199 | -7.633 | 40.558 | 9.966 | 1.00 45.11 | A |
| ATOM | 1249 | O | GLU | A | 199 | -8.452 | 41.250 | 9.362 | 1.00 46.78 | A |
| ATOM | 1250 | N | VAL | A | 200 | -6.400 | 40.974 | 10.218 | 1.00 48.83 | A |
| ATOM | 1251 | CA | VAL | A | 200 | -5.993 | 42.283 | 9.759 | 1.00 49.61 | A |
| ATOM | 1252 | CB | VAL | A | 200 | -4.846 | 42.854 | 10.607 | 1.00 49.72 | A |
| ATOM | 1253 | CG1 | VAL | A | 200 | -4.447 | 44.198 | 10.072 | 1.00 49.86 | A |
| ATOM | 1254 | CG2 | VAL | A | 200 | -5.294 | 43.005 | 12.049 | 1.00 50.06 | A |
| ATOM | 1255 | C | VAL | A | 200 | -5.602 | 42.254 | 8.277 | 1.00 52.10 | A |
| ATOM | 1256 | O | VAL | A | 200 | -6.007 | 43.152 | 7.532 | 1.00 52.16 | A |
| ATOM | 1257 | N | PHE | A | 201 | -4.839 | 41.245 | 7.825 | 1.00 54.70 | A |
| ATOM | 1258 | CA | PHE | A | 201 | -4.494 | 41.235 | 6.402 | 1.00 57.22 | A |
| ATOM | 1259 | CB | PHE | A | 201 | -3.517 | 40.138 | 5.900 | 1.00 55.77 | A |
| ATOM | 1260 | CG | PHE | A | 201 | -2.902 | 39.253 | 6.938 | 1.00 54.60 | A |
| ATOM | 1261 | CD1 | PHE | A | 201 | -1.951 | 39.745 | 7.824 | 1.00 54.39 | A |
| ATOM | 1262 | CD2 | PHE | A | 201 | -3.139 | 37.871 | 6.898 | 1.00 54.30 | A |
| ATOM | 1263 | CE1 | PHE | A | 201 | -1.228 | 38.878 | 8.649 | 1.00 54.19 | A |
| ATOM | 1264 | CE2 | PHE | A | 201 | -2.426 | 36.994 | 7.716 | 1.00 53.81 | A |
| ATOM | 1265 | CZ | PHE | A | 201 | -1.465 | 37.498 | 8.594 | 1.00 53.67 | A |
| ATOM | 1266 | C | PHE | A | 201 | -5.757 | 41.011 | 5.607 | 1.00 59.99 | A |
| ATOM | 1267 | O | PHE | A | 201 | -6.005 | 41.709 | 4.625 | 1.00 61.11 | A |
| ATOM | 1268 | N | GLU | A | 202 | -6.553 | 40.028 | 6.016 | 1.00 62.22 | A |
| ATOM | 1269 | CA | GLU | A | 202 | -7.770 | 39.725 | 5.283 | 1.00 64.62 | A |
| ATOM | 1270 | CB | GLU | A | 202 | -8.431 | 38.464 | 5.846 | 1.00 65.21 | A |
| ATOM | 1271 | CG | GLU | A | 202 | -9.256 | 38.671 | 7.089 | 1.00 66.65 | A |
| ATOM | 1272 | CD | GLU | A | 202 | -9.718 | 37.360 | 7.683 | 1.00 67.44 | A |
| ATOM | 1273 | OE1 | GLU | A | 202 | -10.671 | 37.382 | 8.498 | 1.00 67.65 | A |
| ATOM | 1274 | OE2 | GLU | A | 202 | -9.115 | 36.315 | 7.335 | 1.00 68.40 | A |
| ATOM | 1275 | C | GLU | A | 202 | -8.750 | 40.898 | 5.245 | 1.00 65.39 | A |
| ATOM | 1276 | O | GLU | A | 202 | -9.951 | 40.712 | 5.039 | 1.00 66.43 | A |
| ATOM | 1277 | N | GLY | A | 203 | -8.210 | 42.102 | 5.439 | 1.00 66.13 | A |
| ATOM | 1278 | CA | GLY | A | 203 | -8.992 | 43.324 | 5.392 | 1.00 66.94 | A |
| ATOM | 1279 | C | GLY | A | 203 | -10.076 | 43.452 | 6.437 | 1.00 67.47 | A |
| ATOM | 1280 | O | GLY | A | 203 | -10.247 | 44.520 | 7.027 | 1.00 67.84 | A |
| ATOM | 1281 | N | SER | A | 204 | -10.797 | 42.358 | 6.664 | 1.00 67.72 | A |
| ATOM | 1282 | CA | SER | A | 204 | -11.898 | 42.301 | 7.626 | 1.00 67.36 | A |
| ATOM | 1283 | CB | SER | A | 204 | -12.183 | 40.842 | 8.017 | 1.00 68.13 | A |
| ATOM | 1284 | OG | SER | A | 204 | -11.146 | 40.310 | 8.821 | 1.00 68.81 | A |
| ATOM | 1285 | C | SER | A | 204 | -11.716 | 43.136 | 8.896 | 1.00 66.83 | A |
| ATOM | 1286 | O | SER | A | 204 | -10.598 | 43.507 | 9.279 | 1.00 66.77 | A |
| ATOM | 1287 | N | ASN | A | 205 | -12.843 | 43.428 | 9.539 | 1.00 65.95 | A |
| ATOM | 1288 | CA | ASN | A | 205 | -12.866 | 44.216 | 10.769 | 1.00 65.31 | A |
| ATOM | 1289 | CB | ASN | A | 205 | -14.301 | 44.302 | 11.310 | 1.00 67.55 | A |
| ATOM | 1290 | CG | ASN | A | 205 | -15.305 | 44.709 | 10.243 | 1.00 69.24 | A |
| ATOM | 1291 | OD1 | ASN | A | 205 | -15.190 | 45.782 | 9.643 | 1.00 70.15 | A |
| ATOM | 1292 | ND2 | ASN | A | 205 | -16.297 | 43.850 | 10.001 | 1.00 69.59 | A |
| ATOM | 1293 | C | ASN | A | 205 | -11.966 | 43.584 | 11.829 | 1.00 63.06 | A |

| | | | | | | | | | | |
|---|---|---|---|---|---|---|---|---|---|---|
| ATOM | 1294 | O | ASN | A | 205 | -11.603 | 42.405 | 11.743 | 1.00 63.26 | A |
| ATOM | 1295 | N | TYR | A | 206 | -11.609 | 44.371 | 12.834 | 1.00 59.73 | A |
| ATOM | 1296 | CA | TYR | A | 206 | -10.762 | 43.864 | 13.895 | 1.00 56.76 | A |
| ATOM | 1297 | CB | TYR | A | 206 | -9.320 | 43.735 | 13.394 | 1.00 56.61 | A |
| ATOM | 1298 | CG | TYR | A | 206 | -8.686 | 45.041 | 13.003 | 1.00 56.58 | A |
| ATOM | 1299 | CD1 | TYR | A | 206 | -8.282 | 45.960 | 13.976 | 1.00 56.38 | A |
| ATOM | 1300 | CE1 | TYR | A | 206 | -7.706 | 47.172 | 13.630 | 1.00 57.45 | A |
| ATOM | 1301 | CD2 | TYR | A | 206 | -8.497 | 45.365 | 11.665 | 1.00 56.54 | A |
| ATOM | 1302 | CE2 | TYR | A | 206 | -7.921 | 46.576 | 11.304 | 1.00 56.93 | A |
| ATOM | 1303 | CZ | TYR | A | 206 | -7.528 | 47.477 | 12.292 | 1.00 56.88 | A |
| ATOM | 1304 | OH | TYR | A | 206 | -6.968 | 48.681 | 11.934 | 1.00 57.10 | A |
| ATOM | 1305 | C | TYR | A | 206 | -10.850 | 44.777 | 15.114 | 1.00 54.54 | A |
| ATOM | 1306 | O | TYR | A | 206 | -11.307 | 45.921 | 15.014 | 1.00 54.28 | A |
| ATOM | 1307 | N | SER | A | 207 | -10.413 | 44.266 | 16.263 | 1.00 50.86 | A |
| ATOM | 1308 | CA | SER | A | 207 | -10.466 | 45.014 | 17.515 | 1.00 48.28 | A |
| ATOM | 1309 | CB | SER | A | 207 | -11.448 | 44.320 | 18.466 | 1.00 48.77 | A |
| ATOM | 1310 | OG | SER | A | 207 | -11.128 | 42.944 | 18.616 | 1.00 47.98 | A |
| ATOM | 1311 | C | SER | A | 207 | -9.096 | 45.117 | 18.172 | 1.00 46.26 | A |
| ATOM | 1312 | O | SER | A | 207 | -8.078 | 44.891 | 17.529 | 1.00 45.65 | A |
| ATOM | 1313 | N | GLU | A | 208 | -9.084 | 45.461 | 19.458 | 1.00 43.27 | A |
| ATOM | 1314 | CA | GLU | A | 208 | -7.847 | 45.573 | 20.220 | 1.00 42.28 | A |
| ATOM | 1315 | CB | GLU | A | 208 | -8.110 | 46.105 | 21.633 | 1.00 45.16 | A |
| ATOM | 1316 | CG | GLU | A | 208 | -9.355 | 46.936 | 21.801 | 1.00 48.42 | A |
| ATOM | 1317 | CD | GLU | A | 208 | -10.621 | 46.095 | 21.748 | 1.00 51.10 | A |
| ATOM | 1318 | OE1 | GLU | A | 208 | -10.639 | 45.014 | 22.378 | 1.00 52.25 | A |
| ATOM | 1319 | OE2 | GLU | A | 208 | -11.598 | 46.517 | 21.087 | 1.00 52.55 | A |
| ATOM | 1320 | C | GLU | A | 208 | -7.219 | 44.190 | 20.348 | 1.00 39.12 | A |
| ATOM | 1321 | O | GLU | A | 208 | -6.076 | 44.061 | 20.783 | 1.00 39.71 | A |
| ATOM | 1322 | N | LYS | A | 209 | -7.981 | 43.159 | 19.987 | 1.00 37.28 | A |
| ATOM | 1323 | CA | LYS | A | 209 | -7.510 | 41.780 | 20.057 | 1.00 34.45 | A |
| ATOM | 1324 | CB | LYS | A | 209 | -8.670 | 40.807 | 19.835 | 1.00 32.43 | A |
| ATOM | 1325 | CG | LYS | A | 209 | -9.665 | 40.783 | 20.978 | 1.00 30.93 | A |
| ATOM | 1326 | CD | LYS | A | 209 | -8.946 | 40.505 | 22.275 | 1.00 30.65 | A |
| ATOM | 1327 | CE | LYS | A | 209 | -9.884 | 40.483 | 23.467 | 1.00 31.19 | A |
| ATOM | 1328 | NZ | LYS | A | 209 | -10.792 | 39.324 | 23.421 | 1.00 31.10 | A |
| ATOM | 1329 | C | LYS | A | 209 | -6.415 | 41.517 | 19.036 | 1.00 32.23 | A |
| ATOM | 1330 | O | LYS | A | 209 | -5.731 | 40.489 | 19.094 | 1.00 33.15 | A |
| ATOM | 1331 | N | CYS | A | 210 | -6.250 | 42.442 | 18.092 | 1.00 32.73 | A |
| ATOM | 1332 | CA | CYS | A | 210 | -5.203 | 42.284 | 17.089 | 1.00 33.44 | A |
| ATOM | 1333 | CB | CYS | A | 210 | -5.455 | 43.197 | 15.867 | 1.00 37.94 | A |
| ATOM | 1334 | SG | CYS | A | 210 | -5.227 | 44.987 | 16.096 | 1.00 61.53 | A |
| ATOM | 1335 | C | CYS | A | 210 | -3.860 | 42.590 | 17.770 | 1.00 33.55 | A |
| ATOM | 1336 | O | CYS | A | 210 | -2.835 | 42.016 | 17.407 | 1.00 28.76 | A |
| ATOM | 1337 | N | ASP | A | 211 | -3.883 | 43.468 | 18.778 | 1.00 30.11 | A |
| ATOM | 1338 | CA | ASP | A | 211 | -2.678 | 43.829 | 19.524 | 1.00 25.08 | A |
| ATOM | 1339 | CB | ASP | A | 211 | -2.898 | 45.085 | 20.386 | 1.00 29.38 | A |
| ATOM | 1340 | CG | ASP | A | 211 | -2.863 | 46.384 | 19.582 | 1.00 29.54 | A |
| ATOM | 1341 | OD1 | ASP | A | 211 | -2.274 | 46.405 | 18.480 | 1.00 31.69 | A |
| ATOM | 1342 | OD2 | ASP | A | 211 | -3.407 | 47.397 | 20.066 | 1.00 30.09 | A |
| ATOM | 1343 | C | ASP | A | 211 | -2.273 | 42.678 | 20.432 | 1.00 27.09 | A |
| ATOM | 1344 | O | ASP | A | 211 | -1.097 | 42.513 | 20.736 | 1.00 25.89 | A |
| ATOM | 1345 | N | VAL | A | 212 | -3.252 | 41.892 | 20.873 | 1.00 27.76 | A |
| ATOM | 1346 | CA | VAL | A | 212 | -2.991 | 40.744 | 21.742 | 1.00 25.00 | A |
| ATOM | 1347 | CB | VAL | A | 212 | -4.295 | 40.200 | 22.366 | 1.00 26.31 | A |

| ATOM | 1348 | CG1 | VAL | A | 212 | -4.061 | 38.820 | 22.974 | 1.00 | 26.17 | A |
|------|------|-----|-----|---|-----|--------|--------|--------|------|-------|---|
| ATOM | 1349 | CG2 | VAL | A | 212 | -4.788 | 41.160 | 23.423 | 1.00 | 27.10 | A |
| ATOM | 1350 | C | VAL | A | 212 | -2.297 | 39.630 | 20.960 | 1.00 | 25.07 | A |
| ATOM | 1351 | O | VAL | A | 212 | -1.495 | 38.875 | 21.512 | 1.00 | 25.35 | A |
| ATOM | 1352 | N | PHE | A | 213 | -2.607 | 39.535 | 19.670 | 1.00 | 26.10 | A |
| ATOM | 1353 | CA | PHE | A | 213 | -1.976 | 38.541 | 18.821 | 1.00 | 24.38 | A |
| ATOM | 1354 | CB | PHE | A | 213 | -2.687 | 38.493 | 17.471 | 1.00 | 25.78 | A |
| ATOM | 1355 | CG | PHE | A | 213 | -2.085 | 37.520 | 16.509 | 1.00 | 27.68 | A |
| ATOM | 1356 | CD1 | PHE | A | 213 | -0.912 | 37.826 | 15.840 | 1.00 | 27.37 | A |
| ATOM | 1357 | CD2 | PHE | A | 213 | -2.683 | 36.288 | 16.288 | 1.00 | 27.70 | A |
| ATOM | 1358 | CE1 | PHE | A | 213 | -0.353 | 36.920 | 14.969 | 1.00 | 28.22 | A |
| ATOM | 1359 | CE2 | PHE | A | 213 | -2.126 | 35.372 | 15.416 | 1.00 | 26.96 | A |
| ATOM | 1360 | CZ | PHE | A | 213 | -0.963 | 35.686 | 14.756 | 1.00 | 28.93 | A |
| ATOM | 1361 | C | PHE | A | 213 | -0.488 | 38.894 | 18.642 | 1.00 | 26.85 | A |
| ATOM | 1362 | O | PHE | A | 213 | 0.379 | 38.027 | 18.736 | 1.00 | 24.95 | A |
| ATOM | 1363 | N | SER | A | 214 | -0.205 | 40.171 | 18.395 | 1.00 | 26.81 | A |
| ATOM | 1364 | CA | SER | A | 214 | 1.161 | 40.655 | 18.213 | 1.00 | 26.62 | A |
| ATOM | 1365 | CB | SER | A | 214 | 1.159 | 42.149 | 17.896 | 1.00 | 27.29 | A |
| ATOM | 1366 | OG | SER | A | 214 | 0.389 | 42.422 | 16.748 | 1.00 | 31.29 | A |
| ATOM | 1367 | C | SER | A | 214 | 1.966 | 40.421 | 19.477 | 1.00 | 25.53 | A |
| ATOM | 1368 | O | SER | A | 214 | 3.150 | 40.084 | 19.412 | 1.00 | 24.86 | A |
| ATOM | 1369 | N | TRP | A | 215 | 1.315 | 40.622 | 20.625 | 1.00 | 22.83 | A |
| ATOM | 1370 | CA | TRP | A | 215 | 1.950 | 40.428 | 21.927 | 1.00 | 22.75 | A |
| ATOM | 1371 | CB | TRP | A | 215 | 0.970 | 40.720 | 23.067 | 1.00 | 25.03 | A |
| ATOM | 1372 | CG | TRP | A | 215 | 1.635 | 40.728 | 24.422 | 1.00 | 22.75 | A |
| ATOM | 1373 | CD2 | TRP | A | 215 | 1.746 | 39.628 | 25.344 | 1.00 | 26.32 | A |
| ATOM | 1374 | CE2 | TRP | A | 215 | 2.480 | 40.089 | 26.458 | 1.00 | 25.83 | A |
| ATOM | 1375 | CE3 | TRP | A | 215 | 1.297 | 38.301 | 25.336 | 1.00 | 24.79 | A |
| ATOM | 1376 | CD1 | TRP | A | 215 | 2.289 | 41.778 | 25.002 | 1.00 | 24.87 | A |
| ATOM | 1377 | NE1 | TRP | A | 215 | 2.796 | 41.402 | 26.223 | 1.00 | 24.07 | A |
| ATOM | 1378 | CZ2 | TRP | A | 215 | 2.778 | 39.262 | 27.560 | 1.00 | 23.13 | A |
| ATOM | 1379 | CZ3 | TRP | A | 215 | 1.597 | 37.481 | 26.435 | 1.00 | 23.18 | A |
| ATOM | 1380 | CH2 | TRP | A | 215 | 2.329 | 37.969 | 27.525 | 1.00 | 23.67 | A |
| ATOM | 1381 | C | TRP | A | 215 | 2.431 | 38.989 | 22.057 | 1.00 | 22.88 | A |
| ATOM | 1382 | O | TRP | A | 215 | 3.536 | 38.732 | 22.518 | 1.00 | 20.95 | A |
| ATOM | 1383 | N | GLY | A | 216 | 1.578 | 38.055 | 21.651 | 1.00 | 23.99 | A |
| ATOM | 1384 | CA | GLY | A | 216 | 1.927 | 36.645 | 21.716 | 1.00 | 21.90 | A |
| ATOM | 1385 | C | GLY | A | 216 | 3.178 | 36.315 | 20.921 | 1.00 | 23.01 | A |
| ATOM | 1386 | O | GLY | A | 216 | 4.025 | 35.563 | 21.393 | 1.00 | 22.29 | A |
| ATOM | 1387 | N | ILE | A | 217 | 3.285 | 36.880 | 19.717 | 1.00 | 25.00 | A |
| ATOM | 1388 | CA | ILE | A | 217 | 4.436 | 36.663 | 18.844 | 1.00 | 25.63 | A |
| ATOM | 1389 | CB | ILE | A | 217 | 4.197 | 37.249 | 17.408 | 1.00 | 23.63 | A |
| ATOM | 1390 | CG2 | ILE | A | 217 | 5.409 | 36.984 | 16.519 | 1.00 | 25.94 | A |
| ATOM | 1391 | CG1 | ILE | A | 217 | 2.937 | 36.639 | 16.787 | 1.00 | 25.16 | A |
| ATOM | 1392 | CD1 | ILE | A | 217 | 3.022 | 35.173 | 16.480 | 1.00 | 24.59 | A |
| ATOM | 1393 | C | ILE | A | 217 | 5.687 | 37.299 | 19.464 | 1.00 | 24.09 | A |
| ATOM | 1394 | O | ILE | A | 217 | 6.768 | 36.721 | 19.397 | 1.00 | 25.72 | A |
| ATOM | 1395 | N | ILE | A | 218 | 5.560 | 38.478 | 20.063 | 1.00 | 26.04 | A |
| ATOM | 1396 | CA | ILE | A | 218 | 6.716 | 39.109 | 20.687 | 1.00 | 26.69 | A |
| ATOM | 1397 | CB | ILE | A | 218 | 6.383 | 40.563 | 21.134 | 1.00 | 26.56 | A |
| ATOM | 1398 | CG2 | ILE | A | 218 | 7.490 | 41.110 | 22.032 | 1.00 | 27.31 | A |
| ATOM | 1399 | CG1 | ILE | A | 218 | 6.200 | 41.455 | 19.899 | 1.00 | 28.81 | A |
| ATOM | 1400 | CD1 | ILE | A | 218 | 5.677 | 42.847 | 20.201 | 1.00 | 28.39 | A |
| ATOM | 1401 | C | ILE | A | 218 | 7.229 | 38.292 | 21.882 | 1.00 | 25.19 | A |

| ATOM | 1402 | O | ILE | A | 218 | 8.432 | 38.257 | 22.140 | 1.00 | 26.06 | A |
|------|------|------|-----|---|-----|-------|--------|--------|------|-------|---|
| ATOM | 1403 | N | LEU | A | 219 | 6.320 | 37.626 | 22.594 | 1.00 | 24.70 | A |
| ATOM | 1404 | CA | LEU | A | 219 | 6.679 | 36.787 | 23.746 | 1.00 | 24.55 | A |
| ATOM | 1405 | CB | LEU | A | 219 | 5.412 | 36.292 | 24.463 | 1.00 | 26.28 | A |
| ATOM | 1406 | CG | LEU | A | 219 | 5.555 | 35.375 | 25.687 | 1.00 | 26.58 | A |
| ATOM | 1407 | CD1 | LEU | A | 219 | 6.308 | 36.086 | 26.808 | 1.00 | 25.95 | A |
| ATOM | 1408 | CD2 | LEU | A | 219 | 4.170 | 34.970 | 26.155 | 1.00 | 25.50 | A |
| ATOM | 1409 | C | LEU | A | 219 | 7.507 | 35.583 | 23.278 | 1.00 | 26.66 | A |
| ATOM | 1410 | O | LEU | A | 219 | 8.518 | 35.228 | 23.895 | 1.00 | 25.70 | A |
| ATOM | 1411 | N | TRP | A | 220 | 7.071 | 34.957 | 22.185 | 1.00 | 27.27 | A |
| ATOM | 1412 | CA | TRP | A | 220 | 7.784 | 33.816 | 21.614 | 1.00 | 25.34 | A |
| ATOM | 1413 | CB | TRP | A | 220 | 7.057 | 33.312 | 20.363 | 1.00 | 26.84 | A |
| ATOM | 1414 | CG | TRP | A | 220 | 7.771 | 32.210 | 19.615 | 1.00 | 27.02 | A |
| ATOM | 1415 | CD2 | TRP | A | 220 | 8.736 | 32.369 | 18.555 | 1.00 | 28.68 | A |
| ATOM | 1416 | CE2 | TRP | A | 220 | 9.165 | 31.076 | 18.184 | 1.00 | 24.08 | A |
| ATOM | 1417 | CE3 | TRP | A | 220 | 9.277 | 33.481 | 17.887 | 1.00 | 28.10 | A |
| ATOM | 1418 | CD1 | TRP | A | 220 | 7.658 | 30.866 | 19.833 | 1.00 | 26.66 | A |
| ATOM | 1419 | NE1 | TRP | A | 220 | 8.493 | 30.179 | 18.975 | 1.00 | 26.58 | A |
| ATOM | 1420 | CZ2 | TRP | A | 220 | 10.110 | 30.861 | 17.172 | 1.00 | 27.38 | A |
| ATOM | 1421 | CZ3 | TRP | A | 220 | 10.218 | 33.270 | 16.878 | 1.00 | 26.46 | A |
| ATOM | 1422 | CH2 | TRP | A | 220 | 10.624 | 31.963 | 16.532 | 1.00 | 28.37 | A |
| ATOM | 1423 | C | TRP | A | 220 | 9.158 | 34.333 | 21.223 | 1.00 | 27.31 | A |
| ATOM | 1424 | O | TRP | A | 220 | 10.171 | 33.720 | 21.521 | 1.00 | 27.08 | A |
| ATOM | 1425 | N | GLU | A | 221 | 9.181 | 35.495 | 20.586 | 1.00 | 28.70 | A |
| ATOM | 1426 | CA | GLU | A | 221 | 10.419 | 36.087 | 20.119 | 1.00 | 29.91 | A |
| ATOM | 1427 | CB | GLU | A | 221 | 10.091 | 37.287 | 19.232 | 1.00 | 31.92 | A |
| ATOM | 1428 | CG | GLU | A | 221 | 11.290 | 38.047 | 18.703 | 1.00 | 37.47 | A |
| ATOM | 1429 | CD | GLU | A | 221 | 10.921 | 38.968 | 17.539 | 1.00 | 40.41 | A |
| ATOM | 1430 | OE1 | GLU | A | 221 | 9.733 | 39.370 | 17.462 | 1.00 | 43.09 | A |
| ATOM | 1431 | OE2 | GLU | A | 221 | 11.807 | 39.295 | 16.707 | 1.00 | 41.31 | A |
| ATOM | 1432 | C | GLU | A | 221 | 11.440 | 36.461 | 21.195 | 1.00 | 27.30 | A |
| ATOM | 1433 | O | GLU | A | 221 | 12.629 | 36.255 | 20.979 | 1.00 | 26.35 | A |
| ATOM | 1434 | N | VAL | A | 222 | 11.021 | 36.987 | 22.346 | 1.00 | 30.27 | A |
| ATOM | 1435 | CA | VAL | A | 222 | 12.017 | 37.329 | 23.370 | 1.00 | 27.61 | A |
| ATOM | 1436 | CB | VAL | A | 222 | 11.499 | 38.377 | 24.403 | 1.00 | 27.73 | A |
| ATOM | 1437 | CG1 | VAL | A | 222 | 11.134 | 39.678 | 23.704 | 1.00 | 27.77 | A |
| ATOM | 1438 | CG2 | VAL | A | 222 | 10.327 | 37.813 | 25.169 | 1.00 | 30.44 | A |
| ATOM | 1439 | C | VAL | A | 222 | 12.516 | 36.094 | 24.141 | 1.00 | 26.65 | A |
| ATOM | 1440 | O | VAL | A | 222 | 13.627 | 36.094 | 24.683 | 1.00 | 28.03 | A |
| ATOM | 1441 | N | ILE | A | 223 | 11.701 | 35.044 | 24.172 | 1.00 | 27.09 | A |
| ATOM | 1442 | CA | ILE | A | 223 | 12.057 | 33.798 | 24.861 | 1.00 | 28.50 | A |
| ATOM | 1443 | CB | ILE | A | 223 | 10.799 | 32.911 | 25.134 | 1.00 | 30.22 | A |
| ATOM | 1444 | CG2 | ILE | A | 223 | 11.230 | 31.513 | 25.537 | 1.00 | 27.78 | A |
| ATOM | 1445 | CG1 | ILE | A | 223 | 9.899 | 33.558 | 26.196 | 1.00 | 27.45 | A |
| ATOM | 1446 | CD1 | ILE | A | 223 | 8.567 | 32.845 | 26.410 | 1.00 | 27.45 | A |
| ATOM | 1447 | C | ILE | A | 223 | 13.038 | 32.958 | 24.028 | 1.00 | 27.00 | A |
| ATOM | 1448 | O | ILE | A | 223 | 13.954 | 32.347 | 24.565 | 1.00 | 26.98 | A |
| ATOM | 1449 | N | THR | A | 224 | 12.835 | 32.935 | 22.713 | 1.00 | 29.09 | A |
| ATOM | 1450 | CA | THR | A | 224 | 13.685 | 32.162 | 21.812 | 1.00 | 28.16 | A |
| ATOM | 1451 | CB | THR | A | 224 | 12.860 | 31.543 | 20.658 | 1.00 | 28.18 | A |
| ATOM | 1452 | OG1 | THR | A | 224 | 12.311 | 32.595 | 19.863 | 1.00 | 30.31 | A |
| ATOM | 1453 | CG2 | THR | A | 224 | 11.722 | 30.686 | 21.192 | 1.00 | 28.31 | A |
| ATOM | 1454 | C | THR | A | 224 | 14.797 | 33.000 | 21.186 | 1.00 | 31.35 | A |
| ATOM | 1455 | O | THR | A | 224 | 15.806 | 32.460 | 20.746 | 1.00 | 30.54 | A |

| ATOM | 1456 | N   | ARG | A | 225 | 14.618 | 34.316 | 21.169 | 1.00 | 30.12 | A |
|------|------|-----|-----|---|-----|--------|--------|--------|------|-------|---|
| ATOM | 1457 | CA  | ARG | A | 225 | 15.580 | 35.236 | 20.567 | 1.00 | 30.85 | A |
| ATOM | 1458 | CB  | ARG | A | 225 | 16.908 | 35.260 | 21.334 | 1.00 | 31.80 | A |
| ATOM | 1459 | CG  | ARG | A | 225 | 17.697 | 36.554 | 21.096 | 1.00 | 30.30 | A |
| ATOM | 1460 | CD  | ARG | A | 225 | 19.004 | 36.643 | 21.870 | 1.00 | 29.49 | A |
| ATOM | 1461 | NE  | ARG | A | 225 | 19.591 | 37.983 | 21.776 | 1.00 | 31.70 | A |
| ATOM | 1462 | CZ  | ARG | A | 225 | 20.484 | 38.489 | 22.623 | 1.00 | 28.65 | A |
| ATOM | 1463 | NH1 | ARG | A | 225 | 20.921 | 37.766 | 23.642 | 1.00 | 28.93 | A |
| ATOM | 1464 | NH2 | ARG | A | 225 | 20.899 | 39.739 | 22.480 | 1.00 | 28.47 | A |
| ATOM | 1465 | C   | ARG | A | 225 | 15.809 | 34.854 | 19.101 | 1.00 | 32.51 | A |
| ATOM | 1466 | O   | ARG | A | 225 | 16.941 | 34.813 | 18.609 | 1.00 | 33.07 | A |
| ATOM | 1467 | N   | ARG | A | 226 | 14.696 | 34.566 | 18.422 | 1.00 | 34.83 | A |
| ATOM | 1468 | CA  | ARG | A | 226 | 14.662 | 34.209 | 17.003 | 1.00 | 35.30 | A |
| ATOM | 1469 | CB  | ARG | A | 226 | 14.141 | 32.789 | 16.790 | 1.00 | 37.13 | A |
| ATOM | 1470 | CG  | ARG | A | 226 | 15.104 | 31.694 | 17.098 | 1.00 | 38.28 | A |
| ATOM | 1471 | CD  | ARG | A | 226 | 14.379 | 30.373 | 17.135 | 1.00 | 41.29 | A |
| ATOM | 1472 | NE  | ARG | A | 226 | 15.290 | 29.266 | 17.398 | 1.00 | 44.66 | A |
| ATOM | 1473 | CZ  | ARG | A | 226 | 16.141 | 28.770 | 16.504 | 1.00 | 46.04 | A |
| ATOM | 1474 | NH1 | ARG | A | 226 | 16.195 | 29.285 | 15.278 | 1.00 | 46.80 | A |
| ATOM | 1475 | NH2 | ARG | A | 226 | 16.937 | 27.759 | 16.832 | 1.00 | 47.48 | A |
| ATOM | 1476 | C   | ARG | A | 226 | 13.671 | 35.148 | 16.361 | 1.00 | 34.73 | A |
| ATOM | 1477 | O   | ARG | A | 226 | 12.736 | 35.597 | 17.009 | 1.00 | 35.22 | A |
| ATOM | 1478 | N   | LYS | A | 227 | 13.877 | 35.435 | 15.084 | 1.00 | 37.76 | A |
| ATOM | 1479 | CA  | LYS | A | 227 | 12.958 | 36.287 | 14.351 | 1.00 | 38.82 | A |
| ATOM | 1480 | CB  | LYS | A | 227 | 13.666 | 36.927 | 13.155 | 1.00 | 41.05 | A |
| ATOM | 1481 | CG  | LYS | A | 227 | 14.889 | 37.737 | 13.548 | 1.00 | 43.23 | A |
| ATOM | 1482 | CD  | LYS | A | 227 | 15.404 | 38.582 | 12.404 | 1.00 | 44.60 | A |
| ATOM | 1483 | CE  | LYS | A | 227 | 16.527 | 39.495 | 12.872 | 1.00 | 46.49 | A |
| ATOM | 1484 | NZ  | LYS | A | 227 | 16.998 | 40.409 | 11.793 | 1.00 | 48.32 | A |
| ATOM | 1485 | C   | LYS | A | 227 | 11.827 | 35.379 | 13.884 | 1.00 | 39.64 | A |
| ATOM | 1486 | O   | LYS | A | 227 | 12.074 | 34.325 | 13.304 | 1.00 | 39.91 | A |
| ATOM | 1487 | N   | PRO | A | 228 | 10.570 | 35.760 | 14.156 | 1.00 | 41.20 | A |
| ATOM | 1488 | CD  | PRO | A | 228 | 10.102 | 36.989 | 14.818 | 1.00 | 40.79 | A |
| ATOM | 1489 | CA  | PRO | A | 228 | 9.437  | 34.930 | 13.737 | 1.00 | 41.14 | A |
| ATOM | 1490 | CB  | PRO | A | 228 | 8.245  | 35.646 | 14.366 | 1.00 | 40.93 | A |
| ATOM | 1491 | CG  | PRO | A | 228 | 8.677  | 37.075 | 14.353 | 1.00 | 40.53 | A |
| ATOM | 1492 | C   | PRO | A | 228 | 9.329  | 34.797 | 12.216 | 1.00 | 43.15 | A |
| ATOM | 1493 | O   | PRO | A | 228 | 9.497  | 35.772 | 11.484 | 1.00 | 40.55 | A |
| ATOM | 1494 | N   | PHE | A | 229 | 9.069  | 33.570 | 11.761 | 1.00 | 44.50 | A |
| ATOM | 1495 | CA  | PHE | A | 229 | 8.932  | 33.239 | 10.338 | 1.00 | 47.59 | A |
| ATOM | 1496 | CB  | PHE | A | 229 | 7.624  | 33.799 | 9.794  | 1.00 | 48.22 | A |
| ATOM | 1497 | CG  | PHE | A | 229 | 6.430  | 33.430 | 10.619 | 1.00 | 49.19 | A |
| ATOM | 1498 | CD1 | PHE | A | 229 | 5.779  | 34.386 | 11.396 | 1.00 | 48.58 | A |
| ATOM | 1499 | CD2 | PHE | A | 229 | 5.962  | 32.119 | 10.635 | 1.00 | 48.96 | A |
| ATOM | 1500 | CE1 | PHE | A | 229 | 4.679  | 34.043 | 12.175 | 1.00 | 48.98 | A |
| ATOM | 1501 | CE2 | PHE | A | 229 | 4.862  | 31.765 | 11.410 | 1.00 | 49.06 | A |
| ATOM | 1502 | CZ  | PHE | A | 229 | 4.217  | 32.729 | 12.182 | 1.00 | 49.92 | A |
| ATOM | 1503 | C   | PHE | A | 229 | 10.091 | 33.723 | 9.475  | 1.00 | 50.77 | A |
| ATOM | 1504 | O   | PHE | A | 229 | 9.901  | 34.138 | 8.331  | 1.00 | 51.32 | A |
| ATOM | 1505 | N   | ASP | A | 230 | 11.293 | 33.661 | 10.031 | 1.00 | 54.59 | A |
| ATOM | 1506 | CA  | ASP | A | 230 | 12.490 | 34.082 | 9.322  | 1.00 | 57.29 | A |
| ATOM | 1507 | CB  | ASP | A | 230 | 13.648 | 34.199 | 10.319 | 1.00 | 58.31 | A |
| ATOM | 1508 | CG  | ASP | A | 230 | 14.866 | 34.893 | 9.735  | 1.00 | 59.08 | A |
| ATOM | 1509 | OD1 | ASP | A | 230 | 14.710 | 35.987 | 9.141  | 1.00 | 59.29 | A |

| ATOM | 1510 | OD2 | ASP | A | 230 | 15.983 | 34.346 | 9.885 | 1.00 | 59.83 | A |
|------|------|-----|-----|---|-----|--------|--------|-------|------|-------|---|
| ATOM | 1511 | C | ASP | A | 230 | 12.796 | 33.024 | 8.266 | 1.00 | 59.65 | A |
| ATOM | 1512 | O | ASP | A | 230 | 13.454 | 33.294 | 7.256 | 1.00 | 59.61 | A |
| ATOM | 1513 | N | GLU | A | 231 | 12.282 | 31.821 | 8.515 | 1.00 | 63.90 | A |
| ATOM | 1514 | CA | GLU | A | 231 | 12.465 | 30.664 | 7.639 | 1.00 | 68.01 | A |
| ATOM | 1515 | CB | GLU | A | 231 | 12.411 | 29.383 | 8.484 | 1.00 | 69.94 | A |
| ATOM | 1516 | CG | GLU | A | 231 | 12.872 | 28.119 | 7.768 | 1.00 | 73.29 | A |
| ATOM | 1517 | CD | GLU | A | 231 | 13.067 | 26.935 | 8.714 | 1.00 | 75.51 | A |
| ATOM | 1518 | OE1 | GLU | A | 231 | 12.812 | 27.087 | 9.937 | 1.00 | 76.14 | A |
| ATOM | 1519 | OE2 | GLU | A | 231 | 13.478 | 25.852 | 8.224 | 1.00 | 76.50 | A |
| ATOM | 1520 | C | GLU | A | 231 | 11.439 | 30.590 | 6.493 | 1.00 | 68.57 | A |
| ATOM | 1521 | O | GLU | A | 231 | 11.279 | 29.550 | 5.852 | 1.00 | 69.01 | A |
| ATOM | 1522 | N | ILE | A | 232 | 10.738 | 31.694 | 6.253 | 1.00 | 69.62 | A |
| ATOM | 1523 | CA | ILE | A | 232 | 9.754 | 31.761 | 5.180 | 1.00 | 69.62 | A |
| ATOM | 1524 | CB | ILE | A | 232 | 8.372 | 32.256 | 5.679 | 1.00 | 71.29 | A |
| ATOM | 1525 | CG2 | ILE | A | 232 | 7.483 | 32.593 | 4.494 | 1.00 | 71.31 | A |
| ATOM | 1526 | CG1 | ILE | A | 232 | 7.703 | 31.186 | 6.537 | 1.00 | 71.49 | A |
| ATOM | 1527 | CD1 | ILE | A | 232 | 6.354 | 31.609 | 7.072 | 1.00 | 72.68 | A |
| ATOM | 1528 | C | ILE | A | 232 | 10.303 | 32.765 | 4.181 | 1.00 | 69.95 | A |
| ATOM | 1529 | O | ILE | A | 232 | 10.518 | 32.446 | 3.013 | 1.00 | 70.19 | A |
| ATOM | 1530 | N | GLY | A | 233 | 10.540 | 33.982 | 4.657 | 1.00 | 69.30 | A |
| ATOM | 1531 | CA | GLY | A | 233 | 11.078 | 35.019 | 3.795 | 1.00 | 69.17 | A |
| ATOM | 1532 | C | GLY | A | 233 | 10.352 | 35.207 | 2.472 | 1.00 | 67.86 | A |
| ATOM | 1533 | O | GLY | A | 233 | 9.225 | 34.741 | 2.286 | 1.00 | 68.19 | A |
| ATOM | 1534 | N | GLY | A | 234 | 11.013 | 35.884 | 1.539 | 1.00 | 66.58 | A |
| ATOM | 1535 | CA | GLY | A | 234 | 10.403 | 36.144 | 0.250 | 1.00 | 65.04 | A |
| ATOM | 1536 | C | GLY | A | 234 | 9.488 | 37.332 | 0.448 | 1.00 | 62.81 | A |
| ATOM | 1537 | O | GLY | A | 234 | 9.677 | 38.088 | 1.402 | 1.00 | 64.15 | A |
| ATOM | 1538 | N | PRO | A | 235 | 8.498 | 37.545 | -0.428 | 1.00 | 60.84 | A |
| ATOM | 1539 | CD | PRO | A | 235 | 8.281 | 36.886 | -1.725 | 1.00 | 59.87 | A |
| ATOM | 1540 | CA | PRO | A | 235 | 7.591 | 38.688 | -0.254 | 1.00 | 58.07 | A |
| ATOM | 1541 | CB | PRO | A | 235 | 6.804 | 38.707 | -1.561 | 1.00 | 59.01 | A |
| ATOM | 1542 | CG | PRO | A | 235 | 7.715 | 38.006 | -2.539 | 1.00 | 59.42 | A |
| ATOM | 1543 | C | PRO | A | 235 | 6.677 | 38.474 | 0.968 | 1.00 | 55.95 | A |
| ATOM | 1544 | O | PRO | A | 235 | 6.410 | 37.334 | 1.369 | 1.00 | 54.68 | A |
| ATOM | 1545 | N | ALA | A | 236 | 6.191 | 39.571 | 1.544 | 1.00 | 53.17 | A |
| ATOM | 1546 | CA | ALA | A | 236 | 5.324 | 39.512 | 2.719 | 1.00 | 50.86 | A |
| ATOM | 1547 | CB | ALA | A | 236 | 4.853 | 40.922 | 3.085 | 1.00 | 50.68 | A |
| ATOM | 1548 | C | ALA | A | 236 | 4.115 | 38.588 | 2.560 | 1.00 | 48.74 | A |
| ATOM | 1549 | O | ALA | A | 236 | 3.687 | 37.945 | 3.524 | 1.00 | 47.16 | A |
| ATOM | 1550 | N | PHE | A | 237 | 3.567 | 38.524 | 1.350 | 1.00 | 46.71 | A |
| ATOM | 1551 | CA | PHE | A | 237 | 2.399 | 37.694 | 1.093 | 1.00 | 44.32 | A |
| ATOM | 1552 | CB | PHE | A | 237 | 1.898 | 37.905 | -0.350 | 1.00 | 44.33 | A |
| ATOM | 1553 | CG | PHE | A | 237 | 2.773 | 37.288 | -1.417 | 1.00 | 44.31 | A |
| ATOM | 1554 | CD1 | PHE | A | 237 | 2.812 | 35.910 | -1.604 | 1.00 | 44.91 | A |
| ATOM | 1555 | CD2 | PHE | A | 237 | 3.554 | 38.090 | -2.240 | 1.00 | 44.78 | A |
| ATOM | 1556 | CE1 | PHE | A | 237 | 3.611 | 35.343 | -2.591 | 1.00 | 44.15 | A |
| ATOM | 1557 | CE2 | PHE | A | 237 | 4.354 | 37.527 | -3.227 | 1.00 | 44.97 | A |
| ATOM | 1558 | CZ | PHE | A | 237 | 4.383 | 36.158 | -3.398 | 1.00 | 44.41 | A |
| ATOM | 1559 | C | PHE | A | 237 | 2.595 | 36.197 | 1.385 | 1.00 | 43.39 | A |
| ATOM | 1560 | O | PHE | A | 237 | 1.621 | 35.460 | 1.548 | 1.00 | 42.53 | A |
| ATOM | 1561 | N | ARG | A | 238 | 3.842 | 35.737 | 1.454 | 1.00 | 43.57 | A |
| ATOM | 1562 | CA | ARG | A | 238 | 4.090 | 34.324 | 1.743 | 1.00 | 45.00 | A |
| ATOM | 1563 | CB | ARG | A | 238 | 5.499 | 33.922 | 1.306 | 1.00 | 46.63 | A |

| | | | | | | | | | | | |
|---|---|---|---|---|---|---|---|---|---|---|---|
| ATOM | 1564 | CG | ARG | A | 238 | 5.780 | 34.146 | -0.176 | 1.00 | 49.54 | A |
| ATOM | 1565 | CD | ARG | A | 238 | 7.112 | 33.531 | -0.609 | 1.00 | 52.63 | A |
| ATOM | 1566 | NE | ARG | A | 238 | 7.083 | 32.067 | -0.593 | 1.00 | 55.68 | A |
| ATOM | 1567 | CZ | ARG | A | 238 | 7.792 | 31.311 | 0.240 | 1.00 | 57.09 | A |
| ATOM | 1568 | NH1 | ARG | A | 238 | 8.593 | 31.878 | 1.131 | 1.00 | 58.11 | A |
| ATOM | 1569 | NH2 | ARG | A | 238 | 7.699 | 29.990 | 0.187 | 1.00 | 58.48 | A |
| ATOM | 1570 | C | ARG | A | 238 | 3.904 | 34.057 | 3.238 | 1.00 | 43.28 | A |
| ATOM | 1571 | O | ARG | A | 238 | 3.558 | 32.946 | 3.630 | 1.00 | 42.93 | A |
| ATOM | 1572 | N | ILE | A | 239 | 4.141 | 35.078 | 4.065 | 1.00 | 44.79 | A |
| ATOM | 1573 | CA | ILE | A | 239 | 3.966 | 34.963 | 5.511 | 1.00 | 43.28 | A |
| ATOM | 1574 | CB | ILE | A | 239 | 4.685 | 36.102 | 6.256 | 1.00 | 43.72 | A |
| ATOM | 1575 | CG2 | ILE | A | 239 | 4.305 | 36.094 | 7.721 | 1.00 | 44.43 | A |
| ATOM | 1576 | CG1 | ILE | A | 239 | 6.195 | 35.954 | 6.090 | 1.00 | 44.29 | A |
| ATOM | 1577 | CD1 | ILE | A | 239 | 6.976 | 37.000 | 6.818 | 1.00 | 44.76 | A |
| ATOM | 1578 | C | ILE | A | 239 | 2.478 | 35.050 | 5.802 | 1.00 | 42.71 | A |
| ATOM | 1579 | O | ILE | A | 239 | 1.944 | 34.262 | 6.572 | 1.00 | 43.23 | A |
| ATOM | 1580 | N | MET | A | 240 | 1.816 | 36.011 | 5.166 | 1.00 | 42.58 | A |
| ATOM | 1581 | CA | MET | A | 240 | 0.384 | 36.206 | 5.339 | 1.00 | 43.77 | A |
| ATOM | 1582 | CB | MET | A | 240 | -0.115 | 37.331 | 4.442 | 1.00 | 46.47 | A |
| ATOM | 1583 | CG | MET | A | 240 | 0.395 | 38.687 | 4.833 | 1.00 | 49.51 | A |
| ATOM | 1584 | SD | MET | A | 240 | -0.210 | 39.946 | 3.739 | 1.00 | 68.45 | A |
| ATOM | 1585 | CE | MET | A | 240 | 1.143 | 41.039 | 3.775 | 1.00 | 54.47 | A |
| ATOM | 1586 | C | MET | A | 240 | -0.376 | 34.942 | 5.008 | 1.00 | 42.92 | A |
| ATOM | 1587 | O | MET | A | 240 | -1.348 | 34.600 | 5.673 | 1.00 | 43.82 | A |
| ATOM | 1588 | N | TRP | A | 241 | 0.066 | 34.249 | 3.971 | 1.00 | 42.46 | A |
| ATOM | 1589 | CA | TRP | A | 241 | -0.584 | 33.020 | 3.559 | 1.00 | 41.51 | A |
| ATOM | 1590 | CB | TRP | A | 241 | -0.024 | 32.549 | 2.216 | 1.00 | 43.63 | A |
| ATOM | 1591 | CG | TRP | A | 241 | -0.409 | 31.151 | 1.865 | 1.00 | 44.75 | A |
| ATOM | 1592 | CD2 | TRP | A | 241 | -1.608 | 30.736 | 1.213 | 1.00 | 46.14 | A |
| ATOM | 1593 | CE2 | TRP | A | 241 | -1.573 | 29.326 | 1.131 | 1.00 | 45.79 | A |
| ATOM | 1594 | CE3 | TRP | A | 241 | -2.713 | 31.417 | 0.691 | 1.00 | 46.29 | A |
| ATOM | 1595 | CD1 | TRP | A | 241 | 0.291 | 30.007 | 2.146 | 1.00 | 45.71 | A |
| ATOM | 1596 | NE1 | TRP | A | 241 | -0.404 | 28.908 | 1.709 | 1.00 | 45.77 | A |
| ATOM | 1597 | CZ2 | TRP | A | 241 | -2.607 | 28.584 | 0.545 | 1.00 | 47.07 | A |
| ATOM | 1598 | CZ3 | TRP | A | 241 | -3.741 | 30.681 | 0.110 | 1.00 | 46.91 | A |
| ATOM | 1599 | CH2 | TRP | A | 241 | -3.680 | 29.278 | 0.042 | 1.00 | 47.58 | A |
| ATOM | 1600 | C | TRP | A | 241 | -0.394 | 31.941 | 4.604 | 1.00 | 39.80 | A |
| ATOM | 1601 | O | TRP | A | 241 | -1.339 | 31.258 | 4.976 | 1.00 | 40.35 | A |
| ATOM | 1602 | N | ALA | A | 242 | 0.839 | 31.784 | 5.069 | 1.00 | 39.23 | A |
| ATOM | 1603 | CA | ALA | A | 242 | 1.149 | 30.781 | 6.080 | 1.00 | 39.55 | A |
| ATOM | 1604 | CB | ALA | A | 242 | 2.642 | 30.799 | 6.386 | 1.00 | 38.30 | A |
| ATOM | 1605 | C | ALA | A | 242 | 0.333 | 31.010 | 7.366 | 1.00 | 38.75 | A |
| ATOM | 1606 | O | ALA | A | 242 | -0.343 | 30.099 | 7.842 | 1.00 | 38.04 | A |
| ATOM | 1607 | N | VAL | A | 243 | 0.394 | 32.224 | 7.911 | 1.00 | 40.24 | A |
| ATOM | 1608 | CA | VAL | A | 243 | -0.336 | 32.589 | 9.123 | 1.00 | 41.11 | A |
| ATOM | 1609 | CB | VAL | A | 243 | -0.020 | 34.030 | 9.538 | 1.00 | 40.41 | A |
| ATOM | 1610 | CG1 | VAL | A | 243 | -0.868 | 34.410 | 10.741 | 1.00 | 40.44 | A |
| ATOM | 1611 | CG2 | VAL | A | 243 | 1.456 | 34.176 | 9.842 | 1.00 | 39.55 | A |
| ATOM | 1612 | C | VAL | A | 243 | -1.852 | 32.472 | 8.958 | 1.00 | 41.42 | A |
| ATOM | 1613 | O | VAL | A | 243 | -2.559 | 32.070 | 9.888 | 1.00 | 41.94 | A |
| ATOM | 1614 | N | HIS | A | 244 | -2.340 | 32.832 | 7.774 | 1.00 | 43.57 | A |
| ATOM | 1615 | CA | HIS | A | 244 | -3.764 | 32.766 | 7.457 | 1.00 | 44.61 | A |
| ATOM | 1616 | CB | HIS | A | 244 | -4.022 | 33.434 | 6.105 | 1.00 | 48.09 | A |
| ATOM | 1617 | CG | HIS | A | 244 | -5.466 | 33.463 | 5.710 | 1.00 | 50.30 | A |

| ATOM | 1618 | CD2 | HIS | A | 244 | -6.502 | 34.205 | 6.171 | 1.00 | 51.87 | A |
|------|------|-----|-----|---|-----|--------|--------|-------|------|-------|---|
| ATOM | 1619 | ND1 | HIS | A | 244 | -5.984 | 32.647 | 4.724 | 1.00 | 52.15 | A |
| ATOM | 1620 | CE1 | HIS | A | 244 | -7.277 | 32.887 | 4.595 | 1.00 | 52.33 | A |
| ATOM | 1621 | NE2 | HIS | A | 244 | -7.617 | 33.828 | 5.460 | 1.00 | 52.39 | A |
| ATOM | 1622 | C   | HIS | A | 244 | -4.294 | 31.321 | 7.447 | 1.00 | 44.51 | A |
| ATOM | 1623 | O   | HIS | A | 244 | -5.477 | 31.091 | 7.724 | 1.00 | 44.05 | A |
| ATOM | 1624 | N   | ASN | A | 245 | -3.426 | 30.356 | 7.131 | 1.00 | 44.44 | A |
| ATOM | 1625 | CA  | ASN | A | 245 | -3.824 | 28.953 | 7.119 | 1.00 | 44.73 | A |
| ATOM | 1626 | CB  | ASN | A | 245 | -3.124 | 28.186 | 5.991 | 1.00 | 47.62 | A |
| ATOM | 1627 | CG  | ASN | A | 245 | -3.506 | 28.689 | 4.610 | 1.00 | 50.41 | A |
| ATOM | 1628 | OD1 | ASN | A | 245 | -4.685 | 28.906 | 4.292 | 1.00 | 51.86 | A |
| ATOM | 1629 | ND2 | ASN | A | 245 | -2.502 | 28.869 | 3.771 | 1.00 | 52.29 | A |
| ATOM | 1630 | C   | ASN | A | 245 | -3.538 | 28.263 | 8.455 | 1.00 | 42.80 | A |
| ATOM | 1631 | O   | ASN | A | 245 | -3.562 | 27.032 | 8.543 | 1.00 | 42.02 | A |
| ATOM | 1632 | N   | GLY | A | 246 | -3.250 | 29.051 | 9.490 | 1.00 | 42.05 | A |
| ATOM | 1633 | CA  | GLY | A | 246 | -3.003 | 28.479 | 10.804 | 1.00 | 40.30 | A |
| ATOM | 1634 | C   | GLY | A | 246 | -1.574 | 28.242 | 11.255 | 1.00 | 40.91 | A |
| ATOM | 1635 | O   | GLY | A | 246 | -1.359 | 27.637 | 12.301 | 1.00 | 40.35 | A |
| ATOM | 1636 | N   | THR | A | 247 | -0.597 | 28.712 | 10.488 | 1.00 | 40.08 | A |
| ATOM | 1637 | CA  | THR | A | 247 | 0.801 | 28.527 | 10.858 | 1.00 | 38.89 | A |
| ATOM | 1638 | CB  | THR | A | 247 | 1.745 | 28.740 | 9.662 | 1.00 | 39.80 | A |
| ATOM | 1639 | OG1 | THR | A | 247 | 1.437 | 27.796 | 8.631 | 1.00 | 40.59 | A |
| ATOM | 1640 | CG2 | THR | A | 247 | 3.195 | 28.566 | 10.098 | 1.00 | 40.10 | A |
| ATOM | 1641 | C   | THR | A | 247 | 1.211 | 29.518 | 11.937 | 1.00 | 38.89 | A |
| ATOM | 1642 | O   | THR | A | 247 | 0.930 | 30.716 | 11.831 | 1.00 | 38.31 | A |
| ATOM | 1643 | N   | ARG | A | 248 | 1.883 | 29.004 | 12.965 | 1.00 | 37.70 | A |
| ATOM | 1644 | CA  | ARG | A | 248 | 2.369 | 29.807 | 14.087 | 1.00 | 37.98 | A |
| ATOM | 1645 | CB  | ARG | A | 248 | 1.540 | 29.526 | 15.340 | 1.00 | 38.18 | A |
| ATOM | 1646 | CG  | ARG | A | 248 | 0.491 | 30.576 | 15.654 | 1.00 | 38.61 | A |
| ATOM | 1647 | CD  | ARG | A | 248 | -0.351 | 30.850 | 14.453 | 1.00 | 39.09 | A |
| ATOM | 1648 | NE  | ARG | A | 248 | -1.601 | 31.513 | 14.774 | 1.00 | 38.31 | A |
| ATOM | 1649 | CZ  | ARG | A | 248 | -2.507 | 31.813 | 13.856 | 1.00 | 38.85 | A |
| ATOM | 1650 | NH1 | ARG | A | 248 | -3.636 | 32.413 | 14.197 | 1.00 | 38.71 | A |
| ATOM | 1651 | NH2 | ARG | A | 248 | -2.267 | 31.516 | 12.587 | 1.00 | 38.89 | A |
| ATOM | 1652 | C   | ARG | A | 248 | 3.827 | 29.441 | 14.347 | 1.00 | 37.59 | A |
| ATOM | 1653 | O   | ARG | A | 248 | 4.306 | 28.429 | 13.834 | 1.00 | 36.37 | A |
| ATOM | 1654 | N   | PRO | A | 249 | 4.554 | 30.265 | 15.131 | 1.00 | 36.08 | A |
| ATOM | 1655 | CD  | PRO | A | 249 | 4.150 | 31.566 | 15.701 | 1.00 | 36.43 | A |
| ATOM | 1656 | CA  | PRO | A | 249 | 5.964 | 29.982 | 15.432 | 1.00 | 35.31 | A |
| ATOM | 1657 | CB  | PRO | A | 249 | 6.360 | 31.142 | 16.350 | 1.00 | 37.59 | A |
| ATOM | 1658 | CG  | PRO | A | 249 | 5.481 | 32.270 | 15.882 | 1.00 | 36.95 | A |
| ATOM | 1659 | C   | PRO | A | 249 | 6.079 | 28.613 | 16.122 | 1.00 | 36.73 | A |
| ATOM | 1660 | O   | PRO | A | 249 | 5.196 | 28.234 | 16.898 | 1.00 | 36.56 | A |
| ATOM | 1661 | N   | PRO | A | 250 | 7.163 | 27.858 | 15.855 | 1.00 | 37.37 | A |
| ATOM | 1662 | CD  | PRO | A | 250 | 8.363 | 28.250 | 15.093 | 1.00 | 38.33 | A |
| ATOM | 1663 | CA  | PRO | A | 250 | 7.353 | 26.534 | 16.460 | 1.00 | 37.26 | A |
| ATOM | 1664 | CB  | PRO | A | 250 | 8.703 | 26.098 | 15.907 | 1.00 | 37.78 | A |
| ATOM | 1665 | CG  | PRO | A | 250 | 9.422 | 27.393 | 15.729 | 1.00 | 37.64 | A |
| ATOM | 1666 | C   | PRO | A | 250 | 7.299 | 26.508 | 17.975 | 1.00 | 37.52 | A |
| ATOM | 1667 | O   | PRO | A | 250 | 7.649 | 27.484 | 18.632 | 1.00 | 37.44 | A |
| ATOM | 1668 | N   | LEU | A | 251 | 6.839 | 25.388 | 18.524 | 1.00 | 39.16 | A |
| ATOM | 1669 | CA  | LEU | A | 251 | 6.745 | 25.237 | 19.972 | 1.00 | 40.26 | A |
| ATOM | 1670 | CB  | LEU | A | 251 | 6.147 | 23.875 | 20.330 | 1.00 | 42.90 | A |
| ATOM | 1671 | CG  | LEU | A | 251 | 4.721 | 23.608 | 19.847 | 1.00 | 46.52 | A |

| ATOM | 1672 | CD1 | LEU | A | 251 | 4.390 | 22.151 | 20.125 | 1.00 | 47.61 | A |
|------|------|-----|-----|---|-----|-------|--------|--------|------|-------|---|
| ATOM | 1673 | CD2 | LEU | A | 251 | 3.722 | 24.542 | 20.539 | 1.00 | 46.97 | A |
| ATOM | 1674 | C   | LEU | A | 251 | 8.133 | 25.355 | 20.580 | 1.00 | 39.07 | A |
| ATOM | 1675 | O   | LEU | A | 251 | 9.130 | 25.081 | 19.920 | 1.00 | 38.25 | A |
| ATOM | 1676 | N   | ILE | A | 252 | 8.190 | 25.767 | 21.840 | 1.00 | 37.41 | A |
| ATOM | 1677 | CA  | ILE | A | 252 | 9.456 | 25.933 | 22.541 | 1.00 | 35.60 | A |
| ATOM | 1678 | CB  | ILE | A | 252 | 9.447 | 27.244 | 23.364 | 1.00 | 35.28 | A |
| ATOM | 1679 | CG2 | ILE | A | 252 | 10.755 | 27.408 | 24.096 | 1.00 | 35.99 | A |
| ATOM | 1680 | CG1 | ILE | A | 252 | 9.223 | 28.430 | 22.432 | 1.00 | 33.51 | A |
| ATOM | 1681 | CD1 | ILE | A | 252 | 8.839 | 29.710 | 23.112 | 1.00 | 32.97 | A |
| ATOM | 1682 | C   | ILE | A | 252 | 9.700 | 24.729 | 23.452 | 1.00 | 37.74 | A |
| ATOM | 1683 | O   | ILE | A | 252 | 8.810 | 24.289 | 24.180 | 1.00 | 37.81 | A |
| ATOM | 1684 | N   | LYS | A | 253 | 10.906 | 24.183 | 23.400 | 1.00 | 39.12 | A |
| ATOM | 1685 | CA  | LYS | A | 253 | 11.232 | 23.026 | 24.217 | 1.00 | 41.73 | A |
| ATOM | 1686 | CB  | LYS | A | 253 | 12.539 | 22.406 | 23.738 | 1.00 | 44.06 | A |
| ATOM | 1687 | CG  | LYS | A | 253 | 12.978 | 21.236 | 24.577 | 1.00 | 47.73 | A |
| ATOM | 1688 | CD  | LYS | A | 253 | 14.214 | 20.582 | 23.998 | 1.00 | 50.49 | A |
| ATOM | 1689 | CE  | LYS | A | 253 | 14.702 | 19.449 | 24.893 | 1.00 | 52.90 | A |
| ATOM | 1690 | NZ  | LYS | A | 253 | 15.746 | 18.632 | 24.207 | 1.00 | 53.77 | A |
| ATOM | 1691 | C   | LYS | A | 253 | 11.326 | 23.366 | 25.704 | 1.00 | 41.61 | A |
| ATOM | 1692 | O   | LYS | A | 253 | 12.065 | 24.265 | 26.096 | 1.00 | 40.66 | A |
| ATOM | 1693 | N   | ASN | A | 254 | 10.557 | 22.654 | 26.525 | 1.00 | 42.37 | A |
| ATOM | 1694 | CA  | ASN | A | 254 | 10.561 | 22.848 | 27.983 | 1.00 | 44.84 | A |
| ATOM | 1695 | CB  | ASN | A | 254 | 11.986 | 22.672 | 28.525 | 1.00 | 48.80 | A |
| ATOM | 1696 | CG  | ASN | A | 254 | 12.549 | 21.281 | 28.237 | 1.00 | 52.47 | A |
| ATOM | 1697 | OD1 | ASN | A | 254 | 11.931 | 20.263 | 28.588 | 1.00 | 53.97 | A |
| ATOM | 1698 | ND2 | ASN | A | 254 | 13.731 | 21.228 | 27.602 | 1.00 | 54.04 | A |
| ATOM | 1699 | C   | ASN | A | 254 | 9.980 | 24.159 | 28.518 | 1.00 | 43.09 | A |
| ATOM | 1700 | O   | ASN | A | 254 | 10.299 | 24.563 | 29.636 | 1.00 | 43.05 | A |
| ATOM | 1701 | N   | LEU | A | 255 | 9.136 | 24.823 | 27.734 | 1.00 | 39.82 | A |
| ATOM | 1702 | CA  | LEU | A | 255 | 8.524 | 26.079 | 28.167 | 1.00 | 39.30 | A |
| ATOM | 1703 | CB  | LEU | A | 255 | 7.845 | 26.760 | 26.977 | 1.00 | 38.89 | A |
| ATOM | 1704 | CG  | LEU | A | 255 | 7.114 | 28.089 | 27.172 | 1.00 | 38.18 | A |
| ATOM | 1705 | CD1 | LEU | A | 255 | 8.109 | 29.222 | 27.424 | 1.00 | 37.90 | A |
| ATOM | 1706 | CD2 | LEU | A | 255 | 6.279 | 28.369 | 25.929 | 1.00 | 36.91 | A |
| ATOM | 1707 | C   | LEU | A | 255 | 7.490 | 25.742 | 29.239 | 1.00 | 37.87 | A |
| ATOM | 1708 | O   | LEU | A | 255 | 6.811 | 24.717 | 29.134 | 1.00 | 37.69 | A |
| ATOM | 1709 | N   | PRO | A | 256 | 7.372 | 26.575 | 30.296 | 1.00 | 35.60 | A |
| ATOM | 1710 | CD  | PRO | A | 256 | 8.291 | 27.666 | 30.670 | 1.00 | 36.13 | A |
| ATOM | 1711 | CA  | PRO | A | 256 | 6.401 | 26.327 | 31.371 | 1.00 | 35.64 | A |
| ATOM | 1712 | CB  | PRO | A | 256 | 6.631 | 27.497 | 32.322 | 1.00 | 35.62 | A |
| ATOM | 1713 | CG  | PRO | A | 256 | 8.080 | 27.764 | 32.169 | 1.00 | 35.16 | A |
| ATOM | 1714 | C   | PRO | A | 256 | 4.970 | 26.302 | 30.839 | 1.00 | 36.27 | A |
| ATOM | 1715 | O   | PRO | A | 256 | 4.566 | 27.205 | 30.106 | 1.00 | 35.77 | A |
| ATOM | 1716 | N   | LYS | A | 257 | 4.209 | 25.270 | 31.199 | 1.00 | 38.39 | A |
| ATOM | 1717 | CA  | LYS | A | 257 | 2.816 | 25.132 | 30.764 | 1.00 | 39.41 | A |
| ATOM | 1718 | CB  | LYS | A | 257 | 2.139 | 24.005 | 31.544 | 1.00 | 43.82 | A |
| ATOM | 1719 | CG  | LYS | A | 257 | 2.383 | 22.600 | 31.000 | 1.00 | 49.23 | A |
| ATOM | 1720 | CD  | LYS | A | 257 | 1.673 | 22.382 | 29.662 | 1.00 | 52.42 | A |
| ATOM | 1721 | CE  | LYS | A | 257 | 2.606 | 22.585 | 28.473 | 1.00 | 54.65 | A |
| ATOM | 1722 | NZ  | LYS | A | 257 | 3.560 | 21.453 | 28.321 | 1.00 | 56.72 | A |
| ATOM | 1723 | C   | LYS | A | 257 | 1.997 | 26.414 | 30.911 | 1.00 | 39.31 | A |
| ATOM | 1724 | O   | LYS | A | 257 | 1.272 | 26.791 | 29.989 | 1.00 | 36.91 | A |
| ATOM | 1725 | N   | PRO | A | 258 | 2.077 | 27.092 | 32.081 | 1.00 | 38.94 | A |

| ATOM | 1726 | CD | PRO | A | 258 | 2.710 | 26.728 | 33.363 | 1.00 | 39.54 | A |
|------|------|------|-----|---|-----|-------|--------|--------|------|-------|---|
| ATOM | 1727 | CA | PRO | A | 258 | 1.299 | 28.326 | 32.231 | 1.00 | 39.03 | A |
| ATOM | 1728 | CB | PRO | A | 258 | 1.547 | 28.726 | 33.697 | 1.00 | 38.66 | A |
| ATOM | 1729 | CG | PRO | A | 258 | 2.828 | 28.062 | 34.041 | 1.00 | 38.45 | A |
| ATOM | 1730 | C | PRO | A | 258 | 1.659 | 29.430 | 31.235 | 1.00 | 37.15 | A |
| ATOM | 1731 | O | PRO | A | 258 | 0.811 | 30.246 | 30.899 | 1.00 | 37.21 | A |
| ATOM | 1732 | N | ILE | A | 259 | 2.903 | 29.450 | 30.758 | 1.00 | 35.25 | A |
| ATOM | 1733 | CA | ILE | A | 259 | 3.339 | 30.463 | 29.793 | 1.00 | 35.45 | A |
| ATOM | 1734 | CB | ILE | A | 259 | 4.869 | 30.734 | 29.891 | 1.00 | 34.80 | A |
| ATOM | 1735 | CG2 | ILE | A | 259 | 5.318 | 31.678 | 28.797 | 1.00 | 35.30 | A |
| ATOM | 1736 | CG1 | ILE | A | 259 | 5.202 | 31.365, | 31.246 | 1.00 | 34.49 | A |
| ATOM | 1737 | CD1 | ILE | A | 259 | 6.669 | 31.415 | 31.545 | 1.00 | 34.94 | A |
| ATOM | 1738 | C | ILE | A | 259 | 2.979 | 30.014 | 28.383 | 1.00 | 33.13 | A |
| ATOM | 1739 | O | ILE | A | 259 | 2.587 | 30.822 | 27.542 | 1.00 | 31.26 | A |
| ATOM | 1740 | N | GLU | A | 260 | 3.093 | 28.719 | 28.125 | 1.00 | 33.53 | A |
| ATOM | 1741 | CA | GLU | A | 260 | 2.751 | 28.205 | 26.811 | 1.00 | 34.64 | A |
| ATOM | 1742 | CB | GLU | A | 260 | 3.080 | 26.718 | 26.710 | 1.00 | 37.45 | A |
| ATOM | 1743 | CG | GLU | A | 260 | 2.911 | 26.170 | 25.310 | 1.00 | 44.36 | A |
| ATOM | 1744 | CD | GLU | A | 260 | 3.449 | 24.751 | 25.152 | 1.00 | 47.09 | A |
| ATOM | 1745 | OE1 | GLU | A | 260 | 2.907 | 23.838 | 25.812 | 1.00 | 48.82 | A |
| ATOM | 1746 | OE2 | GLU | A | 260 | 4.416 | 24.547 | 24.370 | 1.00 | 48.98 | A |
| ATOM | 1747 | C | GLU | A | 260 | 1.277 | 28.422 | 26.505 | 1.00 | 33.52 | A |
| ATOM | 1748 | O | GLU | A | 260 | 0.931 | 28.881 | 25.429 | 1.00 | 32.38 | A |
| ATOM | 1749 | N | SER | A | 261 | 0.408 | 28.109 | 27.460 | 1.00 | 35.43 | A |
| ATOM | 1750 | CA | SER | A | 261 | -1.027 | 28.274 | 27.268 | 1.00 | 36.16 | A |
| ATOM | 1751 | CB | SER | A | 261 | -1.794 | 27.705 | 28.459 | 1.00 | 38.00 | A |
| ATOM | 1752 | OG | SER | A | 261 | -1.475 | 28.402 | 29.640 | 1.00 | 42.13 | A |
| ATOM | 1753 | C | SER | A | 261 | -1.417 | 29.731 | 27.050 | 1.00 | 32.89 | A |
| ATOM | 1754 | O | SER | A | 261 | -2.320 | 30.020 | 26.270 | 1.00 | 33.37 | A |
| ATOM | 1755 | N | LEU | A | 262 | -0.740 | 30.650 | 27.727 | 1.00 | 33.56 | A |
| ATOM | 1756 | CA | LEU | A | 262 | -1.034 | 32.063 | 27.560 | 1.00 | 32.96 | A |
| ATOM | 1757 | CB | LEU | A | 262 | -0.288 | 32.880 | 28.611 | 1.00 | 32.57 | A |
| ATOM | 1758 | CG | LEU | A | 262 | -0.469 | 34.397 | 28.510 | 1.00 | 33.06 | A |
| ATOM | 1759 | CD1 | LEU | A | 262 | -1.945 | 34.751 | 28.639 | 1.00 | 34.40 | A |
| ATOM | 1760 | CD2 | LEU | A | 262 | 0.346 | 35.081 | 29.590 | 1.00 | 34.39 | A |
| ATOM | 1761 | C | LEU | A | 262 | -0.607 | 32.526 | 26.175 | 1.00 | 30.71 | A |
| ATOM | 1762 | O | LEU | A | 262 | -1.277 | 33.328 | 25.531 | 1.00 | 30.82 | A |
| ATOM | 1763 | N | MET | A | 263 | 0.531 | 32.016 | 25.730 | 1.00 | 30.95 | A |
| ATOM | 1764 | CA | MET | A | 263 | 1.089 | 32.373 | 24.436 | 1.00 | 32.77 | A |
| ATOM | 1765 | CB | MET | A | 263 | 2.493 | 31.776 | 24.309 | 1.00 | 32.92 | A |
| ATOM | 1766 | CG | MET | A | 263 | 3.252 | 32.117 | 23.035 | 1.00 | 32.34 | A |
| ATOM | 1767 | SD | MET | A | 263 | 4.945 | 31.505 | 23.148 | 1.00 | 34.92 | A |
| ATOM | 1768 | CE | MET | A | 263 | 4.652 | 29.806 | 22.803 | 1.00 | 35.02 | A |
| ATOM | 1769 | C | MET | A | 263 | 0.222 | 31.899 | 23.277 | 1.00 | 32.36 | A |
| ATOM | 1770 | O | MET | A | 263 | -0.052 | 32.659 | 22.338 | 1.00 | 32.64 | A |
| ATOM | 1771 | N | THR | A | 264 | -0.216 | 30.646 | 23.343 | 1.00 | 34.63 | A |
| ATOM | 1772 | CA | THR | A | 264 | -1.033 | 30.079 | 22.277 | 1.00 | 35.11 | A |
| ATOM | 1773 | CB | THR | A | 264 | -1.069 | 28.550 | 22.374 | 1.00 | 33.48 | A |
| ATOM | 1774 | OG1 | THR | A | 264 | -1.695 | 28.173 | 23.601 | 1.00 | 34.31 | A |
| ATOM | 1775 | CG2 | THR | A | 264 | 0.350 | 27.976 | 22.322 | 1.00 | 32.96 | A |
| ATOM | 1776 | C | THR | A | 264 | -2.462 | 30.619 | 22.252 | 1.00 | 34.38 | A |
| ATOM | 1777 | O | THR | A | 264 | -3.105 | 30.606 | 21.201 | 1.00 | 35.24 | A |
| ATOM | 1778 | N | ARG | A | 265 | -2.965 | 31.082 | 23.398 | 1.00 | 34.32 | A |
| ATOM | 1779 | CA | ARG | A | 265 | -4.312 | 31.652 | 23.443 | 1.00 | 32.21 | A |

| ATOM | 1780 | CB | ARG A 265 | -4.777 | 31.921 | 24.873 | 1.00 | 35.12 | A |
|------|------|-----|-----------|---------|--------|--------|------|-------|---|
| ATOM | 1781 | CG | ARG A 265 | -5.350 | 30.722 | 25.597 | 1.00 | 37.78 | A |
| ATOM | 1782 | CD | ARG A 265 | -5.670 | 31.110 | 27.037 | 1.00 | 39.50 | A |
| ATOM | 1783 | NE | ARG A 265 | -6.691 | 32.158 | 27.128 | 1.00 | 42.05 | A |
| ATOM | 1784 | CZ | ARG A 265 | -6.911 | 32.914 | 28.202 | 1.00 | 40.43 | A |
| ATOM | 1785 | NH1 | ARG A 265 | -6.176 | 32.760 | 29.299 | 1.00 | 42.09 | A |
| ATOM | 1786 | NH2 | ARG A 265 | -7.882 | 33.817 | 28.174 | 1.00 | 41.61 | A |
| ATOM | 1787 | C | ARG A 265 | -4.291 | 32.973 | 22.688 | 1.00 | 32.02 | A |
| ATOM | 1788 | O | ARG A 265 | -5.239 | 33.312 | 21.996 | 1.00 | 30.62 | A |
| ATOM | 1789 | N | CYS A 266 | -3.201 | 33.717 | 22.827 | 1.00 | 32.77 | A |
| ATOM | 1790 | CA | CYS A 266 | -3.057 | 34.989 | 22.133 | 1.00 | 31.67 | A |
| ATOM | 1791 | CB | CYS A 266 | -1.786 | 35.714 | 22.598 | 1.00 | 29.92 | A |
| ATOM | 1792 | SG | CYS A 266 | -1.754 | 36.217 | 24.356 | 1.00 | 18.14 | A |
| ATOM | 1793 | C | CYS A 266 | -2.998 | 34.784 | 20.611 | 1.00 | 33.02 | A |
| ATOM | 1794 | O | CYS A 266 | -3.342 | 35.691 | 19.859 | 1.00 | 35.24 | A |
| ATOM | 1795 | N | TRP A 267 | -2.556 | 33.602 | 20.171 | 1.00 | 36.45 | A |
| ATOM | 1796 | CA | TRP A 267 | -2.436 | 33.269 | 18.746 | 1.00 | 35.40 | A |
| ATOM | 1797 | CB | TRP A 267 | -1.364 | 32.203 | 18.503 | 1.00 | 35.31 | A |
| ATOM | 1798 | CG | TRP A 267 | 0.007 | 32.620 | 18.783 | 1.00 | 37.30 | A |
| ATOM | 1799 | CD2 | TRP A 267 | 1.121 | 31.759 | 19.007 | 1.00 | 35.45 | A |
| ATOM | 1800 | CE2 | TRP A 267 | 2.264 | 32.583 | 19.163 | 1.00 | 35.22 | A |
| ATOM | 1801 | CE3 | TRP A 267 | 1.269 | 30.370 | 19.087 | 1.00 | 34.79 | A |
| ATOM | 1802 | CD1 | TRP A 267 | 0.492 | 33.900 | 18.811 | 1.00 | 36.99 | A |
| ATOM | 1803 | NE1 | TRP A 267 | 1.852 | 33.885 | 19.040 | 1.00 | 37.39 | A |
| ATOM | 1804 | CZ2 | TRP A 267 | 3.540 | 32.061 | 19.397 | 1.00 | 34.17 | A |
| ATOM | 1805 | CZ3 | TRP A 267 | 2.544 | 29.847 | 19.321 | 1.00 | 34.37 | A |
| ATOM | 1806 | CH2 | TRP A 267 | 3.660 | 30.695 | 19.473 | 1.00 | 34.93 | A |
| ATOM | 1807 | C | TRP A 267 | -3.710 | 32.735 | 18.123 | 1.00 | 37.20 | A |
| ATOM | 1808 | O | TRP A 267 | -3.732 | 32.453 | 16.931 | 1.00 | 37.19 | A |
| ATOM | 1809 | N | SER A 268 | -4.755 | 32.563 | 18.925 | 1.00 | 38.23 | A |
| ATOM | 1810 | CA | SER A 268 | -6.033 | 32.058 | 18.428 | 1.00 | 38.14 | A |
| ATOM | 1811 | CB | SER A 268 | -7.127 | 32.246 | 19.487 | 1.00 | 39.06 | A |
| ATOM | 1812 | OG | SER A 268 | -6.830 | 31.549 | 20.669 | 1.00 | 40.01 | A |
| ATOM | 1813 | C | SER A 268 | -6.497 | 32.736 | 17.133 | 1.00 | 39.20 | A |
| ATOM | 1814 | O | SER A 268 | -6.456 | 33.962 | 17.011 | 1.00 | 37.90 | A |
| ATOM | 1815 | N | LYS A 269 | -6.951 | 31.935 | 16.173 | 1.00 | 40.63 | A |
| ATOM | 1816 | CA | LYS A 269 | -7.459 | 32.483 | 14.920 | 1.00 | 41.75 | A |
| ATOM | 1817 | CB | LYS A 269 | -7.931 | 31.356 | 13.999 | 1.00 | 43.45 | A |
| ATOM | 1818 | CG | LYS A 269 | -8.601 | 31.830 | 12.720 | 1.00 | 45.27 | A |
| ATOM | 1819 | CD | LYS A 269 | -9.182 | 30.658 | 11.963 | 1.00 | 47.54 | A |
| ATOM | 1820 | CE | LYS A 269 | -10.097 | 31.109 | 10.841 | 1.00 | 49.15 | A |
| ATOM | 1821 | NZ | LYS A 269 | -9.387 | 31.869 | 9.784 | 1.00 | 50.04 | A |
| ATOM | 1822 | C | LYS A 269 | -8.636 | 33.383 | 15.285 | 1.00 | 41.98 | A |
| ATOM | 1823 | O | LYS A 269 | -8.739 | 34.518 | 14.824 | 1.00 | 39.89 | A |
| ATOM | 1824 | N | ASP A 270 | -9.506 | 32.862 | 16.143 | 1.00 | 41.13 | A |
| ATOM | 1825 | CA | ASP A 270 | -10.686 | 33.577 | 16.592 | 1.00 | 41.48 | A |
| ATOM | 1826 | CB | ASP A 270 | -11.664 | 32.578 | 17.201 | 1.00 | 43.86 | A |
| ATOM | 1827 | CG | ASP A 270 | -13.007 | 33.200 | 17.525 | 1.00 | 46.15 | A |
| ATOM | 1828 | OD1 | ASP A 270 | -13.159 | 34.420 | 17.304 | 1.00 | 46.58 | A |
| ATOM | 1829 | OD2 | ASP A 270 | -13.906 | 32.468 | 17.998 | 1.00 | 47.41 | A |
| ATOM | 1830 | C | ASP A 270 | -10.339 | 34.661 | 17.617 | 1.00 | 40.76 | A |
| ATOM | 1831 | O | ASP A 270 | -9.966 | 34.362 | 18.743 | 1.00 | 38.80 | A |
| ATOM | 1832 | N | PRO A 271 | -10.474 | 35.941 | 17.235 | 1.00 | 40.83 | A |
| ATOM | 1833 | CD | PRO A 271 | -10.923 | 36.406 | 15.913 | 1.00 | 39.51 | A |

70

| ATOM | 1834 | CA | PRO | A | 271 | -10.179 | 37.088 | 18.103 | 1.00 | 40.01 | A |
|------|------|-----|-----|---|-----|---------|--------|--------|------|-------|---|
| ATOM | 1835 | CB | PRO | A | 271 | -10.640 | 38.283 | 17.264 | 1.00 | 40.18 | A |
| ATOM | 1836 | CG | PRO | A | 271 | -10.412 | 37.819 | 15.888 | 1.00 | 39.83 | A |
| ATOM | 1837 | C | PRO | A | 271 | -10.854 | 37.058 | 19.477 | 1.00 | 38.10 | A |
| ATOM | 1838 | O | PRO | A | 271 | -10.249 | 37.420 | 20.487 | 1.00 | 38.08 | A |
| ATOM | 1839 | N | SER | A | 272 | -12.112 | 36.635 | 19.515 | 1.00 | 39.77 | A |
| ATOM | 1840 | CA | SER | A | 272 | -12.850 | 36.572 | 20.770 | 1.00 | 38.99 | A |
| ATOM | 1841 | CB | SER | A | 272 | -14.318 | 36.232 | 20.493 | 1.00 | 39.99 | A |
| ATOM | 1842 | OG | SER | A | 272 | -14.435 | 35.114 | 19.636 | 1.00 | 41.82 | A |
| ATOM | 1843 | C | SER | A | 272 | -12.243 | 35.582 | 21.770 | 1.00 | 41.07 | A |
| ATOM | 1844 | O | SER | A | 272 | -12.423 | 35.737 | 22.973 | 1.00 | 40.56 | A |
| ATOM | 1845 | N | GLN | A | 273 | -11.512 | 34.582 | 21.278 | 1.00 | 41.17 | A |
| ATOM | 1846 | CA | GLN | A | 273 | -10.887 | 33.596 | 22.152 | 1.00 | 41.24 | A |
| ATOM | 1847 | CB | GLN | A | 273 | -10.690 | 32.271 | 21.407 | 1.00 | 44.97 | A |
| ATOM | 1848 | CG | GLN | A | 273 | -11.975 | 31.505 | 21.116 | 1.00 | 50.90 | A |
| ATOM | 1849 | CD | GLN | A | 273 | -11.722 | 30.173 | 20.403 | 1.00 | 53.85 | A |
| ATOM | 1850 | OE1 | GLN | A | 273 | -12.474 | 29.778 | 19.499 | 1.00 | 55.96 | A |
| ATOM | 1851 | NE2 | GLN | A | 273 | -10.668 | 29.472 | 20.814 | 1.00 | 55.41 | A |
| ATOM | 1852 | C | GLN | A | 273 | -9.551 | 34.056 | 22.766 | 1.00 | 39.55 | A |
| ATOM | 1853 | O | GLN | A | 273 | -9.010 | 33.395 | 23.665 | 1.00 | 40.23 | A |
| ATOM | 1854 | N | ARG | A | 274 | -9.021 | 35.180 | 22.293 | 1.00 | 35.75 | A |
| ATOM | 1855 | CA | ARG | A | 274 | -7.774 | 35.710 | 22.845 | 1.00 | 34.06 | A |
| ATOM | 1856 | CB | ARG | A | 274 | -7.125 | 36.696 | 21.863 | 1.00 | 32.58 | A |
| ATOM | 1857 | CG | ARG | A | 274 | -6.723 | 36.091 | 20.551 | 1.00 | 30.91 | A |
| ATOM | 1858 | CD | ARG | A | 274 | -6.362 | 37.156 | 19.540 | 1.00 | 31.95 | A |
| ATOM | 1859 | NE | ARG | A | 274 | -6.345 | 36.599 | 18.188 | 1.00 | 31.74 | A |
| ATOM | 1860 | CZ | ARG | A | 274 | -6.360 | 37.311 | 17.069 | 1.00 | 33.37 | A |
| ATOM | 1861 | NH1 | ARG | A | 274 | -6.387 | 38.630 | 17.123 | 1.00 | 31.14 | A |
| ATOM | 1862 | NH2 | ARG | A | 274 | -6.382 | 36.693 | 15.896 | 1.00 | 31.74 | A |
| ATOM | 1863 | C | ARG | A | 274 | -8.070 | 36.444 | 24.156 | 1.00 | 32.37 | A |
| ATOM | 1864 | O | ARG | A | 274 | -9.139 | 37.050 | 24.295 | 1.00 | 33.50 | A |
| ATOM | 1865 | N | PRO | A | 275 | -7.145 | 36.391 | 25.136 | 1.00 | 31.55 | A |
| ATOM | 1866 | CD | PRO | A | 275 | -5.901 | 35.604 | 25.218 | 1.00 | 32.44 | A |
| ATOM | 1867 | CA | PRO | A | 275 | -7.402 | 37.100 | 26.397 | 1.00 | 30.34 | A |
| ATOM | 1868 | CB | PRO | A | 275 | -6.294 | 36.589 | 27.313 | 1.00 | 31.26 | A |
| ATOM | 1869 | CG | PRO | A | 275 | -5.184 | 36.276 | 26.368 | 1.00 | 30.85 | A |
| ATOM | 1870 | C | PRO | A | 275 | -7.329 | 38.622 | 26.177 | 1.00 | 31.82 | A |
| ATOM | 1871 | O | PRO | A | 275 | -6.789 | 39.085 | 25.169 | 1.00 | 29.56 | A |
| ATOM | 1872 | N | SER | A | 276 | -7.887 | 39.400 | 27.100 | 1.00 | 31.19 | A |
| ATOM | 1873 | CA | SER | A | 276 | -7.839 | 40.859 | 26.968 | 1.00 | 31.00 | A |
| ATOM | 1874 | CB | SER | A | 276 | -8.939 | 41.525 | 27.802 | 1.00 | 32.30 | A |
| ATOM | 1875 | OG | SER | A | 276 | -8.791 | 41.260 | 29.186 | 1.00 | 32.48 | A |
| ATOM | 1876 | C | SER | A | 276 | -6.474 | 41.317 | 27.457 | 1.00 | 31.09 | A |
| ATOM | 1877 | O | SER | A | 276 | -5.832 | 40.617 | 28.237 | 1.00 | 29.50 | A |
| ATOM | 1878 | N | MET | A | 277 | -6.015 | 42.478 | 27.008 | 1.00 | 31.70 | A |
| ATOM | 1879 | CA | MET | A | 277 | -4.712 | 42.943 | 27.447 | 1.00 | 31.60 | A |
| ATOM | 1880 | CB | MET | A | 277 | -4.349 | 44.256 | 26.737 | 1.00 | 32.43 | A |
| ATOM | 1881 | CG | MET | A | 277 | -2.852 | 44.534 | 26.651 | 1.00 | 34.38 | A |
| ATOM | 1882 | SD | MET | A | 277 | -1.834 | 43.221 | 25.874 | 1.00 | 32.35 | A |
| ATOM | 1883 | CE | MET | A | 277 | -1.667 | 43.807 | 24.219 | 1.00 | 37.32 | A |
| ATOM | 1884 | C | MET | A | 277 | -4.699 | 43.093 | 28.984 | 1.00 | 30.06 | A |
| ATOM | 1885 | O | MET | A | 277 | -3.666 | 42.874 | 29.618 | 1.00 | 31.95 | A |
| ATOM | 1886 | N | GLU | A | 278 | -5.840 | 43.435 | 29.586 | 1.00 | 33.26 | A |
| ATOM | 1887 | CA | GLU | A | 278 | -5.925 | 43.565 | 31.047 | 1.00 | 34.98 | A |

| ATOM | 1888 | CB  | GLU | A | 278 | -7.302 | 44.078 | 31.493 | 1.00 | 38.80 | A |
|------|------|-----|-----|---|-----|--------|--------|--------|------|-------|---|
| ATOM | 1889 | CG  | GLU | A | 278 | -7.532 | 45.579 | 31.329 | 1.00 | 45.49 | A |
| ATOM | 1890 | CD  | GLU | A | 278 | -6.566 | 46.437 | 32.157 | 1.00 | 49.36 | A |
| ATOM | 1891 | OE1 | GLU | A | 278 | -5.785 | 45.861 | 32.951 | 1.00 | 52.03 | A |
| ATOM | 1892 | OE2 | GLU | A | 278 | -6.594 | 47.687 | 32.011 | 1.00 | 51.34 | A |
| ATOM | 1893 | C   | GLU | A | 278 | -5.673 | 42.229 | 31.737 | 1.00 | 32.34 | A |
| ATOM | 1894 | O   | GLU | A | 278 | -5.019 | 42.174 | 32.773 | 1.00 | 33.05 | A |
| ATOM | 1895 | N   | GLU | A | 279 | -6.208 | 41.150 | 31.184 | 1.00 | 32.11 | A |
| ATOM | 1896 | CA  | GLU | A | 279 | -5.994 | 39.840 | 31.785 | 1.00 | 31.10 | A |
| ATOM | 1897 | CB  | GLU | A | 279 | -6.879 | 38.772 | 31.141 | 1.00 | 33.96 | A |
| ATOM | 1898 | CG  | GLU | A | 279 | -6.595 | 37.375 | 31.679 | 1.00 | 38.56 | A |
| ATOM | 1899 | CD  | GLU | A | 279 | -7.512 | 36.310 | 31.095 | 1.00 | 41.45 | A |
| ATOM | 1900 | OE1 | GLU | A | 279 | -7.272 | 35.114 | 31.364 | 1.00 | 43.21 | A |
| ATOM | 1901 | OE2 | GLU | A | 279 | -8.475 | 36.666 | 30.371 | 1.00 | 43.12 | A |
| ATOM | 1902 | C   | GLU | A | 279 | -4.535 | 39.436 | 31.639 | 1.00 | 28.51 | A |
| ATOM | 1903 | O   | GLU | A | 279 | -3.974 | 38.825 | 32.543 | 1.00 | 29.61 | A |
| ATOM | 1904 | N   | ILE | A | 280 | -3.915 | 39.771 | 30.506 | 1.00 | 26.59 | A |
| ATOM | 1905 | CA  | ILE | A | 280 | -2.502 | 39.452 | 30.304 | 1.00 | 28.01 | A |
| ATOM | 1906 | CB  | ILE | A | 280 | -2.022 | 39.868 | 28.894 | 1.00 | 27.86 | A |
| ATOM | 1907 | CG2 | ILE | A | 280 | -0.500 | 39.838 | 28.833 | 1.00 | 26.02 | A |
| ATOM | 1908 | CG1 | ILE | A | 280 | -2.641 | 38.937 | 27.848 | 1.00 | 26.55 | A |
| ATOM | 1909 | CD1 | ILE | A | 280 | -2.430 | 39.376 | 26.415 | 1.00 | 28.56 | A |
| ATOM | 1910 | C   | ILE | A | 280 | -1.659 | 40.155 | 31.382 | 1.00 | 25.01 | A |
| ATOM | 1911 | O   | ILE | A | 280 | -0.759 | 39.540 | 31.964 | 1.00 | 25.49 | A |
| ATOM | 1912 | N   | VAL | A | 281 | -1.961 | 41.425 | 31.657 | 1.00 | 28.45 | A |
| ATOM | 1913 | CA  | VAL | A | 281 | -1.242 | 42.164 | 32.683 | 1.00 | 27.67 | A |
| ATOM | 1914 | CB  | VAL | A | 281 | -1.795 | 43.606 | 32.848 | 1.00 | 24.93 | A |
| ATOM | 1915 | CG1 | VAL | A | 281 | -1.102 | 44.303 | 34.004 | 1.00 | 26.73 | A |
| ATOM | 1916 | CG2 | VAL | A | 281 | -1.580 | 44.395 | 31.572 | 1.00 | 28.24 | A |
| ATOM | 1917 | C   | VAL | A | 281 | -1.342 | 41.434 | 34.024 | 1.00 | 26.20 | A |
| ATOM | 1918 | O   | VAL | A | 281 | -0.329 | 41.223 | 34.688 | 1.00 | 28.44 | A |
| ATOM | 1919 | N   | LYS | A | 282 | -2.553 | 41.038 | 34.418 | 1.00 | 29.76 | A |
| ATOM | 1920 | CA  | LYS | A | 282 | -2.738 | 40.321 | 35.684 | 1.00 | 30.33 | A |
| ATOM | 1921 | CB  | LYS | A | 282 | -4.218 | 40.029 | 35.961 | 1.00 | 34.39 | A |
| ATOM | 1922 | CG  | LYS | A | 282 | -5.004 | 41.170 | 36.576 | 1.00 | 38.30 | A |
| ATOM | 1923 | CD  | LYS | A | 282 | -6.476 | 40.764 | 36.766 | 1.00 | 43.29 | A |
| ATOM | 1924 | CE  | LYS | A | 282 | -7.416 | 41.973 | 36.991 | 1.00 | 45.54 | A |
| ATOM | 1925 | NZ  | LYS | A | 282 | -7.490 | 42.891 | 35.796 | 1.00 | 45.32 | A |
| ATOM | 1926 | C   | LYS | A | 282 | -1.974 | 39.005 | 35.711 | 1.00 | 28.64 | A |
| ATOM | 1927 | O   | LYS | A | 282 | -1.337 | 38.676 | 36.701 | 1.00 | 29.38 | A |
| ATOM | 1928 | N   | ILE | A | 283 | -2.040 | 38.246 | 34.628 | 1.00 | 29.90 | A |
| ATOM | 1929 | CA  | ILE | A | 283 | -1.339 | 36.981 | 34.586 | 1.00 | 25.51 | A |
| ATOM | 1930 | CB  | ILE | A | 283 | -1.710 | 36.185 | 33.341 | 1.00 | 27.38 | A |
| ATOM | 1931 | CG2 | ILE | A | 283 | -0.754 | 35.002 | 33.171 | 1.00 | 28.54 | A |
| ATOM | 1932 | CG1 | ILE | A | 283 | -3.161 | 35.721 | 33.463 | 1.00 | 27.61 | A |
| ATOM | 1933 | CD1 | ILE | A | 283 | -3.684 | 35.033 | 32.239 | 1.00 | 27.68 | A |
| ATOM | 1934 | C   | ILE | A | 283 | 0.171  | 37.154 | 34.629 | 1.00 | 26.32 | A |
| ATOM | 1935 | O   | ILE | A | 283 | 0.854  | 36.498 | 35.418 | 1.00 | 26.70 | A |
| ATOM | 1936 | N   | MET | A | 284 | 0.693  | 38.038 | 33.787 | 1.00 | 25.44 | A |
| ATOM | 1937 | CA  | MET | A | 284 | 2.130  | 38.284 | 33.759 | 1.00 | 24.64 | A |
| ATOM | 1938 | CB  | MET | A | 284 | 2.497  | 39.265 | 32.637 | 1.00 | 26.81 | A |
| ATOM | 1939 | CG  | MET | A | 284 | 2.414  | 38.682 | 31.235 | 1.00 | 26.98 | A |
| ATOM | 1940 | SD  | MET | A | 284 | 3.551  | 37.298 | 30.977 | 1.00 | 26.74 | A |
| ATOM | 1941 | CE  | MET | A | 284 | 5.142  | 38.159 | 30.868 | 1.00 | 27.75 | A |

| ATOM | 1942 | C   | MET A 284 | 2.617  | 38.826 | 35.092 | 1.00 | 26.75 | A |
|------|------|-----|-----------|--------|--------|--------|------|-------|---|
| ATOM | 1943 | O   | MET A 284 | 3.704  | 38.485 | 35.536 | 1.00 | 25.21 | A |
| ATOM | 1944 | N   | THR A 285 | 1.821  | 39.676 | 35.728 | 1.00 | 29.06 | A |
| ATOM | 1945 | CA  | THR A 285 | 2.218  | 40.223 | 37.022 | 1.00 | 30.66 | A |
| ATOM | 1946 | CB  | THR A 285 | 1.187  | 41.254 | 37.545 | 1.00 | 31.57 | A |
| ATOM | 1947 | OG1 | THR A 285 | 1.124  | 42.361 | 36.636 | 1.00 | 33.78 | A |
| ATOM | 1948 | CG2 | THR A 285 | 1.580  | 41.760 | 38.910 | 1.00 | 32.40 | A |
| ATOM | 1949 | C   | THR A 285 | 2.387  | 39.100 | 38.052 | 1.00 | 30.87 | A |
| ATOM | 1950 | O   | THR A 285 | 3.340  | 39.119 | 38.821 | 1.00 | 32.41 | A |
| ATOM | 1951 | N   | HIS A 286 | 1.483  | 38.117 | 38.051 | 1.00 | 32.33 | A |
| ATOM | 1952 | CA  | HIS A 286 | 1.563  | 36.997 | 38.999 | 1.00 | 34.68 | A |
| ATOM | 1953 | CB  | HIS A 286 | 0.280  | 36.159 | 38.996 | 1.00 | 36.92 | A |
| ATOM | 1954 | CG  | HIS A 286 | -0.927 | 36.886 | 39.499 | 1.00 | 39.19 | A |
| ATOM | 1955 | CD2 | HIS A 286 | -2.200 | 36.917 | 39.037 | 1.00 | 40.16 | A |
| ATOM | 1956 | ND1 | HIS A 286 | -0.905 | 37.676 | 40.630 | 1.00 | 40.71 | A |
| ATOM | 1957 | CE1 | HIS A 286 | -2.115 | 38.162 | 40.841 | 1.00 | 41.26 | A |
| ATOM | 1958 | NE2 | HIS A 286 | -2.919 | 37.716 | 39.890 | 1.00 | 42.31 | A |
| ATOM | 1959 | C   | HIS A 286 | 2.732  | 36.066 | 38.717 | 1.00 | 34.08 | A |
| ATOM | 1960 | O   | HIS A 286 | 3.303  | 35.485 | 39.640 | 1.00 | 34.01 | A |
| ATOM | 1961 | N   | LEU A 287 | 3.072  | 35.911 | 37.441 | 1.00 | 33.11 | A |
| ATOM | 1962 | CA  | LEU A 287 | 4.182  | 35.048 | 37.059 | 1.00 | 30.75 | A |
| ATOM | 1963 | CB  | LEU A 287 | 4.143  | 34.725 | 35.557 | 1.00 | 33.56 | A |
| ATOM | 1964 | CG  | LEU A 287 | 3.169  | 33.669 | 35.057 | 1.00 | 32.81 | A |
| ATOM | 1965 | CD1 | LEU A 287 | 3.259  | 33.584 | 33.550 | 1.00 | 33.43 | A |
| ATOM | 1966 | CD2 | LEU A 287 | 3.491  | 32.344 | 35.703 | 1.00 | 32.19 | A |
| ATOM | 1967 | C   | LEU A 287 | 5.514  | 35.704 | 37.395 | 1.00 | 31.98 | A |
| ATOM | 1968 | O   | LEU A 287 | 6.494  | 35.010 | 37.627 | 1.00 | 29.11 | A |
| ATOM | 1969 | N   | MET A 288 | 5.551  | 37.034 | 37.422 | 1.00 | 33.16 | A |
| ATOM | 1970 | CA  | MET A 288 | 6.792  | 37.734 | 37.731 | 1.00 | 33.34 | A |
| ATOM | 1971 | CB  | MET A 288 | 6.606  | 39.257 | 37.656 | 1.00 | 36.69 | A |
| ATOM | 1972 | CG  | MET A 288 | 6.620  | 39.828 | 36.233 | 1.00 | 38.49 | A |
| ATOM | 1973 | SD  | MET A 288 | 7.925  | 39.118 | 35.201 | 1.00 | 42.46 | A |
| ATOM | 1974 | CE  | MET A 288 | 9.389  | 39.906 | 35.857 | 1.00 | 39.63 | A |
| ATOM | 1975 | C   | MET A 288 | 7.344  | 37.357 | 39.105 | 1.00 | 34.93 | A |
| ATOM | 1976 | O   | MET A 288 | 8.518  | 37.589 | 39.401 | 1.00 | 35.90 | A |
| ATOM | 1977 | N   | ARG A 289 | 6.495  | 36.772 | 39.939 | 1.00 | 34.87 | A |
| ATOM | 1978 | CA  | ARG A 289 | 6.893  | 36.368 | 41.280 | 1.00 | 34.86 | A |
| ATOM | 1979 | CB  | ARG A 289 | 5.653  | 35.901 | 42.040 | 1.00 | 37.28 | A |
| ATOM | 1980 | CG  | ARG A 289 | 5.927  | 35.315 | 43.404 | 1.00 | 42.37 | A |
| ATOM | 1981 | CD  | ARG A 289 | 4.725  | 34.506 | 43.885 | 1.00 | 45.23 | A |
| ATOM | 1982 | NE  | ARG A 289 | 5.016  | 33.793 | 45.123 | 1.00 | 47.27 | A |
| ATOM | 1983 | CZ  | ARG A 289 | 5.229  | 34.383 | 46.293 | 1.00 | 49.75 | A |
| ATOM | 1984 | NH1 | ARG A 289 | 5.178  | 35.709 | 46.401 | 1.00 | 50.71 | A |
| ATOM | 1985 | NH2 | ARG A 289 | 5.504  | 33.637 | 47.353 | 1.00 | 50.53 | A |
| ATOM | 1986 | C   | ARG A 289 | 7.950  | 35.252 | 41.221 | 1.00 | 34.85 | A |
| ATOM | 1987 | O   | ARG A 289 | 8.733  | 35.055 | 42.161 | 1.00 | 33.34 | A |
| ATOM | 1988 | N   | TYR A 290 | 7.967  | 34.540 | 40.097 | 1.00 | 31.71 | A |
| ATOM | 1989 | CA  | TYR A 290 | 8.902  | 33.450 | 39.871 | 1.00 | 31.54 | A |
| ATOM | 1990 | CB  | TYR A 290 | 8.141  | 32.241 | 39.343 | 1.00 | 31.20 | A |
| ATOM | 1991 | CG  | TYR A 290 | 7.014  | 31.859 | 40.261 | 1.00 | 31.27 | A |
| ATOM | 1992 | CD1 | TYR A 290 | 5.724  | 32.349 | 40.061 | 1.00 | 34.20 | A |
| ATOM | 1993 | CE1 | TYR A 290 | 4.702  | 32.074 | 40.977 | 1.00 | 33.56 | A |
| ATOM | 1994 | CD2 | TYR A 290 | 7.257  | 31.081 | 41.395 | 1.00 | 33.56 | A |
| ATOM | 1995 | CE2 | TYR A 290 | 6.253  | 30.801 | 42.317 | 1.00 | 33.50 | A |

| ATOM | 1996 | CZ | TYR A 290 | 4.981 | 31.298 | 42.105 | 1.00 | 33.10 | A |
|------|------|-----|-----------|--------|--------|--------|------|-------|---|
| ATOM | 1997 | OH | TYR A 290 | 3.998 | 31.016 | 43.027 | 1.00 | 35.24 | A |
| ATOM | 1998 | C | TYR A 290 | 10.001 | 33.853 | 38.900 | 1.00 | 29.02 | A |
| ATOM | 1999 | O | TYR A 290 | 10.707 | 33.004 | 38.358 | 1.00 | 29.64 | A |
| ATOM | 2000 | N | PHE A 291 | 10.132 | 35.159 | 38.684 | 1.00 | 28.42 | A |
| ATOM | 2001 | CA | PHE A 291 | 11.139 | 35.696 | 37.786 | 1.00 | 29.11 | A |
| ATOM | 2002 | CB | PHE A 291 | 10.534 | 36.115 | 36.439 | 1.00 | 27.62 | A |
| ATOM | 2003 | CG | PHE A 291 | 10.066 | 34.962 | 35.599 | 1.00 | 26.90 | A |
| ATOM | 2004 | CD1 | PHE A 291 | 8.737 | 34.561 | 35.616 | 1.00 | 27.35 | A |
| ATOM | 2005 | CD2 | PHE A 291 | 10.969 | 34.250 | 34.820 | 1.00 | 29.95 | A |
| ATOM | 2006 | CE1 | PHE A 291 | 8.317 | 33.475 | 34.873 | 1.00 | 29.39 | A |
| ATOM | 2007 | CE2 | PHE A 291 | 10.554 | 33.158 | 34.072 | 1.00 | 29.48 | A |
| ATOM | 2008 | CZ | PHE A 291 | 9.228 | 32.772 | 34.101 | 1.00 | 29.09 | A |
| ATOM | 2009 | C | PHE A 291 | 11.815 | 36.894 | 38.410 | 1.00 | 30.02 | A |
| ATOM | 2010 | O | PHE A 291 | 11.666 | 38.019 | 37.923 | 1.00 | 28.64 | A |
| ATOM | 2011 | N | PRO A 292 | 12.571 | 36.673 | 39.505 | 1.00 | 30.77 | A |
| ATOM | 2012 | CD | PRO A 292 | 12.748 | 35.402 | 40.242 | 1.00 | 33.40 | A |
| ATOM | 2013 | CA | PRO A 292 | 13.275 | 37.772 | 40.186 | 1.00 | 32.58 | A |
| ATOM | 2014 | CB | PRO A 292 | 13.589 | 37.171 | 41.558 | 1.00 | 33.81 | A |
| ATOM | 2015 | CG | PRO A 292 | 13.868 | 35.733 | 41.218 | 1.00 | 32.48 | A |
| ATOM | 2016 | C | PRO A 292 | 14.539 | 38.183 | 39.414 | 1.00 | 34.49 | A |
| ATOM | 2017 | O | PRO A 292 | 15.174 | 37.349 | 38.762 | 1.00 | 34.68 | A |
| ATOM | 2018 | N | GLY A 293 | 14.887 | 39.467 | 39.479 | 1.00 | 33.23 | A |
| ATOM | 2019 | CA | GLY A 293 | 16.070 | 39.952 | 38.786 | 1.00 | 33.99 | A |
| ATOM | 2020 | C | GLY A 293 | 15.833 | 40.777 | 37.529 | 1.00 | 34.43 | A |
| ATOM | 2021 | O | GLY A 293 | 16.749 | 40.962 | 36.731 | 1.00 | 34.74 | A |
| ATOM | 2022 | N | ALA A 294 | 14.618 | 41.280 | 37.351 | 1.00 | 36.76 | A |
| ATOM | 2023 | CA | ALA A 294 | 14.286 | 42.087 | 36.183 | 1.00 | 37.43 | A |
| ATOM | 2024 | CB | ALA A 294 | 12.765 | 42.194 | 36.036 | 1.00 | 36.75 | A |
| ATOM | 2025 | C | ALA A 294 | 14.893 | 43.483 | 36.271 | 1.00 | 39.03 | A |
| ATOM | 2026 | O | ALA A 294 | 14.975 | 44.191 | 35.270 | 1.00 | 40.22 | A |
| ATOM | 2027 | N | ASP A 295 | 15.324 | 43.868 | 37.469 | 1.00 | 41.66 | A |
| ATOM | 2028 | CA | ASP A 295 | 15.919 | 45.184 | 37.720 | 1.00 | 44.30 | A |
| ATOM | 2029 | CB | ASP A 295 | 15.736 | 45.548 | 39.203 | 1.00 | 47.87 | A |
| ATOM | 2030 | CG | ASP A 295 | 16.068 | 44.371 | 40.153 | 1.00 | 51.79 | A |
| ATOM | 2031 | OD1 | ASP A 295 | 15.337 | 43.344 | 40.131 | 1.00 | 54.23 | A |
| ATOM | 2032 | OD2 | ASP A 295 | 17.059 | 44.471 | 40.920 | 1.00 | 54.07 | A |
| ATOM | 2033 | C | ASP A 295 | 17.396 | 45.312 | 37.348 | 1.00 | 40.64 | A |
| ATOM | 2034 | O | ASP A 295 | 17.932 | 46.418 | 37.311 | 1.00 | 39.35 | A |
| ATOM | 2035 | N | GLU A 296 | 18.050 | 44.184 | 37.086 | 1.00 | 40.01 | A |
| ATOM | 2036 | CA | GLU A 296 | 19.461 | 44.174 | 36.720 | 1.00 | 39.02 | A |
| ATOM | 2037 | CB | GLU A 296 | 20.009 | 42.749 | 36.782 | 1.00 | 42.82 | A |
| ATOM | 2038 | CG | GLU A 296 | 19.856 | 42.122 | 38.140 | 1.00 | 47.49 | A |
| ATOM | 2039 | CD | GLU A 296 | 20.579 | 42.905 | 39.225 | 1.00 | 50.98 | A |
| ATOM | 2040 | OE1 | GLU A 296 | 20.083 | 42.922 | 40.377 | 1.00 | 52.28 | A |
| ATOM | 2041 | OE2 | GLU A 296 | 21.650 | 43.491 | 38.928 | 1.00 | 53.10 | A |
| ATOM | 2042 | C | GLU A 296 | 19.667 | 44.721 | 35.316 | 1.00 | 39.22 | A |
| ATOM | 2043 | O | GLU A 296 | 18.998 | 44.298 | 34.372 | 1.00 | 38.69 | A |
| ATOM | 2044 | N | PRO A 297 | 20.608 | 45.663 | 35.156 | 1.00 | 39.62 | A |
| ATOM | 2045 | CD | PRO A 297 | 21.491 | 46.270 | 36.168 | 1.00 | 38.77 | A |
| ATOM | 2046 | CA | PRO A 297 | 20.862 | 46.235 | 33.834 | 1.00 | 38.09 | A |
| ATOM | 2047 | CB | PRO A 297 | 21.703 | 47.452 | 34.160 | 1.00 | 37.94 | A |
| ATOM | 2048 | CG | PRO A 297 | 22.529 | 46.946 | 35.303 | 1.00 | 38.61 | A |
| ATOM | 2049 | C | PRO A 297 | 21.583 | 45.275 | 32.907 | 1.00 | 39.31 | A |

| ATOM | 2050 | O   | PRO | A | 297 | 22.283 | 44.374 | 33.363 | 1.00 | 39.71 | A |
|------|------|-----|-----|---|-----|--------|--------|--------|------|-------|---|
| ATOM | 2051 | N   | LEU | A | 298 | 21.391 | 45.468 | 31.606 | 1.00 | 40.24 | A |
| ATOM | 2052 | CA  | LEU | A | 298 | 22.048 | 44.650 | 30.605 | 1.00 | 40.35 | A |
| ATOM | 2053 | CB  | LEU | A | 298 | 21.384 | 44.844 | 29.236 | 1.00 | 40.45 | A |
| ATOM | 2054 | CG  | LEU | A | 298 | 19.917 | 44.445 | 29.064 | 1.00 | 40.20 | A |
| ATOM | 2055 | CD1 | LEU | A | 298 | 19.522 | 44.685 | 27.631 | 1.00 | 39.67 | A |
| ATOM | 2056 | CD2 | LEU | A | 298 | 19.706 | 42.991 | 29.435 | 1.00 | 39.63 | A |
| ATOM | 2057 | C   | LEU | A | 298 | 23.486 | 45.160 | 30.573 | 1.00 | 41.61 | A |
| ATOM | 2058 | O   | LEU | A | 298 | 23.724 | 46.319 | 30.235 | 1.00 | 42.89 | A |
| ATOM | 2059 | N   | GLN | A | 299 | 24.442 | 44.305 | 30.935 | 1.00 | 41.94 | A |
| ATOM | 2060 | CA  | GLN | A | 299 | 25.844 | 44.714 | 30.973 | 1.00 | 41.73 | A |
| ATOM | 2061 | CB  | GLN | A | 299 | 26.218 | 45.175 | 32.380 | 1.00 | 42.58 | A |
| ATOM | 2062 | CG  | GLN | A | 299 | 26.114 | 44.099 | 33.426 | 1.00 | 44.30 | A |
| ATOM | 2063 | CD  | GLN | A | 299 | 26.344 | 44.649 | 34.807 | 1.00 | 45.55 | A |
| ATOM | 2064 | OE1 | GLN | A | 299 | 25.433 | 44.695 | 35.636 | 1.00 | 46.44 | A |
| ATOM | 2065 | NE2 | GLN | A | 299 | 27.564 | 45.093 | 35.061 | 1.00 | 45.98 | A |
| ATOM | 2066 | C   | GLN | A | 299 | 26.848 | 43.672 | 30.526 | 1.00 | 42.55 | A |
| ATOM | 2067 | O   | GLN | A | 299 | 28.038 | 43.955 | 30.495 | 1.00 | 42.03 | A |
| ATOM | 2068 | N   | TYR | A | 300 | 26.387 | 42.468 | 30.199 | 1.00 | 42.00 | A |
| ATOM | 2069 | CA  | TYR | A | 300 | 27.294 | 41.419 | 29.732 | 1.00 | 44.20 | A |
| ATOM | 2070 | CB  | TYR | A | 300 | 27.053 | 40.108 | 30.481 | 1.00 | 42.75 | A |
| ATOM | 2071 | CG  | TYR | A | 300 | 27.142 | 40.268 | 31.972 | 1.00 | 40.50 | A |
| ATOM | 2072 | CD1 | TYR | A | 300 | 25.991 | 40.410 | 32.744 | 1.00 | 41.00 | A |
| ATOM | 2073 | CE1 | TYR | A | 300 | 26.069 | 40.668 | 34.107 | 1.00 | 42.08 | A |
| ATOM | 2074 | CD2 | TYR | A | 300 | 28.381 | 40.380 | 32.603 | 1.00 | 41.60 | A |
| ATOM | 2075 | CE2 | TYR | A | 300 | 28.472 | 40.640 | 33.972 | 1.00 | 40.04 | A |
| ATOM | 2076 | CZ  | TYR | A | 300 | 27.311 | 40.783 | 34.711 | 1.00 | 40.41 | A |
| ATOM | 2077 | OH  | TYR | A | 300 | 27.389 | 41.043 | 36.047 | 1.00 | 40.64 | A |
| ATOM | 2078 | C   | TYR | A | 300 | 27.079 | 41.207 | 28.249 | 1.00 | 46.03 | A |
| ATOM | 2079 | O   | TYR | A | 300 | 25.943 | 41.209 | 27.772 | 1.00 | 45.90 | A |
| ATOM | 2080 | N   | PRO | A | 301 | 28.171 | 41.053 | 27.487 | 1.00 | 48.92 | A |
| ATOM | 2081 | CD  | PRO | A | 301 | 29.583 | 41.136 | 27.907 | 1.00 | 48.75 | A |
| ATOM | 2082 | CA  | PRO | A | 301 | 28.067 | 40.838 | 26.038 | 1.00 | 50.10 | A |
| ATOM | 2083 | CB  | PRO | A | 301 | 29.368 | 41.415 | 25.519 | 1.00 | 49.69 | A |
| ATOM | 2084 | CG  | PRO | A | 301 | 30.339 | 40.923 | 26.584 | 1.00 | 49.35 | A |
| ATOM | 2085 | C   | PRO | A | 301 | 28.007 | 39.339 | 25.828 | 1.00 | 51.98 | A |
| ATOM | 2086 | O   | PRO | A | 301 | 28.786 | 38.601 | 26.432 | 1.00 | 52.79 | A |
| ATOM | 2087 | N   | CYS | A | 302 | 27.097 | 38.859 | 24.999 | 1.00 | 54.64 | A |
| ATOM | 2088 | CA  | CYS | A | 302 | 27.052 | 37.427 | 24.814 | 1.00 | 57.23 | A |
| ATOM | 2089 | CB  | CYS | A | 302 | 25.688 | 36.871 | 25.238 | 1.00 | 57.69 | A |
| ATOM | 2090 | SG  | CYS | A | 302 | 24.504 | 36.652 | 23.922 | 1.00 | 61.09 | A |
| ATOM | 2091 | C   | CYS | A | 302 | 27.371 | 37.092 | 23.372 | 1.00 | 57.80 | A |
| ATOM | 2092 | O   | CYS | A | 302 | 27.185 | 37.905 | 22.475 | 1.00 | 57.84 | A |
| ATOM | 2093 | N   | GLN | A | 303 | 27.889 | 35.891 | 23.170 | 1.00 | 59.87 | A |
| ATOM | 2094 | CA  | GLN | A | 303 | 28.260 | 35.414 | 21.851 | 1.00 | 61.22 | A |
| ATOM | 2095 | CB  | GLN | A | 303 | 29.634 | 34.720 | 21.938 | 1.00 | 57.95 | A |
| ATOM | 2096 | CG  | GLN | A | 303 | 30.783 | 35.551 | 22.617 | 1.00 | 54.29 | A |
| ATOM | 2097 | CD  | GLN | A | 303 | 30.858 | 35.444 | 24.165 | 1.00 | 51.39 | A |
| ATOM | 2098 | OE1 | GLN | A | 303 | 30.766 | 34.351 | 24.738 | 1.00 | 50.26 | A |
| ATOM | 2099 | NE2 | GLN | A | 303 | 31.063 | 36.587 | 24.830 | 1.00 | 49.00 | A |
| ATOM | 2100 | C   | GLN | A | 303 | 27.160 | 34.427 | 21.416 | 1.00 | 63.97 | A |
| ATOM | 2101 | O   | GLN | A | 303 | 27.381 | 33.216 | 21.352 | 1.00 | 65.24 | A |
| ATOM | 2102 | N   | HIS | A | 468 | 25.974 | 34.964 | 21.125 | 1.00 | 67.64 | A |
| ATOM | 2103 | CA  | HIS | A | 468 | 24.808 | 34.169 | 20.736 | 1.00 | 69.78 | A |

| ATOM | 2104 | CB  | HIS | A | 468 | 23.580 | 35.079 | 20.595 | 1.00 | 68.19 | A |
| ATOM | 2105 | CG  | HIS | A | 468 | 22.295 | 34.332 | 20.401 | 1.00 | 67.57 | A |
| ATOM | 2106 | CD2 | HIS | A | 468 | 21.932 | 33.074 | 20.749 | 1.00 | 66.91 | A |
| ATOM | 2107 | ND1 | HIS | A | 468 | 21.204 | 34.881 | 19.764 | 1.00 | 66.94 | A |
| ATOM | 2108 | CE1 | HIS | A | 468 | 20.226 | 33.993 | 19.722 | 1.00 | 66.67 | A |
| ATOM | 2109 | NE2 | HIS | A | 468 | 20.642 | 32.888 | 20.313 | 1.00 | 66.58 | A |
| ATOM | 2110 | C   | HIS | A | 468 | 24.968 | 33.340 | 19.460 | 1.00 | 72.43 | A |
| ATOM | 2111 | O   | HIS | A | 468 | 25.209 | 33.881 | 18.373 | 1.00 | 72.96 | A |
| ATOM | 2112 | N   | SER | A | 469 | 24.815 | 32.024 | 19.611 | 1.00 | 75.72 | A |
| ATOM | 2113 | CA  | SER | A | 469 | 24.924 | 31.077 | 18.499 | 1.00 | 77.69 | A |
| ATOM | 2114 | CB  | SER | A | 469 | 25.117 | 29.650 | 19.044 | 1.00 | 78.40 | A |
| ATOM | 2115 | OG  | SER | A | 469 | 24.037 | 29.259 | 19.882 | 1.00 | 78.70 | A |
| ATOM | 2116 | C   | SER | A | 469 | 23.682 | 31.139 | 17.591 | 1.00 | 79.56 | A |
| ATOM | 2117 | O   | SER | A | 469 | 23.572 | 32.048 | 16.759 | 1.00 | 79.78 | A |
| ATOM | 2118 | N   | LEU | A | 470 | 22.773 | 30.169 | 17.761 | 1.00 | 81.16 | A |
| ATOM | 2119 | CA  | LEU | A | 470 | 21.506 | 30.040 | 17.012 | 1.00 | 83.30 | A |
| ATOM | 2120 | CB  | LEU | A | 470 | 21.098 | 31.370 | 16.348 | 1.00 | 83.15 | A |
| ATOM | 2121 | CG  | LEU | A | 470 | 19.617 | 31.672 | 16.043 | 1.00 | 82.77 | A |
| ATOM | 2122 | CD1 | LEU | A | 470 | 19.065 | 30.710 | 15.002 | 1.00 | 82.53 | A |
| ATOM | 2123 | CD2 | LEU | A | 470 | 18.815 | 31.605 | 17.338 | 1.00 | 82.50 | A |
| ATOM | 2124 | C   | LEU | A | 470 | 21.512 | 28.929 | 15.951 | 1.00 | 84.67 | A |
| ATOM | 2125 | O   | LEU | A | 470 | 21.784 | 29.176 | 14.766 | 1.00 | 84.43 | A |
| ATOM | 2126 | N   | PRO | A | 471 | 21.217 | 27.684 | 16.374 | 1.00 | 86.38 | A |
| ATOM | 2127 | CD  | PRO | A | 471 | 21.263 | 27.295 | 17.801 | 1.00 | 86.76 | A |
| ATOM | 2128 | CA  | PRO | A | 471 | 21.165 | 26.495 | 15.512 | 1.00 | 87.71 | A |
| ATOM | 2129 | CB  | PRO | A | 471 | 21.761 | 25.420 | 16.405 | 1.00 | 87.42 | A |
| ATOM | 2130 | CG  | PRO | A | 471 | 21.161 | 25.775 | 17.750 | 1.00 | 87.04 | A |
| ATOM | 2131 | C   | PRO | A | 471 | 19.720 | 26.159 | 15.092 | 1.00 | 89.01 | A |
| ATOM | 2132 | O   | PRO | A | 471 | 19.017 | 25.439 | 15.810 | 1.00 | 89.60 | A |
| ATOM | 2133 | N   | PRO | A | 472 | 19.264 | 26.663 | 13.922 | 1.00 | 89.94 | A |
| ATOM | 2134 | CD  | PRO | A | 472 | 20.002 | 27.562 | 13.014 | 1.00 | 90.24 | A |
| ATOM | 2135 | CA  | PRO | A | 472 | 17.900 | 26.423 | 13.410 | 1.00 | 90.67 | A |
| ATOM | 2136 | CB  | PRO | A | 472 | 17.896 | 27.166 | 12.069 | 1.00 | 90.63 | A |
| ATOM | 2137 | CG  | PRO | A | 472 | 18.873 | 28.278 | 12.294 | 1.00 | 90.53 | A |
| ATOM | 2138 | C   | PRO | A | 472 | 17.462 | 24.954 | 13.256 | 1.00 | 91.07 | A |
| ATOM | 2139 | O   | PRO | A | 472 | 16.763 | 24.407 | 14.122 | 1.00 | 90.76 | A |
| ATOM | 2140 | N   | GLY | A | 473 | 17.858 | 24.343 | 12.135 | 1.00 | 91.24 | A |
| ATOM | 2141 | CA  | GLY | A | 473 | 17.524 | 22.953 | 11.847 | 1.00 | 91.05 | A |
| ATOM | 2142 | C   | GLY | A | 473 | 16.090 | 22.549 | 12.158 | 1.00 | 90.61 | A |
| ATOM | 2143 | O   | GLY | A | 473 | 15.868 | 21.638 | 12.961 | 1.00 | 90.66 | A |
| ATOM | 2144 | N   | GLU | A | 474 | 15.131 | 23.214 | 11.508 | 1.00 | 89.97 | A |
| ATOM | 2145 | CA  | GLU | A | 474 | 13.689 | 22.978 | 11.687 | 1.00 | 89.57 | A |
| ATOM | 2146 | CB  | GLU | A | 474 | 12.950 | 23.242 | 10.367 | 1.00 | 90.22 | A |
| ATOM | 2147 | CG  | GLU | A | 474 | 11.429 | 23.273 | 10.500 | 1.00 | 91.12 | A |
| ATOM | 2148 | CD  | GLU | A | 474 | 10.717 | 23.033 |  9.178 | 1.00 | 91.74 | A |
| ATOM | 2149 | OE1 | GLU | A | 474 | 11.128 | 23.628 |  8.159 | 1.00 | 92.39 | A |
| ATOM | 2150 | OE2 | GLU | A | 474 |  9.741 | 22.253 |  9.161 | 1.00 | 92.30 | A |
| ATOM | 2151 | C   | GLU | A | 474 | 13.262 | 21.597 | 12.216 | 1.00 | 88.17 | A |
| ATOM | 2152 | O   | GLU | A | 474 | 12.720 | 20.777 | 11.466 | 1.00 | 88.61 | A |
| ATOM | 2153 | N   | ASP | A | 475 | 13.492 | 21.346 | 13.505 | 1.00 | 86.74 | A |
| ATOM | 2154 | CA  | ASP | A | 475 | 13.101 | 20.073 | 14.113 | 1.00 | 85.44 | A |
| ATOM | 2155 | CB  | ASP | A | 475 | 13.761 | 19.883 | 15.489 | 1.00 | 86.52 | A |
| ATOM | 2156 | CG  | ASP | A | 475 | 15.081 | 20.628 | 15.626 | 1.00 | 87.68 | A |
| ATOM | 2157 | OD1 | ASP | A | 475 | 15.075 | 21.877 | 15.555 | 1.00 | 88.43 | A |

| ATOM | 2158 | OD2 | ASP | A | 475 | 16.125 | 19.966 | 15.814 | 1.00 | 88.22 | A |
|------|------|-----|-----|---|-----|--------|--------|--------|------|-------|---|
| ATOM | 2159 | C | ASP | A | 475 | 11.595 | 20.178 | 14.308 | 1.00 | 82.53 | A |
| ATOM | 2160 | O | ASP | A | 475 | 10.880 | 20.732 | 13.469 | 1.00 | 82.00 | A |
| ATOM | 2161 | N | GLY | A | 476 | 11.117 | 19.648 | 15.424 | 1.00 | 79.47 | A |
| ATOM | 2162 | CA | GLY | A | 476 | 9.704 | 19.735 | 15.724 | 1.00 | 75.13 | A |
| ATOM | 2163 | C | GLY | A | 476 | 9.584 | 20.555 | 16.991 | 1.00 | 72.14 | A |
| ATOM | 2164 | O | GLY | A | 476 | 8.798 | 20.221 | 17.876 | 1.00 | 72.91 | A |
| ATOM | 2165 | N | ARG | A | 477 | 10.373 | 21.628 | 17.068 | 1.00 | 68.18 | A |
| ATOM | 2166 | CA | ARG | A | 477 | 10.416 | 22.512 | 18.232 | 1.00 | 64.02 | A |
| ATOM | 2167 | CB | ARG | A | 477 | 10.436 | 21.690 | 19.527 | 1.00 | 65.85 | A |
| ATOM | 2168 | CG | ARG | A | 477 | 9.080 | 21.455 | 20.162 | 1.00 | 67.43 | A |
| ATOM | 2169 | CD | ARG | A | 477 | 9.069 | 20.213 | 21.052 | 1.00 | 69.84 | A |
| ATOM | 2170 | NE | ARG | A | 477 | 7.761 | 20.047 | 21.682 | 1.00 | 71.62 | A |
| ATOM | 2171 | CZ | ARG | A | 477 | 7.299 | 20.825 | 22.661 | 1.00 | 72.96 | A |
| ATOM | 2172 | NH1 | ARG | A | 477 | 8.049 | 21.815 | 23.131 | 1.00 | 73.88 | A |
| ATOM | 2173 | NH2 | ARG | A | 477 | 6.075 | 20.642 | 23.147 | 1.00 | 73.13 | A |
| ATOM | 2174 | C | ARG | A | 477 | 11.694 | 23.338 | 18.167 | 1.00 | 59.42 | A |
| ATOM | 2175 | O | ARG | A | 477 | 12.545 | 23.100 | 17.310 | 1.00 | 60.05 | A |
| ATOM | 2176 | N | VAL | A | 478 | 11.817 | 24.311 | 19.068 | 1.00 | 54.57 | A |
| ATOM | 2177 | CA | VAL | A | 478 | 13.009 | 25.156 | 19.156 | 1.00 | 48.98 | A |
| ATOM | 2178 | CB | VAL | A | 478 | 12.809 | 26.589 | 18.561 | 1.00 | 46.69 | A |
| ATOM | 2179 | CG1 | VAL | A | 478 | 12.564 | 26.501 | 17.066 | 1.00 | 46.44 | A |
| ATOM | 2180 | CG2 | VAL | A | 478 | 11.687 | 27.323 | 19.274 | 1.00 | 45.74 | A |
| ATOM | 2181 | C | VAL | A | 478 | 13.370 | 25.301 | 20.626 | 1.00 | 46.38 | A |
| ATOM | 2182 | O | VAL | A | 478 | 12.511 | 25.211 | 21.492 | 1.00 | 45.46 | A |
| ATOM | 2183 | N | GLU | A | 479 | 14.646 | 25.516 | 20.907 | 1.00 | 45.43 | A |
| ATOM | 2184 | CA | GLU | A | 479 | 15.093 | 25.670 | 22.276 | 1.00 | 45.27 | A |
| ATOM | 2185 | CB | GLU | A | 479 | 16.543 | 25.203 | 22.406 | 1.00 | 50.71 | A |
| ATOM | 2186 | CG | GLU | A | 479 | 16.717 | 23.741 | 22.067 | 1.00 | 57.36 | A |
| ATOM | 2187 | CD | GLU | A | 479 | 17.167 | 22.916 | 23.253 | 1.00 | 61.17 | A |
| ATOM | 2188 | OE1 | GLU | A | 479 | 16.939 | 23.350 | 24.405 | 1.00 | 63.56 | A |
| ATOM | 2189 | OE2 | GLU | A | 479 | 17.738 | 21.827 | 23.032 | 1.00 | 63.66 | A |
| ATOM | 2190 | C | GLU | A | 479 | 14.977 | 27.118 | 22.704 | 1.00 | 41.96 | A |
| ATOM | 2191 | O | GLU | A | 479 | 15.134 | 28.029 | 21.902 | 1.00 | 40.11 | A |
| ATOM | 2192 | N | PRO | A | 480 | 14.673 | 27.356 | 23.983 | 1.00 | 39.25 | A |
| ATOM | 2193 | CD | PRO | A | 480 | 14.284 | 26.454 | 25.084 | 1.00 | 37.54 | A |
| ATOM | 2194 | CA | PRO | A | 480 | 14.572 | 28.755 | 24.390 | 1.00 | 36.93 | A |
| ATOM | 2195 | CB | PRO | A | 480 | 13.825 | 28.670 | 25.719 | 1.00 | 37.49 | A |
| ATOM | 2196 | CG | PRO | A | 480 | 14.334 | 27.371 | 26.287 | 1.00 | 37.75 | A |
| ATOM | 2197 | C | PRO | A | 480 | 15.965 | 29.347 | 24.561 | 1.00 | 35.71 | A |
| ATOM | 2198 | O | PRO | A | 480 | 16.926 | 28.618 | 24.817 | 1.00 | 36.69 | A |
| ATOM | 2199 | N | TYR | A | 481 | 16.076 | 30.661 | 24.396 | 1.00 | 33.16 | A |
| ATOM | 2200 | CA | TYR | A | 481 | 17.346 | 31.341 | 24.599 | 1.00 | 32.69 | A |
| ATOM | 2201 | CB | TYR | A | 481 | 17.337 | 32.731 | 23.966 | 1.00 | 30.79 | A |
| ATOM | 2202 | CG | TYR | A | 481 | 18.429 | 33.617 | 24.508 | 1.00 | 29.83 | A |
| ATOM | 2203 | CD1 | TYR | A | 481 | 19.768 | 33.359 | 24.213 | 1.00 | 30.40 | A |
| ATOM | 2204 | CE1 | TYR | A | 481 | 20.793 | 34.148 | 24.742 | 1.00 | 30.70 | A |
| ATOM | 2205 | CD2 | TYR | A | 481 | 18.136 | 34.688 | 25.349 | 1.00 | 31.12 | A |
| ATOM | 2206 | CE2 | TYR | A | 481 | 19.154 | 35.482 | 25.887 | 1.00 | 30.32 | A |
| ATOM | 2207 | CZ | TYR | A | 481 | 20.475 | 35.209 | 25.577 | 1.00 | 30.42 | A |
| ATOM | 2208 | OH | TYR | A | 481 | 21.477 | 35.999 | 26.090 | 1.00 | 31.22 | A |
| ATOM | 2209 | C | TYR | A | 481 | 17.507 | 31.493 | 26.105 | 1.00 | 31.08 | A |
| ATOM | 2210 | O | TYR | A | 481 | 18.583 | 31.283 | 26.661 | 1.00 | 29.89 | A |
| ATOM | 2211 | N | VAL | A | 482 | 16.416 | 31.865 | 26.760 | 1.00 | 30.68 | A |

| ATOM | 2212 | CA | VAL | A | 482 | 16.433 | 32.066 | 28.196 | 1.00 | 30.29 | A |
|------|------|-----|-----|---|-----|--------|--------|--------|------|-------|---|
| ATOM | 2213 | CB | VAL | A | 482 | 15.295 | 33.030 | 28.627 | 1.00 | 28.69 | A |
| ATOM | 2214 | CG1 | VAL | A | 482 | 15.473 | 34.388 | 27.934 | 1.00 | 30.22 | A |
| ATOM | 2215 | CG2 | VAL | A | 482 | 13.948 | 32.414 | 28.297 | 1.00 | 27.80 | A |
| ATOM | 2216 | C | VAL | A | 482 | 16.325 | 30.731 | 28.924 | 1.00 | 31.34 | A |
| ATOM | 2217 | O | VAL | A | 482 | 15.925 | 29.729 | 28.338 | 1.00 | 30.49 | A |
| ATOM | 2218 | N | ASP | A | 483 | 16.699 | 30.725 | 30.199 | 1.00 | 33.84 | A |
| ATOM | 2219 | CA | ASP | A | 483 | 16.671 | 29.511 | 31.014 | 1.00 | 34.89 | A |
| ATOM | 2220 | CB | ASP | A | 483 | 17.996 | 29.391 | 31.784 | 1.00 | 39.32 | A |
| ATOM | 2221 | CG | ASP | A | 483 | 18.224 | 27.997 | 32.361 | 1.00 | 42.68 | A |
| ATOM | 2222 | OD1 | ASP | A | 483 | 17.243 | 27.366 | 32.817 | 1.00 | 45.08 | A |
| ATOM | 2223 | OD2 | ASP | A | 483 | 19.391 | 27.534 | 32.373 | 1.00 | 45.33 | A |
| ATOM | 2224 | C | ASP | A | 483 | 15.495 | 29.527 | 32.013 | 1.00 | 33.73 | A |
| ATOM | 2225 | O | ASP | A | 483 | 15.305 | 30.506 | 32.738 | 1.00 | 33.68 | A |
| ATOM | 2226 | N | PHE | A | 484 | 14.717 | 28.445 | 32.054 | 1.00 | 33.53 | A |
| ATOM | 2227 | CA | PHE | A | 484 | 13.572 | 28.357 | 32.967 | 1.00 | 33.43 | A |
| ATOM | 2228 | CB | PHE | A | 484 | 12.325 | 27.840 | 32.245 | 1.00 | 32.16 | A |
| ATOM | 2229 | CG | PHE | A | 484 | 11.772 | 28.793 | 31.239 | 1.00 | 32.10 | A |
| ATOM | 2230 | CD1 | PHE | A | 484 | 11.980 | 28.587 | 29.883 | 1.00 | 32.22 | A |
| ATOM | 2231 | CD2 | PHE | A | 484 | 11.062 | 29.911 | 31.642 | 1.00 | 33.40 | A |
| ATOM | 2232 | CE1 | PHE | A | 484 | 11.488 | 29.484 | 28.933 | 1.00 | 32.74 | A |
| ATOM | 2233 | CE2 | PHE | A | 484 | 10.566 | 30.818 | 30.701 | 1.00 | 31.88 | A |
| ATOM | 2234 | CZ | PHE | A | 484 | 10.781 | 30.599 | 29.341 | 1.00 | 32.32 | A |
| ATOM | 2235 | C | PHE | A | 484 | 13.812 | 27.460 | 34.173 | 1.00 | 33.62 | A |
| ATOM | 2236 | O | PHE | A | 484 | 12.866 | 27.142 | 34.878 | 1.00 | 33.48 | A |
| ATOM | 2237 | N | ALA | A | 485 | 15.056 | 27.050 | 34.405 | 1.00 | 34.21 | A |
| ATOM | 2238 | CA | ALA | A | 485 | 15.375 | 26.185 | 35.533 | 1.00 | 33.39 | A |
| ATOM | 2239 | CB | ALA | A | 485 | 16.822 | 25.707 | 35.443 | 1.00 | 35.35 | A |
| ATOM | 2240 | C | ALA | A | 485 | 15.116 | 26.841 | 36.897 | 1.00 | 33.50 | A |
| ATOM | 2241 | O | ALA | A | 485 | 14.718 | 26.159 | 37.842 | 1.00 | 34.44 | A |
| ATOM | 2242 | N | GLU | A | 486 | 15.320 | 28.153 | 37.005 | 1.00 | 35.13 | A |
| ATOM | 2243 | CA | GLU | A | 486 | 15.077 | 28.840 | 38.271 | 1.00 | 34.07 | A |
| ATOM | 2244 | CB | GLU | A | 486 | 15.777 | 30.207 | 38.277 | 1.00 | 36.67 | A |
| ATOM | 2245 | CG | GLU | A | 486 | 15.885 | 30.875 | 39.654 | 1.00 | 38.50 | A |
| ATOM | 2246 | CD | GLU | A | 486 | 16.553 | 29.992 | 40.736 | 1.00 | 39.27 | A |
| ATOM | 2247 | OE1 | GLU | A | 486 | 17.272 | 29.022 | 40.385 | 1.00 | 41.16 | A |
| ATOM | 2248 | OE2 | GLU | A | 486 | 16.370 | 30.282 | 41.947 | 1.00 | 41.20 | A |
| ATOM | 2249 | C | GLU | A | 486 | 13.564 | 28.998 | 38.507 | 1.00 | 32.96 | A |
| ATOM | 2250 | O | GLU | A | 486 | 13.097 | 28.948 | 39.644 | 1.00 | 34.78 | A |
| ATOM | 2251 | N | PHE | A | 487 | 12.809 | 29.188 | 37.425 | 1.00 | 33.21 | A |
| ATOM | 2252 | CA | PHE | A | 487 | 11.351 | 29.330 | 37.495 | 1.00 | 31.87 | A |
| ATOM | 2253 | CB | PHE | A | 487 | 10.771 | 29.511 | 36.087 | 1.00 | 32.89 | A |
| ATOM | 2254 | CG | PHE | A | 487 | 9.275 | 29.294 | 36.009 | 1.00 | 30.40 | A |
| ATOM | 2255 | CD1 | PHE | A | 487 | 8.390 | 30.319 | 36.323 | 1.00 | 32.14 | A |
| ATOM | 2256 | CD2 | PHE | A | 487 | 8.754 | 28.052 | 35.653 | 1.00 | 31.62 | A |
| ATOM | 2257 | CE1 | PHE | A | 487 | 7.016 | 30.116 | 36.287 | 1.00 | 31.96 | A |
| ATOM | 2258 | CE2 | PHE | A | 487 | 7.382 | 27.843 | 35.617 | 1.00 | 32.17 | A |
| ATOM | 2259 | CZ | PHE | A | 487 | 6.511 | 28.879 | 35.934 | 1.00 | 32.94 | A |
| ATOM | 2260 | C | PHE | A | 487 | 10.727 | 28.085 | 38.130 | 1.00 | 30.37 | A |
| ATOM | 2261 | O | PHE | A | 487 | 9.950 | 28.170 | 39.083 | 1.00 | 31.17 | A |
| ATOM | 2262 | N | TYR | A | 488 | 11.069 | 26.933 | 37.568 | 1.00 | 32.38 | A |
| ATOM | 2263 | CA | TYR | A | 488 | 10.580 | 25.654 | 38.042 | 1.00 | 33.61 | A |
| ATOM | 2264 | CB | TYR | A | 488 | 11.154 | 24.536 | 37.185 | 1.00 | 33.40 | A |
| ATOM | 2265 | CG | TYR | A | 488 | 10.550 | 24.459 | 35.822 | 1.00 | 33.96 | A |

| ATOM | 2266 | CD1 | TYR | A | 488 | . | 11.335 | 24.597 | 34.683 | 1.00 | 34.61 | A |
|------|------|-----|-----|---|-----|---|--------|--------|--------|------|-------|---|
| ATOM | 2267 | CE1 | TYR | A | 488 | | 10.781 | 24.494 | 33.413 | 1.00 | 35.60 | A |
| ATOM | 2268 | CD2 | TYR | A | 488 | | 9.193 | 24.220 | 35.664 | 1.00 | 33.86 | A |
| ATOM | 2269 | CE2 | TYR | A | 488 | | 8.630 | 24.113 | 34.405 | 1.00 | 35.86 | A |
| ATOM | 2270 | CZ | TYR | A | 488 | | 9.427 | 24.250 | 33.282 | 1.00 | 35.68 | A |
| ATOM | 2271 | OH | TYR | A | 488 | | 8.873 | 24.139 | 32.031 | 1.00 | 35.46 | A |
| ATOM | 2272 | C | TYR | A | 488 | | 10.934 | 25.389 | 39.489 | 1.00 | 33.49 | A |
| ATOM | 2273 | O | TYR | A | 488 | | 10.141 | 24.788 | 40.216 | 1.00 | 34.58 | A |
| ATOM | 2274 | N | ARG | A | 489 | | 12.133 | 25.809 | 39.898 | 1.00 | 34.51 | A |
| ATOM | 2275 | CA | ARG | A | 489 | | 12.588 | 25.618 | 41.272 | 1.00 | 36.54 | A |
| ATOM | 2276 | CB | ARG | A | 489 | | 14.090 | 25.909 | 41.375 | 1.00 | 38.59 | A |
| ATOM | 2277 | CG | ARG | A | 489 | | 14.612 | 26.063 | 42.797 | 1.00 | 40.44 | A |
| ATOM | 2278 | CD | ARG | A | 489 | | 16.115 | 26.370 | 42.842 | 1.00 | 44.10 | A |
| ATOM | 2279 | NE | ARG | A | 489 | | 16.927 | 25.216 | 42.456 | 1.00 | 47.53 | A |
| ATOM | 2280 | CZ | ARG | A | 489 | | 17.740 | 25.170 | 41.402 | 1.00 | 49.79 | A |
| ATOM | 2281 | NH1 | ARG | A | 489 | | 17.877 | 26.219 | 40.600 | 1.00 | 50.93 | A |
| ATOM | 2282 | NH2 | ARG | A | 489 | | 18.407 | 24.058 | 41.131 | 1.00 | 51.24 | A |
| ATOM | 2283 | C | ARG | A | 489 | | 11.802 | 26.500 | 42.246 | 1.00 | 35.53 | A |
| ATOM | 2284 | O | ARG | A | 489 | | 11.400 | 26.036 | 43.306 | 1.00 | 34.63 | A |
| ATOM | 2285 | N | LEU | A | 490 | | 11.579 | 27.761 | 41.875 | 1.00 | 36.15 | A |
| ATOM | 2286 | CA | LEU | A | 490 | | 10.829 | 28.709 | 42.702 | 1.00 | 36.09 | A |
| ATOM | 2287 | CB | LEU | A | 490 | | 11.017 | 30.132 | 42.192 | 1.00 | 34.78 | A |
| ATOM | 2288 | CG | LEU | A | 490 | | 12.422 | 30.684 | 42.356 | 1.00 | 32.91 | A |
| ATOM | 2289 | CD1 | LEU | A | 490 | | 12.522 | 32.019 | 41.629 | 1.00 | 33.82 | A |
| ATOM | 2290 | CD2 | LEU | A | 490 | | 12.746 | 30.805 | 43.845 | 1.00 | 33.96 | A |
| ATOM | 2291 | C | LEU | A | 490 | | 9.348 | 28.378 | 42.696 | 1.00 | 34.65 | A |
| ATOM | 2292 | O | LEU | A | 490 | | 8.651 | 28.602 | 43.680 | 1.00 | 35.26 | A |
| ATOM | 2293 | N | TRP | A | 491 | | 8.872 | 27.857 | 41.570 | 1.00 | 36.35 | A |
| ATOM | 2294 | CA | TRP | A | 491 | | 7.474 | 27.464 | 41.420 | 1.00 | 38.34 | A |
| ATOM | 2295 | CB | TRP | A | 491 | | 7.201 | 27.093 | 39.955 | 1.00 | 38.72 | A |
| ATOM | 2296 | CG | TRP | A | 491 | | 5.813 | 26.598 | 39.676 | 1.00 | 37.02 | A |
| ATOM | 2297 | CD2 | TRP | A | 491 | | 4.681 | 27.385 | 39.263 | 1.00 | 37.96 | A |
| ATOM | 2298 | CE2 | TRP | A | 491 | | 3.582 | 26.502 | 39.143 | 1.00 | 38.56 | A |
| ATOM | 2299 | CE3 | TRP | A | 491 | | 4.489 | 28.748 | 38.982 | 1.00 | 37.80 | A |
| ATOM | 2300 | CD1 | TRP | A | 491 | | 5.366 | 25.315 | 39.787 | 1.00 | 38.17 | A |
| ATOM | 2301 | NE1 | TRP | A | 491 | | 4.027 | 25.248 | 39.470 | 1.00 | 39.05 | A |
| ATOM | 2302 | CZ2 | TRP | A | 491 | | 2.307 | 26.934 | 38.756 | 1.00 | 38.22 | A |
| ATOM | 2303 | CZ3 | TRP | A | 491 | | 3.217 | 29.179 | 38.597 | 1.00 | 36.81 | A |
| ATOM | 2304 | CH2 | TRP | A | 491 | | 2.143 | 28.270 | 38.488 | 1.00 | 37.45 | A |
| ATOM | 2305 | C | TRP | A | 491 | | 7.248 | 26.268 | 42.343 | 1.00 | 38.68 | A |
| ATOM | 2306 | O | TRP | A | 491 | | 6.341 | 26.271 | 43.167 | 1.00 | 38.57 | A |
| ATOM | 2307 | N | SER | A | 492 | | 8.105 | 25.262 | 42.196 | 1.00 | 41.50 | A |
| ATOM | 2308 | CA | SER | A | 492 | | 8.081 | 24.044 | 42.996 | 1.00 | 42.68 | A |
| ATOM | 2309 | CB | SER | A | 492 | | 9.312 | 23.201 | 42.655 | 1.00 | 44.39 | A |
| ATOM | 2310 | OG | SER | A | 492 | | 9.460 | 22.123 | 43.559 | 1.00 | 47.02 | A |
| ATOM | 2311 | C | SER | A | 492 | | 8.060 | 24.337 | 44.502 | 1.00 | 44.23 | A |
| ATOM | 2312 | O | SER | A | 492 | | 7.394 | 23.643 | 45.270 | 1.00 | 43.67 | A |
| ATOM | 2313 | N | VAL | A | 493 | | 8.796 | 25.362 | 44.917 | 1.00 | 46.02 | A |
| ATOM | 2314 | CA | VAL | A | 493 | | 8.851 | 25.749 | 46.319 | 1.00 | 47.52 | A |
| ATOM | 2315 | CB | VAL | A | 493 | | 9.920 | 26.853 | 46.543 | 1.00 | 47.91 | A |
| ATOM | 2316 | CG1 | VAL | A | 493 | | 9.773 | 27.461 | 47.922 | 1.00 | 46.65 | A |
| ATOM | 2317 | CG2 | VAL | A | 493 | | 11.314 | 26.263 | 46.377 | 1.00 | 46.95 | A |
| ATOM | 2318 | C | VAL | A | 493 | | 7.480 | 26.236 | 46.809 | 1.00 | 48.74 | A |
| ATOM | 2319 | O | VAL | A | 493 | | 7.077 | 25.934 | 47.929 | 1.00 | 50.10 | A |

| ATOM | 2320 | N | ASP | A | 494 | 6.759 | 26.973 | 45.969 | 1.00 | 52.73 | A |
|------|------|------|------|---|-----|--------|--------|--------|------|-------|---|
| ATOM | 2321 | CA | ASP | A | 494 | 5.433 | 27.492 | 46.327 | 1.00 | 54.83 | A |
| ATOM | 2322 | CB | ASP | A | 494 | 5.108 | 28.714 | 45.468 | 1.00 | 54.23 | A |
| ATOM | 2323 | CG | ASP | A | 494 | 5.427 | 30.013 | 46.164 | 1.00 | 54.85 | A |
| ATOM | 2324 | OD1 | ASP | A | 494 | 6.227 | 30.007 | 47.127 | 1.00 | 55.34 | A |
| ATOM | 2325 | OD2 | ASP | A | 494 | 4.884 | 31.047 | 45.740 | 1.00 | 55.10 | A |
| ATOM | 2326 | C | ASP | A | 494 | 4.288 | 26.478 | 46.202 | 1.00 | 56.70 | A |
| ATOM | 2327 | O | ASP | A | 494 | 3.247 | 26.613 | 46.843 | 1.00 | 57.32 | A |
| ATOM | 2328 | N | HIS | A | 495 | 4.478 | 25.468 | 45.368 | 1.00 | 58.72 | A |
| ATOM | 2329 | CA | HIS | A | 495 | 3.461 | 24.451 | 45.160 | 1.00 | 60.90 | A |
| ATOM | 2330 | CB | HIS | A | 495 | 3.131 | 24.382 | 43.666 | 1.00 | 58.81 | A |
| ATOM | 2331 | CG | HIS | A | 495 | 2.657 | 25.686 | 43.102 | 1.00 | 56.94 | A |
| ATOM | 2332 | CD2 | HIS | A | 495 | 3.345 | 26.767 | 42.664 | 1.00 | 56.34 | A |
| ATOM | 2333 | ND1 | HIS | A | 495 | 1.324 | 26.025 | 43.024 | 1.00 | 56.19 | A |
| ATOM | 2334 | CE1 | HIS | A | 495 | 1.211 | 27.258 | 42.566 | 1.00 | 55.92 | A |
| ATOM | 2335 | NE2 | HIS | A | 495 | 2.423 | 27.730 | 42.341 | 1.00 | 55.95 | A |
| ATOM | 2336 | C | HIS | A | 495 | 3.983 | 23.109 | 45.666 | 1.00 | 64.17 | A |
| ATOM | 2337 | O | HIS | A | 495 | 4.273 | 22.958 | 46.861 | 1.00 | 65.79 | A |
| ATOM | 2338 | N | GLY | A | 496 | 4.104 | 22.148 | 44.750 | 1.00 | 67.62 | A |
| ATOM | 2339 | CA | GLY | A | 496 | 4.603 | 20.824 | 45.093 | 1.00 | 70.38 | A |
| ATOM | 2340 | C | GLY | A | 496 | 5.775 | 20.444 | 44.200 | 1.00 | 71.93 | A |
| ATOM | 2341 | O | GLY | A | 496 | 6.634 | 19.643 | 44.589 | 1.00 | 72.23 | A |
| ATOM | 2342 | N | GLU | A | 497 | 5.785 | 21.026 | 42.997 | 1.00 | 73.01 | A |
| ATOM | 2343 | CA | GLU | A | 497 | 6.824 | 20.832 | 41.974 | 1.00 | 74.31 | A |
| ATOM | 2344 | CB | GLU | A | 497 | 7.272 | 19.362 | 41.890 | 1.00 | 75.66 | A |
| ATOM | 2345 | CG | GLU | A | 497 | 8.272 | 19.016 | 40.758 | 1.00 | 77.72 | A |
| ATOM | 2346 | CD | GLU | A | 497 | 9.635 | 19.705 | 40.904 | 1.00 | 78.75 | A |
| ATOM | 2347 | OE1 | GLU | A | 497 | 10.293 | 19.533 | 41.958 | 1.00 | 79.26 | A |
| ATOM | 2348 | OE2 | GLU | A | 497 | 10.054 | 20.413 | 39.956 | 1.00 | 79.29 | A |
| ATOM | 2349 | C | GLU | A | 497 | 6.296 | 21.283 | 40.613 | 1.00 | 73.64 | A |
| TER | 2352 | | GLU | A | 497 | | | | | | A |
| ATOM | 2353 | O5* | ADN | B | 1 | 11.263 | 53.936 | 14.307 | 1.00 | 57.85 | B |
| ATOM | 2354 | C5* | ADN | B | 1 | 12.498 | 53.959 | 13.564 | 1.00 | 50.15 | B |
| ATOM | 2355 | C4* | ADN | B | 1 | 13.696 | 53.837 | 14.578 | 1.00 | 48.75 | B |
| ATOM | 2356 | O4* | ADN | B | 1 | 13.237 | 54.849 | 15.500 | 1.00 | 43.84 | B |
| ATOM | 2357 | C3* | ADN | B | 1 | 13.537 | 52.620 | 15.496 | 1.00 | 49.12 | B |
| ATOM | 2358 | O3* | ADN | B | 1 | 14.353 | 51.562 | 14.947 | 1.00 | 48.03 | B |
| ATOM | 2359 | C2* | ADN | B | 1 | 14.017 | 53.090 | 16.881 | 1.00 | 49.28 | B |
| ATOM | 2360 | O2* | ADN | B | 1 | 15.227 | 52.489 | 17.352 | 1.00 | 53.69 | B |
| ATOM | 2361 | C1* | ADN | B | 1 | 14.031 | 54.618 | 16.644 | 1.00 | 45.46 | B |
| ATOM | 2362 | N9 | ADN | B | 1 | 13.520 | 55.504 | 17.715 | 1.00 | 42.85 | B |
| ATOM | 2363 | C8 | ADN | B | 1 | 12.192 | 56.028 | 17.777 | 1.00 | 42.41 | B |
| ATOM | 2364 | N7 | ADN | B | 1 | 11.935 | 56.777 | 18.803 | 1.00 | 41.48 | B |
| ATOM | 2365 | C5 | ADN | B | 1 | 13.134 | 56.787 | 19.501 | 1.00 | 42.09 | B |
| ATOM | 2366 | C6 | ADN | B | 1 | 13.551 | 57.421 | 20.749 | 1.00 | 40.16 | B |
| ATOM | 2367 | N6 | ADN | B | 1 | 12.716 | 58.179 | 21.464 | 1.00 | 40.62 | B |
| ATOM | 2368 | N1 | ADN | B | 1 | 14.881 | 57.219 | 21.197 | 1.00 | 40.98 | B |
| ATOM | 2369 | C2 | ADN | B | 1 | 15.771 | 56.429 | 20.469 | 1.00 | 40.21 | B |
| ATOM | 2370 | N3 | ADN | B | 1 | 15.472 | 55.782 | 19.274 | 1.00 | 41.46 | B |
| ATOM | 2371 | C4 | ADN | B | 1 | 14.143 | 55.999 | 18.850 | 1.00 | 41.81 | B |
| TER | 2372 | | ADN | B | 1 | | | | | | B |
| ATOM | 2373 | Q | HOH | W | 1 | 17.709 | 33.102 | 31.761 | 1.00 | 30.41 | W |
| ATOM | 2374 | O | HOH | W | 2 | -7.433 | 44.511 | 25.624 | 1.00 | 28.38 | W |
| ATOM | 2375 | O | HOH | W | 3 | 13.854 | 30.912 | 34.931 | 1.00 | 32.63 | W |

| | | | | | | | | | | |
|---|---|---|---|---|---|---|---|---|---|---|
| ATOM | 2376 | O | HOH | W | 4 | -0.273 | 45.256 | 16.855 | 1.00 31.59 | W |
| ATOM | 2377 | O | HOH | W | 5 | 11.821 | 40.738 | 38.742 | 1.00 36.37 | W |
| ATOM | 2378 | O | HOH | W | 6 | 13.190 | 32.675 | 36.957 | 1.00 26.52 | W |
| ATOM | 2379 | O | HOH | W | 7 | 15.995 | 35.009 | 35.279 | 1.00 29.21 | W |
| ATOM | 2380 | O | HOH | W | 8 | 23.627 | 41.598 | 30.555 | 1.00 31.94 | W |
| ATOM | 2381 | O | HOH | W | 9 | -2.294 | 42.418 | 14.584 | 1.00 37.26 | W |
| ATOM | 2382 | O | HOH | W | 10 | 16.173 | 29.283 | 44.448 | 1.00 36.62 | W |
| ATOM | 2383 | O | HOH | W | 11 | 14.718 | 23.683 | 38.453 | 1.00 38.55 | W |
| ATOM | 2384 | O | HOH | W | 12 | 19.337 | 33.695 | 29.371 | 1.00 36.96 | W |
| ATOM | 2385 | O | HOH | W | 13 | -9.804 | 38.426 | 29.099 | 1.00 33.05 | W |
| ATOM | 2386 | O | HOH | W | 14 | -9.808 | 43.297 | 24.576 | 1.00 33.41 | W |
| ATOM | 2387 | O | HOH | W | 15 | -7.729 | 45.338 | 28.140 | 1.00 35.30 | W |
| ATOM | 2388 | O | HOH | W | 16 | 1.596 | 33.209 | 13.436 | 1.00 41.02 | W |
| ATOM | 2389 | O | HOH | W | 17 | 3.622 | 58.803 | 28.275 | 1.00 40.21 | W |
| ATOM | 2390 | O | HOH | W | 18 | 25.364 | 40.766 | 37.360 | 1.00 35.11 | W |
| ATOM | 2391 | O | HOH | W | 19 | -7.371 | 47.684 | 28.885 | 1.00 33.86 | W |
| ATOM | 2392 | O | HOH | W | 20 | 8.414 | 23.038 | 39.087 | 1.00 49.71 | W |
| ATOM | 2393 | O | HOH | W | 21 | 2.900 | 44.555 | 13.785 | 1.00 44.94 | W |
| ATOM | 2394 | O | HOH | W | 22 | -2.293 | 49.712 | 32.379 | 1.00 42.52 | W |
| ATOM | 2395 | O | HOH | W | 23 | 9.016 | 30.822 | 45.534 | 1.00 42.67 | W |
| ATOM | 2396 | O | HOH | W | 24 | -8.759 | 35.816 | 12.505 | 1.00 41.54 | W |
| ATOM | 2397 | O | HOH | W | 25 | -13.178 | 40.291 | 21.830 | 1.00 34.37 | W |
| ATOM | 2398 | O | HOH | W | 26 | 2.118 | 26.067 | 13.299 | 1.00 49.94 | W |
| ATOM | 2399 | O | HOH | W | 27 | 18.361 | 39.193 | 35.510 | 1.00 34.94 | W |
| ATOM | 2400 | O | HOH | W | 28 | 16.382 | 33.207 | 42.212 | 1.00 40.88 | W |
| ATOM | 2401 | O | HOH | W | 29 | 7.482 | 40.034 | 8.705 | 1.00 45.35 | W |
| ATOM | 2402 | O | HOH | W | 30 | 14.929 | 34.609 | 37.930 | 1.00 39.24 | W |
| ATOM | 2403 | O | HOH | W | 31 | 19.169 | 51.775 | 28.626 | 1.00 47.56 | W |
| ATOM | 2404 | O | HOH | W | 32 | 15.823 | 29.844 | 20.159 | 1.00 38.89 | W |
| ATOM | 2405 | O | HOH | W | 33 | -1.697 | 27.805 | 40.898 | 1.00 44.92 | W |
| ATOM | 2406 | O | HOH | W | 34 | 13.187 | 44.754 | 33.080 | 1.00 35.95 | W |
| ATOM | 2407 | O | HOH | W | 35 | 16.145 | 34.033 | 13.750 | 1.00 43.64 | W |
| ATOM | 2408 | O | HOH | W | 36 | 6.896 | 57.860 | 29.788 | 1.00 51.48 | W |
| ATOM | 2409 | O | HOH | W | 37 | -11.197 | 35.233 | 30.309 | 1.00 47.53 | W |
| ATOM | 2410 | O | HOH | W | 38 | 14.686 | 25.790 | 29.998 | 1.00 44.32 | W |
| ATOM | 2411 | O | HOH | W | 39 | 14.818 | 48.854 | 15.680 | 1.00 49.67 | W |
| ATOM | 2412 | O | HOH | W | 40 | -5.006 | 31.703 | 11.310 | 1.00 41.05 | W |
| ATOM | 2413 | O | HOH | W | 41 | 8.330 | 49.849 | 33.748 | 1.00 38.88 | W |
| ATOM | 2414 | O | HOH | W | 42 | -12.389 | 51.589 | 26.013 | 1.00 51.86 | W |
| ATOM | 2415 | O | HOH | W | 43 | -14.281 | 45.559 | 21.180 | 1.00 42.23 | W |
| ATOM | 2416 | O | HOH | W | 44 | 9.975 | 39.027 | 41.312 | 1.00 49.64 | W |
| ATOM | 2417 | O | HOH | W | 45 | -5.000 | 44.503 | 34.723 | 1.00 44.49 | W |
| ATOM | 2418 | O | HOH | W | 46 | 11.738 | 21.764 | 31.213 | 1.00 51.93 | W |
| ATOM | 2419 | O | HOH | W | 47 | -0.175 | 50.163 | 34.288 | 1.00 54.12 | W |
| ATOM | 2420 | O | HOH | W | 48 | 19.023 | 48.759 | 21.324 | 1.00 46.40 | W |
| ATOM | 2421 | O | HOH | W | 49 | -13.297 | 40.287 | 19.109 | 1.00 53.40 | W |
| ATOM | 2422 | O | HOH | W | 50 | 6.511 | 22.926 | 24.745 | 1.00 56.27 | W |
| ATOM | 2423 | O | HOH | W | 51 | 23.700 | 34.281 | 26.228 | 1.00 43.01 | W |
| ATOM | 2424 | O | HOH | W | 52 | -3.613 | 28.881 | 19.235 | 1.00 43.53 | W |
| ATOM | 2425 | O | HOH | W | 53 | -8.040 | 32.568 | 32.275 | 1.00 40.66 | W |
| ATOM | 2426 | O | HOH | W | 54 | 22.309 | 41.478 | 33.196 | 1.00 45.75 | W |
| ATOM | 2427 | O | HOH | W | 55 | -14.120 | 37.918 | 23.299 | 1.00 41.66 | W |
| ATOM | 2428 | O | HOH | W | 56 | 3.623 | 60.062 | 26.197 | 1.00 59.43 | W |
| ATOM | 2429 | O | HOH | W | 57 | -9.342 | 41.757 | 16.021 | 1.00 44.56 | W |

| ATOM | 2430 | O | HOH W | 58 | 8.782 | 52.521 | 34.509 | 1.00 | 44.91 | W |
|------|------|---|-------|-----|--------|--------|--------|------|-------|---|
| ATOM | 2431 | O | HOH W | 59 | 5.981 | 26.995 | 12.320 | 1.00 | 56.61 | W |
| ATOM | 2432 | O | HOH W | 60 | 18.613 | 27.892 | 27.701 | 1.00 | 48.65 | W |
| ATOM | 2433 | O | HOH W | 61 | 15.834 | 59.041 | 6.239 | 1.00 | 62.23 | W |
| ATOM | 2434 | O | HOH W | 62 | -4.686 | 50.630 | 32.246 | 1.00 | 52.76 | W |
| ATOM | 2435 | O | HOH W | 63 | 17.203 | 34.454 | 39.957 | 1.00 | 40.88 | W |
| ATOM | 2436 | O | HOH W | 64 | 2.443 | 35.770 | 12.936 | 1.00 | 42.27 | W |
| ATOM | 2437 | O | HOH W | 65 | 20.341 | 53.522 | 30.264 | 1.00 | 60.14 | W |
| ATOM | 2438 | O | HOH W | 66 | 5.221 | 22.956 | 33.347 | 1.00 | 44.47 | W |
| ATOM | 2439 | O | HOH W | 67 | 10.222 | 57.229 | 11.730 | 1.00 | 50.07 | W |
| ATOM | 2440 | O | HOH W | 68 | 9.701 | 33.129 | 44.197 | 1.00 | 47.25 | W |
| ATOM | 2441 | O | HOH W | 69 | 8.686 | 30.970 | 13.168 | 1.00 | 53.78 | W |
| ATOM | 2442 | O | HOH W | 70 | 4.198 | 52.036 | 13.312 | 1.00 | 55.66 | W |
| ATOM | 2443 | O | HOH W | 71 | 30.996 | 38.997 | 23.665 | 1.00 | 48.22 | W |
| ATOM | 2444 | O | HOH W | 72 | 3.876 | 30.357 | 3.073 | 1.00 | 49.81 | W |
| ATOM | 2445 | O | HOH W | 73 | 12.684 | 34.920 | 44.571 | 1.00 | 44.72 | W |
| ATOM | 2446 | O | HOH W | 74 | 11.522 | 30.106 | 46.693 | 1.00 | 52.84 | W |
| ATOM | 2447 | O | HOH W | 75 | -10.920 | 32.546 | 7.681 | 1.00 | 64.15 | W |
| ATOM | 2448 | O | HOH W | 76 | 30.611 | 39.333 | 20.777 | 1.00 | 46.75 | W |
| ATOM | 2449 | O | HOH W | 77 | 0.955 | 37.017 | 11.073 | 1.00 | 47.95 | W |
| ATOM | 2450 | O | HOH W | 78 | -7.362 | 53.306 | 17.550 | 1.00 | 50.77 | W |
| ATOM | 2451 | O | HOH W | 79 | -3.768 | 44.577 | 36.969 | 1.00 | 44.89 | W |
| ATOM | 2452 | O | HOH W | 80 | 12.358 | 31.675 | 13.166 | 1.00 | 48.45 | W |
| ATOM | 2453 | O | HOH W | 81 | 13.611 | 41.858 | 41.195 | 1.00 | 62.67 | W |
| ATOM | 2454 | O | HOH W | 82 | 4.823 | 27.624 | 20.097 | 1.00 | 63.91 | W |
| ATOM | 2455 | O | HOH W | 83 | 3.232 | 25.851 | 16.656 | 1.00 | 58.48 | W |
| ATOM | 2456 | O | HOH W | 84 | 8.846 | 54.811 | 32.981 | 1.00 | 51.97 | W |
| ATOM | 2457 | O | HOH W | 85 | 9.744 | 42.130 | 38.862 | 1.00 | 54.57 | W |
| ATOM | 2458 | O | HOH W | 86 | 17.719 | 51.505 | 18.654 | 1.00 | 58.98 | W |
| ATOM | 2459 | O | HOH W | 87 | 10.534 | 30.332 | 10.561 | 1.00 | 59.44 | W |
| ATOM | 2460 | O | HOH W | 88 | 33.650 | 34.219 | 23.155 | 1.00 | 61.87 | W |
| ATOM | 2461 | O | HOH W | 89 | -16.638 | 38.460 | 20.062 | 1.00 | 58.38 | W |
| ATOM | 2462 | O | HOH W | 90 | 5.056 | 26.811 | 22.400 | 1.00 | 62.13 | W |
| ATOM | 2463 | O | HOH W | 91 | 17.476 | 54.009 | 18.717 | 1.00 | 58.55 | W |
| ATOM | 2464 | O | HOH W | 92 | 0.060 | 44.480 | 13.491 | 1.00 | 49.31 | W |
| ATOM | 2465 | O | HOH W | 93 | 2.357 | 20.459 | 42.895 | 1.00 | 64.88 | W |
| ATOM | 2466 | O | HOH W | 94 | 19.524 | 41.591 | 12.076 | 1.00 | 52.98 | W |
| ATOM | 2467 | O | HOH W | 95 | 8.180 | 42.571 | 24.606 | 1.00 | 44.57 | W |
| ATOM | 2468 | O | HOH W | 96 | 4.515 | 38.576 | 41.472 | 1.00 | 57.06 | W |
| ATOM | 2469 | O | HOH W | 97 | 3.860 | 68.127 | 26.485 | 1.00 | 56.50 | W |
| ATOM | 2470 | O | HOH W | 98 | 6.923 | 53.447 | 12.957 | 1.00 | 58.28 | W |
| ATOM | 2471 | O | HOH W | 99 | 15.202 | 23.120 | 26.711 | 1.00 | 54.04 | W |
| ATOM | 2472 | O | HOH W | 100 | 5.853 | 43.024 | 5.981 | 1.00 | 51.39 | W |
| ATOM | 2473 | O | HOH W | 101 | 13.059 | 19.683 | 18.151 | 1.00 | 63.97 | W |
| ATOM | 2474 | O | HOH W | 102 | 5.419 | 24.656 | 35.646 | 1.00 | 54.16 | W |
| ATOM | 2475 | O | HOH W | 103 | 9.738 | 20.221 | 25.454 | 1.00 | 59.30 | W |
| ATOM | 2476 | O | HOH W | 104 | 3.664 | 41.325 | -0.328 | 1.00 | 55.70 | W |
| ATOM | 2477 | O | HOH W | 105 | 8.634 | 44.587 | 34.933 | 1.00 | 54.14 | W |
| ATOM | 2478 | O | HOH W | 106 | 23.537 | 42.865 | 35.840 | 1.00 | 65.35 | W |
| ATOM | 2479 | O | HOH W | 107 | 5.607 | 23.054 | 27.202 | 1.00 | 64.75 | W |
| ATOM | 2480 | O | HOH W | 108 | 20.647 | 49.655 | 27.570 | 1.00 | 56.26 | W |
| ATOM | 2481 | O | HOH W | 109 | -9.740 | 48.835 | 32.327 | 1.00 | 56.76 | W |
| ATOM | 2482 | O | HOH W | 110 | 10.140 | 45.836 | 10.916 | 1.00 | 54.61 | W |
| ATOM | 2483 | O | HOH W | 111 | -11.419 | 35.698 | 26.003 | 1.00 | 54.08 | W |

| | | | | | | | | | | |
|---|---|---|---|---|---|---|---|---|---|---|
| ATOM | 2484 | O | HOH | W | 112 | 6.182 | 57.641 | 18.641 | 1.00 65.09 | W |
| ATOM | 2485 | O | HOH | W | 113 | 31.063 | 36.636 | 20.152 | 1.00 64.05 | W |
| ATOM | 2486 | O | HOH | W | 114 | 8.465 | 41.465 | 1.229 | 1.00 65.22 | W |
| ATOM | 2487 | O | HOH | W | 115 | 12.705 | 23.348 | 44.234 | 1.00 55.67 | W |
| ATOM | 2488 | O | HOH | W | 116 | 20.576 | 37.498 | 18.216 | 1.00 48.30 | W |
| ATOM | 2489 | O | HOH | W | 117 | -9.299 | 30.063 | 17.311 | 1.00 50.89 | W |
| ATOM | 2490 | O | HOH | W | 118 | 9.237 | 50.529 | 12.698 | 1.00 59.73 | W |
| ATOM | 2491 | O | HOH | W | 119 | -16.938 | 33.476 | 17.517 | 1.00 63.20 | W |
| ATOM | 2492 | O | HOH | W | 120 | 28.589 | 46.818 | 30.664 | 1.00 52.14 | W |
| ATOM | 2493 | O | HOH | W | 121 | 9.480 | 56.521 | 14.213 | 1.00 50.06 | W |
| ATOM | 2494 | O | HOH | W | 122 | -8.504 | 40.810 | 33.931 | 1.00 60.17 | W |
| ATOM | 2495 | O | HOH | W | 123 | 13.111 | 38.111 | 9.322 | 1.00 60.42 | W |
| ATOM | 2496 | O | HOH | W | 124 | 11.327 | 37.045 | 6.256 | 1.00 70.36 | W |
| ATOM | 2497 | O | HOH | W | 125 | 18.525 | 36.709 | 16.641 | 1.00 55.61 | W |
| ATOM | 2498 | O | HOH | W | 126 | 12.135 | 42.078 | 15.281 | 1.00 59.90 | W |
| ATOM | 2499 | O | HOH | W | 127 | -6.387 | 29.133 | 16.033 | 1.00 57.09 | W |
| ATOM | 2500 | O | HOH | W | 128 | 17.028 | 25.523 | 18.846 | 1.00 57.15 | W |
| ATOM | 2501 | O | HOH | W | 129 | 11.766 | 40.738 | 0.833 | 1.00 58.75 | W |
| ATOM | 2502 | O | HOH | W | 130 | 14.643 | 48.020 | 13.455 | 1.00 63.54 | W |
| ATOM | 2503 | O | HOH | W | 131 | -1.857 | 43.305 | 38.096 | 1.00 53.27 | W |
| ATOM | 2504 | O | HOH | W | 132 | 12.026 | 76.237 | 22.992 | 1.00 69.89 | W |
| ATOM | 2505 | O | HOH | W | 133 | 3.334 | 21.178 | 24.568 | 1.00 59.22 | W |
| ATOM | 2506 | O | HOH | W | 134 | 20.742 | 48.210 | 25.733 | 1.00 50.24 | W |
| ATOM | 2507 | O | HOH | W | 135 | 20.622 | 39.040 | 13.268 | 1.00 69.23 | W |
| ATOM | 2508 | O | HOH | W | 136 | 14.403 | 22.736 | 35.830 | 1.00 53.14 | W |
| ATOM | 2509 | O | HOH | W | 137 | -1.395 | 25.059 | 9.497 | 1.00 75.15 | W |
| ATOM | 2510 | O | HOH | W | 138 | 1.150 | 41.979 | 0.019 | 1.00 75.01 | W |
| ATOM | 2511 | O | HOH | W | 139 | 18.373 | 25.974 | 25.769 | 1.00 63.56 | W |
| ATOM | 2512 | O | HOH | W | 140 | 21.001 | 52.773 | 21.612 | 1.00 63.78 | W |
| ATOM | 2513 | O | HOH | W | 141 | 22.926 | 56.999 | 14.293 | 1.00 71.71 | W |
| ATOM | 2514 | O | HOH | W | 142 | 19.542 | 27.102 | 23.317 | 1.00 63.17 | W |
| ATOM | 2515 | O | HOH | W | 143 | 12.820 | 22.318 | 40.101 | 1.00 56.07 | W |
| ATOM | 2516 | O | HOH | W | 144 | 14.279 | 23.048 | 33.094 | 1.00 53.56 | W |
| ATOM | 2517 | O | HOH | W | 145 | -10.140 | 42.095 | 31.570 | 1.00 58.73 | W |
| ATOM | 2518 | O | HOH | W | 146 | 12.515 | 40.181 | 13.221 | 1.00 57.67 | W |
| ATOM | 2519 | O | HOH | W | 147 | 3.696 | 58.815 | 33.013 | 1.00 62.45 | W |
| ATOM | 2520 | O | HOH | W | 148 | 22.839 | 44.889 | 20.218 | 1.00 62.60 | W |
| ATOM | 2521 | O | HOH | W | 149 | 7.877 | 57.651 | 10.865 | 1.00 62.32 | W |
| ATOM | 2522 | O | HOH | W | 150 | 13.264 | 24.082 | 30.722 | 1.00 60.70 | W |
| ATOM | 2523 | O | HOH | W | 151 | 9.267 | 20.075 | 29.603 | 1.00 71.80 | W |
| ATOM | 2524 | O | HOH | W | 152 | -8.577 | 34.803 | 10.127 | 1.00 55.69 | W |
| ATOM | 2525 | O | HOH | W | 153 | 15.244 | 31.899 | 13.283 | 1.00 61.30 | W |
| ATOM | 2526 | O | HOH | W | 154 | -11.993 | 49.796 | 29.155 | 1.00 62.81 | W |
| ATOM | 2527 | O | HOH | W | 155 | 10.967 | 48.029 | 35.711 | 1.00 70.98 | W |
| ATOM | 2528 | O | HOH | W | 156 | -14.279 | 38.663 | 17.166 | 1.00 84.43 | W |
| ATOM | 2529 | O | HOH | W | 157 | 4.407 | 69.749 | 5.878 | 1.00 85.05 | W |
| ATOM | 2530 | O | HOH | W | 158 | 0.937 | 67.420 | 22.292 | 1.00 33.72 | W |
| ATOM | 2531 | O | HOH | W | 159 | 5.672 | 55.919 | 12.568 | 1.00 36.58 | W |
| ATOM | 2532 | O | HOH | W | 160 | 10.291 | 55.962 | 7.193 | 1.00 34.73 | W |
| ATOM | 2533 | O | HOH | W | 161 | 6.783 | 59.979 | 18.718 | 1.00 36.49 | W |
| ATOM | 2534 | O | HOH | W | 162 | -0.200 | 64.710 | 27.156 | 1.00 35.65 | W |
| ATOM | 2535 | O | HOH | W | 163 | 2.646 | 73.465 | 15.334 | 1.00 33.16 | W |
| ATOM | 2536 | O | HOH | W | 164 | 13.952 | 64.898 | 29.746 | 1.00 35.63 | W |
| ATOM | 2537 | O | HOH | W | 165 | 14.152 | 46.249 | 11.710 | 1.00 33.90 | W |

| ATOM | 2538 | O | HOH W 166 | 21.442 | 40.535 | 10.347 | 1.00 | 36.03 | W |
|------|------|---|-----------|--------|--------|--------|------|-------|---|
| ATOM | 2539 | O | HOH W 167 | 21.685 | 42.439 | 7.655 | 1.00 | 33.23 | W |
| ATOM | 2540 | O | HOH W 168 | 30.818 | 46.839 | 18.032 | 1.00 | 33.90 | W |
| ATOM | 2541 | O | HOH W 169 | 27.421 | 39.072 | 20.637 | 1.00 | 32.03 | W |
| ATOM | 2542 | O | HOH W 170 | 28.928 | 48.243 | 21.337 | 1.00 | 33.41 | W |
| ATOM | 2543 | O | HOH W 171 | 24.392 | 46.257 | 26.371 | 1.00 | 33.63 | W |
| ATOM | 2544 | O | HOH W 172 | 27.320 | 38.077 | 28.232 | 1.00 | 34.04 | W |
| ATOM | 2545 | O | HOH W 173 | 17.008 | 32.606 | 34.817 | 1.00 | 34.60 | W |
| ATOM | 2546 | O | HOH W 174 | 7.674 | 40.154 | 41.597 | 1.00 | 33.28 | W |
| ATOM | 2547 | O | HOH W 175 | 5.528 | 43.436 | 38.812 | 1.00 | 33.85 | W |
| ATOM | 2548 | O | HOH W 176 | 7.847 | 47.179 | 38.784 | 1.00 | 33.42 | W |
| ATOM | 2549 | O | HOH W 177 | 4.762 | 40.989 | 39.662 | 1.00 | 33.88 | W |
| ATOM | 2550 | O | HOH W 178 | 13.238 | 47.290 | 36.960 | 1.00 | 34.43 | W |
| ATOM | 2551 | O | HOH W 179 | 8.404 | 43.322 | 36.889 | 1.00 | 34.64 | W |
| ATOM | 2552 | O | HOH W 180 | 6.698 | 48.398 | 35.124 | 1.00 | 33.21 | W |
| ATOM | 2553 | O | HOH W 181 | -4.226 | 49.925 | 35.939 | 1.00 | 35.29 | W |
| ATOM | 2554 | O | HOH W 182 | 1.953 | 50.698 | 35.258 | 1.00 | 34.94 | W |
| ATOM | 2555 | O | HOH W 183 | -9.974 | 47.651 | 29.513 | 1.00 | 34.62 | W |
| ATOM | 2556 | O | HOH W 184 | -8.422 | 60.657 | 26.256 | 1.00 | 34.37 | W |
| ATOM | 2557 | O | HOH W 185 | 10.349 | 38.565 | 11.399 | 1.00 | 36.63 | W |
| ATOM | 2558 | O | HOH W 186 | 11.062 | 48.386 | 11.357 | 1.00 | 33.85 | W |
| ATOM | 2559 | O | HOH W 187 | 13.867 | 51.085 | 12.547 | 1.00 | 33.13 | W |
| ATOM | 2560 | O | HOH W 188 | 18.882 | 50.611 | 35.726 | 1.00 | 33.90 | W |
| ATOM | 2561 | O | HOH W 189 | 21.299 | 51.608 | 34.682 | 1.00 | 32.51 | W |
| ATOM | 2562 | O | HOH W 190 | 12.885 | 51.483 | 36.855 | 1.00 | 35.05 | W |
| ATOM | 2563 | O | HOH W 191 | 14.906 | 49.954 | 36.845 | 1.00 | 35.05 | W |
| ATOM | 2564 | O | HOH W 192 | 7.445 | 40.742 | 5.823 | 1.00 | 34.19 | W |
| ATOM | 2565 | O | HOH W 193 | 12.913 | 40.392 | 6.165 | 1.00 | 34.87 | W |
| ATOM | 2566 | O | HOH W 194 | 8.432 | 46.731 | 7.735 | 1.00 | 33.72 | W |
| ATOM | 2567 | O | HOH W 195 | 2.299 | 44.066 | 2.714 | 1.00 | 35.00 | W |
| ATOM | 2568 | O | HOH W 196 | -6.920 | 32.395 | 9.802 | 1.00 | 34.26 | W |
| ATOM | 2569 | O | HOH W 197 | -9.157 | 47.238 | 7.475 | 1.00 | 34.22 | W |
| ATOM | 2570 | O | HOH W 198 | 12.459 | 42.138 | -2.551 | 1.00 | 33.72 | W |
| ATOM | 2571 | O | HOH W 199 | 4.878 | 26.047 | 2.253 | 1.00 | 35.75 | W |
| ATOM | 2572 | O | HOH W 200 | 3.528 | 23.824 | 11.451 | 1.00 | 36.33 | W |
| ATOM | 2573 | O | HOH W 201 | -3.537 | 29.132 | 16.194 | 1.00 | 32.94 | W |
| ATOM | 2574 | O | HOH W 202 | 7.911 | 28.530 | 11.730 | 1.00 | 34.43 | W |
| ATOM | 2575 | O | HOH W 203 | 5.365 | 24.290 | 15.777 | 1.00 | 33.67 | W |
| ATOM | 2576 | O | HOH W 204 | 6.993 | 24.747 | 13.884 | 1.00 | 36.62 | W |
| ATOM | 2577 | O | HOH W 205 | 12.954 | 18.885 | 31.610 | 1.00 | 35.03 | W |
| ATOM | 2578 | O | HOH W 206 | 6.168 | 22.766 | 37.103 | 1.00 | 34.58 | W |
| ATOM | 2579 | O | HOH W 207 | 3.340 | 24.918 | 36.436 | 1.00 | 33.06 | W |
| ATOM | 2580 | O | HOH W 208 | 7.664 | 20.852 | 26.749 | 1.00 | 35.14 | W |
| ATOM | 2581 | O | HOH W 209 | 3.208 | 21.618 | 33.885 | 1.00 | 32.39 | W |
| ATOM | 2582 | O | HOH W 210 | -6.388 | 29.043 | 21.992 | 1.00 | 33.51 | W |
| ATOM | 2583 | O | HOH W 211 | -8.259 | 27.471 | 13.979 | 1.00 | 33.83 | W |
| ATOM | 2584 | O | HOH W 212 | -15.180 | 35.884 | 16.800 | 1.00 | 32.67 | W |
| ATOM | 2585 | O | HOH W 213 | -11.148 | 30.220 | 25.248 | 1.00 | 32.65 | W |
| ATOM | 2586 | O | HOH W 214 | -2.248 | 39.233 | 43.463 | 1.00 | 34.30 | W |
| ATOM | 2587 | O | HOH W 215 | -4.831 | 39.456 | 43.741 | 1.00 | 34.16 | W |
| ATOM | 2588 | O | HOH W 216 | -6.726 | 41.180 | 44.628 | 1.00 | 33.64 | W |
| ATOM | 2589 | O | HOH W 217 | 21.552 | 47.888 | 39.008 | 1.00 | 32.41 | W |
| ATOM | 2590 | O | HOH W 218 | 22.546 | 49.623 | 43.029 | 1.00 | 34.76 | W |
| ATOM | 2591 | O | HOH W 219 | 7.867 | 24.204 | 50.140 | 1.00 | 35.68 | W |

```
ATOM   2592  O    HOH W 220        6.935  20.800  50.471  1.00 27.36        W
ATOM   2593  O    HOH W 221        5.988  17.233  42.462  1.00 26.71        W
ATOM   2594  O    HOH W 222       11.539  50.722  12.082  1.00 20.87        W
ATOM   2595  O    HOH W 223       15.479  52.221  11.330  1.00 20.87        W
ATOM   2596  O    HOH W 224       -1.373  56.557  10.796  1.00 20.87        W
ATOM   2597  O    HOH W 225       -5.511  53.267  15.801  1.00 20.87        W
ATOM   2598  O    HOH W 226       -6.744  58.854  18.789  1.00 20.87        W
ATOM   2599  O    HOH W 227       16.612  26.628  28.568  1.00 20.87        W
ATOM   2600  O    HOH W 228       18.005  27.976  19.373  1.00 20.86        W
ATOM   2601  O    HOH W 229       16.727  29.882  34.872  1.00 20.86        W
END
```

Figure 2: VRT complex coordinates

| ATOM | 1 | CB | SER | A | 27 | 33.157 | 24.746 | 68.703 | 1.00 | 78.21 | A |
|------|-----|------|-----|---|----|--------|--------|--------|------|-------|---|
| ATOM | 2 | OG | SER | A | 27 | 32.800 | 25.935 | 68.014 | 1.00 | 78.89 | A |
| ATOM | 3 | C | SER | A | 27 | 30.731 | 24.262 | 68.846 | 1.00 | 77.90 | A |
| ATOM | 4 | O | SER | A | 27 | 30.540 | 25.444 | 68.661 | 1.00 | 78.14 | A |
| ATOM | 5 | N | SER | A | 27 | 32.165 | 23.141 | 67.121 | 1.00 | 78.00 | A |
| ATOM | 6 | CA | SER | A | 27 | 32.098 | 23.666 | 68.512 | 1.00 | 78.04 | A |
| ATOM | 7 | N | LEU | A | 28 | 29.795 | 23.441 | 69.332 | 1.00 | 78.01 | A |
| ATOM | 8 | CA | LEU | A | 28 | 28.471 | 23.946 | 69.703 | 1.00 | 78.28 | A |
| ATOM | 9 | CB | LEU | A | 28 | 27.914 | 24.892 | 68.586 | 1.00 | 78.63 | A |
| ATOM | 10 | CG | LEU | A | 28 | 28.088 | 26.439 | 68.772 | 1.00 | 79.17 | A |
| ATOM | 11 | CD1 | LEU | A | 28 | 26.753 | 27.131 | 68.547 | 1.00 | 78.78 | A |
| ATOM | 12 | CD2 | LEU | A | 28 | 28.641 | 26.763 | 70.164 | 1.00 | 78.48 | A |
| ATOM | 13 | C | LEU | A | 28 | 27.335 | 23.008 | 70.121 | 1.00 | 78.20 | A |
| ATOM | 14 | O | LEU | A | 28 | 27.242 | 21.852 | 69.715 | 1.00 | 78.03 | A |
| ATOM | 15 | N | HIS | A | 29 | 26.479 | 23.605 | 70.947 | 1.00 | 78.37 | A |
| ATOM | 16 | CA | HIS | A | 29 | 25.241 | 23.090 | 71.537 | 1.00 | 78.69 | A |
| ATOM | 17 | CB | HIS | A | 29 | 24.098 | 23.775 | 70.847 | 1.00 | 78.68 | A |
| ATOM | 18 | CG | HIS | A | 29 | 23.098 | 24.366 | 71.785 | 1.00 | 78.50 | A |
| ATOM | 19 | CD2 | HIS | A | 29 | 22.930 | 25.635 | 72.225 | 1.00 | 77.41 | A |
| ATOM | 20 | ND1 | HIS | A | 29 | 22.122 | 23.614 | 72.400 | 1.00 | 78.10 | A |
| ATOM | 21 | CE1 | HIS | A | 29 | 21.393 | 24.395 | 73.178 | 1.00 | 77.38 | A |
| ATOM | 22 | NE2 | HIS | A | 29 | 21.863 | 25.626 | 73.091 | 1.00 | 76.50 | A |
| ATOM | 23 | C | HIS | A | 29 | 24.863 | 21.626 | 71.715 | 1.00 | 78.97 | A |
| ATOM | 24 | O | HIS | A | 29 | 24.832 | 20.859 | 70.768 | 1.00 | 79.36 | A |
| ATOM | 25 | N | MET | A | 30 | 24.531 | 21.277 | 72.958 | 1.00 | 79.32 | A |
| ATOM | 26 | CA | MET | A | 30 | 24.090 | 19.929 | 73.307 | 1.00 | 79.67 | A |
| ATOM | 27 | CB | MET | A | 30 | 24.750 | 19.434 | 74.604 | 1.00 | 79.71 | A |
| ATOM | 28 | CG | MET | A | 30 | 26.259 | 19.442 | 74.572 | 1.00 | 79.99 | A |
| ATOM | 29 | SD | MET | A | 30 | 26.892 | 18.589 | 73.121 | 1.00 | 79.99 | A |
| ATOM | 30 | CE | MET | A | 30 | 26.857 | 16.909 | 73.690 | 1.00 | 81.09 | A |
| ATOM | 31 | C | MET | A | 30 | 22.594 | 20.057 | 73.527 | 1.00 | 80.34 | A |
| ATOM | 32 | O | MET | A | 30 | 22.099 | 21.142 | 73.756 | 1.00 | 80.86 | A |
| ATOM | 33 | N | ILE | A | 31 | 21.879 | 18.943 | 73.463 | 1.00 | 80.75 | A |
| ATOM | 34 | CA | ILE | A | 31 | 20.436 | 18.957 | 73.652 | 1.00 | 80.63 | A |
| ATOM | 35 | CB | ILE | A | 31 | 19.705 | 19.270 | 72.334 | 1.00 | 80.66 | A |
| ATOM | 36 | CG2 | ILE | A | 31 | 18.256 | 18.810 | 72.426 | 1.00 | 80.87 | A |
| ATOM | 37 | CG1 | ILE | A | 31 | 19.797 | 20.773 | 72.020 | 1.00 | 80.42 | A |
| ATOM | 38 | CD1 | ILE | A | 31 | 19.348 | 21.139 | 70.622 | 1.00 | 80.43 | A |
| ATOM | 39 | C | ILE | A | 31 | 19.987 | 17.598 | 74.133 | 1.00 | 80.68 | A |
| ATOM | 40 | O | ILE | A | 31 | 20.488 | 16.602 | 73.686 | 1.00 | 80.38 | A |
| ATOM | 41 | N | ASP | A | 32 | 19.034 | 17.567 | 75.052 | 1.00 | 80.96 | A |
| ATOM | 42 | CA | ASP | A | 32 | 18.554 | 16.284 | 75.542 | 1.00 | 80.95 | A |
| ATOM | 43 | CB | ASP | A | 32 | 18.289 | 16.338 | 77.064 | 1.00 | 81.38 | A |
| ATOM | 44 | CG | ASP | A | 32 | 17.397 | 17.492 | 77.461 | 1.00 | 82.37 | A |
| ATOM | 45 | OD1 | ASP | A | 32 | 17.437 | 18.538 | 76.765 | 1.00 | 83.73 | A |
| ATOM | 46 | OD2 | ASP | A | 32 | 16.673 | 17.352 | 78.477 | 1.00 | 83.68 | A |
| ATOM | 47 | C | ASP | A | 32 | 17.307 | 15.859 | 74.782 | 1.00 | 80.53 | A |
| ATOM | 48 | O | ASP | A | 32 | 16.355 | 16.628 | 74.616 | 1.00 | 80.09 | A |
| ATOM | 49 | N | TYR | A | 33 | 17.349 | 14.620 | 74.311 | 1.00 | 80.13 | A |
| ATOM | 50 | CA | TYR | A | 33 | 16.282 | 14.016 | 73.532 | 1.00 | 79.95 | A |
| ATOM | 51 | CB | TYR | A | 33 | 16.549 | 12.512 | 73.412 | 1.00 | 80.16 | A |

| ATOM | 52 | CG | TYR | A | 33 | 15.850 | 11.845 | 72.257 | 1.00 | 80.46 | A |
|------|-----|-----|-----|---|----|--------|--------|--------|------|-------|---|
| ATOM | 53 | CD1 | TYR | A | 33 | 15.979 | 12.335 | 70.963 | 1.00 | 81.11 | A |
| ATOM | 54 | CE1 | TYR | A | 33 | 15.332 | 11.719 | 69.891 | 1.00 | 80.40 | A |
| ATOM | 55 | CD2 | TYR | A | 33 | 15.059 | 10.719 | 72.459 | 1.00 | 80.60 | A |
| ATOM | 56 | CE2 | TYR | A | 33 | 14.406 | 10.094 | 71.395 | 1.00 | 80.74 | A |
| ATOM | 57 | CZ | TYR | A | 33 | 14.545 | 10.599 | 70.114 | 1.00 | 80.87 | A |
| ATOM | 58 | OH | TYR | A | 33 | 13.891 | 9.988 | 69.067 | 1.00 | 81.39 | A |
| ATOM | 59 | C | TYR | A | 33 | 14.884 | 14.254 | 74.096 | 1.00 | 79.67 | A |
| ATOM | 60 | O | TYR | A | 33 | 13.955 | 14.557 | 73.361 | 1.00 | 79.27 | A |
| ATOM | 61 | N | LYS | A | 34 | 14.747 | 14.118 | 75.409 | 1.00 | 79.23 | A |
| ATOM | 62 | CA | LYS | A | 34 | 13.457 | 14.293 | 76.068 | 1.00 | 78.95 | A |
| ATOM | 63 | CB | LYS | A | 34 | 13.589 | 13.924 | 77.566 | 1.00 | 79.09 | A |
| ATOM | 64 | CG | LYS | A | 34 | 14.716 | 14.659 | 78.263 | 1.00 | 80.34 | A |
| ATOM | 65 | CD | LYS | A | 34 | 15.099 | 13.994 | 79.573 | 1.00 | 80.53 | A |
| ATOM | 66 | CE | LYS | A | 34 | 16.299 | 14.702 | 80.203 | 1.00 | 81.41 | A |
| ATOM | 67 | NZ | LYS | A | 34 | 16.835 | 13.965 | 81.387 | 1.00 | 81.45 | A |
| ATOM | 68 | C | LYS | A | 34 | 12.869 | 15.700 | 75.916 | 1.00 | 78.07 | A |
| ATOM | 69 | O | LYS | A | 34 | 11.780 | 15.975 | 76.424 | 1.00 | 77.80 | A |
| ATOM | 70 | N | GLU | A | 35 | 13.577 | 16.586 | 75.217 | 1.00 | 77.40 | A |
| ATOM | 71 | CA | GLU | A | 35 | 13.086 | 17.949 | 75.016 | 1.00 | 77.35 | A |
| ATOM | 72 | CB | GLU | A | 35 | 14.194 | 18.979 | 75.344 | 1.00 | 77.69 | A |
| ATOM | 73 | CG | GLU | A | 35 | 14.613 | 19.020 | 76.810 | 1.00 | 80.02 | A |
| ATOM | 74 | CD | GLU | A | 35 | 13.437 | 19.165 | 77.768 | 1.00 | 83.34 | A |
| ATOM | 75 | OE1 | GLU | A | 35 | 12.647 | 20.126 | 77.611 | 1.00 | 84.14 | A |
| ATOM | 76 | OE2 | GLU | A | 35 | 13.313 | 18.317 | 78.685 | 1.00 | 85.16 | A |
| ATOM | 77 | C | GLU | A | 35 | 12.597 | 18.165 | 73.583 | 1.00 | 76.51 | A |
| ATOM | 78 | O | GLU | A | 35 | 12.248 | 19.282 | 73.194 | 1.00 | 75.86 | A |
| ATOM | 79 | N | ILE | A | 36 | 12.558 | 17.081 | 72.810 | 1.00 | 75.66 | A |
| ATOM | 80 | CA | ILE | A | 36 | 12.142 | 17.130 | 71.407 | 1.00 | 75.28 | A |
| ATOM | 81 | CB | ILE | A | 36 | 13.315 | 16.745 | 70.474 | 1.00 | 75.29 | A |
| ATOM | 82 | CG2 | ILE | A | 36 | 12.861 | 16.816 | 69.021 | 1.00 | 75.56 | A |
| ATOM | 83 | CG1 | ILE | A | 36 | 14.531 | 17.672 | 70.719 | 1.00 | 75.28 | A |
| ATOM | 84 | CD1 | ILE | A | 36 | 15.870 | 17.024 | 70.388 | 1.00 | 75.06 | A |
| ATOM | 85 | C | ILE | A | 36 | 10.988 | 16.186 | 71.078 | 1.00 | 74.74 | A |
| ATOM | 86 | O | ILE | A | 36 | 11.081 | 14.990 | 71.337 | 1.00 | 74.42 | A |
| ATOM | 87 | N | GLU | A | 37 | 9.918 | 16.727 | 70.490 | 1.00 | 74.08 | A |
| ATOM | 88 | CA | GLU | A | 37 | 8.758 | 15.913 | 70.105 | 1.00 | 73.62 | A |
| ATOM | 89 | CB | GLU | A | 37 | 7.419 | 16.709 | 70.243 | 1.00 | 73.73 | A |
| ATOM | 90 | CG | GLU | A | 37 | 6.184 | 15.800 | 70.421 | 1.00 | 74.93 | A |
| ATOM | 91 | CD | GLU | A | 37 | 4.966 | 16.236 | 69.594 | 1.00 | 76.12 | A |
| ATOM | 92 | OE1 | GLU | A | 37 | 4.620 | 17.435 | 69.612 | 1.00 | 79.89 | A |
| ATOM | 93 | OE2 | GLU | A | 37 | 4.342 | 15.373 | 68.934 | 1.00 | 77.69 | A |
| ATOM | 94 | C | GLU | A | 37 | 8.889 | 15.437 | 68.664 | 1.00 | 72.43 | A |
| ATOM | 95 | O | GLU | A | 37 | 8.629 | 16.188 | 67.734 | 1.00 | 72.54 | A |
| ATOM | 96 | N | VAL | A | 38 | 9.292 | 14.184 | 68.486 | 1.00 | 71.16 | A |
| ATOM | 97 | CA | VAL | A | 38 | 9.449 | 13.618 | 67.153 | 1.00 | 70.49 | A |
| ATOM | 98 | CB | VAL | A | 38 | 10.104 | 12.223 | 67.205 | 1.00 | 70.36 | A |
| ATOM | 99 | CG1 | VAL | A | 38 | 10.159 | 11.620 | 65.810 | 1.00 | 70.29 | A |
| ATOM | 100 | CG2 | VAL | A | 38 | 11.505 | 12.328 | 67.776 | 1.00 | 70.58 | A |
| ATOM | 101 | C | VAL | A | 38 | 8.105 | 13.494 | 66.454 | 1.00 | 69.92 | A |
| ATOM | 102 | O | VAL | A | 38 | 7.104 | 13.136 | 67.058 | 1.00 | 70.50 | A |
| ATOM | 103 | N | GLU | A | 39 | 8.091 | 13.799 | 65.166 | 1.00 | 68.73 | A |
| ATOM | 104 | CA | GLU | A | 39 | 6.865 | 13.713 | 64.389 | 1.00 | 67.84 | A |
| ATOM | 105 | CB | GLU | A | 39 | 6.356 | 15.151 | 64.066 | 1.00 | 67.85 | A |

| ATOM | 106 | CG | GLU | A | 39 | 5.557 | 15.780 | 65.226 | 1.00 | 69.32 | A |
|------|-----|-----|-----|---|-----|--------|--------|--------|------|-------|---|
| ATOM | 107 | CD | GLU | A | 39 | 5.622 | 17.304 | 65.255 | 1.00 | 70.45 | A |
| ATOM | 108 | OE1 | GLU | A | 39 | 5.375 | 17.926 | 64.201 | 1.00 | 74.90 | A |
| ATOM | 109 | OE2 | GLU | A | 39 | 5.911 | 17.881 | 66.331 | 1.00 | 74.17 | A |
| ATOM | 110 | C | GLU | A | 39 | 7.135 | 12.893 | 63.126 | 1.00 | 66.45 | A |
| ATOM | 111 | O | GLU | A | 39 | 8.176 | 12.256 | 63.016 | 1.00 | 66.44 | A |
| ATOM | 112 | N | GLU | A | 40 | 6.200 | 12.894 | 62.184 | 1.00 | 64.80 | A |
| ATOM | 113 | CA | GLU | A | 40 | 6.367 | 12.117 | 60.959 | 1.00 | 64.07 | A |
| ATOM | 114 | CB | GLU | A | 40 | 5.386 | 12.603 | 59.866 | 1.00 | 64.51 | A |
| ATOM | 115 | CG | GLU | A | 40 | 5.794 | 13.915 | 59.200 | 1.00 | 66.52 | A |
| ATOM | 116 | CD | GLU | A | 40 | 5.220 | 15.114 | 59.915 | 1.00 | 69.27 | A |
| ATOM | 117 | OE1 | GLU | A | 40 | 5.209 | 15.114 | 61.165 | 1.00 | 70.86 | A |
| ATOM | 118 | OE2 | GLU | A | 40 | 4.785 | 16.061 | 59.230 | 1.00 | 70.72 | A |
| ATOM | 119 | C | GLU | A | 40 | 7.778 | 12.148 | 60.377 | 1.00 | 62.48 | A |
| ATOM | 120 | O | GLU | A | 40 | 8.488 | 13.152 | 60.485 | 1.00 | 61.49 | A |
| ATOM | 121 | N | VAL | A | 41 | 8.186 | 11.040 | 59.762 | 1.00 | 61.10 | A |
| ATOM | 122 | CA | VAL | A | 41 | 9.498 | 10.981 | 59.119 | 1.00 | 60.46 | A |
| ATOM | 123 | CB | VAL | A | 41 | 9.911 | 9.531 | 58.733 | 1.00 | 60.07 | A |
| ATOM | 124 | CG1 | VAL | A | 41 | 11.061 | 9.558 | 57.709 | 1.00 | 59.92 | A |
| ATOM | 125 | CG2 | VAL | A | 41 | 10.365 | 8.767 | 59.980 | 1.00 | 60.45 | A |
| ATOM | 126 | C | VAL | A | 41 | 9.360 | 11.811 | 57.848 | 1.00 | 60.02 | A |
| ATOM | 127 | O | VAL | A | 41 | 8.278 | 11.890 | 57.258 | 1.00 | 60.28 | A |
| ATOM | 128 | N | VAL | A | 42 | 10.451 | 12.433 | 57.427 | 1.00 | 59.15 | A |
| ATOM | 129 | CA | VAL | A | 42 | 10.406 | 13.266 | 56.239 | 1.00 | 58.12 | A |
| ATOM | 130 | CB | VAL | A | 42 | 10.628 | 14.765 | 56.615 | 1.00 | 57.36 | A |
| ATOM | 131 | CG1 | VAL | A | 42 | 12.075 | 15.197 | 56.324 | 1.00 | 57.11 | A |
| ATOM | 132 | CG2 | VAL | A | 42 | 9.613 | 15.639 | 55.882 | 1.00 | 58.18 | A |
| ATOM | 133 | C | VAL | A | 42 | 11.427 | 12.819 | 55.197 | 1.00 | 57.52 | A |
| ATOM | 134 | O | VAL | A | 42 | 11.756 | 13.570 | 54.269 | 1.00 | 57.84 | A |
| ATOM | 135 | N | GLY | A | 43 | 11.926 | 11.597 | 55.346 | 1.00 | 56.94 | A |
| ATOM | 136 | CA | GLY | A | 43 | 12.890 | 11.107 | 54.386 | 1.00 | 57.03 | A |
| ATOM | 137 | C | GLY | A | 43 | 14.161 | 10.546 | 54.984 | 1.00 | 56.73 | A |
| ATOM | 138 | O | GLY | A | 43 | 14.564 | 10.906 | 56.094 | 1.00 | 55.63 | A |
| ATOM | 139 | N | ARG | A | 44 | 14.802 | 9.666 | 54.220 | 1.00 | 57.02 | A |
| ATOM | 140 | CA | ARG | A | 44 | 16.038 | 9.006 | 54.637 | 1.00 | 58.19 | A |
| ATOM | 141 | CB | ARG | A | 44 | 15.800 | 7.480 | 54.702 | 1.00 | 58.34 | A |
| ATOM | 142 | CG | ARG | A | 44 | 14.578 | 7.100 | 55.536 | 1.00 | 61.18 | A |
| ATOM | 143 | CD | ARG | A | 44 | 13.991 | 5.757 | 55.124 | 1.00 | 63.72 | A |
| ATOM | 144 | NE | ARG | A | 44 | 13.504 | 5.748 | 53.741 | 1.00 | 68.16 | A |
| ATOM | 145 | CZ | ARG | A | 44 | 12.574 | 6.572 | 53.256 | 1.00 | 69.58 | A |
| ATOM | 146 | NH1 | ARG | A | 44 | 12.019 | 7.490 | 54.037 | 1.00 | 70.58 | A |
| ATOM | 147 | NH2 | ARG | A | 44 | 12.184 | 6.467 | 51.987 | 1.00 | 70.08 | A |
| ATOM | 148 | C | ARG | A | 44 | 17.173 | 9.328 | 53.665 | 1.00 | 57.38 | A |
| ATOM | 149 | O | ARG | A | 44 | 17.011 | 9.232 | 52.452 | 1.00 | 57.07 | A |
| ATOM | 150 | N | GLY | A | 45 | 18.324 | 9.703 | 54.213 | 1.00 | 57.11 | A |
| ATOM | 151 | CA | GLY | A | 45 | 19.460 | 10.054 | 53.380 | 1.00 | 56.92 | A |
| ATOM | 152 | C | GLY | A | 45 | 20.537 | 9.004 | 53.221 | 1.00 | 56.56 | A |
| ATOM | 153 | O | GLY | A | 45 | 20.228 | 7.806 | 53.204 | 1.00 | 56.33 | A |
| ATOM | 154 | N | ALA | A | 46 | 21.789 | 9.458 | 53.085 | 1.00 | 55.79 | A |
| ATOM | 155 | CA | ALA | A | 46 | 22.934 | 8.563 | 52.942 | 1.00 | 55.79 | A |
| ATOM | 156 | CB | ALA | A | 46 | 24.210 | 9.320 | 53.059 | 1.00 | 55.77 | A |
| ATOM | 157 | C | ALA | A | 46 | 22.835 | 7.527 | 54.050 | 1.00 | 55.78 | A |
| ATOM | 158 | O | ALA | A | 46 | 22.914 | 6.323 | 53.775 | 1.00 | 56.30 | A |
| ATOM | 159 | N | PHE | A | 47 | 22.664 | 7.981 | 55.299 | 1.00 | 55.68 | A |

| ATOM | 160 | CA | PHE | A | 47 | 22.497 | 7.040 | 56.409 | 1.00 | 54.98 | A |
|------|-----|-----|-----|---|----|--------|--------|--------|------|-------|---|
| ATOM | 161 | CB | PHE | A | 47 | 23.769 | 7.010 | 57.366 | 1.00 | 55.25 | A |
| ATOM | 162 | CG | PHE | A | 47 | 24.462 | 8.333 | 57.550 | 1.00 | 55.69 | A |
| ATOM | 163 | CD1 | PHE | A | 47 | 23.976 | 9.277 | 58.456 | 1.00 | 55.83 | A |
| ATOM | 164 | CD2 | PHE | A | 47 | 25.657 | 8.598 | 56.882 | 1.00 | 56.39 | A |
| ATOM | 165 | CE1 | PHE | A | 47 | 24.683 | 10.469 | 58.703 | 1.00 | 55.57 | A |
| ATOM | 166 | CE2 | PHE | A | 47 | 26.371 | 9.783 | 57.118 | 1.00 | 56.05 | A |
| ATOM | 167 | CZ | PHE | A | 47 | 25.884 | 10.718 | 58.030 | 1.00 | 55.10 | A |
| ATOM | 168 | C | PHE | A | 47 | 21.205 | 7.202 | 57.230 | 1.00 | 54.66 | A |
| ATOM | 169 | O | PHE | A | 47 | 20.163 | 6.661 | 56.846 | 1.00 | 56.26 | A |
| ATOM | 170 | N | GLY | A | 48 | 21.267 | 7.940 | 58.338 | 1.00 | 53.18 | A |
| ATOM | 171 | CA | GLY | A | 48 | 20.109 | 8.129 | 59.205 | 1.00 | 51.65 | A |
| ATOM | 172 | C | GLY | A | 48 | 18.717 | 8.341 | 58.622 | 1.00 | 50.37 | A |
| ATOM | 173 | O | GLY | A | 48 | 18.443 | 8.063 | 57.455 | 1.00 | 49.38 | A |
| ATOM | 174 | N | VAL | A | 49 | 17.817 | 8.822 | 59.470 | 1.00 | 48.90 | A |
| ATOM | 175 | CA | VAL | A | 49 | 16.446 | 9.097 | 59.064 | 1.00 | 48.64 | A |
| ATOM | 176 | CB | VAL | A | 49 | 15.445 | 8.104 | 59.711 | 1.00 | 49.11 | A |
| ATOM | 177 | CG1 | VAL | A | 49 | 15.665 | 8.044 | 61.233 | 1.00 | 48.89 | A |
| ATOM | 178 | CG2 | VAL | A | 49 | 13.983 | 8.546 | 59.397 | 1.00 | 49.10 | A |
| ATOM | 179 | C | VAL | A | 49 | 16.112 | 10.508 | 59.533 | 1.00 | 47.74 | A |
| ATOM | 180 | O | VAL | A | 49 | 16.323 | 10.834 | 60.699 | 1.00 | 47.60 | A |
| ATOM | 181 | N | VAL | A | 50 | 15.603 | 11.350 | 58.636 | 1.00 | 45.85 | A |
| ATOM | 182 | CA | VAL | A | 50 | 15.260 | 12.714 | 59.015 | 1.00 | 45.48 | A |
| ATOM | 183 | CB | VAL | A | 50 | 15.709 | 13.700 | 57.949 | 1.00 | 44.11 | A |
| ATOM | 184 | CG1 | VAL | A | 50 | 15.372 | 15.131 | 58.389 | 1.00 | 41.69 | A |
| ATOM | 185 | CG2 | VAL | A | 50 | 17.214 | 13.542 | 57.716 | 1.00 | 42.65 | A |
| ATOM | 186 | C | VAL | A | 50 | 13.770 | 12.838 | 59.210 | 1.00 | 44.98 | A |
| ATOM | 187 | O | VAL | A | 50 | 13.014 | 12.487 | 58.349 | 1.00 | 44.41 | A |
| ATOM | 188 | N | CYS | A | 51 | 13.361 | 13.349 | 60.359 | 1.00 | 46.27 | A |
| ATOM | 189 | CA | CYS | A | 51 | 11.943 | 13.475 | 60.640 | 1.00 | 48.33 | A |
| ATOM | 190 | CB | CYS | A | 51 | 11.551 | 12.494 | 61.734 | 1.00 | 48.80 | A |
| ATOM | 191 | SG | CYS | A | 51 | 12.897 | 11.358 | 62.177 | 1.00 | 55.03 | A |
| ATOM | 192 | C | CYS | A | 51 | 11.623 | 14.880 | 61.077 | 1.00 | 49.61 | A |
| ATOM | 193 | O | CYS | A | 51 | 12.501 | 15.604 | 61.516 | 1.00 | 48.67 | A |
| ATOM | 194 | N | LYS | A | 52 | 10.359 | 15.261 | 60.939 | 1.00 | 52.17 | A |
| ATOM | 195 | CA | LYS | A | 52 | 9.912 | 16.585 | 61.346 | 1.00 | 55.16 | A |
| ATOM | 196 | CB | LYS | A | 52 | 8.586 | 16.932 | 60.609 | 1.00 | 55.16 | A |
| ATOM | 197 | CG | LYS | A | 52 | 7.848 | 18.175 | 61.108 | 1.00 | 57.01 | A |
| ATOM | 198 | CD | LYS | A | 52 | 8.642 | 19.450 | 60.900 | 1.00 | 58.60 | A |
| ATOM | 199 | CE | LYS | A | 52 | 7.807 | 20.677 | 61.255 | 1.00 | 59.37 | A |
| ATOM | 200 | NZ | LYS | A | 52 | 6.624 | 20.842 | 60.364 | 1.00 | 60.92 | A |
| ATOM | 201 | C | LYS | A | 52 | 9.716 | 16.521 | 62.863 | 1.00 | 56.10 | A |
| ATOM | 202 | O | LYS | A | 52 | 9.293 | 15.502 | 63.388 | 1.00 | 55.68 | A |
| ATOM | 203 | N | ALA | A | 53 | 10.039 | 17.601 | 63.564 | 1.00 | 58.18 | A |
| ATOM | 204 | CA | ALA | A | 53 | 9.895 | 17.612 | 65.015 | 1.00 | 60.49 | A |
| ATOM | 205 | CB | ALA | A | 53 | 11.110 | 16.928 | 65.654 | 1.00 | 60.14 | A |
| ATOM | 206 | C | ALA | A | 53 | 9.715 | 19.018 | 65.600 | 1.00 | 62.45 | A |
| ATOM | 207 | O | ALA | A | 53 | 9.563 | 19.994 | 64.870 | 1.00 | 62.52 | A |
| ATOM | 208 | N | LYS | A | 54 | 9.725 | 19.100 | 66.929 | 1.00 | 64.82 | A |
| ATOM | 209 | CA | LYS | A | 54 | 9.572 | 20.363 | 67.648 | 1.00 | 67.09 | A |
| ATOM | 210 | CB | LYS | A | 54 | 8.186 | 20.429 | 68.354 | 1.00 | 67.81 | A |
| ATOM | 211 | CG | LYS | A | 54 | 7.006 | 20.624 | 67.423 | 1.00 | 69.13 | A |
| ATOM | 212 | CD | LYS | A | 54 | 5.795 | 21.086 | 68.201 | 1.00 | 69.60 | A |
| ATOM | 213 | CE | LYS | A | 54 | 4.767 | 21.716 | 67.278 | 1.00 | 71.24 | A |

| ATOM | 214 | NZ | LYS | A | 54 | 3.702 | 22.425 | 68.044 | 1.00 | 71.69 | A |
|------|-----|-----|-----|---|----|-------|--------|--------|------|-------|---|
| ATOM | 215 | C | LYS | A | 54 | 10.669 | 20.440 | 68.697 | 1.00 | 68.52 | A |
| ATOM | 216 | O | LYS | A | 54 | 11.145 | 19.413 | 69.159 | 1.00 | 69.24 | A |
| ATOM | 217 | N | TRP | A | 55 | 11.065 | 21.653 | 69.072 | 1.00 | 70.47 | A |
| ATOM | 218 | CA | TRP | A | 55 | 12.105 | 21.834 | 70.089 | 1.00 | 71.29 | A |
| ATOM | 219 | CB | TRP | A | 55 | 13.484 | 21.534 | 69.499 | 1.00 | 72.22 | A |
| ATOM | 220 | CG | TRP | A | 55 | 14.612 | 21.796 | 70.444 | 1.00 | 72.57 | A |
| ATOM | 221 | CD2 | TRP | A | 55 | 15.537 | 22.887 | 70.383 | 1.00 | 74.03 | A |
| ATOM | 222 | CE2 | TRP | A | 55 | 16.430 | 22.739 | 71.467 | 1.00 | 74.42 | A |
| ATOM | 223 | CE3 | TRP | A | 55 | 15.696 | 23.977 | 69.519 | 1.00 | 74.16 | A |
| ATOM | 224 | CD1 | TRP | A | 55 | 14.966 | 21.051 | 71.531 | 1.00 | 73.79 | A |
| ATOM | 225 | NE1 | TRP | A | 55 | 16.060 | 21.612 | 72.152 | 1.00 | 74.02 | A |
| ATOM | 226 | CZ2 | TRP | A | 55 | 17.471 | 23.639 | 71.707 | 1.00 | 73.84 | A |
| ATOM | 227 | CZ3 | TRP | A | 55 | 16.728 | 24.871 | 69.759 | 1.00 | 73.89 | A |
| ATOM | 228 | CH2 | TRP | A | 55 | 17.603 | 24.696 | 70.845 | 1.00 | 73.86 | A |
| ATOM | 229 | C | TRP | A | 55 | 12.065 | 23.261 | 70.622 | 1.00 | 72.27 | A |
| ATOM | 230 | O | TRP | A | 55 | 12.177 | 24.209 | 69.863 | 1.00 | 72.12 | A |
| ATOM | 231 | N | ARG | A | 56 | 11.925 | 23.402 | 71.937 | 1.00 | 73.26 | A |
| ATOM | 232 | CA | ARG | A | 56 | 11.829 | 24.724 | 72.545 | 1.00 | 73.69 | A |
| ATOM | 233 | CB | ARG | A | 56 | 13.174 | 25.450 | 72.510 | 1.00 | 74.06 | A |
| ATOM | 234 | CG | ARG | A | 56 | 14.134 | 24.926 | 73.559 | 1.00 | 74.73 | A |
| ATOM | 235 | CD | ARG | A | 56 | 15.405 | 25.739 | 73.642 | 1.00 | 75.75 | A |
| ATOM | 236 | NE | ARG | A | 56 | 16.409 | 25.064 | 74.464 | 1.00 | 77.42 | A |
| ATOM | 237 | CZ | ARG | A | 56 | 17.643 | 25.518 | 74.668 | 1.00 | 79.14 | A |
| ATOM | 238 | NH1 | ARG | A | 56 | 18.037 | 26.659 | 74.110 | 1.00 | 78.95 | A |
| ATOM | 239 | NH2 | ARG | A | 56 | 18.488 | 24.827 | 75.424 | 1.00 | 79.88 | A |
| ATOM | 240 | C | ARG | A | 56 | 10.775 | 25.497 | 71.762 | 1.00 | 73.90 | A |
| ATOM | 241 | O | ARG | A | 56 | 10.909 | 26.700 | 71.510 | 1.00 | 74.26 | A |
| ATOM | 242 | N | ALA | A | 57 | 9.730 | 24.765 | 71.374 | 1.00 | 74.12 | A |
| ATOM | 243 | CA | ALA | A | 57 | 8.598 | 25.298 | 70.622 | 1.00 | 73.97 | A |
| ATOM | 244 | CB | ALA | A | 57 | 7.869 | 26.389 | 71.461 | 1.00 | 74.38 | A |
| ATOM | 245 | C | ALA | A | 57 | 8.964 | 25.848 | 69.241 | 1.00 | 74.19 | A |
| ATOM | 246 | O | ALA | A | 57 | 8.225 | 26.658 | 68.676 | 1.00 | 74.71 | A |
| ATOM | 247 | N | LYS | A | 58 | 10.107 | 25.417 | 68.707 | 1.00 | 73.51 | A |
| ATOM | 248 | CA | LYS | A | 58 | 10.548 | 25.846 | 67.376 | 1.00 | 72.52 | A |
| ATOM | 249 | CB | LYS | A | 58 | 12.056 | 26.312 | 67.383 | 1.00 | 73.26 | A |
| ATOM | 250 | CG | LYS | A | 58 | 12.557 | 26.953 | 68.676 | 1.00 | 74.46 | A |
| ATOM | 251 | CD | LYS | A | 58 | 12.022 | 28.364 | 68.879 | 1.00 | 75.95 | A |
| ATOM | 252 | CE | LYS | A | 58 | 12.671 | 29.021 | 70.102 | 1.00 | 76.54 | A |
| ATOM | 253 | NZ | LYS | A | 58 | 12.193 | 30.419 | 70.334 | 1.00 | 76.54 | A |
| ATOM | 254 | C | LYS | A | 58 | 10.410 | 24.625 | 66.467 | 1.00 | 71.50 | A |
| ATOM | 255 | O | LYS | A | 58 | 10.741 | 23.506 | 66.880 | 1.00 | 70.70 | A |
| ATOM | 256 | N | ASP | A | 59 | 9.910 | 24.818 | 65.247 | 1.00 | 70.03 | A |
| ATOM | 257 | CA | ASP | A | 59 | 9.779 | 23.688 | 64.334 | 1.00 | 69.25 | A |
| ATOM | 258 | CB | ASP | A | 59 | 8.870 | 24.022 | 63.181 | 1.00 | 69.42 | A |
| ATOM | 259 | CG | ASP | A | 59 | 7.420 | 23.701 | 63.490 | 1.00 | 72.29 | A |
| ATOM | 260 | OD1 | ASP | A | 59 | 7.175 | 22.731 | 64.242 | 1.00 | 74.33 | A |
| ATOM | 261 | OD2 | ASP | A | 59 | 6.524 | 24.405 | 62.980 | 1.00 | 75.71 | A |
| ATOM | 262 | C | ASP | A | 59 | 11.163 | 23.299 | 63.840 | 1.00 | 65.99 | A |
| ATOM | 263 | O | ASP | A | 59 | 11.876 | 24.088 | 63.236 | 1.00 | 65.58 | A |
| ATOM | 264 | N | VAL | A | 60 | 11.529 | 22.057 | 64.115 | 1.00 | 62.77 | A |
| ATOM | 265 | CA | VAL | A | 60 | 12.839 | 21.555 | 63.766 | 1.00 | 59.93 | A |
| ATOM | 266 | CB | VAL | A | 60 | 13.665 | 21.402 | 65.069 | 1.00 | 60.27 | A |
| ATOM | 267 | CG1 | VAL | A | 60 | 14.670 | 20.275 | 64.953 | 1.00 | 59.89 | A |

| ATOM | 268 | CG2 | VAL | A | 60 | 14.374 | 22.724 | 65.378 | 1.00 | 61.09 | A |
|------|-----|-----|-----|---|----|--------|--------|--------|------|-------|---|
| ATOM | 269 | C | VAL | A | 60 | 12.826 | 20.239 | 62.986 | 1.00 | 58.71 | A |
| ATOM | 270 | O | VAL | A | 60 | 11.803 | 19.549 | 62.906 | 1.00 | 58.18 | A |
| ATOM | 271 | N | ALA | A | 61 | 13.971 | 19.923 | 62.385 | 1.00 | 56.93 | A |
| ATOM | 272 | CA | ALA | A | 61 | 14.157 | 18.688 | 61.636 | 1.00 | 56.10 | A |
| ATOM | 273 | CB | ALA | A | 61 | 14.569 | 18.983 | 60.234 | 1.00 | 55.73 | A |
| ATOM | 274 | C | ALA | A | 61 | 15.270 | 17.959 | 62.358 | 1.00 | 54.84 | A |
| ATOM | 275 | O | ALA | A | 61 | 16.245 | 18.577 | 62.767 | 1.00 | 53.84 | A |
| ATOM | 276 | N | ILE | A | 62 | 15.118 | 16.649 | 62.522 | 1.00 | 54.63 | A |
| ATOM | 277 | CA | ILE | A | 62 | 16.124 | 15.859 | 63.219 | 1.00 | 54.90 | A |
| ATOM | 278 | CB | ILE | A | 62 | 15.682 | 15.553 | 64.669 | 1.00 | 54.63 | A |
| ATOM | 279 | CG2 | ILE | A | 62 | 15.392 | 16.833 | 65.399 | 1.00 | 54.47 | A |
| ATOM | 280 | CG1 | ILE | A | 62 | 14.424 | 14.695 | 64.670 | 1.00 | 54.58 | A |
| ATOM | 281 | CD1 | ILE | A | 62 | 14.591 | 13.364 | 65.371 | 1.00 | 55.78 | A |
| ATOM | 282 | C | ILE | A | 62 | 16.414 | 14.540 | 62.528 | 1.00 | 54.83 | A |
| ATOM | 283 | O | ILE | A | 62 | 15.513 | 13.881 | 62.021 | 1.00 | 53.16 | A |
| ATOM | 284 | N | LYS | A | 63 | 17.685 | 14.171 | 62.493 | 1.00 | 56.09 | A |
| ATOM | 285 | CA | LYS | A | 63 | 18.079 | 12.904 | 61.905 | 1.00 | 58.74 | A |
| ATOM | 286 | CB | LYS | A | 63 | 18.943 | 13.104 | 60.633 | 1.00 | 58.18 | A |
| ATOM | 287 | CG | LYS | A | 63 | 20.341 | 13.656 | 60.891 | 1.00 | 58.73 | A |
| ATOM | 288 | CD | LYS | A | 63 | 21.204 | 13.658 | 59.630 | 1.00 | 59.18 | A |
| ATOM | 289 | CE | LYS | A | 63 | 21.616 | 12.256 | 59.233 | 1.00 | 59.65 | A |
| ATOM | 290 | NZ | LYS | A | 63 | 22.485 | 12.259 | 58.035 | 1.00 | 59.44 | A |
| ATOM | 291 | C | LYS | A | 63 | 18.874 | 12.178 | 62.980 | 1.00 | 59.65 | A |
| ATOM | 292 | O | LYS | A | 63 | 19.678 | 12.775 | 63.670 | 1.00 | 59.27 | A |
| ATOM | 293 | N | GLN | A | 64 | 18.623 | 10.886 | 63.124 | 1.00 | 62.06 | A |
| ATOM | 294 | CA | GLN | A | 64 | 19.303 | 10.071 | 64.122 | 1.00 | 63.50 | A |
| ATOM | 295 | CB | GLN | A | 64 | 18.315 | 9.702 | 65.236 | 1.00 | 63.96 | A |
| ATOM | 296 | CG | GLN | A | 64 | 16.865 | 9.840 | 64.808 | 1.00 | 64.96 | A |
| ATOM | 297 | CD | GLN | A | 64 | 15.900 | 9.419 | 65.881 | 1.00 | 65.46 | A |
| ATOM | 298 | OE1 | GLN | A | 64 | 14.713 | 9.191 | 65.609 | 1.00 | 66.34 | A |
| ATOM | 299 | NE2 | GLN | A | 64 | 16.392 | 9.317 | 67.115 | 1.00 | 67.05 | A |
| ATOM | 300 | C | GLN | A | 64 | 19.800 | 8.816 | 63.413 | 1.00 | 65.52 | A |
| ATOM | 301 | O | GLN | A | 64 | 19.704 | 8.727 | 62.186 | 1.00 | 65.50 | A |
| ATOM | 302 | N | ILE | A | 65 | 20.332 | 7.856 | 64.170 | 1.00 | 68.10 | A |
| ATOM | 303 | CA | ILE | A | 65 | 20.814 | 6.608 | 63.571 | 1.00 | 70.66 | A |
| ATOM | 304 | CB | ILE | A | 65 | 22.218 | 6.197 | 64.076 | 1.00 | 70.84 | A |
| ATOM | 305 | CG2 | ILE | A | 65 | 23.181 | 7.329 | 63.915 | 1.00 | 72.02 | A |
| ATOM | 306 | CG1 | ILE | A | 65 | 22.146 | 5.765 | 65.531 | 1.00 | 71.24 | A |
| ATOM | 307 | CD1 | ILE | A | 65 | 23.336 | 4.919 | 65.987 | 1.00 | 71.56 | A |
| ATOM | 308 | C | ILE | A | 65 | 19.880 | 5.438 | 63.850 | 1.00 | 71.15 | A |
| ATOM | 309 | O | ILE | A | 65 | 19.233 | 5.366 | 64.895 | 1.00 | 71.16 | A |
| ATOM | 310 | N | GLU | A | 66 | 19.824 | 4.524 | 62.892 | 1.00 | 72.37 | A |
| ATOM | 311 | CA | GLU | A | 66 | 19.000 | 3.333 | 62.996 | 1.00 | 73.32 | A |
| ATOM | 312 | CB | GLU | A | 66 | 18.034 | 3.256 | 61.800 | 1.00 | 73.29 | A |
| ATOM | 313 | CG | GLU | A | 66 | 17.307 | 4.566 | 61.521 | 1.00 | 72.84 | A |
| ATOM | 314 | CD | GLU | A | 66 | 16.478 | 4.518 | 60.256 | 1.00 | 73.54 | A |
| ATOM | 315 | OE1 | GLU | A | 66 | 17.069 | 4.359 | 59.172 | 1.00 | 74.63 | A |
| ATOM | 316 | OE2 | GLU | A | 66 | 15.238 | 4.637 | 60.340 | 1.00 | 73.15 | A |
| ATOM | 317 | C | GLU | A | 66 | 19.990 | 2.180 | 62.973 | 1.00 | 74.25 | A |
| ATOM | 318 | O | GLU | A | 66 | 20.281 | 1.610 | 61.919 | 1.00 | 74.72 | A |
| ATOM | 319 | N | SER | A | 67 | 20.517 | 1.868 | 64.152 | 1.00 | 75.14 | A |
| ATOM | 320 | CA | SER | A | 67 | 21.506 | 0.810 | 64.328 | 1.00 | 75.54 | A |
| ATOM | 321 | CB | SER | A | 67 | 21.278 | -0.355 | 63.306 | 1.00 | 76.25 | A |

| ATOM | 322 | OG | SER | A | 67 | 21.997 | -1.517 | 63.681 | 1.00 | 77.61 | A |
|------|-----|-----|-----|---|----|--------|--------|--------|------|-------|---|
| ATOM | 323 | C | SER | A | 67 | 22.885 | 1.424 | 64.125 | 1.00 | 75.62 | A |
| ATOM | 324 | O | SER | A | 67 | 23.093 | 2.190 | 63.181 | 1.00 | 75.64 | A |
| ATOM | 325 | N | GLU | A | 68 | 23.818 | 1.094 | 65.019 | 1.00 | 75.44 | A |
| ATOM | 326 | CA | GLU | A | 68 | 25.172 | 1.626 | 64.928 | 1.00 | 75.28 | A |
| ATOM | 327 | CB | GLU | A | 68 | 26.042 | 1.114 | 66.068 | 1.00 | 75.71 | A |
| ATOM | 328 | CG | GLU | A | 68 | 25.893 | 1.983 | 67.301 | 1.00 | 77.57 | A |
| ATOM | 329 | CD | GLU | A | 68 | 27.017 | 1.817 | 68.297 | 1.00 | 77.92 | A |
| ATOM | 330 | OE1 | GLU | A | 68 | 28.196 | 1.903 | 67.887 | 1.00 | 80.49 | A |
| ATOM | 331 | OE2 | GLU | A | 68 | 26.717 | 1.618 | 69.494 | 1.00 | 81.97 | A |
| ATOM | 332 | C | GLU | A | 68 | 25.802 | 1.307 | 63.588 | 1.00 | 74.37 | A |
| ATOM | 333 | O | GLU | A | 68 | 25.175 | 0.674 | 62.748 | 1.00 | 74.67 | A |
| ATOM | 334 | N | SER | A | 69 | 27.047 | 1.744 | 63.417 | 1.00 | 73.13 | A |
| ATOM | 335 | CA | SER | A | 69 | 27.797 | 1.596 | 62.171 | 1.00 | 72.37 | A |
| ATOM | 336 | CB | SER | A | 69 | 27.305 | 0.349 | 61.313 | 1.00 | 72.19 | A |
| ATOM | 337 | OG | SER | A | 69 | 26.063 | 0.589 | 60.670 | 1.00 | 71.48 | A |
| ATOM | 338 | C | SER | A | 69 | 27.497 | 2.909 | 61.454 | 1.00 | 71.40 | A |
| ATOM | 339 | O | SER | A | 69 | 28.272 | 3.388 | 60.628 | 1.00 | 71.04 | A |
| ATOM | 340 | N | GLU | A | 70 | 26.347 | 3.480 | 61.805 | 1.00 | 70.77 | A |
| ATOM | 341 | CA | GLU | A | 70 | 25.894 | 4.758 | 61.275 | 1.00 | 70.33 | A |
| ATOM | 342 | CB | GLU | A | 70 | 24.335 | 4.791 | 61.195 | 1.00 | 70.12 | A |
| ATOM | 343 | CG | GLU | A | 70 | 23.755 | 4.718 | 59.780 | 1.00 | 69.63 | A |
| ATOM | 344 | CD | GLU | A | 70 | 22.246 | 4.978 | 59.749 | 1.00 | 70.45 | A |
| ATOM | 345 | OE1 | GLU | A | 70 | 21.783 | 5.896 | 60.462 | 1.00 | 70.22 | A |
| ATOM | 346 | OE2 | GLU | A | 70 | 21.518 | 4.285 | 59.001 | 1.00 | 70.89 | A |
| ATOM | 347 | C | GLU | A | 70 | 26.397 | 5.769 | 62.310 | 1.00 | 69.65 | A |
| ATOM | 348 | O | GLU | A | 70 | 26.285 | 6.979 | 62.129 | 1.00 | 69.69 | A |
| ATOM | 349 | N | ARG | A | 71 | 26.959 | 5.233 | 63.394 | 1.00 | 69.24 | A |
| ATOM | 350 | CA | ARG | A | 71 | 27.499 | 6.013 | 64.503 | 1.00 | 68.36 | A |
| ATOM | 351 | CB | ARG | A | 71 | 27.889 | 5.093 | 65.628 | 1.00 | 69.26 | A |
| ATOM | 352 | CG | ARG | A | 71 | 28.605 | 5.782 | 66.768 | 1.00 | 70.74 | A |
| ATOM | 353 | CD | ARG | A | 71 | 27.618 | 6.379 | 67.757 | 1.00 | 75.67 | A |
| ATOM | 354 | NE | ARG | A | 71 | 27.280 | 5.445 | 68.830 | 1.00 | 77.27 | A |
| ATOM | 355 | CZ | ARG | A | 71 | 26.446 | 5.716 | 69.833 | 1.00 | 79.14 | A |
| ATOM | 356 | NH1 | ARG | A | 71 | 25.848 | 6.898 | 69.910 | 1.00 | 79.92 | A |
| ATOM | 357 | NH2 | ARG | A | 71 | 26.219 | 4.805 | 70.771 | 1.00 | 79.64 | A |
| ATOM | 358 | C | ARG | A | 71 | 28.727 | 6.794 | 64.070 | 1.00 | 67.04 | A |
| ATOM | 359 | O | ARG | A | 71 | 28.765 | 8.001 | 64.159 | 1.00 | 66.03 | A |
| ATOM | 360 | N | LYS | A | 72 | 29.740 | 6.068 | 63.620 | 1.00 | 65.70 | A |
| ATOM | 361 | CA | LYS | A | 72 | 30.981 | 6.673 | 63.158 | 1.00 | 65.48 | A |
| ATOM | 362 | CB | LYS | A | 72 | 31.774 | 5.646 | 62.341 | 1.00 | 66.15 | A |
| ATOM | 363 | CG | LYS | A | 72 | 32.708 | 4.761 | 63.155 | 1.00 | 68.11 | A |
| ATOM | 364 | CD | LYS | A | 72 | 34.011 | 5.482 | 63.461 | 1.00 | 69.81 | A |
| ATOM | 365 | CE | LYS | A | 72 | 35.049 | 4.532 | 64.041 | 1.00 | 70.13 | A |
| ATOM | 366 | NZ | LYS | A | 72 | 36.370 | 5.204 | 64.212 | 1.00 | 69.51 | A |
| ATOM | 367 | C | LYS | A | 72 | 30.685 | 7.897 | 62.296 | 1.00 | 64.34 | A |
| ATOM | 368 | O | LYS | A | 72 | 31.115 | 8.991 | 62.599 | 1.00 | 63.81 | A |
| ATOM | 369 | N | ALA | A | 73 | 29.941 | 7.686 | 61.215 | 1.00 | 62.89 | A |
| ATOM | 370 | CA | ALA | A | 73 | 29.576 | 8.765 | 60.298 | 1.00 | 61.80 | A |
| ATOM | 371 | CB | ALA | A | 73 | 28.505 | 8.259 | 59.268 | 1.00 | 61.70 | A |
| ATOM | 372 | C | ALA | A | 73 | 29.040 | 9.979 | 61.050 | 1.00 | 60.61 | A |
| ATOM | 373 | O | ALA | A | 73 | 29.536 | 11.089 | 60.879 | 1.00 | 60.02 | A |
| ATOM | 374 | N | PHE | A | 74 | 28.026 | 9.745 | 61.884 | 1.00 | 59.44 | A |
| ATOM | 375 | CA | PHE | A | 74 | 27.389 | 10.802 | 62.662 | 1.00 | 59.12 | A |

EP 1 851 310 B1

| ATOM | 376 | CB | PHE | A | 74 | 26.393 | 10.234 | 63.614 | 1.00 | 59.72 | A |
| ATOM | 377 | CG | PHE | A | 74 | 24.980 | 10.542 | 63.252 | 1.00 | 60.67 | A |
| ATOM | 378 | CD1 | PHE | A | 74 | 24.313 | 9.780 | 62.293 | 1.00 | 61.94 | A |
| ATOM | 379 | CD2 | PHE | A | 74 | 24.314 | 11.598 | 63.859 | 1.00 | 62.17 | A |
| ATOM | 380 | CE1 | PHE | A | 74 | 22.991 | 10.064 | 61.942 | 1.00 | 61.56 | A |
| ATOM | 381 | CE2 | PHE | A | 74 | 22.994 | 11.897 | 63.519 | 1.00 | 61.47 | A |
| ATOM | 382 | CZ | PHE | A | 74 | 22.328 | 11.128 | 62.558 | 1.00 | 61.20 | A |
| ATOM | 383 | C | PHE | A | 74 | 28.371 | 11.638 | 63.448 | 1.00 | 58.38 | A |
| ATOM | 384 | O | PHE | A | 74 | 28.095 | 12.776 | 63.761 | 1.00 | 58.05 | A |
| ATOM | 385 | N | ILE | A | 75 | 29.514 | 11.057 | 63.783 | 1.00 | 57.31 | A |
| ATOM | 386 | CA | ILE | A | 75 | 30.527 | 11.786 | 64.535 | 1.00 | 56.94 | A |
| ATOM | 387 | CB | ILE | A | 75 | 31.541 | 10.826 | 65.185 | 1.00 | 56.92 | A |
| ATOM | 388 | CG2 | ILE | A | 75 | 32.669 | 11.621 | 65.809 | 1.00 | 57.17 | A |
| ATOM | 389 | CG1 | ILE | A | 75 | 30.829 | 9.950 | 66.245 | 1.00 | 57.19 | A |
| ATOM | 390 | CD1 | ILE | A | 75 | 29.985 | 10.745 | 67.241 | 1.00 | 57.47 | A |
| ATOM | 391 | C | ILE | A | 75 | 31.261 | 12.750 | 63.609 | 1.00 | 56.35 | A |
| ATOM | 392 | O | ILE | A | 75 | 31.474 | 13.908 | 63.950 | 1.00 | 56.20 | A |
| ATOM | 393 | N | VAL | A | 76 | 31.646 | 12.261 | 62.435 | 1.00 | 55.60 | A |
| ATOM | 394 | CA | VAL | A | 76 | 32.329 | 13.099 | 61.468 | 1.00 | 55.83 | A |
| ATOM | 395 | CB | VAL | A | 76 | 32.640 | 12.336 | 60.176 | 1.00 | 55.69 | A |
| ATOM | 396 | CG1 | VAL | A | 76 | 33.367 | 13.285 | 59.168 | 1.00 | 55.92 | A |
| ATOM | 397 | CG2 | VAL | A | 76 | 33.501 | 11.105 | 60.493 | 1.00 | 55.23 | A |
| ATOM | 398 | C | VAL | A | 76 | 31.411 | 14.264 | 61.127 | 1.00 | 55.63 | A |
| ATOM | 399 | O | VAL | A | 76 | 31.757 | 15.411 | 61.371 | 1.00 | 55.42 | A |
| ATOM | 400 | N | GLU | A | 77 | 30.236 | 13.960 | 60.571 | 1.00 | 55.70 | A |
| ATOM | 401 | CA | GLU | A | 77 | 29.282 | 15.003 | 60.200 | 1.00 | 56.25 | A |
| ATOM | 402 | CB | GLU | A | 77 | 27.846 | 14.422 | 60.001 | 1.00 | 56.29 | A |
| ATOM | 403 | CG | GLU | A | 77 | 27.324 | 14.511 | 58.570 | 1.00 | 58.84 | A |
| ATOM | 404 | CD | GLU | A | 77 | 25.807 | 14.458 | 58.488 | 1.00 | 59.89 | A |
| ATOM | 405 | OE1 | GLU | A | 77 | 25.208 | 13.551 | 59.105 | 1.00 | 64.74 | A |
| ATOM | 406 | OE2 | GLU | A | 77 | 25.213 | 15.320 | 57.798 | 1.00 | 65.29 | A |
| ATOM | 407 | C | GLU | A | 77 | 29.250 | 16.056 | 61.293 | 1.00 | 55.68 | A |
| ATOM | 408 | O | GLU | A | 77 | 29.276 | 17.249 | 61.014 | 1.00 | 54.92 | A |
| ATOM | 409 | N | LEU | A | 78 | 29.217 | 15.593 | 62.540 | 1.00 | 55.66 | A |
| ATOM | 410 | CA | LEU | A | 78 | 29.170 | 16.477 | 63.701 | 1.00 | 55.58 | A |
| ATOM | 411 | CB | LEU | A | 78 | 29.103 | 15.662 | 64.978 | 1.00 | 55.66 | A |
| ATOM | 412 | CG | LEU | A | 78 | 27.736 | 15.586 | 65.633 | 1.00 | 56.23 | A |
| ATOM | 413 | CD1 | LEU | A | 78 | 27.804 | 14.688 | 66.853 | 1.00 | 58.11 | A |
| ATOM | 414 | CD2 | LEU | A | 78 | 27.281 | 16.973 | 66.016 | 1.00 | 57.31 | A |
| ATOM | 415 | C | LEU | A | 78 | 30.360 | 17.426 | 63.770 | 1.00 | 54.92 | A |
| ATOM | 416 | O | LEU | A | 78 | 30.174 | 18.651 | 63.884 | 1.00 | 54.09 | A |
| ATOM | 417 | N | ARG | A | 79 | 31.573 | 16.872 | 63.712 | 1.00 | 54.68 | A |
| ATOM | 418 | CA | ARG | A | 79 | 32.779 | 17.696 | 63.758 | 1.00 | 55.21 | A |
| ATOM | 419 | CB | ARG | A | 79 | 34.051 | 16.834 | 63.598 | 1.00 | 55.36 | A |
| ATOM | 420 | CG | ARG | A | 79 | 34.225 | 16.249 | 62.186 | 1.00 | 58.41 | A |
| ATOM | 421 | CD | ARG | A | 79 | 35.666 | 15.855 | 61.878 | 1.00 | 59.86 | A |
| ATOM | 422 | NE | ARG | A | 79 | 36.565 | 17.007 | 61.903 | 1.00 | 64.19 | A |
| ATOM | 423 | CZ | ARG | A | 79 | 36.461 | 18.075 | 61.110 | 1.00 | 65.71 | A |
| ATOM | 424 | NH1 | ARG | A | 79 | 35.491 | 18.159 | 60.207 | 1.00 | 65.01 | A |
| ATOM | 425 | NH2 | ARG | A | 79 | 37.335 | 19.069 | 61.223 | 1.00 | 64.68 | A |
| ATOM | 426 | C | ARG | A | 79 | 32.719 | 18.717 | 62.623 | 1.00 | 53.87 | A |
| ATOM | 427 | O | ARG | A | 79 | 33.016 | 19.889 | 62.817 | 1.00 | 53.58 | A |
| ATOM | 428 | N | GLN | A | 80 | 32.325 | 18.256 | 61.438 | 1.00 | 52.25 | A |
| ATOM | 429 | CA | GLN | A | 80 | 32.235 | 19.125 | 60.281 | 1.00 | 51.90 | A |

93

```
ATOM    430  CB   GLN A  80      31.901  18.294  59.013  1.00 51.28           A
ATOM    431  CG   GLN A  80      33.127  17.712  58.309  1.00 52.01           A
ATOM    432  CD   GLN A  80      32.775  16.867  57.092  1.00 53.40           A
ATOM    433  OE1  GLN A  80      33.661  16.358  56.401  1.00 54.01           A
ATOM    434  NE2  GLN A  80      31.479  16.714  56.826  1.00 55.96           A
ATOM    435  C    GLN A  80      31.185  20.211  60.486  1.00 50.65           A
ATOM    436  O    GLN A  80      31.506  21.397  60.502  1.00 49.65           A
ATOM    437  N    LEU A  81      29.934  19.791  60.660  1.00 50.20           A
ATOM    438  CA   LEU A  81      28.812  20.710  60.836  1.00 50.39           A
ATOM    439  CB   LEU A  81      27.545  19.936  61.095  1.00 50.50           A
ATOM    440  CG   LEU A  81      26.751  19.563  59.859  1.00 51.53           A
ATOM    441  CD1  LEU A  81      25.599  18.669  60.240  1.00 51.65           A
ATOM    442  CD2  LEU A  81      26.234  20.820  59.188  1.00 51.49           A
ATOM    443  C    LEU A  81      29.011  21.732  61.939  1.00 49.45           A
ATOM    444  O    LEU A  81      28.371  22.785  61.934  1.00 50.26           A
ATOM    445  N    SER A  82      29.895  21.419  62.883  1.00 48.35           A
ATOM    446  CA   SER A  82      30.191  22.320  63.998  1.00 47.35           A
ATOM    447  CB   SER A  82      30.848  21.546  65.109  1.00 47.33           A
ATOM    448  OG   SER A  82      32.191  21.243  64.777  1.00 46.64           A
ATOM    449  C    SER A  82      31.113  23.467  63.555  1.00 46.64           A
ATOM    450  O    SER A  82      31.186  24.515  64.212  1.00 46.89           A
ATOM    451  N    ARG A  83      31.808  23.256  62.439  1.00 45.81           A
ATOM    452  CA   ARG A  83      32.716  24.249  61.884  1.00 46.12           A
ATOM    453  CB   ARG A  83      33.887  23.549  61.153  1.00 46.33           A
ATOM    454  CG   ARG A  83      34.977  22.981  62.051  1.00 48.61           A
ATOM    455  CD   ARG A  83      35.913  22.085  61.258  1.00 49.49           A
ATOM    456  NE   ARG A  83      36.716  22.834  60.297  1.00 52.21           A
ATOM    457  CZ   ARG A  83      37.194  22.325  59.162  1.00 54.20           A
ATOM    458  NH1  ARG A  83      36.941  21.060  58.836  1.00 55.25           A
ATOM    459  NH2  ARG A  83      37.944  23.074  58.362  1.00 54.54           A
ATOM    460  C    ARG A  83      32.005  25.187  60.903  1.00 45.25           A
ATOM    461  O    ARG A  83      32.326  26.375  60.828  1.00 45.66           A
ATOM    462  N    VAL A  84      31.037  24.653  60.157  1.00 44.28           A
ATOM    463  CA   VAL A  84      30.309  25.437  59.157  1.00 43.24           A
ATOM    464  CB   VAL A  84      29.622  24.538  58.139  1.00 43.00           A
ATOM    465  CG1  VAL A  84      30.632  23.728  57.419  1.00 43.30           A
ATOM    466  CG2  VAL A  84      28.624  23.656  58.833  1.00 45.03           A
ATOM    467  C    VAL A  84      29.256  26.409  59.658  1.00 42.35           A
ATOM    468  O    VAL A  84      28.506  26.129  60.590  1.00 43.35           A
ATOM    469  N    ASN A  85      29.212  27.555  58.993  1.00 41.50           A
ATOM    470  CA   ASN A  85      28.272  28.627  59.283  1.00 39.81           A
ATOM    471  CB   ASN A  85      28.782  29.509  60.356  1.00 41.38           A
ATOM    472  CG   ASN A  85      27.994  30.792  60.459  1.00 43.69           A
ATOM    473  OD1  ASN A  85      28.540  31.837  60.788  1.00 50.44           A
ATOM    474  ND2  ASN A  85      26.692  30.713  60.183  1.00 50.49           A
ATOM    475  C    ASN A  85      28.156  29.453  58.012  1.00 37.64           A
ATOM    476  O    ASN A  85      29.056  30.233  57.680  1.00 38.64           A
ATOM    477  N    HIS A  86      27.053  29.286  57.299  1.00 34.61           A
ATOM    478  CA   HIS A  86      26.866  30.023  56.072  1.00 32.33           A
ATOM    479  CB   HIS A  86      27.766  29.417  54.973  1.00 31.83           A
ATOM    480  CG   HIS A  86      27.706  30.152  53.672  1.00 31.49           A
ATOM    481  CD2  HIS A  86      28.591  30.984  53.080  1.00 29.86           A
ATOM    482  ND1  HIS A  86      26.592  30.136  52.861  1.00 24.89           A
ATOM    483  CE1  HIS A  86      26.791  30.930  51.828  1.00 24.74           A
```

| ATOM | 484 | NE2 | HIS | A | 86 | 27.996 | 31.459 | 51.938 | 1.00 | 26.73 | A |
|------|-----|-----|-----|---|-----|--------|--------|--------|------|-------|---|
| ATOM | 485 | C | HIS | A | 86 | 25.403 | 30.029 | 55.634 | 1.00 | 30.40 | A |
| ATOM | 486 | O | HIS | A | 86 | 24.686 | 29.052 | 55.794 | 1.00 | 29.47 | A |
| ATOM | 487 | N | PRO | A | 87 | 24.946 | 31.154 | 55.073 | 1.00 | 28.55 | A |
| ATOM | 488 | CD | PRO | A | 87 | 25.747 | 32.348 | 54.750 | 1.00 | 28.85 | A |
| ATOM | 489 | CA | PRO | A | 87 | 23.570 | 31.311 | 54.608 | 1.00 | 27.88 | A |
| ATOM | 490 | CB | PRO | A | 87 | 23.616 | 32.621 | 53.833 | 1.00 | 28.33 | A |
| ATOM | 491 | CG | PRO | A | 87 | 24.693 | 33.382 | 54.494 | 1.00 | 28.40 | A |
| ATOM | 492 | C | PRO | A | 87 | 23.026 | 30.172 | 53.756 | 1.00 | 25.57 | A |
| ATOM | 493 | O | PRO | A | 87 | 21.882 | 29.825 | 53.878 | 1.00 | 25.46 | A |
| ATOM | 494 | N | ASN | A | 88 | 23.845 | 29.603 | 52.888 | 1.00 | 23.99 | A |
| ATOM | 495 | CA | ASN | A | 88 | 23.356 | 28.544 | 52.021 | 1.00 | 24.63 | A |
| ATOM | 496 | CB | ASN | A | 88 | 23.770 | 28.834 | 50.597 | 1.00 | 22.61 | A |
| ATOM | 497 | CG | ASN | A | 88 | 23.273 | 30.181 | 50.117 | 1.00 | 21.94 | A |
| ATOM | 498 | OD1 | ASN | A | 88 | 22.142 | 30.321 | 49.709 | 1.00 | 23.78 | A |
| ATOM | 499 | ND2 | ASN | A | 88 | 24.129 | 31.183 | 50.186 | 1.00 | 18.14 | A |
| ATOM | 500 | C | ASN | A | 88 | 23.786 | 27.139 | 52.419 | 1.00 | 24.77 | A |
| ATOM | 501 | O | ASN | A | 88 | 24.131 | 26.328 | 51.581 | 1.00 | 21.48 | A |
| ATOM | 502 | N | ILE | A | 89 | 23.742 | 26.870 | 53.717 | 1.00 | 26.32 | A |
| ATOM | 503 | CA | ILE | A | 89 | 24.100 | 25.574 | 54.279 | 1.00 | 29.38 | A |
| ATOM | 504 | CB | ILE | A | 89 | 25.585 | 25.565 | 54.707 | 1.00 | 28.69 | A |
| ATOM | 505 | CG2 | ILE | A | 89 | 25.899 | 24.337 | 55.510 | 1.00 | 28.29 | A |
| ATOM | 506 | CG1 | ILE | A | 89 | 26.454 | 25.638 | 53.495 | 1.00 | 29.06 | A |
| ATOM | 507 | CD1 | ILE | A | 89 | 27.902 | 25.435 | 53.786 | 1.00 | 30.17 | A |
| ATOM | 508 | C | ILE | A | 89 | 23.201 | 25.347 | 55.500 | 1.00 | 29.86 | A |
| ATOM | 509 | O | ILE | A | 89 | 23.240 | 26.108 | 56.434 | 1.00 | 28.73 | A |
| ATOM | 510 | N | VAL | A | 90 | 22.381 | 24.305 | 55.481 | 1.00 | 32.64 | A |
| ATOM | 511 | CA | VAL | A | 90 | 21.481 | 24.046 | 56.601 | 1.00 | 37.11 | A |
| ATOM | 512 | CB | VAL | A | 90 | 21.003 | 22.593 | 56.616 | 1.00 | 36.71 | A |
| ATOM | 513 | CG1 | VAL | A | 90 | 20.130 | 22.325 | 55.405 | 1.00 | 39.46 | A |
| ATOM | 514 | CG2 | VAL | A | 90 | 22.202 | 21.654 | 56.615 | 1.00 | 38.79 | A |
| ATOM | 515 | C | VAL | A | 90 | 22.138 | 24.338 | 57.928 | 1.00 | 39.33 | A |
| ATOM | 516 | O | VAL | A | 90 | 23.266 | 23.965 | 58.157 | 1.00 | 39.08 | A |
| ATOM | 517 | N | LYS | A | 91 | 21.418 | 25.022 | 58.806 | 1.00 | 43.97 | A |
| ATOM | 518 | CA | LYS | A | 91 | 21.973 | 25.344 | 60.110 | 1.00 | 47.15 | A |
| ATOM | 519 | CB | LYS | A | 91 | 21.356 | 26.662 | 60.664 | 1.00 | 47.20 | A |
| ATOM | 520 | CG | LYS | A | 91 | 22.081 | 27.218 | 61.899 | 1.00 | 48.44 | A |
| ATOM | 521 | CD | LYS | A | 91 | 21.386 | 28.464 | 62.466 | 1.00 | 51.07 | A |
| ATOM | 522 | CE | LYS | A | 91 | 21.546 | 29.720 | 61.578 | 1.00 | 54.62 | A |
| ATOM | 523 | NZ | LYS | A | 91 | 22.864 | 30.426 | 61.749 | 1.00 | 55.89 | A |
| ATOM | 524 | C | LYS | A | 91 | 21.742 | 24.209 | 61.101 | 1.00 | 49.47 | A |
| ATOM | 525 | O | LYS | A | 91 | 20.657 | 23.622 | 61.172 | 1.00 | 50.06 | A |
| ATOM | 526 | N | LEU | A | 92 | 22.782 | 23.891 | 61.856 | 1.00 | 52.73 | A |
| ATOM | 527 | CA | LEU | A | 92 | 22.688 | 22.851 | 62.862 | 1.00 | 54.44 | A |
| ATOM | 528 | CB | LEU | A | 92 | 23.985 | 22.063 | 62.920 | 1.00 | 54.34 | A |
| ATOM | 529 | CG | LEU | A | 92 | 24.062 | 20.957 | 63.926 | 1.00 | 55.07 | A |
| ATOM | 530 | CD1 | LEU | A | 92 | 22.856 | 20.044 | 63.814 | 1.00 | 57.07 | A |
| ATOM | 531 | CD2 | LEU | A | 92 | 25.315 | 20.187 | 63.690 | 1.00 | 55.41 | A |
| ATOM | 532 | C | LEU | A | 92 | 22.447 | 23.568 | 64.184 | 1.00 | 56.75 | A |
| ATOM | 533 | O | LEU | A | 92 | 23.155 | 24.509 | 64.509 | 1.00 | 58.49 | A |
| ATOM | 534 | N | TYR | A | 93 | 21.438 | 23.145 | 64.938 | 1.00 | 58.84 | A |
| ATOM | 535 | CA | TYR | A | 93 | 21.163 | 23.787 | 66.213 | 1.00 | 58.90 | A |
| ATOM | 536 | CB | TYR | A | 93 | 19.715 | 23.744 | 66.527 | 1.00 | 59.94 | A |
| ATOM | 537 | CG | TYR | A | 93 | 18.869 | 24.645 | 65.673 | 1.00 | 60.85 | A |

| ATOM | 538 | CD1 | TYR | A | 93 | 19.421 | 25.741 | 65.021 | 1.00 | 62.73 | A |
|------|-----|-----|-----|---|-----|--------|--------|--------|------|-------|---|
| ATOM | 539 | CE1 | TYR | A | 93 | 18.635 | 26.581 | 64.246 | 1.00 | 63.34 | A |
| ATOM | 540 | CD2 | TYR | A | 93 | 17.503 | 24.410 | 65.532 | 1.00 | 61.86 | A |
| ATOM | 541 | CE2 | TYR | A | 93 | 16.707 | 25.242 | 64.763 | 1.00 | 61.67 | A |
| ATOM | 542 | CZ | TYR | A | 93 | 17.278 | 26.323 | 64.121 | 1.00 | 62.65 | A |
| ATOM | 543 | OH | TYR | A | 93 | 16.485 | 27.130 | 63.342 | 1.00 | 62.50 | A |
| ATOM | 544 | C | TYR | A | 93 | 21.923 | 23.113 | 67.337 | 1.00 | 60.29 | A |
| ATOM | 545 | O | TYR | A | 93 | 22.195 | 23.727 | 68.347 | 1.00 | 60.95 | A |
| ATOM | 546 | N | GLY | A | 94 | 22.256 | 21.841 | 67.149 | 1.00 | 60.68 | A |
| ATOM | 547 | CA | GLY | A | 94 | 22.975 | 21.116 | 68.178 | 1.00 | 61.62 | A |
| ATOM | 548 | C | GLY | A | 94 | 22.833 | 19.631 | 67.970 | 1.00 | 62.83 | A |
| ATOM | 549 | O | GLY | A | 94 | 22.366 | 19.205 | 66.929 | 1.00 | 63.29 | A |
| ATOM | 550 | N | ALA | A | 95 | 23.228 | 18.849 | 68.969 | 1.00 | 64.27 | A |
| ATOM | 551 | CA | ALA | A | 95 | 23.151 | 17.399 | 68.876 | 1.00 | 65.68 | A |
| ATOM | 552 | CB | ALA | A | 95 | 24.430 | 16.846 | 68.218 | 1.00 | 65.54 | A |
| ATOM | 553 | C | ALA | A | 95 | 22.956 | 16.774 | 70.250 | 1.00 | 67.01 | A |
| ATOM | 554 | O | ALA | A | 95 | 22.442 | 17.413 | 71.146 | 1.00 | 66.84 | A |
| ATOM | 555 | N | CYS | A | 96 | 23.372 | 15.517 | 70.390 | 1.00 | 69.02 | A |
| ATOM | 556 | CA | CYS | A | 96 | 23.257 | 14.780 | 71.644 | 1.00 | 71.21 | A |
| ATOM | 557 | CB | CYS | A | 96 | 21.885 | 14.898 | 72.194 | 1.00 | 71.62 | A |
| ATOM | 558 | SG | CYS | A | 96 | 20.597 | 14.918 | 70.937 | 1.00 | 74.98 | A |
| ATOM | 559 | C | CYS | A | 96 | 23.544 | 13.315 | 71.405 | 1.00 | 72.06 | A |
| ATOM | 560 | O | CYS | A | 96 | 23.398 | 12.828 | 70.293 | 1.00 | 72.42 | A |
| ATOM | 561 | N | LEU | A | 97 | 23.955 | 12.610 | 72.454 | 1.00 | 72.94 | A |
| ATOM | 562 | CA | LEU | A | 97 | 24.226 | 11.189 | 72.320 | 1.00 | 73.45 | A |
| ATOM | 563 | CB | LEU | A | 97 | 25.670 | 10.825 | 72.789 | 1.00 | 73.66 | A |
| ATOM | 564 | CG | LEU | A | 97 | 26.350 | 9.626 | 71.968 | 1.00 | 74.12 | A |
| ATOM | 565 | CD1 | LEU | A | 97 | 27.866 | 9.820 | 71.923 | 1.00 | 75.02 | A |
| ATOM | 566 | CD2 | LEU | A | 97 | 25.993 | 8.248 | 72.586 | 1.00 | 74.33 | A |
| ATOM | 567 | C | LEU | A | 97 | 23.213 | 10.382 | 73.108 | 1.00 | 73.68 | A |
| ATOM | 568 | O | LEU | A | 97 | 23.361 | 9.172 | 73.225 | 1.00 | 73.80 | A |
| ATOM | 569 | N | ASN | A | 98 | 22.195 | 11.051 | 73.661 | 1.00 | 73.97 | A |
| ATOM | 570 | CA | ASN | A | 98 | 21.161 | 10.339 | 74.412 | 1.00 | 74.06 | A |
| ATOM | 571 | CB | ASN | A | 98 | 19.945 | 11.241 | 74.673 | 1.00 | 74.70 | A |
| ATOM | 572 | CG | ASN | A | 98 | 20.257 | 12.714 | 74.465 | 1.00 | 76.99 | A |
| ATOM | 573 | OD1 | ASN | A | 98 | 21.210 | 13.254 | 75.039 | 1.00 | 79.55 | A |
| ATOM | 574 | ND2 | ASN | A | 98 | 19.451 | 13.375 | 73.641 | 1.00 | 78.84 | A |
| ATOM | 575 | C | ASN | A | 98 | 20.861 | 9.220 | 73.427 | 1.00 | 73.86 | A |
| ATOM | 576 | O | ASN | A | 98 | 21.137 | 8.046 | 73.710 | 1.00 | 74.46 | A |
| ATOM | 577 | N | PRO | A | 99 | 20.279 | 9.548 | 72.263 | 1.00 | 73.13 | A |
| ATOM | 578 | CD | PRO | A | 99 | 19.334 | 10.630 | 71.922 | 1.00 | 73.13 | A |
| ATOM | 579 | CA | PRO | A | 99 | 20.082 | 8.390 | 71.388 | 1.00 | 72.42 | A |
| ATOM | 580 | CB | PRO | A | 99 | 18.695 | 8.603 | 70.854 | 1.00 | 72.91 | A |
| ATOM | 581 | CG | PRO | A | 99 | 18.633 | 10.096 | 70.697 | 1.00 | 73.29 | A |
| ATOM | 582 | C | PRO | A | 99 | 21.174 | 8.580 | 70.318 | 1.00 | 71.96 | A |
| ATOM | 583 | O | PRO | A | 99 | 21.712 | 7.618 | 69.762 | 1.00 | 72.61 | A |
| ATOM | 584 | N | VAL | A | 100 | 21.499 | 9.862 | 70.119 | 1.00 | 70.27 | A |
| ATOM | 585 | CA | VAL | A | 100 | 22.467 | 10.416 | 69.167 | 1.00 | 69.45 | A |
| ATOM | 586 | CB | VAL | A | 100 | 23.571 | 9.403 | 68.706 | 1.00 | 69.22 | A |
| ATOM | 587 | CG1 | VAL | A | 100 | 23.058 | 8.490 | 67.590 | 1.00 | 69.64 | A |
| ATOM | 588 | CG2 | VAL | A | 100 | 24.812 | 10.179 | 68.213 | 1.00 | 69.78 | A |
| ATOM | 589 | C | VAL | A | 100 | 21.681 | 10.926 | 67.961 | 1.00 | 67.49 | A |
| ATOM | 590 | O | VAL | A | 100 | 21.102 | 10.145 | 67.192 | 1.00 | 67.04 | A |
| ATOM | 591 | N | CYS | A | 101 | 21.634 | 12.246 | 67.811 | 1.00 | 65.51 | A |

```
ATOM    592  CA   CYS A 101       20.894  12.841  66.706  1.00 63.63       A
ATOM    593  CB   CYS A 101       19.462  12.765  66.975  1.00 64.03       A
ATOM    594  SG   CYS A 101       18.978  14.020  68.153  1.00 66.98       A
ATOM    595  C    CYS A 101       21.246  14.297  66.486  1.00 62.50       A
ATOM    596  O    CYS A 101       21.667  14.976  67.389  1.00 62.69       A
ATOM    597  N    LEU A 102       21.029  14.760  65.261  1.00 60.33       A
ATOM    598  CA   LEU A 102       21.306  16.135  64.890  1.00 58.65       A
ATOM    599  CB   LEU A 102       22.013  16.179  63.552  1.00 58.41       A
ATOM    600  CG   LEU A 102       23.552  16.169  63.563  1.00 60.19       A
ATOM    601  CD1  LEU A 102       24.075  15.133  64.548  1.00 61.79       A
ATOM    602  CD2  LEU A 102       24.059  15.860  62.165  1.00 59.56       A
ATOM    603  C    LEU A 102       20.008  16.923  64.801  1.00 56.68       A
ATOM    604  O    LEU A 102       19.037  16.479  64.185  1.00 56.25       A
ATOM    605  N    VAL A 103       20.002  18.094  65.429  1.00 54.92       A
ATOM    606  CA   VAL A 103       18.843  18.982  65.433  1.00 53.96       A
ATOM    607  CB   VAL A 103       18.533  19.487  66.869  1.00 53.81       A
ATOM    608  CG1  VAL A 103       17.259  20.264  66.883  1.00 53.02       A
ATOM    609  CG2  VAL A 103       18.431  18.325  67.819  1.00 53.17       A
ATOM    610  C    VAL A 103       19.213  20.167  64.539  1.00 53.01       A
ATOM    611  O    VAL A 103       20.154  20.888  64.833  1.00 52.29       A
ATOM    612  N    MET A 104       18.480  20.350  63.445  1.00 51.81       A
ATOM    613  CA   MET A 104       18.761  21.436  62.512  1.00 51.39       A
ATOM    614  CB   MET A 104       19.322  20.876  61.222  1.00 50.48       A
ATOM    615  CG   MET A 104       20.136  19.621  61.422  1.00 52.23       A
ATOM    616  SD   MET A 104       20.323  18.628  59.947  1.00 53.66       A
ATOM    617  CE   MET A 104       18.972  17.479  60.172  1.00 54.57       A
ATOM    618  C    MET A 104       17.501  22.209  62.203  1.00 49.51       A
ATOM    619  O    MET A 104       16.429  21.855  62.647  1.00 48.99       A
ATOM    620  N    GLU A 105       17.648  23.268  61.420  1.00 48.12       A
ATOM    621  CA   GLU A 105       16.513  24.088  61.040  1.00 47.43       A
ATOM    622  CB   GLU A 105       16.997  25.374  60.413  1.00 47.53       A
ATOM    623  CG   GLU A 105       17.477  25.220  58.980  1.00 50.17       A
ATOM    624  CD   GLU A 105       17.887  26.537  58.362  1.00 50.51       A
ATOM    625  OE1  GLU A 105       19.035  26.982  58.606  1.00 51.21       A
ATOM    626  OE2  GLU A 105       17.053  27.129  57.645  1.00 53.48       A
ATOM    627  C    GLU A 105       15.612  23.349  60.054  1.00 45.24       A
ATOM    628  O    GLU A 105       16.080  22.666  59.160  1.00 44.17       A
ATOM    629  N    TYR A 106       14.306  23.493  60.229  1.00 43.79       A
ATOM    630  CA   TYR A 106       13.351  22.845  59.342  1.00 43.11       A
ATOM    631  CB   TYR A 106       12.017  22.619  60.074  1.00 43.01       A
ATOM    632  CG   TYR A 106       10.878  22.170  59.185  1.00 41.62       A
ATOM    633  CD1  TYR A 106       10.953  20.971  58.481  1.00 42.32       A
ATOM    634  CE1  TYR A 106        9.888  20.535  57.675  1.00 42.28       A
ATOM    635  CD2  TYR A 106        9.718  22.932  59.067  1.00 41.17       A
ATOM    636  CE2  TYR A 106        8.652  22.515  58.274  1.00 42.39       A
ATOM    637  CZ   TYR A 106        8.734  21.319  57.582  1.00 43.25       A
ATOM    638  OH   TYR A 106        7.660  20.901  56.823  1.00 45.30       A
ATOM    639  C    TYR A 106       13.110  23.722  58.127  1.00 43.05       A
ATOM    640  O    TYR A 106       12.528  24.802  58.246  1.00 42.92       A
ATOM    641  N    ALA A 107       13.570  23.270  56.965  1.00 42.76       A
ATOM    642  CA   ALA A 107       13.371  24.015  55.723  1.00 42.47       A
ATOM    643  CB   ALA A 107       14.364  23.535  54.654  1.00 42.17       A
ATOM    644  C    ALA A 107       11.932  23.770  55.267  1.00 41.75       A
ATOM    645  O    ALA A 107       11.623  22.745  54.668  1.00 41.38       A
```

| ATOM | 646 | N | GLU A 108 | 11.052 | 24.714 | 55.567 | 1.00 | 41.84 | A |
|------|-----|-----|-----------|--------|--------|--------|------|-------|---|
| ATOM | 647 | CA | GLU A 108 | 9.646 | 24.582 | 55.219 | 1.00 | 41.56 | A |
| ATOM | 648 | CB | GLU A 108 | 8.891 | 25.885 | 55.576 | 1.00 | 43.03 | A |
| ATOM | 649 | CG | GLU A 108 | 9.428 | 27.141 | 54.883 | 1.00 | 46.71 | A |
| ATOM | 650 | CD | GLU A 108 | 10.542 | 27.824 | 55.658 | 1.00 | 51.43 | A |
| ATOM | 651 | OE1 | GLU A 108 | 11.268 | 27.128 | 56.406 | 1.00 | 51.32 | A |
| ATOM | 652 | OE2 | GLU A 108 | 10.694 | 29.059 | 55.498 | 1.00 | 53.10 | A |
| ATOM | 653 | C | GLU A 108 | 9.391 | 24.230 | 53.761 | 1.00 | 40.48 | A |
| ATOM | 654 | O | GLU A 108 | 8.461 | 23.517 | 53.455 | 1.00 | 41.09 | A |
| ATOM | 655 | N | GLY A 109 | 10.238 | 24.732 | 52.871 | 1.00 | 38.33 | A |
| ATOM | 656 | CA | GLY A 109 | 10.073 | 24.485 | 51.447 | 1.00 | 37.36 | A |
| ATOM | 657 | C | GLY A 109 | 10.285 | 23.066 | 50.953 | 1.00 | 36.05 | A |
| ATOM | 658 | O | GLY A 109 | 9.740 | 22.685 | 49.928 | 1.00 | 35.87 | A |
| ATOM | 659 | N | GLY A 110 | 11.082 | 22.290 | 51.678 | 1.00 | 34.25 | A |
| ATOM | 660 | CA | GLY A 110 | 11.342 | 20.918 | 51.284 | 1.00 | 32.29 | A |
| ATOM | 661 | C | GLY A 110 | 12.463 | 20.788 | 50.272 | 1.00 | 30.92 | A |
| ATOM | 662 | O | GLY A 110 | 13.153 | 21.748 | 49.981 | 1.00 | 30.37 | A |
| ATOM | 663 | N | SER A 111 | 12.625 | 19.587 | 49.728 | 1.00 | 28.47 | A |
| ATOM | 664 | CA | SER A 111 | 13.671 | 19.277 | 48.745 | 1.00 | 28.22 | A |
| ATOM | 665 | CB | SER A 111 | 13.696 | 17.672 | 48.481 | 1.00 | 27.89 | A |
| ATOM | 666 | OG | SER A 111 | 14.164 | 17.322 | 47.197 | 1.00 | 29.05 | A |
| ATOM | 667 | C | SER A 111 | 13.525 | 20.028 | 47.422 | 1.00 | 27.02 | A |
| ATOM | 668 | O | SER A 111 | 12.438 | 20.377 | 47.034 | 1.00 | 24.93 | A |
| ATOM | 669 | N | LEU A 112 | 14.642 | 20.290 | 46.754 | 1.00 | 24.70 | A |
| ATOM | 670 | CA | LEU A 112 | 14.607 | 20.942 | 45.460 | 1.00 | 26.16 | A |
| ATOM | 671 | CB | LEU A 112 | 15.967 | 21.482 | 45.076 | 1.00 | 25.06 | A |
| ATOM | 672 | CG | LEU A 112 | 16.143 | 21.846 | 43.570 | 1.00 | 25.48 | A |
| ATOM | 673 | CD1 | LEU A 112 | 15.037 | 22.751 | 43.140 | 1.00 | 26.18 | A |
| ATOM | 674 | CD2 | LEU A 112 | 17.481 | 22.515 | 43.332 | 1.00 | 25.98 | A |
| ATOM | 675 | C | LEU A 112 | 14.195 | 19.882 | 44.445 | 1.00 | 25.01 | A |
| ATOM | 676 | O | LEU A 112 | 13.580 | 20.190 | 43.448 | 1.00 | 23.34 | A |
| ATOM | 677 | N | TYR A 113 | 14.551 | 18.628 | 44.711 | 1.00 | 25.45 | A |
| ATOM | 678 | CA | TYR A 113 | 14.208 | 17.537 | 43.815 | 1.00 | 25.90 | A |
| ATOM | 679 | CB | TYR A 113 | 14.749 | 16.216 | 44.341 | 1.00 | 28.69 | A |
| ATOM | 680 | CG | TYR A 113 | 14.055 | 14.977 | 43.787 | 1.00 | 29.47 | A |
| ATOM | 681 | CD1 | TYR A 113 | 14.525 | 14.332 | 42.641 | 1.00 | 32.17 | A |
| ATOM | 682 | CE1 | TYR A 113 | 13.857 | 13.210 | 42.116 | 1.00 | 33.51 | A |
| ATOM | 683 | CD2 | TYR A 113 | 12.901 | 14.468 | 44.401 | 1.00 | 33.15 | A |
| ATOM | 684 | CE2 | TYR A 113 | 12.229 | 13.355 | 43.891 | 1.00 | 35.12 | A |
| ATOM | 685 | CZ | TYR A 113 | 12.705 | 12.731 | 42.749 | 1.00 | 35.38 | A |
| ATOM | 686 | OH | TYR A 113 | 12.002 | 11.653 | 42.242 | 1.00 | 36.49 | A |
| ATOM | 687 | C | TYR A 113 | 12.701 | 17.481 | 43.747 | 1.00 | 25.05 | A |
| ATOM | 688 | O | TYR A 113 | 12.153 | 17.365 | 42.683 | 1.00 | 22.39 | A |
| ATOM | 689 | N | ASN A 114 | 12.043 | 17.568 | 44.903 | 1.00 | 25.13 | A |
| ATOM | 690 | CA | ASN A 114 | 10.579 | 17.536 | 44.966 | 1.00 | 26.85 | A |
| ATOM | 691 | CB | ASN A 114 | 10.094 | 17.529 | 46.444 | 1.00 | 27.71 | A |
| ATOM | 692 | CG | ASN A 114 | 10.047 | 16.134 | 47.023 | 1.00 | 37.59 | A |
| ATOM | 693 | OD1 | ASN A 114 | 9.497 | 15.214 | 46.405 | 1.00 | 48.36 | A |
| ATOM | 694 | ND2 | ASN A 114 | 10.615 | 15.961 | 48.213 | 1.00 | 40.65 | A |
| ATOM | 695 | C | ASN A 114 | 9.932 | 18.707 | 44.223 | 1.00 | 25.48 | A |
| ATOM | 696 | O | ASN A 114 | 8.889 | 18.557 | 43.610 | 1.00 | 26.06 | A |
| ATOM | 697 | N | VAL A 115 | 10.544 | 19.882 | 44.303 | 1.00 | 25.05 | A |
| ATOM | 698 | CA | VAL A 115 | 10.006 | 21.025 | 43.606 | 1.00 | 23.94 | A |
| ATOM | 699 | CB | VAL A 115 | 10.803 | 22.274 | 43.913 | 1.00 | 23.71 | A |

| | | | | | | | | | | | |
|---|---|---|---|---|---|---|---|---|---|---|---|
| ATOM | 700 | CG1 | VAL | A | 115 | 10.544 | 23.320 | 42.899 | 1.00 | 25.63 | A |
| ATOM | 701 | CG2 | VAL | A | 115 | 10.422 | 22.773 | 45.238 | 1.00 | 26.44 | A |
| ATOM | 702 | C | VAL | A | 115 | 10.093 | 20.714 | 42.128 | 1.00 | 22.91 | A |
| ATOM | 703 | O | VAL | A | 115 | 9.126 | 20.811 | 41.409 | 1.00 | 21.90 | A |
| ATOM | 704 | N | LEU | A | 116 | 11.270 | 20.310 | 41.681 | 1.00 | 21.86 | A |
| ATOM | 705 | CA | LEU | A | 116 | 11.449 | 19.989 | 40.281 | 1.00 | 22.87 | A |
| ATOM | 706 | CB | LEU | A | 116 | 12.865 | 19.555 | 40.021 | 1.00 | 22.99 | A |
| ATOM | 707 | CG | LEU | A | 116 | 13.880 | 20.625 | 40.160 | 1.00 | 24.78 | A |
| ATOM | 708 | CD1 | LEU | A | 116 | 15.271 | 20.058 | 39.838 | 1.00 | 27.66 | A |
| ATOM | 709 | CD2 | LEU | A | 116 | 13.529 | 21.767 | 39.233 | 1.00 | 25.04 | A |
| ATOM | 710 | C | LEU | A | 116 | 10.513 | 18.899 | 39.802 | 1.00 | 24.07 | A |
| ATOM | 711 | O | LEU | A | 116 | 9.625 | 19.145 | 38.990 | 1.00 | 21.80 | A |
| ATOM | 712 | N | HIS | A | 117 | 10.722 | 17.696 | 40.337 | 1.00 | 23.17 | A |
| ATOM | 713 | CA | HIS | A | 117 | 9.976 | 16.496 | 39.952 | 1.00 | 26.99 | A |
| ATOM | 714 | CB | HIS | A | 117 | 10.992 | 15.400 | 39.604 | 1.00 | 26.39 | A |
| ATOM | 715 | CG | HIS | A | 117 | 12.245 | 15.924 | 38.953 | 1.00 | 26.56 | A |
| ATOM | 716 | CD2 | HIS | A | 117 | 13.533 | 15.936 | 39.374 | 1.00 | 27.10 | A |
| ATOM | 717 | ND1 | HIS | A | 117 | 12.246 | 16.543 | 37.720 | 1.00 | 26.15 | A |
| ATOM | 718 | CE1 | HIS | A | 117 | 13.477 | 16.913 | 37.412 | 1.00 | 29.77 | A |
| ATOM | 719 | NE2 | HIS | A | 117 | 14.277 | 16.557 | 38.398 | 1.00 | 27.42 | A |
| ATOM | 720 | C | HIS | A | 117 | 8.935 | 15.955 | 40.935 | 1.00 | 28.60 | A |
| ATOM | 721 | O | HIS | A | 117 | 8.418 | 14.890 | 40.725 | 1.00 | 29.94 | A |
| ATOM | 722 | N | GLY | A | 118 | 8.628 | 16.724 | 41.983 | 1.00 | 32.13 | A |
| ATOM | 723 | CA | GLY | A | 118 | 7.647 | 16.335 | 42.994 | 1.00 | 35.84 | A |
| ATOM | 724 | C | GLY | A | 118 | 6.267 | 15.898 | 42.533 | 1.00 | 39.03 | A |
| ATOM | 725 | O | GLY | A | 118 | 6.044 | 15.646 | 41.363 | 1.00 | 39.54 | A |
| ATOM | 726 | N | ALA | A | 119 | 5.326 | 15.838 | 43.470 | 1.00 | 42.42 | A |
| ATOM | 727 | CA | ALA | A | 119 | 3.967 | 15.364 | 43.179 | 1.00 | 43.72 | A |
| ATOM | 728 | CB | ALA | A | 119 | 3.413 | 14.564 | 44.397 | 1.00 | 44.30 | A |
| ATOM | 729 | C | ALA | A | 119 | 2.922 | 16.369 | 42.731 | 1.00 | 45.58 | A |
| ATOM | 730 | O | ALA | A | 119 | 2.765 | 17.426 | 43.311 | 1.00 | 45.98 | A |
| ATOM | 731 | N | GLU | A | 120 | 2.197 | 15.960 | 41.694 | 1.00 | 46.83 | A |
| ATOM | 732 | CA | GLU | A | 120 | 1.126 | 16.709 | 41.056 | 1.00 | 47.40 | A |
| ATOM | 733 | CB | GLU | A | 120 | -0.208 | 16.202 | 41.548 | 1.00 | 49.57 | A |
| ATOM | 734 | CG | GLU | A | 120 | -0.738 | 14.989 | 40.788 | 1.00 | 55.30 | A |
| ATOM | 735 | CD | GLU | A | 120 | -0.631 | 13.701 | 41.592 | 1.00 | 60.75 | A |
| ATOM | 736 | OE1 | GLU | A | 120 | 0.501 | 13.180 | 41.745 | 1.00 | 63.90 | A |
| ATOM | 737 | OE2 | GLU | A | 120 | -1.686 | 13.221 | 42.078 | 1.00 | 62.76 | A |
| ATOM | 738 | C | GLU | A | 120 | 1.152 | 18.240 | 41.132 | 1.00 | 48.42 | A |
| ATOM | 739 | O | GLU | A | 120 | 1.584 | 18.905 | 40.177 | 1.00 | 50.71 | A |
| ATOM | 740 | N | PRO | A | 121 | 0.674 | 18.840 | 42.239 | 1.00 | 45.50 | A |
| ATOM | 741 | CD | PRO | A | 121 | 0.095 | 18.424 | 43.532 | 1.00 | 45.46 | A |
| ATOM | 742 | CA | PRO | A | 121 | 0.772 | 20.295 | 42.140 | 1.00 | 42.42 | A |
| ATOM | 743 | CB | PRO | A | 121 | -0.040 | 20.769 | 43.337 | 1.00 | 42.87 | A |
| ATOM | 744 | CG | PRO | A | 121 | 0.175 | 19.688 | 44.347 | 1.00 | 44.90 | A |
| ATOM | 745 | C | PRO | A | 121 | 2.245 | 20.732 | 42.182 | 1.00 | 39.19 | A |
| ATOM | 746 | O | PRO | A | 121 | 2.793 | 20.992 | 43.255 | 1.00 | 39.49 | A |
| ATOM | 747 | N | LEU | A | 122 | 2.891 | 20.769 | 41.016 | 1.00 | 36.02 | A |
| ATOM | 748 | CA | LEU | A | 122 | 4.294 | 21.197 | 40.908 | 1.00 | 34.14 | A |
| ATOM | 749 | CB | LEU | A | 122 | 4.976 | 20.498 | 39.779 | 1.00 | 33.59 | A |
| ATOM | 750 | CG | LEU | A | 122 | 5.252 | 19.058 | 39.976 | 1.00 | 34.74 | A |
| ATOM | 751 | CD1 | LEU | A | 122 | 5.686 | 18.394 | 38.623 | 1.00 | 37.23 | A |
| ATOM | 752 | CD2 | LEU | A | 122 | 6.315 | 18.937 | 41.020 | 1.00 | 34.15 | A |
| ATOM | 753 | C | LEU | A | 122 | 4.297 | 22.705 | 40.633 | 1.00 | 31.08 | A |

```
ATOM    754  O    LEU A 122      3.631  23.179  39.701  1.00 28.92        A
ATOM    755  N    PRO A 123      5.052  23.480  41.426  1.00 29.69        A
ATOM    756  CD   PRO A 123      6.098  23.030  42.351  1.00 29.61        A
ATOM    757  CA   PRO A 123      5.111  24.934  41.251  1.00 28.91        A
ATOM    758  CB   PRO A 123      5.955  25.360  42.336  1.00 29.13        A
ATOM    759  CG   PRO A 123      6.955  24.249  42.433  1.00 28.66        A
ATOM    760  C    PRO A 123      5.701  25.339  39.933  1.00 26.20        A
ATOM    761  O    PRO A 123      6.456  24.608  39.326  1.00 25.65        A
ATOM    762  N    TYR A 124      5.338  26.523  39.490  1.00 24.48        A
ATOM    763  CA   TYR A 124      5.859  27.027  38.241  1.00 22.16        A
ATOM    764  CB   TYR A 124      4.832  27.901  37.590  1.00 23.29        A
ATOM    765  CG   TYR A 124      5.369  29.064  36.795  1.00 23.57        A
ATOM    766  CD1  TYR A 124      5.713  28.907  35.467  1.00 23.57        A
ATOM    767  CE1  TYR A 124      6.179  29.963  34.714  1.00 23.27        A
ATOM    768  CD2  TYR A 124      5.507  30.320  37.367  1.00 24.97        A
ATOM    769  CE2  TYR A 124      5.975  31.397  36.623  1.00 25.17        A
ATOM    770  CZ   TYR A 124      6.312  31.209  35.290  1.00 24.09        A
ATOM    771  OH   TYR A 124      6.802  32.249  34.519  1.00 28.64        A
ATOM    772  C    TYR A 124      7.015  27.866  38.704  1.00 20.78        A
ATOM    773  O    TYR A 124      6.885  28.545  39.727  1.00 19.95        A
ATOM    774  N    TYR A 125      8.150  27.793  38.002  1.00 19.08        A
ATOM    775  CA   TYR A 125      9.306  28.622  38.348  1.00 18.81        A
ATOM    776  CB   TYR A 125     10.402  27.834  39.134  1.00 16.52        A
ATOM    777  CG   TYR A 125     11.062  26.717  38.398  1.00 16.66        A
ATOM    778  CD1  TYR A 125     11.947  26.971  37.368  1.00 15.47        A
ATOM    779  CE1  TYR A 125     12.568  25.928  36.689  1.00 16.90        A
ATOM    780  CD2  TYR A 125     10.808  25.391  38.738  1.00 17.14        A
ATOM    781  CE2  TYR A 125     11.419  24.349  38.069  1.00 15.17        A
ATOM    782  CZ   TYR A 125     12.298  24.626  37.051  1.00 15.24        A
ATOM    783  OH   TYR A 125     12.939  23.611  36.413  1.00 13.16        A
ATOM    784  C    TYR A 125      9.865  29.249  37.095  1.00 18.66        A
ATOM    785  O    TYR A 125      9.604  28.802  35.993  1.00 18.93        A
ATOM    786  N    THR A 126     10.626  30.311  37.288  1.00 18.83        A
ATOM    787  CA   THR A 126     11.199  31.061  36.198  1.00 20.72        A
ATOM    788  CB   THR A 126     10.948  32.511  36.463  1.00 20.43        A
ATOM    789  OG1  THR A 126     11.565  32.886  37.696  1.00 20.40        A
ATOM    790  CG2  THR A 126      9.488  32.733  36.611  1.00 21.38        A
ATOM    791  C    THR A 126     12.688  30.838  35.968  1.00 20.33        A
ATOM    792  O    THR A 126     13.351  30.136  36.710  1.00 19.71        A
ATOM    793  N    ALA A 127     13.214  31.423  34.907  1.00 20.88        A
ATOM    794  CA   ALA A 127     14.633  31.285  34.642  1.00 20.79        A
ATOM    795  CB   ALA A 127     15.010  32.060  33.387  1.00 21.50        A
ATOM    796  C    ALA A 127     15.359  31.865  35.852  1.00 19.81        A
ATOM    797  O    ALA A 127     16.282  31.286  36.366  1.00 18.64        A
ATOM    798  N    ALA A 128     14.893  33.020  36.297  1.00 19.27        A
ATOM    799  CA   ALA A 128     15.471  33.709  37.423  1.00 18.89        A
ATOM    800  CB   ALA A 128     14.643  34.872  37.772  1.00 18.08        A
ATOM    801  C    ALA A 128     15.609  32.826  38.622  1.00 18.66        A
ATOM    802  O    ALA A 128     16.578  32.899  39.313  1.00 19.74        A
ATOM    803  N    HIS A 129     14.615  31.998  38.887  1.00 18.34        A
ATOM    804  CA   HIS A 129     14.714  31.120  40.038  1.00 18.93        A
ATOM    805  CB   HIS A 129     13.417  30.311  40.240  1.00 20.07        A
ATOM    806  CG   HIS A 129     12.254  31.131  40.703  1.00 22.40        A
ATOM    807  CD2  HIS A 129     11.030  31.326  40.163  1.00 24.41        A
```

| ATOM | 808 | ND1 | HIS | A | 129 | 12.287 | 31.882 | 41.856 | 1.00 | 26.57 | A |
|------|-----|-----|-----|---|-----|--------|--------|--------|------|-------|---|
| ATOM | 809 | CE1 | HIS | A | 129 | 11.137 | 32.508 | 42.002 | 1.00 | 22.21 | A |
| ATOM | 810 | NE2 | HIS | A | 129 | 10.357 | 32.189 | 40.988 | 1.00 | 26.52 | A |
| ATOM | 811 | C | HIS | A | 129 | 15.858 | 30.184 | 39.744 | 1.00 | 17.45 | A |
| ATOM | 812 | O | HIS | A | 129 | 16.818 | 30.145 | 40.439 | 1.00 | 17.83 | A |
| ATOM | 813 | N | ALA | A | 130 | 15.723 | 29.461 | 38.647 | 1.00 | 18.07 | A |
| ATOM | 814 | CA | ALA | A | 130 | 16.704 | 28.490 | 38.219 | 1.00 | 18.02 | A |
| ATOM | 815 | CB | ALA | A | 130 | 16.419 | 28.085 | 36.803 | 1.00 | 17.14 | A |
| ATOM | 816 | C | ALA | A | 130 | 18.152 | 28.921 | 38.346 | 1.00 | 18.33 | A |
| ATOM | 817 | O | ALA | A | 130 | 18.994 | 28.128 | 38.753 | 1.00 | 16.13 | A |
| ATOM | 818 | N | MET | A | 131 | 18.457 | 30.163 | 37.989 | 1.00 | 17.95 | A |
| ATOM | 819 | CA | MET | A | 131 | 19.829 | 30.632 | 38.060 | 1.00 | 18.21 | A |
| ATOM | 820 | CB | MET | A | 131 | 20.020 | 31.793 | 37.175 | 1.00 | 20.06 | A |
| ATOM | 821 | CG | MET | A | 131 | 19.740 | 31.527 | 35.706 | 1.00 | 22.18 | A |
| ATOM | 822 | SD | MET | A | 131 | 20.925 | 30.496 | 34.895 | 1.00 | 25.30 | A |
| ATOM | 823 | CE | MET | A | 131 | 22.328 | 31.391 | 35.094 | 1.00 | 28.47 | A |
| ATOM | 824 | C | MET | A | 131 | 20.163 | 31.019 | 39.471 | 1.00 | 17.27 | A |
| ATOM | 825 | O | MET | A | 131 | 21.268 | 30.864 | 39.895 | 1.00 | 15.69 | A |
| ATOM | 826 | N | SER | A | 132 | 19.164 | 31.514 | 40.193 | 1.00 | 17.95 | A |
| ATOM | 827 | CA | SER | A | 132 | 19.321 | 31.945 | 41.586 | 1.00 | 17.26 | A |
| ATOM | 828 | CB | SER | A | 132 | 18.097 | 32.595 | 42.054 | 1.00 | 17.00 | A |
| ATOM | 829 | OG | SER | A | 132 | 18.009 | 32.465 | 43.444 | 1.00 | 18.64 | A |
| ATOM | 830 | C | SER | A | 132 | 19.627 | 30.791 | 42.515 | 1.00 | 16.51 | A |
| ATOM | 831 | O | SER | A | 132 | 20.391 | 30.922 | 43.426 | 1.00 | 16.49 | A |
| ATOM | 832 | N | TRP | A | 133 | 18.989 | 29.658 | 42.261 | 1.00 | 17.30 | A |
| ATOM | 833 | CA | TRP | A | 133 | 19.182 | 28.457 | 43.052 | 1.00 | 17.92 | A |
| ATOM | 834 | CB | TRP | A | 133 | 18.253 | 27.413 | 42.619 | 1.00 | 18.17 | A |
| ATOM | 835 | CG | TRP | A | 133 | 16.864 | 27.650 | 43.024 | 1.00 | 18.41 | A |
| ATOM | 836 | CD2 | TRP | A | 133 | 15.704 | 27.152 | 42.380 | 1.00 | 17.29 | A |
| ATOM | 837 | CE2 | TRP | A | 133 | 14.596 | 27.526 | 43.167 | 1.00 | 16.38 | A |
| ATOM | 838 | CE3 | TRP | A | 133 | 15.487 | 26.412 | 41.214 | 1.00 | 20.13 | A |
| ATOM | 839 | CD1 | TRP | A | 133 | 16.434 | 28.295 | 44.143 | 1.00 | 19.16 | A |
| ATOM | 840 | NE1 | TRP | A | 133 | 15.072 | 28.225 | 44.242 | 1.00 | 20.43 | A |
| ATOM | 841 | CZ2 | TRP | A | 133 | 13.287 | 27.186 | 42.824 | 1.00 | 19.44 | A |
| ATOM | 842 | CZ3 | TRP | A | 133 | 14.194 | 26.071 | 40.875 | 1.00 | 18.44 | A |
| ATOM | 843 | CH2 | TRP | A | 133 | 13.106 | 26.458 | 41.676 | 1.00 | 18.41 | A |
| ATOM | 844 | C | TRP | A | 133 | 20.594 | 27.950 | 42.884 | 1.00 | 17.73 | A |
| ATOM | 845 | O | TRP | A | 133 | 21.252 | 27.631 | 43.852 | 1.00 | 15.91 | A |
| ATOM | 846 | N | CYS | A | 134 | 21.059 | 27.877 | 41.646 | 1.00 | 16.21 | A |
| ATOM | 847 | CA | CYS | A | 134 | 22.406 | 27.404 | 41.405 | 1.00 | 17.67 | A |
| ATOM | 848 | CB | CYS | A | 134 | 22.604 | 27.169 | 39.959 | 1.00 | 18.43 | A |
| ATOM | 849 | SG | CYS | A | 134 | 21.430 | 25.977 | 39.319 | 1.00 | 26.24 | A |
| ATOM | 850 | C | CYS | A | 134 | 23.437 | 28.380 | 41.939 | 1.00 | 16.81 | A |
| ATOM | 851 | O | CYS | A | 134 | 24.375 | 27.988 | 42.572 | 1.00 | 16.48 | A |
| ATOM | 852 | N | LEU | A | 135 | 23.243 | 29.669 | 41.683 | 1.00 | 16.68 | A |
| ATOM | 853 | CA | LEU | A | 135 | 24.187 | 30.666 | 42.157 | 1.00 | 17.45 | A |
| ATOM | 854 | CB | LEU | A | 135 | 23.696 | 32.029 | 41.889 | 1.00 | 17.21 | A |
| ATOM | 855 | CG | LEU | A | 135 | 24.398 | 33.039 | 42.765 | 1.00 | 19.39 | A |
| ATOM | 856 | CD1 | LEU | A | 135 | 25.767 | 33.289 | 42.213 | 1.00 | 22.07 | A |
| ATOM | 857 | CD2 | LEU | A | 135 | 23.633 | 34.317 | 42.850 | 1.00 | 18.97 | A |
| ATOM | 858 | C | LEU | A | 135 | 24.304 | 30.465 | 43.649 | 1.00 | 16.92 | A |
| ATOM | 859 | O | LEU | A | 135 | 25.388 | 30.385 | 44.172 | 1.00 | 16.79 | A |
| ATOM | 860 | N | GLN | A | 136 | 23.160 | 30.388 | 44.321 | 1.00 | 18.21 | A |
| ATOM | 861 | CA | GLN | A | 136 | 23.119 | 30.178 | 45.757 | 1.00 | 18.03 | A |

| ATOM | 862 | CB  | GLN | A | 136 | 21.740 | 30.020 | 46.218 | 1.00 | 18.66 | A |
|------|-----|-----|-----|---|-----|--------|--------|--------|------|-------|---|
| ATOM | 863 | CG  | GLN | A | 136 | 20.958 | 31.290 | 46.285 | 1.00 | 20.30 | A |
| ATOM | 864 | CD  | GLN | A | 136 | 19.568 | 31.051 | 46.802 | 1.00 | 19.15 | A |
| ATOM | 865 | OE1 | GLN | A | 136 | 19.388 | 30.665 | 47.939 | 1.00 | 23.65 | A |
| ATOM | 866 | NE2 | GLN | A | 136 | 18.574 | 31.272 | 45.954 | 1.00 | 16.59 | A |
| ATOM | 867 | C   | GLN | A | 136 | 23.863 | 28.924 | 46.142 | 1.00 | 18.83 | A |
| ATOM | 868 | O   | GLN | A | 136 | 24.615 | 28.933 | 47.079 | 1.00 | 18.43 | A |
| ATOM | 869 | N   | CYS | A | 137 | 23.619 | 27.832 | 45.432 | 1.00 | 18.57 | A |
| ATOM | 870 | CA  | CYS | A | 137 | 24.300 | 26.589 | 45.750 | 1.00 | 18.66 | A |
| ATOM | 871 | CB  | CYS | A | 137 | 23.863 | 25.533 | 44.809 | 1.00 | 18.68 | A |
| ATOM | 872 | SG  | CYS | A | 137 | 24.927 | 24.114 | 44.758 | 1.00 | 24.22 | A |
| ATOM | 873 | C   | CYS | A | 137 | 25.796 | 26.828 | 45.621 | 1.00 | 19.35 | A |
| ATOM | 874 | O   | CYS | A | 137 | 26.570 | 26.457 | 46.479 | 1.00 | 20.62 | A |
| ATOM | 875 | N   | SER | A | 138 | 26.167 | 27.478 | 44.524 | 1.00 | 18.63 | A |
| ATOM | 876 | CA  | SER | A | 138 | 27.541 | 27.832 | 44.205 | 1.00 | 18.99 | A |
| ATOM | 877 | CB  | SER | A | 138 | 27.530 | 28.777 | 43.050 | 1.00 | 19.84 | A |
| ATOM | 878 | OG  | SER | A | 138 | 28.821 | 28.960 | 42.536 | 1.00 | 30.98 | A |
| ATOM | 879 | C   | SER | A | 138 | 28.158 | 28.508 | 45.414 | 1.00 | 18.44 | A |
| ATOM | 880 | O   | SER | A | 138 | 29.233 | 28.167 | 45.860 | 1.00 | 17.24 | A |
| ATOM | 881 | N   | GLN | A | 139 | 27.438 | 29.477 | 45.946 | 1.00 | 18.50 | A |
| ATOM | 882 | CA  | GLN | A | 139 | 27.897 | 30.209 | 47.104 | 1.00 | 19.87 | A |
| ATOM | 883 | CB  | GLN | A | 139 | 26.868 | 31.247 | 47.482 | 1.00 | 19.15 | A |
| ATOM | 884 | CG  | GLN | A | 139 | 26.782 | 32.371 | 46.473 | 1.00 | 21.63 | A |
| ATOM | 885 | CD  | GLN | A | 139 | 25.798 | 33.439 | 46.864 | 1.00 | 22.81 | A |
| ATOM | 886 | OE1 | GLN | A | 139 | 25.729 | 34.492 | 46.234 | 1.00 | 26.59 | A |
| ATOM | 887 | NE2 | GLN | A | 139 | 25.019 | 33.173 | 47.904 | 1.00 | 27.89 | A |
| ATOM | 888 | C   | GLN | A | 139 | 28.189 | 29.305 | 48.280 | 1.00 | 19.66 | A |
| ATOM | 889 | O   | GLN | A | 139 | 29.149 | 29.509 | 48.959 | 1.00 | 17.95 | A |
| ATOM | 890 | N   | GLY | A | 140 | 27.343 | 28.305 | 48.493 | 1.00 | 18.59 | A |
| ATOM | 891 | CA  | GLY | A | 140 | 27.532 | 27.387 | 49.598 | 1.00 | 19.63 | A |
| ATOM | 892 | C   | GLY | A | 140 | 28.754 | 26.524 | 49.395 | 1.00 | 19.17 | A |
| ATOM | 893 | O   | GLY | A | 140 | 29.659 | 26.523 | 50.196 | 1.00 | 17.24 | A |
| ATOM | 894 | N   | VAL | A | 141 | 28.783 | 25.782 | 48.302 | 1.00 | 20.44 | A |
| ATOM | 895 | CA  | VAL | A | 141 | 29.927 | 24.936 | 48.037 | 1.00 | 20.80 | A |
| ATOM | 896 | CB  | VAL | A | 141 | 29.786 | 24.254 | 46.685 | 1.00 | 20.90 | A |
| ATOM | 897 | CG1 | VAL | A | 141 | 30.994 | 23.476 | 46.375 | 1.00 | 21.99 | A |
| ATOM | 898 | CG2 | VAL | A | 141 | 28.626 | 23.360 | 46.706 | 1.00 | 20.62 | A |
| ATOM | 899 | C   | VAL | A | 141 | 31.231 | 25.732 | 48.081 | 1.00 | 21.09 | A |
| ATOM | 900 | O   | VAL | A | 141 | 32.160 | 25.356 | 48.780 | 1.00 | 19.43 | A |
| ATOM | 901 | N   | ALA | A | 142 | 31.297 | 26.835 | 47.339 | 1.00 | 20.80 | A |
| ATOM | 902 | CA  | ALA | A | 142 | 32.502 | 27.660 | 47.328 | 1.00 | 21.25 | A |
| ATOM | 903 | CB  | ALA | A | 142 | 32.239 | 28.963 | 46.584 | 1.00 | 21.78 | A |
| ATOM | 904 | C   | ALA | A | 142 | 32.987 | 27.944 | 48.764 | 1.00 | 21.66 | A |
| ATOM | 905 | O   | ALA | A | 142 | 34.192 | 28.086 | 49.002 | 1.00 | 22.17 | A |
| ATOM | 906 | N   | TYR | A | 143 | 32.052 | 28.017 | 49.714 | 1.00 | 23.67 | A |
| ATOM | 907 | CA  | TYR | A | 143 | 32.398 | 28.270 | 51.104 | 1.00 | 25.01 | A |
| ATOM | 908 | CB  | TYR | A | 143 | 31.111 | 28.572 | 51.956 | 1.00 | 27.77 | A |
| ATOM | 909 | CG  | TYR | A | 143 | 31.337 | 28.622 | 53.461 | 1.00 | 27.65 | A |
| ATOM | 910 | CD1 | TYR | A | 143 | 31.481 | 27.451 | 54.207 | 1.00 | 32.55 | A |
| ATOM | 911 | CE1 | TYR | A | 143 | 31.742 | 27.499 | 55.578 | 1.00 | 33.18 | A |
| ATOM | 912 | CD2 | TYR | A | 143 | 31.454 | 29.843 | 54.129 | 1.00 | 32.58 | A |
| ATOM | 913 | CE2 | TYR | A | 143 | 31.717 | 29.901 | 55.502 | 1.00 | 34.52 | A |
| ATOM | 914 | CZ  | TYR | A | 143 | 31.862 | 28.730 | 56.220 | 1.00 | 34.17 | A |
| ATOM | 915 | OH  | TYR | A | 143 | 32.139 | 28.791 | 57.571 | 1.00 | 35.44 | A |

| ATOM | 916 | C | TYR | A | 143 | 33.105 | 27.034 | 51.629 | 1.00 | 25.95 | A |
|------|-----|-----|-----|---|-----|--------|--------|--------|------|-------|---|
| ATOM | 917 | O | TYR | A | 143 | 34.165 | 27.117 | 52.228 | 1.00 | 26.35 | A |
| ATOM | 918 | N | LEU | A | 144 | 32.503 | 25.880 | 51.387 | 1.00 | 25.64 | A |
| ATOM | 919 | CA | LEU | A | 144 | 33.069 | 24.616 | 51.832 | 1.00 | 24.70 | A |
| ATOM | 920 | CB | LEU | A | 144 | 32.225 | 23.460 | 51.315 | 1.00 | 24.15 | A |
| ATOM | 921 | CG | LEU | A | 144 | 30.798 | 23.452 | 51.707 | 1.00 | 24.90 | A |
| ATOM | 922 | CD1 | LEU | A | 144 | 30.143 | 22.322 | 51.071 | 1.00 | 29.11 | A |
| ATOM | 923 | CD2 | LEU | A | 144 | 30.679 | 23.343 | 53.204 | 1.00 | 28.65 | A |
| ATOM | 924 | C | LEU | A | 144 | 34.485 | 24.474 | 51.302 | 1.00 | 25.18 | A |
| ATOM | 925 | O | LEU | A | 144 | 35.399 | 24.132 | 52.018 | 1.00 | 24.59 | A |
| ATOM | 926 | N | HIS | A | 145 | 34.644 | 24.743 | 50.022 | 1.00 | 24.33 | A |
| ATOM | 927 | CA | HIS | A | 145 | 35.935 | 24.640 | 49.395 | 1.00 | 25.25 | A |
| ATOM | 928 | CB | HIS | A | 145 | 35.776 | 24.959 | 47.951 | 1.00 | 23.35 | A |
| ATOM | 929 | CG | HIS | A | 145 | 35.120 | 23.869 | 47.172 | 1.00 | 21.27 | A |
| ATOM | 930 | CD2 | HIS | A | 145 | 35.216 | 23.515 | 45.870 | 1.00 | 18.92 | A |
| ATOM | 931 | ND1 | HIS | A | 145 | 34.268 | 22.958 | 47.754 | 1.00 | 21.59 | A |
| ATOM | 932 | CE1 | HIS | A | 145 | 33.876 | 22.083 | 46.849 | 1.00 | 21.57 | A |
| ATOM | 933 | NE2 | HIS | A | 145 | 34.438 | 22.398 | 45.698 | 1.00 | 24.08 | A |
| ATOM | 934 | C | HIS | A | 145 | 36.954 | 25.576 | 50.036 | 1.00 | 25.32 | A |
| ATOM | 935 | O | HIS | A | 145 | 38.131 | 25.267 | 50.097 | 1.00 | 24.94 | A |
| ATOM | 936 | N | SER | A | 146 | 36.483 | 26.716 | 50.528 | 1.00 | 26.45 | A |
| ATOM | 937 | CA | SER | A | 146 | 37.351 | 27.724 | 51.133 | 1.00 | 30.70 | A |
| ATOM | 938 | CB | SER | A | 146 | 36.622 | 28.995 | 51.237 | 1.00 | 30.13 | A |
| ATOM | 939 | OG | SER | A | 146 | 35.845 | 29.009 | 52.426 | 1.00 | 35.16 | A |
| ATOM | 940 | C | SER | A | 146 | 37.897 | 27.411 | 52.507 | 1.00 | 33.09 | A |
| ATOM | 941 | O | SER | A | 146 | 38.874 | 28.006 | 52.934 | 1.00 | 32.91 | A |
| ATOM | 942 | N | MET | A | 147 | 37.235 | 26.489 | 53.193 | 1.00 | 35.73 | A |
| ATOM | 943 | CA | MET | A | 147 | 37.590 | 26.080 | 54.544 | 1.00 | 39.57 | A |
| ATOM | 944 | CB | MET | A | 147 | 36.712 | 24.946 | 54.949 | 1.00 | 40.64 | A |
| ATOM | 945 | CG | MET | A | 147 | 36.362 | 24.944 | 56.421 | 1.00 | 46.42 | A |
| ATOM | 946 | SD | MET | A | 147 | 34.641 | 25.352 | 56.730 | 1.00 | 55.88 | A |
| ATOM | 947 | CE | MET | A | 147 | 33.895 | 23.823 | 56.327 | 1.00 | 51.13 | A |
| ATOM | 948 | C | MET | A | 147 | 39.042 | 25.693 | 54.835 | 1.00 | 41.07 | A |
| ATOM | 949 | O | MET | A | 147 | 39.655 | 24.918 | 54.107 | 1.00 | 39.09 | A |
| ATOM | 950 | N | GLN | A | 148 | 39.568 | 26.235 | 55.932 | 1.00 | 44.37 | A |
| ATOM | 951 | CA | GLN | A | 148 | 40.926 | 25.953 | 56.397 | 1.00 | 46.77 | A |
| ATOM | 952 | CB | GLN | A | 148 | 41.668 | 27.218 | 56.582 | 1.00 | 47.34 | A |
| ATOM | 953 | CG | GLN | A | 148 | 41.421 | 28.242 | 55.498 | 1.00 | 48.34 | A |
| ATOM | 954 | CD | GLN | A | 148 | 41.677 | 29.664 | 55.978 | 1.00 | 51.43 | A |
| ATOM | 955 | OE1 | GLN | A | 148 | 42.822 | 30.060 | 56.227 | 1.00 | 59.62 | A |
| ATOM | 956 | NE2 | GLN | A | 148 | 40.604 | 30.439 | 56.118 | 1.00 | 58.22 | A |
| ATOM | 957 | C | GLN | A | 148 | 40.757 | 25.271 | 57.751 | 1.00 | 48.31 | A |
| ATOM | 958 | O | GLN | A | 148 | 39.779 | 25.519 | 58.441 | 1.00 | 49.25 | A |
| ATOM | 959 | N | PRO | A | 149 | 41.721 | 24.437 | 58.174 | 1.00 | 49.98 | A |
| ATOM | 960 | CD | PRO | A | 149 | 41.394 | 23.577 | 59.324 | 1.00 | 50.41 | A |
| ATOM | 961 | CA | PRO | A | 149 | 42.995 | 24.017 | 57.577 | 1.00 | 50.24 | A |
| ATOM | 962 | CB | PRO | A | 149 | 43.509 | 22.938 | 58.540 | 1.00 | 50.51 | A |
| ATOM | 963 | CG | PRO | A | 149 | 42.752 | 23.195 | 59.818 | 1.00 | 50.24 | A |
| ATOM | 964 | C | PRO | A | 149 | 42.871 | 23.468 | 56.177 | 1.00 | 50.25 | A |
| ATOM | 965 | O | PRO | A | 149 | 43.673 | 23.797 | 55.301 | 1.00 | 50.48 | A |
| ATOM | 966 | N | LYS | A | 150 | 41.884 | 22.604 | 55.975 | 1.00 | 50.23 | A |
| ATOM | 967 | CA | LYS | A | 150 | 41.688 | 22.025 | 54.661 | 1.00 | 49.74 | A |
| ATOM | 968 | CB | LYS | A | 150 | 42.255 | 20.609 | 54.617 | 1.00 | 50.99 | A |
| ATOM | 969 | CG | LYS | A | 150 | 41.514 | 19.594 | 55.440 | 1.00 | 53.74 | A |

| ATOM | 970 | CD | LYS | A | 150 | 42.145 | 18.223 | 55.262 | 1.00 | 53.85 | A |
|------|-----|-----|-----|---|-----|--------|--------|--------|------|-------|---|
| ATOM | 971 | CE | LYS | A | 150 | 41.260 | 17.125 | 55.832 | 1.00 | 56.59 | A |
| ATOM | 972 | NZ | LYS | A | 150 | 41.718 | 15.773 | 55.414 | 1.00 | 58.83 | A |
| ATOM | 973 | C | LYS | A | 150 | 40.230 | 22.052 | 54.211 | 1.00 | 47.57 | A |
| ATOM | 974 | O | LYS | A | 150 | 39.309 | 21.941 | 55.021 | 1.00 | 47.70 | A |
| ATOM | 975 | N | ALA | A | 151 | 40.050 | 22.217 | 52.900 | 1.00 | 45.24 | A |
| ATOM | 976 | CA | ALA | A | 151 | 38.745 | 22.307 | 52.255 | 1.00 | 43.90 | A |
| ATOM | 977 | CB | ALA | A | 151 | 38.928 | 22.553 | 50.758 | 1.00 | 43.38 | A |
| ATOM | 978 | C | ALA | A | 151 | 37.851 | 21.107 | 52.462 | 1.00 | 42.32 | A |
| ATOM | 979 | O | ALA | A | 151 | 38.310 | 19.995 | 52.622 | 1.00 | 42.42 | A |
| ATOM | 980 | N | LEU | A | 152 | 36.553 | 21.368 | 52.447 | 1.00 | 39.48 | A |
| ATOM | 981 | CA | LEU | A | 152 | 35.553 | 20.333 | 52.616 | 1.00 | 38.40 | A |
| ATOM | 982 | CB | LEU | A | 152 | 34.505 | 20.765 | 53.656 | 1.00 | 39.15 | A |
| ATOM | 983 | CG | LEU | A | 152 | 34.911 | 20.927 | 55.149 | 1.00 | 43.25 | A |
| ATOM | 984 | CD1 | LEU | A | 152 | 33.729 | 20.462 | 56.040 | 1.00 | 45.53 | A |
| ATOM | 985 | CD2 | LEU | A | 152 | 36.192 | 20.102 | 55.470 | 1.00 | 47.32 | A |
| ATOM | 986 | C | LEU | A | 152 | 34.865 | 20.102 | 51.275 | 1.00 | 36.08 | A |
| ATOM | 987 | O | LEU | A | 152 | 34.283 | 21.026 | 50.708 | 1.00 | 35.88 | A |
| ATOM | 988 | N | ILE | A | 153 | 34.945 | 18.873 | 50.769 | 1.00 | 33.41 | A |
| ATOM | 989 | CA | ILE | A | 153 | 34.310 | 18.510 | 49.505 | 1.00 | 33.23 | A |
| ATOM | 990 | CB | ILE | A | 153 | 35.173 | 17.515 | 48.735 | 1.00 | 32.51 | A |
| ATOM | 991 | CG2 | ILE | A | 153 | 34.484 | 17.128 | 47.439 | 1.00 | 32.03 | A |
| ATOM | 992 | CG1 | ILE | A | 153 | 36.532 | 18.118 | 48.493 | 1.00 | 33.60 | A |
| ATOM | 993 | CD1 | ILE | A | 153 | 37.393 | 17.333 | 47.552 | 1.00 | 34.29 | A |
| ATOM | 994 | C | ILE | A | 153 | 32.954 | 17.865 | 49.793 | 1.00 | 32.51 | A |
| ATOM | 995 | O | ILE | A | 153 | 32.883 | 16.885 | 50.505 | 1.00 | 33.87 | A |
| ATOM | 996 | N | HIS | A | 154 | 31.880 | 18.424 | 49.242 | 1.00 | 30.22 | A |
| ATOM | 997 | CA | HIS | A | 154 | 30.538 | 17.885 | 49.470 | 1.00 | 28.95 | A |
| ATOM | 998 | CB | HIS | A | 154 | 29.519 | 18.702 | 48.685 | 1.00 | 28.12 | A |
| ATOM | 999 | CG | HIS | A | 154 | 28.113 | 18.516 | 49.150 | 1.00 | 27.73 | A |
| ATOM | 1000 | CD2 | HIS | A | 154 | 27.287 | 19.334 | 49.839 | 1.00 | 28.03 | A |
| ATOM | 1001 | ND1 | HIS | A | 154 | 27.415 | 17.346 | 48.949 | 1.00 | 24.83 | A |
| ATOM | 1002 | CE1 | HIS | A | 154 | 26.219 | 17.451 | 49.496 | 1.00 | 28.77 | A |
| ATOM | 1003 | NE2 | HIS | A | 154 | 26.117 | 18.647 | 50.043 | 1.00 | 26.68 | A |
| ATOM | 1004 | C | HIS | A | 154 | 30.460 | 16.408 | 49.078 | 1.00 | 28.33 | A |
| ATOM | 1005 | O | HIS | A | 154 | 30.024 | 15.576 | 49.858 | 1.00 | 26.90 | A |
| ATOM | 1006 | N | ARG | A | 155 | 30.893 | 16.108 | 47.856 | 1.00 | 26.83 | A |
| ATOM | 1007 | CA | ARG | A | 155 | 30.938 | 14.745 | 47.332 | 1.00 | 29.14 | A |
| ATOM | 1008 | CB | ARG | A | 155 | 31.581 | 13.795 | 48.360 | 1.00 | 31.63 | A |
| ATOM | 1009 | CG | ARG | A | 155 | 32.921 | 14.281 | 48.925 | 1.00 | 39.09 | A |
| ATOM | 1010 | CD | ARG | A | 155 | 33.162 | 13.750 | 50.339 | 1.00 | 51.85 | A |
| ATOM | 1011 | NE | ARG | A | 155 | 34.027 | 12.575 | 50.378 | 1.00 | 55.42 | A |
| ATOM | 1012 | CZ | ARG | A | 155 | 35.358 | 12.610 | 50.294 | 1.00 | 59.02 | A |
| ATOM | 1013 | NH1 | ARG | A | 155 | 35.995 | 13.766 | 50.165 | 1.00 | 58.54 | A |
| ATOM | 1014 | NH2 | ARG | A | 155 | 36.062 | 11.484 | 50.350 | 1.00 | 61.26 | A |
| ATOM | 1015 | C | ARG | A | 155 | 29.594 | 14.198 | 46.927 | 1.00 | 28.19 | A |
| ATOM | 1016 | O | ARG | A | 155 | 29.519 | 13.188 | 46.237 | 1.00 | 27.94 | A |
| ATOM | 1017 | N | ASP | A | 156 | 28.529 | 14.863 | 47.345 | 1.00 | 27.60 | A |
| ATOM | 1018 | CA | ASP | A | 156 | 27.207 | 14.379 | 46.990 | 1.00 | 27.35 | A |
| ATOM | 1019 | CB | ASP | A | 156 | 26.673 | 13.514 | 48.081 | 1.00 | 28.06 | A |
| ATOM | 1020 | CG | ASP | A | 156 | 25.709 | 12.486 | 47.561 | 1.00 | 29.91 | A |
| ATOM | 1021 | OD1 | ASP | A | 156 | 25.681 | 12.363 | 46.319 | 1.00 | 32.01 | A |
| ATOM | 1022 | OD2 | ASP | A | 156 | 25.009 | 11.820 | 48.375 | 1.00 | 35.16 | A |
| ATOM | 1023 | C | ASP | A | 156 | 26.196 | 15.458 | 46.664 | 1.00 | 25.95 | A |

| ATOM | 1024 | O   | ASP A 156 | 25.183 | 15.591 | 47.341 | 1.00 | 23.19 | A |
| ATOM | 1025 | N   | LEU A 157 | 26.481 | 16.223 | 45.612 | 1.00 | 25.48 | A |
| ATOM | 1026 | CA  | LEU A 157 | 25.595 | 17.295 | 45.179 | 1.00 | 26.01 | A |
| ATOM | 1027 | CB  | LEU A 157 | 26.369 | 18.402 | 44.539 | 1.00 | 26.98 | A |
| ATOM | 1028 | CG  | LEU A 157 | 26.876 | 19.473 | 45.427 | 1.00 | 31.67 | A |
| ATOM | 1029 | CD1 | LEU A 157 | 27.238 | 20.638 | 44.586 | 1.00 | 32.97 | A |
| ATOM | 1030 | CD2 | LEU A 157 | 25.844 | 19.860 | 46.439 | 1.00 | 33.25 | A |
| ATOM | 1031 | C   | LEU A 157 | 24.582 | 16.763 | 44.193 | 1.00 | 25.58 | A |
| ATOM | 1032 | O   | LEU A 157 | 24.928 | 16.154 | 43.186 | 1.00 | 25.53 | A |
| ATOM | 1033 | N   | LYS A 158 | 23.321 | 16.993 | 44.518 | 1.00 | 23.75 | A |
| ATOM | 1034 | CA  | LYS A 158 | 22.213 | 16.565 | 43.691 | 1.00 | 22.97 | A |
| ATOM | 1035 | CB  | LYS A 158 | 22.118 | 15.032 | 43.655 | 1.00 | 22.99 | A |
| ATOM | 1036 | CG  | LYS A 158 | 21.866 | 14.405 | 45.001 | 1.00 | 20.97 | A |
| ATOM | 1037 | CD  | LYS A 158 | 21.790 | 12.905 | 44.909 | 1.00 | 24.26 | A |
| ATOM | 1038 | CE  | LYS A 158 | 21.581 | 12.298 | 46.284 | 1.00 | 26.00 | A |
| ATOM | 1039 | NZ  | LYS A 158 | 21.247 | 10.849 | 46.186 | 1.00 | 27.44 | A |
| ATOM | 1040 | C   | LYS A 158 | 20.981 | 17.154 | 44.347 | 1.00 | 21.37 | A |
| ATOM | 1041 | O   | LYS A 158 | 20.998 | 17.433 | 45.525 | 1.00 | 21.55 | A |
| ATOM | 1042 | N   | PRO A 159 | 19.898 | 17.351 | 43.576 | 1.00 | 21.58 | A |
| ATOM | 1043 | CD  | PRO A 159 | 19.750 | 16.973 | 42.158 | 1.00 | 21.96 | A |
| ATOM | 1044 | CA  | PRO A 159 | 18.650 | 17.916 | 44.094 | 1.00 | 21.75 | A |
| ATOM | 1045 | CB  | PRO A 159 | 17.649 | 17.572 | 43.021 | 1.00 | 22.21 | A |
| ATOM | 1046 | CG  | PRO A 159 | 18.456 | 17.646 | 41.784 | 1.00 | 22.23 | A |
| ATOM | 1047 | C   | PRO A 159 | 18.231 | 17.409 | 45.479 | 1.00 | 22.24 | A |
| ATOM | 1048 | O   | PRO A 159 | 18.030 | 18.187 | 46.393 | 1.00 | 21.35 | A |
| ATOM | 1049 | N   | PRO A 160 | 18.105 | 16.097 | 45.652 | 1.00 | 22.27 | A |
| ATOM | 1050 | CD  | PRO A 160 | 18.444 | 14.995 | 44.751 | 1.00 | 22.38 | A |
| ATOM | 1051 | CA  | PRO A 160 | 17.705 | 15.588 | 46.956 | 1.00 | 22.53 | A |
| ATOM | 1052 | CB  | PRO A 160 | 18.014 | 14.139 | 46.855 | 1.00 | 22.63 | A |
| ATOM | 1053 | CG  | PRO A 160 | 17.785 | 13.849 | 45.425 | 1.00 | 22.31 | A |
| ATOM | 1054 | C   | PRO A 160 | 18.412 | 16.233 | 48.145 | 1.00 | 23.54 | A |
| ATOM | 1055 | O   | PRO A 160 | 17.824 | 16.386 | 49.198 | 1.00 | 24.27 | A |
| ATOM | 1056 | N   | ASN A 161 | 19.671 | 16.614 | 47.974 | 1.00 | 23.79 | A |
| ATOM | 1057 | CA  | ASN A 161 | 20.429 | 17.208 | 49.067 | 1.00 | 24.03 | A |
| ATOM | 1058 | CB  | ASN A 161 | 21.898 | 16.856 | 48.942 | 1.00 | 24.28 | A |
| ATOM | 1059 | CG  | ASN A 161 | 22.147 | 15.368 | 48.946 | 1.00 | 28.36 | A |
| ATOM | 1060 | OD1 | ASN A 161 | 21.592 | 14.621 | 49.763 | 1.00 | 27.08 | A |
| ATOM | 1061 | ND2 | ASN A 161 | 22.995 | 14.924 | 48.043 | 1.00 | 29.31 | A |
| ATOM | 1062 | C   | ASN A 161 | 20.304 | 18.712 | 49.111 | 1.00 | 24.46 | A |
| ATOM | 1063 | O   | ASN A 161 | 21.082 | 19.379 | 49.780 | 1.00 | 24.95 | A |
| ATOM | 1064 | N   | LEU A 162 | 19.326 | 19.246 | 48.394 | 1.00 | 23.61 | A |
| ATOM | 1065 | CA  | LEU A 162 | 19.137 | 20.682 | 48.356 | 1.00 | 22.85 | A |
| ATOM | 1066 | CB  | LEU A 162 | 19.318 | 21.203 | 46.935 | 1.00 | 22.78 | A |
| ATOM | 1067 | CG  | LEU A 162 | 20.681 | 20.946 | 46.333 | 1.00 | 23.25 | A |
| ATOM | 1068 | CD1 | LEU A 162 | 20.714 | 21.391 | 44.895 | 1.00 | 22.59 | A |
| ATOM | 1069 | CD2 | LEU A 162 | 21.715 | 21.661 | 47.133 | 1.00 | 25.01 | A |
| ATOM | 1070 | C   | LEU A 162 | 17.763 | 21.036 | 48.875 | 1.00 | 24.27 | A |
| ATOM | 1071 | O   | LEU A 162 | 16.779 | 20.544 | 48.386 | 1.00 | 25.57 | A |
| ATOM | 1072 | N   | LEU A 163 | 17.721 | 21.911 | 49.875 | 1.00 | 23.07 | A |
| ATOM | 1073 | CA  | LEU A 163 | 16.468 | 22.323 | 50.476 | 1.00 | 24.67 | A |
| ATOM | 1074 | CB  | LEU A 163 | 16.515 | 22.134 | 51.952 | 1.00 | 25.47 | A |
| ATOM | 1075 | CG  | LEU A 163 | 17.255 | 20.957 | 52.433 | 1.00 | 27.83 | A |
| ATOM | 1076 | CD1 | LEU A 163 | 17.159 | 20.982 | 53.908 | 1.00 | 31.12 | A |
| ATOM | 1077 | CD2 | LEU A 163 | 16.703 | 19.636 | 51.840 | 1.00 | 29.92 | A |

| ATOM | 1078 | C | LEU | A | 163 | 16.150 | 23.767 | 50.177 | 1.00 | 25.91 | A |
|------|------|------|-----|---|-----|--------|--------|--------|------|-------|---|
| ATOM | 1079 | O | LEU | A | 163 | 17.040 | 24.593 | 50.068 | 1.00 | 26.13 | A |
| ATOM | 1080 | N | LEU | A | 164 | 14.855 | 24.051 | 50.059 | 1.00 | 27.31 | A |
| ATOM | 1081 | CA | LEU | A | 164 | 14.370 | 25.388 | 49.767 | 1.00 | 27.26 | A |
| ATOM | 1082 | CB | LEU | A | 164 | 13.385 | 25.362 | 48.589 | 1.00 | 26.47 | A |
| ATOM | 1083 | CG | LEU | A | 164 | 13.784 | 24.864 | 47.219 | 1.00 | 29.11 | A |
| ATOM | 1084 | CD1 | LEU | A | 164 | 12.733 | 25.244 | 46.278 | 1.00 | 32.39 | A |
| ATOM | 1085 | CD2 | LEU | A | 164 | 15.074 | 25.456 | 46.770 | 1.00 | 29.65 | A |
| ATOM | 1086 | C | LEU | A | 164 | 13.652 | 25.954 | 50.967 | 1.00 | 27.97 | A |
| ATOM | 1087 | O | LEU | A | 164 | 12.943 | 25.243 | 51.641 | 1.00 | 27.80 | A |
| ATOM | 1088 | N | VAL | A | 165 | 13.854 | 27.249 | 51.204 | 1.00 | 28.30 | A |
| ATOM | 1089 | CA | VAL | A | 165 | 13.209 | 27.990 | 52.290 | 1.00 | 29.64 | A |
| ATOM | 1090 | CB | VAL | A | 165 | 14.168 | 28.302 | 53.433 | 1.00 | 30.05 | A |
| ATOM | 1091 | CG1 | VAL | A | 165 | 14.698 | 27.044 | 53.998 | 1.00 | 30.86 | A |
| ATOM | 1092 | CG2 | VAL | A | 165 | 15.289 | 29.191 | 52.947 | 1.00 | 29.52 | A |
| ATOM | 1093 | C | VAL | A | 165 | 12.718 | 29.315 | 51.731 | 1.00 | 30.11 | A |
| ATOM | 1094 | O | VAL | A | 165 | 12.843 | 29.577 | 50.562 | 1.00 | 28.32 | A |
| ATOM | 1095 | N | ALA | A | 166 | 12.156 | 30.150 | 52.582 | 1.00 | 30.86 | A |
| ATOM | 1096 | CA | ALA | A | 166 | 11.677 | 31.441 | 52.135 | 1.00 | 31.10 | A |
| ATOM | 1097 | CB | ALA | A | 166 | 12.841 | 32.353 | 51.877 | 1.00 | 31.57 | A |
| ATOM | 1098 | C | ALA | A | 166 | 10.798 | 31.379 | 50.894 | 1.00 | 31.57 | A |
| ATOM | 1099 | O | ALA | A | 166 | 10.937 | 32.185 | 50.022 | 1.00 | 33.38 | A |
| ATOM | 1100 | N | GLY | A | 167 | 9.895 | 30.411 | 50.826 | 1.00 | 30.78 | A |
| ATOM | 1101 | CA | GLY | A | 167 | 9.007 | 30.335 | 49.685 | 1.00 | 30.10 | A |
| ATOM | 1102 | C | GLY | A | 167 | 9.682 | 29.832 | 48.445 | 1.00 | 30.19 | A |
| ATOM | 1103 | O | GLY | A | 167 | 9.332 | 30.232 | 47.349 | 1.00 | 29.55 | A |
| ATOM | 1104 | N | GLY | A | 168 | 10.660 | 28.951 | 48.639 | 1.00 | 28.61 | A |
| ATOM | 1105 | CA | GLY | A | 168 | 11.398 | 28.363 | 47.531 | 1.00 | 28.79 | A |
| ATOM | 1106 | C | GLY | A | 168 | 12.444 | 29.280 | 46.943 | 1.00 | 28.36 | A |
| ATOM | 1107 | O | GLY | A | 168 | 13.174 | 28.903 | 46.039 | 1.00 | 29.58 | A |
| ATOM | 1108 | N | THR | A | 169 | 12.495 | 30.491 | 47.484 | 1.00 | 27.54 | A |
| ATOM | 1109 | CA | THR | A | 169 | 13.404 | 31.550 | 47.071 | 1.00 | 27.84 | A |
| ATOM | 1110 | CB | THR | A | 169 | 12.971 | 32.856 | 47.734 | 1.00 | 28.85 | A |
| ATOM | 1111 | OG1 | THR | A | 169 | 12.120 | 33.563 | 46.841 | 1.00 | 33.06 | A |
| ATOM | 1112 | CG2 | THR | A | 169 | 14.144 | 33.711 | 48.111 | 1.00 | 27.60 | A |
| ATOM | 1113 | C | THR | A | 169 | 14.871 | 31.289 | 47.374 | 1.00 | 27.12 | A |
| ATOM | 1114 | O | THR | A | 169 | 15.702 | 31.395 | 46.501 | 1.00 | 26.63 | A |
| ATOM | 1115 | N | VAL | A | 170 | 15.170 | 30.963 | 48.627 | 1.00 | 25.15 | A |
| ATOM | 1116 | CA | VAL | A | 170 | 16.529 | 30.691 | 49.065 | 1.00 | 24.92 | A |
| ATOM | 1117 | CB | VAL | A | 170 | 16.774 | 31.232 | 50.457 | 1.00 | 24.75 | A |
| ATOM | 1118 | CG1 | VAL | A | 170 | 18.134 | 30.844 | 50.917 | 1.00 | 25.52 | A |
| ATOM | 1119 | CG2 | VAL | A | 170 | 16.625 | 32.727 | 50.458 | 1.00 | 26.38 | A |
| ATOM | 1120 | C | VAL | A | 170 | 16.777 | 29.204 | 49.107 | 1.00 | 23.93 | A |
| ATOM | 1121 | O | VAL | A | 170 | 15.883 | 28.436 | 49.366 | 1.00 | 21.51 | A |
| ATOM | 1122 | N | LEU | A | 171 | 18.017 | 28.808 | 48.872 | 1.00 | 23.28 | A |
| ATOM | 1123 | CA | LEU | A | 171 | 18.378 | 27.406 | 48.867 | 1.00 | 23.24 | A |
| ATOM | 1124 | CB | LEU | A | 171 | 18.852 | 27.039 | 47.496 | 1.00 | 23.08 | A |
| ATOM | 1125 | CG | LEU | A | 171 | 19.243 | 25.640 | 47.243 | 1.00 | 23.96 | A |
| ATOM | 1126 | CD1 | LEU | A | 171 | 18.609 | 25.174 | 45.986 | 1.00 | 25.10 | A |
| ATOM | 1127 | CD2 | LEU | A | 171 | 20.740 | 25.551 | 47.141 | 1.00 | 24.26 | A |
| ATOM | 1128 | C | LEU | A | 171 | 19.464 | 27.130 | 49.900 | 1.00 | 23.29 | A |
| ATOM | 1129 | O | LEU | A | 171 | 20.241 | 27.994 | 50.232 | 1.00 | 21.95 | A |
| ATOM | 1130 | N | LYS | A | 172 | 19.505 | 25.909 | 50.410 | 1.00 | 24.47 | A |
| ATOM | 1131 | CA | LYS | A | 172 | 20.503 | 25.551 | 51.401 | 1.00 | 25.22 | A |

```
ATOM   1132  CB   LYS A 172     19.905  25.634  52.795  1.00 25.55        A
ATOM   1133  CG   LYS A 172     19.947  27.021  53.380  1.00 28.34        A
ATOM   1134  CD   LYS A 172     19.132  27.132  54.641  1.00 28.01        A
ATOM   1135  CE   LYS A 172     19.179  28.558  55.126  1.00 30.64        A
ATOM   1136  NZ   LYS A 172     18.221  28.794  56.221  1.00 35.97        A
ATOM   1137  C    LYS A 172     20.997  24.144  51.141  1.00 25.69        A
ATOM   1138  O    LYS A 172     20.217  23.281  50.843  1.00 25.55        A
ATOM   1139  N    ILE A 173     22.306  23.936  51.259  1.00 27.47        A
ATOM   1140  CA   ILE A 173     22.912  22.625  51.039  1.00 28.63        A
ATOM   1141  CB   ILE A 173     24.363  22.758  50.627  1.00 28.14        A
ATOM   1142  CG2  ILE A 173     24.873  21.456  50.166  1.00 28.64        A
ATOM   1143  CG1  ILE A 173     24.507  23.733  49.566  1.00 28.39        A
ATOM   1144  CD1  ILE A 173     25.948  24.024  49.252  1.00 31.31        A
ATOM   1145  C    ILE A 173     22.917  21.798  52.315  1.00 29.25        A
ATOM   1146  O    ILE A 173     22.952  22.344  53.394  1.00 26.11        A
ATOM   1147  N    CYS A 174     22.909  20.478  52.191  1.00 31.38        A
ATOM   1148  CA   CYS A 174     22.945  19.641  53.377  1.00 34.77        A
ATOM   1149  CB   CYS A 174     21.655  19.705  54.076  1.00 34.93        A
ATOM   1150  SG   CYS A 174     20.421  18.726  53.279  1.00 42.51        A
ATOM   1151  C    CYS A 174     23.243  18.204  53.000  1.00 36.00        A
ATOM   1152  O    CYS A 174     23.211  17.869  51.848  1.00 34.96        A
ATOM   1153  N    ASP A 175     23.556  17.375  53.999  1.00 38.41        A
ATOM   1154  CA   ASP A 175     23.845  15.948  53.809  1.00 39.94        A
ATOM   1155  CB   ASP A 175     22.698  15.265  53.100  1.00 41.62        A
ATOM   1156  CG   ASP A 175     22.070  14.166  53.937  1.00 47.23        A
ATOM   1157  OD1  ASP A 175     22.102  14.276  55.185  1.00 54.73        A
ATOM   1158  OD2  ASP A 175     21.527  13.202  53.354  1.00 54.72        A
ATOM   1159  C    ASP A 175     25.112  15.632  53.061  1.00 41.52        A
ATOM   1160  O    ASP A 175     25.093  14.814  52.163  1.00 41.29        A
ATOM   1161  N    PHE A 176     26.214  16.267  53.452  1.00 44.20        A
ATOM   1162  CA   PHE A 176     27.493  16.025  52.793  1.00 47.82        A
ATOM   1163  CB   PHE A 176     28.662  16.736  53.553  1.00 48.11        A
ATOM   1164  CG   PHE A 176     28.295  18.079  54.131  1.00 49.61        A
ATOM   1165  CD1  PHE A 176     27.398  18.918  53.483  1.00 49.98        A
ATOM   1166  CD2  PHE A 176     28.871  18.519  55.323  1.00 51.31        A
ATOM   1167  CE1  PHE A 176     27.081  20.175  54.019  1.00 49.97        A
ATOM   1168  CE2  PHE A 176     28.558  19.778  55.862  1.00 49.51        A
ATOM   1169  CZ   PHE A 176     27.661  20.603  55.207  1.00 49.78        A
ATOM   1170  C    PHE A 176     27.762  14.520  52.720  1.00 49.06        A
ATOM   1171  O    PHE A 176     27.425  13.775  53.659  1.00 49.67        A
ATOM   1172  N    GLY A 177     28.342  14.072  51.601  1.00 49.95        A
ATOM   1173  CA   GLY A 177     28.666  12.660  51.434  1.00 51.31        A
ATOM   1174  C    GLY A 177     29.637  12.284  52.542  1.00 52.72        A
ATOM   1175  O    GLY A 177     29.652  11.151  53.030  1.00 52.78        A
ATOM   1176  N    THR A 178     30.447  13.272  52.926  1.00 54.16        A
ATOM   1177  CA   THR A 178     31.440  13.165  53.993  1.00 54.71        A
ATOM   1178  CB   THR A 178     32.720  12.389  53.547  1.00 55.90        A
ATOM   1179  OG1  THR A 178     32.388  11.021  53.253  1.00 58.13        A
ATOM   1180  CG2  THR A 178     33.791  12.419  54.674  1.00 55.08        A
ATOM   1181  C    THR A 178     31.829  14.597  54.348  1.00 55.66        A
ATOM   1182  O    THR A 178     31.333  15.551  53.737  1.00 55.14        A
ATOM   1183  N    GLY A 191     23.632  10.052  46.299  1.00 45.03        A
ATOM   1184  CA   GLY A 191     24.207  10.787  45.188  1.00 43.71        A
ATOM   1185  C    GLY A 191     24.446   9.911  43.985  1.00 42.34        A
```

| ATOM | 1186 | O | GLY A 191 | 25.580 | 9.694 | 43.566 | 1.00 | 43.95 | A |
|------|------|------|-----------|--------|-------|--------|------|-------|---|
| ATOM | 1187 | N | SER A 192 | 23.349 | 9.414 | 43.429 | 1.00 | 40.98 | A |
| ATOM | 1188 | CA | SER A 192 | 23.395 | 8.527 | 42.280 | 1.00 | 38.26 | A |
| ATOM | 1189 | CB | SER A 192 | 22.031 | 8.474 | 41.597 | 1.00 | 38.92 | A |
| ATOM | 1190 | OG | SER A 192 | 20.993 | 8.415 | 42.563 | 1.00 | 39.75 | A |
| ATOM | 1191 | C | SER A 192 | 24.470 | 8.886 | 41.268 | 1.00 | 35.79 | A |
| ATOM | 1192 | O | SER A 192 | 25.051 | 9.972 | 41.287 | 1.00 | 34.95 | A |
| ATOM | 1193 | N | ALA A 193 | 24.714 | 7.935 | 40.379 | 1.00 | 33.17 | A |
| ATOM | 1194 | CA | ALA A 193 | 25.726 | 8.061 | 39.354 | 1.00 | 31.38 | A |
| ATOM | 1195 | CB | ALA A 193 | 25.896 | 6.711 | 38.638 | 1.00 | 31.51 | A |
| ATOM | 1196 | C | ALA A 193 | 25.424 | 9.152 | 38.351 | 1.00 | 29.92 | A |
| ATOM | 1197 | O | ALA A 193 | 26.260 | 9.462 | 37.518 | 1.00 | 28.56 | A |
| ATOM | 1198 | N | ALA A 194 | 24.241 | 9.745 | 38.436 | 1.00 | 28.95 | A |
| ATOM | 1199 | CA | ALA A 194 | 23.884 | 10.774 | 37.481 | 1.00 | 29.45 | A |
| ATOM | 1200 | CB | ALA A 194 | 22.430 | 11.053 | 37.564 | 1.00 | 30.05 | A |
| ATOM | 1201 | C | ALA A 194 | 24.675 | 12.060 | 37.652 | 1.00 | 28.28 | A |
| ATOM | 1202 | O | ALA A 194 | 25.100 | 12.671 | 36.653 | 1.00 | 28.32 | A |
| ATOM | 1203 | N | TRP A 195 | 24.897 | 12.462 | 38.902 | 1.00 | 28.07 | A |
| ATOM | 1204 | CA | TRP A 195 | 25.627 | 13.698 | 39.204 | 1.00 | 29.49 | A |
| ATOM | 1205 | CB | TRP A 195 | 24.946 | 14.417 | 40.298 | 1.00 | 29.10 | A |
| ATOM | 1206 | CG | TRP A 195 | 23.545 | 14.726 | 40.011 | 1.00 | 28.09 | A |
| ATOM | 1207 | CD2 | TRP A 195 | 22.412 | 13.898 | 40.280 | 1.00 | 28.89 | A |
| ATOM | 1208 | CE2 | TRP A 195 | 21.272 | 14.598 | 39.845 | 1.00 | 29.07 | A |
| ATOM | 1209 | CE3 | TRP A 195 | 22.248 | 12.632 | 40.846 | 1.00 | 29.14 | A |
| ATOM | 1210 | CD1 | TRP A 195 | 23.067 | 15.856 | 39.442 | 1.00 | 27.90 | A |
| ATOM | 1211 | NE1 | TRP A 195 | 21.699 | 15.797 | 39.340 | 1.00 | 27.40 | A |
| ATOM | 1212 | CZ2 | TRP A 195 | 19.982 | 14.074 | 39.954 | 1.00 | 28.78 | A |
| ATOM | 1213 | CZ3 | TRP A 195 | 20.962 | 12.112 | 40.957 | 1.00 | 29.66 | A |
| ATOM | 1214 | CH2 | TRP A 195 | 19.848 | 12.834 | 40.510 | 1.00 | 30.81 | A |
| ATOM | 1215 | C | TRP A 195 | 27.088 | 13.516 | 39.615 | 1.00 | 29.65 | A |
| ATOM | 1216 | O | TRP A 195 | 27.732 | 14.479 | 40.018 | 1.00 | 28.54 | A |
| ATOM | 1217 | N | MET A 196 | 27.607 | 12.295 | 39.513 | 1.00 | 30.44 | A |
| ATOM | 1218 | CA | MET A 196 | 28.978 | 12.022 | 39.917 | 1.00 | 32.65 | A |
| ATOM | 1219 | CB | MET A 196 | 29.121 | 10.598 | 40.411 | 1.00 | 33.00 | A |
| ATOM | 1220 | CG | MET A 196 | 28.312 | 10.271 | 41.642 | 1.00 | 38.75 | A |
| ATOM | 1221 | SD | MET A 196 | 29.103 | 9.004 | 42.636 | 1.00 | 46.01 | A |
| ATOM | 1222 | CE | MET A 196 | 28.867 | 7.580 | 41.609 | 1.00 | 48.99 | A |
| ATOM | 1223 | C | MET A 196 | 30.002 | 12.237 | 38.838 | 1.00 | 29.12 | A |
| ATOM | 1224 | O | MET A 196 | 29.823 | 11.816 | 37.739 | 1.00 | 26.56 | A |
| ATOM | 1225 | N | ALA A 197 | 31.091 | 12.899 | 39.199 | 1.00 | 28.04 | A |
| ATOM | 1226 | CA | ALA A 197 | 32.186 | 13.147 | 38.277 | 1.00 | 28.06 | A |
| ATOM | 1227 | CB | ALA A 197 | 33.266 | 14.000 | 38.979 | 1.00 | 25.99 | A |
| ATOM | 1228 | C | ALA A 197 | 32.743 | 11.770 | 37.903 | 1.00 | 28.75 | A |
| ATOM | 1229 | O | ALA A 197 | 32.887 | 10.916 | 38.745 | 1.00 | 28.97 | A |
| ATOM | 1230 | N | PRO A 198 | 33.069 | 11.543 | 36.630 | 1.00 | 29.20 | A |
| ATOM | 1231 | CD | PRO A 198 | 33.042 | 12.403 | 35.444 | 1.00 | 28.86 | A |
| ATOM | 1232 | CA | PRO A 198 | 33.591 | 10.220 | 36.301 | 1.00 | 31.11 | A |
| ATOM | 1233 | CB | PRO A 198 | 33.895 | 10.327 | 34.800 | 1.00 | 31.21 | A |
| ATOM | 1234 | CG | PRO A 198 | 34.093 | 11.775 | 34.584 | 1.00 | 30.84 | A |
| ATOM | 1235 | C | PRO A 198 | 34.796 | 9.763 | 37.128 | 1.00 | 32.33 | A |
| ATOM | 1236 | O | PRO A 198 | 34.838 | 8.637 | 37.585 | 1.00 | 32.35 | A |
| ATOM | 1237 | N | GLU A 199 | 35.768 | 10.636 | 37.340 | 1.00 | 33.02 | A |
| ATOM | 1238 | CA | GLU A 199 | 36.931 | 10.222 | 38.101 | 1.00 | 34.60 | A |
| ATOM | 1239 | CB | GLU A 199 | 37.868 | 11.414 | 38.410 | 1.00 | 34.37 | A |

| ATOM | 1240 | CG   | GLU | A | 199 | 37.252 | 12.488 | 39.260 | 1.00 | 32.90 | A |
| ATOM | 1241 | CD   | GLU | A | 199 | 36.708 | 13.643 | 38.444 | 1.00 | 28.99 | A |
| ATOM | 1242 | OE1  | GLU | A | 199 | 36.366 | 13.448 | 37.261 | 1.00 | 24.57 | A |
| ATOM | 1243 | OE2  | GLU | A | 199 | 36.605 | 14.756 | 38.992 | 1.00 | 26.70 | A |
| ATOM | 1244 | C    | GLU | A | 199 | 36.504 | 9.566  | 39.396 | 1.00 | 36.38 | A |
| ATOM | 1245 | O    | GLU | A | 199 | 37.257 | 8.807  | 39.969 | 1.00 | 36.52 | A |
| ATOM | 1246 | N    | VAL | A | 200 | 35.283 | 9.841  | 39.843 | 1.00 | 38.42 | A |
| ATOM | 1247 | CA   | VAL | A | 200 | 34.812 | 9.270  | 41.101 | 1.00 | 40.07 | A |
| ATOM | 1248 | CB   | VAL | A | 200 | 33.672 | 10.102 | 41.691 | 1.00 | 40.12 | A |
| ATOM | 1249 | CG1  | VAL | A | 200 | 33.174 | 9.469  | 42.943 | 1.00 | 40.62 | A |
| ATOM | 1250 | CG2  | VAL | A | 200 | 34.163 | 11.493 | 41.982 | 1.00 | 38.63 | A |
| ATOM | 1251 | C    | VAL | A | 200 | 34.395 | 7.802  | 41.055 | 1.00 | 42.33 | A |
| ATOM | 1252 | O    | VAL | A | 200 | 34.747 | 7.053  | 41.967 | 1.00 | 42.05 | A |
| ATOM | 1253 | N    | PHE | A | 201 | 33.651 | 7.377  | 40.027 | 1.00 | 44.58 | A |
| ATOM | 1254 | CA   | PHE | A | 201 | 33.265 | 5.965  | 39.966 | 1.00 | 45.74 | A |
| ATOM | 1255 | CB   | PHE | A | 201 | 32.234 | 5.589  | 38.825 | 1.00 | 45.88 | A |
| ATOM | 1256 | CG   | PHE | A | 201 | 31.703 | 6.736  | 38.022 | 1.00 | 43.94 | A |
| ATOM | 1257 | CD1  | PHE | A | 201 | 30.908 | 7.712  | 38.605 | 1.00 | 44.38 | A |
| ATOM | 1258 | CD2  | PHE | A | 201 | 31.906 | 6.774  | 36.642 | 1.00 | 43.44 | A |
| ATOM | 1259 | CE1  | PHE | A | 201 | 30.312 | 8.708  | 37.823 | 1.00 | 45.31 | A |
| ATOM | 1260 | CE2  | PHE | A | 201 | 31.317 | 7.760  | 35.856 | 1.00 | 41.94 | A |
| ATOM | 1261 | CZ   | PHE | A | 201 | 30.517 | 8.728  | 36.445 | 1.00 | 44.16 | A |
| ATOM | 1262 | C    | PHE | A | 201 | 34.505 | 5.149  | 39.702 | 1.00 | 48.97 | A |
| ATOM | 1263 | O    | PHE | A | 201 | 34.626 | 4.037  | 40.192 | 1.00 | 50.74 | A |
| ATOM | 1264 | N    | GLU | A | 202 | 35.412 | 5.714  | 38.905 | 1.00 | 51.86 | A |
| ATOM | 1265 | CA   | GLU | A | 202 | 36.661 | 5.049  | 38.552 | 1.00 | 54.43 | A |
| ATOM | 1266 | CB   | GLU | A | 202 | 37.366 | 5.815  | 37.362 | 1.00 | 53.69 | A |
| ATOM | 1267 | CG   | GLU | A | 202 | 38.590 | 6.653  | 37.723 | 1.00 | 56.65 | A |
| ATOM | 1268 | CD   | GLU | A | 202 | 39.181 | 7.376  | 36.519 | 1.00 | 56.43 | A |
| ATOM | 1269 | OE1  | GLU | A | 202 | 40.263 | 7.990  | 36.661 | 1.00 | 56.72 | A |
| ATOM | 1270 | OE2  | GLU | A | 202 | 38.558 | 7.333  | 35.433 | 1.00 | 59.88 | A |
| ATOM | 1271 | C    | GLU | A | 202 | 37.579 | 4.932  | 39.767 | 1.00 | 55.02 | A |
| ATOM | 1272 | O    | GLU | A | 202 | 38.774 | 4.655  | 39.640 | 1.00 | 56.38 | A |
| ATOM | 1273 | N    | GLY | A | 203 | 36.989 | 5.130  | 40.946 | 1.00 | 56.06 | A |
| ATOM | 1274 | CA   | GLY | A | 203 | 37.719 | 5.034  | 42.199 | 1.00 | 56.30 | A |
| ATOM | 1275 | C    | GLY | A | 203 | 38.797 | 6.086  | 42.356 | 1.00 | 56.62 | A |
| ATOM | 1276 | O    | GLY | A | 203 | 38.928 | 6.696  | 43.425 | 1.00 | 58.01 | A |
| ATOM | 1277 | N    | SER | A | 204 | 39.557 | 6.287  | 41.280 | 1.00 | 56.53 | A |
| ATOM | 1278 | CA   | SER | A | 204 | 40.658 | 7.243  | 41.217 | 1.00 | 56.68 | A |
| ATOM | 1279 | CB   | SER | A | 204 | 40.839 | 7.744  | 39.758 | 1.00 | 56.89 | A |
| ATOM | 1280 | OG   | SER | A | 204 | 40.012 | 8.864  | 39.485 | 1.00 | 59.28 | A |
| ATOM | 1281 | C    | SER | A | 204 | 40.565 | 8.446  | 42.155 | 1.00 | 55.85 | A |
| ATOM | 1282 | O    | SER | A | 204 | 39.510 | 8.759  | 42.728 | 1.00 | 56.21 | A |
| ATOM | 1283 | N    | ASN | A | 205 | 41.696 | 9.120  | 42.309 | 1.00 | 55.05 | A |
| ATOM | 1284 | CA   | ASN | A | 205 | 41.768 | 10.284 | 43.169 | 1.00 | 54.90 | A |
| ATOM | 1285 | CB   | ASN | A | 205 | 43.227 | 10.705 | 43.318 | 1.00 | 55.24 | A |
| ATOM | 1286 | CG   | ASN | A | 205 | 44.111 | 9.567  | 43.784 | 1.00 | 58.74 | A |
| ATOM | 1287 | OD1  | ASN | A | 205 | 43.930 | 9.033  | 44.874 | 1.00 | 62.91 | A |
| ATOM | 1288 | ND2  | ASN | A | 205 | 45.074 | 9.189  | 42.951 | 1.00 | 60.97 | A |
| ATOM | 1289 | C    | ASN | A | 205 | 40.931 | 11.426 | 42.583 | 1.00 | 52.10 | A |
| ATOM | 1290 | O    | ASN | A | 205 | 40.761 | 11.518 | 41.372 | 1.00 | 52.30 | A |
| ATOM | 1291 | N    | TYR | A | 206 | 40.400 | 12.282 | 43.452 | 1.00 | 48.43 | A |
| ATOM | 1292 | CA   | TYR | A | 206 | 39.586 | 13.410 | 43.010 | 1.00 | 44.80 | A |
| ATOM | 1293 | CB   | TYR | A | 206 | 38.103 | 13.001 | 42.939 | 1.00 | 45.60 | A |

| ATOM | 1294 | CG | TYR A 206 | 37.518 | 12.558 | 44.257 | 1.00 | 45.42 | A |
|------|------|------|-----------|--------|--------|--------|------|-------|---|
| ATOM | 1295 | CD1 | TYR A 206 | 37.254 | 13.479 | 45.279 | 1.00 | 44.21 | A |
| ATOM | 1296 | CE1 | TYR A 206 | 36.723 | 13.069 | 46.497 | 1.00 | 45.64 | A |
| ATOM | 1297 | CD2 | TYR A 206 | 37.235 | 11.214 | 44.488 | 1.00 | 46.95 | A |
| ATOM | 1298 | CE2 | TYR A 206 | 36.702 | 10.789 | 45.704 | 1.00 | 47.23 | A |
| ATOM | 1299 | CZ | TYR A 206 | 36.449 | 11.718 | 46.701 | 1.00 | 48.47 | A |
| ATOM | 1300 | OH | TYR A 206 | 35.930 | 11.271 | 47.892 | 1.00 | 47.19 | A |
| ATOM | 1301 | C | TYR A 206 | 39.752 | 14.619 | 43.933 | 1.00 | 43.54 | A |
| ATOM | 1302 | O | TYR A 206 | 40.382 | 14.521 | 45.007 | 1.00 | 43.88 | A |
| ATOM | 1303 | N | SER A 207 | 39.174 | 15.749 | 43.531 | 1.00 | 40.47 | A |
| ATOM | 1304 | CA | SER A 207 | 39.292 | 16.972 | 44.312 | 1.00 | 37.57 | A |
| ATOM | 1305 | CB | SER A 207 | 40.262 | 17.915 | 43.623 | 1.00 | 38.24 | A |
| ATOM | 1306 | OG | SER A 207 | 39.915 | 18.062 | 42.253 | 1.00 | 36.27 | A |
| ATOM | 1307 | C | SER A 207 | 37.963 | 17.677 | 44.497 | 1.00 | 35.39 | A |
| ATOM | 1308 | O | SER A 207 | 36.914 | 17.083 | 44.396 | 1.00 | 34.46 | A |
| ATOM | 1309 | N | GLU A 208 | 38.037 | 18.968 | 44.779 | 1.00 | 32.41 | A |
| ATOM | 1310 | CA | GLU A 208 | 36.849 | 19.763 | 44.964 | 1.00 | 31.48 | A |
| ATOM | 1311 | CB | GLU A 208 | 37.197 | 21.160 | 45.450 | 1.00 | 32.14 | A |
| ATOM | 1312 | CG | GLU A 208 | 38.492 | 21.296 | 46.260 | 1.00 | 36.87 | A |
| ATOM | 1313 | CD | GLU A 208 | 39.719 | 20.910 | 45.463 | 1.00 | 43.90 | A |
| ATOM | 1314 | OE1 | GLU A 208 | 39.699 | 21.103 | 44.229 | 1.00 | 44.13 | A |
| ATOM | 1315 | OE2 | GLU A 208 | 40.704 | 20.426 | 46.066 | 1.00 | 41.87 | A |
| ATOM | 1316 | C | GLU A 208 | 36.194 | 19.853 | 43.609 | 1.00 | 28.30 | A |
| ATOM | 1317 | O | GLU A 208 | 35.027 | 19.959 | 43.504 | 1.00 | 27.66 | A |
| ATOM | 1318 | N | LYS A 209 | 36.993 | 19.803 | 42.559 | 1.00 | 25.85 | A |
| ATOM | 1319 | CA | LYS A 209 | 36.451 | 19.863 | 41.215 | 1.00 | 24.75 | A |
| ATOM | 1320 | CB | LYS A 209 | 37.555 | 19.754 | 40.204 | 1.00 | 24.10 | A |
| ATOM | 1321 | CG | LYS A 209 | 38.502 | 20.949 | 40.166 | 1.00 | 21.36 | A |
| ATOM | 1322 | CD | LYS A 209 | 37.812 | 22.198 | 39.661 | 1.00 | 21.70 | A |
| ATOM | 1323 | CE | LYS A 209 | 38.712 | 23.396 | 39.741 | 1.00 | 22.33 | A |
| ATOM | 1324 | NZ | LYS A 209 | 39.939 | 23.226 | 38.912 | 1.00 | 21.10 | A |
| ATOM | 1325 | C | LYS A 209 | 35.492 | 18.700 | 41.043 | 1.00 | 23.79 | A |
| ATOM | 1326 | O | LYS A 209 | 35.120 | 18.353 | 39.947 | 1.00 | 20.62 | A |
| ATOM | 1327 | N | CYS A 210 | 35.130 | 18.081 | 42.156 | 1.00 | 22.59 | A |
| ATOM | 1328 | CA | CYS A 210 | 34.200 | 16.975 | 42.160 | 1.00 | 24.33 | A |
| ATOM | 1329 | CB | CYS A 210 | 34.350 | 16.248 | 43.358 | 1.00 | 23.21 | A |
| ATOM | 1330 | SG | CYS A 210 | 33.842 | 14.601 | 43.135 | 1.00 | 43.31 | A |
| ATOM | 1331 | C | CYS A 210 | 32.793 | 17.547 | 42.117 | 1.00 | 20.48 | A |
| ATOM | 1332 | O | CYS A 210 | 31.951 | 17.127 | 41.332 | 1.00 | 20.18 | A |
| ATOM | 1333 | N | ASP A 211 | 32.563 | 18.528 | 42.980 | 1.00 | 19.77 | A |
| ATOM | 1334 | CA | ASP A 211 | 31.285 | 19.204 | 43.093 | 1.00 | 19.51 | A |
| ATOM | 1335 | CB | ASP A 211 | 31.275 | 20.066 | 44.340 | 1.00 | 19.94 | A |
| ATOM | 1336 | CG | ASP A 211 | 31.672 | 19.276 | 45.579 | 1.00 | 20.95 | A |
| ATOM | 1337 | OD1 | ASP A 211 | 31.271 | 18.087 | 45.676 | 1.00 | 18.72 | A |
| ATOM | 1338 | OD2 | ASP A 211 | 32.376 | 19.829 | 46.452 | 1.00 | 22.55 | A |
| ATOM | 1339 | C | ASP A 211 | 31.007 | 20.037 | 41.865 | 1.00 | 18.66 | A |
| ATOM | 1340 | O | ASP A 211 | 29.865 | 20.170 | 41.449 | 1.00 | 18.66 | A |
| ATOM | 1341 | N | VAL A 212 | 32.055 | 20.585 | 41.268 | 1.00 | 18.09 | A |
| ATOM | 1342 | CA | VAL A 212 | 31.864 | 21.401 | 40.073 | 1.00 | 17.95 | A |
| ATOM | 1343 | CB | VAL A 212 | 33.215 | 21.963 | 39.523 | 1.00 | 18.45 | A |
| ATOM | 1344 | CG1 | VAL A 212 | 33.004 | 22.638 | 38.206 | 1.00 | 18.40 | A |
| ATOM | 1345 | CG2 | VAL A 212 | 33.793 | 22.953 | 40.483 | 1.00 | 19.88 | A |
| ATOM | 1346 | C | VAL A 212 | 31.159 | 20.563 | 38.993 | 1.00 | 16.40 | A |
| ATOM | 1347 | O | VAL A 212 | 30.206 | 21.021 | 38.349 | 1.00 | 15.94 | A |

```
ATOM   1348  N    PHE A 213       31.595  19.328  38.803  1.00  16.19      A
ATOM   1349  CA   PHE A 213       30.963  18.522  37.788  1.00  16.49      A
ATOM   1350  CB   PHE A 213       31.578  17.231  37.737  1.00  17.38      A
ATOM   1351  CG   PHE A 213  .  . 31.010  16.337  36.687  1.00  18.05      A
ATOM   1352  CD1  PHE A 213       29.811  15.707  36.879  1.00  16.85      A
ATOM   1353  CD2  PHE A 213       31.691  16.119  35.509  1.00  17.59      A
ATOM   1354  CE1  PHE A 213       29.306  14.874  35.919  1.00  18.96      A
ATOM   1355  CE2  PHE A 213       31.189  15.290  34.548  1.00  19.03      A
ATOM   1356  CZ   PHE A 213       30.001  14.667  34.749  1.00  19.34      A
ATOM   1357  C    PHE A 213       29.495  18.365  38.097  1.00  16.24      A
ATOM   1358  O    PHE A 213       28.680  18.363  37.207  1.00  17.47      A
ATOM   1359  N    SER A 214       29.174  18.237  39.381  1.00  16.83      A
ATOM   1360  CA   SER A 214       27.795  18.057  39.816  1.00  16.68      A
ATOM   1361  CB   SER A 214       27.756  17.767  41.292  1.00  15.99      A
ATOM   1362  OG   SER A 214       28.249  16.469  41.556  1.00  22.18      A
ATOM   1363  C    SER A 214       26.988  19.299  39.509  1.00  15.70      A
ATOM   1364  O    SER A 214       25.950  19.256  38.842  1.00  14.24      A
ATOM   1365  N    TRP A 215       27.479  20.417  39.998  1.00  16.19      A
ATOM   1366  CA   TRP A 215       26.806  21.671  39.769  1.00  15.15      A
ATOM   1367  CB   TRP A 215       27.693  22.729  40.102  1.00  16.54      A
ATOM   1368  CG   TRP A 215       27.058  24.048  40.199  1.00  16.09      A
ATOM   1369  CD2  TRP A 215       26.929  24.991  39.147  1.00  13.61      A
ATOM   1370  CE2  TRP A 215       26.335  26.145  39.689  1.00  17.04      A
ATOM   1371  CE3  TRP A 215       27.262  24.975  37.792  1.00  16.28      A
ATOM   1372  CD1  TRP A 215       26.547  24.636  41.310  1.00  13.58      A
ATOM   1373  NE1  TRP A 215       26.113  25.906  41.015  1.00  16.08      A
ATOM   1374  CZ2  TRP A 215       26.065  27.262  38.933  1.00  16.99.     A
ATOM   1375  CZ3  TRP A 215       26.993  26.088  37.043  1.00  14.42      A
ATOM   1376  CH2  TRP A 215       26.398  27.222  37.615  1.00  14.62      A
ATOM   1377  C    TRP A 215       26.439  21.754  38.302  1.00  14.59      A
ATOM   1378  O    TRP A 215       25.330  22.059  37.952  1.00  14.22      A
ATOM   1379  N    GLY A 216       27.406  21.462  37.448  1.00  15.02      A
ATOM   1380  CA   GLY A 216       27.162  21.506  36.020  1.00  13.94      A
ATOM   1381  C    GLY A 216       25.895  20.770  35.656  1.00  14.42      A
ATOM   1382  O    GLY A 216       25.056  21.326  35.007  1.00  14.55      A
ATOM   1383  N    ILE A 217       25.765  19.517  36.088  1.00  16.59      A
ATOM   1384  CA   ILE A 217       24.568  18.732  35.806  1.00  17.45      A
ATOM   1385  CB   ILE A 217       24.720  17.310  36.319  1.00  17.32      A
ATOM   1386  CG2  ILE A 217       23.479  16.488  35.986  1.00  18.12      A
ATOM   1387  CG1  ILE A 217       25.940  16.694  35.707  1.00  16.16      A
ATOM   1388  CD1  ILE A 217       25.817  16.447  34.247  1.00  14.13      A
ATOM   1389  C    ILE A 217       23.338  19.357  36.466  1.00  15.92      A
ATOM   1390  O    ILE A 217       22.299  19.531  35.831  1.00  15.75      A
ATOM   1391  N    ILE A 218       23.442  19.700  37.744  1.00  17.62      A
ATOM   1392  CA   ILE A 218       22.296  20.310  38.411  1.00  17.65      A
ATOM   1393  CB   ILE A 218       22.619  20.719  39.869  1.00  19.26      A
ATOM   1394  CG2  ILE A 218       21.402  21.310  40.531  1.00  19.62      A
ATOM   1395  CG1  ILE A 218       23.060  19.518  40.656  1.00  17.84      A
ATOM   1396  CD1  ILE A 218       23.662  19.863  41.976  1.00  20.17      A
ATOM   1397  C    ILE A 218       21.851  21.546  37.628  1.00  16.98      A
ATOM   1398  O    ILE A 218       20.695  21.880  37.651  1.00  13.90      A
ATOM   1399  N    LEU A 219       22.776  22.217  36.939  1.00  15.65      A
ATOM   1400  CA   LEU A 219       22.411  23.392  36.151  1.00  16.95      A
ATOM   1401  CB   LEU A 219       23.599  24.014  35.521  1.00  16.60      A
```

| ATOM | 1402 | CG | LEU | A | 219 | 23.323 | 25.283 | 34.756 | 1.00 | 16.00 | A |
|------|------|-----|-----|---|-----|--------|--------|--------|------|-------|---|
| ATOM | 1403 | CD1 | LEU | A | 219 | 22.295 | 26.087 | 35.454 | 1.00 | 18.10 | A |
| ATOM | 1404 | CD2 | LEU | A | 219 | 24.584 | 26.069 | 34.620 | 1.00 | 18.28 | A |
| ATOM | 1405 | C | LEU | A | 219 | 21.489 | 22.929 | 35.056 | 1.00 | 16.73 | A |
| ATOM | 1406 | O | LEU | A | 219 | 20.383 | 23.407 | 34.921 | 1.00 | 17.44 | A |
| ATOM | 1407 | N | TRP | A | 220 | 21.979 | 21.977 | 34.276 | 1.00 | 17.49 | A |
| ATOM | 1408 | CA | TRP | A | 220 | 21.229 | 21.402 | 33.180 | 1.00 | 17.57 | A |
| ATOM | 1409 | CB | TRP | A | 220 | 21.921 | 20.218 | 32.692 | 1.00 | 18.24 | A |
| ATOM | 1410 | CG | TRP | A | 220 | 21.224 | 19.523 | 31.580 | 1.00 | 15.53 | A |
| ATOM | 1411 | CD2 | TRP | A | 220 | 20.252 | 18.480 | 31.693 | 1.00 | 14.85 | A |
| ATOM | 1412 | CE2 | TRP | A | 220 | 19.846 | 18.146 | 30.382 | 1.00 | 17.69 | A |
| ATOM | 1413 | CE3 | TRP | A | 220 | 19.678 | 17.799 | 32.771 | 1.00 | 16.72 | A |
| ATOM | 1414 | CD1 | TRP | A | 220 | 21.364 | 19.771 | 30.246 | 1.00 | 17.52 | A |
| ATOM | 1415 | NE1 | TRP | A | 220 | 20.544 | 18.950 | 29.520 | 1.00 | 17.82 | A |
| ATOM | 1416 | CZ2 | TRP | A | 220 | 18.893 | 17.160 | 30.121 | 1.00 | 17.64 | A |
| ATOM | 1417 | CZ3 | TRP | A | 220 | 18.734 | 16.826 | 32.515 | 1.00 | 17.70 | A |
| ATOM | 1418 | CH2 | TRP | A | 220 | 18.350 | 16.515 | 31.199 | 1.00 | 18.97 | A |
| ATOM | 1419 | C | TRP | A | 220 | 19.865 | 20.981 | 33.656 | 1.00 | 17.35 | A |
| ATOM | 1420 | O | TRP | A | 220 | 18.892 | 21.188 | 32.995 | 1.00 | 17.23 | A |
| ATOM | 1421 | N | GLU | A | 221 | 19.827 | 20.383 | 34.837 | 1.00 | 19.26 | A |
| ATOM | 1422 | CA | GLU | A | 221 | 18.584 | 19.878 | 35.391 | 1.00 | 19.96 | A |
| ATOM | 1423 | CB | GLU | A | 221 | 18.845 | 19.068 | 36.621 | 1.00 | 19.71 | A |
| ATOM | 1424 | CG | GLU | A | 221 | 17.591 | 18.359 | 37.098 | 1.00 | 23.27 | A |
| ATOM | 1425 | CD | GLU | A | 221 | 17.873 | 17.334 | 38.157 | 1.00 | 28.38 | A |
| ATOM | 1426 | OE1 | GLU | A | 221 | 19.000 | 16.792 | 38.151 | 1.00 | 37.96 | A |
| ATOM | 1427 | OE2 | GLU | A | 221 | 16.967 | 17.062 | 38.975 | 1.00 | 32.14 | A |
| ATOM | 1428 | C | GLU | A | 221 | 17.496 | 20.868 | 35.707 | 1.00 | 18.17 | A |
| ATOM | 1429 | O | GLU | A | 221 | 16.354 | 20.589 | 35.478 | 1.00 | 18.03 | A |
| ATOM | 1430 | N | VAL | A | 222 | 17.849 | 22.022 | 36.251 | 1.00 | 17.39 | A |
| ATOM | 1431 | CA | VAL | A | 222 | 16.817 | 22.989 | 36.589 | 1.00 | 18.02 | A |
| ATOM | 1432 | CB | VAL | A | 222 | 17.324 | 24.011 | 37.647 | 1.00 | 18.16 | A |
| ATOM | 1433 | CG1 | VAL | A | 222 | 17.780 | 23.283 | 38.893 | 1.00 | 19.76 | A |
| ATOM | 1434 | CG2 | VAL | A | 222 | 18.442 | 24.832 | 37.090 | 1.00 | 20.45 | A |
| ATOM | 1435 | C | VAL | A | 222 | 16.294 | 23.717 | 35.354 | 1.00 | 18.02 | A |
| ATOM | 1436 | O | VAL | A | 222 | 15.135 | 24.034 | 35.270 | 1.00 | 18.20 | A |
| ATOM | 1437 | N | ILE | A | 223 | 17.177 | 23.945 | 34.391 | 1.00 | 17.43 | A |
| ATOM | 1438 | CA | ILE | A | 223 | 16.849 | 24.636 | 33.149 | 1.00 | 17.79 | A |
| ATOM | 1439 | CB | ILE | A | 223 | 18.149 | 25.004 | 32.388 | 1.00 | 17.59 | A |
| ATOM | 1440 | CG2 | ILE | A | 223 | 17.885 | 25.136 | 30.924 | 1.00 | 16.74 | A |
| ATOM | 1441 | CG1 | ILE | A | 223 | 18.760 | 26.266 | 32.969 | 1.00 | 17.55 | A |
| ATOM | 1442 | CD1 | ILE | A | 223 | 20.194 | 26.506 | 32.529 | 1.00 | 17.18 | A |
| ATOM | 1443 | C | ILE | A | 223 | 15.964 | 23.810 | 32.225 | 1.00 | 18.39 | A |
| ATOM | 1444 | O | ILE | A | 223 | 15.299 | 24.341 | 31.377 | 1.00 | 16.16 | A |
| ATOM | 1445 | N | THR | A | 224 | 15.977 | 22.496 | 32.405 | 1.00 | 19.53 | A |
| ATOM | 1446 | CA | THR | A | 224 | 15.189 | 21.590 | 31.577 | 1.00 | 20.23 | A |
| ATOM | 1447 | CB | THR | A | 224 | 16.076 | 20.512 | 30.985 | 1.00 | 19.87 | A |
| ATOM | 1448 | OG1 | THR | A | 224 | 16.773 | 19.845 | 32.043 | 1.00 | 19.07 | A |
| ATOM | 1449 | CG2 | THR | A | 224 | 17.080 | 21.112 | 30.042 | 1.00 | 19.08 | A |
| ATOM | 1450 | C | THR | A | 224 | 14.116 | 20.888 | 32.384 | 1.00 | 20.76 | A |
| ATOM | 1451 | O | THR | A | 224 | 13.231 | 20.289 | 31.843 | 1.00 | 20.92 | A |
| ATOM | 1452 | N | ARG | A | 225 | 14.222 | 20.989 | 33.699 | 1.00 | 22.04 | A |
| ATOM | 1453 | CA | ARG | A | 225 | 13.300 | 20.334 | 34.603 | 1.00 | 21.41 | A |
| ATOM | 1454 | CB | ARG | A | 225 | 11.930 | 20.982 | 34.557 | 1.00 | 22.02 | A |
| ATOM | 1455 | CG | ARG | A | 225 | 11.177 | 20.714 | 35.845 | 1.00 | 20.66 | A |

112

| ATOM | 1456 | CD | ARG | A | 225 | 9.946 | 21.561 | 36.044 | 1.00 | 20.47 | A |
|------|------|------|-----|---|-----|-------|--------|--------|------|-------|---|
| ATOM | 1457 | NE | ARG | A | 225 | 9.469 | 21.438 | 37.416 | 1.00 | 21.92 | A |
| ATOM | 1458 | CZ | ARG | A | 225 | 8.512 | 22.183 | 37.958 | 1.00 | 21.00 | A |
| ATOM | 1459 | NH1 | ARG | A | 225 | 7.903 | 23.126 | 37.252 | 1.00 | 21.05 | A |
| ATOM | 1460 | NH2 | ARG | A | 225 | 8.163 | 21.986 | 39.221 | 1.00 | 19.98 | A |
| ATOM | 1461 | C | ARG | A | 225 | 13.213 | 18.846 | 34.255 | 1.00 | 23.54 | A |
| ATOM | 1462 | O | ARG | A | 225 | 12.207 | 18.203 | 34.464 | 1.00 | 24.73 | A |
| ATOM | 1463 | N | ARG | A | 226 | 14.305 | 18.308 | 33.727 | 1.00 | 24.84 | A |
| ATOM | 1464 | CA | ARG | A | 226 | 14.383 | 16.899 | 33.365 | 1.00 | 25.74 | A |
| ATOM | 1465 | CB | ARG | A | 226 | 14.886 | 16.756 | 31.973 | 1.00 | 24.42 | A |
| ATOM | 1466 | CG | ARG | A | 226 | 13.840 | 16.784 | 30.906 | 1.00 | 27.50 | A |
| ATOM | 1467 | CD | ARG | A | 226 | 14.498 | 17.010 | 29.570 | 1.00 | 29.18 | A |
| ATOM | 1468 | NE | ARG | A | 226 | 13.518 | 17.522 | 28.631 | 1.00 | 34.09 | A |
| ATOM | 1469 | CZ | ARG | A | 226 | 12.623 | 16.761 | 28.022 | 1.00 | 37.74 | A |
| ATOM | 1470 | NH1 | ARG | A | 226 | 12.606 | 15.452 | 28.253 | 1.00 | 39.12 | A |
| ATOM | 1471 | NH2 | ARG | A | 226 | 11.731 | 17.313 | 27.208 | 1.00 | 38.82 | A |
| ATOM | 1472 | C | ARG | A | 226 | 15.353 | 16.178 | 34.294 | 1.00 | 25.03 | A |
| ATOM | 1473 | O | ARG | A | 226 | 16.300 | 16.748 | 34.750 | 1.00 | 24.22 | A |
| ATOM | 1474 | N | LYS | A | 227 | 15.097 | 14.908 | 34.559 | 1.00 | 27.93 | A |
| ATOM | 1475 | CA | LYS | A | 227 | 15.983 | 14.151 | 35.409 | 1.00 | 30.18 | A |
| ATOM | 1476 | CB | LYS | A | 227 | 15.270 | 12.876 | 35.909 | 1.00 | 30.14 | A |
| ATOM | 1477 | CG | LYS | A | 227 | 14.059 | 13.133 | 36.798 | 1.00 | 32.52 | A |
| ATOM | 1478 | CD | LYS | A | 227 | 13.625 | 11.891 | 37.588 | 1.00 | 33.32 | A |
| ATOM | 1479 | CE | LYS | A | 227 | 12.447 | 12.223 | 38.515 | 1.00 | 37.44 | A |
| ATOM | 1480 | NZ | LYS | A | 227 | 11.940 | 11.077 | 39.334 | 1.00 | 38.91 | A |
| ATOM | 1481 | C | LYS | A | 227 | 17.190 | 13.785 | 34.543 | 1.00 | 30.31 | A |
| ATOM | 1482 | O | LYS | A | 227 | 17.029 | 13.340 | 33.411 | 1.00 | 30.89 | A |
| ATOM | 1483 | N | PRO | A | 228 | 18.418 | 13.995 | 35.046 | 1.00 | 31.14 | A |
| ATOM | 1484 | CD | PRO | A | 228 | 18.894 | 14.648 | 36.274 | 1.00 | 31.55 | A |
| ATOM | 1485 | CA | PRO | A | 228 | 19.538 | 13.627 | 34.186 | 1.00 | 32.29 | A |
| ATOM | 1486 | CB | PRO | A | 228 | 20.757 | 14.103 | 34.966 | 1.00 | 31.44 | A |
| ATOM | 1487 | CG | PRO | A | 228 | 20.297 | 14.141 | 36.366 | 1.00 | 31.25 | A |
| ATOM | 1488 | C | PRO | A | 228 | 19.528 | 12.128 | 33.952 | 1.00 | 32.69 | A |
| ATOM | 1489 | O | PRO | A | 228 | 19.156 | 11.345 | 34.834 | 1.00 | 29.22 | A |
| ATOM | 1490 | N | PHE | A | 229 | 19.928 | 11.743 | 32.746 | 1.00 | 34.78 | A |
| ATOM | 1491 | CA | PHE | A | 229 | 19.966 | 10.347 | 32.332 | 1.00 | 38.17 | A |
| ATOM | 1492 | CB | PHE | A | 229 | 21.235 | 9.699 | 32.783 | 1.00 | 38.30 | A |
| ATOM | 1493 | CG | PHE | A | 229 | 22.459 | 10.459 | 32.401 | 1.00 | 38.21 | A |
| ATOM | 1494 | CD1 | PHE | A | 229 | 23.195 | 11.131 | 33.360 | 1.00 | 38.47 | A |
| ATOM | 1495 | CD2 | PHE | A | 229 | 22.871 | 10.514 | 31.082 | 1.00 | 39.92 | A |
| ATOM | 1496 | CE1 | PHE | A | 229 | 24.320 | 11.841 | 33.008 | 1.00 | 37.97 | A |
| ATOM | 1497 | CE2 | PHE | A | 229 | 23.988 | 11.219 | 30.723 | 1.00 | 39.63 | A |
| ATOM | 1498 | CZ | PHE | A | 229 | 24.718 | 11.886 | 31.685 | 1.00 | 40.21 | A |
| ATOM | 1499 | C | PHE | A | 229 | 18.793 | 9.562 | 32.861 | 1.00 | 40.31 | A |
| ATOM | 1500 | O | PHE | A | 229 | 18.971 | 8.564 | 33.525 | 1.00 | 41.45 | A |
| ATOM | 1501 | N | ASP | A | 230 | 17.584 | 10.036 | 32.591 | 1.00 | 43.27 | A |
| ATOM | 1502 | CA | ASP | A | 230 | 16.418 | 9.298 | 33.031 | 1.00 | 46.74 | A |
| ATOM | 1503 | CB | ASP | A | 230 | 15.230 | 10.176 | 33.159 | 1.00 | 46.83 | A |
| ATOM | 1504 | CG | ASP | A | 230 | 14.111 | 9.514 | 33.933 | 1.00 | 47.72 | A |
| ATOM | 1505 | OD1 | ASP | A | 230 | 14.407 | 8.943 | 35.009 | 1.00 | 48.83 | A |
| ATOM | 1506 | OD2 | ASP | A | 230 | 12.944 | 9.568 | 33.473 | 1.00 | 51.82 | A |
| ATOM | 1507 | C | ASP | A | 230 | 16.227 | 8.338 | 31.891 | 1.00 | 49.68 | A |
| ATOM | 1508 | O | ASP | A | 230 | 15.795 | 7.218 | 32.075 | 1.00 | 49.48 | A |
| ATOM | 1509 | N | GLU | A | 231 | 16.601 | 8.805 | 30.706 | 1.00 | 53.68 | A |

| ATOM | 1510 | CA  | GLU | A | 231 | 16.502 | 8.009  | 29.496 | 1.00 | 55.37 | A |
|------|------|-----|-----|---|-----|--------|--------|--------|------|-------|---|
| ATOM | 1511 | CB  | GLU | A | 231 | 17.011 | 8.798  | 28.326 | 1.00 | 56.93 | A |
| ATOM | 1512 | CG  | GLU | A | 231 | 16.972 | 8.037  | 27.017 | 1.00 | 58.65 | A |
| ATOM | 1513 | CD  | GLU | A | 231 | 16.210 | 8.786  | 25.943 | 1.00 | 66.18 | A |
| ATOM | 1514 | OE1 | GLU | A | 231 | 16.043 | 10.022 | 26.086 | 1.00 | 68.15 | A |
| ATOM | 1515 | OE2 | GLU | A | 231 | 15.791 | 8.140  | 24.954 | 1.00 | 68.77 | A |
| ATOM | 1516 | C   | GLU | A | 231 | 17.311 | 6.724  | 29.643 | 1.00 | 57.49 | A |
| ATOM | 1517 | O   | GLU | A | 231 | 18.534 | 6.750  | 29.753 | 1.00 | 57.98 | A |
| ATOM | 1518 | N   | ILE | A | 232 | 16.607 | 5.600  | 29.638 | 1.00 | 58.88 | A |
| ATOM | 1519 | CA  | ILE | A | 232 | 17.229 | 4.288  | 29.784 | 1.00 | 59.35 | A |
| ATOM | 1520 | CB  | ILE | A | 232 | 17.573 | 3.659  | 28.386 | 1.00 | 60.61 | A |
| ATOM | 1521 | CG2 | ILE | A | 232 | 18.004 | 2.160  | 28.552 | 1.00 | 60.06 | A |
| ATOM | 1522 | CG1 | ILE | A | 232 | 16.305 | 3.727  | 27.438 | 1.00 | 62.83 | A |
| ATOM | 1523 | CD1 | ILE | A | 232 | 15.049 | 3.039  | 27.980 | 1.00 | 64.60 | A |
| ATOM | 1524 | C   | ILE | A | 232 | 18.473 | 4.291  | 30.688 | 1.00 | 59.34 | A |
| ATOM | 1525 | O   | ILE | A | 232 | 19.545 | 4.799  | 30.347 | 1.00 | 59.50 | A |
| ATOM | 1526 | N   | GLY | A | 233 | 18.282 | 3.724  | 31.868 | 1.00 | 58.48 | A |
| ATOM | 1527 | CA  | GLY | A | 233 | 19.345 | 3.610  | 32.832 | 1.00 | 57.89 | A |
| ATOM | 1528 | C   | GLY | A | 233 | 19.240 | 2.204  | 33.382 | 1.00 | 57.45 | A |
| ATOM | 1529 | O   | GLY | A | 233 | 19.869 | 1.281  | 32.892 | 1.00 | 57.80 | A |
| ATOM | 1530 | N   | GLY | A | 234 | 18.392 | 2.031  | 34.381 | 1.00 | 55.95 | A |
| ATOM | 1531 | CA  | GLY | A | 234 | 18.259 | 0.723  | 34.974 | 1.00 | 54.41 | A |
| ATOM | 1532 | C   | GLY | A | 234 | 19.279 | 0.716  | 36.080 | 1.00 | 53.23 | A |
| ATOM | 1533 | O   | GLY | A | 234 | 19.243 | 1.592  | 36.933 | 1.00 | 55.03 | A |
| ATOM | 1534 | N   | PRO | A | 235 | 20.216 | -0.241 | 36.087 | 1.00 | 50.00 | A |
| ATOM | 1535 | CD  | PRO | A | 235 | 20.398 | -1.403 | 35.205 | 1.00 | 50.25 | A |
| ATOM | 1536 | CA  | PRO | A | 235 | 21.203 | -0.239 | 37.171 | 1.00 | 47.70 | A |
| ATOM | 1537 | CB  | PRO | A | 235 | 21.962 | -1.547 | 36.957 | 1.00 | 47.89 | A |
| ATOM | 1538 | CG  | PRO | A | 235 | 21.003 | -2.398 | 36.150 | 1.00 | 49.29 | A |
| ATOM | 1539 | C   | PRO | A | 235 | 22.096 | 0.988  | 37.060 | 1.00 | 45.05 | A |
| ATOM | 1540 | O   | PRO | A | 235 | 22.274 | 1.525  | 35.985 | 1.00 | 43.63 | A |
| ATOM | 1541 | N   | ALA | A | 236 | 22.644 | 1.427  | 38.185 | 1.00 | 42.61 | A |
| ATOM | 1542 | CA  | ALA | A | 236 | 23.511 | 2.596  | 38.199 | 1.00 | 40.73 | A |
| ATOM | 1543 | CB  | ALA | A | 236 | 24.025 | 2.854  | 39.617 | 1.00 | 41.12 | A |
| ATOM | 1544 | C   | ALA | A | 236 | 24.688 | 2.465  | 37.247 | 1.00 | 38.63 | A |
| ATOM | 1545 | O   | ALA | A | 236 | 25.044 | 3.423  | 36.594 | 1.00 | 38.03 | A |
| ATOM | 1546 | N   | PHE | A | 237 | 25.277 | 1.272  | 37.169 | 1.00 | 35.84 | A |
| ATOM | 1547 | CA  | PHE | A | 237 | 26.441 | 1.046  | 36.313 | 1.00 | 34.94 | A |
| ATOM | 1548 | CB  | PHE | A | 237 | 26.967 | -0.412 | 36.496 | 1.00 | 34.80 | A |
| ATOM | 1549 | CG  | PHE | A | 237 | 26.151 | -1.449 | 35.800 | 1.00 | 33.57 | A |
| ATOM | 1550 | CD1 | PHE | A | 237 | 26.209 | -1.592 | 34.421 | 1.00 | 33.21 | A |
| ATOM | 1551 | CD2 | PHE | A | 237 | 25.310 | -2.277 | 36.524 | 1.00 | 35.67 | A |
| ATOM | 1552 | CE1 | PHE | A | 237 | 25.442 | -2.541 | 33.774 | 1.00 | 34.93 | A |
| ATOM | 1553 | CE2 | PHE | A | 237 | 24.538 | -3.230 | 35.887 | 1.00 | 34.71 | A |
| ATOM | 1554 | CZ  | PHE | A | 237 | 24.604 | -3.361 | 34.506 | 1.00 | 35.90 | A |
| ATOM | 1555 | C   | PHE | A | 237 | 26.290 | 1.379  | 34.828 | 1.00 | 33.37 | A |
| ATOM | 1556 | O   | PHE | A | 237 | 27.271 | 1.629  | 34.165 | 1.00 | 31.08 | A |
| ATOM | 1557 | N   | ARG | A | 238 | 25.066 | 1.381  | 34.308 | 1.00 | 32.81 | A |
| ATOM | 1558 | CA  | ARG | A | 238 | 24.867 | 1.720  | 32.899 | 1.00 | 34.48 | A |
| ATOM | 1559 | CB  | ARG | A | 238 | 23.481 | 1.291  | 32.421 | 1.00 | 34.14 | A |
| ATOM | 1560 | CG  | ARG | A | 238 | 23.201 | -0.179 | 32.680 | 1.00 | 37.48 | A |
| ATOM | 1561 | CD  | ARG | A | 238 | 21.890 | -0.652 | 32.068 | 1.00 | 40.56 | A |
| ATOM | 1562 | NE  | ARG | A | 238 | 21.952 | -0.689 | 30.612 | 1.00 | 46.54 | A |
| ATOM | 1563 | CZ  | ARG | A | 238 | 21.106 | -0.050 | 29.813 | 1.00 | 48.29 | A |

114

```
ATOM   1564  NH1 ARG A 238      20.123    0.683   30.322   1.00 50.50      A
ATOM   1565  NH2 ARG A 238      21.246   -0.146   28.500   1.00 50.82      A
ATOM   1566  C   ARG A 238      25.045    3.227   32.732   1.00 32.81      A
ATOM   1567  O   ARG A 238      25.521    3.697   31.711   1.00 31.47      A
ATOM   1568  N   ILE A 239      24.666    3.984   33.753   1.00 33.03      A
ATOM   1569  CA  ILE A 239      24.827    5.420   33.702   1.00 33.75      A
ATOM   1570  CB  ILE A 239      24.070    6.108   34.829   1.00 33.77      A
ATOM   1571  CG2 ILE A 239      24.433    7.541   34.891   1.00 34.66      A
ATOM   1572  CG1 ILE A 239      22.621    5.968   34.607   1.00 34.41      A
ATOM   1573  CD1 ILE A 239      21.806    6.357   35.773   1.00 35.51      A
ATOM   1574  C   ILE A 239      26.306    5.714   33.831   1.00 33.23      A
ATOM   1575  O   ILE A 239      26.841    6.469   33.087   1.00 33.58      A
ATOM   1576  N   MET A 240      26.970    5.081   34.781   1.00 32.87      A
ATOM   1577  CA  MET A 240      28.391    5.328   34.958   1.00 33.72      A
ATOM   1578  CB  MET A 240      28.948    4.442   36.057   1.00 33.32      A
ATOM   1579  CG  MET A 240      28.441    4.826   37.426   1.00 36.00      A
ATOM   1580  SD  MET A 240      29.084    3.800   38.719   1.00 39.69      A
ATOM   1581  CE  MET A 240      27.730    2.651   39.029   1.00 47.26      A
ATOM   1582  C   MET A 240      29.150    5.097   33.667   1.00 33.11      A
ATOM   1583  O   MET A 240      29.919    5.937   33.252   1.00 32.46      A
ATOM   1584  N   TRP A 241      28.914    3.952   33.034   1.00 32.36      A
ATOM   1585  CA  TRP A 241      29.593    3.616   31.793   1.00 31.98      A
ATOM   1586  CB  TRP A 241      29.230    2.184   31.371   1.00 33.67      A
ATOM   1587  CG  TRP A 241      29.529    1.854   29.936   1.00 33.42      A
ATOM   1588  CD2 TRP A 241      30.655    1.129   29.443   1.00 34.22      A
ATOM   1589  CE2 TRP A 241      30.522    1.054   28.040   1.00 35.43      A
ATOM   1590  CE3 TRP A 241      31.768    0.533   30.051   1.00 35.59      A
ATOM   1591  CD1 TRP A 241      28.778    2.180   28.839   1.00 37.55      A
ATOM   1592  NE1 TRP A 241      29.368    1.704   27.699   1.00 37.61      A
ATOM   1593  CZ2 TRP A 241      31.462    0.406   27.233   1.00 38.03      A
ATOM   1594  CZ3 TRP A 241      32.707   -0.114   29.247   1.00 38.49      A
ATOM   1595  CH2 TRP A 241      32.544   -0.171   27.853   1.00 37.70      A
ATOM   1596  C   TRP A 241      29.258    4.614   30.693   1.00 30.41      A
ATOM   1597  O   TRP A 241      30.117    5.008   29.949   1.00 30.45      A
ATOM   1598  N   ALA A 242      28.000    5.025   30.601   1.00 30.00      A
ATOM   1599  CA  ALA A 242      27.617    5.982   29.577   1.00 29.79      A
ATOM   1600  CB  ALA A 242      26.151    6.243   29.627   1.00 28.96      A
ATOM   1601  C   ALA A 242      28.386    7.270   29.794   1.00 28.82      A
ATOM   1602  O   ALA A 242      28.978    7.778   28.881   1.00 28.19      A
ATOM   1603  N   VAL A 243      28.377    7.776   31.026   1.00 29.79      A
ATOM   1604  CA  VAL A 243      29.089    9.000   31.385   1.00 31.01      A
ATOM   1605  CB  VAL A 243      28.707    9.461   32.777   1.00 30.45      A
ATOM   1606  CG1 VAL A 243      29.521   10.649   33.168   1.00 30.77      A
ATOM   1607  CG2 VAL A 243      27.267    9.785   32.811   1.00 29.61      A
ATOM   1608  C   VAL A 243      30.600    8.813   31.364   1.00 32.60      A
ATOM   1609  O   VAL A 243      31.343    9.754   31.191   1.00 31.92      A
ATOM   1610  N   HIS A 244      31.048    7.583   31.561   1.00 33.38      A
ATOM   1611  CA  HIS A 244      32.476    7.301   31.551   1.00 33.44      A
ATOM   1612  CB  HIS A 244      32.747    5.881   32.125   1.00 35.26      A
ATOM   1613  CG  HIS A 244      34.188    5.484   32.104   1.00 37.00      A
ATOM   1614  CD2 HIS A 244      35.219    5.828   32.912   1.00 42.57      A
ATOM   1615  ND1 HIS A 244      34.714    4.655   31.139   1.00 44.63      A
ATOM   1616  CE1 HIS A 244      36.010    4.507   31.352   1.00 44.89      A
ATOM   1617  NE2 HIS A 244      36.341    5.208   32.422   1.00 44.28      A
```

| ATOM | 1618 | C | HIS | A | 244 | 33.015 | 7.404 | 30.133 | 1.00 | 33.43 | A |
|------|------|------|-----|---|-----|--------|-------|--------|------|-------|---|
| ATOM | 1619 | O | HIS | A | 244 | 34.188 | 7.689 | 29.932 | 1.00 | 33.13 | A |
| ATOM | 1620 | N | ASN | A | 245 | 32.143 | 7.172 | 29.155 | 1.00 | 33.14 | A |
| ATOM | 1621 | CA | ASN | A | 245 | 32.541 | 7.225 | 27.761 | 1.00 | 33.22 | A |
| ATOM | 1622 | CB | ASN | A | 245 | 31.794 | 6.166 | 26.949 | 1.00 | 34.94 | A |
| ATOM | 1623 | CG | ASN | A | 245 | 32.432 | 4.789 | 27.070 | 1.00 | 38.82 | A |
| ATOM | 1624 | OD1 | ASN | A | 245 | 33.656 | 4.657 | 27.102 | 1.00 | 43.93 | A |
| ATOM | 1625 | ND2 | ASN | A | 245 | 31.596 | 3.755 | 27.130 | 1.00 | 45.29 | A |
| ATOM | 1626 | C | ASN | A | 245 | 32.313 | 8.601 | 27.175 | 1.00 | 32.45 | A |
| ATOM | 1627 | O | ASN | A | 245 | 32.432 | 8.810 | 25.972 | 1.00 | 32.10 | A |
| ATOM | 1628 | N | GLY | A | 246 | 31.979 | 9.543 | 28.042 | 1.00 | 32.06 | A |
| ATOM | 1629 | CA | GLY | A | 246 | 31.776 | 10.906 | 27.592 | 1.00 | 31.46 | A |
| ATOM | 1630 | C | GLY | A | 246 | 30.357 | 11.350 | 27.311 | 1.00 | 30.50 | A |
| ATOM | 1631 | O | GLY | A | 246 | 30.158 | 12.430 | 26.785 | 1.00 | 30.41 | A |
| ATOM | 1632 | N | THR | A | 247 | 29.369 | 10.527 | 27.648 | 1.00 | 29.35 | A |
| ATOM | 1633 | CA | THR | A | 247 | 27.995 | 10.916 | 27.408 | 1.00 | 29.55 | A |
| ATOM | 1634 | CB | THR | A | 247 | 27.000 | 9.783 | 27.662 | 1.00 | 30.15 | A |
| ATOM | 1635 | OG1 | THR | A | 247 | 27.438 | 8.586 | 27.027 | 1.00 | 31.66 | A |
| ATOM | 1636 | CG2 | THR | A | 247 | 25.675 | 10.145 | 27.078 | 1.00 | 29.87 | A |
| ATOM | 1637 | C | THR | A | 247 | 27.708 | 12.012 | 28.406 | 1.00 | 28.80 | A |
| ATOM | 1638 | O | THR | A | 247 | 28.167 | 11.959 | 29.541 | 1.00 | 27.80 | A |
| ATOM | 1639 | N | ARG | A | 248 | 26.954 | 13.010 | 27.975 | 1.00 | 28.56 | A |
| ATOM | 1640 | CA | ARG | A | 248 | 26.609 | 14.131 | 28.827 | 1.00 | 28.24 | A |
| ATOM | 1641 | CB | ARG | A | 248 | 27.388 | 15.335 | 28.435 | 1.00 | 27.97 | A |
| ATOM | 1642 | CG | ARG | A | 248 | 28.490 | 15.692 | 29.400 | 1.00 | 30.68 | A |
| ATOM | 1643 | CD | ARG | A | 248 | 29.405 | 14.520 | 29.603 | 1.00 | 30.46 | A |
| ATOM | 1644 | NE | ARG | A | 248 | 30.422 | 14.780 | 30.604 | 1.00 | 30.08 | A |
| ATOM | 1645 | CZ | ARG | A | 248 | 31.363 | 13.910 | 30.927 | 1.00 | 26.64 | A |
| ATOM | 1646 | NH1 | ARG | A | 248 | 32.255 | 14.220 | 31.844 | 1.00 | 29.68 | A |
| ATOM | 1647 | NH2 | ARG | A | 248 | 31.412 | 12.729 | 30.333 | 1.00 | 27.78 | A |
| ATOM | 1648 | C | ARG | A | 248 | 25.165 | 14.379 | 28.550 | 1.00 | 27.29 | A |
| ATOM | 1649 | O | ARG | A | 248 | 24.631 | 13.825 | 27.630 | 1.00 | 26.48 | A |
| ATOM | 1650 | N | PRO | A | 249 | 24.508 | 15.209 | 29.360 | 1.00 | 26.82 | A |
| ATOM | 1651 | CD | PRO | A | 249 | 24.911 | 15.835 | 30.627 | 1.00 | 26.33 | A |
| ATOM | 1652 | CA | PRO | A | 249 | 23.093 | 15.454 | 29.079 | 1.00 | 26.96 | A |
| ATOM | 1653 | CB | PRO | A | 249 | 22.657 | 16.338 | 30.239 | 1.00 | 26.22 | A |
| ATOM | 1654 | CG | PRO | A | 249 | 23.600 | 15.975 | 31.336 | 1.00 | 28.13 | A |
| ATOM | 1655 | C | PRO | A | 249 | 22.985 | 16.154 | 27.731 | 1.00 | 26.44 | A |
| ATOM | 1656 | O | PRO | A | 249 | 23.873 | 16.888 | 27.340 | 1.00 | 27.44 | A |
| ATOM | 1657 | N | PRO | A | 250 | 21.891 | 15.928 | 27.004 | 1.00 | 27.07 | A |
| ATOM | 1658 | CD | PRO | A | 250 | 20.662 | 15.232 | 27.409 | 1.00 | 28.08 | A |
| ATOM | 1659 | CA | PRO | A | 250 | 21.726 | 16.566 | 25.698 | 1.00 | 27.17 | A |
| ATOM | 1660 | CB | PRO | A | 250 | 20.421 | 16.058 | 25.244 | 1.00 | 28.09 | A |
| ATOM | 1661 | CG | PRO | A | 250 | 19.651 | 15.894 | 26.528 | 1.00 | 27.10 | A |
| ATOM | 1662 | C | PRO | A | 250 | 21.725 | 18.069 | 25.838 | 1.00 | 27.77 | A |
| ATOM | 1663 | O | PRO | A | 250 | 21.345 | 18.566 | 26.845 | 1.00 | 26.40 | A |
| ATOM | 1664 | N | LEU | A | 251 | 22.163 | 18.777 | 24.805 | 1.00 | 27.91 | A |
| ATOM | 1665 | CA | LEU | A | 251 | 22.196 | 20.230 | 24.838 | 1.00 | 29.68 | A |
| ATOM | 1666 | CB | LEU | A | 251 | 22.773 | 20.757 | 23.592 | 1.00 | 31.49 | A |
| ATOM | 1667 | CG | LEU | A | 251 | 24.138 | 20.259 | 23.233 | 1.00 | 36.10 | A |
| ATOM | 1668 | CD1 | LEU | A | 251 | 24.388 | 20.593 | 21.759 | 1.00 | 41.48 | A |
| ATOM | 1669 | CD2 | LEU | A | 251 | 25.216 | 20.899 | 24.139 | 1.00 | 37.92 | A |
| ATOM | 1670 | C | LEU | A | 251 | 20.799 | 20.787 | 25.009 | 1.00 | 27.61 | A |
| ATOM | 1671 | O | LEU | A | 251 | 19.847 | 20.061 | 25.082 | 1.00 | 27.19 | A |

```
ATOM   1672  N    ILE A 252      20.699  22.104  25.068  1.00 26.65           A
ATOM   1673  CA   ILE A 252      19.421  22.771  25.247  1.00 27.13           A
ATOM   1674  CB   ILE A 252      19.366  23.481  26.625  1.00 25.94           A
ATOM   1675  CG2  ILE A 252      18.022  24.187  26.793  1.00 26.19           A
ATOM   1676  CG1  ILE A 252      19.582  22.454  27.750  1.00 24.41           A
ATOM   1677  CD1  ILE A 252      20.036  23.033  29.040  1.00 24.61           A
ATOM   1678  C    ILE A 252      19.220  23.794  24.131  1.00 28.02           A
ATOM   1679  O    ILE A 252      20.131  24.553  23.782  1.00 28.03           A
ATOM   1680  N    LYS A 253      18.025  23.807  23.561  1.00 30.03           A
ATOM   1681  CA   LYS A 253      17.744  24.725  22.478  1.00 31.99           A
ATOM   1682  CB   LYS A 253      16.344  24.439  21.860  1.00 32.85           A
ATOM   1683  CG   LYS A 253      16.259  23.118  21.078  1.00 38.43           A
ATOM   1684  CD   LYS A 253      14.818  22.780  20.651  1.00 38.32           A
ATOM   1685  CE   LYS A 253      14.681  21.303  20.224  1.00 41.25           A
ATOM   1686  NZ   LYS A 253      13.258  20.826  20.070  1.00 42.73           A
ATOM   1687  C    LYS A 253      17.817  26.137  23.002  1.00 31.23           A
ATOM   1688  O    LYS A 253      17.551  26.386  24.131  1.00 29.47           A
ATOM   1689  N    ASN A 254      18.217  27.045  22.126  1.00 31.71           A
ATOM   1690  CA   ASN A 254      18.351  28.469  22.416  1.00 33.37           A
ATOM   1691  CB   ASN A 254      17.040  29.165  22.198  1.00 34.07           A
ATOM   1692  CG   ASN A 254      16.435  28.819  20.858  1.00 42.81           A
ATOM   1693  OD1  ASN A 254      17.111  28.935  19.802  1.00 47.01           A
ATOM   1694  ND2  ASN A 254      15.167  28.378  20.868  1.00 40.76           A
ATOM   1695  C    ASN A 254      18.867  28.834  23.775  1.00 31.86           A
ATOM   1696  O    ASN A 254      18.304  29.671  24.421  1.00 31.66           A
ATOM   1697  N    LEU A 255      19.953  28.216  24.198  1.00 30.30           A
ATOM   1698  CA   LEU A 255      20.502  28.544  25.496  1.00 28.96           A
ATOM   1699  CB   LEU A 255      21.078  27.295  26.161  1.00 28.69           A
ATOM   1700  CG   LEU A 255      21.664  27.518  27.509  1.00 29.69           A
ATOM   1701  CD1  LEU A 255      20.596  27.330  28.518  1.00 27.92           A
ATOM   1702  CD2  LEU A 255      22.811  26.582  27.747  1.00 27.13           A
ATOM   1703  C    LEU A 255      21.593  29.580  25.320  1.00 27.33           A
ATOM   1704  O    LEU A 255      22.422  29.452  24.460  1.00 26.78           A
ATOM   1705  N    PRO A 256      21.586  30.627  26.146  1.00 27.19           A
ATOM   1706  CD   PRO A 256      20.595  30.884  27.192  1.00 26.82           A
ATOM   1707  CA   PRO A 256      22.573  31.702  26.108  1.00 26.88           A
ATOM   1708  CB   PRO A 256      22.316  32.423  27.372  1.00 25.87           A
ATOM   1709  CG   PRO A 256      20.885  32.296  27.530  1.00 26.34           A
ATOM   1710  C    PRO A 256      23.999  31.165  26.052  1.00 27.45           A
ATOM   1711  O    PRO A 256      24.453  30.547  26.978  1.00 24.74           A
ATOM   1712  N    LYS A 257      24.707  31.420  24.960  1.00 27.55           A
ATOM   1713  CA   LYS A 257      26.074  30.934  24.835  1.00 29.63           A
ATOM   1714  CB   LYS A 257      26.827  31.739  23.797  1.00 31.89           A
ATOM   1715  CG   LYS A 257      26.637  31.265  22.357  1.00 36.73           A
ATOM   1716  CD   LYS A 257      27.162  29.850  22.143  1.00 44.56           A
ATOM   1717  CE   LYS A 257      26.063  28.797  22.307  1.00 47.66           A
ATOM   1718  NZ   LYS A 257      25.108  28.776  21.154  1.00 48.26           A
ATOM   1719  C    LYS A 257      26.837  30.951  26.156  1.00 29.49           A
ATOM   1720  O    LYS A 257      27.337  29.944  26.575  1.00 28.22           A
ATOM   1721  N    PRO A 258      26.925  32.110  26.827  1.00 29.63           A
ATOM   1722  CD   PRO A 258      26.468  33.449  26.446  1.00 29.93           A
ATOM   1723  CA   PRO A 258      27.649  32.165  28.103  1.00 28.52           A
ATOM   1724  CB   PRO A 258      27.385  33.607  28.611  1.00 29.67           A
ATOM   1725  CG   PRO A 258      26.265  34.090  27.786  1.00 30.82           A
```

| ATOM | 1726 | C | PRO A 258 | 27.249 | 31.097 | 29.120 | 1.00 | 27.48 | A |
|------|------|------|------------|--------|--------|--------|------|-------|---|
| ATOM | 1727 | O | PRO A 258 | 28.104 | 30.542 | 29.810 | 1.00 | 25.44 | A |
| ATOM | 1728 | N | ILE A 259 | 25.957 | 30.809 | 29.214 | 1.00 | 25.45 | A |
| ATOM | 1729 | CA | ILE A 259 | 25.501 | 29.783 | 30.134 | 1.00 | 25.49 | A |
| ATOM | 1730 | CB | ILE A 259 | 23.987 | 29.840 | 30.342 | 1.00 | 25.37 | A |
| ATOM | 1731 | CG2 | ILE A 259 | 23.500 | 28.601 | 31.073 | 1.00 | 26.39 | A |
| ATOM | 1732 | CG1 | ILE A 259 | 23.651 | 31.034 | 31.095 | 1.00 | 25.05 | A |
| ATOM | 1733 | CD1 | ILE A 259 | 22.208 | 31.165 | 31.361 | 1.00 | 25.75 | A |
| ATOM | 1734 | C | ILE A 259 | 25.890 | 28.409 | 29.591 | 1.00 | 24.40 | A |
| ATOM | 1735 | O | ILE A 259 | 26.298 | 27.537 | 30.335 | 1.00 | 21.07 | A |
| ATOM | 1736 | N | GLU A 260 | 25.778 | 28.210 | 28.290 | 1.00 | 24.92 | A |
| ATOM | 1737 | CA | GLU A 260 | 26.152 | 26.924 | 27.770 | 1.00 | 25.98 | A |
| ATOM | 1738 | CB | GLU A 260 | 25.894 | 26.866 | 26.327 | 1.00 | 25.25 | A |
| ATOM | 1739 | CG | GLU A 260 | 25.701 | 25.461 | 25.815 | 1.00 | 28.74 | A |
| ATOM | 1740 | CD | GLU A 260 | 25.253 | 25.438 | 24.377 | 1.00 | 34.35 | A |
| ATOM | 1741 | OE1 | GLU A 260 | 26.000 | 25.937 | 23.511 | 1.00 | 43.42 | A |
| ATOM | 1742 | OE2 | GLU A 260 | 24.150 | 24.920 | 24.108 | 1.00 | 45.65 | A |
| ATOM | 1743 | C | GLU A 260 | 27.625 | 26.632 | 28.058 | 1.00 | 24.35 | A |
| ATOM | 1744 | O | GLU A 260 | 27.938 | 25.689 | 28.717 | 1.00 | 23.00 | A |
| ATOM | 1745 | N | SER A 261 | 28.526 | 27.470 | 27.572 | 1.00 | 23.71 | A |
| ATOM | 1746 | CA | SER A 261 | 29.954 | 27.241 | 27.778 | 1.00 | 25.28 | A |
| ATOM | 1747 | CB | SER A 261 | 30.765 | 28.401 | 27.240 | 1.00 | 26.32 | A |
| ATOM | 1748 | OG | SER A 261 | 30.113 | 29.632 | 27.493 | 1.00 | 34.41 | A |
| ATOM | 1749 | C | SER A 261 | 30.329 | 27.008 | 29.224 | 1.00 | 23.81 | A |
| ATOM | 1750 | O | SER A 261 | 31.233 | 26.239 | 29.496 | 1.00 | 23.26 | A |
| ATOM | 1751 | N | LEU A 262 | 29.646 | 27.685 | 30.148 | 1.00 | 23.09 | A |
| ATOM | 1752 | CA | LEU A 262 | 29.929 | 27.518 | 31.579 | 1.00 | 22.68 | A |
| ATOM | 1753 | CB | LEU A 262 | 29.192 | 28.545 | 32.403 | 1.00 | 21.72 | A |
| ATOM | 1754 | CG | LEU A 262 | 29.188 | 28.304 | 33.889 | 1.00 | 24.13 | A |
| ATOM | 1755 | CD1 | LEU A 262 | 30.586 | 28.214 | 34.406 | 1.00 | 27.35 | A |
| ATOM | 1756 | CD2 | LEU A 262 | 28.477 | 29.403 | 34.559 | 1.00 | 25.61 | A |
| ATOM | 1757 | C | LEU A 262 | 29.452 | 26.142 | 31.975 | 1.00 | 21.61 | A |
| ATOM | 1758 | O | LEU A 262 | 30.140 | 25.398 | 32.618 | 1.00 | 19.88 | A |
| ATOM | 1759 | N | MET A 263 | 28.235 | 25.826 | 31.581 | 1.00 | 22.40 | A |
| ATOM | 1760 | CA | MET A 263 | 27.704 | 24.522 | 31.868 | 1.00 | 23.29 | A |
| ATOM | 1761 | CB | MET A 263 | 26.381 | 24.345 | 31.131 | 1.00 | 23.75 | A |
| ATOM | 1762 | CG | MET A 263 | 25.633 | 23.073 | 31.432 | 1.00 | 23.16 | A |
| ATOM | 1763 | SD | MET A 263 | 23.941 | 23.291 | 30.985 | 1.00 | 23.91 | A |
| ATOM | 1764 | CE | MET A 263 | 24.063 | 23.040 | 29.262 | 1.00 | 31.25 | A |
| ATOM | 1765 | C | MET A 263 | 28.729 | 23.478 | 31.405 | 1.00 | 23.77 | A |
| ATOM | 1766 | O | MET A 263 | 29.331 | 22.813 | 32.213 | 1.00 | 22.72 | A |
| ATOM | 1767 | N | THR A 264 | 28.943 | 23.379 | 30.096 | 1.00 | 22.71 | A |
| ATOM | 1768 | CA | THR A 264 | 29.862 | 22.394 | 29.533 | 1.00 | 24.33 | A |
| ATOM | 1769 | CB | THR A 264 | 29.989 | 22.542 | 28.028 | 1.00 | 24.18 | A |
| ATOM | 1770 | OG1 | THR A 264 | 30.632 | 23.778 | 27.735 | 1.00 | 24.61 | A |
| ATOM | 1771 | CG2 | THR A 264 | 28.669 | 22.524 | 27.397 | 1.00 | 26.60 | A |
| ATOM | 1772 | C | THR A 264 | 31.280 | 22.383 | 30.091 | 1.00 | 24.78 | A |
| ATOM | 1773 | O | THR A 264 | 31.946 | 21.336 | 30.093 | 1.00 | 23.81 | A |
| ATOM | 1774 | N | ARG A 265 | 31.765 | 23.527 | 30.547 | 1.00 | 24.15 | A |
| ATOM | 1775 | CA | ARG A 265 | 33.110 | 23.563 | 31.084 | 1.00 | 24.50 | A |
| ATOM | 1776 | CB | ARG A 265 | 33.502 | 24.896 | 31.337 | 1.00 | 23.81 | A |
| ATOM | 1777 | CG | ARG A 265 | 34.501 | 25.402 | 30.372 | 1.00 | 29.12 | A |
| ATOM | 1778 | CD | ARG A 265 | 34.803 | 26.815 | 30.718 | 1.00 | 29.43 | A |
| ATOM | 1779 | NE | ARG A 265 | 35.656 | 26.903 | 31.889 | 1.00 | 30.96 | A |

```
ATOM   1780  CZ   ARG A 265      35.738  27.985  32.647  1.00 32.56          A
ATOM   1781  NH1  ARG A 265      35.002  29.060  32.358  1.00 32.76          A
ATOM   1782  NH2  ARG A 265      36.582  28.002  33.666  1.00 32.65          A
ATOM   1783  C    ARG A 265      33.183  22.800  32.382  1.00 21.82          A
ATOM   1784  O    ARG A 265      34.264  22.432  32.810  1.00 19.98          A
ATOM   1785  N    CYS A 266      32.024  22.582  33.005  1.00 22.35          A
ATOM   1786  CA   CYS A 266      31.950  21.873  34.273  1.00 22.69          A
ATOM   1787  CB   CYS A 266      30.811  22.395  35.119  1.00 21.57          A
ATOM   1788  SG   CYS A 266      30.719  24.169  35.294  1.00 21.28          A
ATOM   1789  C    CYS A 266      31.744  20.397  34.034  1.00 23.84          A
ATOM   1790  O    CYS A 266      31.691  19.649  34.958  1.00 23.82          A
ATOM   1791  N    TRP A 267      31.605  20.002  32.776  1.00 25.01          A
ATOM   1792  CA   TRP A 267      31.426  18.598  32.450  1.00 26.66          A
ATOM   1793  CB   TRP A 267      30.378  18.418  31.439  1.00 27.05          A
ATOM   1794  CG   TRP A 267      29.007  18.714  31.847  1.00 25.40          A
ATOM   1795  CD2  TRP A 267      27.905  18.910  30.972  1.00 25.06          A
ATOM   1796  CE2  TRP A 267      26.760  19.113  31.777  1.00 23.09          A
ATOM   1797  CE3  TRP A 267      27.768  18.934  29.582  1.00 23.19          A
ATOM   1798  CD1  TRP A 267      28.517  18.805  33.116  1.00 30.52          A
ATOM   1799  NE1  TRP A 267      27.161  19.047  33.088  1.00 28.35          A
ATOM   1800  CZ2  TRP A 267      25.493  19.334  31.229  1.00 25.85          A
ATOM   1801  CZ3  TRP A 267      26.514  19.153  29.044  1.00 25.30          A
ATOM   1802  CH2  TRP A 267      25.393  19.349  29.863  1.00 25.42          A
ATOM   1803  C    TRP A 267      32.727  18.032  31.874  1.00 26.99          A
ATOM   1804  O    TRP A 267      32.837  16.823  31.594  1.00 26.21          A
ATOM   1805  N    SER A 268      33.697  18.913  31.658  1.00 27.76          A
ATOM   1806  CA   SER A 268      35.001  18.514  31.135  1.00 28.69          A
ATOM   1807  CB   SER A 268      36.003  19.589  31.394  1.00 28.62          A
ATOM   1808  OG   SER A 268      35.676  20.754  30.663  1.00 29.67          A
ATOM   1809  C    SER A 268      35.454  17.257  31.844  1.00 29.51          A
ATOM   1810  O    SER A 268      35.472  17.206  33.053  1.00 28.01          A
ATOM   1811  N    LYS A 269      35.821  16.241  31.085  1.00 31.34          A
ATOM   1812  CA   LYS A 269      36.267  15.011  31.699  1.00 32.09          A
ATOM   1813  CB   LYS A 269      36.570  13.975  30.611  1.00 32.66          A
ATOM   1814  CG   LYS A 269      37.601  12.960  31.018  1.00 35.56          A
ATOM   1815  CD   LYS A 269      37.515  11.700  30.202  1.00 35.89          A
ATOM   1816  CE   LYS A 269      38.578  10.717  30.671  1.00 40.35          A
ATOM   1817  NZ   LYS A 269      38.473   9.402  29.972  1.00 41.14          A
ATOM   1818  C    LYS A 269      37.503  15.283  32.560  1.00 31.51          A
ATOM   1819  O    LYS A 269      37.652  14.742  33.640  1.00 31.31          A
ATOM   1820  N    ASP A 270      38.388  16.131  32.062  1.00 31.73          A
ATOM   1821  CA   ASP A 270      39.591  16.477  32.791  1.00 31.35          A
ATOM   1822  CB   ASP A 270      40.596  17.073  31.857  1.00 32.73          A
ATOM   1823  CG   ASP A 270      41.956  17.243  32.495  1.00 33.71          A
ATOM   1824  OD1  ASP A 270      42.023  17.576  33.699  1.00 39.22          A
ATOM   1825  OD2  ASP A 270      42.966  17.060  31.785  1.00 37.64          A
ATOM   1826  C    ASP A 270      39.197  17.513  33.828  1.00 30.14          A
ATOM   1827  O    ASP A 270      38.865  18.622  33.483  1.00 29.07          A
ATOM   1828  N    PRO A 271      39.224  17.154  35.116  1.00 29.57          A
ATOM   1829  CD   PRO A 271      39.508  15.812  35.630  1.00 30.22          A
ATOM   1830  CA   PRO A 271      38.869  18.047  36.219  1.00 29.67          A
ATOM   1831  CB   PRO A 271      39.194  17.222  37.429  1.00 28.75          A
ATOM   1832  CG   PRO A 271      38.877  15.866  36.988  1.00 30.46          A
ATOM   1833  C    PRO A 271      39.582  19.407  36.238  1.00 28.49          A
```

| ATOM | 1834 | O | PRO | A | 271 | 38.986 | 20.422 | 36.626 | 1.00 | 27.32 | A |
|------|------|------|------|---|-----|--------|--------|--------|------|-------|---|
| ATOM | 1835 | N | SER | A | 272 | 40.846 | 19.418 | 35.818 | 1.00 | 28.96 | A |
| ATOM | 1836 | CA | SER | A | 272 | 41.653 | 20.637 | 35.802 | 1.00 | 29.70 | A |
| ATOM | 1837 | CB | SER | A | 272 | 43.053 | 20.323 | 35.434 | 1.00 | 30.08 | A |
| ATOM | 1838 | OG | SER | A | 272 | 43.128 | 20.036 | 34.051 | 1.00 | 32.42 | A |
| ATOM | 1839 | C | SER | A | 272 | 41.123 | 21.663 | 34.829 | 1.00 | 28.59 | A |
| ATOM | 1840 | O | SER | A | 272 | 41.450 | 22.823 | 34.928 | 1.00 | 30.11 | A |
| ATOM | 1841 | N | GLN | A | 273 | 40.310 | 21.225 | 33.880 | 1.00 | 29.58 | A |
| ATOM | 1842 | CA | GLN | A | 273 | 39.755 | 22.160 | 32.927 | 1.00 | 29.81 | A |
| ATOM | 1843 | CB | GLN | A | 273 | 39.691 | 21.535 | 31.530 | 1.00 | 31.53 | A |
| ATOM | 1844 | CG | GLN | A | 273 | 41.084 | 21.231 | 30.954 | 1.00 | 38.14 | A |
| ATOM | 1845 | CD | GLN | A | 273 | 42.155 | 22.312 | 31.295 | 1.00 | 45.56 | A |
| ATOM | 1846 | OE1 | GLN | A | 273 | 41.982 | 23.508 | 31.010 | 1.00 | 50.84 | A |
| ATOM | 1847 | NE2 | GLN | A | 273 | 43.268 | 21.878 | 31.886 | 1.00 | 45.17 | A |
| ATOM | 1848 | C | GLN | A | 273 | 38.392 | 22.645 | 33.389 | 1.00 | 29.37 | A |
| ATOM | 1849 | O | GLN | A | 273 | 37.756 | 23.427 | 32.723 | 1.00 | 30.00 | A |
| ATOM | 1850 | N | ARG | A | 274 | 37.963 | 22.171 | 34.555 | 1.00 | 26.99 | A |
| ATOM | 1851 | CA | ARG | A | 274 | 36.696 | 22.597 | 35.144 | 1.00 | 24.29 | A |
| ATOM | 1852 | CB | ARG | A | 274 | 36.120 | 21.500 | 36.052 | 1.00 | 23.23 | A |
| ATOM | 1853 | CG | ARG | A | 274 | 35.575 | 20.276 | 35.342 | 1.00 | 20.15 | A |
| ATOM | 1854 | CD | ARG | A | 274 | 35.130 | 19.264 | 36.378 | 1.00 | 24.68 | A |
| ATOM | 1855 | NE | ARG | A | 274 | 35.060 | 17.905 | 35.851 | 1.00 | 23.00 | A |
| ATOM | 1856 | CZ | ARG | A | 274 | 35.135 | 16.812 | 36.603 | 1.00 | 24.94 | A |
| ATOM | 1857 | NH1 | ARG | A | 274 | 35.284 | 16.912 | 37.911 | 1.00 | 22.65 | A |
| ATOM | 1858 | NH2 | ARG | A | 274 | 35.056 | 15.615 | 36.050 | 1.00 | 22.72 | A |
| ATOM | 1859 | C | ARG | A | 274 | 36.932 | 23.855 | 35.988 | 1.00 | 22.39 | A |
| ATOM | 1860 | O | ARG | A | 274 | 37.853 | 23.932 | 36.764 | 1.00 | 22.38 | A |
| ATOM | 1861 | N | PRO | A | 275 | 36.073 | 24.855 | 35.848 | 1.00 | 20.84 | A |
| ATOM | 1862 | CD | PRO | A | 275 | 34.813 | 24.898 | 35.100 | 1.00 | 20.94 | A |
| ATOM | 1863 | CA | PRO | A | 275 | 36.250 | 26.079 | 36.628 | 1.00 | 21.57 | A |
| ATOM | 1864 | CB | PRO | A | 275 | 35.162 | 26.939 | 36.134 | 1.00 | 21.19 | A |
| ATOM | 1865 | CG | PRO | A | 275 | 34.075 | 25.962 | 35.845 | 1.00 | 21.23 | A |
| ATOM | 1866 | C | PRO | A | 275 | 36.126 | 25.848 | 38.129 | 1.00 | 21.25 | A |
| ATOM | 1867 | O | PRO | A | 275 | 35.366 | 25.023 | 38.560 | 1.00 | 20.98 | A |
| ATOM | 1868 | N | SER | A | 276 | 36.873 | 26.602 | 38.922 | 1.00 | 21.23 | A |
| ATOM | 1869 | CA | SER | A | 276 | 36.785 | 26.459 | 40.359 | 1.00 | 21.37 | A |
| ATOM | 1870 | CB | SER | A | 276 | 37.849 | 27.226 | 41.021 | 1.00 | 21.46 | A |
| ATOM | 1871 | OG | SER | A | 276 | 37.498 | 28.588 | 41.082 | 1.00 | 22.27 | A |
| ATOM | 1872 | C | SER | A | 276 | 35.426 | 26.986 | 40.795 | 1.00 | 21.18 | A |
| ATOM | 1873 | O | SER | A | 276 | 34.801 | 27.748 | 40.094 | 1.00 | 18.83 | A |
| ATOM | 1874 | N | MET | A | 277 | 34.974 | 26.562 | 41.969 | 1.00 | 21.11 | A |
| ATOM | 1875 | CA | MET | A | 277 | 33.674 | 26.980 | 42.470 | 1.00 | 21.81 | A |
| ATOM | 1876 | CB | MET | A | 277 | 33.329 | 26.165 | 43.746 | 1.00 | 22.57 | A |
| ATOM | 1877 | CG | MET | A | 277 | 31.845 | 26.139 | 44.127 | 1.00 | 24.25 | A |
| ATOM | 1878 | SD | MET | A | 277 | 30.676 | 25.634 | 42.830 | 1.00 | 30.20 | A |
| ATOM | 1879 | CE | MET | A | 277 | 30.831 | 23.929 | 42.790 | 1.00 | 31.26 | A |
| ATOM | 1880 | C | MET | A | 277 | 33.654 | 28.484 | 42.724 | 1.00 | 21.66 | A |
| ATOM | 1881 | O | MET | A | 277 | 32.622 | 29.110 | 42.620 | 1.00 | 20.09 | A |
| ATOM | 1882 | N | GLU | A | 278 | 34.816 | 29.049 | 43.035 | 1.00 | 21.81 | A |
| ATOM | 1883 | CA | GLU | A | 278 | 34.942 | 30.476 | 43.279 | 1.00 | 23.64 | A |
| ATOM | 1884 | CB | GLU | A | 278 | 36.349 | 30.820 | 43.700 | 1.00 | 26.26 | A |
| ATOM | 1885 | CG | GLU | A | 278 | 36.530 | 30.974 | 45.199 | 1.00 | 33.56 | A |
| ATOM | 1886 | CD | GLU | A | 278 | 35.379 | 31.737 | 45.854 | 1.00 | 41.57 | A |
| ATOM | 1887 | OE1 | GLU | A | 278 | 34.829 | 32.675 | 45.224 | 1.00 | 47.24 | A |

| ATOM | 1888 | OE2 | GLU | A | 278 | 35.032 | 31.405 | 47.011 | 1.00 | 40.04 | A |
|------|------|-----|-----|---|-----|--------|--------|--------|------|-------|---|
| ATOM | 1889 | C   | GLU | A | 278 | 34.614 | 31.218 | 42.003 | 1.00 | 21.64 | A |
| ATOM | 1890 | O   | GLU | A | 278 | 33.970 | 32.238 | 42.025 | 1.00 | 22.67 | A |
| ATOM | 1891 | N   | GLU | A | 279 | 35.081 | 30.687 | 40.887 | 1.00 | 21.33 | A |
| ATOM | 1892 | CA  | GLU | A | 279 | 34.815 | 31.297 | 39.603 | 1.00 | 22.05 | A |
| ATOM | 1893 | CB  | GLU | A | 279 | 35.590 | 30.616 | 38.562 | 1.00 | 21.63 | A |
| ATOM | 1894 | CG  | GLU | A | 279 | 35.363 | 31.140 | 37.175 | 1.00 | 25.92 | A |
| ATOM | 1895 | CD  | GLU | A | 279 | 36.427 | 30.677 | 36.209 | 1.00 | 29.02 | A |
| ATOM | 1896 | OE1 | GLU | A | 279 | 36.294 | 30.991 | 35.006 | 1.00 | 33.56 | A |
| ATOM | 1897 | OE2 | GLU | A | 279 | 37.397 | 30.010 | 36.644 | 1.00 | 39.94 | A |
| ATOM | 1898 | C   | GLU | A | 279 | 33.327 | 31.204 | 39.284 | 1.00 | 20.25 | A |
| ATOM | 1899 | O   | GLU | A | 279 | 32.692 | 32.198 | 39.000 | 1.00 | 19.20 | A |
| ATOM | 1900 | N   | ILE | A | 280 | 32.772 | 30.001 | 39.343 | 1.00 | 19.02 | A |
| ATOM | 1901 | CA  | ILE | A | 280 | 31.356 | 29.810 | 39.067 | 1.00 | 18.99 | A |
| ATOM | 1902 | CB  | ILE | A | 280 | 30.895 | 28.392 | 39.420 | 1.00 | 18.96 | A |
| ATOM | 1903 | CG2 | ILE | A | 280 | 29.408 | 28.272 | 39.267 | 1.00 | 18.88 | A |
| ATOM | 1904 | CG1 | ILE | A | 280 | 31.538 | 27.408 | 38.520 | 1.00 | 19.61 | A |
| ATOM | 1905 | CD1 | ILE | A | 280 | 31.244 | 26.004 | 38.877 | 1.00 | 20.19 | A |
| ATOM | 1906 | C   | ILE | A | 280 | 30.526 | 30.786 | 39.857 | 1.00 | 17.73 | A |
| ATOM | 1907 | O   | ILE | A | 280 | 29.565 | 31.233 | 39.386 | 1.00 | 17.37 | A |
| ATOM | 1908 | N   | VAL | A | 281 | 30.921 | 31.098 | 41.079 | 1.00 | 18.04 | A |
| ATOM | 1909 | CA  | VAL | A | 281 | 30.156 | 32.053 | 41.864 | 1.00 | 19.10 | A |
| ATOM | 1910 | CB  | VAL | A | 281 | 30.648 | 32.127 | 43.315 | 1.00 | 19.60 | A |
| ATOM | 1911 | CG1 | VAL | A | 281 | 30.198 | 33.441 | 43.964 | 1.00 | 20.68 | A |
| ATOM | 1912 | CG2 | VAL | A | 281 | 30.091 | 30.970 | 44.094 | 1.00 | 19.50 | A |
| ATOM | 1913 | C   | VAL | A | 281 | 30.229 | 33.447 | 41.273 | 1.00 | 17.93 | A |
| ATOM | 1914 | O   | VAL | A | 281 | 29.273 | 34.168 | 41.319 | 1.00 | 16.95 | A |
| ATOM | 1915 | N   | LYS | A | 282 | 31.388 | 33.806 | 40.724 | 1.00 | 20.00 | A |
| ATOM | 1916 | CA  | LYS | A | 282 | 31.603 | 35.122 | 40.119 | 1.00 | 22.26 | A |
| ATOM | 1917 | CB  | LYS | A | 282 | 33.097 | 35.333 | 39.827 | 1.00 | 21.85 | A |
| ATOM | 1918 | CG  | LYS | A | 282 | 33.910 | 35.925 | 40.983 | 1.00 | 27.14 | A |
| ATOM | 1919 | CD  | LYS | A | 282 | 35.430 | 35.841 | 40.683 | 1.00 | 31.56 | A |
| ATOM | 1920 | CE  | LYS | A | 282 | 36.344 | 36.273 | 41.876 | 1.00 | 37.45 | A |
| ATOM | 1921 | NZ  | LYS | A | 282 | 36.510 | 35.280 | 43.002 | 1.00 | 36.46 | A |
| ATOM | 1922 | C   | LYS | A | 282 | 30.806 | 35.254 | 38.828 | 1.00 | 20.83 | A |
| ATOM | 1923 | O   | LYS | A | 282 | 30.176 | 36.245 | 38.581 | 1.00 | 20.71 | A |
| ATOM | 1924 | N   | ILE | A | 283 | 30.853 | 34.223 | 38.007 | 1.00 | 20.41 | A |
| ATOM | 1925 | CA  | ILE | A | 283 | 30.121 | 34.224 | 36.755 | 1.00 | 18.56 | A |
| ATOM | 1926 | CB  | ILE | A | 283 | 30.512 | 33.005 | 35.883 | 1.00 | 21.65 | A |
| ATOM | 1927 | CG2 | ILE | A | 283 | 29.452 | 32.731 | 34.860 | 1.00 | 20.07 | A |
| ATOM | 1928 | CG1 | ILE | A | 283 | 31.867 | 33.267 | 35.199 | 1.00 | 21.40 | A |
| ATOM | 1929 | CD1 | ILE | A | 283 | 32.813 | 32.101 | 35.206 | 1.00 | 19.32 | A |
| ATOM | 1930 | C   | ILE | A | 283 | 28.621 | 34.182 | 37.000 | 1.00 | 17.01 | A |
| ATOM | 1931 | O   | ILE | A | 283 | 27.865 | 34.741 | 36.273 | 1.00 | 15.78 | A |
| ATOM | 1932 | N   | MET | A | 284 | 28.196 | 33.508 | 38.046 | 1.00 | 16.12 | A |
| ATOM | 1933 | CA  | MET | A | 284 | 26.785 | 33.415 | 38.295 | 1.00 | 17.42 | A |
| ATOM | 1934 | CB  | MET | A | 284 | 26.493 | 32.301 | 39.278 | 1.00 | 18.47 | A |
| ATOM | 1935 | CG  | MET | A | 284 | 26.570 | 30.909 | 38.715 | 1.00 | 16.76 | A |
| ATOM | 1936 | SD  | MET | A | 284 | 25.472 | 30.758 | 37.376 | 1.00 | 19.47 | A |
| ATOM | 1937 | CE  | MET | A | 284 | 24.039 | 30.597 | 38.194 | 1.00 | 19.10 | A |
| ATOM | 1938 | C   | MET | A | 284 | 26.277 | 34.720 | 38.827 | 1.00 | 18.25 | A |
| ATOM | 1939 | O   | MET | A | 284 | 25.272 | 35.127 | 38.456 | 1.00 | 17.64 | A |
| ATOM | 1940 | N   | THR | A | 285 | 27.009 | 35.366 | 39.720 | 1.00 | 19.63 | A |
| ATOM | 1941 | CA  | THR | A | 285 | 26.546 | 36.645 | 40.262 | 1.00 | 21.24 | A |

```
ATOM   1942  CB   THR A 285    27.559  37.267  41.232  1.00 21.59      A
ATOM   1943  OG1  THR A 285    27.735  36.412  42.356  1.00 22.48      A
ATOM   1944  CG2  THR A 285    27.071  38.573  41.729  1.00 22.40      A
ATOM   1945  C    THR A 285    26.321  37.659  39.147  1.00 20.82      A
ATOM   1946  O    THR A 285    25.321  38.348  39.115  1.00 20.44      A
ATOM   1947  N    HIS A 286    27.282  37.742  38.237  1.00 21.59      A
ATOM   1948  CA   HIS A 286    27.218  38.664  37.117  1.00 23.27      A
ATOM   1949  CB   HIS A 286    28.494  38.591  36.291  1.00 24.63      A
ATOM   1950  CG   HIS A 286    29.725  38.987  37.050  1.00 27.93      A
ATOM   1951  CD2  HIS A 286    30.999  38.537  36.978  1.00 29.50      A
ATOM   1952  ND1  HIS A 286    29.729  40.001  37.985  1.00 30.73      A
ATOM   1953  CE1  HIS A 286    30.953  40.159  38.455  1.00 32.04      A
ATOM   1954  NE2  HIS A 286    31.742  39.284  37.859  1.00 33.61      A
ATOM   1955  C    HIS A 286    26.030  38.361  36.253  1.00 23.56      A
ATOM   1956  O    HIS A 286    25.289  39.217  35.975  1.00 23.53      A
ATOM   1957  N    LEU A 287    25.861  37.117  35.837  1.00 22.40      A
ATOM   1958  CA   LEU A 287    24.722  36.784  35.001  1.00 22.13      A
ATOM   1959  CB   LEU A 287    24.712  35.310  34.685  1.00 22.42      A
ATOM   1960  CG   LEU A 287    25.545  34.882  33.507  1.00 21.46      A
ATOM   1961  CD1  LEU A 287    25.430  33.417  33.340  1.00 25.14      A
ATOM   1962  CD2  LEU A 287    25.100  35.572  32.257  1.00 25.71      A
ATOM   1963  C    LEU A 287    23.380  37.176  35.615  1.00 22.14      A
ATOM   1964  O    LEU A 287    22.472  37.474  34.911  1.00 19.21      A
ATOM   1965  N    MET A 288    23.285  37.188  36.941  1.00 22.47      A
ATOM   1966  CA   MET A 288    22.040  37.511  37.636  1.00 24.27      A
ATOM   1967  CB   MET A 288    22.221  37.390  39.143  1.00 24.75      A
ATOM   1968  CG   MET A 288    22.301  35.980  39.669  1.00 28.50      A
ATOM   1969  SD   MET A 288    20.959  34.956  39.104  1.00 32.30      A
ATOM   1970  CE   MET A 288    19.687  35.329  40.233  1.00 31.23      A
ATOM   1971  C    MET A 288    21.507  38.893  37.306  1.00 25.62      A
ATOM   1972  O    MET A 288    20.376  39.215  37.607  1.00 24.03      A
ATOM   1973  N    ARG A 289    22.346  39.709  36.689  1.00 25.61      A
ATOM   1974  CA   ARG A 289    21.976  41.063  36.285  1.00 26.30      A
ATOM   1975  CB   ARG A 289    23.260  41.820  35.867  1.00 26.23      A
ATOM   1976  CG   ARG A 289    23.012  43.208  35.363  1.00 30.27      A
ATOM   1977  CD   ARG A 289    24.267  43.856  34.781  1.00 33.28      A
ATOM   1978  NE   ARG A 289    23.931  45.112  34.105  1.00 37.44      A
ATOM   1979  CZ   ARG A 289    23.398  46.177  34.708  1.00 39.94      A
ATOM   1980  NH1  ARG A 289    23.145  46.158  36.012  1.00 43.23      A
ATOM   1981  NH2  ARG A 289    23.079  47.256  33.998  1.00 41.68      A
ATOM   1982  C    ARG A 289    20.962  41.012  35.114  1.00 23.71      A
ATOM   1983  O    ARG A 289    20.368  42.012  34.731  1.00 23.58      A
ATOM   1984  N    TYR A 290    20.771  39.817  34.567  1.00 22.35      A
ATOM   1985  CA   TYR A 290    19.863  39.590  33.454  1.00 21.66      A
ATOM   1986  CB   TYR A 290    20.624  39.026  32.345  1.00 23.05      A
ATOM   1987  CG   TYR A 290    21.768  39.927  31.978  1.00 23.71      A
ATOM   1988  CD1  TYR A 290    23.010  39.820  32.605  1.00 23.34      A
ATOM   1989  CE1  TYR A 290    24.019  40.722  32.333  1.00 24.74      A
ATOM   1990  CD2  TYR A 290    21.578  40.961  31.066  1.00 22.82      A
ATOM   1991  CE2  TYR A 290    22.579  41.864  30.790  1.00 22.04      A
ATOM   1992  CZ   TYR A 290    23.787  41.745  31.423  1.00 25.42      A
ATOM   1993  OH   TYR A 290    24.731  42.698  31.147  1.00 25.39      A
ATOM   1994  C    TYR A 290    18.740  38.656  33.866  1.00 20.96      A
ATOM   1995  O    TYR A 290    17.877  38.324  33.075  1.00 19.23      A
```

| ATOM | 1996 | N   | PHE | A | 291 | 18.777 | 38.249 | 35.133 | 1.00 | 19.15 | A |
|------|------|-----|-----|---|-----|--------|--------|--------|------|-------|---|
| ATOM | 1997 | CA  | PHE | A | 291 | 17.777 | 37.373 | 35.711 | 1.00 | 19.65 | A |
| ATOM | 1998 | CB  | PHE | A | 291 | 18.384 | 36.130 | 36.065 | 1.00 | 18.50 | A |
| ATOM | 1999 | CG  | PHE | A | 291 | 18.752 | 35.319 | 34.894 | 1.00 | 18.43 | A |
| ATOM | 2000 | CD1 | PHE | A | 291 | 20.056 | 35.221 | 34.495 | 1.00 | 18.54 | A |
| ATOM | 2001 | CD2 | PHE | A | 291 | 17.780 | 34.680 | 34.159 | 1.00 | 19.11 | A |
| ATOM | 2002 | CE1 | PHE | A | 291 | 20.395 | 34.494 | 33.372 | 1.00 | 20.10 | A |
| ATOM | 2003 | CE2 | PHE | A | 291 | 18.107 | 33.949 | 33.032 | 1.00 | 18.74 | A |
| ATOM | 2004 | CZ  | PHE | A | 291 | 19.421 | 33.857 | 32.639 | 1.00 | 21.43 | A |
| ATOM | 2005 | C   | PHE | A | 291 | 17.114 | 38.007 | 36.923 | 1.00 | 20.88 | A |
| ATOM | 2006 | O   | PHE | A | 291 | 17.384 | 37.669 | 38.040 | 1.00 | 20.11 | A |
| ATOM | 2007 | N   | PRO | A | 292 | 16.223 | 38.965 | 36.677 | 1.00 | 21.50 | A |
| ATOM | 2008 | CD  | PRO | A | 292 | 16.077 | 39.749 | 35.438 | 1.00 | 22.10 | A |
| ATOM | 2009 | CA  | PRO | A | 292 | 15.547 | 39.623 | 37.782 | 1.00 | 22.86 | A |
| ATOM | 2010 | CB  | PRO | A | 292 | 15.292 | 41.003 | 37.239 | 1.00 | 23.05 | A |
| ATOM | 2011 | CG  | PRO | A | 292 | 15.044 | 40.759 | 35.824 | 1.00 | 23.10 | A |
| ATOM | 2012 | C   | PRO | A | 292 | 14.269 | 38.918 | 38.189 | 1.00 | 22.78 | A |
| ATOM | 2013 | O   | PRO | A | 292 | 13.593 | 38.346 | 37.372 | 1.00 | 24.66 | A |
| ATOM | 2014 | N   | GLY | A | 293 | 13.956 | 38.976 | 39.478 | 1.00 | 23.98 | A |
| ATOM | 2015 | CA  | GLY | A | 293 | 12.746 | 38.356 | 39.977 | 1.00 | 24.44 | A |
| ATOM | 2016 | C   | GLY | A | 293 | 12.988 | 37.114 | 40.808 | 1.00 | 24.63 | A |
| ATOM | 2017 | O   | GLY | A | 293 | 12.062 | 36.501 | 41.291 | 1.00 | 25.46 | A |
| ATOM | 2018 | N   | ALA | A | 294 | 14.242 | 36.738 | 40.971 | 1.00 | 25.29 | A |
| ATOM | 2019 | CA  | ALA | A | 294 | 14.544 | 35.567 | 41.750 | 1.00 | 27.80 | A |
| ATOM | 2020 | CB  | ALA | A | 294 | 16.018 | 35.470 | 41.941 | 1.00 | 26.40 | A |
| ATOM | 2021 | C   | ALA | A | 294 | 13.834 | 35.591 | 43.103 | 1.00 | 29.22 | A |
| ATOM | 2022 | O   | ALA | A | 294 | 13.406 | 34.558 | 43.578 | 1.00 | 30.83 | A |
| ATOM | 2023 | N   | ASP | A | 295 | 13.687 | 36.775 | 43.700 | 1.00 | 30.23 | A |
| ATOM | 2024 | CA  | ASP | A | 295 | 13.061 | 36.930 | 45.020 | 1.00 | 30.69 | A |
| ATOM | 2025 | CB  | ASP | A | 295 | 13.269 | 38.356 | 45.540 | 1.00 | 32.76 | A |
| ATOM | 2026 | CG  | ASP | A | 295 | 13.061 | 39.416 | 44.454 | 1.00 | 41.25 | A |
| ATOM | 2027 | OD1 | ASP | A | 295 | 13.851 | 39.429 | 43.474 | 1.00 | 45.30 | A |
| ATOM | 2028 | OD2 | ASP | A | 295 | 12.111 | 40.235 | 44.577 | 1.00 | 50.86 | A |
| ATOM | 2029 | C   | ASP | A | 295 | 11.584 | 36.585 | 45.157 | 1.00 | 29.93 | A |
| ATOM | 2030 | O   | ASP | A | 295 | 11.132 | 36.378 | 46.247 | 1.00 | 28.59 | A |
| ATOM | 2031 | N   | GLU | A | 296 | 10.841 | 36.540 | 44.052 | 1.00 | 27.97 | A |
| ATOM | 2032 | CA  | GLU | A | 296 | 9.407  | 36.228 | 44.083 | 1.00 | 29.07 | A |
| ATOM | 2033 | CB  | GLU | A | 296 | 8.789  | 36.450 | 42.706 | 1.00 | 28.34 | A |
| ATOM | 2034 | CG  | GLU | A | 296 | 9.086  | 37.826 | 42.118 | 1.00 | 35.74 | A |
| ATOM | 2035 | CD  | GLU | A | 296 | 8.290  | 38.964 | 42.781 | 1.00 | 42.18 | A |
| ATOM | 2036 | OE1 | GLU | A | 296 | 8.820  | 40.101 | 42.839 | 1.00 | 46.84 | A |
| ATOM | 2037 | OE2 | GLU | A | 296 | 7.135  | 38.729 | 43.226 | 1.00 | 46.24 | A |
| ATOM | 2038 | C   | GLU | A | 296 | 9.188  | 34.788 | 44.507 | 1.00 | 28.01 | A |
| ATOM | 2039 | O   | GLU | A | 296 | 9.691  | 33.891 | 43.887 | 1.00 | 27.50 | A |
| ATOM | 2040 | N   | PRO | A | 297 | 8.411  | 34.559 | 45.574 | 1.00 | 28.90 | A |
| ATOM | 2041 | CD  | PRO | A | 297 | 7.598  | 35.529 | 46.314 | 1.00 | 29.23 | A |
| ATOM | 2042 | CA  | PRO | A | 297 | 8.148  | 33.201 | 46.062 | 1.00 | 28.70 | A |
| ATOM | 2043 | CB  | PRO | A | 297 | 7.310  | 33.438 | 47.251 | 1.00 | 29.46 | A |
| ATOM | 2044 | CG  | PRO | A | 297 | 6.535  | 34.637 | 46.872 | 1.00 | 29.69 | A |
| ATOM | 2045 | C   | PRO | A | 297 | 7.427  | 32.346 | 45.039 | 1.00 | 28.69 | A |
| ATOM | 2046 | O   | PRO | A | 297 | 6.784  | 32.875 | 44.158 | 1.00 | 27.87 | A |
| ATOM | 2047 | N   | LEU | A | 298 | 7.552  | 31.024 | 45.166 | 1.00 | 29.19 | A |
| ATOM | 2048 | CA  | LEU | A | 298 | 6.887  | 30.088 | 44.261 | 1.00 | 30.58 | A |
| ATOM | 2049 | CB  | LEU | A | 298 | 7.495  | 28.757 | 44.382 | 1.00 | 30.84 | A |

| ATOM | 2050 | CG | LEU | A | 298 | 8.923 | 28.600 | 44.011 | 1.00 | 31.61 | A |
|------|------|-----|-----|---|-----|-------|--------|--------|------|-------|---|
| ATOM | 2051 | CD1 | LEU | A | 298 | 9.436 | 27.241 | 44.449 | 1.00 | 32.11 | A |
| ATOM | 2052 | CD2 | LEU | A | 298 | 9.036 | 28.735 | 42.531 | 1.00 | 26.67 | A |
| ATOM | 2053 | C | LEU | A | 298 | 5.446 | 30.002 | 44.731 | 1.00 | 31.55 | A |
| ATOM | 2054 | O | LEU | A | 298 | 5.217 | 29.777 | 45.903 | 1.00 | 31.63 | A |
| ATOM | 2055 | N | GLN | A | 299 | 4.473 | 30.171 | 43.836 | 1.00 | 30.21 | A |
| ATOM | 2056 | CA | GLN | A | 299 | 3.075 | 30.126 | 44.263 | 1.00 | 31.27 | A |
| ATOM | 2057 | CB | GLN | A | 299 | 2.675 | 31.468 | 44.851 | 1.00 | 30.93 | A |
| ATOM | 2058 | CG | GLN | A | 299 | 2.614 | 32.603 | 43.841 | 1.00 | 32.98 | A |
| ATOM | 2059 | CD | GLN | A | 299 | 2.697 | 33.964 | 44.490 | 1.00 | 33.56 | A |
| ATOM | 2060 | OE1 | GLN | A | 299 | 3.755 | 34.429 | 44.816 | 1.00 | 42.33 | A |
| ATOM | 2061 | NE2 | GLN | A | 299 | 1.554 | 34.592 | 44.691 | 1.00 | 36.82 | A |
| ATOM | 2062 | C | GLN | A | 299 | 2.049 | 29.724 | 43.218 | 1.00 | 31.56 | A |
| ATOM | 2063 | O | GLN | A | 299 | 0.936 | 29.412 | 43.558 | 1.00 | 31.62 | A |
| ATOM | 2064 | N | TYR | A | 300 | 2.415 | 29.739 | 41.945 | 1.00 | 32.27 | A |
| ATOM | 2065 | CA | TYR | A | 300 | 1.471 | 29.352 | 40.908 | 1.00 | 33.17 | A |
| ATOM | 2066 | CB | TYR | A | 300 | 1.701 | 30.158 | 39.678 | 1.00 | 32.87 | A |
| ATOM | 2067 | CG | TYR | A | 300 | 1.764 | 31.635 | 39.953 | 1.00 | 30.37 | A |
| ATOM | 2068 | CD1 | TYR | A | 300 | 2.929 | 32.230 | 40.433 | 1.00 | 28.14 | A |
| ATOM | 2069 | CE1 | TYR | A | 300 | 2.968 | 33.586 | 40.747 | 1.00 | 30.52 | A |
| ATOM | 2070 | CD2 | TYR | A | 300 | 0.643 | 32.430 | 39.792 | 1.00 | 34.29 | A |
| ATOM | 2071 | CE2 | TYR | A | 300 | 0.671 | 33.781 | 40.106 | 1.00 | 31.83 | A |
| ATOM | 2072 | CZ | TYR | A | 300 | 1.833 | 34.356 | 40.583 | 1.00 | 29.09 | A |
| ATOM | 2073 | OH | TYR | A | 300 | 1.842 | 35.695 | 40.902 | 1.00 | 29.91 | A |
| ATOM | 2074 | C | TYR | A | 300 | 1.634 | 27.886 | 40.595 | 1.00 | 35.21 | A |
| ATOM | 2075 | O | TYR | A | 300 | 2.739 | 27.393 | 40.520 | 1.00 | 34.29 | A |
| ATOM | 2076 | N | PRO | A | 301 | 0.525 | 27.166 | 40.398 | 1.00 | 37.95 | A |
| ATOM | 2077 | CD | PRO | A | 301 | -0.849 | 27.583 | 40.089 | 1.00 | 38.92 | A |
| ATOM | 2078 | CA | PRO | A | 301 | 0.696 | 25.745 | 40.101 | 1.00 | 39.38 | A |
| ATOM | 2079 | CB | PRO | A | 301 | -0.639 | 25.226 | 40.225 | 1.00 | 40.13 | A |
| ATOM | 2080 | CG | PRO | A | 301 | -1.438 | 26.300 | 39.507 | 1.00 | 38.79 | A |
| ATOM | 2081 | C | PRO | A | 301 | 1.148 | 25.688 | 38.667 | 1.00 | 41.69 | A |
| ATOM | 2082 | O | PRO | A | 301 | 1.231 | 26.707 | 38.005 | 1.00 | 41.62 | A |
| ATOM | 2083 | N | CYS | A | 302 | 1.414 | 24.484 | 38.190 | 1.00 | 44.13 | A |
| ATOM | 2084 | CA | CYS | A | 302 | 1.847 | 24.301 | 36.825 | 1.00 | 46.65 | A |
| ATOM | 2085 | CB | CYS | A | 302 | 3.331 | 24.637 | 36.714 | 1.00 | 47.45 | A |
| ATOM | 2086 | SG | CYS | A | 302 | 4.216 | 23.930 | 35.307 | 1.00 | 50.88 | A |
| ATOM | 2087 | C | CYS | A | 302 | 1.576 | 22.864 | 36.417 | 1.00 | 47.68 | A |
| ATOM | 2088 | O | CYS | A | 302 | 2.004 | 21.924 | 37.072 | 1.00 | 47.56 | A |
| ATOM | 2089 | N | GLN | A | 303 | 0.815 | 22.714 | 35.344 | 1.00 | 49.80 | A |
| ATOM | 2090 | CA | GLN | A | 303 | 0.479 | 21.408 | 34.810 | 1.00 | 52.54 | A |
| ATOM | 2091 | CB | GLN | A | 303 | -0.935 | 21.449 | 34.185 | 1.00 | 49.88 | A |
| ATOM | 2092 | CG | GLN | A | 303 | -2.047 | 22.047 | 35.100 | 1.00 | 47.50 | A |
| ATOM | 2093 | CD | GLN | A | 303 | -2.048 | 23.595 | 35.220 | 1.00 | 42.00 | A |
| ATOM | 2094 | OE1 | GLN | A | 303 | -2.056 | 24.318 | 34.211 | 1.00 | 34.56 | A |
| ATOM | 2095 | NE2 | GLN | A | 303 | -2.071 | 24.097 | 36.462 | 1.00 | 30.24 | A |
| ATOM | 2096 | C | GLN | A | 303 | 1.560 | 21.148 | 33.762 | 1.00 | 55.05 | A |
| ATOM | 2097 | O | GLN | A | 303 | 1.378 | 21.362 | 32.575 | 1.00 | 56.52 | A |
| ATOM | 2098 | N | HIS | A | 304 | 2.710 | 20.708 | 34.255 | 1.00 | 57.63 | A |
| ATOM | 2099 | CA | HIS | A | 304 | 3.889 | 20.437 | 33.443 | 1.00 | 59.57 | A |
| ATOM | 2100 | CB | HIS | A | 304 | 5.079 | 20.288 | 34.367 | 1.00 | 59.15 | A |
| ATOM | 2101 | CG | HIS | A | 304 | 6.391 | 20.319 | 33.660 | 1.00 | 57.65 | A |
| ATOM | 2102 | CD2 | HIS | A | 304 | 6.703 | 20.589 | 32.370 | 1.00 | 55.14 | A |
| ATOM | 2103 | ND1 | HIS | A | 304 | 7.577 | 20.001 | 34.284 | 1.00 | 56.57 | A |

| ATOM | 2104 | CE1 | HIS | A | 304 | 8.564 | 20.065 | 33.408 | 1.00 | 56.80 | A |
|------|------|-----|-----|---|-----|-------|--------|--------|------|-------|---|
| ATOM | 2105 | NE2 | HIS | A | 304 | 8.060 | 20.420 | 32.239 | 1.00 | 54.57 | A |
| ATOM | 2106 | C | HIS | A | 304 | 3.770 | 19.215 | 32.520 | 1.00 | 62.16 | A |
| ATOM | 2107 | O | HIS | A | 304 | 3.396 | 18.109 | 32.958 | 1.00 | 62.13 | A |
| ATOM | 2108 | N | SER | A | 305 | 4.113 | 19.419 | 31.246 | 1.00 | 65.23 | A |
| ATOM | 2109 | CA | SER | A | 305 | 4.026 | 18.371 | 30.221 | 1.00 | 67.30 | A |
| ATOM | 2110 | CB | SER | A | 305 | 3.862 | 19.019 | 28.834 | 1.00 | 67.92 | A |
| ATOM | 2111 | OG | SER | A | 305 | 5.045 | 19.700 | 28.446 | 1.00 | 68.06 | A |
| ATOM | 2112 | C | SER | A | 305 | 5.183 | 17.369 | 30.167 | 1.00 | 69.11 | A |
| ATOM | 2113 | O | SER | A | 305 | 5.169 | 16.357 | 30.868 | 1.00 | 69.15 | A |
| ATOM | 2114 | N | LEU | A | 306 | 6.156 | 17.658 | 29.300 | 1.00 | 71.17 | A |
| ATOM | 2115 | CA | LEU | A | 306 | 7.355 | 16.839 | 29.087 | 1.00 | 72.51 | A |
| ATOM | 2116 | CB | LEU | A | 306 | 7.766 | 16.111 | 30.389 | 1.00 | 72.72 | A |
| ATOM | 2117 | CG | LEU | A | 306 | 9.272 | 15.973 | 30.641 | 1.00 | 72.18 | A |
| ATOM | 2118 | CD1 | LEU | A | 306 | 9.821 | 14.794 | 29.833 | 1.00 | 72.39 | A |
| ATOM | 2119 | CD2 | LEU | A | 306 | 9.981 | 17.280 | 30.265 | 1.00 | 72.96 | A |
| ATOM | 2120 | C | LEU | A | 306 | 7.234 | 15.822 | 27.941 | 1.00 | 74.26 | A |
| ATOM | 2121 | O | LEU | A | 306 | 6.686 | 14.734 | 28.109 | 1.00 | 74.08 | A |
| ATOM | 2122 | N | PRO | A | 307 | 7.744 | 16.191 | 26.752 | 1.00 | 76.24 | A |
| ATOM | 2123 | CD | PRO | A | 307 | 7.946 | 17.619 | 26.440 | 1.00 | 76.31 | A |
| ATOM | 2124 | CA | PRO | A | 307 | 7.754 | 15.399 | 25.516 | 1.00 | 77.08 | A |
| ATOM | 2125 | CB | PRO | A | 307 | 7.116 | 16.303 | 24.565 | 1.00 | 77.43 | A |
| ATOM | 2126 | CG | PRO | A | 307 | 7.768 | 17.659 | 24.922 | 1.00 | 77.14 | A |
| ATOM | 2127 | C | PRO | A | 307 | 9.192 | 15.079 | 25.094 | 1.00 | 78.56 | A |
| ATOM | 2128 | O | PRO | A | 307 | 9.826 | 15.857 | 24.374 | 1.00 | 78.89 | A |
| ATOM | 2129 | N | PRO | A | 308 | 9.716 | 13.918 | 25.523 | 1.00 | 79.80 | A |
| ATOM | 2130 | CD | PRO | A | 308 | 8.983 | 12.917 | 26.318 | 1.00 | 79.91 | A |
| ATOM | 2131 | CA | PRO | A | 308 | 11.081 | 13.448 | 25.227 | 1.00 | 79.76 | A |
| ATOM | 2132 | CB | PRO | A | 308 | 11.155 | 12.087 | 25.964 | 1.00 | 80.06 | A |
| ATOM | 2133 | CG | PRO | A | 308 | 10.101 | 12.200 | 27.028 | 1.00 | 80.16 | A |
| ATOM | 2134 | C | PRO | A | 308 | 11.492 | 13.319 | 23.754 | 1.00 | 80.27 | A |
| ATOM | 2135 | O | PRO | A | 308 | 12.095 | 14.257 | 23.167 | 1.00 | 80.20 | A |
| ATOM | 2136 | N | GLY | A | 309 | 11.188 | 12.151 | 23.177 | 1.00 | 80.57 | A |
| ATOM | 2137 | CA | GLY | A | 309 | 11.533 | 11.862 | 21.792 | 1.00 | 80.12 | A |
| ATOM | 2138 | C | GLY | A | 309 | 12.988 | 12.187 | 21.472 | 1.00 | 80.07 | A |
| ATOM | 2139 | O | GLY | A | 309 | 13.247 | 13.013 | 20.592 | 1.00 | 79.78 | A |
| ATOM | 2140 | N | GLU | A | 310 | 13.924 | 11.528 | 22.169 | 1.00 | 79.43 | A |
| ATOM | 2141 | CA | GLU | A | 310 | 15.371 | 11.758 | 22.004 | 1.00 | 78.65 | A |
| ATOM | 2142 | CB | GLU | A | 310 | 16.199 | 10.444 | 22.206 | 1.00 | 78.65 | A |
| ATOM | 2143 | CG | GLU | A | 310 | 17.711 | 10.731 | 22.336 | 1.00 | 79.62 | A |
| ATOM | 2144 | CD | GLU | A | 310 | 18.606 | 9.536 | 22.024 | 1.00 | 80.56 | A |
| ATOM | 2145 | OE1 | GLU | A | 310 | 18.259 | 8.398 | 22.408 | 1.00 | 84.08 | A |
| ATOM | 2146 | OE2 | GLU | A | 310 | 19.676 | 9.743 | 21.408 | 1.00 | 82.78 | A |
| ATOM | 2147 | C | GLU | A | 310 | 15.826 | 12.385 | 20.688 | 1.00 | 77.80 | A |
| ATOM | 2148 | O | GLU | A | 310 | 16.461 | 11.723 | 19.852 | 1.00 | 78.32 | A |
| ATOM | 2149 | N | ASP | A | 311 | 15.522 | 13.665 | 20.514 | 1.00 | 76.10 | A |
| ATOM | 2150 | CA | ASP | A | 311 | 15.924 | 14.375 | 19.314 | 1.00 | 74.85 | A |
| ATOM | 2151 | CB | ASP | A | 311 | 15.229 | 15.738 | 19.248 | 1.00 | 74.95 | A |
| ATOM | 2152 | CG | ASP | A | 311 | 13.873 | 15.746 | 19.949 | 1.00 | 77.37 | A |
| ATOM | 2153 | OD1 | ASP | A | 311 | 13.802 | 15.409 | 21.155 | 1.00 | 79.37 | A |
| ATOM | 2154 | OD2 | ASP | A | 311 | 12.874 | 16.100 | 19.288 | 1.00 | 81.13 | A |
| ATOM | 2155 | C | ASP | A | 311 | 17.423 | 14.572 | 19.466 | 1.00 | 71.53 | A |
| ATOM | 2156 | O | ASP | A | 311 | 18.106 | 13.765 | 20.114 | 1.00 | 71.79 | A |
| ATOM | 2157 | N | GLY | A | 312 | 17.930 | 15.641 | 18.867 | 1.00 | 67.80 | A |

125

| ATOM | 2158 | CA | GLY | A | 312 | 19.341 | 15.951 | 18.984 | 1.00 | 63.45 | A |
|------|------|-----|-----|---|-----|--------|--------|--------|------|-------|---|
| ATOM | 2159 | C | GLY | A | 312 | 19.446 | 17.192 | 19.850 | 1.00 | 61.93 | A |
| ATOM | 2160 | O | GLY | A | 312 | 20.279 | 18.056 | 19.597 | 1.00 | 62.83 | A |
| ATOM | 2161 | N | ARG | A | 313 | 18.584 | 17.262 | 20.868 | 1.00 | 57.09 | A |
| ATOM | 2162 | CA | ARG | A | 313 | 18.503 | 18.387 | 21.799 | 1.00 | 54.38 | A |
| ATOM | 2163 | CB | ARG | A | 313 | 18.553 | 19.698 | 21.063 | 1.00 | 54.83 | A |
| ATOM | 2164 | CG | ARG | A | 313 | 19.878 | 20.422 | 21.160 | 1.00 | 56.43 | A |
| ATOM | 2165 | CD | ARG | A | 313 | 20.114 | 21.304 | 19.935 | 1.00 | 58.22 | A |
| ATOM | 2166 | NE | ARG | A | 313 | 21.445 | 21.906 | 19.951 | 1.00 | 61.69 | A |
| ATOM | 2167 | CZ | ARG | A | 313 | 21.737 | 23.066 | 20.532 | 1.00 | 63.26 | A |
| ATOM | 2168 | NH1 | ARG | A | 313 | 20.787 | 23.764 | 21.142 | 1.00 | 65.12 | A |
| ATOM | 2169 | NH2 | ARG | A | 313 | 22.985 | 23.521 | 20.517 | 1.00 | 62.20 | A |
| ATOM | 2170 | C | ARG | A | 313 | 17.162 | 18.292 | 22.505 | 1.00 | 50.28 | A |
| ATOM | 2171 | O | ARG | A | 313 | 16.388 | 17.376 | 22.246 | 1.00 | 50.62 | A |
| ATOM | 2172 | N | VAL | A | 314 | 16.897 | 19.255 | 23.389 | 1.00 | 44.22 | A |
| ATOM | 2173 | CA | VAL | A | 314 | 15.653 | 19.328 | 24.168 | 1.00 | 40.87 | A |
| ATOM | 2174 | CB | VAL | A | 314 | 15.804 | 18.672 | 25.570 | 1.00 | 38.69 | A |
| ATOM | 2175 | CG1 | VAL | A | 314 | 16.006 | 17.151 | 25.436 | 1.00 | 37.61 | A |
| ATOM | 2176 | CG2 | VAL | A | 314 | 16.969 | 19.297 | 26.313 | 1.00 | 35.86 | A |
| ATOM | 2177 | C | VAL | A | 314 | 15.316 | 20.797 | 24.369 | 1.00 | 36.27 | A |
| ATOM | 2178 | O | VAL | A | 314 | 16.095 | 21.651 | 24.034 | 1.00 | 32.78 | A |
| ATOM | 2179 | N | GLU | A | 315 | 14.146 | 21.090 | 24.912 | 1.00 | 34.14 | A |
| ATOM | 2180 | CA | GLU | A | 315 | 13.772 | 22.476 | 25.148 | 1.00 | 34.06 | A |
| ATOM | 2181 | CB | GLU | A | 315 | 12.390 | 22.708 | 24.700 | 1.00 | 35.71 | A |
| ATOM | 2182 | CG | GLU | A | 315 | 12.114 | 22.283 | 23.277 | 1.00 | 41.96 | A |
| ATOM | 2183 | CD | GLU | A | 315 | 11.738 | 23.461 | 22.392 | 1.00 | 52.76 | A |
| ATOM | 2184 | OE1 | GLU | A | 315 | 11.250 | 24.471 | 22.939 | 1.00 | 60.01 | A |
| ATOM | 2185 | OE2 | GLU | A | 315 | 11.913 | 23.379 | 21.155 | 1.00 | 59.06 | A |
| ATOM | 2186 | C | GLU | A | 315 | 13.878 | 22.803 | 26.638 | 1.00 | 30.74 | A |
| ATOM | 2187 | O | GLU | A | 315 | 13.682 | 21.934 | 27.492 | 1.00 | 29.27 | A |
| ATOM | 2188 | N | PRO | A | 316 | 14.205 | 24.060 | 26.973 | 1.00 | 27.71 | A |
| ATOM | 2189 | CD | PRO | A | 316 | 14.650 | 25.148 | 26.097 | 1.00 | 27.55 | A |
| ATOM | 2190 | CA | PRO | A | 316 | 14.331 | 24.472 | 28.364 | 1.00 | 26.18 | A |
| ATOM | 2191 | CB | PRO | A | 316 | 15.076 | 25.751 | 28.261 | 1.00 | 25.10 | A |
| ATOM | 2192 | CG | PRO | A | 316 | 14.598 | 26.318 | 27.028 | 1.00 | 28.54 | A |
| ATOM | 2193 | C | PRO | A | 316 | 12.965 | 24.683 | 28.963 | 1.00 | 25.52 | A |
| ATOM | 2194 | O | PRO | A | 316 | 12.064 | 25.064 | 28.267 | 1.00 | 26.70 | A |
| ATOM | 2195 | N | TYR | A | 317 | 12.824 | 24.417 | 30.260 | 1.00 | 23.91 | A |
| ATOM | 2196 | CA | TYR | A | 317 | 11.555 | 24.630 | 30.939 | 1.00 | 22.47 | A |
| ATOM | 2197 | CB | TYR | A | 317 | 11.524 | 23.962 | 32.279 | 1.00 | 21.58 | A |
| ATOM | 2198 | CG | TYR | A | 317 | 10.304 | 24.342 | 33.084 | 1.00 | 19.49 | A |
| ATOM | 2199 | CD1 | TYR | A | 317 | 9.021 | 24.217 | 32.542 | 1.00 | 20.35 | A |
| ATOM | 2200 | CE1 | TYR | A | 317 | 7.882 | 24.560 | 33.264 | 1.00 | 19.98 | A |
| ATOM | 2201 | CD2 | TYR | A | 317 | 10.419 | 24.823 | 34.376 | 1.00 | 19.61 | A |
| ATOM | 2202 | CE2 | TYR | A | 317 | 9.280 | 25.172 | 35.113 | 1.00 | 19.24 | A |
| ATOM | 2203 | CZ | TYR | A | 317 | 8.011 | 25.032 | 34.546 | 1.00 | 20.96 | A |
| ATOM | 2204 | OH | TYR | A | 317 | 6.871 | 25.328 | 35.267 | 1.00 | 21.28 | A |
| ATOM | 2205 | C | TYR | A | 317 | 11.423 | 26.127 | 31.138 | 1.00 | 21.58 | A |
| ATOM | 2206 | O | TYR | A | 317 | 10.393 | 26.701 | 30.873 | 1.00 | 20.15 | A |
| ATOM | 2207 | N | VAL | A | 318 | 12.487 | 26.753 | 31.622 | 1.00 | 20.74 | A |
| ATOM | 2208 | CA | VAL | A | 318 | 12.478 | 28.189 | 31.820 | 1.00 | 21.58 | A |
| ATOM | 2209 | CB | VAL | A | 318 | 13.714 | 28.632 | 32.583 | 1.00 | 20.16 | A |
| ATOM | 2210 | CG1 | VAL | A | 318 | 13.687 | 28.053 | 33.969 | 1.00 | 21.78 | A |
| ATOM | 2211 | CG2 | VAL | A | 318 | 14.948 | 28.183 | 31.845 | 1.00 | 18.31 | A |

```
ATOM   2212  C    VAL A 318    12.457  28.872  30.444  1.00  21.57      A
ATOM   2213  O    VAL A 318    12.554  28.204  29.411  1.00  20.75      A
ATOM   2214  N    ASP A 319    12.327  30.196  30.431  1.00  23.34      A
ATOM   2215  CA   ASP A 319    12.288  30.943  29.179  1.00  24.62      A
ATOM   2216  CB   ASP A 319    10.951  31.629  29.057  1.00  26.78      A
ATOM   2217  CG   ASP A 319    10.774  32.354  27.739  1.00  29.38      A
ATOM   2218  OD1  ASP A 319    11.795  32.649  27.076  1.00  33.18      A
ATOM   2219  OD2  ASP A 319     9.604  32.640  27.379  1.00  44.47      A
ATOM   2220  C    ASP A 319    13.414  31.973  29.202  1.00  24.64      A
ATOM   2221  O    ASP A 319    13.573  32.675  30.176  1.00  24.72      A
ATOM   2222  N    PHE A 320    14.188  32.056  28.121  1.00  23.78      A
ATOM   2223  CA   PHE A 320    15.314  32.992  28.049  1.00  24.19      A
ATOM   2224  CB   PHE A 320    16.541  32.262  27.495  1.00  23.35      A
ATOM   2225  CG   PHE A 320    17.135  31.295  28.450  1.00  23.10      A
ATOM   2226  CD1  PHE A 320    17.083  29.945  28.199  1.00  21.44      A
ATOM   2227  CD2  PHE A 320    17.679  31.730  29.650  1.00  23.50      A
ATOM   2228  CE1  PHE A 320    17.563  29.023  29.144  1.00  24.96      A
ATOM   2229  CE2  PHE A 320    18.156  30.827  30.593  1.00  23.33      A
ATOM   2230  CZ   PHE A 320    18.095  29.469  30.338  1.00  22.32      A
ATOM   2231  C    PHE A 320    15.052  34.279  27.243  1.00  23.84      A
ATOM   2232  O    PHE A 320    15.958  35.036  26.960  1.00  23.87      A
ATOM   2233  N    ALA A 321    13.797  34.511  26.887  1.00  24.86      A
ATOM   2234  CA   ALA A 321    13.435  35.694  26.140  1.00  25.82      A
ATOM   2235  CB   ALA A 321    11.977  35.706  25.910  1.00  26.52      A
ATOM   2236  C    ALA A 321    13.853  36.950  26.892  1.00  24.73      A
ATOM   2237  O    ALA A 321    14.542  37.777  26.371  1.00  23.60      A
ATOM   2238  N    GLU A 322    13.418  37.078  28.135  1.00  23.48      A
ATOM   2239  CA   GLU A 322    13.757  38.242  28.946  1.00  24.47      A
ATOM   2240  CB   GLU A 322    13.164  38.097  30.324  1.00  23.90      A
ATOM   2241  CG   GLU A 322    13.139  39.370  31.137  1.00  28.85      A
ATOM   2242  CD   GLU A 322    12.449  40.502  30.415  1.00  33.38      A
ATOM   2243  OE1  GLU A 322    11.811  40.271  29.359  1.00  33.65      A
ATOM   2244  OE2  GLU A 322    12.539  41.636  30.919  1.00  30.88      A
ATOM   2245  C    GLU A 322    15.261  38.449  29.074  1.00  24.42      A
ATOM   2246  O    GLU A 322    15.743  39.583  29.055  1.00  23.76      A
ATOM   2247  N    PHE A 323    15.996  37.351  29.224  1.00  22.50      A
ATOM   2248  CA   PHE A 323    17.440  37.423  29.355  1.00  22.53      A
ATOM   2249  CB   PHE A 323    18.033  36.026  29.495  1.00  21.15      A
ATOM   2250  CG   PHE A 323    19.519  35.972  29.263  1.00  21.61      A
ATOM   2251  CD1  PHE A 323    20.389  36.584  30.143  1.00  21.85      A
ATOM   2252  CD2  PHE A 323    20.038  35.346  28.147  1.00  21.32      A
ATOM   2253  CE1  PHE A 323    21.735  36.575  29.915  1.00  22.95      A
ATOM   2254  CE2  PHE A 323    21.383  35.336  27.914  1.00  24.36      A
ATOM   2255  CZ   PHE A 323    22.232  35.950  28.798  1.00  23.77      A
ATOM   2256  C    PHE A 323    18.065  38.108  28.145  1.00  21.57      A
ATOM   2257  O    PHE A 323    18.830  39.045  28.287  1.00  21.69      A
ATOM   2258  N    TYR A 324    17.742  37.621  26.951  1.00  22.17      A
ATOM   2259  CA   TYR A 324    18.299  38.192  25.741  1.00  23.50      A
ATOM   2260  CB   TYR A 324    17.850  37.467  24.552  1.00  23.75      A
ATOM   2261  CG   TYR A 324    18.421  36.094  24.418  1.00  25.64      A
ATOM   2262  CD1  TYR A 324    17.608  34.972  24.492  1.00  24.03      A
ATOM   2263  CE1  TYR A 324    18.118  33.710  24.313  1.00  25.58      A
ATOM   2264  CD2  TYR A 324    19.762  35.916  24.171  1.00  25.74      A
ATOM   2265  CE2  TYR A 324    20.285  34.658  23.994  1.00  26.07      A
```

| ATOM | 2266 | CZ | TYR | A | 324 | 19.456 | 33.559 | 24.063 | 1.00 | 25.06 | A |
|------|------|-----|-----|---|-----|--------|--------|--------|------|-------|---|
| ATOM | 2267 | OH | TYR | A | 324 | 19.979 | 32.311 | 23.876 | 1.00 | 27.89 | A |
| ATOM | 2268 | C | TYR | A | 324 | 17.863 | 39.616 | 25.617 | 1.00 | 24.11 | A |
| ATOM | 2269 | O | TYR | A | 324 | 18.655 | 40.453 | 25.342 | 1.00 | 23.90 | A |
| ATOM | 2270 | N | ARG | A | 325 | 16.582 | 39.886 | 25.819 | 1.00 | 25.52 | A |
| ATOM | 2271 | CA | ARG | A | 325 | 16.117 | 41.254 | 25.701 | 1.00 | 26.11 | A |
| ATOM | 2272 | CB | ARG | A | 325 | 14.693 | 41.350 | 26.061 | 1.00 | 26.47 | A |
| ATOM | 2273 | CG | ARG | A | 325 | 14.259 | 42.722 | 26.516 | 1.00 | 27.99 | A |
| ATOM | 2274 | CD | ARG | A | 325 | 12.751 | 42.776 | 26.719 | 1.00 | 32.21 | A |
| ATOM | 2275 | NE | ARG | A | 325 | 12.040 | 42.800 | 25.442 | 1.00 | 40.02 | A |
| ATOM | 2276 | CZ | ARG | A | 325 | 11.303 | 41.803 | 24.968 | 1.00 | 40.10 | A |
| ATOM | 2277 | NH1 | ARG | A | 325 | 11.166 | 40.687 | 25.668 | 1.00 | 40.11 | A |
| ATOM | 2278 | NH2 | ARG | A | 325 | 10.712 | 41.922 | 23.788 | 1.00 | 43.37 | A |
| ATOM | 2279 | C | ARG | A | 325 | 16.945 | 42.143 | 26.606 | 1.00 | 25.83 | A |
| ATOM | 2280 | O | ARG | A | 325 | 17.392 | 43.159 | 26.182 | 1.00 | 25.86 | A |
| ATOM | 2281 | N | LEU | A | 326 | 17.153 | 41.720 | 27.852 | 1.00 | 24.77 | A |
| ATOM | 2282 | CA | LEU | A | 326 | 17.944 | 42.475 | 28.827 | 1.00 | 25.18 | A |
| ATOM | 2283 | CB | LEU | A | 326 | 17.784 | 41.886 | 30.162 | 1.00 | 24.49 | A |
| ATOM | 2284 | CG | LEU | A | 326 | 16.603 | 42.369 | 30.862 | 1.00 | 24.73 | A |
| ATOM | 2285 | CD1 | LEU | A | 326 | 16.458 | 41.700 | 32.213 | 1.00 | 22.91 | A |
| ATOM | 2286 | CD2 | LEU | A | 326 | 16.752 | 43.858 | 31.011 | 1.00 | 25.72 | A |
| ATOM | 2287 | C | LEU | A | 326 | 19.425 | 42.498 | 28.487 | 1.00 | 25.20 | A |
| ATOM | 2288 | O | LEU | A | 326 | 20.117 | 43.434 | 28.773 | 1.00 | 24.10 | A |
| ATOM | 2289 | N | TRP | A | 327 | 19.902 | 41.424 | 27.888 | 1.00 | 26.11 | A |
| ATOM | 2290 | CA | TRP | A | 327 | 21.288 | 41.336 | 27.495 | 1.00 | 28.01 | A |
| ATOM | 2291 | CB | TRP | A | 327 | 21.582 | 39.939 | 27.078 | 1.00 | 27.10 | A |
| ATOM | 2292 | CG | TRP | A | 327 | 22.971 | 39.698 | 26.613 | 1.00 | 28.79 | A |
| ATOM | 2293 | CD2 | TRP | A | 327 | 24.063 | 39.198 | 27.398 | 1.00 | 26.56 | A |
| ATOM | 2294 | CE2 | TRP | A | 327 | 25.163 | 39.050 | 26.530 | 1.00 | 28.79 | A |
| ATOM | 2295 | CE3 | TRP | A | 327 | 24.215 | 38.857 | 28.754 | 1.00 | 25.38 | A |
| ATOM | 2296 | CD1 | TRP | A | 327 | 23.439 | 39.839 | 25.349 | 1.00 | 28.06 | A |
| ATOM | 2297 | NE1 | TRP | A | 327 | 24.754 | 39.450 | 25.286 | 1.00 | 29.80 | A |
| ATOM | 2298 | CZ2 | TRP | A | 327 | 26.407 | 38.572 | 26.966 | 1.00 | 27.11 | A |
| ATOM | 2299 | CZ3 | TRP | A | 327 | 25.449 | 38.382 | 29.193 | 1.00 | 28.59 | A |
| ATOM | 2300 | CH2 | TRP | A | 327 | 26.531 | 38.243 | 28.295 | 1.00 | 26.36 | A |
| ATOM | 2301 | C | TRP | A | 327 | 21.531 | 42.291 | 26.346 | 1.00 | 28.87 | A |
| ATOM | 2302 | O | TRP | A | 327 | 22.523 | 42.901 | 26.291 | 1.00 | 27.65 | A |
| ATOM | 2303 | N | SER | A | 328 | 20.572 | 42.399 | 25.437 | 1.00 | 30.75 | A |
| ATOM | 2304 | CA | SER | A | 328 | 20.679 | 43.274 | 24.275 | 1.00 | 32.76 | A |
| ATOM | 2305 | CB | SER | A | 328 | 19.492 | 43.040 | 23.337 | 1.00 | 32.34 | A |
| ATOM | 2306 | OG | SER | A | 328 | 19.549 | 43.878 | 22.195 | 1.00 | 39.99 | A |
| ATOM | 2307 | C | SER | A | 328 | 20.729 | 44.744 | 24.662 | 1.00 | 33.57 | A |
| ATOM | 2308 | O | SER | A | 328 | 21.445 | 45.528 | 24.048 | 1.00 | 33.25 | A |
| ATOM | 2309 | N | VAL | A | 329 | 19.953 | 45.109 | 25.679 | 1.00 | 35.17 | A |
| ATOM | 2310 | CA | VAL | A | 329 | 19.898 | 46.481 | 26.163 | 1.00 | 37.51 | A |
| ATOM | 2311 | CB | VAL | A | 329 | 18.816 | 46.650 | 27.228 | 1.00 | 37.26 | A |
| ATOM | 2312 | CG1 | VAL | A | 329 | 18.948 | 47.976 | 27.881 | 1.00 | 37.99 | A |
| ATOM | 2313 | CG2 | VAL | A | 329 | 17.455 | 46.526 | 26.595 | 1.00 | 37.82 | A |
| ATOM | 2314 | C | VAL | A | 329 | 21.227 | 46.943 | 26.733 | 1.00 | 40.03 | A |
| ATOM | 2315 | O | VAL | A | 329 | 21.568 | 48.093 | 26.627 | 1.00 | 40.17 | A |
| ATOM | 2316 | N | ASP | A | 330 | 21.978 | 46.037 | 27.337 | 1.00 | 42.63 | A |
| ATOM | 2317 | CA | ASP | A | 330 | 23.273 | 46.401 | 27.890 | 1.00 | 44.26 | A |
| ATOM | 2318 | CB | ASP | A | 330 | 23.711 | 45.373 | 28.912 | 1.00 | 44.68 | A |
| ATOM | 2319 | CG | ASP | A | 330 | 23.501 | 45.835 | 30.334 | 1.00 | 44.46 | A |

| ATOM | 2320 | OD1 | ASP | A | 330 | 22.658 | 46.722 | 30.549 | 1.00 | 45.00 | A |
|------|------|-----|-----|---|-----|--------|--------|--------|------|-------|---|
| ATOM | 2321 | OD2 | ASP | A | 330 | 24.164 | 45.301 | 31.244 | 1.00 | 44.38 | A |
| ATOM | 2322 | C | ASP | A | 330 | 24.317 | 46.484 | 26.787 | 1.00 | 45.62 | A |
| ATOM | 2323 | O | ASP | A | 330 | 25.020 | 47.443 | 26.686 | 1.00 | 46.41 | A |
| ATOM | 2324 | N | HIS | A | 331 | 24.374 | 45.457 | 25.950 | 1.00 | 48.65 | A |
| ATOM | 2325 | CA | HIS | A | 331 | 25.353 | 45.364 | 24.870 | 1.00 | 50.92 | A |
| ATOM | 2326 | CB | HIS | A | 331 | 25.821 | 43.917 | 24.738 | 1.00 | 49.90 | A |
| ATOM | 2327 | CG | HIS | A | 331 | 26.123 | 43.258 | 26.046 | 1.00 | 48.14 | A |
| ATOM | 2328 | CD2 | HIS | A | 331 | 25.441 | 42.330 | 26.756 | 1.00 | 43.21 | A |
| ATOM | 2329 | ND1 | HIS | A | 331 | 27.247 | 43.553 | 26.785 | 1.00 | 46.25 | A |
| ATOM | 2330 | CE1 | HIS | A | 331 | 27.245 | 42.835 | 27.893 | 1.00 | 43.23 | A |
| ATOM | 2331 | NE2 | HIS | A | 331 | 26.158 | 42.085 | 27.900 | 1.00 | 44.66 | A |
| ATOM | 2332 | C | HIS | A | 331 | 24.903 | 45.847 | 23.495 | 1.00 | 54.86 | A |
| ATOM | 2333 | O | HIS | A | 331 | 24.900 | 47.050 | 23.210 | 1.00 | 56.50 | A |
| ATOM | 2334 | N | GLY | A | 332 | 24.559 | 44.887 | 22.636 | 1.00 | 57.61 | A |
| ATOM | 2335 | CA | GLY | A | 332 | 24.124 | 45.201 | 21.283 | 1.00 | 58.93 | A |
| ATOM | 2336 | C | GLY | A | 332 | 22.925 | 44.389 | 20.808 | 1.00 | 61.02 | A |
| ATOM | 2337 | O | GLY | A | 332 | 22.042 | 44.922 | 20.119 | 1.00 | 62.36 | A |
| ATOM | 2338 | N | GLU | A | 333 | 22.905 | 43.100 | 21.157 | 1.00 | 62.66 | A |
| ATOM | 2339 | CA | GLU | A | 333 | 21.809 | 42.189 | 20.804 | 1.00 | 62.67 | A |
| ATOM | 2340 | CB | GLU | A | 333 | 21.551 | 42.175 | 19.247 | 1.00 | 63.80 | A |
| ATOM | 2341 | CG | GLU | A | 333 | 20.655 | 41.013 | 18.740 | 1.00 | 64.96 | A |
| ATOM | 2342 | CD | GLU | A | 333 | 19.202 | 41.084 | 19.223 | 1.00 | 68.35 | A |
| ATOM | 2343 | OE1 | GLU | A | 333 | 18.515 | 42.083 | 18.917 | 1.00 | 69.46 | A |
| ATOM | 2344 | OE2 | GLU | A | 333 | 18.741 | 40.139 | 19.902 | 1.00 | 72.12 | A |
| ATOM | 2345 | C | GLU | A | 333 | 22.111 | 40.772 | 21.284 | 1.00 | 62.94 | A |
| TER | 2348 | | GLU | A | 333 | | | | | | A |
| ATOM | 2349 | C1 | VRT | B | 1 | 13.252 | 20.004 | 55.373 | 1.00 | 31.89 | B |
| ATOM | 2350 | N1 | VRT | B | 1 | 13.979 | 20.577 | 56.376 | 1.00 | 31.89 | B |
| ATOM | 2351 | C3 | VRT | B | 1 | 15.217 | 19.983 | 56.754 | 1.00 | 31.89 | B |
| ATOM | 2352 | C4 | VRT | B | 1 | 15.714 | 18.814 | 56.138 | 1.00 | 31.89 | B |
| ATOM | 2353 | C5 | VRT | B | 1 | 14.927 | 18.267 | 55.123 | 1.00 | 31.89 | B |
| ATOM | 2354 | C6 | VRT | B | 1 | 13.748 | 18.807 | 54.715 | 1.00 | 31.89 | B |
| ATOM | 2355 | N7 | VRT | B | 1 | 16.158 | 20.408 | 57.687 | 1.00 | 31.89 | B |
| ATOM | 2356 | C8 | VRT | B | 1 | 17.191 | 19.469 | 57.606 | 1.00 | 31.89 | B |
| ATOM | 2357 | C9 | VRT | B | 1 | 16.955 | 18.432 | 56.633 | 1.00 | 31.89 | B |
| ATOM | 2358 | C2 | VRT | B | 1 | 13.129 | 18.010 | 53.589 | 1.00 | 31.89 | B |
| ATOM | 2359 | O1 | VRT | B | 1 | 13.611 | 17.450 | 52.562 | 1.00 | 31.89 | B |
| ATOM | 2360 | O2 | VRT | B | 1 | 11.709 | 18.010 | 54.232 | 1.00 | 31.89 | B |
| ATOM | 2361 | C11 | VRT | B | 1 | 9.986 | 18.329 | 54.071 | 1.00 | 31.89 | B |
| ATOM | 2362 | C81 | VRT | B | 1 | 19.169 | 15.001 | 55.180 | 1.00 | 31.89 | B |
| ATOM | 2363 | C82 | VRT | B | 1 | 19.679 | 15.777 | 56.262 | 1.00 | 31.89 | B |
| ATOM | 2364 | C83 | VRT | B | 1 | 18.961 | 16.903 | 56.747 | 1.00 | 31.89 | B |
| ATOM | 2365 | C84 | VRT | B | 1 | 17.737 | 17.257 | 56.156 | 1.00 | 31.89 | B |
| ATOM | 2366 | N85 | VRT | B | 1 | 17.281 | 16.468 | 55.101 | 1.00 | 31.89 | B |
| ATOM | 2367 | C86 | VRT | B | 1 | 17.917 | 15.381 | 54.590 | 1.00 | 31.89 | B |
| ATOM | 2368 | C91 | VRT | B | 1 | 17.913 | 13.646 | 51.483 | 1.00 | 31.89 | B |
| ATOM | 2369 | C92 | VRT | B | 1 | 18.246 | 14.855 | 52.318 | 1.00 | 31.89 | B |
| ATOM | 2370 | N92 | VRT | B | 1 | 17.380 | 14.650 | 53.506 | 1.00 | 31.89 | B |
| ATOM | 2371 | C94 | VRT | B | 1 | 16.149 | 15.213 | 52.928 | 1.00 | 31.89 | B |
| ATOM | 2372 | C95 | VRT | B | 1 | 15.470 | 14.052 | 52.252 | 1.00 | 31.89 | B |
| ATOM | 2373 | N91 | VRT | B | 1 | 16.435 | 13.555 | 51.269 | 1.00 | 31.89 | B |
| ATOM | 2374 | C93 | VRT | B | 1 | 18.321 | 12.426 | 52.204 | 1.00 | 31.89 | B |
| ATOM | 2375 | C97 | VRT | B | 1 | 15.181 | 12.984 | 53.275 | 1.00 | 31.89 | B |

```
ATOM   2376  C99 VRT B   1        16.020  12.942  50.134  1.00 31.89        B
ATOM   2377  O91 VRT B   1        16.600  11.943  49.684  1.00 31.89        B
ATOM   2378  C98 VRT B   1        14.803  13.566  49.480  1.00 31.89        B
END
```

Figure 3: Ribbon diagram of overall adenosine complex

Figure 4: detail of catalytic site in adenosine complex

Figure 5: Detail of TAB1 binding site on TAK1 kinase domain

Figure 6: Sequence alignment of C-terminus (Phe439 in AKT2, Phe327 in PKA, Phe319 in TAK1 and Phe484 in TAB1)

```
AKT2  VDTRYFDDEFTAQSITIT

PKA   GDTSNFDD-YEEEEIRVZ
TAK1  TSTGSFMD-IASTNTSNK
TAB1  EPYVDFAEFYRLWSVDHG
            F484 in
             TAB1
```

Figure 7: Sequence alignment of N-terminus

```
FGF    -----------MVAGVSEYELPEDP-RWELPRDRLVLGK
VEGF   -------MDPDELPLDEHCERLPYDASKWEFPRDRLKLGK
TIE2   ----------MKKHHHHHHGKNNPDPTIYPVLDWNDIKFQD
BTK    ----------------------------------IDPKDLTFLK
SRC    -------------MGGSVAAQDEFYRS-GWALNMKELKLLQ
FAK    -----------------------GAMGSSTRDYEIQRERIELGR
TAK1   MSTASAASSSSSSSSAGEMIEAPSQVLNFEEIDYKEIEVEE
A2     -----------------------GAMGSKRQWALEDFEIGR
```

I31 in
TAK1

FIG. 8

FIG. 9

~100

~102

~101

~102

FIG. 10

113

~110

~114

~112

~111

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 60337513 B
- US 4886646 A
- US 5096676 A
- US 5130105 A
- US 5221410 A
- US 5400741 A
- US 5884230 A

### Non-patent literature cited in the description

- **Hardie, G. ; Hanks, S.** The Protein Kinase Facts Book. Academic Press, 1995, vol. I, II
- **Hanks, S.K. ; Hunter, T.** *FASEB J.,* 1995, vol. 9, 576-596
- **Knighton et al.** *Science,* 1991, vol. 253, 407-414
- **Hiles et al.** *Cell,* 1992, vol. 70, 419-429
- **Kunz et al.** *Cell,* 1993, vol. 73, 585-596
- **Garcia-Bustos et al.** *EMBO J.,* 1994, vol. 13, 2352-2361
- **Davis D.J.** *Trends in Biochem Sci.,* 1994, vol. 19, 470-473
- **Su B. ; Karin M.** *Curr. Opin. Immunol,* 1996, vol. 8, 402-411
- **Treisman R.** *Curr. Opin. Cell Biol.,* 1996, vol. 8, 205-215
- **Yamaguchi K et al.** *Science,* 1995, vol. 270, 2008-11
- **Ninomiya-Tsuji J et al.** *Nature,* 1999, vol. 398, 252-256
- **Sakurai H. et al.** *J. Biol. Chem,* 1999, vol. 274, 10641-10648
- **Irie T. et al.** *FEBS Lett.,* 2000, vol. 467, 160-164
- **Lee J. et al.** *J. Leukoc Biol.,* 2000, vol. 68, 909-915
- **Mizukami J et al.** *Mol. Cell. Biol.,* 2002, vol. 22, 992-1000
- **Wald D. et al.** *J. Immunol,* 2002, vol. 31, 3747-3754
- **Wang W. et al.** *J. Biol. Chem.,* 1997, vol. 272, 22771-22775
- **Moriguchi T. et al.** *J. Biol. Chem.,* 1996, vol. 271, 13675-13679
- **Barkett M ; Gilmore TD.** *Oncogene,* 1999, vol. 18, 6910-6924
- **Takaesu G. et al.** *J. Mol. Biol,* 2003, vol. 326, 110-115
- **Shibuya H. et al.** *EMBO J.,* 1998, vol. 17, 1019-1028
- **Meneghini M.D. et al.** *Nature,* 1999, vol. 399, 793-797
- **Ishitani T. et al.** *Nature,* 1999, vol. 399, 798-802
- **Takaesu G. et al.** *J. Mol. Biol.,* 2003, vol. 326, 105-115
- **Zhang D. et al.** *Nature Med.,* 2000, vol. 6, 556-563
- **Shibuya H et al.** *Science,* 1996, vol. 272, 1179-1182
- **Ono K. et al.** *J. Biol. Chem.,* 2001, vol. 276, 24396-24400
- **Sakurai H. et al.** *FEBS Lett,* 2000, vol. 474, 141-145
- **Kishimoto K. et al.** *J. Biol. Chem.,* 2000, vol. 275, 7359-7364
- **Irie T et al.** *FEBS Lett.,* 2000, vol. 467, 160-164
- **Sakurai H. et al.** *J. Biol. Chem.,* 1999, vol. 274, 10641-10648
- **Vidal S. et al.** *Genes Dev.,* 1999, vol. 15, 1900-1912
- **Ninomiya-Tsuji J. et al.** *J. Biol. Chem.,* 2003, vol. 278, 18485
- **Wilson et al.** *J. Biol. Chem.,* 1996, vol. 271, 27696-27700
- **Dayhoff et al.** Atlas of Protein Sequence and Structure. 1978, vol. 5, 345-352
- **Smith ; Waterman.** *Adv. Appl. Math.,* 1981, vol. 2, 482
- **Xie et al.** *Structure,* 1998, vol. 6, 983-991
- **R. Giet ; C. Prigent.** *J. Cell Sci.,* 1999, vol. 112, 3591-3601
- **Sakurai H. ; Nishi A. ; Sato N. ; Mizukami J. ; Miyoshi H. ; Sugita T.** *Biochem Biophys Res Comm.,* 2002, vol. 297, 1277-1281
- **Frodin M ; Antal TL ; Dummler BA ; Jensen CJ ; Deak M ; Gammeltoft S ; Biondi RM.** *EMBO J.,* 15 October 2002, vol. 21 (20), 5396-407

- **Yang J ; Cron P ; Thompson V ; Good VM ; Hess D ; Hemmings BA ; Barford D.** *Mol Cell,* June 2002, vol. 9 (6), 1227-40
- **Pav et al.** *Proteins: Structure. Function, and Genetics,* 1994, vol. 20, 98-102
- **D'Arcy et al.** *J. Cryst. Growth,* 1996, vol. 168, 175-180
- **Chayen, J.** *Appl. Cryst.,* 1997, vol. 30, 198-202
- **T.A. Jones et al.** *Acta Cryst. A,* 1991, vol. 47, 110-119
- **Carson.** *J. Appl. Cryst.,* 1991, vol. 24, 958-961
- **Gerstein et al.** *J. Mol. Biol.,* 1995, vol. 251, 161-175
- **K. P. Wilson et al.** *J. Biol. Chem.,* 1996, vol. 271, 27696-27700
- **Z. Wang et al.** *Proc. Natl. Acad. Sci. U.S.A.,* 1997, vol. 94, 2327-32
- **Xu, W. et al.** *Cell,* 1999, vol. 3, 629-638
- **Yamaguchi H. ; Hendrickson W.A.** *Nature,* 1996, vol. 384, 484-489
- **Guex et al.** *Electrophoresis,* 1997, vol. 18, 2714-2723
- **Smith ; Waterman.** *Advances in Applied Mathematics,* 1981, vol. 2, 482
- **Hanks et al.** *Science,* 1988, vol. 241, 42
- **Hanks ; Quinn.** *Meth. Enzymol.,* 1991, vol. 200, 38
- **Z. Wang et al.** *Proc. Natl. Acad. Sci. U.S.A.,* 1997, vol. 94, 2327-2332
- **Griffith et al.** *Mol. Cell,* 2004, vol. 13, 169
- **Brown et al.** *J. Biol. Chem.,* 2004, vol. 279, 18727
- **Jones et al.** *Acta Cryst. A,* 1991, vol. 47, 110-119
- **M. Carson.** *J. Appl. Cryst.,* 1991, vol. 24, 958-961
- **Jones et al.** *Acta Crystallogr.,* 1991, vol. A47, 110-119
- **Carson.** *J. Appl. Crystallogr.,* 1991, vol. 24, 9589-961
- **Wishart, D. S. et al.** *Comput Appl. Biosci.,* 1994, vol. 10, 687-88
- **Higgins D. G. et al.** *Methods Enzymol,* 1996, vol. 266, 383-402
- **Waterman.** *Advances in Applied Mathematics,* 1981, vol. 2, 482
- **Jones et al.** *Acta Cryst, Sect. A,* 1991, vol. 47, 110-119
- **Schnare et al.** *J. Mol. Biol,* 1996, vol. 256, 701-719
- **Blundell et al.** *Nature,* 1987, vol. 326, 347-352
- **Fetrow ; Bryant.** *Bio/Technology,* 1993, vol. 11, 479-484
- **Greer.** *Methods in Enzymology,* 1991, vol. 202, 239-252
- **Johnson et al.** *Crit. Rev. Biochem: Mol Biol.,* 1994, vol. 29, 1-68
- **Szklarz G.D.** *Life Sci.,* 1997, vol. 61, 2507-2520
- **P. J. Goodford.** A Computational Procedure for Determining Energetically Favorable Binding Sites on Biologically Important Macromolecules. *J. Med. Chem.,* 1985, vol. 28, 849-857
- **A. Miranker et al.** Functionality Maps of Binding Sites: A Multiple Copy Simultaneous Search Method. *Proteins: Structure, Function and Genetics,* 1991, vol. 11, 29-34
- **D. S. Goodsell et al.** Automated Docking of Substrates to Proteins by Simulated Annealing. *Proteins: Structure, Function, and Genetics,* 1990, vol. 8, 195-202
- **I. D. Kuntz et al.** A Geometric Approach to Macromolecule-Ligand Interactions. *J. Mol. Biol.,* 1982, vol. 161, 269-288
- **P. A. Bartlett et al.** CAVEAT: A Program to Facilitate the Structure-Derived Design of Biologically Active Molecules'', in Molecular Recognition in Chemical and Biological Problems. *Special Pub., Royal Chem. Soc.,* 1989, vol. 78, 182-196
- **G. Lauri ; P. A. Bartlett.** CAVEAT: a Program to Facilitate the Design of Organic Molecules. *J. Comput. Aided Mol. Des.,* 1994, vol. 8, 51-66
- **Y. C. Martin.** 3D Database Searching in Drug Design. *J. Med. Chem.,* 1992, vol. 35, 2145-2154
- **M. B. Eisen et al.** HOOK: A Program for Finding Novel Molecular Architectures that Satisfy the Chemical and Steric Requirements of a Macromolecule Binding Site. *Proteins: Struct., Funct., Genet.,* 1994, vol. 19, 199-221
- **H.-J. Bohm.** The Computer Program LUDI: A New Method for the De Novo Design of Enzyme Inhibitors. *J. Comp. Aid. Molec. Design,* 1992, vol. 6, 61-78
- **Y. Nishibata et al.** *Tetrahedron,* 1991, vol. 47, 8985
- **V. Gillet et al.** SPROUT: A Program for Structure Generation. *J. Comput. Aided Mol. Design,* 1993, vol. 7, 127-153
- **N. C. Cohen et al.** Molecular Modeling Software and Methods for Medicinal Chemistry. *J. Med. Chem.,* 1990, vol. 33, 883-894
- **M. A. Navia ; M. A. Murcko.** The Use of Structural Information in Drug Design. *Current Opinions in Structural Biology,* 1992, vol. 2, 202-210
- A Perspective of Modem Methods in Computer-Aided Drug Design. **L. M. Balbes et al.** Reviews in Computational Chemistry. VCH, 1994, vol. 5, 337-380
- **W. C. Guida.** Software For Structure-Based Drug Design. *Curr. Opin. Struct. Biology,* 1994, vol. 4, 777-781
- **E. C. Meng et al.** *J. Comp. Chem.,* 1992, vol. 13, 505-524

- **E. Lattman.** Use of the Rotation and Translation Functions. *Meth. Enzymol.,* 1985, vol. 115, 55-77
- The Molecular Replacement Method. Int. Sci. Rev. Ser. Gordon & Breach, 1972
- Meth. Enzymol. Academic Press, 1985, vol. 114, 115
- **Brunger et al.** *Acta Crystallogr. D. Biol. Crystallogr.,* 1998, vol. 54, 905-921
- **Fox et al.** *Protein Sci.,* 1998, vol. 7, 2249
- **A.G. Leslie.** *Acta Cryst. D,* 1999, vol. 55, 1696-1702
- Collaborative Computational Project. *N., Acta Cryst. D,* 1994, vol. 50, 760-763
- **J. Navaza.** *Acta. Cryst. A,* 1994, vol. 50, 157-163
- **S. K. Hanks et al.** *Science,* 1988, vol. 241, 42-52
- **Hanks, S.K. ; A.M. Quinn.** *Meth. Enzymol.,* 1991, vol. 200, 38-62
- **J. A. Tainer et al.** *Nature,* 1984, vol. 312, 127-134